# EUROPEAN PATENT APPLICATION

(11) **EP 3 192 794 A1**
(43) Date of publication of application: **19.07.2017**
(21) Application number: 15839901.4
(22) Date of filing: 10.09.2015
(51) Int. Cl.: C07D 409/12, A61K 31/381, A61K 31/397, A61K 31/40, A61K 31/416, A61K 31/427, A61K 31/428, A61K 31/4436, A61K 31/4525, A61P 31/18, A61P 43/00, C07D 493/04, C12N 9/99

(54) **SUSTAINED HIV PROTEASE INHIBITOR**

(30) Priority: 11.09.2014 JP 2014185150
(71) Applicant: Shionogi & Co., Ltd., Osaka-shi, Osaka 541-0045 (JP)
(72) Inventor: KAWASUJI, Takashi, Toyonaka-shi Osaka 561-0825 (JP); MIKAMIYAMA, Hidenori, Toyonaka-shi Osaka 561-0825 (JP); SUZUKI, Naoyuki, Toyonaka-shi Osaka 561-0825 (JP); MASUDA, Koji, Toyonaka-shi Osaka 561-0825 (JP); SUGIMOTO, Hideki, Toyonaka-shi Osaka 561-0825 (JP); OKANO, Azusa, Toyonaka-shi Osaka 561-0825 (JP); YOSHIDA, Miho, Toyonaka-shi Osaka 561-0825 (JP); SUGIYAMA, Shuichi, Toyonaka-shi Osaka 561-0825 (JP); ASAHI, Kentarou, Toyonaka-shi Osaka 561-0825 (JP); KOZONO, Iori, Toyonaka-shi Osaka 561-0825 (JP); MIYAZAKI, Keisuke, Toyonaka-shi Osaka 561-0825 (JP); OZASA, Hiroki, Toyonaka-shi Osaka 561-0825 (JP); MIYAGAWA, Masayoshi, Toyonaka-shi Osaka 561-0825 (JP)
(74) Representative: Hoffmann Eitle
(86) International application number: PCT/JP2015/075669
(87) International publication number: WO 2016/039403

(57) **Abstract**

The present invention provides useful compounds for HIV protease inhibitor. A compound represented by formula or its pharmaceutically acceptable salt Formula: 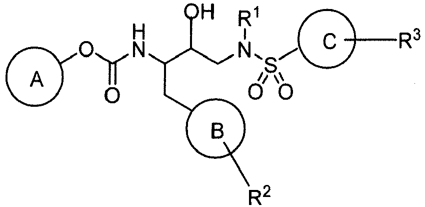 wherein ring A is 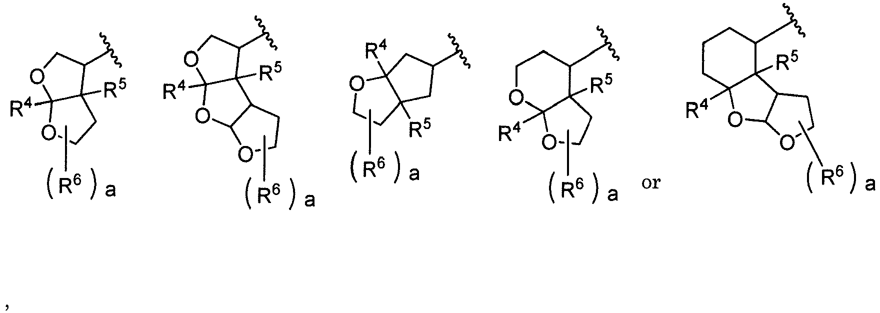 R⁴ is -Y-Z, hydrogen atom, halogen, hydroxy and the like,
R⁵ is hydrogen atom, halogen, hydroxy and the like,
R⁶ is each independently halogen, hydroxy, carboxy and the like,
ring A may be substituted with said R⁶ at any substitutable position(s),
a is an integer of 0 to 7,
ring B is substituted or unsubstituted aromatic carbocyclyl, or substituted or unsubstituted aromatic heterocyclyl,
ring C is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R¹ is -Y-Z, substituted or unsubstituted alkyl, substituted or unsubstituted alkenyl, substituted or unsubstituted alkynyl and the like,
R² and R³ are each independently -Y-Z or hydrogen atom,
provided that at least one of R¹, R², R³ and R⁴ is a group represented by formula: -Y-Z,
Y is a bond, or a spacer of any combination selected from the group consisting of -O-, -S-, -NR⁷-, -C(=O)-, -SO-, -SO₂-, -NR⁷-C(=O)-, -C(=O)-NR⁷-, -NR⁷-C(=O)-NR⁷-, -NR⁷-C(=O)-O-, -SO₂-NR⁷-, -NR⁷-SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl and substituted or unsubstituted non-aromatic heterocyclediyl,
R⁷ are each independently hydrogen atom, hydroxy, carboxy and the like, and
Z is substituted aromatic carbocyclyl, substituted non-aromatic carbocyclyl, substituted aromatic heterocyclyl or substituted non-aromatic heterocyclyl.

SEQUENCE LISTING

<110>  SHIONOGI & CO.,LTD.

<120>  HIV protease inhibitor

<130>  15P00061WO

<160>  1

<170>  PatentIn version 3.5

<210>  1
<211>  1224
<212>  DNA
<213>  Artificial sequence

<220>
<223>  Actinomycete DNA mixture

<400>  1
atgaccgata cgcacaccgg cccgacaccg gccgacgcgg tacccgccta cccgttcagc      60

ctgccgcacg ccctggacct cgacccgcac tacgccgaac tccgccgcga cgaacccgtc     120

tccagggtgc gcctgcccta cggcgagggc acggcctggc tggtcacccg catgtccgac     180

gcccgtatcg tcctgggcga ctcccgcttc agcaccgcgg ccgccaccga tcccgccact     240

ccccggatgt tccccacccc gcccgagccg gacggcgtcc tcgcccagga cccgccggac     300

cacacccggc tgcggcggct cgtgggcaag gccttcacgg cacgccgggt ggaggagatg     360

cggcctcgtg tccgcgccct cgtcgactcc ctgctcgacg acatggtggc gcacggttca     420

cccgccgacc tcgtcgagtt cctcgcggtt cccttccccg tcgcggtcat ctgcgaactg     480

ctcggcgtgc cccttgagga ccgcgacctg ttccggacct tctccgacgc catgctctcc     540

tcgacccggc tcaccgccgc ggagattcag caggtccagc aggacttcat ggtctacatg     600

gacggcctgg tcgcccagcg ccgcgacgcc cccaccgagg atctgctcgg cgccctcgcc     660

ctcgccaccg acaacgacga ccacctgacc aagggcgaga tcgtcaacat gggggtgagc     720

ctgctcatcg cgggccacga gacgtcggtc aaccagatca ccaacctcgt ccacctcctg     780

ctgaccgaac gcaagcgcta cgagtcgctg gtcgccgacc cggccctcgt gcccgcggcg     840

gtggaggaga tgctgcggta cacaccgctc gtgtccgccg gcagcttcgt ccgcgtggcg     900

accgaggacg tggagctgag caccgtgacc gtgcgggccg gggagccctg cgtcgtccac     960

ttcgcgtcgg ccaaccggga cgaggaggtc ttcgaccacg ccgacgagct ggacttccac    1020

cgtgagcgca acccgcacat agcgttcggg cacggagcgc accactgcat cggcgcccaa    1080

ctgggccgac tggaactcca ggaggccctg tccgccctcg tccggcgctt ccccaccctc    1140

gacctggccg agccggtcgc gggactgaag tggaagcagg gcatgctgat ccgcggactg    1200

gaacgccaga tcgtctcctg gtga                                           1224

## Description

### TECHNICAL FIELD

The present invention relates to long-acting HIV protease inhibitor. Especially, the present invention provides the compound having partial structure such as a protein affinity group, and its medicaments which are useful for HIV protease inhibitor.

### BACKGROUND ART

Human Immunodeficiency Virus (HIV) which is a kind of retrovirus is known to cause Acquired immunodeficiency syndrome (AIDS).

Now, it is reported that multidrug therapy is effective because resistant virus occur in treatments of AIDS. Four type anti-HIV agents such as reverse transcriptase inhibitor, protease inhibitor, integrase inhibitor and CCR5 inhibitor are used in clinical.

Above all, HIV protease inhibitor is very strong agent to extend lifetime of infected individual by inhibiting replication of HIV.

Now, Saquinavir, Ritonavir, Indinavir, Nelfinavir, Amprenavir, Lopinavir, Fosamprenavir, Atazanavir, Darunavir and Tipranavir are known as HIV protease inhibitor (Non-Patent Document 1).

Darunavir is designed for a target to 29th and 30th aspartic acids which are active center of protease. Darunavir was approved by FDA in 2006. chemical name of Darunavir is (3R,3aS,6aR)-hexahydrofuro[2,3-b]furan-3-yl[(1S,2R)-3-{[(4-aminophenyl)sulfonyl](2-methylpropyl)amino}-1-benzyl-2-hydroxypropyl]carbamate, and chemical structure of Darunavir is following:

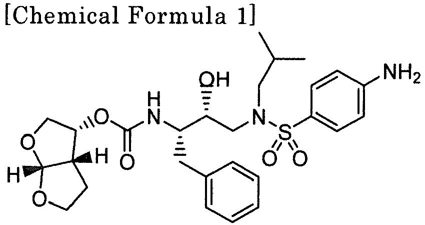

Darunavir and some compounds having protease inhibitory activities are disclosed in Patent Document 1. However compound having a protein affinity group such as the present compound is not disclosed.

### PRIOR ART REFERENCES

### [Patent Document]

[Patent Document 1] International Publication No. 1995/06030

### [Non-Patent Document]

[Non-Patent Document 1] International Journal of Antimicrobial Agents 33 (2009)

### SUMMARY OF THE INVENTION

### PROBLEMS TO BE SOLVED BY THE INVENTION

There are problems such as bad metabolic stability and high clearance in HIV protease inhibitor.

Therefore, the present invention provides the long-acting HIV protease inhibitor.

### MEANS TO SOLVE THE PROBLEMS

The inventors invented the long-acting HIV protease inhibitor which is improved clearance without decrease in drug efficacy by introducing spacer (Y) and a protein affinity group (Z) to a compound having HIV protease inhibitory activity.

For example, inventors found that long acting of the compound is improved by introducing a group represented by formula: -Y-Z to at least one of R¹, R², R³ or R⁴ of a compound represented by formula (I): 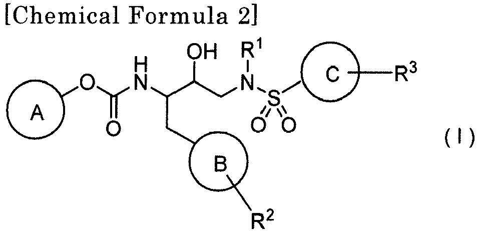 wherein ring A is

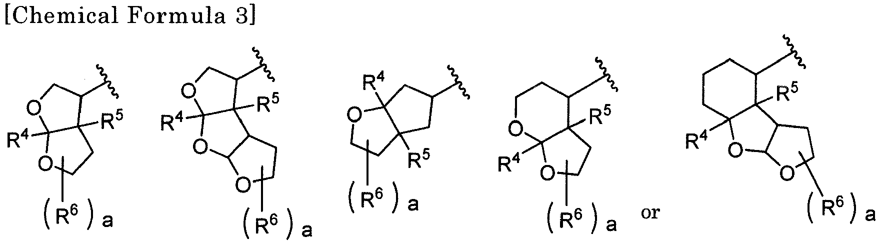

Moreover, metabolic stability is improved by introducing a group except hydrogen to R⁴, and the following invention has been accomplished.
(1) A compound represented by formula (I): 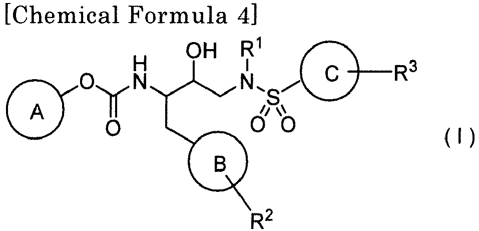 wherein ring A is a group represented by formula: 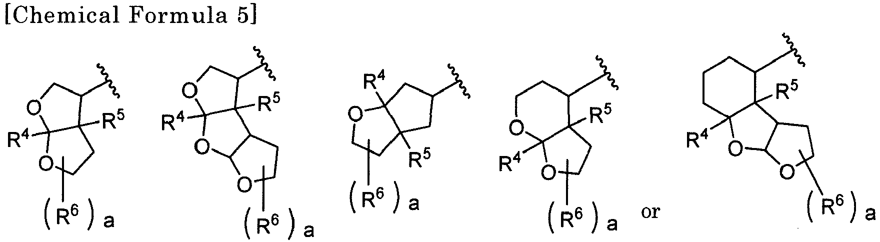
   R⁴ is a group represented by formula: -Y-Z, hydrogen atom, halogen, hydroxy, carboxy, cyano, nitro, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted amino, substituted or unsubstituted carbamoyl, substituted or unsubstituted sulfamoyl, substituted or unsubstituted aminocarbonyloxyalkyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R⁵ is hydrogen atom, halogen, hydroxy, carboxy, cyano, nitro, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted carbamoyl, or substituted or unsubstituted sulfamoyl,
   R⁶ are each independently halogen, hydroxy, carboxy, formyl, formyloxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   ring A may be substituted with said R⁶ at any substitutable position(s),
   a is an integer of 0 to 7,
   ring B is substituted or unsubstituted aromatic carbocyclyl, or substituted or unsubstituted aromatic heterocyclyl,
   ring C is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹ is a group represented by formula: -Y-Z, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylalkyl, substituted or unsubstituted non-aromatic carbocyclylalkyl, substituted or unsubstituted aromatic heterocyclylalkyl, or substituted or unsubstituted non-aromatic heterocyclylalkyl,
   R² and R³ are each independently a group represented by formula: -Y-Z, or hydrogen atom,
   provided that at least one of R¹, R², R³ and R⁴ is a group represented by formula: -Y-Z,
   Y is each independently a bond, or a spacer of any combination selected from the group consisting of -O-, -S-, -NR⁷-, -C(=O)-, -SO-, -SO₂-, -NR⁷-C(=O)-, -C(=O)-NR⁷-, -NR⁷ -C(=O)-NR7 -, -O-C(=O)-NR⁷ -, -NR⁷-C(=O)-O-, -SO₂-NR⁷-, -NR⁷-SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   provided that the groups selected from the group consisting of -O-, -S- and-NR⁷- are not connected adjacently in Y, and
   provided that the groups selected from the group consisting of -C(=O)-, -SO-,-SO₂-, -NR⁷ -C(=O)-, -C(=O)-NR⁷-, -NR⁷-C(=O)-NR⁷-, -O-C(=O)-NR⁷-, -NR⁷-C(=O)-O-,-SO₂-NR⁷- and -NR⁷ -SO₂ - are not connected adjacently in Y,
   R⁷ are each independently hydrogen atom, hydroxy, carboxy, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, substituted or unsubstituted non-aromatic heterocyclylsulfonyl,
   Z is each independently substituted aromatic carbocyclyl, substituted non-aromatic carbocyclyl, substituted aromatic heterocyclyl, or substituted non-aromatic heterocyclyl,
   provided that when R⁴ is hydrogen atom, at least one of substituents on Z is - COOH,
   provided that the following compounds are excluded: 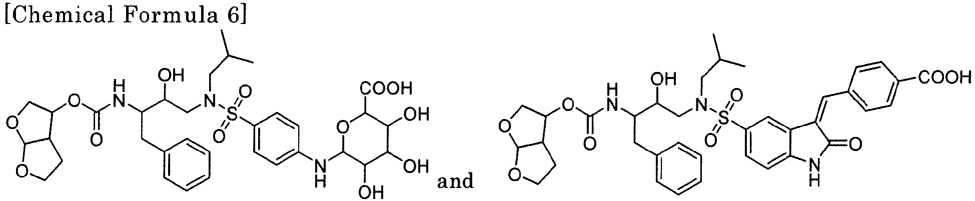 or its pharmaceutically acceptable salt.
(1') A compound represented by formula (I): 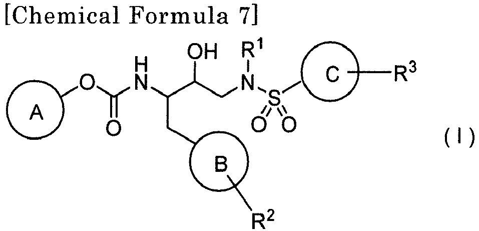 wherein ring A is a group represented by formula: 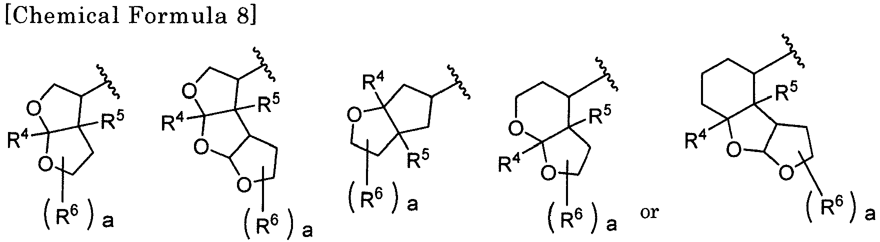
   R⁴ is a group represented by formula: a group represented by formula: -Y-Z, halogen, hydroxy, carboxy, amino, carbamoyl, sulfamoyl, cyano, nitro, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted monoalkylcarbonylamino, substituted or unsubstituted dialkylcarbonylamino, substituted or unsubstituted monoalkylsulfonylamino, substituted or unsubstituted dialkylsulfonylamino, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R⁵ is hydrogen atom, halogen, hydroxy, carboxy, amino, carbamoyl, sulfamoyl, cyano, nitro, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl,
   R⁶ are each independently halogen, hydroxy, carboxy, amino, imino, hydroxyamino, hydroxyimino, formyl, formyloxy, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   ring A may be substituted with said R⁶ at any substitutable position(s),
   a is an integer of 0 to 7,
   ring B is substituted or unsubstituted aromatic carbocyclyl, or substituted or unsubstituted aromatic heterocyclyl,
   ring C is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹ is a group represented by formula: -Y-Z, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   R² and R³ are each independently a group represented by formula: -Y-Z, or hydrogen atom,
   provided that at least one of R¹, R², R³ and R⁴ is a group represented by formula: -Y-Z,
   Y is each independently a bond, or a spacer of any combination selected from the group consisting of -O-, -S-, -NR⁷ -, -C(=O)-, -SO-, -SO₂ -, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   R⁷ are each independently hydrogen atom, hydroxy, carboxy, amino, carbamoyl, sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   Z is each independently aromatic carbocyclyl having acid group, non-aromatic carbocyclyl having acid group, aromatic heterocyclyl having acid group, or non-aromatic heterocyclyl having acid group
   or its pharmaceutically acceptable salt.
(2) The compound or its pharmaceutically acceptable salt according to above item (1) or (1'), wherein R¹ is a group represented by formula: -Y-Z.
(3) The compound or its pharmaceutically acceptable salt according to above item (1) or (1'), wherein R² is a group represented by formula: -Y-Z.
(4) The compound or its pharmaceutically acceptable salt according to above item (1) or (1'), wherein R³ is a group represented by formula: -Y-Z.
(5) The compound or its pharmaceutically acceptable salt according to above item (1) or (1'), wherein R⁴ is a group represented by formula: -Y-Z.
(6) The compound or its pharmaceutically acceptable salt according to any one of above items (2) to (4), wherein R⁴ is substituted or unsubstituted alkyl.
(7) The compound or its pharmaceutically acceptable salt according to any one of above items (1) to (6) and (1'), wherein ring B is substituted or unsubstituted aromatic carbocyclyl.
(8) The compound or its pharmaceutically acceptable salt according to above item (7), wherein ring B is substituted or unsubstituted phenyl.
(9) The compound or its pharmaceutically acceptable salt according to any one of above items (1) to (8) and (1'), wherein ring C is substituted or unsubstituted aromatic carbocyclyl or substituted or unsubstituted bicyclic aromatic heterocyclyl.
(9') The compound or its pharmaceutically acceptable salt according to any one of above items (1) to (8) and (1'), wherein ring C is substituted or unsubstituted aromatic carbocyclyl.
(10) The compound or its pharmaceutically acceptable salt according to any one of above items (1) to (8) and (1'), wherein ring C is substituted or unsubstituted bicyclic aromatic heterocyclyl.
(11) The compound or its pharmaceutically acceptable salt according to above item (2), wherein Y is a group represented by formula: 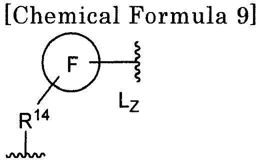
   wherein a bond Lz is connecting to Z,
   ring F is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹⁴ is substituted or unsubstituted alkylene which may be intervened with one or more groups selected from the group consisting of -O-, -NR⁷ -, -C(=O)-NR⁷- and-NR⁷-C(=O)-,
   substituted or unsubstituted alkenylene which may be intervened with one or more groups selected from the group consisting of -O-, -NR⁷ -, -C(=O)-NR⁷- and -NR⁷-C(=O)-, or
   substituted or unsubstituted alkynylene which may be intervened with one or more groups selected from the group consisting of -O-, -NR⁷ -, -C(=O)-NR⁷ - and -NR⁷-C(=O)-,
   provided that the groups selected from the group consisting of -O-, -NR⁷-,-C(=O)-NR⁷ - and -NR⁷-C(=O)- are not connected adjacently in R¹⁴,
   R⁷ is defined as the same in above item (1) or (1').
(11') The compound or its pharmaceutically acceptable salt according to above item (2), wherein Y is a group represented by formula: 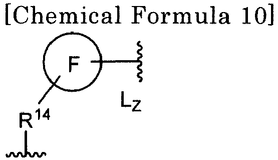 wherein a bond Lz is connecting to Z,
   ring F is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹⁴ is substituted or unsubstituted alkylene which may be intervened with one or more groups selected from the group consisting of -O-, -NR⁷ -, -C(=O)-NR⁷- and-NR⁷-C(=O)-,
   substituted or unsubstituted alkenylene which may be intervened with one or more groups selected from the group consisting of -O-, -NR⁷ -, -C(=O)-NR⁷ - and -NR⁷-C(=O)-, or
   substituted or unsubstituted alkynylene which may be intervened with one or more groups selected from the group consisting of -O-, -NR⁷ -, -C(=O)-NR⁷ - and -NR⁷-C(=O)-,
   R⁷ is defined as the same in above item (1').
(12) The compound or its pharmaceutically acceptable salt according to above item (11), wherein Y is a group represented by formula: 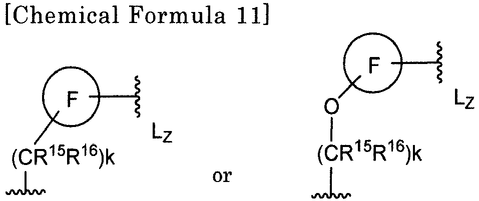 wherein a bond Lz is connecting to Z,
   ring F is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹⁵ and R¹⁶ are each independently hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   k is an integer of 0 to 4.
(12') The compound or its pharmaceutically acceptable salt according to above item (11'), wherein Y is a group represented by formula: 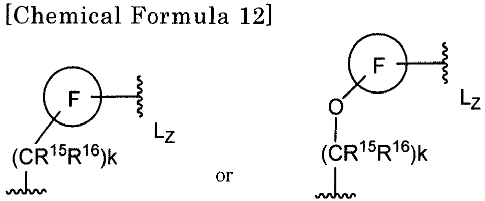 wherein a bond Lz is connecting to Z,
   ring F is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹⁵ and R¹⁶ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   k is an integer of 0 to 4.
(13) The compound or its pharmaceutically acceptable salt according to above item (12), wherein Y is a group represented by formula: 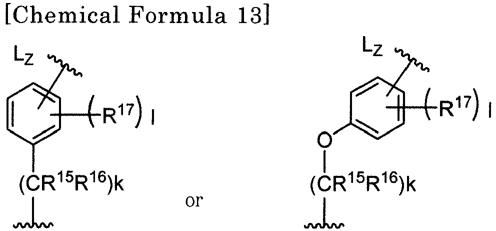 wherein a bond Lz is connecting to Z,
   R¹⁵ and R¹⁶ are defined as the same in above item (12),
   R¹⁷ are each independently halogen, hydroxy, carboxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   k is defined as the same in above item (12),
   1 is an integer of 0 to 4.
(13') The compound or its pharmaceutically acceptable salt according to above item (12'), wherein Y is a group represented by formula: 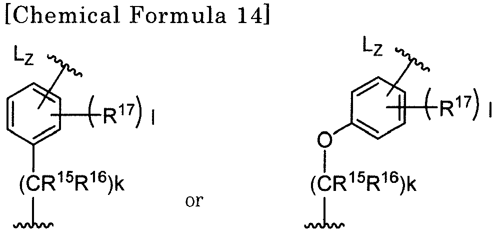
   wherein a bond Lz is connecting to Z,
   R¹⁵ and R¹⁶ are defined as the same in (12'),
   R¹⁷ are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   k is defined as the same in above item (12'),
   1 is an integer of 0 to 4.
(14) The compound or its pharmaceutically acceptable salt according to above item (3), wherein Y is a bond or a group represented by formula: 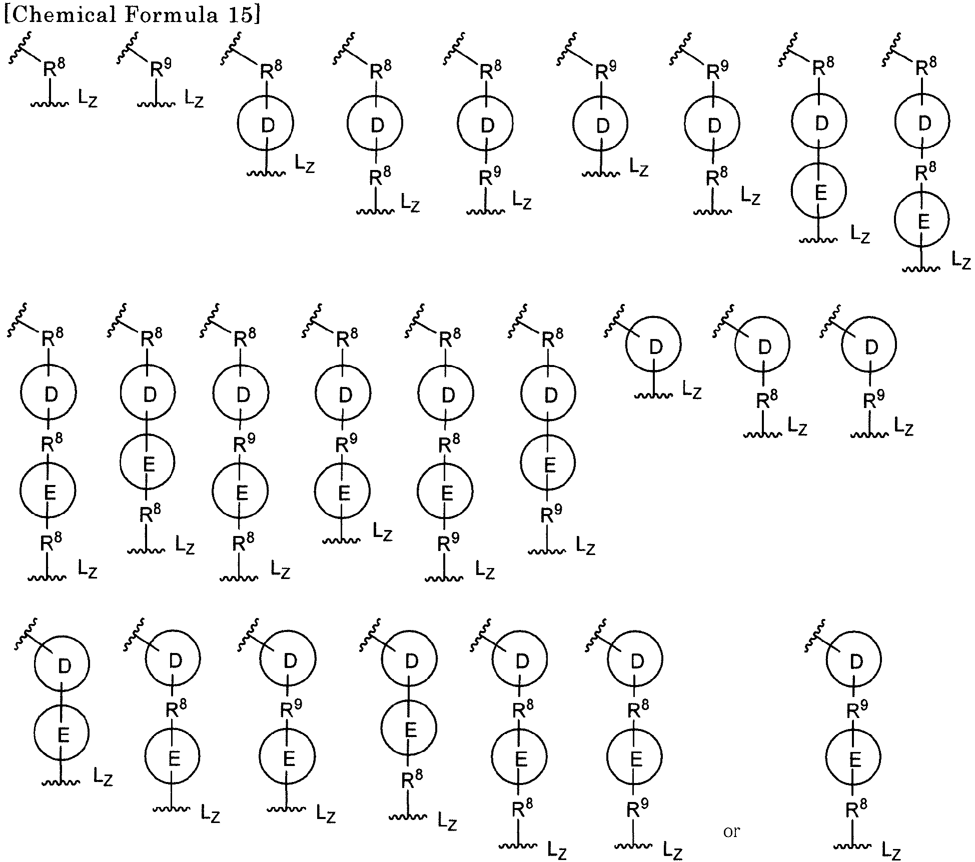 wherein a bond Lz is connecting to Z,
   R⁸ are each independently -O-, -S-, -NR⁷-, substituted or unsubstituted alkylene which may be intervened with one or more groups selected from the group consisting of -O-, -NR⁷ -, -C(=O)-NR⁷- and -NR⁷-C(=O)-,
   substituted or unsubstituted alkenylene which may be intervened with one or more groups selected from the group consisting of -O-, -NR⁷-, -C(=O)-NR⁷- and -NR⁷-C(=O)-, or
   substituted or unsubstituted alkynylene which may be intervened with one or more groups selected from the group consisting of -O-, -NR⁷-, -C(=O)-NR⁷- and -NR⁷-C(=O)-,
   provided that the groups selected from the group consisting of -O-, -NR⁷-,-C(=O)-NR⁷- and -NR⁷-C(=O)- are not connected adjacently in R⁸,
   ring D and ring E are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R⁹ is -C(=O)-, -C(=O)-NR⁷-, -NR⁷-C(=O)-, -NR⁷-C(=O)-NR⁷-, -NR⁷SO₂- or-SO₂NR⁷-,
   R⁷ is defined as the same in above item (1).
(14') The compound or its pharmaceutically acceptable salt according to above item (3), wherein Y is a bond or a group represented by formula: 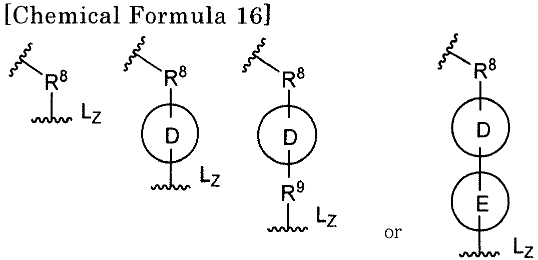
   wherein a bond Lz is connecting to Z,
   R⁸ are each independently -O-, -NR⁷-, substituted or unsubstituted alkylene which may be intervened with one or more groups selected from the group consisting of -O-, -NR⁷-, -C(=O)-NR⁷- and -NR⁷-C(=O)-,
   substituted or unsubstituted alkenylene which may be intervened with one or more groups selected from the group consisting of -O-, -NR⁷-, -C(=O)-NR⁷- and -NR⁷-C(=O)-, or
   substituted or unsubstituted alkynylene which may be intervened with one or more groups selected from the group consisting of -O-, -NR⁷-, -C(=O)-NR⁷- and -NR⁷-C(=O)-,
   ring D and ring E are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R⁹ is -C(=O)-NR⁷- or -NR⁷-C(=O)-,
   R⁷ is defined as the same in above item (1').
(15) The compound or its pharmaceutically acceptable salt according to above item (14), wherein Y is a group represented by formula: 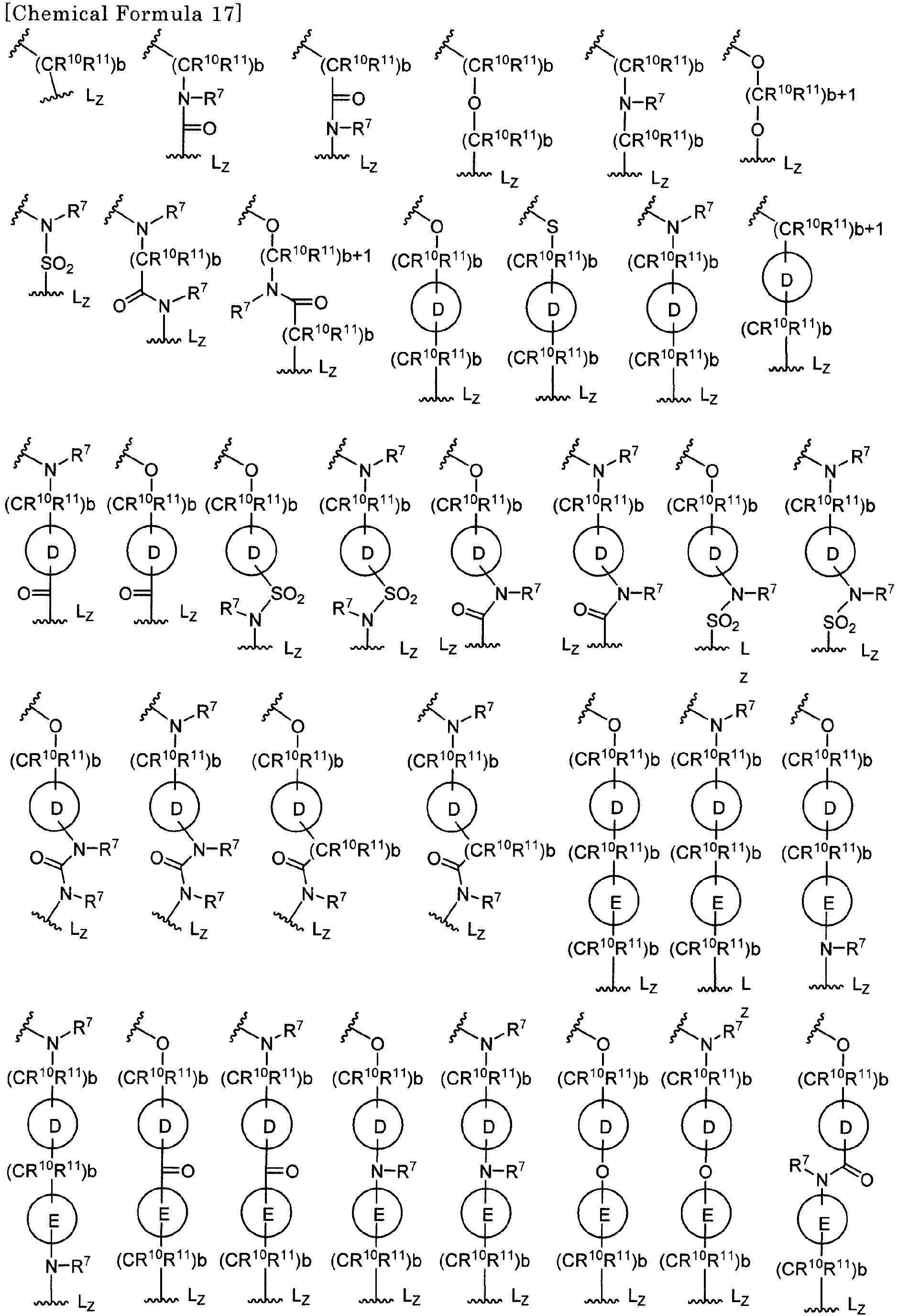 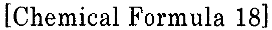 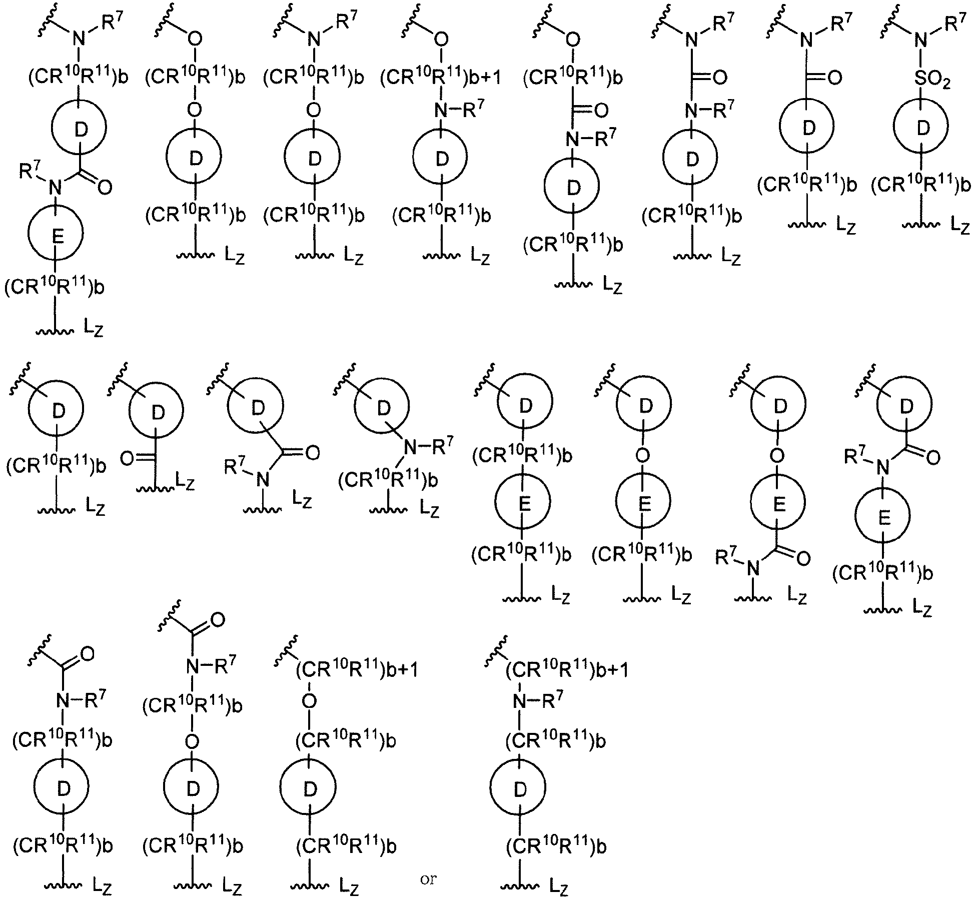
   wherein a bond Lz is connecting to Z,
   ring D and ring E are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹⁰ and R¹¹ are each independently hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   R¹⁰ and R¹¹ connected to the same carbon atom may be taken together with the said carbon atom to form substituted or unsubstituted imino, substituted or unsubstituted non-aromatic carbocycle, or non-aromatic heterocycle,
   the two R¹⁰ and/or R¹¹ connected to the adjacent carbon atoms may be taken together to form a bond,
   R⁷ is defined as the same in above item (1) or (1'),
   b are each independently an integer of 0 to 4.
(15') The compound or its pharmaceutically acceptable salt according to above item (14'), wherein Y is a bond or a group represented by formula: 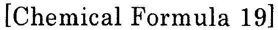 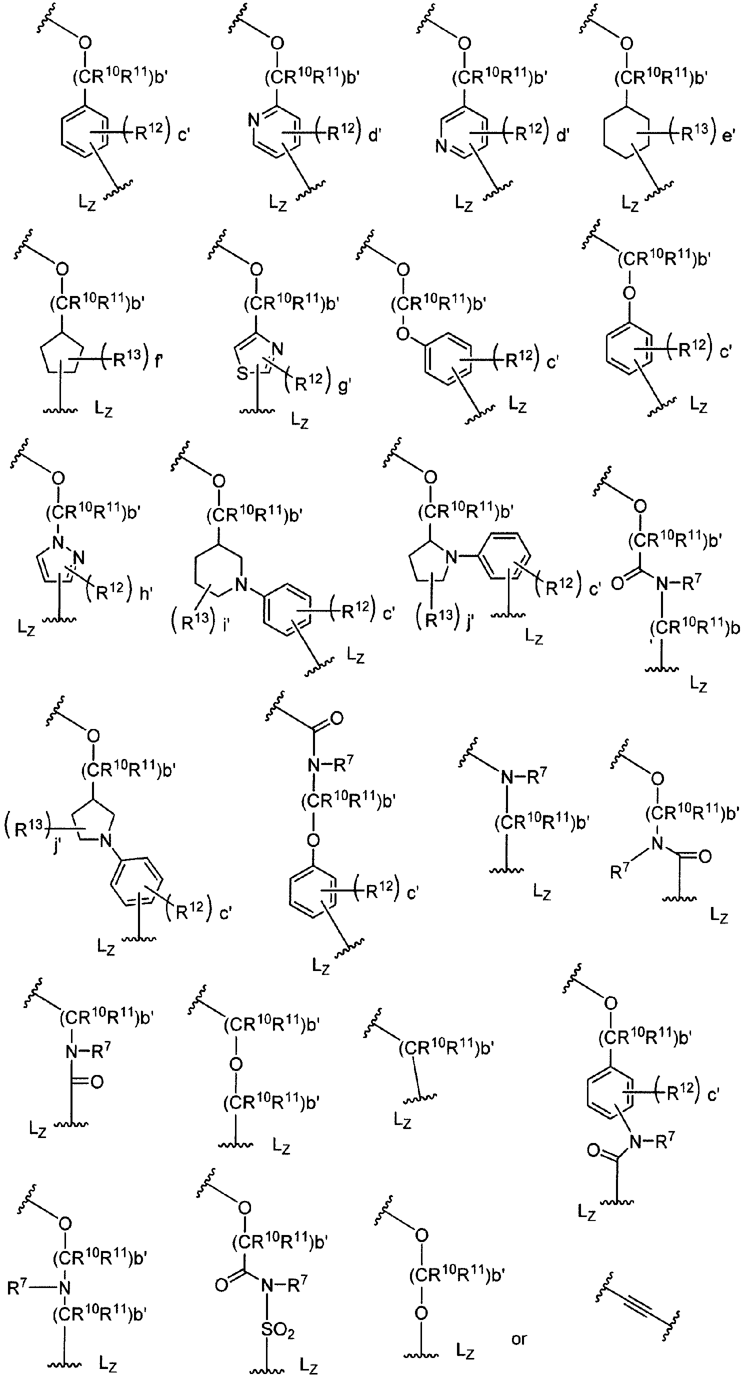
   wherein a bond Lz is connecting to Z,
   R¹⁰ and R¹¹ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   R¹⁰ and R¹¹ connected to the same carbon atom may be taken together with the said carbon atom to form substituted or unsubstituted imino,
   R¹² are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   the two R¹² connected to the adjacent carbon atoms constituting the ring may be taken together to form substituted or unsubstituted aromatic carbocycle, substituted or unsubstituted non-aromatic carbocycle, substituted or unsubstituted aromatic heterocycle, or substituted or unsubstituted non-aromatic heterocycle,
   R¹³ are each independently halogen, hydroxy, carboxy, amino, imino, hydroxyamino, hydroxyimino, carbamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted monoalkylcarbamoyl, substituted or unsubstituted dialkylcarbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
   R⁷ is defined as the same in above item (1'),
   b' are each independently an integer of 0 to 4,
   c' is an integer of 0 to 4,
   d' is an integer of 0 to 3,
   e' is an integer of 0 to 10,
   f' is an integer of 0 to 8,
   g' is an integer of 0 or 1,
   h' is an integer of 0 to 2,
   i' is an integer of 0 to 9,
   j' is an integer of 0 to 7.
(16) The compound or its pharmaceutically acceptable salt according to above item (15), wherein Y is a group represented by formula: 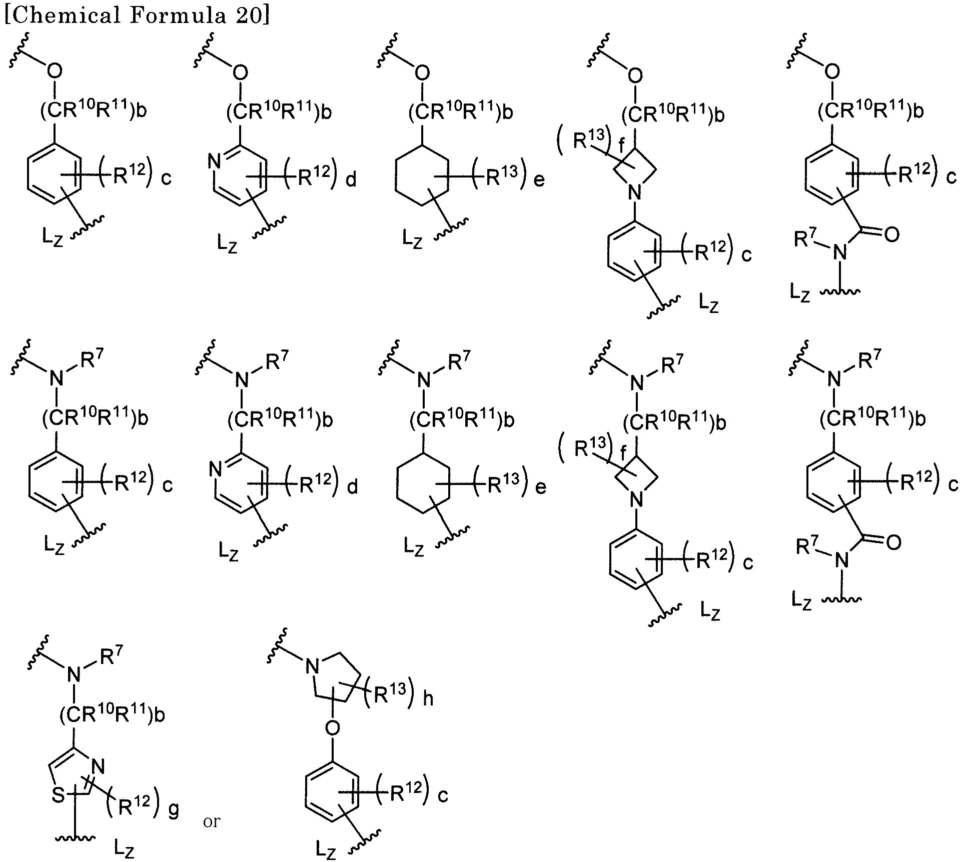
   wherein a bond Lz is connecting to Z,
   R¹² are each independently halogen, hydroxy, carboxy, sulfo, cyano, nitro, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   the two R¹² connected to the adjacent carbon atoms constituting the ring may be taken together to form substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹³ are each independently halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
   R¹³ connected to the non-adjacent and different carbon atoms may be taken together to form alkylene,
   R⁷ is defined as the same in above item (1),
   R¹⁰, R¹¹ and b are defined as the same in above item (14),
   c is an integer of 0 to 4,
   d is an integer of 0 to 3,
   e is an integer of 0 to 10,
   f is an integer of 0 to 5,
   g is an integer of 0 or 1,
   h is an integer of 0 to 7.
(17) The compound or its pharmaceutically acceptable salt according to above item (4), wherein Y is a bond or a group represented by formula: 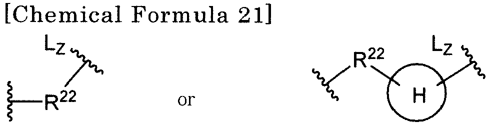 wherein a bond Lz is connecting to Z,
   R²² are each independently substituted or unsubstituted alkylene which may be intervened with one or more groups selected from the group consisting of -O-, - NR⁷ -, -C(=O)-NR⁷ - and -NR⁷ -C(=O)-,
   substituted or unsubstituted alkenylene which may be intervened with one or more groups selected from the group consisting of -O-, -NR⁷-, -C(=O)-NR⁷- and -NR⁷-C(=O)-, or
   substituted or unsubstituted alkynylene which may be intervened with one or more groups selected from the group consisting of -O-, -NR⁷-, -C(=O)-NR⁷- and -NR⁷-C(=O)-,
   provided that the groups selected from the group consisting of -O-, -NR⁷-,-C(=O)-NR⁷- and -NR⁷-C(=O)- are not connected adjacently in R²²,
   R⁷ is defined as the same in above item (1) or (1'),
   ring H is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.
(17') The compound or its pharmaceutically acceptable salt according to above item (4), wherein Y is a bond or a group represented by formula: 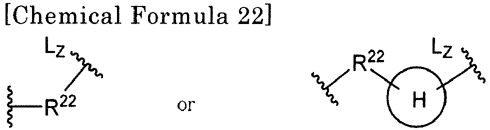 wherein a bond Lz is connecting to Z,
   R²² are each independently substituted or unsubstituted alkylene which may be intervened with one or more groups selected from the group consisting of -O-,-NR⁷-, -C(=O)-NR⁷- and -NR⁷-C(=O)-,
   substituted or unsubstituted alkenylene which may be intervened with one or more groups selected from the group consisting of -O-, -NR⁷-, -C(=O)-NR⁷- and -NR⁷-C(=O)-, or
   substituted or unsubstituted alkynylene which may be intervened with one or more groups selected from the group consisting of -O-, -NR⁷-, -C(=O)-NR⁷- and -NR⁷-C(=O)-,
   R⁷ is defined as the same in above item (1'),
   ring H is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.
(18) The compound or its pharmaceutically acceptable salt according to above item (17), wherein Y is a bond or a group represented by formula: 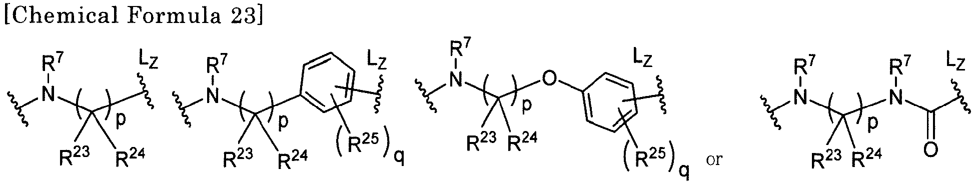
   wherein a bond Lz is connecting to Z,
   R²³ and R²⁴ are each independently hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   R⁷ is defined as the same in above item (1) or (1'),
   R²⁵ are each independently halogen, hydroxy, carboxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   p is an integer of 0 to 4,
   q is an integer of 0 to 4.
(18') The compound or its pharmaceutically acceptable salt according to above item (17'), wherein Y is a bond or a group represented by formula: 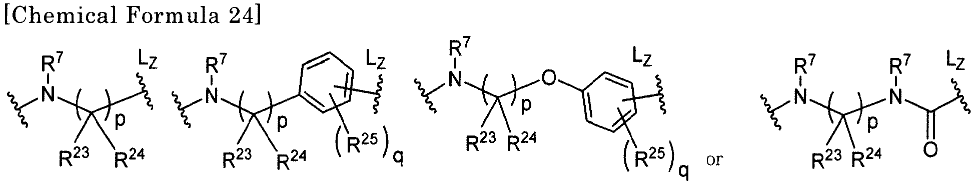
   wherein a bond Lz is connecting to Z,
   R²³ and R²⁴ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   R⁷ is defined as the same in above item (1'),
   R²⁵ are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   p is an integer of 0 to 4,
   q is an integer of 0 to 4.
(19) The compound or its pharmaceutically acceptable salt according to above item (5), wherein Y is a group represented by formula: 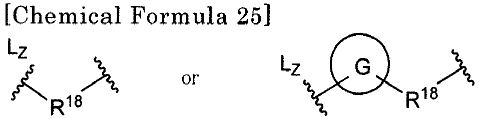
   wherein a bond Lz is connecting to Z,
   R¹⁸ is substituted or unsubstituted alkylene which may be intervened with one or more groups selected from the group consisting of -O-, -NR⁷-, -C(=O)-NR⁷- and-NR⁷-C(=O)-,
   substituted or unsubstituted alkenylene which may be intervened with one or more groups selected from the group consisting of -O-, -NR⁷-, -C(=O)-NR⁷-, and -NR⁷-C(=O)-, or
   substituted or unsubstituted alkynylene which may be intervened with one or more groups selected from the group consisting of -O-, -NR⁷-, -C(=O)-NR⁷- and -NR⁷-C(=O)-,
   provided that the groups selected from the group consisting of -O-, -NR⁷-,-C(=O)-NR⁷- and -NR⁷-C(=O)- are not connected adjacently in R¹⁸,
   R⁷ is defined as the same in above item (1) or (1'),
   ring G is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.
(19') The compound or its pharmaceutically acceptable salt according to above item (5), wherein Y is a group represented by formula: 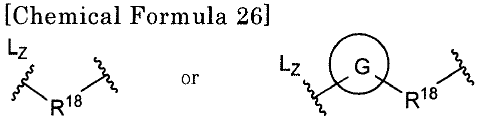
   wherein a bond Lz is connecting to Z,
   R¹⁸ are each independently substituted or unsubstituted alkylene which may be intervened with one or more groups selected from the group consisting of -O-, - NR⁷-, -C(=O)-NR⁷- and -NR⁷-C(=O)-,
   substituted or unsubstituted alkenylene which may be intervened with one or more groups selected from the group consisting of -O-, -NR⁷-, -C(=O)-NR⁷- and -NR⁷-C(=O)-, or
   substituted or unsubstituted alkynylene which may be intervened with one or more groups selected from the group consisting of -O-, -NR⁷-, -C(=O)-NR⁷- and -NR⁷-C(=O)-,
   R⁷ is defined as the same in above item (1'),
   ring G is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.
(20) The compound or its pharmaceutically acceptable salt according to above item (18) or (18'), wherein Y is a group represented by formula: 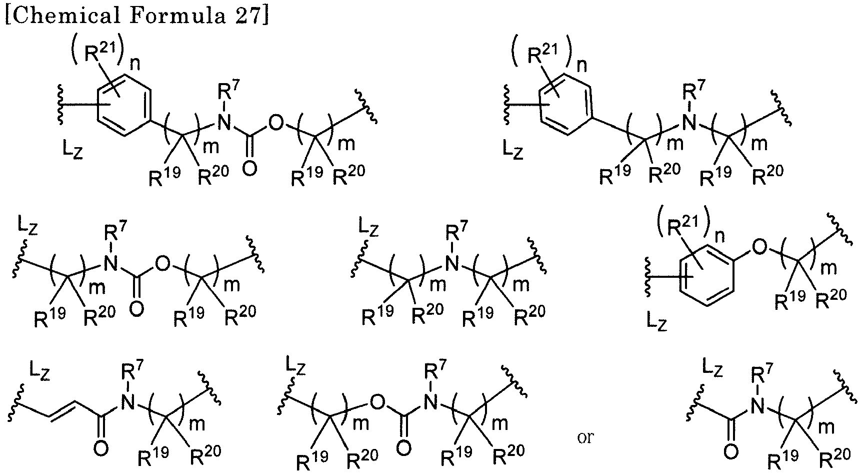
   wherein a bond Lz is connecting to Z,
   R¹⁹ and R²⁰ are each independently hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   R⁷ is defined as the same in above item (1) or (1'),
   R²¹ are each independently halogen, hydroxy, carboxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   m is an integer of 0 to 4,
   n is an integer of 0 to 4.
(20') The compound or its pharmaceutically acceptable salt according to above item (18'), wherein Y is a group represented by formula: 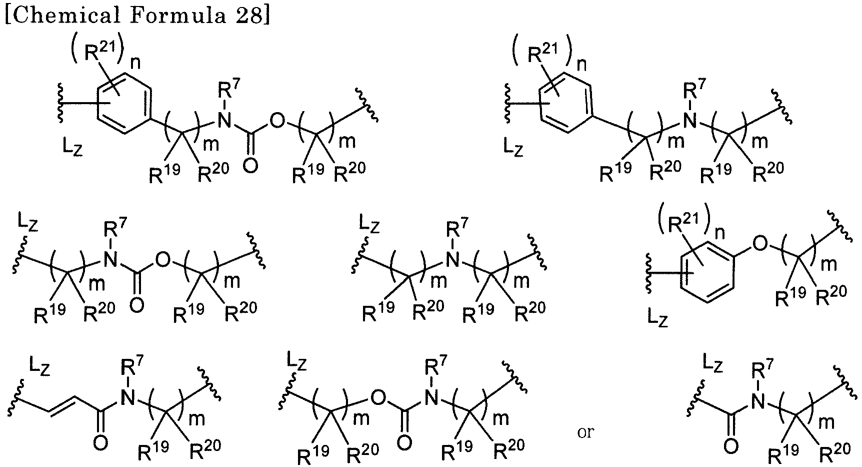
   wherein a bond Lz is connecting to Z,
   R¹⁹ and R²⁰ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   R⁷ is defined as the same in above item (1'),
   R²¹ are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   m are each independently an integer of 0 to 4,
   n is an integer of 0 to 4.
(21) The compound or its pharmaceutically acceptable salt according to any one of above items (1) to (20), (1'), (9'), (11') to (15') and (17') to (20'), wherein Z is bicyclic or tricyclic substituted non-aromatic carbocyclyl or bicyclic or tricyclic substituted non-aromatic heterocyclyl.
(21') The compound or its pharmaceutically acceptable salt according to any one of above items (2) to (8), (1'), (9'), (11') to (15') and (17') to (20'), wherein Z is bicyclic non-aromatic carbocyclyl or bicyclic non-aromatic heterocyclyl.
(22) The compound or its pharmaceutically acceptable salt according to above item (21), wherein one of the substituents of bicyclic or tricyclic substituted non-aromatic carbocyclyl or bicyclic or tricyclic substituted non-aromatic heterocyclyl is -COOH or its biologically equivalent group.
(22') The compound or its pharmaceutically acceptable salt according to above item (21'), wherein acid group is -COOH or its biologically equivalent group.
(23) The compound or its pharmaceutically acceptable salt according to above item (22), wherein Z is a group represented by formula: 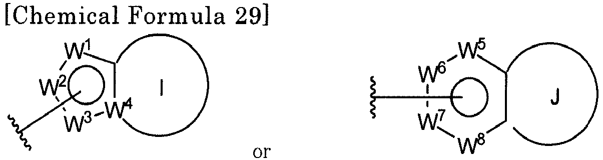
   wherein W¹, W², W³ , W⁵, W⁶, W⁷ and W⁸ are each independently C, CR²⁶, O, S, N or NR²⁷,
   W⁴ is C or N,
   R²⁶ are each independently -COOH or its biologically equivalent group, hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   provided that at least one of W¹, W² and W³ is CR²⁶, and at least one of said R²⁶ is -COOH or its biologically equivalent group,
   provided that at least one of W⁵, W⁶, W⁷ and W⁸ is CR²⁶, and at least one of said R²⁶ is -COOH or its biologically equivalent group,
   R²⁷ are each independently hydrogen atom, carboxy, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   ring I and ring J are each independently substituted or unsubstituted non-aromatic carbocycle, or substituted or unsubstituted non-aromatic heterocycle,
   the ring containing W¹, W², W³ and W⁴ as atoms constituting said ring is an aromatic ring,
   the ring containing W⁵, W⁶, W⁷ and W⁸ as atoms constituting said ring is an aromatic ring.
(23') The compound or its pharmaceutically acceptable salt according to above item (22), wherein Z is a group represented by formula: 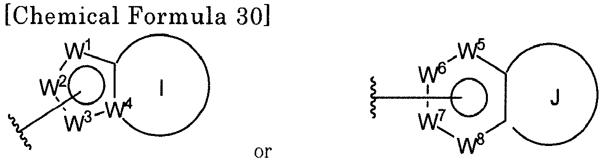
   wherein W¹, W², W³, W⁵, W⁶, W⁷ and W⁸ are each independently C, CR²⁶, O, S, N or NR²⁷,
   W⁴ and W⁹ are each independently C, CR²⁶ or N,
   R²⁶ are each independently -COOH or its biologically equivalent group, hydrogen atom, halogen, hydroxy, carboxy, amino, imino, hydroxyamino, hydroxyimino, carbamoyl, sulfamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   provided that at least one of W¹, W², W³ and W⁴ is CR²⁶, and at least one of said R²⁶ is -COOH or its biologically equivalent group,
   provided that at least one of W⁵, W⁶, W⁷, W⁸ and W⁹ is CR²⁶, and at least one of said R²⁶ is -COOH or its biologically equivalent group,
   R²⁷ are each independently hydrogen atom, carboxy, carbamoyl, sulfamoyl, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   ring I and ring J are each independently substituted or unsubstituted non-aromatic carbocycle, or substituted or unsubstituted non-aromatic heterocycle,
   the ring containing W¹, W², W³ and W⁴ as atoms constituting said ring is an aromatic ring,
   the ring containing W⁵, W⁶, W⁷ and W⁸ as atoms constituting said ring is an aromatic ring.
(24) The compound or its pharmaceutically acceptable salt according to any one of above items (1) to (23), (1'), (9'), (11') to (15') and (17') to (23'), wherein Z is a group represented by formula: 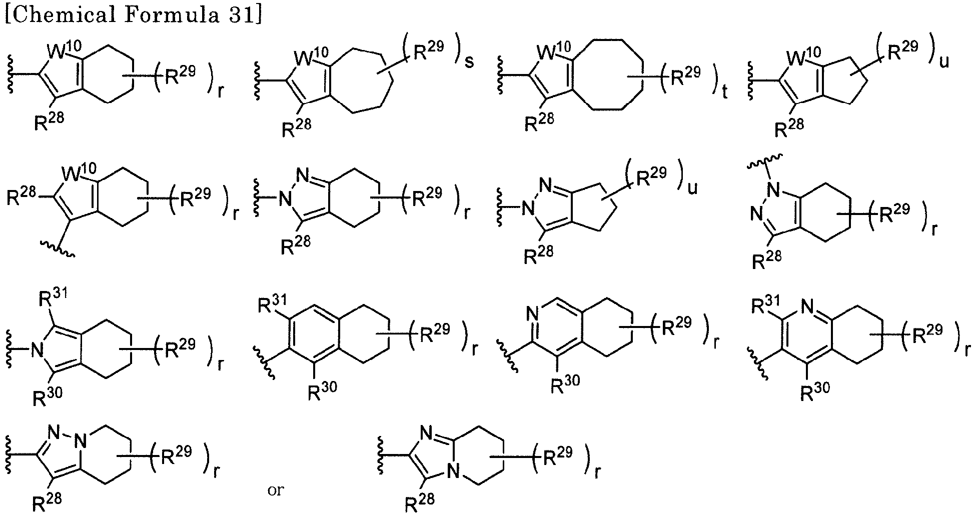
   wherein W¹⁰ is -S-, -O- or -NR²⁷-,
   R²⁷ is defined as the same in above item (23),
   R²⁸ is -COOH or its biologically equivalent group,
   R³⁰ and R³¹ are each independently -COOH or its biologically equivalent group, hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   provided that at least one of R³⁰ and R³¹ is -COOH or its biologically equivalent group,
   R²⁹ are each independently halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
   two R²⁹ connected to the adjacent carbon atoms may be taken together to form substituted or unsubstituted aromatic carbocycle, substituted or unsubstituted non-aromatic carbocycle, or substituted or unsubstituted non-aromatic heterocycle,
   two R²⁹ connected to the non-adjacent and different carbon atoms may be taken together to form substituted or unsubstituted alkylene,
   two R²⁹ connected to the same carbon atom may be taken together to form substituted or unsubstituted non-aromatic carbocycle or substituted or unsubstituted non-aromatic heterocycle,
   two R²⁹ connected to the same carbon atom may be taken together to form oxo,
   r is an integer of 0 to 8,
   s is an integer of 0 to 10,
   t is an integer of 0 to 12,
   u is an integer of 0 to 6.
(24') The compound or its pharmaceutically acceptable salt according to any one of above items (2) to (8), (1'), (9'), (11') to (15') and (17') to (23'), wherein Z is a group represented by formula: 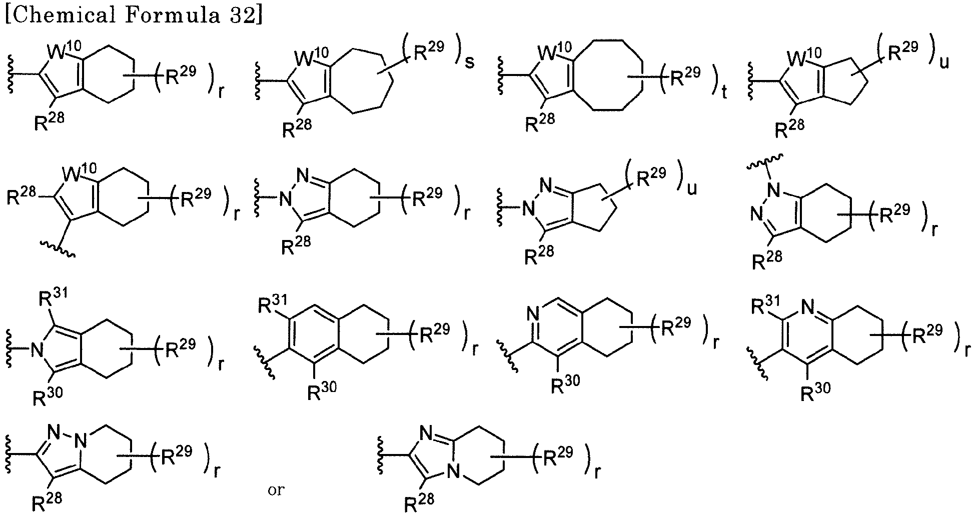
   wherein W¹⁰ are each independently S, O or NR²⁷,
   R²⁷ is defined as the same in above item (23'),
   R²⁸ are each independently -COOH or its biologically equivalent group,
   R³⁰ and R³¹ are each independently -COOH or its biologically equivalent group, hydrogen atom, halogen, hydroxy, carboxy, amino, imino, hydroxyamino, hydroxyimino, carbamoyl, sulfamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   provided that at least one of R³⁰ and R³¹ is -COOH or its biologically equivalent group,
   R²⁹ are each independently halogen, hydroxy, carboxy, amino, imino, hydroxyamino, hydroxyimino, carbamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted monoalkylcarbamoyl, substituted or unsubstituted dialkylcarbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
   two R²⁹ connected to the adjacent carbon atoms may be taken together to form substituted or unsubstituted ring,
   two R²⁹ connected to the non-adjacent and different carbon atoms may be taken together to form substituted or unsubstituted bridge,
   two R²⁹ connected to the same carbon atom may be taken together to form substituted or unsubstituted spiro ring,
   two R²⁹ connected to the same carbon atom may be taken together to form oxo,
   r is an integer of 0 to 8,
   s is an integer of 0 to 10,
   t is an integer of 0 to 12,
   u is an integer of 0 to 6.
(25) The compound or its pharmaceutically acceptable salt according to any one of above items (1) to (20), (1'), (9'), (11') to (15') and (17') to (20'), wherein Z is a group represented by formula: 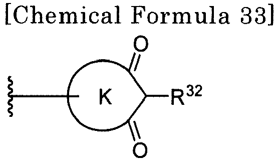
   wherein ring K is substituted or unsubstituted non-aromatic carbocyclyl or substituted or unsubstituted non-aromatic heterocyclyl,
   R³² is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.
(26) The compound or its pharmaceutically acceptable salt according to above items (25), wherein Z is a group represented by formula: 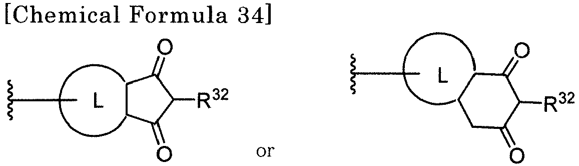
   wherein ring L is substituted or unsubstituted aromatic carbocyclyl, or substituted or unsubstituted aromatic heterocyclyl,
   R³² is defined as the same in above item (25).
(27) The compound or its pharmaceutically acceptable salt according to any one of above items (1) to (20), (1'), (9'), (11') to (15') and (17') to (20'), wherein Z is a group represented by formula: 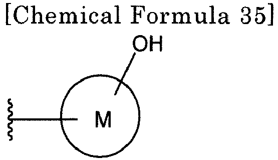 wherein ring M is substituted or unsubstituted aromatic carbocyclyl, or substituted or unsubstituted aromatic heterocyclyl.
(28) The compound or its pharmaceutically acceptable salt according to above item (27), wherein ring M is substituted or unsubstituted benzene ring.
(29) The compound or its pharmaceutically acceptable salt according to any one of above items (1) to (20), (1'), (9'), (11') to (15') and (17') to (20'), wherein Z is a group represented by formula: 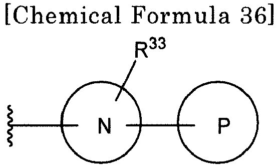
   wherein ring N and ring P are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R³³ is -OH, -COOH or its biologically equivalent group.
(30) A compound represented by formula (II): 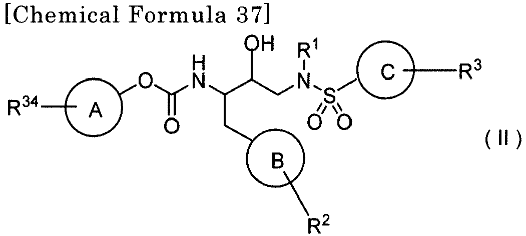
   wherein ring A is substituted or unsubstituted non-aromatic carbocyclyl, or substituted or unsubstituted non-aromatic heterocyclyl,
   ring B is substituted or unsubstituted aromatic carbocyclyl, or substituted or unsubstituted aromatic heterocyclyl,
   ring C is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹ is a group represented by formula: -Y-Z, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   R², R³ and R³⁴ are each independently a group represented by formula: -Y-Z, or hydrogen atom,
   provided that at least one of R¹, R², R³ and R⁴ is a group represented by formula: -Y-Z,
   Y is each independently a bond, or a spacer of any combination selected from the group consisting of -O-, -S-, -NR⁷-, -C(=O)-, -SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   R⁷ are each independently hydrogen atom, hydroxy, carboxy, amino, carbamoyl, sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   Z are each independently aromatic carbocyclyl having acid group, non-aromatic carbocyclyl having acid group, aromatic heterocyclyl having acid group or non-aromatic heterocyclyl having acid group,
   or its pharmaceutically acceptable salt.
(31) The compound or its pharmaceutically acceptable salt according to above item (30), wherein ring A is substituted or unsubstituted bicyclic non-aromatic carbocyclyl, or substituted or unsubstituted bicyclic non-aromatic heterocyclyl.
(32) The compound or its pharmaceutically acceptable salt according to above item (31), wherein ring A is substituted or unsubstituted bicyclic non-aromatic heterocyclyl.
(33) The compound or its pharmaceutically acceptable salt according to above item (30), wherein ring A is a group represented by formula: 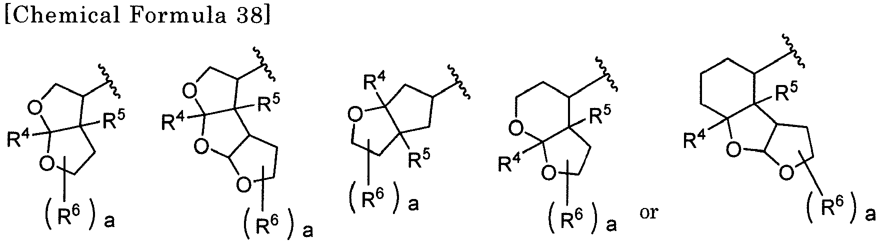
   wherein R⁵ are each independently hydrogen atom, halogen, hydroxy, carboxy, amino, carbamoyl, sulfamoyl, cyano, nitro, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl,
   R⁶ are each independently halogen, hydroxy, carboxy, amino, imino, hydroxyamino, hydroxyimino, formyl, formyloxy, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
   ring A may be substituted with said R⁶ at any substitutable position(s),
   a is an integer of 0 to 7.
(34) A compound represented by formula (III): 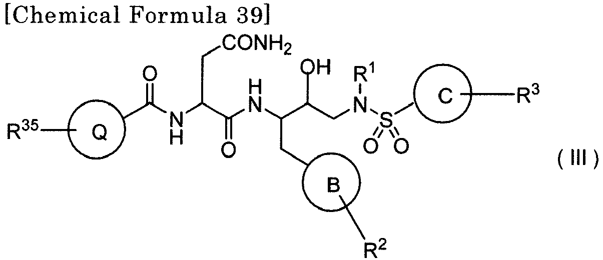
   wherein ring Q is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   ring B is substituted or unsubstituted aromatic carbocyclyl, or substituted or unsubstituted aromatic heterocyclyl,
   ring C is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R¹ is a group represented by formula: -Y-Z, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   R², R³ and R³⁵ are each independently a group represented by formula: -Y-Z, or hydrogen atom,
   provided that at least one of R¹, R², R³ and R³⁵ is a group represented by formula: -Y-Z,
   Y is each independently a bond, or a spacer of any combination selected from the group consisting of -O-, -S-, -NR⁷-, -C(=O)-, -SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   R⁷ are each independently hydrogen atom, hydroxy, carboxy, amino, carbamoyl, sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   Z are each independently aromatic carbocyclyl having acid group, non-aromatic carbocyclyl having acid group, aromatic heterocyclyl having acid group or non-aromatic heterocyclyl having acid group, or its pharmaceutically acceptable salt.
(35) A compound represented by formula (IV):

   X-Y-Z

   wherein X is a compound residue of active ingredient,
   Y is each independently a bond, or a spacer of any combination selected from the group consisting of -O-, -S-, -NR⁷-, -C(=O)-, -SO-, -SO₂-, -NR⁷-C(=O)-, -C(=O)-NR⁷-, -NR⁷-C(=O)-NR⁷-, -O-C(=O)-NR⁷-, -NR⁷-C(=O)-O-, -SO₂-NR⁷-, -NR⁷-SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   provided that the groups selected from the group consisting of -O-, -S- and - NR⁷ - are not connected adjacently in Y, and
   provided that the groups selected from the group consisting of -C(=O)-, -SO-,-SO₂-, -NR⁷-C(=O)-, -C(=O)-NR⁷-, -NR⁷-C(=O)-NR⁷-, -O-C(=O)-NR⁷-, -NR -C(=O)-O-,-SO₂-NR⁷- and -NR⁷-SO₂- are not connected adjacently in Y,
   R⁷ are each independently hydrogen atom, hydroxy, carboxy, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, substituted or unsubstituted non-aromatic heterocyclylsulfonyl,
   Z is a group represented by formula: 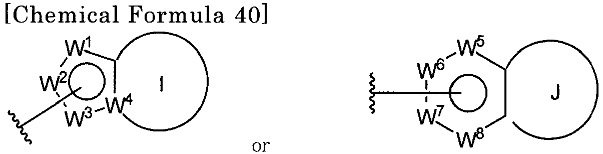
      wherein W¹, W², W³, W⁵, W⁶, W⁷ and W⁸ are each independently C, CR²⁶, O, S, N or NR²⁷,
      W⁴ is C, or N,
      R²⁶ are each independently -COOH or its biologically equivalent group, hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
      provided that at least one of W¹, W² and W³ is CR²⁶, and at least one of said R²⁶ is -COOH or its biologically equivalent group,
      provided that at least one of W⁵, W⁶, W⁷ and W⁸ is CR²⁶, and at least one of said R²⁶ is -COOH or its biologically equivalent group,
      R²⁷ are each independently hydrogen atom, carboxy, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
      ring I and ring J are each independently substituted or unsubstituted non-aromatic carbocycle, or substituted or unsubstituted non-aromatic heterocycle, or its pharmaceutically acceptable salt.
(35') A compound represented by formula (IV):

   X-Y-Z

   wherein X is a residue of compound having HIV protease inhibitor activity,
   Y is each independently a bond, or a spacer of any combination selected from the group consisting of -O-, -S-, -NR⁷-, -C(=O)-, -SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   Z are each independently aromatic carbocyclyl having acid group, non-aromatic carbocyclyl having acid group, aromatic heterocyclyl having acid group or non-aromatic heterocyclyl having acid group,
   or its pharmaceutically acceptable salt.
(36) The compound or its pharmaceutically acceptable salt according to above item (35), wherein X is a residue of compound having HIV protease inhibitor activity.
(37) The compound or its pharmaceutically acceptable salt according to any one of above items (35), (35') and (36), wherein X is a residue of Amprenavir, Atazanavir, Darunavir, Fosamprenavir, Indinavir, Lopinavir, Ritonavir, Nelfinavir, Saquinavir, Tipranavir or its derivative.
(38) The compound or its pharmaceutically acceptable salt according to above item (37), wherein X is a residue of Darunavir derivative or Atazanavir derivative.
(39) The compound or its pharmaceutically acceptable salt according to any one of above items (35) to (38) and (35'), wherein Z is a group represented by formula: 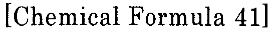 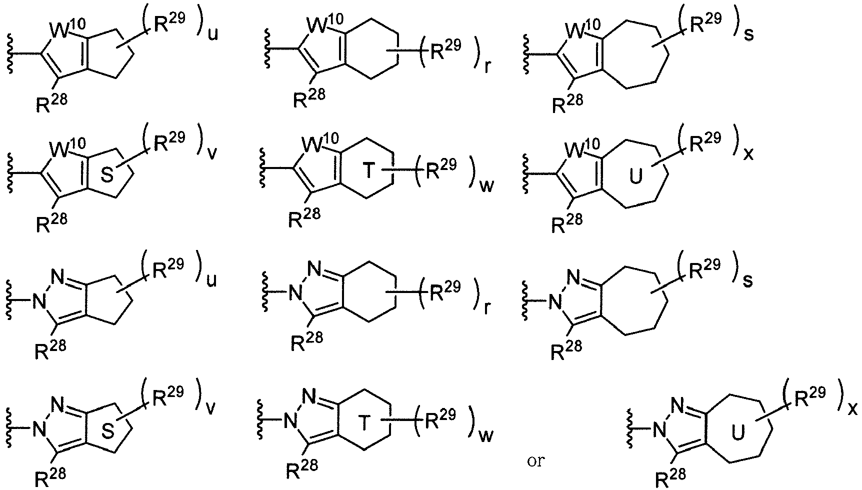
   wherein W¹⁰ is -S-, -O- or -NR²⁷-,
   ring S is 5-membered non-aromatic heterocycle having one hetero atom selected from O, S or NR²⁷, and said hetero atom is not a condensed positional atom,
   ring T is 6-membered non-aromatic heterocycle having one hetero atom selected from O, S or NR²⁷, and said hetero atom is not a condensed positional atom,
   ring U is 7-membered non-aromatic heterocycle having one hetero atom selected from O, S or NR²⁷, and said hetero atom is not a condensed positional atom,
   R²⁸ is -COOH,
   R²⁹ are each independently halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
   two R²⁹ connected to the adjacent carbon atoms may be taken together to form substituted or unsubstituted aromatic carbocycle, substituted or unsubstituted non-aromatic carbocycle, or substituted or unsubstituted non-aromatic heterocycle,
   two R²⁹ connected to the non-adjacent and different carbon atoms may be taken together to form substituted or unsubstituted alkylene,
   two R²⁹ connected to the same carbon atom may be taken together to form substituted or unsubstituted non-aromatic carbocycle or substituted or unsubstituted non-aromatic heterocycle,
   two R²⁹ connected to the same carbon atom may be taken together to form oxo,
   v is an integer of 0 to 4,
   w is an integer of 0 to 6,
   x is an integer of 0 to 8.
(40) The compound or its pharmaceutically acceptable salt according to any one of above items (35) to (39) and (35'), wherein Z is a group represented by formula: 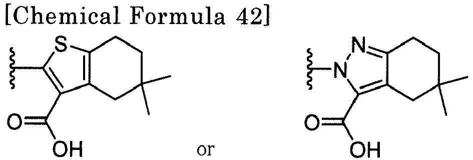
(41) A compound represented by formula (V): 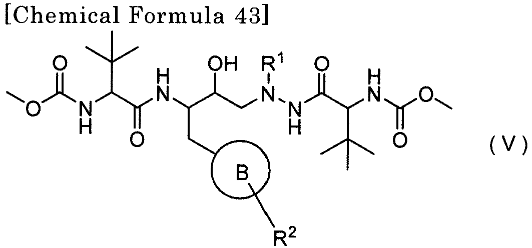
   wherein ring B is substituted or unsubstituted aromatic carbocyclyl, or substituted or unsubstituted aromatic heterocyclyl,
   R¹ is a group represented by formula: -Y-Z, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   R² is a group represented by formula: -Y-Z or hydrogen atom,
   provided that at least one of R¹ and R² is a group represented by formula: -Y-Z,
   Y and Z are defined as the same in above item (35').
   or its pharmaceutically acceptable salt.
(42) A compound represented by any one of the following formula or its pharmaceutically acceptable salt. 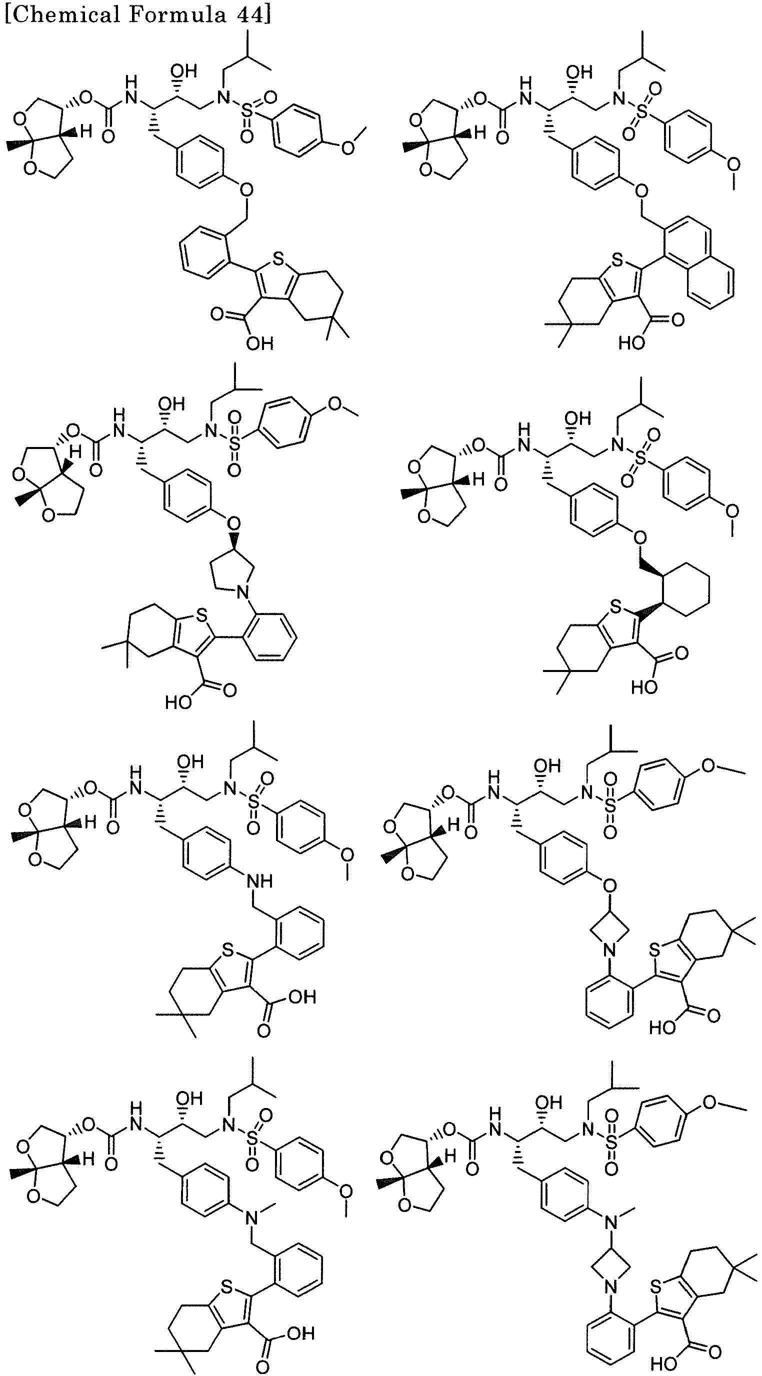 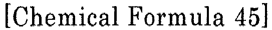 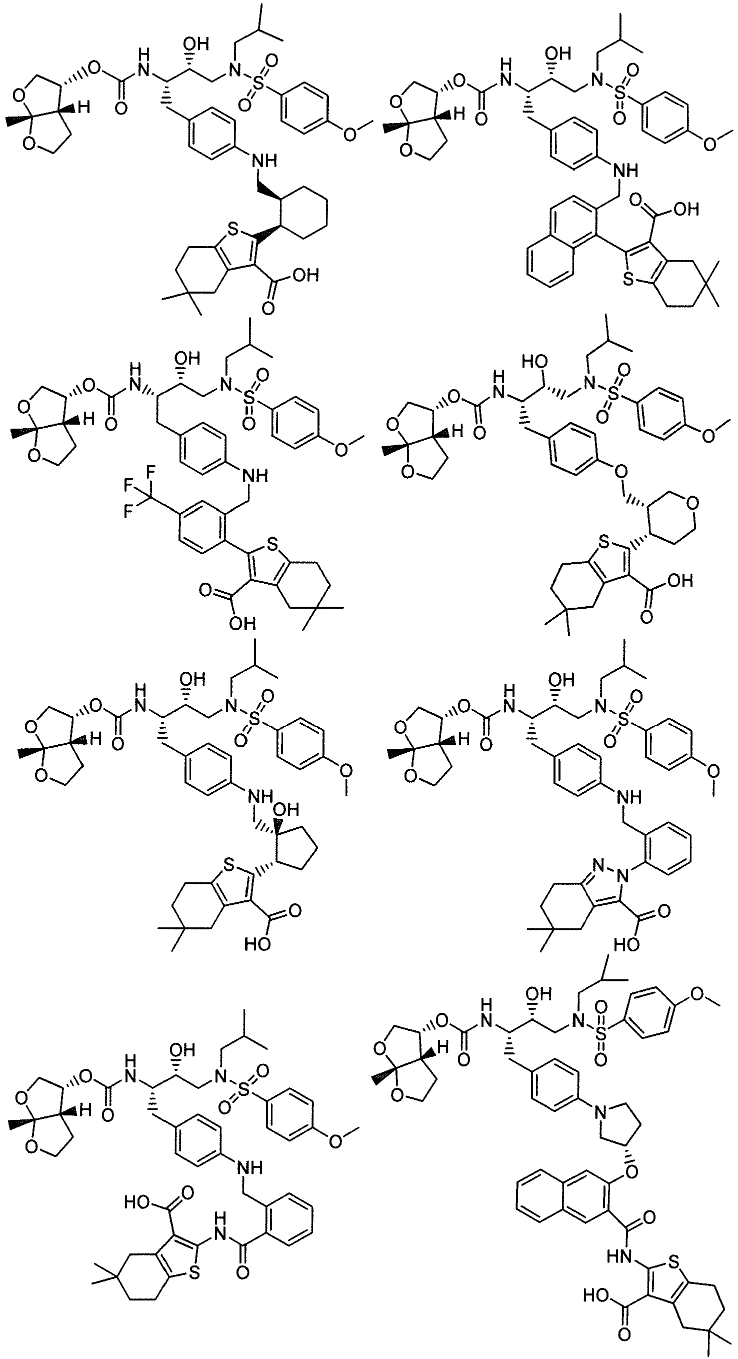 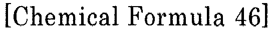 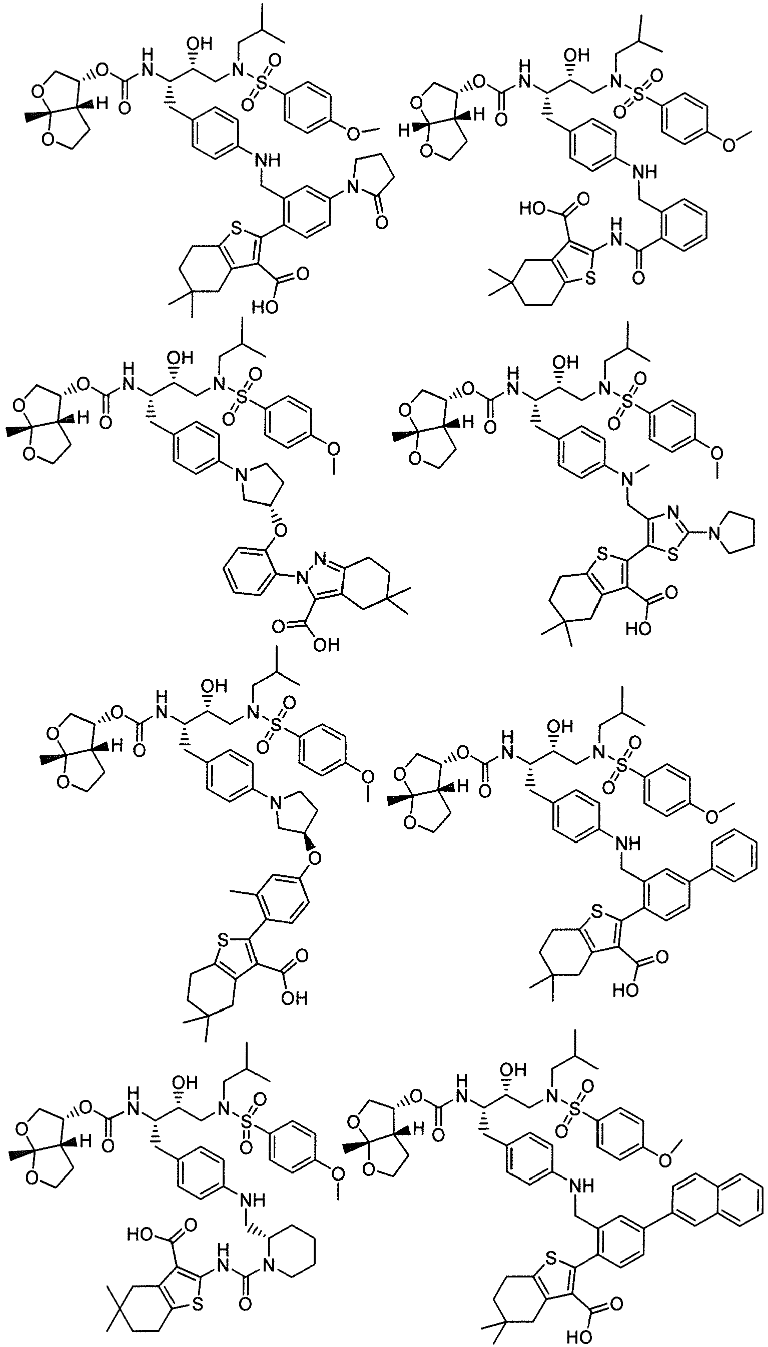 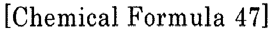 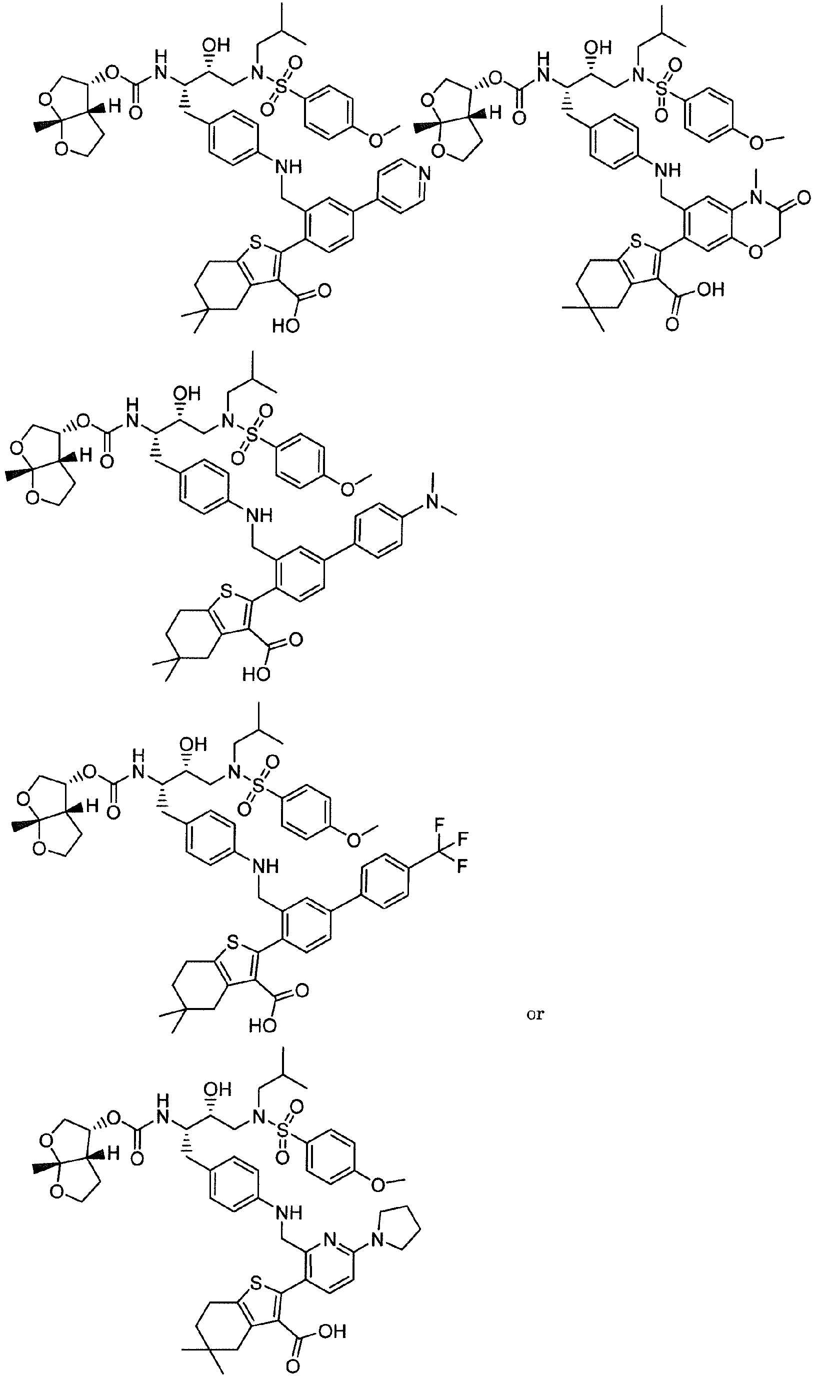
(43) A method of lengthening half-life of active ingredient in pharmacokinetics and/or decreasing clearance by introducing a group represented by the following formula into active ingredient, 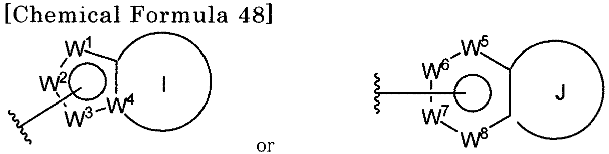 wherein each symbols are defined as the same in above item (35).
(44) The method according to above item (43), wherein the group represented by the following formula: 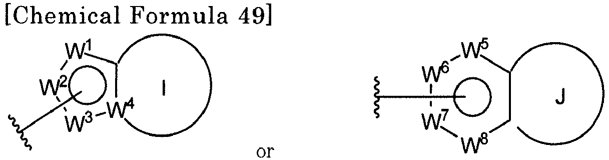 wherein each symbols are defined as the same in above item (35) is any one of the group represented by the following formulae: 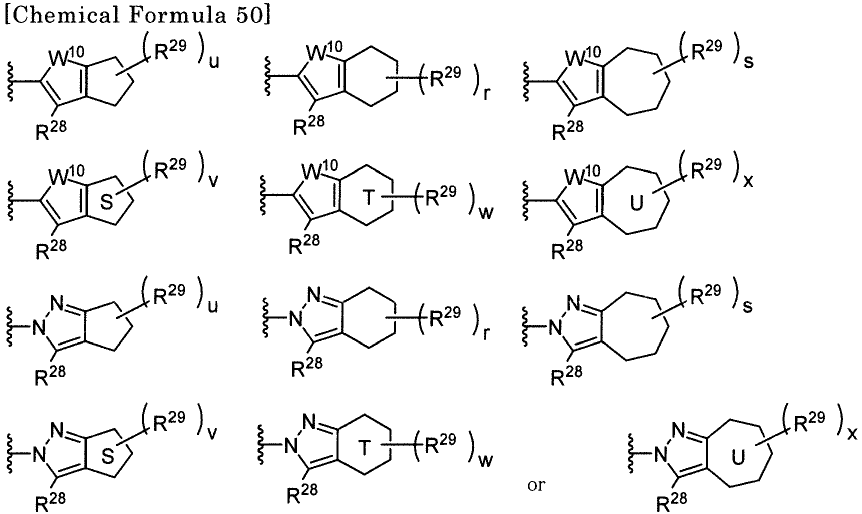 wherein each symbols are defined as the same in above item (39)
(45) The method according to above item (43), wherein the group represented by the following formula: 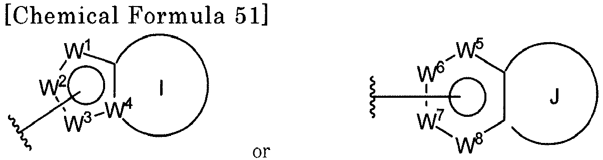 wherein each symbols are defined as the same in above item (35) is any one of the group represented by the following formula. 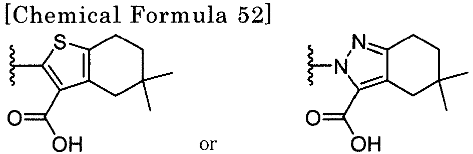
(46) A pharmaceutical composition comprising the compound according to any of above items (1) to (42), (1'), (9'), (11') to (15'), (17') to (24') and (35').
(47) The pharmaceutical composition according to above (46), which has an HIV protease inhibitory activity.
(48) The pharmaceutical composition according to above item (46) or (47), for medical treatment or prevention of HIV infection disease.
(49) The pharmaceutical composition according to any one of above items (46) to (48), which is long acting injection.
(50) The pharmaceutical composition according to any one of above items (46) to (49), wherein dosage interval is once in a month or more.
(51) A method for treating or preventing HIV infection disease by administering the compound of any one of above items (1) to (42), (1'), (9'), (11') to (15'), (17') to (24') and (35'), or its pharmaceutically acceptable salt.
(52) The compound of any one of above items (1) to (42), (1'), (9'), (11') to (15'), (17') to (24') and (35'), or its pharmaceutically acceptable salt for treating or preventing HIV infection disease.
(53) A pharmaceutical composition comprising the compound represented by formula (I), or its pharmaceutically acceptable salt, for oral administration.
(54) A pharmaceutical composition comprising the compound represented by formula (II), or its pharmaceutically acceptable salt, for oral administration.
(55) A pharmaceutical composition comprising the compound represented by formula (III), or its pharmaceutically acceptable salt, for oral administration.
(56) A pharmaceutical composition comprising the compound represented by formula (IV), or its pharmaceutically acceptable salt, for oral administration.
(57) The pharmaceutical composition of any one of above items (53) to (56), which is a tablet, powder, granule, capsule, pill, film, suspension, emulsion, elixir, syrup, lemonade, spirit, aromatic water, extract, decoction or tincture.
(58) The pharmaceutical composition of (57), which is a sugar-coated tablet, film-coated tablet, enteric-coated tablet, sustained-release tablet, troche tablet, sublingual tablet, buccal tablet, chewable tablet, orally disintegrated tablet, dry syrup, soft capsule, micro capsule or sustained-release capsule.
(59) A pharmaceutical composition comprising the compound represented by formula (I), or its pharmaceutically acceptable salt, for parenteral administration.
(60) A pharmaceutical composition comprising the compound represented by formula (II), or its pharmaceutically acceptable salt, for parenteral administration.
(61) A pharmaceutical composition comprising the compound represented by formula (III), or its pharmaceutically acceptable salt, for parenteral administration.
(62) A pharmaceutical composition comprising the compound represented by formula (IV), or its pharmaceutically acceptable salt, for parenteral administration.
(63) The pharmaceutical composition of any one of above items (59) to (62), for dermal, subcutaneous, intravenous, intra-arterial, intramuscular, intraperitoneal, trans mucosal, inhalation, trans nasal, ophthalmic, inner ear or vaginal administration.
(64) The pharmaceutical composition of (63), which is injection, infusion, eye drop, nose drop, ear drop, aerosol, inhalation, lotion, impregnation, liniment, mouthwash, enema, ointment, plaster, jelly, cream, patch, cataplasm, external powder or suppository.
(65) A pharmaceutical composition comprising the compound represented by formula (I), or its pharmaceutically acceptable salt, for a pediatric or geriatric patient.
(66) A pharmaceutical composition comprising the compound represented by formula (II), or its pharmaceutically acceptable salt, for a pediatric or geriatric patient.
(67) A pharmaceutical composition comprising the compound represented by formula (III), or its pharmaceutically acceptable salt, for a pediatric or geriatric patient.
(68) A pharmaceutical composition comprising the compound represented by formula (IV), or its pharmaceutically acceptable salt, for a pediatric or geriatric patient.
(69) A pharmaceutical composition comprising a combination of the compound represented by formula (I) or its pharmaceutically acceptable salt, and reverse transcriptase inhibitor, protease inhibitor, integrase inhibitor or CCR5 inhibitor.
(70) A pharmaceutical composition comprising a combination of the compound represented by formula (II) or its pharmaceutically acceptable salt, and reverse transcriptase inhibitor, protease inhibitor, integrase inhibitor or CCR5 inhibitor.
(71) A pharmaceutical composition comprising a combination of the compound represented by formula (III) or its pharmaceutically acceptable salt, and reverse transcriptase inhibitor, protease inhibitor, integrase inhibitor or CCR5 inhibitor.
(72) A pharmaceutical composition comprising a combination of the compound represented by formula (IV) or its pharmaceutically acceptable salt, and reverse transcriptase inhibitor, protease inhibitor, integrase inhibitor or CCR5 inhibitor.
(73) A pharmaceutical composition comprising the compound represented by formula (I), or its pharmaceutically acceptable salt, for a combination therapy with reverse transcriptase inhibitor, protease inhibitor, integrase inhibitor or CCR5 inhibitor.
(74) A pharmaceutical composition comprising the compound represented by formula (II), or its pharmaceutically acceptable salt, for a combination therapy with reverse transcriptase inhibitor, protease inhibitor, integrase inhibitor or CCR5 inhibitor.
(75) A pharmaceutical composition comprising the compound represented by formula (III), or its pharmaceutically acceptable salt, for a combination therapy with reverse transcriptase inhibitor, protease inhibitor, integrase inhibitor or CCR5 inhibitor.
(76) A pharmaceutical composition comprising the compound represented by formula (IV), or its pharmaceutically acceptable salt, for a combination therapy with reverse transcriptase inhibitor, protease inhibitor, integrase inhibitor or CCR5 inhibitor.
(77) A method for manufacturing of a pharmaceutical composition which is long acting injection of HIV protease inhibitor, by introducing a group represented by formula: -Y-Z into HIV protease inhibitor,
   wherein Y is each independently a bond, or a spacer of any combination selected from the group consisting of -O-, -S-, -NR⁷ -, -C(=O)-, -SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   R⁷ are each independently hydrogen atom, hydroxy, carboxy, amino, carbamoyl, sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   Z are each independently aromatic carbocyclyl having acid group, non-aromatic carbocyclyl having acid group, aromatic heterocyclyl having acid group or non-aromatic heterocyclyl having acid group.
(78) A method of improving a biokinetics by introducing a group represented by formula: -Y-Z into HIV protease inhibitor,
   wherein Y is each independently a bond, or a spacer of any combination selected from the group consisting of -O-, -S-, -NR⁷ -, -C(=O)-, -SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
   R⁷ are each independently hydrogen atom, hydroxy, carboxy, amino, carbamoyl, sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
   Z are each independently aromatic carbocyclyl having acid group, non-aromatic carbocyclyl having acid group, aromatic heterocyclyl having acid group or non-aromatic heterocyclyl having acid group.
(79) A method for treating or preventing HIV infection disease by administering the compound of any one of above items (1) to (42), (1'), (9'), (11') to (15'), (17') to (24') and (35'), or its pharmaceutically acceptable salt.
(80) The compound of any one of above items (1) to (42), (1'), (9'), (11') to (15'), (17') to (24') and (35'), or its pharmaceutically acceptable salt for treating or preventing HIV infection disease.
(81) A compound represented by the following formula or its pharmaceutically acceptable salt, 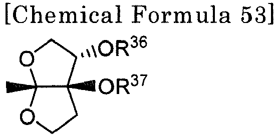
   wherein R³⁶ is hydrogen atom, a protecting group for hydroxy group or -C(=O)-R³⁸,
   wherein R³⁸ is leaving group,
   R³⁷ is hydrogen atom or a protecting group for hydroxy group.
(82) A compound represented by the following formula or its pharmaceutically acceptable salt, 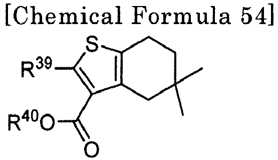
   wherein R³⁹ is hydrogen atom, halogen, boronate, boronate ester, or a group represented by formula: -OR⁴¹, or -NH(R⁴²),
   R⁴¹ is methanesulfonyl group, trifluoromethanesulfonyl group, p-toluenesulfonyl group or nonafluorobutanesulfonyl group,
   R⁴² is hydrogen atom or a protecting group for amino group,
   R⁴⁰ is hydrogen atom or a protecting group for hydroxy group,
   provided that the following compounds are excluded: 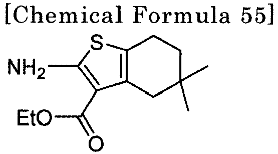
(83) A compound represented by the following formula or its pharmaceutically acceptable salt, 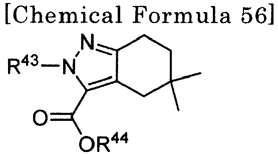
   wherein R⁴³ is hydrogen, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, or a group represented by formula: -C(=O)-R⁴⁵ , or -SO₂-R⁴⁶ ,
   R⁴⁵ is substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted amino, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R⁴⁶ is substituted or unsubstituted alkyl, substituted or unsubstituted alkenyl, substituted or unsubstituted alkynyl, substituted or unsubstituted amino, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R⁴⁴ is hydrogen atom or a protecting group for hydroxy group,
   provided that the following compounds are excluded: 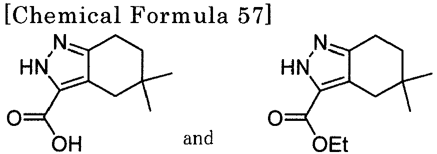
(84) A compound represented by the following formula or its pharmaceutically acceptable salt, 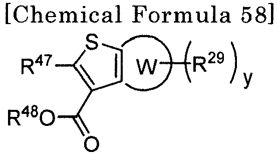
   wherein ring W is 5- to 8-membered non-aromatic carbocycle,
   R²⁹ is defined as the same in above item (24),
   when ring W is 5-membered ring, Y is an integer of 0 to 6,
   when ring W is 6-membered ring, Y is an integer of 0 to 8,
   when ring W is 7-membered ring, Y is an integer of 0 to 10,
   when ring W is 8-membered ring, Y is an integer of 0 to 12,
   R⁴ is halogen, boronate, boronate ester, or a group represented by formula:-OR⁴⁹,
   R⁴⁹ is methanesulfonyl group, trifluoromethanesulfonyl group, p-toluenesulfonyl group, or nonafluorobutanesulfonyl group,
   R⁴⁸ is a protecting group for hydroxy group.
   provided that the following compounds are excluded: 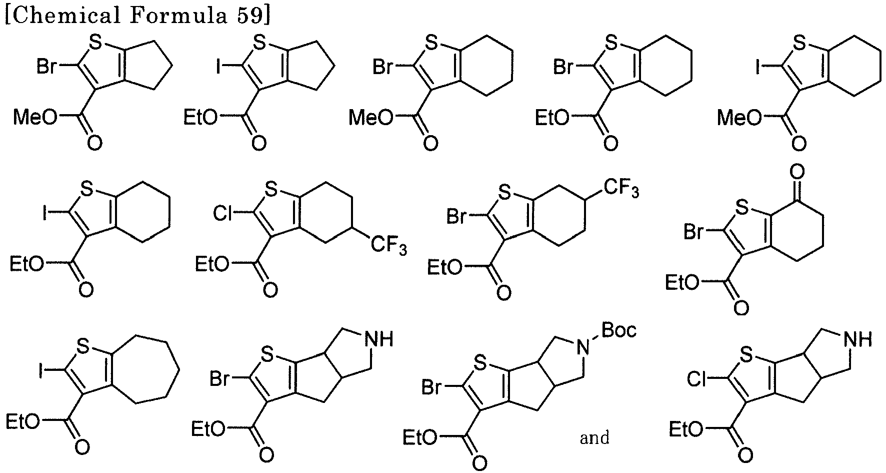
(85) A compound represented by the following formula or its pharmaceutically acceptable salt, 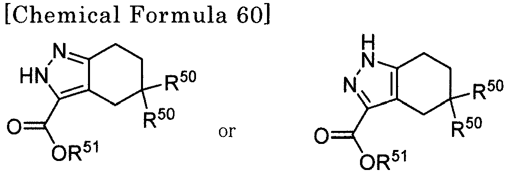
   wherein R⁵ are each independently hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
   R⁵⁰ may be taken together with the adjacent carbon atom to form substituted or unsubstituted non-aromatic carbocycle,
   provided that R⁵⁰ is not hydrogen atom at the same time,
   R⁵¹ is a protecting group for hydroxy group.
   provided that the following compounds are excluded:

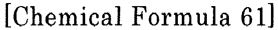

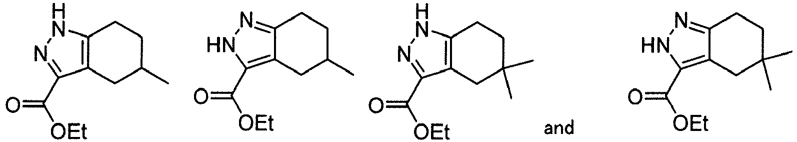

### EFFECT OF THE INVENTION

The compound of the present invention has protease inhibitory activity and/or cell growth inhibitory activity against virus especially HIV or resistant virus. Therefore, it is useful for treatment or prevention against a variety of disease relating to protease or virus infections (ex. AIDS). More preferably, the compound of the present invention is useful for long-acting HIV protease inhibitor improving clearance. Moreover, the compound is superior to resistant profile such as hardly occurring HIV resistant virus.

The others, the compound of the present invention is useful for medicament. High metabolic stability, less induction of drug metabolic enzyme, low inhibition of drug metabolic enzyme to other drugs, high oral absorbance, long half-life, high enzyme activity, safety or low possibility of cytotoxicity or side effect (ex. mutagenesis, QT prolongation in electrocardiogram) are include as usabilities for medicament.

### EMBODIMENTS FOR CARRYING OUT THE INVENTION

Terms used in this description are explained below. Each term, unless otherwise indicated, has the same meaning when it is used alone or together with other terms.

The term of "consisting of" means having only components.

The term of "comprising" means not restricting with components and not excluding undescribed factors.

The term "halogen" includes a fluorine atom, a chlorine atom, a bromine atom and an iodine atom. A fluorine atom and a chlorine atom are especially preferable.

The term "alkyl" includes a C1 to C15, preferably C1 to C10, more preferably C1 to C6 and further preferably C1 to C4 linear or branched hydrocarbon group. Examples For example, it includes methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, n-decyl and the like.

A preferred embodiment of "alkyl" is methyl, ethyl, n-propyl, isopropyl, n-butyl, isobutyl, sec-butyl, tert-butyl or n-pentyl. A more preferred embodiment is methyl, ethyl, n-propyl, isopropyl or tert-butyl.

The term "alkenyl" includes a C2 to C15, preferably a C2 to C10, more preferably a C2 to C6 and further preferably a C2 to C4 linear or branched hydrocarbon group having one or more double bond(s) at any position(s). Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, pentadecenyl and the like.

A preferred embodiment of "alkenyl" is vinyl, allyl, propenyl, isopropenyl or butenyl.

The term "alkynyl" includes a C2 to C10, preferably a C2 to C8, more preferably a C2 to C6 and further preferably a C2 to C4 linear or branched hydrocarbon group having one or more triple bond(s) at any position(s). Furthermore, it may have double bond(s) at any position(s). Examples include ethynyl, propynyl, butynyl, pentynyl, hexynyl, heptynyl, octynyl, nonynyl, decynyl and the like.

A preferred embodiment of "alkynyl" is ethynyl, propynyl, butynyl or pentynyl.

The term "alkylene" includes a C1 to C15, preferably a C1 to C10, more preferably a C1 to C6 and further preferably a C1 to C4 liner or branched bivalent hydrocarbon group. Examples include methylene, ethylene, trimethylene, propylene, tetramethylene, pentamethylene, hexamethylene and the like.

The term "alkenylene" includes a C2 to C15, preferably a C2 to C10, more preferably a C2 to C6 and further preferably a C2 to C4 liner or branched bivalent hydrocarbon group having one or more double bond(s) at any position(s). Examples include vinylene, prenylene, butenylene, pentenylene and the like.

The term "alkynylene" includes a C2 to C15, preferably a C2 to C10, more preferably a C2 to C6 and further preferably a C2 to C4 liner or branched bivalent hydrocarbon group having one or more triple bond(s) at any position(s). Furthermore, it may have double bond(s) at any position(s). Examples include ethynylene, propynylene, butynylene, pentynylene, hexynylene and the like.

The term "aromatic carbocyclyl" means a cyclic aromatic hydrocarbon group which is monocyclic or polycyclic having two or more rings, preferably C6 to C14, more preferably C6 to C10. Examples include phenyl, naphthyl, anthryl, phenanthryl and the like.

A preferred embodiment of "aromatic carbocyclyl" is phenyl.

The term "non-aromatic carbocyclyl" means a cyclic saturated hydrocarbon group or a cyclic unsaturated non-aromatic hydrocarbon group, which is monocyclic or polycyclic having two or more rings. Examples of the "non-aromatic carbocyclyl", which is polycyclic having two or more rings, include a fused ring group wherein a non-aromatic carbocyclyl, which is monocyclic or polycyclic having two or more rings, is fused with a ring of the above "aromatic carbocyclyl".

In addition, examples of the "non-aromatic carbocyclyl" also include a group having a bridge or a group to form a spiro ring as follows:

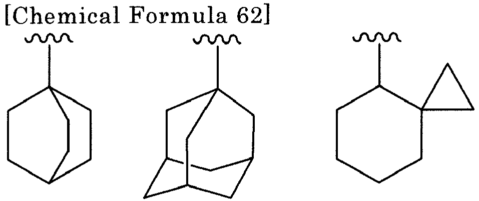

The non-aromatic carbocyclyl which is monocyclic is preferably C3 to C16, more preferably C3 to C12 and further preferably C4 to C8 carbocyclyl. Examples include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, cyclohexadienyl and the like.

The non-aromatic carbocyclyl, which is polycyclic having two or more rings preferably C8 to C13, more preferably C9 to C10. Its Example includes indanyl, indenyl, acenaphthyl, tetrahydronaphthyl, fluorenyl and the like.

The non-aromatic carbocycle means a ring induced from above non-aromatic carbocyclyl. It includes saturated aromatic carbocycle and non-aromatic carbocycle.

The term "aromatic heterocyclyl" means an aromatic cyclyl, which is monocyclic or polycyclic having two or more rings, containing one or more of heteroatom(s) selected independently from O, S and N. Examples of "aromatic heterocyclyl", which is polycyclic having two or more rings, include a fused ring group wherein an aromatic heterocyclyl, which is monocyclic or polycyclic having two or more rings, is fused with a ring of the above "aromatic carbocyclyl".

The aromatic heterocyclyl, which is monocyclic, is preferably a 5- to 8-membered and more preferably 5- to 6- membered ring. Examples include pyrrolyl, imidazolyl, pyrazolyl, pyridyl, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl, thiadiazolyl and the like.

The bicyclic aromatic heterocyclyl is preferably 8- to 18-membered ring, more preferably 9- or 10-membered ring. Examples of aromatic heterocyclyl, which is bicyclic, include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, thiazolopyridyl and the like.

The aromatic heterocyclyl having three or more rings is preferably 11- to 26-membered ring, more preferably 13- or 14-membered ring. Examples of aromatic heterocyclyl, which is polycyclic having three or more rings, include carbazolyl, acridinyl, xanthenyl, phenothiazinyl, phenoxathiinyl, phenoxazinyl, dibenzofuryl and the like.

The term "non-aromatic heterocyclyl" means a non-aromatic cyclyl, which is monocyclic or polycyclic having two or more rings, containing one or more heteroatom(s) selected independently from O, S and N. Examples of "non-aromatic heterocyclyl", which is polycyclic having two or more rings, include a fused ring group wherein a non-aromatic heterocycle, which is monocyclic or polycyclic having two or more ring(s), is fused with a ring of the above "aromatic carbocyclyl", "non-aromatic carbocyclyl" and/or "aromatic heterocyclyl".

In addition, examples of the "non-aromatic heterocyclyl" also include a group having a bridge or a group to form a spiro ring as follows:

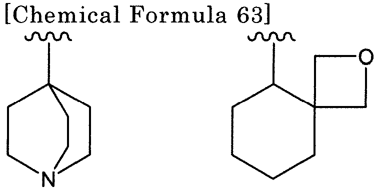

The non-aromatic heterocyclyl, which is monocyclic, is preferably a 3- to 8-membered and more preferably 5- to 6- membered ring. Examples include dioxanyl, thiiranyl, oxiranyl, oxetanyl, oxathiolanyl, azetidinyl, thianyl, thiazolidinyl, pyrrolidinyl, pyrrolinyl, imidazolidinyl, imidazolinyl, pyrazolidinyl, pyrazolinyl, piperidinyl, piperazinyl, morpholinyl, morpholino, thiomorpholinyl, thiomorpholino, dihydropyridinyl, tetrahydropyridinyl, tetrahydrofuryl, tetrahydropyranyl, dihydrothiazolinyl, tetrahydrothiazolinyl, tetrahydroisothiazolinyl, dihydrooxazinyl, hexahydroazepinyl, tetrahydrodiazepinyl, tetrahydropyridazinyl, hexahydropyrimidinyl, dioxolanyl, dioxazinyl, aziridinyl, dioxolinyl, oxepanyl, thiolanyl, thiinyl, thiazinyl and the like.

The non-aromatic heterocyclyl having two or more rings is preferably 8- to 20-membered ring, more preferably 8- to 16-membered ring. Examples of non-aromatic heterocyclyl, which is polycyclic having two or more rings, include indolinyl, isoindolinyl, chromanyl, isochromanyl and the like.

The non-aromatic heterocycle means a ring induced from above non-aromatic heterocyclyl.

The term "hydroxyalkyl" means a group wherein 1 or more hydroxyl group(s) is replaced with hydrogen atom(s) attached to a carbon atom(s) of the above "alkyl". Examples include hydroxymethyl, 1-hydroxyethyl, 2-hydroxyethyl, 1-hydroxypropyl, 2-hydroxypropyl, 1,2-hydroxyethyl and the like.

A preferred embodiment of "hydroxyalkyl" is hydroxymethyl.

The term "alkyloxy" means a group wherein the above "alkyl" is bonded to an oxygen atom. Examples include methyloxy, ethyloxy, n-propyloxy, isopropyloxy, n-butyloxy, tert-butyloxy, isobutyloxy, sec-butyloxy, pentyloxy, isopentyloxy, hexyloxy and the like.

A preferred embodiment of "alkyloxy" is methyloxy, ethyloxy, n-propyloxy, isopropyloxy or tert-butyloxy.

The term "alkenyloxy" means a group wherein the above "alkenyl" is bonded to an oxygen atom. Examples of include vinyloxy, allyloxy, 1-propenyloxy, 2-butenyloxy, 2-pentenyloxy, 2-hexenyloxy, 2-heptenyloxy, 2-octenyloxy and the like.

The term "alkynyloxy" means a group wherein the above "alkynyl" is bonded to an oxygen atom.

Examples include ethynyloxy, 1-propynyloxy, 2-propynyloxy, 2- butynyloxy, 2-pentynyloxy, 2-hexynyloxy, 2-heptynyloxy, 2-octynyloxy and the like.

The term "haloalkyl" means a group wherein 1 or more "halogen" described above is bonded to the above "alkyl". Examples include monofluoromethyl, monofluoroethyl, monofluoropropyl, 2,2,3,3,3-pentafluoropropyl, monochloromethyl, trifluoromethyl, trichloromethyl, 2,2,2-trifluoroethyl, 2,2,2-trichloroethyl, 1,2-dibromoethyl, 1,1,1-trifluoropropan-2-yl and the like.

A preferred embodiment of "haloalkyl" is trifluoromethyl or trichloromethyl.

The term "haloalkyloxy" means a group wherein the above "haloalkyl" is bonded to an oxygen atom. Examples include monofluoromethoxy, monofluoroethoxy, trifluoromethoxy, trichloromethoxy, trifluoroethoxy, trichloroethoxy and the like.

A preferred embodiment of "haloalkyloxy" is trifluoromethoxy or trichloromethoxy.

The term "alkyloxyalkyl" means a group wherein the above "alkyloxy" is bonded to the above "alkyl". Examples include methoxymethyl, methoxyethyl, ethoxymethyl and the like.

The term "alkyloxyalkyloxy" means a group wherein the above "alkyloxy" is bonded to the above "alkyloxy". Examples include methoxymethoxy, methoxyethoxy, ethoxymethoxy, ethoxyethoxy and the like.

The term "alkylcarbonyl" means a group wherein the above "alkyl" is bonded to a carbonyl group. Examples include methylcarbonyl, ethylcarbonyl, propylcarbonyl, isopropylcarbonyl, tert-butylcarbonyl, isobutylcarbonyl, sec-butylcarbonyl, penthylcarbonyl, isopenthylcarbonyl, hexylcarbonyl and the like.

A preferred embodiment of "alkylcarbonyl" is methylcarbonyl, ethylcarbonyl or n-propylcarbonyl.

The term "Alkenylcarbonyl" means a group wherein the above "alkenyl" is bonded to a carbonyl group. Examples include ethylenylcarbonyl, propenylcarbonyl and the like.

The term "Alkynylcarbonyl" means a group wherein the above "alkynyl" is bonded to a carbonyl group. Examples include ethynylcarbonyl, propynylcarbonyl and the like.

The term "Monoalkylamino" means a group wherein a hydrogen atom attached to a nitrogen atom of an amino group is replaced with the above "alkyl". Examples include methylamino, ethylamino, isopropylamino and the like.

A preferred embodiment of "monoalkylamino" is methylamino or ethylamino.

The term "dialkylamino" means a group wherein two hydrogen atoms attached to a nitrogen atom of an amino group are replaced with two "alkyl" described above. These two alkyl groups may be the same or different. Examples include dimethylamino, diethylamino, N,N-diisopropylamino, N-methyl-N-ethylamino, N-isopropyl-N-ethylamino and the like.

A preferred embodiment of "dialkylamino" is dimethylamino or diethylamino.

The term "alkylsulfonyl" means a group wherein the above "alkyl" is bonded to a sulfonyl group. Examples include methylsulfonyl, ethylsulfonyl, propylsulfonyl, isopropylsulfonyl, tert-butylsulfonyl, isobutylsulfonyl, sec-butylsulfonyl and the like.

A preferred embodiment of "alkylsulfonyl" is methylsulfonyl or ethylsulfonyl.

The term "alkenylsulfonyl" means a group wherein the above "alkenyl" is bonded to a sulfonyl group. Examples include ethylenylsulfonyl, propenylsulfonyl and the like.

The term "alkynylsulfonyl" means a group wherein the above "alkynyl" is bonded to a sulfonyl group. Examples include ethynylsulfonyl, propynylsulfonyl and the like.

The term "monoalkylcarbonylamino" means a group wherein the above "alkylcarbonyl" is replaced with a hydrogen atom bonded to a nitrogen atom of an amino group. Examples include methylcarbonylamino, ethylcarbonylamino, propylcarbonylamino, isopropylcarbonylamino, tert-butylcarbonylamino, isobutylcarbonylamino, sec-butylcarbonylamino and the like.

A preferred embodiment of "monoalkylcarbonylamino" is methylcarbonylamino or ethylcarbonylamino.

The term "dialkylcarbonylamino" means a group wherein the above "alkylcarbonyl" is replaced with two hydrogen atoms bonded to a nitrogen atom of an amino group. Two alkylcarbonyl groups may be the same or different. Examples include dimethylcarbonylamino, diethylcarbonylamino, N, N-diisopropylcarbonylamino and the like.

A preferred embodiment of "dialkylcarbonylamino" is dimethylcarbonylamino or diethylcarbonylamino.

The term "monoalkylsulfonylamino" means a group wherein the above "alkylsulfonyl" is replaced with a hydrogen atom bonded to a nitrogen atom of an amino group. Examples include methylsulfonylamino, ethylsulfonylamino, propylsulfonylamino, isopropylsulfonylamino, tert-butylsulfonylamino, isobutylsulfonylamino, sec-butylsulfonylamino and the like.

A preferred embodiment of "monoalkylsulfonylamino" is methylsulfonylamino or ethylsulfonylamino.

The term "dialkylsulfonylamino" means a group wherein the above "alkylsulfonyl" is replaced with two hydrogen atoms bonded to a nitrogen atom of an amino group. Two alkylsulfonyl groups may be the same or different. Examples include dimethylsulfonylamino, diethylsulfonylamino, N,N-diisopropylsulfonylamino and the like.

A preferred embodiment of "dialkylcarbonylamino" is dimethylsulfonylamino or diethylsulfonylamino.

The term "alkylimino" means a group wherein the above "alkyl" is replaced with a hydrogen atom bonded to a nitrogen atom of an imino group. Examples include methylimino, ethylimino, n-propylimino, isopropylimino and the like.

The term "alkenylimino" means a group wherein the above "alkenyl" is replaced with a hydrogen atom bonded to a nitrogen atom of an imino group. Examples include ethylenylimino, propenylimino and the like.

"Alkynylimino" means a group wherein the above "alkynyl" is replaced with a hydrogen atom bonded to a nitrogen atom of an imino group. For example, it includes ethynylimino, propynylimino and the like.

The term "alkylcarbonylimino" means a group wherein the above "alkylcarbonyl" is replaced with a hydrogen atom bonded to a nitrogen atom of an imino group. Examples include methylcarbonylimino, ethylcarbonylimino, n-propylcarbonylimino, isopropylcarbonylimino and the like.

The term "alkenylcarbonylimino" means a group wherein the above "alkenylcarbonyl" is replaced with a hydrogen atom bonded to a nitrogen atom of an imino group. Examples include ethylenylcarbonylimino, propenylcarbonylimino and the like.

The term "alkynylcarbonylimino" means a group wherein the above "alkynylcarbonyl" is replaced with a hydrogen atom bonded to a nitrogen atom of an imino group. Examples include ethynylcarbonylimino, propynylcarbonylimino and the like.

The "alkyloxyimino" means a group wherein the above "alkyloxy" is replaced with a hydrogen atom bonded to a nitrogen atom of an imino group. Examples include methyloxyimino, ethyloxyimino, n-propyloxyimino, isopropyloxyimino and the like.

The "alkenyloxyimino" means a group wherein the above "alkenyloxy" is replaced with a hydrogen atom bonded to a nitrogen atom of an imino group. Examples include ethylenyloxyimino, propenyloxyimino and the like.

The term "alkynyloxyimino" means a group wherein the above "alkynyloxy" is replaced with a hydrogen atom bonded to a nitrogen atom of an imino group. Examples include ethynyloxyimino, propynyloxyimino and the like.

The term "alkylcarbonyloxy" means a group wherein the above "alkylcarbonyl" is bonded to an oxygen atom. Examples include methylcarbonyloxy, ethylcarbonyloxy, propylcarbonyloxy, isopropylcarbonyloxy, tert-butylcarbonyloxy, isobutylcarbonyloxy, sec-butylcarbonyloxy and the like.

A preferred embodiment of "alkylcarbonyloxy" is methylcarbonyloxy or ethylcarbonyloxy.

The term "alkenylcarbonyloxy" means a group wherein the above "alkenylcarbonyl" is bonded to an oxygen atom. Examples include ethylenylcarbonyloxy, propenylcarbonyloxy and the like.

The term "alkynylcarbonyloxy" means a group wherein the above "alkynylcarbonyl" is bonded to an oxygen atom. Examples include ethynylcarbonyloxy, propynylcarbonyloxy and the like.

The term "alkyloxycarbonyl" means a group wherein the above "alkyloxy" is bonded to a carbonyl group. Examples include methyloxycarbonyl, ethyloxycarbonyl, propyloxycarbonyl, isopropyloxycarbonyl, tert-butyloxycarbonyl, isobutyloxycarbonyl, sec-butyloxycarbonyl, penthyloxycarbonyl, isopenthyloxycarbonyl, hexyloxycarbonyl 1 and the like.

A preferred embodiment of "alkyloxycarbonyl" is methyloxycarbonyl, ethyloxycarbonyl or propyloxycarbonyl.

The term "alkenyloxycarbonyl" means a group wherein the above "alkenyloxy" is bonded to a carbonyl group. Examples include ethylenyloxycarbonyl, propenyloxycarbonyl and the like.

The term "alkynyloxycarbonyl" means a group wherein the above "alkynyloxy" is bonded to a carbonyl group. Examples include ethynyloxycarbonyl, propynyloxycarbonyl and the like.

The term "alkylsulfanyl" means a group wherein the above "alkyl" is replaced with a hydrogen atom bonded to a sulfur atom of a sulfanyl group. Examples include methylsulfanyl, ethylsulfanyl, n-propylsulfanyl, isopropylsulfanyl and the like.

The term "alkenylsulfanyl" means a group wherein the above "alkenyl" is replaced with a hydrogen atom bonded to a sulfur atom of a sulfanyl group. Examples include ethylenylsulfanyl, propenylsulfanyl and the like.

The term "alkynylsulfanyl" means a group wherein the above "alkynyl" is replaced with a hydrogen atom bonded to a sulfur atom of a sulfanyl group. Examples include ethynylsulfanyl, propynylsulfanyl and the like.

The term "alkylsulfinyl" means a group wherein the above "alkyl" is bonded to a sulfinyl group. Examples include methylsulfinyl, ethylsulfinyl, n-propylsulfinyl, isopropylsulfinyl and the like.

The term "alkenylsulfinyl" means a group wherein the above "alkenyl" is bonded to a sulfinyl group. Examples include ethylenylsulfinyl, propenylsulfinyl and the like.

The term "alkynylsulfinyl" means a group wherein the above "alkynyl" is bonded to a sulfinyl group. Examples include ethynylsulfinyl, propynylsulfinyl and the like.

The term "monoalkylcarbamoyl" means a group wherein the above "alkyl" is replaced with one hydrogen atom bonded to a nitrogen atom of a carbamoyl group. Examples include methylcarbamoyl, ethylcarbamoyl and the like.

The term "dialkylcarbamoyl" means a group wherein the above "alkyl" are replaced with two hydrogen atoms bonded to a nitrogen atom of a carbamoyl group. Two alkyl groups may be the same or different. Examples include dimethylcarbamoyl, diethylcarbamoyl and the like.

The term "monoalkylsulfamoyl" means a group wherein the above "alkyl" is replaced with one hydrogen atom bonded to a nitrogen atom of a sulfamoyl group. Examples include methylsulfamoyl, dimethylsulfamoyl and the like.

The term "dialkylsulfamoyl" means a group wherein the above "alkyl" are replaced with two hydrogen atoms bonded to a nitrogen atom of a sulfamoyl group. Two alkyl groups may be the same or different. Examples include dimethylsulfamoyl, diethylsulfamoyl and the like.

The term "trialkylsilyl" means a group wherein three of the above "alkyl" are bonded to a silicon atom. Three alkyl groups may be the same or different. Examples include trimethylsilyl, triethylsilyl, tert-butyldimethylsilyl and the like.

The alkyl part of "aromatic carbocyclylalkyl", "non-aromatic carbocyclylalkyl", "aromatic heterocyclylalkyl", "non-aromatic heterocyclylalkyl", "aromatic carbocyclylalkyloxy", "non-aromatic carbocyclylalkyloxy", "aromatic heterocyclylalkyloxy", "non-aromatic heterocyclylalkyloxy", "aromatic carbocyclylalkyloxycarbonyl", "non-aromatic carbocyclylalkyloxycarbonyl", "aromatic heterocyclylalkyloxycarbonyl", "non-aromatic heterocyclylalkyloxycarbonyl", "aromatic carbocyclylalkyloxyalkyl", "non-aromatic carbocyclylalkyloxyalkyl", "aromatic heterocyclylalkyloxyalkyl", "non-aromatic heterocyclylalkyloxyalkyl", "aromatic carbocyclylalkylamino", "non-aromatic carbocyclylalkylamino", "aromatic heterocyclylalkylamino" or "non-aromatic heterocyclylalkylamino" is also same as the above "alkyl".

The term "aromatic carbocyclylalkyl" means an alkyl substituted with one or more "aromatic carbocyclyl" described above. Examples include benzyl, phenethyl, phenylpropyl, benzhydryl, trityl, naphthylmethyl, a group of the formula of 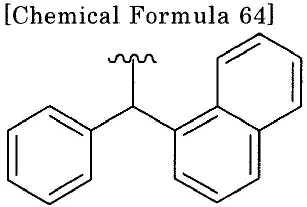 and the like.

A preferred embodiment of "aromatic carbocyclylalkyl" is benzyl, phenethyl or benzhydryl.

The term "non-aromatic carbocyclylalkyl" means an alkyl substituted with one or more "non-aromatic carbocyclyl" described above. The "non-aromatic carbocyclylalkyl" also includes "non-aromatic carbocyclylalkyl" wherein the alkyl part is substituted with the above "aromatic carbocyclyl". Examples include cyclopropylmethyl, cyclobutylmethyl, cyclopenthylmethyl, cyclohexylmethyl, a group of the formula of 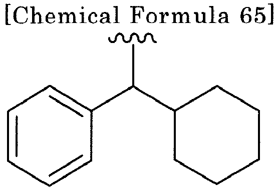 and the like.

The term "aromatic heterocyclylalkyl" means an alkyl substituted with one or more "aromatic heterocyclyl" described above. The "aromatic heterocyclylalkyl" also includes "aromatic heterocyclylalkyl" wherein the alkyl part is substituted with the above "aromatic carbocyclyl" and/or "non-aromatic carbocyclyl". Examples include pyridylmethyl, furanylmethyl, imidazolylmethyl, indolylmethyl, benzothiophenylmethyl, oxazolylmethyl, isoxazolylmethyl, thiazolylmethyl, isothiazolylmethyl, pyrazolylmethyl, isopyrazolylmethyl, pyrrolidinylmethyl, benzoxazolylmethyl, groups of the formula of 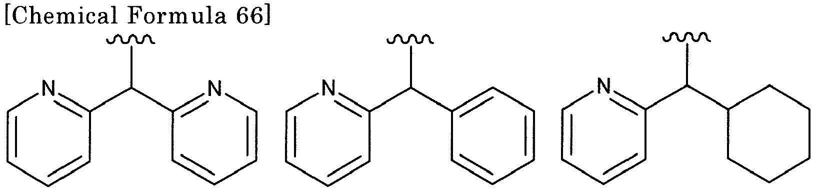 and the like.

The term "non-aromatic heterocyclylalkyl" means an alkyl substituted with one or more "non-aromatic heterocyclyl" described above. The "non-aromatic heterocyclylalkyl" also includes "non-aromatic heterocyclylalkyl" wherein the alkyl part is substituted with the above "aromatic carbocyclyl", "non-aromatic carbocyclyl" and/or "aromatic heterocyclyl". Examples include tetrahydropyranylmethyl, morpholinylethyl, piperidinylmethyl, piperazinylmethyl, groups of the formula of 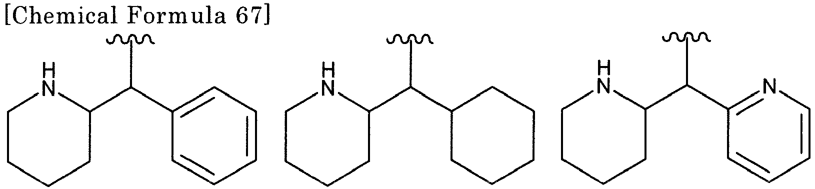 and the like.

The term "aromatic carbocyclylalkyloxy" means an alkyloxy substituted with one or more "aromatic carbocyclyl" described above. Examples include benzyloxy, phenethyloxy, phenylpropyloxy, benzhydryloxy, trityloxy, naphthylmethyloxy, a group of the formula of 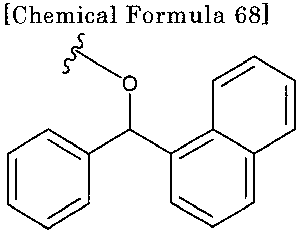 and the like.

The term "non-aromatic carbocyclylalkyloxy" means an alkyloxy substituted with one or more "non-aromatic carbocyclyl" described above. The "non-aromatic carbocyclylalkyloxy" also includes "non-aromatic carbocyclylalkyloxy" wherein the alkyl part is substituted with the above "aromatic carbocyclyl". Examples include cyclopropylmethyloxy, cyclobutylmethyloxy, cyclopenthylmethyloxy, cyclohexylmethyloxy, a group of the formula of 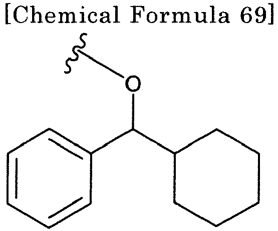 and the like.

The term "aromatic heterocyclylalkyloxy" means an alkyloxy substituted with one or more "aromatic heterocyclyl" described above. The "aromatic heterocyclylalkyloxy" also includes "aromatic heterocyclylalkyloxy" wherein the alkyl part is substituted with the above "aromatic carbocyclyl" and/or "non-aromatic carbocyclyl". Examples include pyridylmethyloxy, furanylmethyloxy, imidazolylmethyloxy, indolylmethyloxy, benzothiophenylmethyloxy, oxazolylmethyloxy, isoxazolylmethyloxy, thiazolylmethyloxy, isothiazolylmethyloxy, pyrazolylmethyloxy, isopyrazolylmethyloxy, pyrrolidinylmethyloxy, benzoxazolylmethyloxy, groups of the formula of 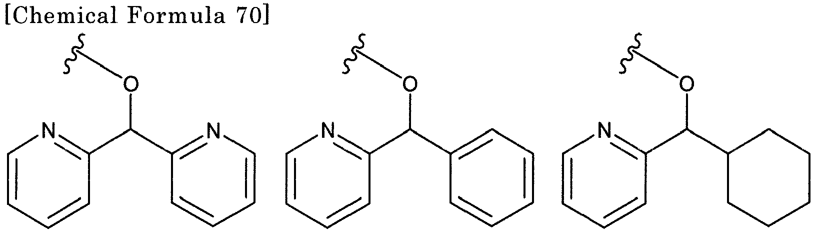 and the like.

The term "non-aromatic heterocyclylalkyloxy" means an alkyloxy substituted with one or more "non-aromatic heterocyclyl" described above. The "non-aromatic heterocyclylalkyloxy" also includes "non-aromatic heterocyclylalkyloxy" wherein the alkyl part is substituted with the above "aromatic carbocyclyl", "non-aromatic carbocyclyl" and/or "aromatic heterocyclyl". Examples include tetrahydropyranylmethyloxy, morpholinylethyloxy, piperidinylmethyloxy, piperazinylmethyloxy, groups of the formula of 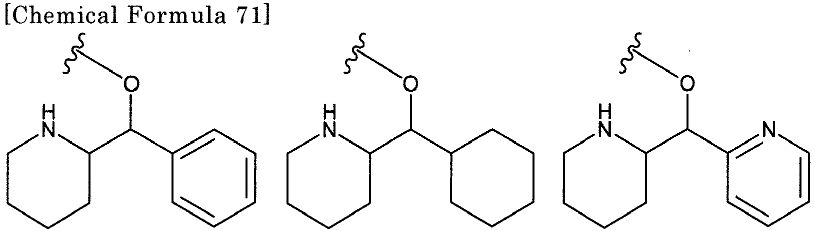 and the like.

The term "aromatic carbocyclylalkyloxycarbonyl" means an alkyloxycarbonyl substituted with one or more "aromatic carbocyclyl" described above. Examples include benzyloxycarbonyl, phenethyloxycarbonyl, phenylpropyloxycarbonyl, benzhydryloxycarbonyl, trityloxycarbonyl, naphthylmethyloxycarbonyl, a group of the formula of 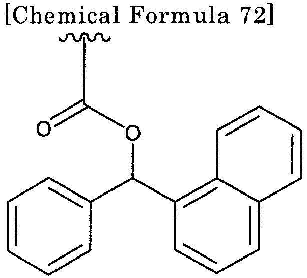 and the like.

The term "non-aromatic carbocyclylalkyloxycarbonyl" means an alkyloxycarbonyl substituted with one or more "non-aromatic carbocyclyl" described above. The "non-aromatic carbocyclylalkyloxycarbonyl" also includes "non-aromatic carbocyclylalkyloxycarbonyl" wherein the alkyl part is substituted with the above "aromatic carbocyclyl". Examples include cyclopropylmethyloxycarbonyl, cyclobutylmethyloxycarbonyl, cyclopenthylmethyloxycarbonyl, cyclohexylmethyloxycarbonyl, a group of the formula of 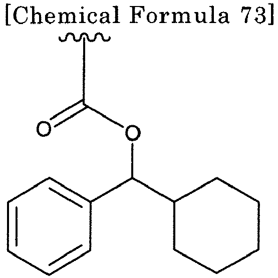 and the like.

The term "aromatic heterocyclylalkyloxycarbonyl" means an alkyloxycarbonyl substituted with one or more "aromatic heterocyclyl" described above. The "aromatic heterocyclylalkyloxycarbonyl" also include "aromatic heterocyclylalkyloxycarbonyl" wherein the alkyl part is substituted with the above "aromatic carbocyclyl" and/or "non-aromatic carbocyclyl". Examples include pyridylmethyloxycarbonyl, furanylmethyloxycarbonyl, imidazolylmethyloxycarbonyl, indolylmethyloxycarbonyl, benzothiophenylmethyloxycarbonyl, oxazolylmethyloxycarbonyl, isoxazolylmethyloxycarbonyl, thiazolylmethyloxycarbonyl, isothiazolylmethyloxycarbonyl, pyrazolylmethyloxycarbonyl, isopyrazolylmethyloxycarbonyl, pyrrolidinylmethyloxycarbonyl, benzoxazolylmethyloxycarbonyl, groups of the formula of 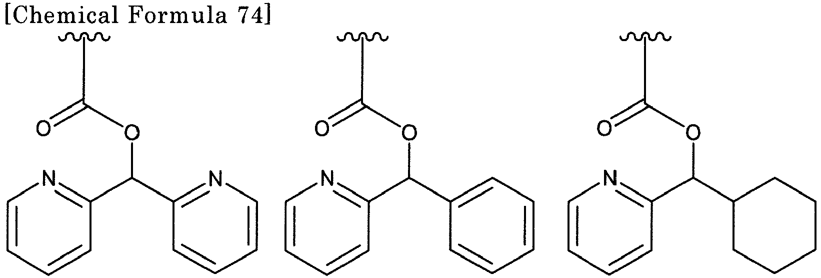 and the like.

The term "non-aromatic heterocyclylalkyloxycarbonyl" means an alkyloxycarbonyl substituted with one or more "non-aromatic heterocyclyl" described above. The "non-aromatic heterocyclylalkyloxycarbonyl" also includes "non-aromatic heterocyclylalkyloxycarbonyl" wherein the alkyl part is substituted with the above "aromatic carbocyclyl", "non-aromatic carbocyclyl" and/or "aromatic heterocyclyl". Examples include tetrahydropyranylmethyloxy, morpholinylethyloxy, piperidinylmethyloxy, piperazinylmethyloxy, groups of the formula of 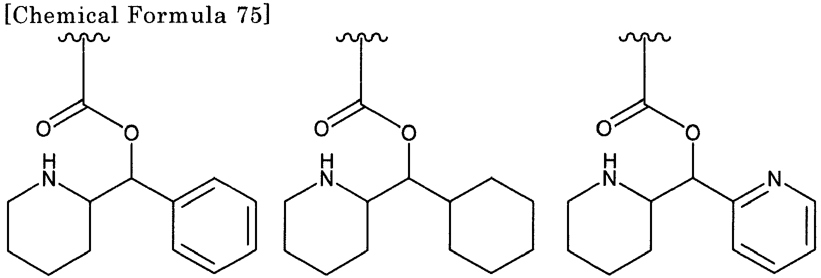 and the like.

The term "aromatic carbocyclylalkyloxyalkyl" means an alkyloxyalkyl substituted with one or more "aromatic carbocyclyl" described above. Examples include benzyloxymethyl, phenethyloxymethyl, phenylpropyloxymethyl, benzhydryloxymethyl, trityloxymethyl, naphthylmethyloxymethyl, a group of the formula of 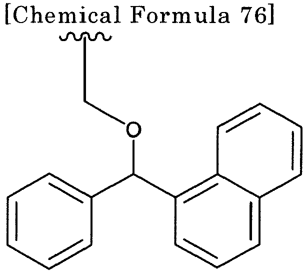 and the like.

The term "non-aromatic carbocyclylalkyloxyalkyl" means an alkyloxyalkyl substituted with one or more "non-aromatic carbocyclyl" described above. The "non-aromatic carbocyclylalkyloxyalkyl" also includes "non-aromatic carbocyclylalkyloxyalkyl" wherein the alkyl part bonded to the non-aromatic carbocycle is substituted with the above "aromatic carbocyclyl". Examples include cyclopropylmethyloxymethyl, cyclobutylmethyloxymethyl, cyclopenthylmethyloxymethyl, cyclohexylmethyloxymethyl, a group of the formula of 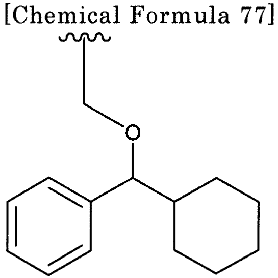 and the like.

The term "aromatic heterocyclylalkyloxyalkyl" means an alkyloxyalkyl substituted with one or more "aromatic heterocyclyl" described above. The "aromatic heterocyclylalkyloxyalkyl" also includes "aromatic heterocyclylalkyloxyalkyl" wherein the alkyl part bonded to the aromatic heterocycle is replaced with the above "aromatic carbocyclyl" and/or "non-aromatic carbocyclyl". Examples include pyridylmethyloxymethyl, furanylmethyloxymethyl, imidazolylmethyloxymethyl, indolylmethyloxymethyl, benzothiophenylmethyloxymethyl, oxazolylmethyloxymethyl, isoxazolylmethyloxymethyl, thiazolylmethyloxymethyl, isothiazolylmethyloxymethyl, pyrazolylmethyloxymethyl, isopyrazolylmethyloxymethyl, pyrrolidinylmethyloxymethyl, benzoxazolylmethyloxymethyl, groups of the formula of 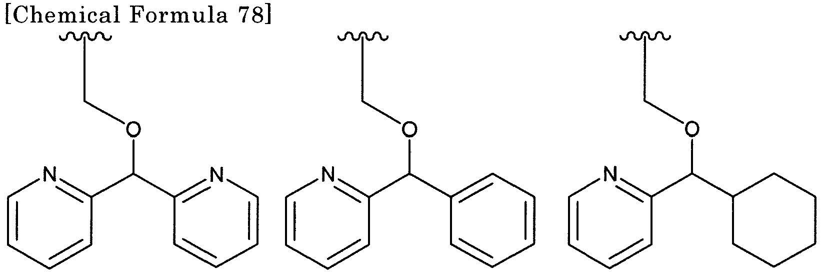 and the like.

The term "non-aromatic heterocyclylalkyloxyalkyl" means an alkyloxyalkyl substituted with one or more "non-aromatic heterocyclyl" described above. The "non-aromatic heterocyclylalkyloxyalkyl" also includes "non-aromatic heterocyclylalkyloxyalkyl" wherein the alkyl part bonded to the non-aromatic heterocycle is substituted with the above "aromatic carbocyclyl", "non-aromatic carbocyclyl" and/or "aromatic heterocyclyl". Examples include tetrahydropyranylmethyloxymethyl, morpholinylethyloxymethyl, piperidinylmethyloxymethyl, piperazinylmethyloxymethyl, groups of the formula of 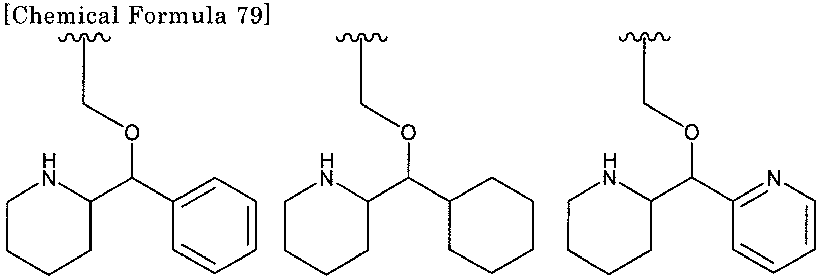 and the like.

The term "aromatic carbocyclylalkylamino" means a group wherein the above "aromatic carbocyclylalkyl" is replaced with one or two hydrogen atom(s) bonded to a nitrogen atom of an amino group. Examples include benzylamino, phenethylamino, phenylpropylamino, benzhydrylamino, tritylamino, naphthylmethylamino, dibenzylamino and the like.

The term "non-aromatic carbocyclylalkylamino" means a group wherein the above "non-aromatic carbocyclylalkyl" is replaced with one or two hydrogen atom(s) bonded to a nitrogen atom of an amino group. Examples include cyclopropylmethylamino, cyclobutylmethylamino, cyclopenthylmethylamino, cyclohexylmethylamino and the like.

The term "aromatic heterocyclylalkylamino" means a group wherein the above "aromatic heterocyclylalkyl" is replaced with one or two hydrogen atom(s) bonded to a nitrogen atom of an amino group. Examples include pyridylmethylamino, furanylmethylamino, imidazolylmethylamino, indolylmethylamino, benzothiophenylmethylamino, oxazolylmethylamino, isoxazolylmethylamino, thiazolylmethylamino, isothiazolylmethylamino, pyrazolylmethylamino, isopyrazolylmethylamino, pyrrolidinylmethylamino, benzoxazolylmethylamino and the like.

The term "non-aromatic heterocyclylalkylamino" means a group wherein the above "non-aromatic heterocyclylalkyl" is replaced with one or two hydrogen atom(s) bonded to a nitrogen atom of an amino group. Examples include tetrahydropyranylmethylamino, morpholinylethylamino, piperidinylmethylamino, piperazinylmethylamino and the like.

The "carbocycle" part of "aromatic carbocyclyloxy", "aromatic carbocyclylcarbonyl", "aromatic carbocyclyloxycarbonyl", "aromatic carbocyclylsulfanyl" or "aromatic carbocyclylsulfonyl" is same as the above "aromatic carbocyclyl".

The term "aromatic carbocyclyloxy" means a group wherein "aromatic carbocycle" is bonded to an oxygen atom. Examples include phenyloxy, naphthyloxy and the like.

The term "aromatic carbocyclylcarbonyl" means a group wherein "aromatic carbocycle" is bonded to a carbonyl group. Examples include phenylcarbonyl, naphthylcarbonyl and the like.

The term "aromatic carbocyclyloxycarbonyl" means a group wherein the above "aromatic carbocyclyloxy" is bonded to a carbonyl group. Examples include phenyloxycarbonyl, naphthyloxycarbonyl and the like.

The term "aromatic carbocyclylsulfanyl" means a group wherein "aromatic carbocycle" is bonded to a hydrogen atom bonded to a sulfur atom of a sulfanyl group. Examples include phenylsulfanyl, naphthylsulfanyl and the like.

The term "aromatic carbocyclylsulfonyl" means a group wherein "aromatic carbocycle" is bonded to a sulfonyl group. Examples include phenylsulfonyl, naphthylsulfonyl and the like.

The "non-aromatic carbocycle" part of "non-aromatic carbocyclyloxy", "non-aromatic carbocyclylcarbonyl", "non-aromatic carbocyclyloxycarbonyl", "non-aromatic carbocyclylsulfanyl" or "non-aromatic carbocyclylsulfonyl" is same as the above "non-aromatic carbocyclyl".

The term "non-aromatic carbocyclyloxy" means a group wherein "non-aromatic carbocycle" is bonded to an oxygen atom. Examples include cyclopropyloxy, cyclohexyloxy, cyclohexenyloxy and the like.

The term "non-aromatic carbocyclylcarbonyl" means a group wherein "non-aromatic carbocycle" is bonded to a carbonyl group. Examples include cyclopropylcarbonyl, cyclohexylcarbonyl, cyclohexenylcarbonyl and the like.

The term "non-aromatic carbocyclyloxycarbonyl" means a group wherein the above "non-aromatic carbocyclyloxy" is bonded to a carbonyl group. Examples include cyclopropyloxycarbonyl, cyclohexyloxycarbonyl, cyclohexenyloxycarbonyl and the like.

The term "non-aromatic carbocyclylsulfanyl" means a group wherein "non-aromatic carbocycle" is replaced with a hydrogen atom bonded to a sulfur atom of a sulfanyl group. Examples include cyclopropylsulfanyl, cyclohexylsulfanyl, cyclohexenylsulfanyl and the like.

The term "non-aromatic carbocyclylsulfonyl" means a group wherein "non-aromatic carbocycle" is bonded to a sulfonyl group. Examples include cyclopropylsulfonyl, cyclohexylsulfonyl, cyclohexenylsulfonyl and the like.

The "aromatic heterocycle" part of "aromatic heterocyclyloxy", "aromatic heterocyclylcarbonyl", "aromatic heterocyclyloxycarbonyl", "aromatic heterocyclylsulfanyl" or "aromatic heterocyclylsulfonyl" is also same as the above "aromatic heterocyclyl".

The term "aromatic heterocyclyloxy" means a group wherein "aromatic heterocycle" is bonded to an oxygen atom. Examples include pyridyloxy, oxazolyloxy and the like.

The term "aromatic heterocyclylcarbonyl" means a group wherein "aromatic heterocycle" is bonded to a carbonyl group. Examples include pyridylcarbonyl, oxazolylcarbonyl and the like.

The term "aromatic heterocyclyloxycarbonyl" means a group wherein the above "aromatic heterocyclyloxy" is bonded to a carbonyl group. Examples include pyridyloxycarbonyl, oxazolyloxycarbonyl and the like.

The term "aromatic heterocyclylsulfanyl" means a group wherein "aromatic heterocycle" is replaced with a hydrogen atom bonded to a sulfur atom of a sulfanyl group. Examples include pyridylsulfanyl, oxazolylsulfanyl and the like.

The term "aromatic heterocyclylsulfonyl" means a group wherein "aromatic heterocycle" is bonded to a sulfonyl group. Examples include pyridylsulfonyl, oxazolylsulfonyl and the like.

The "non-aromatic heterocycle" part of "non-aromatic heterocyclyloxy", "non-aromatic heterocyclylcarbonyl", "non-aromatic heterocyclyloxycarbonyl", "non-aromatic heterocyclylsulfanyl" or "non-aromatic heterocyclylsulfonyl" is also same as the above "non-aromatic heterocyclyl".

The term "non-aromatic heterocyclyloxy" means a group wherein "non-aromatic heterocycle" is bonded to an oxygen atom. Examples include piperidinyloxy, tetrahydrofuryloxy and the like.

The term "non-aromatic heterocyclylcarbonyl" means a group wherein "non-aromatic heterocycle" is bonded to a carbonyl group. Examples include piperidinylcarbonyl, tetrahydrofurylcarbonyl and the like.

The term "non-aromatic heterocyclyloxycarbonyl" means a group wherein the above "non-aromatic heterocyclyloxy" is bonded to a carbonyl group. Examples include piperidinyloxycarbonyl, tetrahydrofuryloxycarbonyl and the like.

The term "non-aromatic heterocyclylsulfanyl" means a group wherein "non-aromatic heterocycle" is replaced with a hydrogen atom bonded to a sulfur atom of a sulfanyl group. Examples include piperidinylsulfanyl, tetrahydrofurylsulfanyl and the like.

The term "non-aromatic heterocyclylsulfonyl" means a group wherein "non-aromatic heterocycle" is bonded to a sulfonyl group. Examples include piperidinylsulfonyl, tetrahydrofurylsulfonyl and the like.

Examples of the substituents for "substituted or unsubstituted alkyl", "substituted or unsubstituted alkenyl", "substituted or unsubstituted alkynyl", "substituted or unsubstituted alkylcarbonyl", "substituted or unsubstituted alkenylcarbonyl", "substituted or unsubstituted alkynylcarbonyl", "substituted or unsubstituted monoalkylamino", "substituted or unsubstituted dialkylamino", "substituted or unsubstituted monoalkylcarbonylamino", "substituted or unsubstituted dialkylcarbonylamino", "substituted or unsubstituted monoalkylsulfonylamino", "substituted or unsubstituted dialkylsulfonylamino", "substituted or unsubstituted monoalkylcarbamoyl", "substituted or unsubstituted dialkylcarbamoyl", "substituted or unsubstituted monoalkylsulfamoyl", "substituted or unsubstituted dialkylsulfamoyl", "substituted or unsubstituted alkyloxy", "substituted or unsubstituted alkenyloxy", "substituted or unsubstituted alkynyloxy", "substituted or unsubstituted alkylsulfonyl", "substituted or unsubstituted alkenylsulfonyl", "substituted or unsubstituted alkynylsulfonyl", "substituted or unsubstituted alkylimino", "substituted or unsubstituted alkenylimino", "substituted or unsubstituted alkynylimino", "substituted or unsubstituted alkylcarbonylimino", "substituted or unsubstituted alkenylcarbonylimino", "substituted or unsubstituted alkynylcarbonylimino", "substituted or unsubstituted alkyloxyimino", "substituted or unsubstituted alkenyloxyimino", "substituted or unsubstituted alkynyloxyimino", "substituted or unsubstituted alkylcarbonyloxy", "substituted or unsubstituted alkenylcarbonyloxy", "substituted or unsubstituted alkynylcarbonyloxy", "substituted or unsubstituted alkyloxycarbonyl", "substituted or unsubstituted alkenyloxycarbonyl", "substituted or unsubstituted alkynyloxycarbonyl", "substituted or unsubstituted alkylsulfanyl", "substituted or unsubstituted alkenylsulfanyl", "substituted or unsubstituted alkynylsulfanyl", "substituted or unsubstituted alkylsulfinyl", "substituted or unsubstituted alkenylsulfinyl", "substituted or unsubstituted alkynylsulfinyl", "substituted or unsubstituted aminocarbonyloxyalkyl", "substituted or unsubstituted alkylene", "substituted or unsubstituted alkenylene", "substituted or unsubstituted alkynylene", and "substituted or unsubstituted imino which R¹⁰ and R¹¹ connected to the same carbon atom are taken together with the said carbon atom to form" include the following substituents. A carbon atom(s) at any position(s) may be substituted with one or more group(s) selected from the following substituents.

Substituents: halogen, hydroxy, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, guanidino, trialkylsilyl, alkyloxy, alkenyloxy, alkynyloxy, haloalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, alkenyloxyimino, alkynyloxyimino, alkylcarbonyloxy, alkenylcarbonyloxy, alkynylcarbonyloxy, alkyloxycarbonyl, alkenyloxycarbonyl, alkynyloxycarbonyl, alkylsulfanyl, alkenylsulfanyl, alkynylsulfanyl, alkylsulfinyl, alkenylsulfinyl, alkynylsulfinyl, monoalkylcarbamoyl, dialkylcarbamoyl, monoalkylsulfamoyl, dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylalkyloxy, substituted or unsubstituted non-aromatic carbocyclylalkyloxy, substituted or unsubstituted aromatic heterocyclylalkyloxy, substituted or unsubstituted non-aromatic heterocyclylalkyloxy, substituted or unsubstituted aromatic carbocyclylalkyloxycarbonyl, substituted or unsubstituted non-aromatic carbocyclylalkyloxycarbonyl, substituted or unsubstituted aromatic heterocyclylalkyloxycarbonyl, substituted or unsubstituted non-aromatic heterocyclylalkyloxycarbonyl, substituted or unsubstituted aromatic carbocyclylalkylamino, substituted or unsubstituted non-aromatic carbocyclylalkylamino, substituted or unsubstituted aromatic heterocyclylalkylamino, substituted or unsubstituted non-aromatic heterocyclylalkylamino, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted non-aromatic carbocyclylsulfonyl, substituted or unsubstituted aromatic carbocyclylsulfonyl, substituted or unsubstituted aromatic heterocyclylsulfonyl, and substituted or unsubstituted non-aromatic heterocyclylsulfonyl.

Examples of the substituents on the ring of "substituted aromatic carbocyclyl", "substituted non-aromatic carbocyclyl", "substituted aromatic heterocyclyl", "substituted non-aromatic heterocyclyl", "substituted or unsubstituted aromatic carbocyclyl", "substituted or unsubstituted non-aromatic carbocyclyl", "substituted or unsubstituted aromatic heterocyclyl", "substituted or unsubstituted non-aromatic heterocyclyl", "substituted or unsubstituted aromatic carbocyclyl", "substituted or unsubstituted non-aromatic carbocyclyl", "substituted or unsubstituted aromatic heterocyclyl", "substituted or unsubstituted non-aromatic heterocyclyl", "substituted or unsubstituted aromatic carbocyclylalkyl", "substituted or unsubstituted non-aromatic carbocyclylalkyl", "substituted or unsubstituted aromatic heterocyclylalkyl", "substituted or unsubstituted non-aromatic heterocyclylalkyl", "substituted or unsubstituted aromatic carbocyclyloxy", "substituted or unsubstituted non-aromatic carbocyclyloxy", "substituted or unsubstituted aromatic heterocyclyloxy", "substituted or unsubstituted non-aromatic heterocyclyloxy", "substituted or unsubstituted aromatic carbocyclylcarbonyl", "substituted or unsubstituted non-aromatic carbocyclylcarbonyl", "substituted or unsubstituted aromatic heterocyclylcarbonyl", "substituted or unsubstituted non-aromatic heterocyclylcarbonyl", "substituted or unsubstituted aromatic carbocyclyloxycarbonyl", "substituted or unsubstituted non-aromatic carbocyclyloxycarbonyl", "substituted or unsubstituted aromatic heterocyclyloxycarbonyl", "substituted or unsubstituted non-aromatic heterocyclyloxycarbonyl", "substituted or unsubstituted aromatic carbocyclylsulfanyl", "substituted or unsubstituted non-aromatic carbocyclylsulfanyl", "substituted or unsubstituted aromatic heterocyclylsulfanyl", "substituted or unsubstituted non-aromatic heterocyclylsulfanyl", "substituted or unsubstituted aromatic carbocyclylsulfonyl", "substituted or unsubstituted non-aromatic carbocyclylsulfonyl", "substituted or unsubstituted aromatic heterocyclylsulfonyl", "substituted or unsubstituted non-aromatic heterocyclylsulfonyl"., "substituted or unsubstituted aromatic carbocyclyldiyl", "substituted or unsubstituted non-aromatic carbocyclyldiyl", "substituted or unsubstituted aromatic heterocyclyldiyl", "substituted or unsubstituted non-aromatic heterocyclyldiyl", "substituted or unsubstituted phenyl", "substituted or unsubstituted bicyclic aromatic heterocyclyl", "substituted or unsubstituted aromatic carbocycle which two R¹² connected to the adjacent carbon atoms constituting the ring are taken together to form", "substituted or unsubstituted non-aromatic carbocycle which two R¹² connected to the adjacent carbon atoms constituting the ring are taken together to form", "substituted or unsubstituted aromatic heterocycle which two R¹² connected to the adjacent carbon atoms constituting the ring are taken together to form", "substituted or unsubstituted non-aromatic heterocycle which two R¹² connected to the adjacent carbon atoms constituting the ring are taken together to form", "substituted or unsubstituted ring which two R²⁹ connected to the adjacent carbon atoms are taken together to form", "substituted or unsubstituted bridge structure which two R²⁹ connected to the non-adjacent and different carbon atoms are taken together to form", "substituted or unsubstituted alkylene which two R²⁹ connected to the non-adjacent and different carbon atoms are taken together to form", "substituted or unsubstituted spiro ring which two R²⁹ connected to the same carbon atom are taken together to form", "substituted or unsubstituted benzene ring", "substituted or unsubstituted bicyclic non-aromatic carbocyclyl", and "substituted or unsubstituted bicyclic non-aromatic heterocyclyl" include the following substituents. An atom at any position(s) on the ring may be substituted with to one or more group(s) selected from the following substituents.

Substituents: halogen, hydroxy, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, guanidino, trialkylsilyl, alkyl, alkenyl, alkynyl, haloalkyl, alkyloxy, alkenyloxy, alkynyloxy, haloalkyloxy, alkyloxyalkyl, alkyloxyalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, alkenyloxyimino, alkynyloxyimino, alkylcarbonyloxy, alkenylcarbonyloxy, alkynylcarbonyloxy, alkyloxycarbonyl, alkenyloxycarbonyl, alkynyloxycarbonyl, alkylsulfanyl, alkenylsulfanyl, alkynylsulfanyl, alkylsulfinyl, alkenylsulfinyl, alkynylsulfinyl, monoalkylcarbamoyl, dialkylcarbamoyl, monoalkylsulfamoyl, dialkylsulfamoyl, aromatic carbocyclyl optionally substituted with Substituent group A, non-aromatic carbocyclyl optionally substituted with Substituent group A, aromatic heterocyclyl optionally substituted with Substituent group A, non-aromatic heterocyclyl optionally substituted with Substituent group A, aromatic carbocyclyloxy optionally substituted with Substituent group A, non-aromatic carbocyclyloxy optionally substituted with Substituent group A, aromatic heterocyclyloxy optionally substituted with Substituent group A, non-aromatic heterocyclyloxy optionally substituted with Substituent group A, aromatic carbocyclylcarbonyl optionally substituted with Substituent group A, non-aromatic carbocyclylcarbonyl optionally substituted with Substituent group A, aromatic heterocyclylcarbonyl optionally substituted with Substituent group A, non-aromatic heterocyclylcarbonyl optionally substituted with Substituent group A, aromatic carbocyclyloxycarbonyl optionally substituted with Substituent group A, non-aromatic carbocyclyloxycarbonyl optionally substituted with Substituent group A, aromatic heterocyclyloxycarbonyl optionally substituted with Substituent group A, non-aromatic heterocyclyloxycarbonyl optionally substituted with Substituent group A, aromatic carbocyclylalkyl optionally substituted with Substituent group A, non-aromatic carbocyclylalkyl optionally substituted with Substituent group A, aromatic heterocyclylalkyl optionally substituted with Substituent group A, non-aromatic heterocyclylalkyl optionally substituted with Substituent group A, aromatic carbocyclylalkyloxy optionally substituted with Substituent group A, non-aromatic carbocyclylalkyloxy optionally substituted with Substituent group A, aromatic heterocyclylalkyloxy optionally substituted with Substituent group A, non-aromatic heterocyclylalkyloxy optionally substituted with Substituent group A, aromatic carbocyclylalkyloxycarbonyl optionally substituted with Substituent group A, non-aromatic carbocyclylalkyloxycarbonyl optionally substituted with Substituent group A, aromatic heterocyclylalkyloxycarbonyl optionally substituted with Substituent group A, non-aromatic heterocyclylalkyloxycarbonyl optionally substituted with Substituent group A, aromatic carbocyclylalkyloxyalkyl optionally substituted with Substituent group A, non-aromatic carbocyclylalkyloxyalkyl optionally substituted with Substituent group A, aromatic heterocyclylalkyloxyalkyl optionally substituted with Substituent group A, non-aromatic heterocyclylalkyloxyalkyl optionally substituted with Substituent group A, aromatic carbocyclylalkylamino optionally substituted with Substituent group A, non-aromatic carbocyclylalkylamino optionally substituted with Substituent group A, aromatic heterocyclylalkylamino optionally substituted with Substituent group A, non-aromatic heterocyclylalkylamino optionally substituted with Substituent group A, aromatic carbocyclylsulfanyl optionally substituted with Substituent group A, non-aromatic carbocyclylsulfanyl optionally substituted with Substituent group A, aromatic heterocyclylsulfanyl optionally substituted with Substituent group A, non-aromatic heterocyclylsulfanyl optionally substituted with Substituent group A, non-aromatic carbocyclylsulfonyl optionally substituted with Substituent group A, aromatic carbocyclylsulfonyl optionally substituted with Substituent group A, aromatic heterocyclylsulfonyl optionally substituted with Substituent group A, and non-aromatic heterocyclylsulfonyl optionally substituted with Substituent group A.

Substituent group A: halogen, hydroxy, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, guanidino, trialkylsilyl, alkyl, alkenyl, alkynyl, haloalkyl, alkyloxy, alkenyloxy, alkynyloxy, haloalkyloxy, alkyloxyalkyl, alkyloxyalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, alkenyloxyimino, alkynyloxyimino, alkylcarbonyloxy, alkenylcarbonyloxy, alkynylcarbonyloxy, alkyloxycarbonyl, alkenyloxycarbonyl, alkynyloxycarbonyl, alkylsulfanyl, alkenylsulfanyl, alkynylsulfanyl, alkylsulfinyl, alkenylsulfinyl, alkynylsulfinyl, monoalkylcarbamoyl, dialkylcarbamoyl, monoalkylsulfamoyl, and dialkylsulfamoyl.

"optionally substituted with Substituent group A" means that one or more same or different group(s) selected from Substituent group A may substitute. As one embodiment, 1 to 6 same or different group(s) selected from Substituent group A may substitute. As the other embodiment, 1 to 3 same or different group(s) selected from Substituent group A may substitute.

Additionally, "substituted or unsubstituted non-aromatic carbocyclyl" and "substituted or unsubstituted non-aromatic heterocyclyl" may be substituted with "oxo". In this case, it means a group wherein two hydrogen atoms on the same carbon atom are substituted as below.

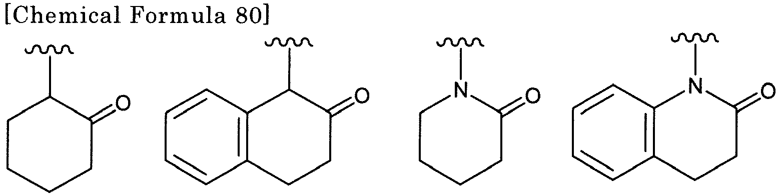

The non-aromatic carbocycle or non-aromatic heterocycle part of the above "substituted or unsubstituted non-aromatic carbocyclyloxy", "substituted or unsubstituted non-aromatic heterocyclyloxy", "substituted or unsubstituted non-aromatic carbocyclylcarbonyl", "substituted or unsubstituted non-aromatic heterocyclylcarbonyl", "substituted or unsubstituted non-aromatic carbocyclyloxycarbonyl", "substituted or unsubstituted non-aromatic heterocyclyloxycarbonyl", "substituted or unsubstituted non-aromatic carbocyclylsulfanyl", "substituted or unsubstituted non-aromatic heterocyclylsulfanyl", "substituted or unsubstituted non-aromatic carbocyclylsulfonyl" or and "substituted or unsubstituted non-aromatic heterocyclylsulfonyl" may be substituted with "oxo" as above.

"Substituted or unsubstituted amino" includes amino optionally substituted with one or two group(s) selected from the following substituents.

Substituents: hydroxy, cyano, alkyl, alkenyl, alkynyl, haloalkyl, haloalkenyl, haloalkynyl, alkyloxy, alkenyloxy, alkynyloxy, haloalkyloxy, haloalkenyloxy, haloalkynyloxy, alkenylcarbonyl, alkynylcarbonyl, haloalkylcarbonyl, haloalkenylcarbonyl, haloalkynylcarbonyl, alkylsulfonyl, haloalkylsulfonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclylcarbamoyl, substituted or unsubstituted non-aromatic carbocyclylcarbamoyl, substituted or unsubstituted aromatic heterocyclylcarbamoyl, and substituted or unsubstituted non-aromatic heterocyclylcarbamoyl

An embodiment of "substituted or unsubstituted amino" is amino, methylamino, dimethylamino, ethylamino, diethylamino, ethylmethylamino, cyclopropylamino, cyclohexylamino, benzylamino, acetylamino, benzoylamino, methylsulfonylamino, tetrahydropyranylamino, tetrahydrofuranylamino, morpholinoamino, morpholinylamino, piperidinylamino, piperazinylamino and the like. Other embodiment is amino, methylamino, dimethylamino, ethylmethylamino, diethylamino, acetylamino, methylsulfonylamino, tetrahydropyranylamino, tetrahydrofuranylamino, morpholinoamino, piperidinylamino and the like.

"Substituted or unsubstituted imino" includes imino optionally substituted with one group selected from the following substituents. Substituents: hydroxy, alkyl, alkenyl, alkynyl, haloalkyl, haloalkenyl, haloalkynyl, alkyloxy, alkenyloxy, alkynyloxy, haloalkyloxy, haloalkenyloxy, haloalkynyloxy, alkylcarbonyl, alkenylcarbonyl, alkynylcarbonyl, haloalkylcarbonyl, haloalkenylcarbonyl, haloalkynylcarbonyl, amino, alkylamino, haloalkylamino, substituted or unsubstituted aromatic carbocyclyl, substituted or unsubstituted non-aromatic carbocyclyl, substituted or unsubstituted aromatic heterocyclyl, and substituted or unsubstituted non-aromatic heterocyclyl

An embodiment of "substituted or unsubstituted imino" is imino, methylimino, ethylimino, cyclopropylimino, cyclohexylimino, acetylimino, tetrahydropyranylimino, tetrahydrofuranylimino, morpholinoimino, morpholinylimino, piperidinylimino, piperazinylimino and the like.

"Substituted or unsubstituted carbamoyl" includes carbamoyl optionally substituted with one or two group(s) selected from the following substituents.

Substituents: hydroxy, cyano, amino, alkyl, alkenyl, alkynyl, haloalkyl, haloalkenyl, haloalkynyl, hydroxyalkyl, alkylamino, alkylcarbonyl, alkenylcarbonyl, alkynylcarbonyl, alkylsulfonyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylalkyl, substituted or unsubstituted non-aromatic carbocyclylalkyl, substituted or unsubstituted aromatic heterocyclylalkyl, and substituted or unsubstituted non-aromatic heterocyclylalkyl_{∘},

An embodiment of "substituted or unsubstituted carbamoyl" is carbamoyl, N-methylcarbamoyl, N,N-dimethylcarbamoyl, N-ethyl-N-methylcarbamoyl, N,N-diethylcarbamoyl, N-n-propylaminocarbamoyl, N-isopropylcarbamoyl, N-morpholinocarbamoyl, N-tetrahydrofuranylcarbamoyl, N-piperidylcarbamoyl, N-tetrahydropyranylcarbamoyl, N-benzylcarbamoyl, N-acetylcarbamoyl, N-methylsulfonylcarbamoyl, N-(2,2,2-trifluoroethyl)carbamoyl, N-(2-hydroxy-1-methylethyl)carbamoyl and the like. Other embodiment is carbamoyl, N-methylcarbamoyl, N,N-dimethylcarbamoyl, N-n-propylaminocarbamoyl, N-isopropylcarbamoyl, N-morpholinocarbamoyl, N-tetrahydrofuranylcarbamoyl, N-piperidylcarbamoyl, N-tetrahydropyranylcarbamoyl, N-methylsulfonylcarbamoyl, N-(2,2,2-trifluoroethyl)carbamoyl, N-(2-hydroxy-1-methylethyl)carbamoyl and the like.

"Substituted or unsubstituted sulfamoyl" includes aminosulfonyl optionally substituted with one or two group(s) selected from the following substituents.

Substituents: alkyl, alkenyl, alkynyl, haloalkyl, haloalkenyl, haloalkynyl, hydroxyalkyl, alkylcarbonyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylalkyl, substituted or unsubstituted non-aromatic carbocyclylalkyl, substituted or unsubstituted aromatic heterocyclylalkyl, and substituted or unsubstituted non-aromatic heterocyclylalkyl_{∘}

An embodiment of "substituted or unsubstituted sulfamoyl" is sulfamoyl, N-methylsulfamoyl, N,N-dimethylsulfamoyl, N-ethyl-N-methylsulfamoyl, N,N-diethylsulfamoyl, N-n-propylaminosulfamoyl, N-isopropylsulfamoyl, N-morpholinosulfamoyl, N-tetrahydrofuranylsulfamoyl, N-piperidylsulfamoyl, N-tetrahydropyranylsulfamoyl, N-benzylsulfamoyl, N-acetylsulfamoyl, N-methylsulfonylsulfamoyl and the like. The other embodiment is sulfamoyl, N-methylsulfamoyl, N,N-dimethylsulfamoyl, N-n-propylaminosulfamoyl, N-isopropylsulfamoyl, N-morpholinosulfamoyl, N-tetrahydrofuranylsulfamoyl, N-piperidylsulfamoyl, N-tetrahydropyranylsulfamoyl, N-methylsulfonylsulfamoyl and the like.

"active ingredient" means a compound having medical activities, whose half-life time in pharmacokinetics is 0 to 10 hours or clearance is 1 to 100, preferably half-life time is 0 to 5 hours or clearance is 10 to 100.

"indroduce into active ingredient" means introducing substituent(s) into position(s) without disappearing activity of active ingredient.

The present invention can extend half-life time in pharmacokinetics of active ingredient and/or decrease clearance. Half-life time can be extended or clearance can be decrease to compare after with before introducing introducing the group represented by the following formula: 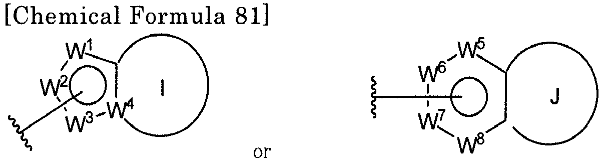 wherein each symbol is defined as above item (35).

"protecting group for hydroxy group" includes benzyl group, p-methoxyphenylbenzyl group, acetyl group, formyl group, benzoyl group, chloroacetyl group, pivaloyl group, methyl carbonate group, isobutyl carbonate group, benzyl carbonate group, vinyl carbonate group, phenyl carbamate group, mesyl group, tosyl group, trimethylsilyl group, triethylsilyl group, t-butyldimethylsilyl group, methoxymethyl group, benzyloxymethyl group, methoxyethoxymethyl group, 2-(trimethylsilyl)ethoxymethyl group, propenyl group, phenacyl group, tetrahydropyranyl group and the like.

"protecting group for amino group" includes t-butyldimethylsilyl group, t-butoxycarbonyl group, benzyloxycarbonyl group, allyloxycarbonyl group, allyl group, 9-fluorenylmethyloxycarbonyl group, benzyl group, p-methoxybenzyl group, p-toluenesulfonyl group, 2-nitrobenzenesulfonyl group, methoxymethyl group, benzyloxymethyl group, benzhydryl group, trityl group and the like.

"protecting group for carboxy group" includes substituted or unsubstituted alkyl, substituted or unsubstituted alkenyl, substituted or unsubstituted aromatic carbocyclylalkyl and the like. For example, methyl, ethyl, allyl, t-butyl, benzyl and p-methoxybenzyl are exemplified.

"substituted or unsubstituted" in ring B means that ring B may be substituted with further substituent(s) at any position other than R² position.

"substituted or unsubstituted" in ring C means that ring C may be substituted with further substituent(s) at any position other than R³ position.

"substituted or unsubstituted" in ring D, E, F, G, H, I, J, K, L, M, N, P, Q means that each ring may be substituted with further substituent(s) listed as substituted or unsubstituted groups.

Preferred embodiments of the compound of the present invention are disclosed below.

Ring A is

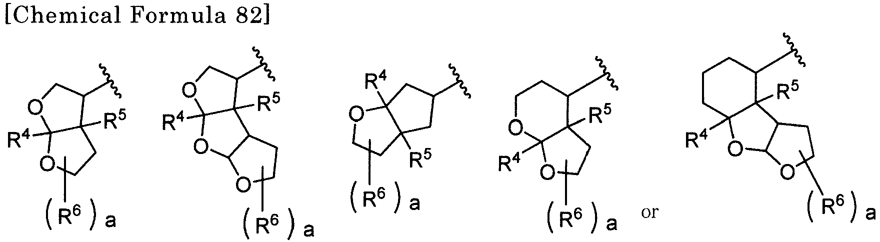

R⁴ are each independently a group represented by formula: -Y-Z, halogen, hydroxy, carboxy, amino, carbamoyl, sulfamoyl, cyano, nitro, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted monoalkylcarbonylamino, substituted or unsubstituted dialkylcarbonylamino, substituted or unsubstituted monoalkylsulfonylamino, substituted or unsubstituted dialkylsulfonylamino, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

The other embodiment of R⁴ is a group represented by formula: -Y-Z, halogen, hydroxy, carboxy, cyano, nitro, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted amino, substituted or unsubstituted carbamoyl, substituted or unsubstituted sulfamoyl, substituted or unsubstituted aminocarbonyloxyalkyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R⁴ is preferably substituted or unsubstituted alkyl, or substituted or unsubstituted aminocarbonyloxyalkyl.

As substituents for R⁴, halogen, hydroxy, amino, aromatic carbocyclylalkyl substituted with halogen and the like are exemplified. Further preferable substituents are alkyl.

R⁵ are each independently hydrogen atom, halogen, hydroxy, carboxy, amino, carbamoyl, sulfamoyl, cyano, nitro, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl.

The other embodiment of R⁵ is hydrogen atom, halogen, hydroxy, carboxy, cyano, nitro, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted carbamoyl, or substituted or unsubstituted sulfamoyl.

R⁵ is preferably hydrogen atom, or hydroxy.

R⁶ are each independently halogen, hydroxy, carboxy, amino, imino, hydroxyamino, hydroxyimino, formyl, formyloxy, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

Ring A may be substituted with said R⁶ at any substitutable position(s).

The other embodiment of R⁶ is halogen, hydroxy, carboxy, formyl, formyloxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R⁶ is preferably hydroxy, substituted or unsubstituted alkyloxy.

As substituents for R⁶, halogen, hydroxy and the like are exemplified.

a is an integer of 0 to 7, preferably an integer of 0 to 3, further preferably 0.

Ring B is substituted or unsubstituted aromatic carbocyclyl, or substituted or unsubstituted aromatic heterocyclyl.

Ring B is preferably substituted or unsubstituted phenyl or substituted or unsubstituted pyridine, more preferablysubstituted or unsubstituted phenyl.

As substituents for ring B, halogen, alkyl, haloalkyl, alkyloxy, haloalkyloxy and the like are exemplified.

Ring C is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

Ring C is preferably substituted or unsubstituted aromatic carbocyclyl or substituted or unsubstituted bicyclic aromatic heterocyclyl.

As substituents for ring C, hydroxy, amino, alkyloxy, haloalkyloxy, alkylamino and the like are exemplified.

R¹ is a group represented by formula: -Y-Z, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy.

R² and R³ are each independently a group represented by formula: -Y-Z, or hydrogen atom, provided that at least one of R¹, R², R³ and R⁴ is a group represented by formula: -Y-Z.

More preferably, R² is a group represented by formula: -Y-Z.

Y is each independently a bond, or a spacer of any combination selected from the group consisting of -O-, -S-, -NR⁷-, -C(=O)-, -SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl.

The other embodiment of Y is a bond, or a spacer of any combination selected from the group consisting of -O-, -S-, -NR⁷-, -C(=O)-, -SO-, -SO₂-, -NR⁷-C(=O)-,-C(=O)-NR⁷ -, -NR⁷ -C(=O)-NR⁷ -, -O-C(=O)-NR⁷-, -NR⁷-C(=O)-O-, -SO₂ -NR⁷-, -NR⁷-SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl,
provided that the groups selected from the group consisting of -O-, -S- and - NR⁷- are not connected adjacently in Y, and
provided that the groups selected from the group consisting of -C(=O)-, -SO-,-SO₂-, -NR⁷-C(=O)-, -C(=O)-NR⁷-, -NR⁷-C(=O)-NR⁷-, -O-C(=O)-NR⁷- -, NR⁷-C(=O)-O-,-SO₂ -NR⁷- and -NR⁷ -SO₂- are not connected adjacently in Y.

R⁷ are each independently hydrogen atom, hydroxy, carboxy, amino, carbamoyl, sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy.

The other embodiment of R⁷ is hydrogen atom, hydroxy, carboxy, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, substituted or unsubstituted non-aromatic heterocyclylsulfonyl.

R⁷ are preferably each independently hydrogen atom, substituted or unsubstituted alkyl, substituted or unsubstituted alkylcarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, substituted or unsubstituted non-aromatic heterocyclylsulfonyl.

R⁷ are further preferably, each independently hydrogen atom, substituted or unsubstituted alkyl, substituted or unsubstituted alkylcarbonyl, substituted or unsubstituted aromatic carbocyclylalkyl, substituted or unsubstituted non-aromatic carbocyclylalkyl, substituted or unsubstituted aromatic heterocyclylalkyl, or substituted or unsubstituted non-aromatic heterocyclylalkyl.

As substituents for R⁷, halogen, alkyloxy, haloalkyloxy and the like are exemplified.

R⁷ are more preferably, each independently hydrogen atom, alkyl, haloalkyl.

Z are each independently aromatic carbocyclyl having acid group, non-aromatic carbocyclyl having acid group, aromatic heterocyclyl having acid group or non-aromatic heterocyclyl having acid group, which function as an affinity group to protein.

The other embodiment of Z is substituted aromatic carbocyclyl, substituted non-aromatic carbocyclyl, substituted aromatic heterocyclyl, or substituted non-aromatic heterocyclyl.

When R¹ is a group represented by formula: -Y-Z, Y is preferably, 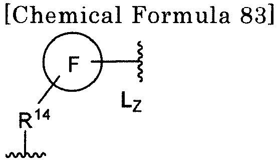 wherein a bond Lz is connecting to Z.

When R¹ is a group represented by formula: -Y-Z, Y is further preferably, 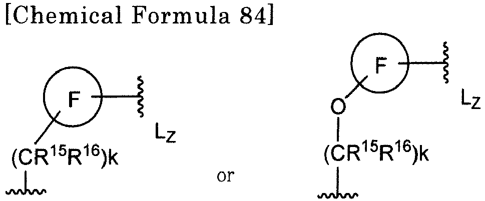 wherein a bond Lz is connecting to Z.

When R¹ is a group represented by formula: -Y-Z, Y is especially preferably, 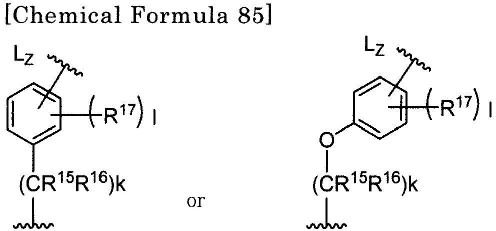 wherein a bond Lz is connecting to Z.

Ring F is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

As preertable substituents for ring F, halogen, carboxy, hydroxy, cyano, nitro, alkyl, haloalkyl, alkyloxy, haloalkyloxy, alkylamino, dialkylamino, alkyloxycarbonyl, alkylaminocarbonyl, dialkylaminocarbonyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy and the like are exemplified.

R¹⁴ is substituted or unsubstituted alkylene which may be intervened with one or more groups selected from the group consisting of -O-, -NR⁷ -, -C(=O)-NR⁷- and-NR⁷-C(=O)-,
substituted or unsubstituted alkenylene which may be intervened with one or more groups selected from the group consisting of -O-, -NR⁷ -, -C(=O)-NR⁷ - and -NR⁷-C(=O)-, or
substituted or unsubstituted alkynylene which may be intervened with one or more groups selected from the group consisting of -O-, -NR⁷ -, -C(=O)-NR⁷- and -NR⁷-C(=O)-,
provided that the groups selected from the group consisting of -O-, -NR⁷ -, -C(=O)-NR⁷- and -NR⁷ -C(=O)- are not connected adjacently in R¹⁴.

R¹⁵ and R¹⁶ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The other embodiment of R¹⁵ and R¹⁶ is hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R¹⁵ and R¹⁶ are preferably hydrogen atom.

k is an integer of 0 to 4, preferably an integer of 1 to 3.

R¹⁷ are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The other embodiment of R¹⁷ is halogen, hydroxy, carboxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R¹⁷ are preferably each independently substituted or unsubstituted alkyl, more preferably alkyl or haloalkyl.

1 is an integer of 0 to 4.

When R² is a group represented by formula: -Y-Z, Y is preferably a bond, 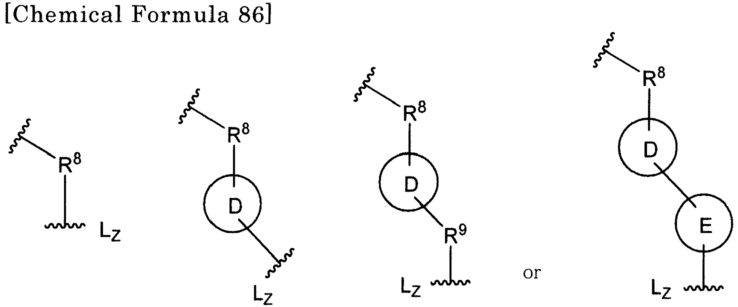 wherein a bond Lz is connecting to Z.

When R² is a group represented by formula: -Y-Z, Y is further preferably a bond, 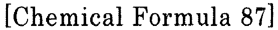 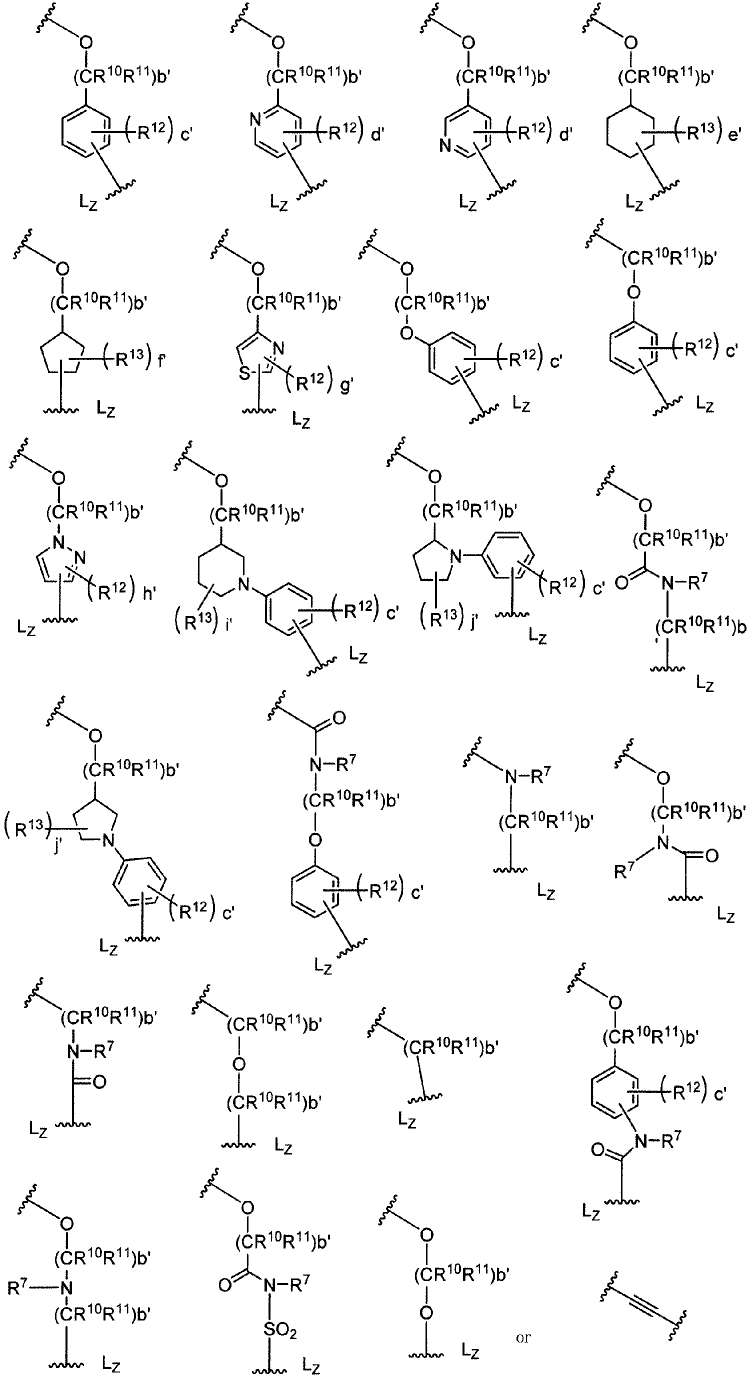 wherein a bond Lz is connecting to Z.

When R² is a group represented by formula: -Y-Z, Y is further preferably a bond, 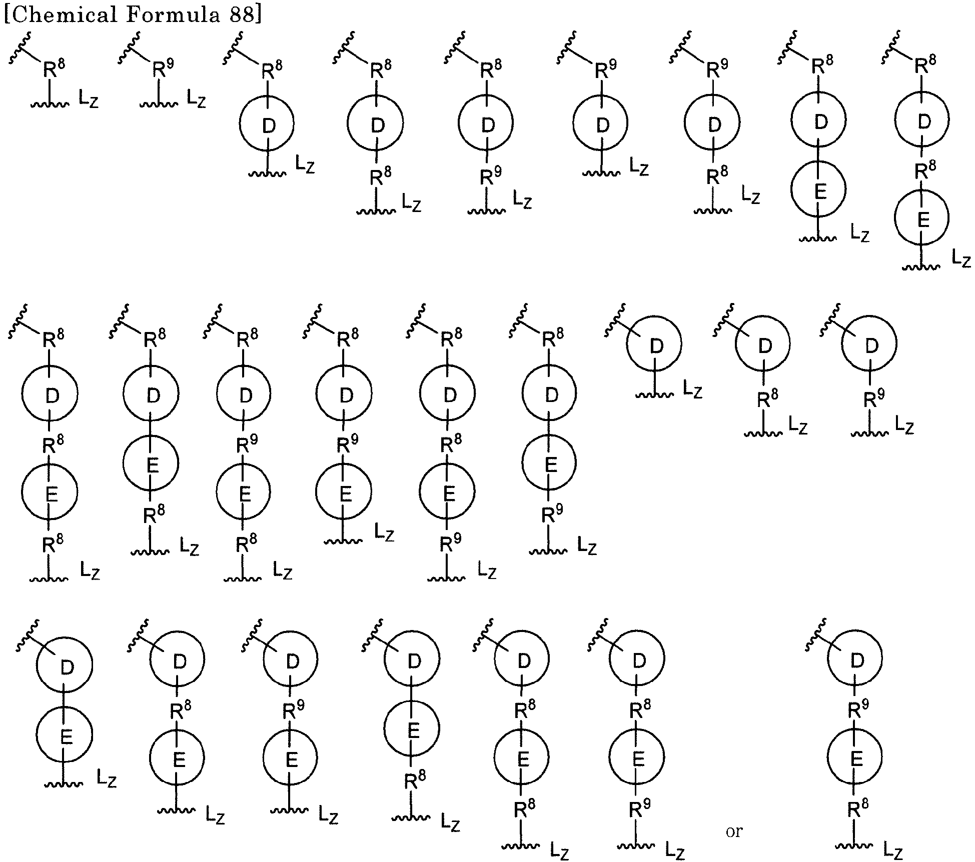 wherein a bond Lz is connecting to Z.

When R² is a group represented by formula: -Y-Z, Y is further preferably 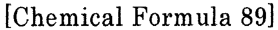 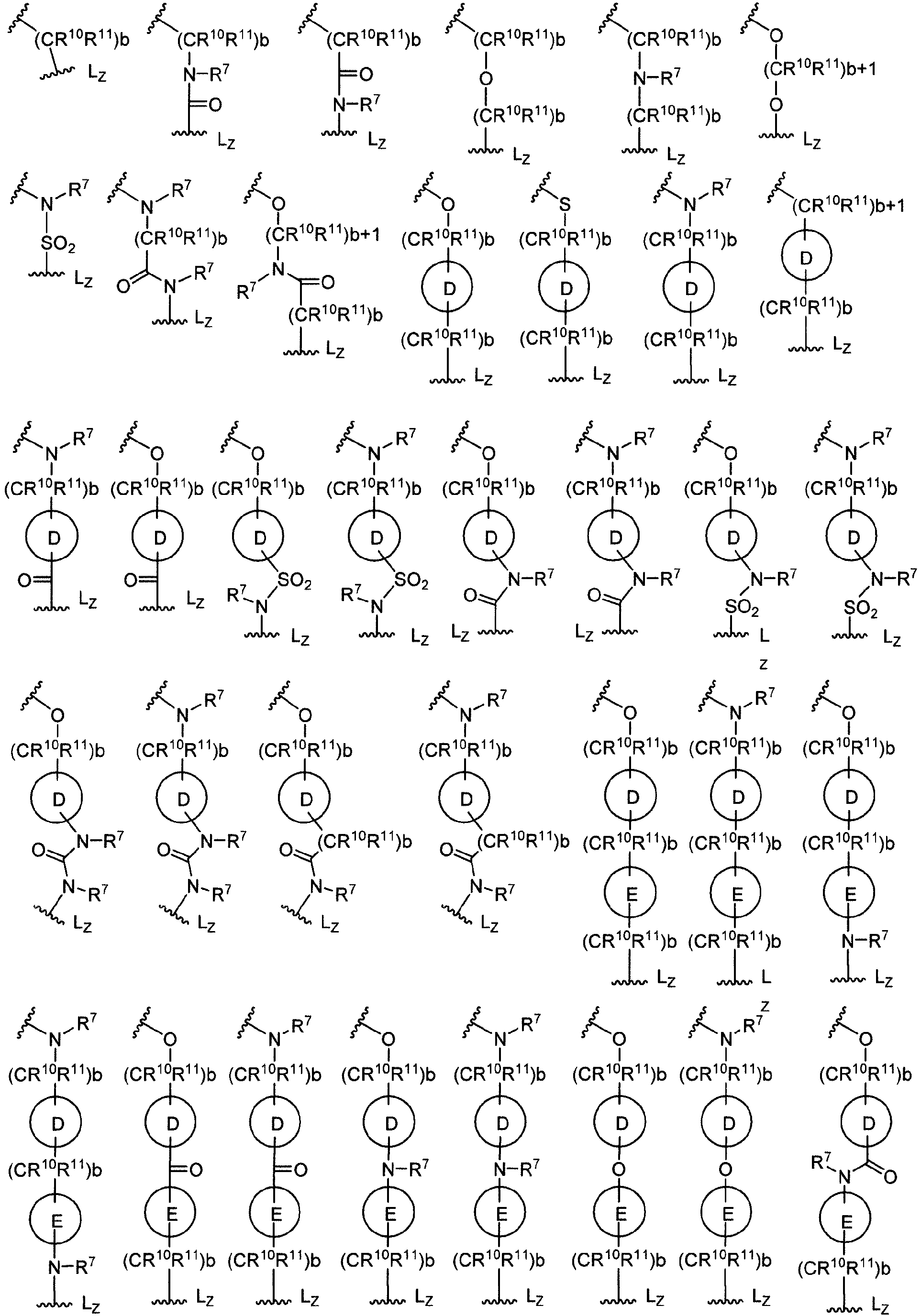 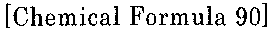 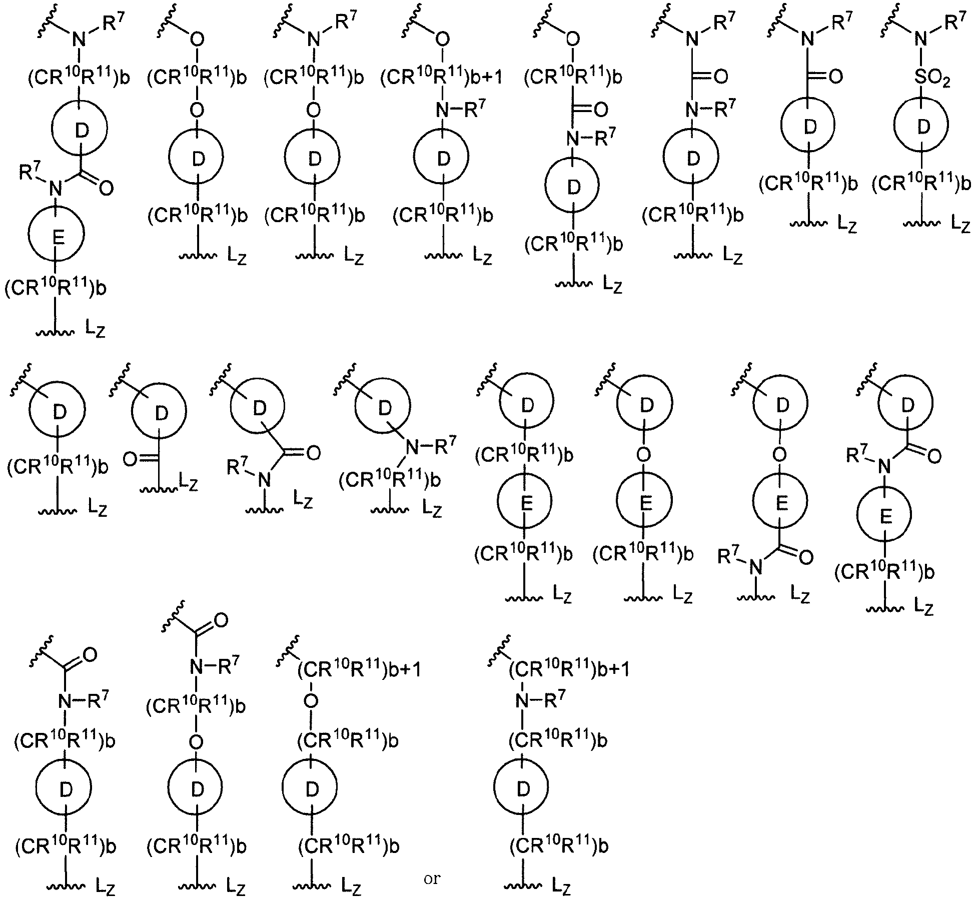 wherein a bond Lz is connecting to Z.

When R² is a group represented by formula: -Y-Z, Y is further preferably 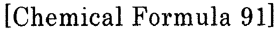 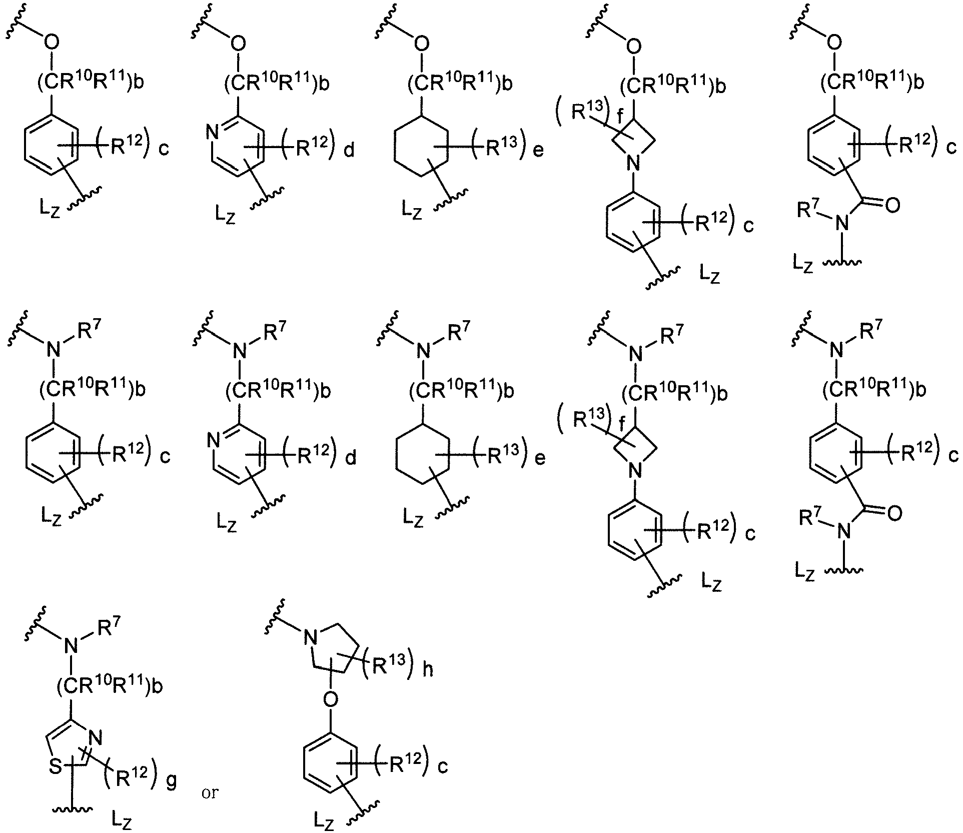 wherein a bond Lz is connecting to Z.

R⁸ are each independently -O-, -NR⁷ -, substituted or unsubstituted alkylene which may be intervened with one or more groups selected from the group consisting -NR⁷-, -C(=O)-NR⁷- and -NR⁷-C(=O)-,
substituted or unsubstituted alkenylene which may be intervened with one or more groups selected from the group consisting of -O-, -NR⁷ -, -C(=O)-NR⁷- and -NR⁷-C(=O)-, or
substituted or unsubstituted alkynylene which may be intervened with one or more groups selected from the group consisting of -O-, -NR⁷ -, -C(=O)-NR⁷- and -NR⁷-C(=O)-.

The other embodiment of R⁸ is -O-, -S-, -NR⁷-, substituted or unsubstituted alkylene which may be intervened with one or more groups selected from the group consisting -NR⁷-, -C(=O)-NR⁷- and -NR⁷-C(=O)-,
substituted or unsubstituted alkenylene which may be intervened with one or more groups selected from the group consisting of -O-, -NR⁷ -, -C(=O)-NR⁷- and -NR⁷-C(=O)-, or
substituted or unsubstituted alkynylene which may be intervened with one or more groups selected from the group consisting of -O-, -NR⁷ -, -C(=O)-NR⁷- and -NR⁷-C(=O)-,
provided that the groups selected from the group consisting of -O-, -NR⁷ -,-C(=O)-NR⁷- and -NR⁷ -C(=O)- are not connected adjacently in R⁸.

Ring D and ring E are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

As preferable substituents for ring D or ring E, halogen, carboxy, hydroxy, cyano, nitro, alkyl, haloalkyl, alkyloxy, haloalkyloxy, alkylamino, dialkylamino, alkyloxycarbonyl, alkylaminocarbonyl, dialkylaminocarbonyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy and the like are exemplified.

R⁹ is -C(=O)-NR⁷ -, or -NR⁷-C(=O)-.

The other embodiment of R⁹ is -C(=O)-, -C(=O)-NR⁷ -, -NR⁷-C(=O)-, -NR⁷-C(=O)-NR⁷ -, -NR⁷SO₂-, -SO₂ NR⁷-.

R¹ and R¹¹ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,

R¹⁰ and R¹¹ connected to the same carbon atom may be taken together with the said carbon atom to form substituted or unsubstituted imino.

The other embodiment of R¹⁰ and R¹¹ is hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R¹⁰ and R¹ connected to the same carbon atom may be taken together with the said carbon atom to form substituted or unsubstituted imino, substituted or unsubstituted non-aromatic carbocycle, or non-aromatic heterocycle.

The two R¹⁰ and/or R¹¹ connected to the adjacent carbon atoms may be taken together to form a bond.

R¹⁰ and R¹¹ are preferably each independently hydrogen atom, substituted or unsubstituted alkyl.

R¹⁰ and R¹¹ connected to the same carbon atom may be taken together with the said carbon atom to form substituted or unsubstituted non-aromatic carbocycle.

As substituents for R¹⁰ or R¹¹, halogen and the like are exemplified.

R¹² are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The two R¹² connected to the adjacent carbon atoms constituting the ring may be taken together to form substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

The other embodiment of R¹² is halogen, hydroxy, carboxy, sulfo, cyano, nitro, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The two R¹² connected to the adjacent carbon atoms constituting the ring may be taken together to form substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R¹² are preferably each independently halogen, hydroxy, carboxy, cyano, nitro, substituted or unsubstituted alkyl, substituted or unsubstituted alkenyl, substituted or unsubstituted alkyloxy, substituted or unsubstituted amino, substituted or unsubstituted alkyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, or substituted or unsubstituted aromatic heterocyclyloxy.

R¹² are more preferably each independently halogen, cyano, alkyl, haloalkyl, alkyloxy, haloalkyloxy, dimethylamino, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

As substituents for R¹², halogen, oxo, alkyl, haloalkyl, alkylamino and the like are exemplified.

R¹³ are each independently halogen, hydroxy, carboxy, amino, imino, hydroxyamino, hydroxyimino, carbamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted monoalkylcarbamoyl, substituted or unsubstituted dialkylcarbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl.

The other embodiment of R¹³ is halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl.

R¹³ connected to the non-adjacent and different carbon atoms may be taken together to form alkylene.

R¹³ are preferably each independently hydroxy, halogen or substituted or unsubstituted alkyl, further preferably, alkyl or haloalkyl.

b are each independently an integer of 0 to 4, preferably an integer of 0 to 2.

b' are each independently an integer of 0 to 4.

c is an integer of 0 to 4, preferably an integer of 0 to 2.

c' is an integer of 0 to 4.

d is an integer of 0 to 3, preferably an integer of 0 to 2.

d' is an integer of 0 to 3.

e is an integer of 0 to 10, preferably an integer of 0 to 3.

e' is an integer of 0 to 10.

f is an integer of 0 to 5, preferably an integer of 0 to 2.

f is an integer of 0 to 8.

g is 0 or 1.

g' is 0 or 1.

h is an integer of 0 to 7, preferably an integer of 0 to 2.

h' is an integer of 0 to 2.

i' is an integer of 0 to 9.

j' is an integer of 0 to 7.

When R³ is a group represented by formula: -Y-Z, Y is preferably a bond, 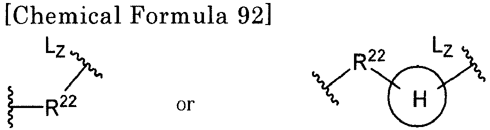 wherein a bond Lz is connecting to Z.

When R³ is a group represented by formula: -Y-Z, Y is further preferably a bond, 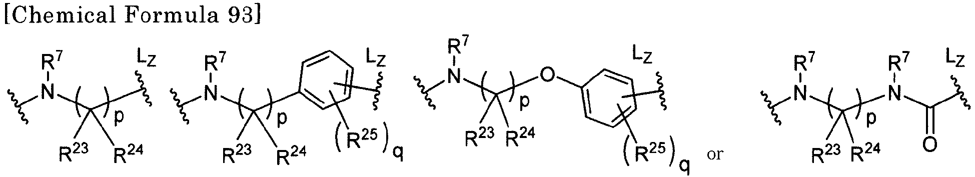 wherein a bond Lz is connecting to Z.

R²² are each independently substituted or unsubstituted alkylene which may be intervened with one or more groups selected from the group consisting of -O-,-NR⁷-, -C(=O)-NR⁷- and -NR⁷-C(=O)-,
substituted or unsubstituted alkenylene which may be intervened with one or more groups selected from the group consisting of -O-, -NR⁷-, -C(=O)-NR⁷- and -NR⁷-C(=O)-, or
substituted or unsubstituted alkynylene which may be intervened with one or more groups selected from the group consisting of -O-, -NR⁷-, -C(=O)-NR⁷- and -NR⁷-C(=O)-,
provided that the groups selected from the group consisting of -O-, -NR⁷-,-C(=O)-NR⁷- and -NR⁷ -C(=O)- are not connected adjacently in R²².

Ring H is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

As preferable substituents for ring H, halogen, carboxy, hydroxy, cyano, nitro, alkyl, haloalkyl, alkyloxy, haloalkyloxy, alkylamino, dialkylamino, alkyloxycarbonyl, alkylaminocarbonyl, dialkylaminocarbonyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy and the like are exemplified.

R²³ and R²⁴ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The other embodiment of R²³ and R²⁴ is hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R²³ and R²⁴ are preferably each independently hydrogen atom.

R²⁵ are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The other embodiment of R²⁵ is halogen, hydroxy, carboxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R²⁵ are preferably each independently halogen or substituted or unsubstituted alkyl, more preferably halogen, alkyl or haloalkyl.

p is an integer of 0 to 4.

q is an integer of 0 to 4.

When R⁴ is a group represented by formula: -Y-Z, Y is preferably 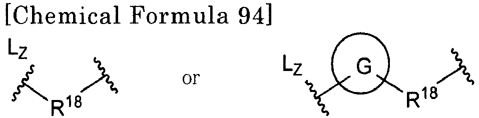 wherein a bond Lz is connecting to Z.

When R⁴ is a group represented by formula: -Y-Z, Y is further preferably 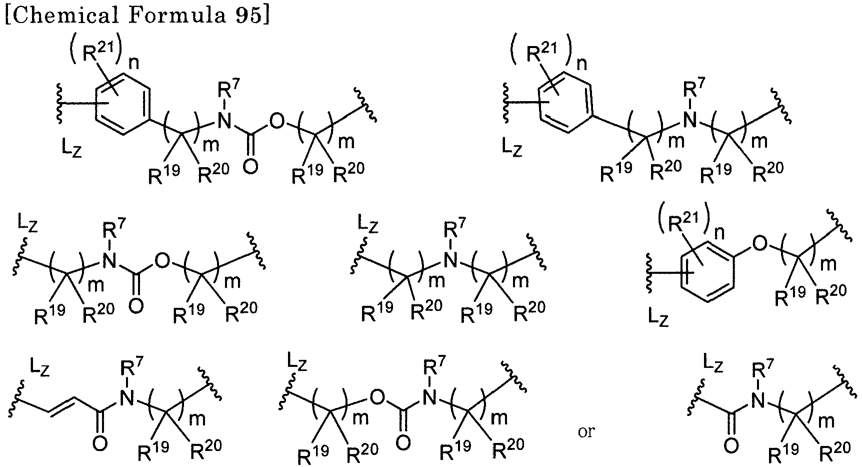 wherein a bond Lz is connecting to Z.

R¹⁸ are each independently substituted or unsubstituted alkylene which may be intervened with one or more groups selected from the group consisting of -O-, NR⁷-, -C(=O)-NR⁷- and -NR⁷-C(=O)-,
substituted or unsubstituted alkenylene which may be intervened with one or more groups selected from the group consisting of -O-, -NR⁷-, -C(=O)-NR⁷- and -NR⁷-C(=O)-, or
substituted or unsubstituted alkynylene which may be intervened with one or more groups selected from the group consisting of -O-, -NR⁷-, -C(=O)-NR⁷- and -NR⁷-C(=O)-,
provided that the groups selected from the group consisting of -O-, -NR⁷-,-C(=O)-NR⁷- and -NR⁷-C(=O)- are not connected adjacently in R¹⁸.

Ring G is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R¹⁹ and R²⁰ are each independently hydrogen atom, halogen, hydroxy, carboxy, amino, carbamoyl, sulfamoyl, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The other embodiment of R¹⁹ and R²⁰ is hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R¹⁹ and R²⁰ are preferably each independently hydrogen atom.

R²¹ are each independently halogen, hydroxy, carboxy, amino, imino, hydroxyamino, hydroxyimino, carbamoyl, sulfamoyl, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The other embodiment of R²¹ is halogen, hydroxy, carboxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

R²¹ are preferably each independently substituted or unsubstituted alkyl, more preferably alkyl or haloalkyl.

m are each independently an integer of 0 to 4.

n is an integer of 0 to 4.

"substituted aromatic carbocyclyl", "substituted non-aromatic carbocyclyl", "substituted aromatic heterocyclyl", or "substituted non-aromatic heterocyclyl" in Z is preferably substituted with at least one of acid group or neutral group such as substituted or unsubstituted carbamoyl group or the like. Moreover, these cyclyl may be substituted with other substituents, more preferably, substituted with at least one of acid group, and may be substituted with other substituents.

"acid group" means a group functions as a proton donor. The kind of acid group is not limited. Acid group includes cyclic or a non-cyclic, or its combination.

As non-cyclic acid group, for example, -COOH, -OH, -CONHOH, -CONHCN,-SO₃H, sulfonamide [example: -SO₂NH₂ or -NR³⁶SO₃H], acylsulfonamide (example:-CONHSO₂R³⁶ or -SO₂NHCOR³⁶], -P(=O)(OH)₂, =P(=O)OH, -P(=O)(OH)(NH₂),-C₆H₄OH and the like.

R³⁶ is hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

As cyclic acid group, for example, cyclic having 1,3-diketon structure as follows: 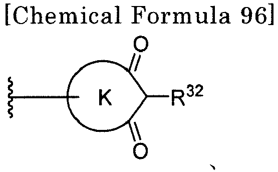 or, a group as follows are exemplified.

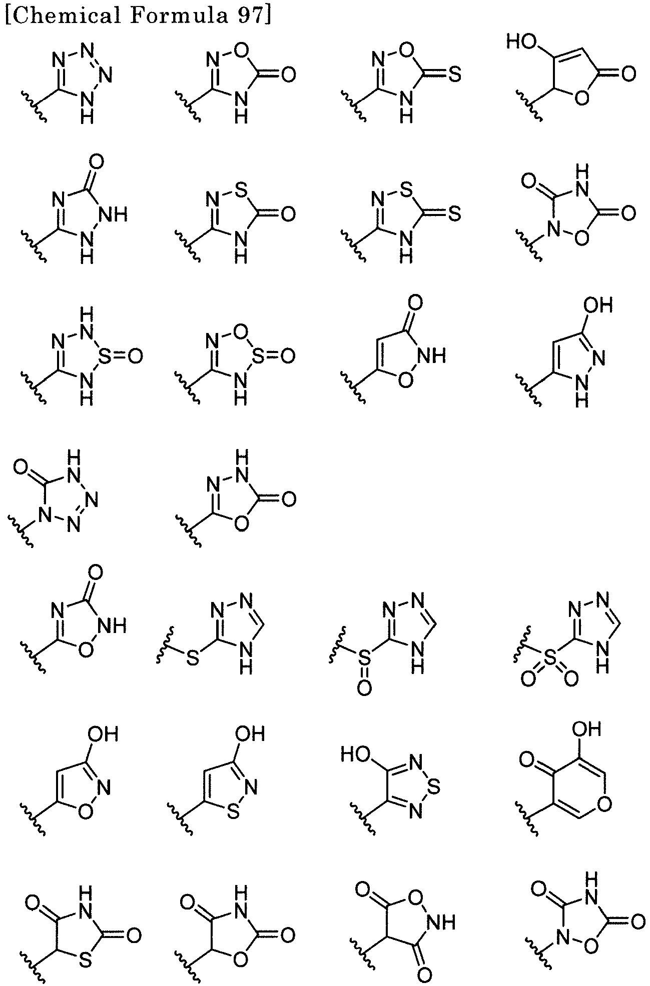

Preferable acid group is -COOH or its biologically equivalent group, more preferable acid group is -COOH.

As an embodiment of Z, bicyclic substituted or unsubstituted non-aromatic carbocyclyl having acid group or bicyclic substituted or unsubstituted non-aromatic heterocyclyl having acid group are exemplified.

As the other embodiment of Z, bicyclic or tricyclic substituted non-aromatic carbocyclyl or bicyclic or tricyclic substituted non-aromatic heterocyclyl are exemplified.

Z is preferably

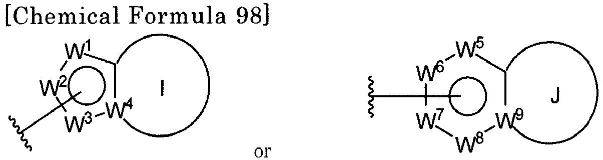

Z is further preferably

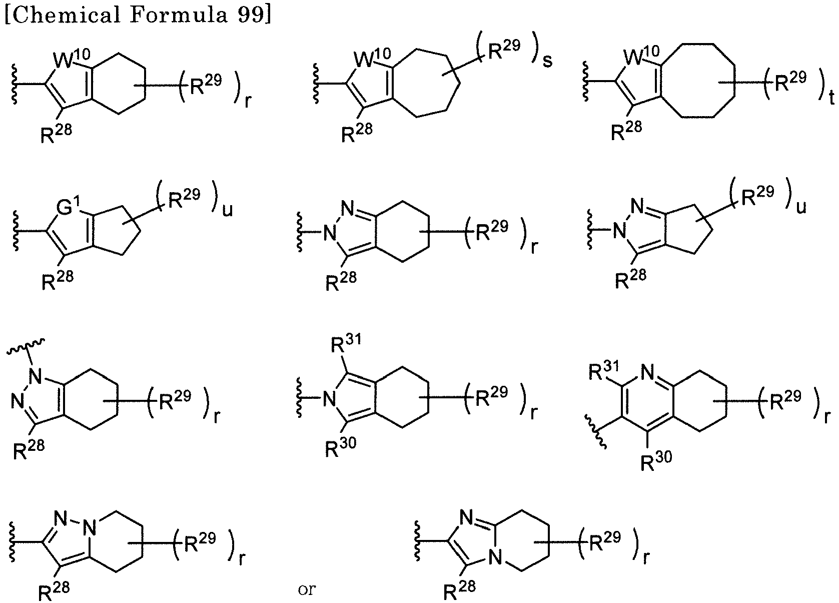

The other embodiment of Z is preferably

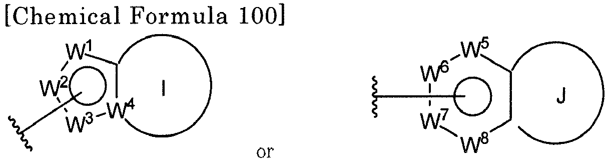

Z is further preferably

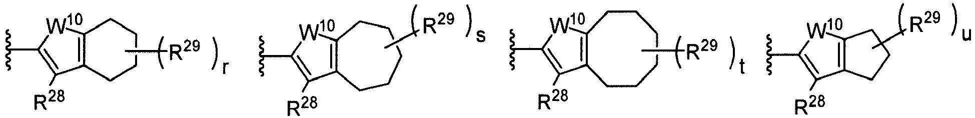

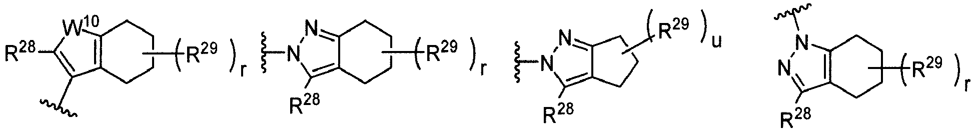

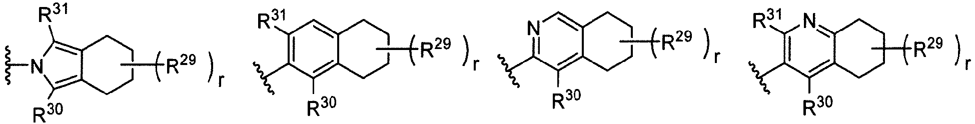

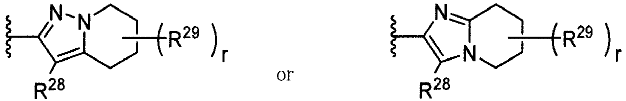

W¹ W², W³ W⁵, W⁶, W⁷ and W⁸ are each independently C, CR²⁶, O, S, N or NR²⁷.

W⁴ and W⁹ are each independently C, CR²⁶ or N.

The other embodiment of W⁴ is C, or N.

R²⁶ are each independently -COOH or its biologically equivalent group, hydrogen atom, halogen, hydroxy, carboxy, amino, imino, hydroxyamino, hydroxyimino, carbamoyl, sulfamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
provided that at least one of W¹, W², W³ and W⁴ is CR²⁶, and at least one of said R²⁶ is -COOH or its biologically equivalent group,
provided that at least one of W⁵, W⁶, W⁷, W⁸ and W⁹ is CR²⁶, and at least one of said R²⁶ is -COOH or its biologically equivalent group.

The other embodiment of R²⁶ is -COOH or its biologically equivalent group, hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
provided that at least one of W¹, W² and W³ is CR²⁶, and at least one of said R²⁶ is -COOH or its biologically equivalent group,
provided that at least one of W⁵, W⁶, W⁷ and W⁸ is CR²⁶, and at least one of said R²⁶ is -COOH or its biologically equivalent group.

R²⁶ is preferably each independently -COOH, its biologically equivalent group, or hydrogen atom,
provided that at least one of W¹, W² and W³ is CR²⁶, and at least one of said R²⁶ is -COOH or its biologically equivalent group,
provided that at least one of W⁵, W⁶, W⁷ and W⁸ is CR²⁶, and at least one of said R²⁶ is -COOH or its biologically equivalent group.

A biologically equivalent group of "-COOH" means generally a bioisosteric group which can be substituted with "-COOH" by person skilled with expecting biologically equivalent.

Specifically, a biologically equivalent group of "-COOH" means it is comparatively similar to chemical structure of "-COOH" and similar property in the aspect of physical property such as acidity, water solubility and/or biokinetics and the like, and it has acid proton(s).

The position of said acid proton may be taken to form salt (example: alkali metal salt (example: sodium salt)).

These are introduced in for example, J. Med. Chem. 1992, 35, 1176-1183, J Med. Chem. 1993, 36, 2485-2493, J Med. Chem. 1992, 35, 3691-3698, J Med. Chem. 1995, 38, 617-628, Med. Res. Rev. 1983, 3, 91-118, J Med. Chem. 2001, 44, 1560-1563, Bioorganic & Medicinal Chemistry Letters, Vol. 4, No. 1, 41-44, 1994 and the like.

A biologically equivalent group of "-COOH" is preferably -CONHR³⁷, -SO₃H,-SO₂NHR³⁷ (R³⁷ is amino residue (example: hydrogen, OH, lower alkyl, substituted sulfonyl (example: lower alkylsulfonyl, aminosulfonyl, halogenated lower alkylsulfonyl), aromatic carbocyclyl or aromatic heterocyclyl)), -PO₃H₂, -OH,-COCH=C(OH)CF₃, -NHSO₂CF₃, -CONHSO₂CF₃, - -NHSO₂Me, -CONHCOMe,-CONHSO₂Me, -NHCOMe, -COCH₂COMe, or optionally substituted (example of substituent: electron accepting group such as =O, =S, -OH and the like, lower alkyl (example: methyl)) heterocyclyl having -NH- and other hetero atom as an atom constituting ring (example: N, NRa (Ra is hydrogen, lower alkyl and the like), O, S), more preferably 5- to 6-membered heterocyclyl. In detail, it has a structure such as C=N, N=N, =O, =S and the like at the position adjacent to -NH-.

Said heterocyclyl is tetrazole or its derivative, or other heterocyclyl.

Examples are described below. These isomers include in the present invention.

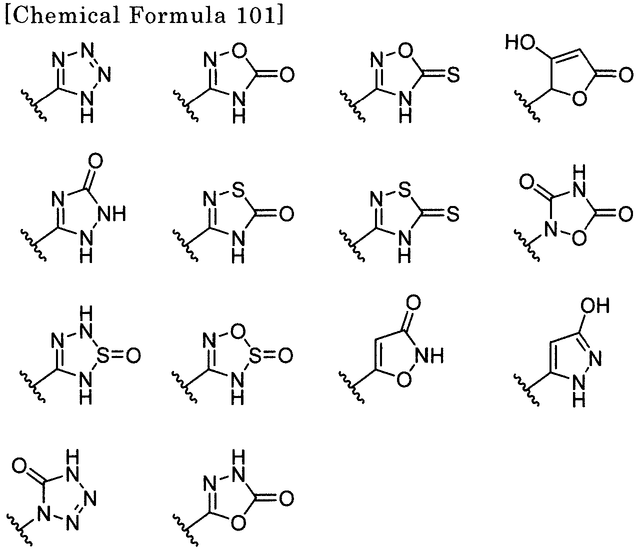

R² are each independently hydrogen atom, carboxy, carbamoyl, sulfamoyl, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl.

The other embodiment of R²⁷ is hydrogen atom, carboxy, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,

R²⁷ are preferably each independently hydrogen atom, substituted or unsubstituted alkyl, or substituted or unsubstituted aromatic carbocyclylalkyl.

Ring I and ring J are each independently substituted or unsubstituted non-aromatic carbocycle, or substituted or unsubstituted non-aromatic heterocycle.

W¹⁰ is -S-, -O- or -NR²⁷-.

R²⁸ are each independently -COOH or its biologically equivalent group, more preferably-COOH.

R³⁰ and R³¹are each independently -COOH or its biologically equivalent group, hydrogen atom, halogen, hydroxy, carboxy, amino, imino, hydroxyamino, hydroxyimino, carbamoyl, sulfamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted alkylsulfonyl, substituted or unsubstituted alkenylsulfonyl, substituted or unsubstituted alkynylsulfonyl, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted monoalkylcarbamoyl, substituted or unsubstituted dialkylcarbamoyl, substituted or unsubstituted monoalkylsulfamoyl, substituted or unsubstituted dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
provided that at least one of R³⁰ and R³¹ is -COOH or its biologically equivalent group.

The other embodiment of R³⁰ and R³¹ is -COOH or its biologically equivalent group, hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
provided that at least one of R³ an R³¹ is -COOH or its biologically equivalent group.

At least one of R³⁰ and R³¹ is preferably -COOH or its biologically equivalent group, the other is hydrogen atom.

R²⁹ are each independently halogen, hydroxy, carboxy, amino, imino, hydroxyamino, hydroxyimino, carbamoyl, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted monoalkylamino, substituted or unsubstituted dialkylamino, substituted or unsubstituted monoalkylcarbonylamino, substituted or unsubstituted dialkynylcarbonylamino, substituted or unsubstituted monoalkylsulfonylamino, substituted or unsubstituted dialkylsulfonylamino, substituted or unsubstituted alkylimino, substituted or unsubstituted alkenylimino, substituted or unsubstituted alkynylimino, substituted or unsubstituted alkylcarbonylimino, substituted or unsubstituted alkenylcarbonylimino, substituted or unsubstituted alkynylcarbonylimino, substituted or unsubstituted alkyloxyimino, substituted or unsubstituted alkenyloxyimino, substituted or unsubstituted alkynyloxyimino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted monoalkylcarbamoyl, substituted or unsubstituted dialkylcarbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl.

Two R²⁹ connected to the adjacent carbon atoms may be taken together to form substituted or unsubstituted ring.

Two R²⁹ connected to the non-adjacent and different carbon atoms may be taken together to form substituted or unsubstituted bridge.

Two R²⁹ connected to the same carbon atom may be taken together to form substituted or unsubstituted spiro ring.

Two R²⁹ connected to the same carbon atom may be taken together to form oxo.

The other embodiment of R²⁹ is halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl.

Two R²⁹ connected to the adjacent carbon atoms may be taken together to form substituted or unsubstituted aromatic carbocycle, substituted or unsubstituted non-aromatic carbocycle, or substituted or unsubstituted non-aromatic heterocycle.

Two R²⁹ connected to the non-adjacent and different carbon atoms may be taken together to form substituted or unsubstituted alkylene.

Two R²⁹ connected to the same carbon atom may be taken together to form substituted or unsubstituted non-aromatic carbocycle or substituted or unsubstituted non-aromatic heterocycle.

Two R²⁹ connected to the same carbon atom may be taken together to form oxo.

R²⁹ are preferably each independently halogen, substituted or unsubstituted alkyl, substituted or unsubstituted aromatic carbocyclyl.

Two R²⁹ connected to the same carbon atom may be taken together to form substituted or unsubstituted non-aromatic carbocycle.

Two R²⁹ connected to the same carbon atom may be taken together to form oxo.

Further preferably, R²⁹ are each independently alkyl or haloalkyl.

As the other embodiment, Z is a group represented by formula: 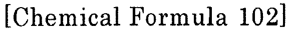 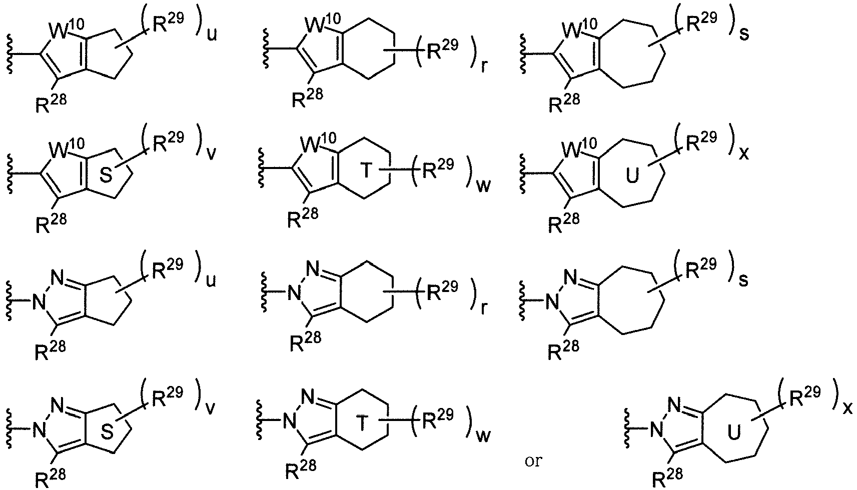
wherein W¹⁰ is -S-, -O- or -NR²⁷-.
Ring S is 5-membered non-aromatic heterocycle having one hetero atom selected from O, S or NR²⁷, and said hetero atom is not a condensed positional atom.
Ring T is 6-membered non-aromatic heterocycle having one hetero atom selected from O, S or NR²⁷, and said hetero atom is not a condensed positional atom.
Ring U is 7-membered non-aromatic heterocycle having one hetero atom selected from O, S or NR²⁷, and said hetero atom is not a condensed positional atom.

R²⁸ and R²⁹ are defined as the same above.

r is an integer of 0 to 8.

s is an integer of 0 to 10.

t is an integer of 0 to 12.

u is an integer of 0 to 6.

As an embodiment of Z, preferably 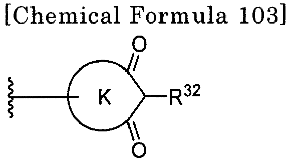 further preferably,

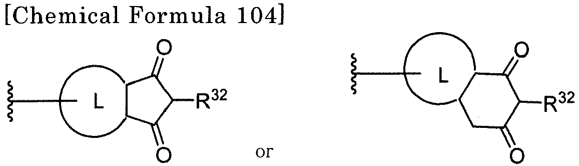

Ring K is substituted or unsubstituted non-aromatic carbocyclyl or substituted or unsubstituted non-aromatic heterocyclyl.

R³² is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

Ring L is substituted or unsubstituted aromatic carbocyclyl, or substituted or unsubstituted aromatic heterocyclyl.

As an embodiment of Z, preferably

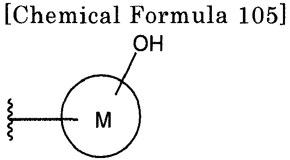

Ring M is substituted or unsubstituted aromatic carbocyclyl, or substituted or unsubstituted aromatic heterocyclyl.

Ring M is preferably substituted or unsubstituted benzene ring.

As an embodiment of Z, preferably

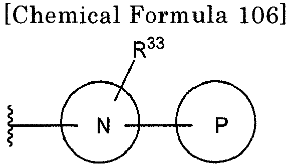

Ring N and ring P are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R³³ is -OH, -COOH or its biologically equivalent group.

Ring A is substituted or unsubstituted non-aromatic carbocyclyl, or substituted or unsubstituted non-aromatic heterocyclyl in above formula (II).

Ring A is preferably substituted or unsubstituted bicyclic non-aromatic carbocyclyl, or substituted or unsubstituted bicyclic non-aromatic heterocyclyl in above formula (II).

Ring A is further preferably substituted or unsubstituted bicyclic non-aromatic heterocyclyl in above formula (II).

Ring A is especially preferably, 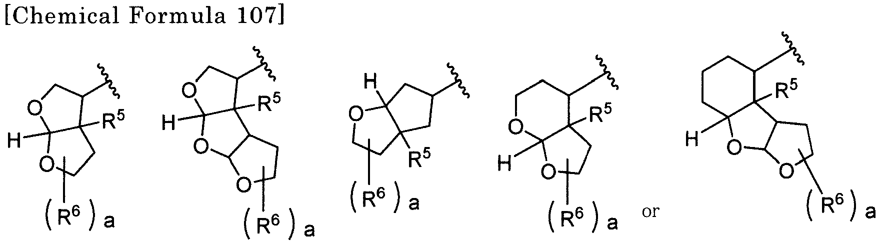 in above formula (II).

R³⁴ is a group represented by formula: -Y-Z, or hydrogen atom.

At least one of R¹, R², R³ and R³⁴ is a group represented by formula: -Y-Z in above formula (II).

Ring Q is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R³⁵ is a group represented by formula: -Y-Z, or hydrogen atom.

At least one of R¹, R², R³ and R³⁵ is a group represented by formula: -Y-Z in above formula (III).

X is a residue of compound having HIV protease inhibitory activities.

As X, a residue of Amprenavir, Atazanavir, Darunavir, Fosamprenavir, Indinavir, Lopinavir, Ritonavir, Nelfinavir, Saquinavir, Tipranavir and the like or its derivative are exemplified.

X is preferably a residue of Atazanavir, Darunavir or its derivative.

X is further preferably a residue of Darunavir or its derivative.

"a residue of compound having HIV protease inhibitory activity" means a group which is formed by removing one hydrogen from a compound having HIV protease inhibitory activity.

R³⁶ is hydrogen atom, a protecting group for hydroxy group or a group represented by formula: -C(=O)-R³⁸, wherein R³⁸ is leaving group.

R³⁷ is hydrogen atom or a protecting group for hydroxy group.

As "leaving group" in R³⁸, halogen, methanesulfonic acid, trifluoromethanesulfonate, nonafluorobutanesulfonate, 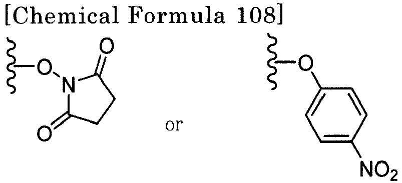 and the like are exemplified.

R³⁹ is hydrogen atom, halogen, boronate, boronate ester, or a group represented by formula: -OR⁴¹ or -NH(R⁴²).

R⁴¹ is methanesulfonyl group, trifluoromethanesulfonyl group, p-toluenesulfonyl group, or nonafluorobutanesulfonyl group.

R⁴² is hydrogen atom or a protecting group for amino group.

R⁴⁰ is hydrogen atom or a protecting group for carboxy group.

R⁴³ is hydrogen, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, or a group represented by formula: -C(=O)-R⁴⁵ or -SO₂ -R⁴⁶.

R⁴⁵ is substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted amino, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R⁴⁶ is substituted or unsubstituted alkyl, substituted or unsubstituted alkenyl, substituted or unsubstituted alkynyl, substituted or unsubstituted amino, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R⁴⁴ is hydrogen atom or a protecting group for carboxy group.

Ring W is 5- to 8-membered non-aromatic carbocycle.

When ring W is 5-membered ring, Y is an integer of 0 to 6.

When ring W is 6-membered ring, Y is an integer of 0 to 8.

When ring W is 7-membered ring, Y is an integer of 0 to 10.

When ring W is 8-membered ring, Y is an integer of 0 to 12.

R⁴⁷ is halogen, boronate, boronate ester, or a group represented by formula: - OR⁴⁹.

R⁴⁹ is methanesulfonyl group, trifluoromethanesulfonyl group, p-toluenesulfonyl group or nonafluorobutanesulfonyl group.

R⁴⁸ is a protecting group for carboxy group.

R⁵⁰ are each independently hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

Two R⁵⁰ may be taken together with the adjacent carbon atom to form substituted or unsubstituted non-aromatic carbocycle,
provided that two R⁵⁰ is not hydrogen atom at the same time.

As substituents for R⁵⁰, halogen, alkyl, haloalkyl and the like are exemplified.

R⁵¹ is a protecting group for carboxy group.

The compounds of formula (I), formula (II), formula (III) and formula (IV) are not limited to specific isomers but include all possible isomers (e.g., keto-enol isomers, imine-enamine isomers, diastereoisomers, enantiomers, rotamers or the like), racemates or mixtures thereof.

One or more hydrogen, carbon and/or other atoms in the compounds of formula (I), formula (II), formula (III) and formula (IV) may be replaced with isotopes of hydrogen, carbon and/or other atoms respectively. Examples of isotopes include hydrogen, carbon, nitrogen, oxygen, phosphorus, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl respectively. The compounds of formula (I), formula (II), formula (III) and formula (IV) include compounds replaced with these isotopes. The compounds replaced with the above isotopes are useful as medicines and include all of radiolabeled compounds of the compound of formula (I), formula (II), formula (III) and formula (IV). A "method of radiolabeling" in the manufacture of the "radiolabeled compounds" is encompassed by the present invention, and the "radiolabeled compounds" are useful for studies on metabolized drug pharmacokinetics, studies on binding assay and/or diagnostic tools.

A radiolabeled compound of formula (I), formula (II), formula (III) and formula (IV) can be prepared using well-known methods in this field of the invention. For example, a tritium-labeled compound of formula (I) can be prepared by introducing a tritium to a certain compound of formula (I), through a catalytic dehalogenation reaction using a tritium. This method comprises reacting with an appropriately-halogenated precursor of the compound of formula (I) with tritium gas in the presence of an appropriate catalyst, such as Pd/C, and in the presence or absent of a base. The other appropriate method of preparing a tritium-labeled compound can be referred to "Isotopes in the Physical and Biomedical Sciences, Vol. 1, Labeled Compounds (Part A), Chapter 6 (1987)". A ¹⁴C-labeled compound can be prepared by using a raw material having ¹⁴C.

The pharmaceutically acceptable salts of the compounds of formula (I), formula (II), formula (III) and formula (IV) include, for example, salts with alkaline metal (e.g., lithium, sodium, potassium or the like), alkaline earth metal (e.g., calcium, barium or the like), magnesium, transition metal (e.g., zinc, iron or the like), ammonia, organic bases (e.g., trimethylamine, triethylamine, dicyclohexylamine, ethanolamine, diethanolamine, triethanolamine, meglumine, ethylenediamine, pyridine, picoline, quinoline or the like) or amino acids, or salts with inorganic acids (e.g., hydrochloric acid, sulfuric acid, nitric acid, carbonic acid, hydrobromic acid, phosphoric acid, hydroiodic acid or the like) or organic acids (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid or the like). Especially, salts with hydrochloric acid, sulfuric acid, phosphoric acid, tartaric acid, methanesulfonic acid and the like are included. These salts can be formed by the usual methods.

The compounds of formula (I), formula (II), formula (III) and formula (IV) of the present invention or pharmaceutically acceptable salts thereof may form solvates (e.g., hydrates or the like), cocrystal and/or crystal polymorphs. The present invention encompasses those various solvates, cocrystal and crystal polymorphs. "Solvates" may be those wherein any numbers of solvent molecules (e.g., water molecules or the like) are coordinated with the compounds of formula (I). When the compounds of formula (I) or pharmaceutically acceptable salts thereof are allowed to stand in the atmosphere, the compounds may absorb water, resulting in attachment of adsorbed water or formation of hydrates. Recrystallization of the compounds of formula (I), formula (II), formula (III) and formula (IV) or pharmaceutically acceptable salts thereof may produce crystal polymorphs. The term "cocrystal" means that a compound of formula (I) or a salt thereof and a counter-molecule exists in the same crystal lattice, and it can be formed with any number of counter-molecules.

The intermediate (example. compounds described in above (81) to (85)) using in the present invention includes not only compounds but also its salts. The above salts are used as salts. These compounds or its salts include its solvents.

The compounds of formula (I), formula (II), formula (III) and formula (IV) of the present invention or pharmaceutically acceptable salts thereof may form prodrugs. The present invention also encompasses such various prodrugs. Prodrugs are derivatives of the compounds of the present invention that have chemically or metabolically degradable groups, and compounds that are converted to the pharmaceutically active compounds of the present invention through solvolysis or under physiological conditions in vivo. For example, prodrugs include compounds that are converted to the compounds of formula (I) through enzymatic oxidation, reduction, hydrolysis or the like under physiological conditions in vivo, compounds that are converted to the compounds of formula (I) through hydrolysis by gastric acid etc., and the like. Methods for selecting and preparing suitable prodrug derivatives are described in, for example, "Design of Prodrugs, Elsevier, Amsrdam, 1985". Prodrugs themselves may have some activity.

When the compounds of formula (I), formula (II), formula (III) and formula (IV) or pharmaceutically acceptable salts thereof have hydroxyl group(s), prodrugs include acyloxy derivatives and sulfonyloxy derivatives that are prepared by, for example, reacting compounds having hydroxyl group(s) with suitable acyl halide, suitable acid anhydride, suitable sulfonyl chloride, suitable sulfonyl anhydride or mixed anhydride, or with a condensing agent. For example, they include CH₃COO-, C₂H₅COO-, tert-BuCOO-, C₁₅H₃₁COO-, PhCOO-, (m-NaOOCPh)COO-, NaOOCCH₂ CH₂ COO-, CH₃ CH(NH₂)COO-, CH₂N(CH₃)₂ COO-, CH₃SO₃-, CH₃CH₂SO₃ -, CF₃SO₃-, CH₂FSO₃-, CF₃CH₂SO₃ -, p-CH₃ O-PhSO₃ -, PhSO₃ - and p-CH₃PhSO₃.

### (Synthetic methods for the compounds of the present invention)

For example, the compounds of formula (I) of the present invention can be prepared by the general synthetic methods described below. The methods for extraction, purification and the like may be carried out by using the usual method for the experiments of organic chemistry.

The compounds of the present invention can be synthesized by referring to the known methods in this field.

### (Synthesis of source compound)

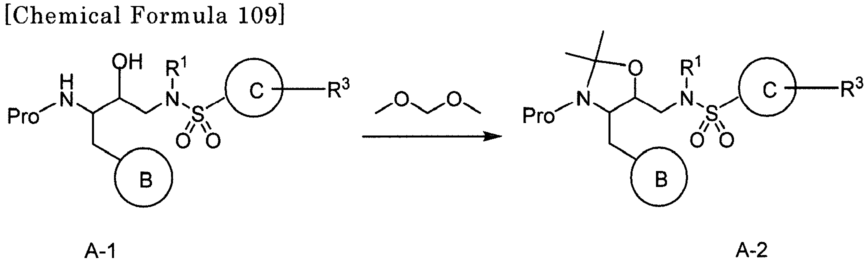

wherein each symbol is defined as the same above, known compound may be used for compound represented by formula (A-1), or compound which is induced from known compound in accordance with a conventional manner may be used.
"Pro" means protecting group.
"Pro" includes benzyl group, benzoyl group, benzyloxycarbonyl group, benzyloxycarbonyl group, t-butoxycarbonyl group and the like.

### Step 1

Compound represented by formula (A-2) can be manufactured by reacting dimethoxypropane and tosilate with Compound represented by formula (A-1).

Pyridinium p-toluenesulfonate, camphorsulfonic acid or the like may be used instead of tosilate.

A kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of ester (example: methyl acetate, ethyl acetate and the like), a kind of nitrile (example: acetonitrile and the like) and the like are exemplified as reaction solvent. These can be used solely or by mixture.

Reaction solvent is preferably a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like) and the like. These can be used solely or by mixture.

Reaction temperature is 0°C to reflux temperature of solvent, preferably room temperature to reflux temperature of solvent.

Reaction time is 0.5 hours to 12 hours, preferably 2 hours to 12 hours. 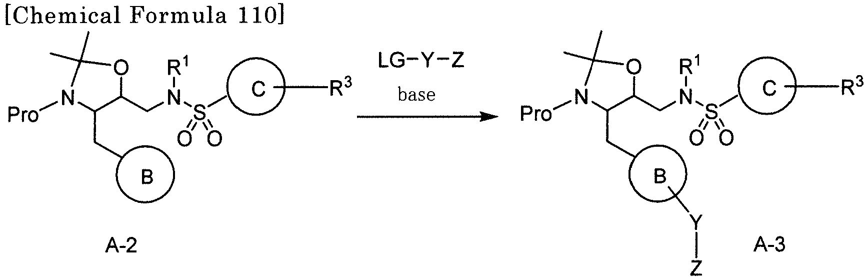
wherein each symbol is defined as the same above.
"LG" means leaving group. LG is halogen, hydroxy group, mesylate and the like.

### Step 2

Compound represented by formula (A-3) can be manufactured by reacting Compound represented by formula (A-2) with Compound represented by formula: LG-Y-Z optionally under presence of base.

Reaction solvent is N,N-dimethylformamide, dimethyl sulfoxide, a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of ester (example: methyl acetate, ethyl acetate and the like), a kind of ketone (example: acetone, methylethyl ketone and the like), a kind of nitrile (example: acetonitrile and the like), a kind of alcohol (example: methanol, ethanol, t-buthanol and the like), water and the like. These can be used solely or by mixture.

Reaction solvent is preferably, N,N-dimethylformamide, dimethyl sulfoxide, a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of nitrile (example: acetonitrile and the like), a kind of alcohol (example: methanol, ethanol, t-buthanol and the like), water and the like. These can be used solely or by mixture.

Base is, for example, metal hydride (example: sodium hydride and the like), metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, metal amide, organic amine (example: triethylamine, diisopropylethylamine, DBU, 2,6-lutidine and the like), pyridine, alkyllithium (n-BuLi, sec-BuLi, tert-BuLi) and the like.

Base is preferably, for example, metal hydride (example: sodium hydride and the like), metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, organic amine (example: triethylamine, diisopropylethylamine, DBU, 2,6-lutidine and the like), pyridine and the like.

Reaction temperature is -78 to 150°C, preferably 0 to 130°C.

Reaction time is 0.5 to 48 hours, preferably 0.5 hours to 12 hours.

Compound represented by formula (I) whose R² is -Y-Z, and -Y- is -NR⁷-CHR¹⁰ -Y¹- (wherein a bond from Y¹ conects with Z. -Y¹- is a spacer of any combination selected from the group consisting of a bond, -O-, -S-, -NR⁷-, -C (=O)-,-SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl.) can be synthesized as following: 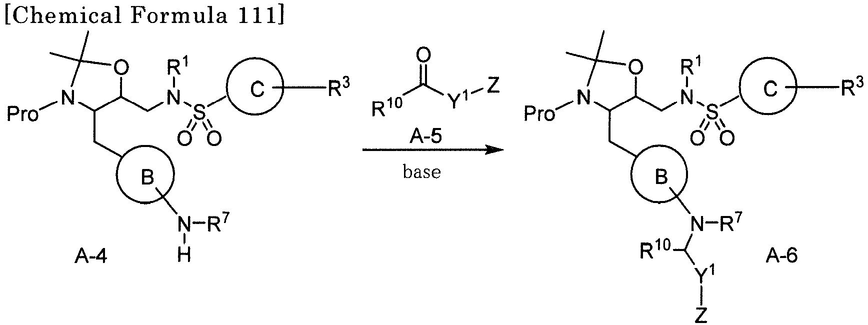 wherein each symbol is defined as the same above.

### Step 3

The reaction can be used by reaction condition known as reductive amination.

In presence or absence of condensing agent, Compound represented by formula (A-6) can be obtained by condensing Compound represented by formula (A-5) and amine (A-4) under the condition of reductive amination, and reducing with reducing agent.

Condensing agent is 4-toluenesulfonic acid, methanesulfonic acid, acetic acid, magnesium sulfate anhydrous, tetraisopropyl orthotitanate, titanium (IV) chloride, molecular sieve and the like. 1 to 10 molar equivalent of condensing agent to Compound represented by formula (A-5) can be used.

1 to 10 molar equivalent of amine (A-4) to Compound represented by formula (A-5) can be used.

Reducing agent is sodium borohydride, sodium cyanoborohydride, sodium triacetoxyborohydride, borane and its complex, lithium borohydride, potassium borohydride, diisobutylaluminium hydride and the like. 1 to 10 molar equivalent of reducing agent to Compound represented by formula (A-5) can be used.

Reaction solvent is tetrahydrofuran, toluene, dichloromethane, chloroform, methanol, ethanol and the like. These can be used solely or by mixture.

Reaction temperature is -78°C to reflux temperature of solvent, preferably 0 to 25°C.

Reaction time is 0.5 to 48 hours, preferably an hour to 6 hours.

Compound represented by formula (I) whose R² is -Y-Z, and -Y- is -O-Y¹-(wherein -Y¹- is defined as the same above.) can be synthesized as following: 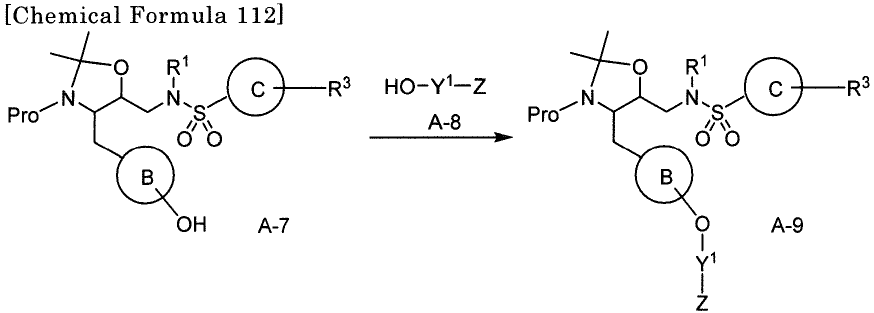 wherein each symbol is defined as the same above.

### Step 4

The reaction condition known as Mitsunobu reaction can be used.

Compound represented by formula (A-9) can be obtained by reacting Compound represented by formula (A-8) to Compound represented by formula (A-7) under presence of triphenylphosphine and condensing agent.

Condensing agent includes DEAD, DIAD and the like.

Reaction solvent includes tetrahydrofuran, dioxane, ethyl acetate, toluene, acetonitrile and the like. These can be used solely or by mixture.

Reaction temperature is 0°C to 60°C, preferably 10°C to 40°C.

Reaction time is 0.1 hour to 12 hours, preferably 0.2 hours to 6 hours.

Compound represented by formula (I), whose R² is -Y-Z, and Y is a spacer of any combination selected from the group consisting of substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl, can be synthesized as following: 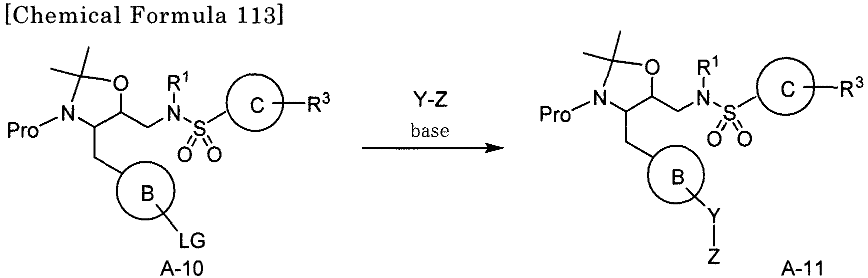 wherein each symbol is defined as the same above.

"LG" means leaving group. "LG" includes halogen, trifluoromethanesulfonate, nonafluorobutanesulfonate group and the like.

"Pro" includes benzyl group, benzoyl group, benzyloxycarbonyl group, benzyl group, benzoyl group, benzyloxycarbonyl group, t-butoxycarbonyl group and the like.

### Step 5

The reaction condition known as cross-coupling reaction can be used.

Compound represented by formula (A-11) can be obtained by coupling Compound represented by formula (A-10) and Y-Z under the condition of cross-coupling reaction under presence of metal catalyst and ligand.

Metal catalyst includes palladium (II) acetate, palladium (II) dichloride, tris (dibenzylideneacetone)dipalladium (0), palladium (II) acetylacetonate, dichloro[1,1'-bis (diphenylphosphino) ferrocene] palladium, bis(triphenylphosphine) palladium (II) dichloride, [1,1'-bis (di-tert-buthylphosphino) ferrocene] palladium (II) dichloride, RuPhos Pd G2 and the like. 0.05 to 0.5 molar equivalent of metal catalyst to Compound represented by formula (A-10) can be used.

Ligand includes triphenylphosphine, dppf, XPhos, DavePhos, RuPhos, BrettPhos, PEPPSI and the like. 0.05 to 0.5 molar equivalent of ligand to Compound represented by formula (A-10) can be used. 1 to 10 molar equivalent of Y-Z to Compound represented by formula (A-10) can be used.

Reaction solvent includes N,N-dimethylformamide, dimethyl sulfoxide, a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of ester (example: methyl acetate, ethyl acetate and the like), a kind of ketone (example: acetone, methylethyl ketone and the like), a kind of nitrile (example: acetonitrile and the like), a kind of alcohol (example: methanol, ethanol, t-buthanol and the like), 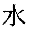 and the like. These can be used solely or by mixture.

Reaction solvent is preferably, N,N-dimethylformamide, a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of nitrile (example: acetonitrile and the like), a kind of alcohol (example: methanol, ethanol, t-buthanol and the like), water and the like. These can be used solely or by mixture.

Base includes, for example, metal hydride (example: sodium hydride and the like), metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, metal amide, organic amine (example: triethylamine, diisopropylethylamine, DBU, 2,6-lutidine and the like), pyridine, alkyllithium (n-BuLi, sec-BuLi, tert-BuLi) and the like.

Base is preferably, for example, metal hydride (example: sodium hydride and the like), metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, organic amine (example: triethylamine, diisopropylethylamine, DBU, 2,6-lutidine and the like), pyridine and the like.

Reaction temperature is -78 to 150°C, preferably 0 to 130°C.

Reaction time is 0.5 to 48 hours, preferably 0.5 hours to 12 hours.

### (Synthetic method 1)

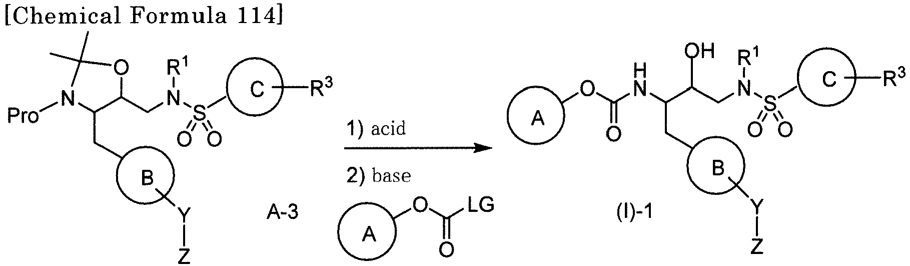

wherein each symbol is defined as the same above.

### Step 6

Compound represented by formula (I)-1 can be obtained by carbamating preferably under presence of base after deprotecting Compound represented by formula (A-3) preferably under presence of acid.

Reaction solvent includes N,N-dimethylformamide, a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of nitrile (example: acetonitrile and the like), water and the like. These can be used solely or by mixture.

Reaction solvent is preferably a kind of halogenated hydrocarbon (example: dichloromethane, chloroform, 1,2-dichloroethane and the like), a kind of nitrile (example: acetonitrile and the like) and the like. These can be used solely or by mixture.

Acid includes TFA, hydrochloric acid, sulfuric acid, sulfonic acid and the like. Preferably TFA, hydrochloric acid can be used as acid.

Base includes, for example, metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, organic amine (example: triethylamine, diisopropylethylamine, DBU, 2,6-lutidine and the like), pyridine and the like.

Base is preferably metal carbonate (example: sodium carbonate, calcium carbonate, cesium carbonate and the like), sodium hydrogen carbonate, organic amine (example: triethylamine, diisopropylethylamine, DBU, 2,6-lutidine and the like), pyridine and the like.

Reaction temperature is 0°C to 60°C, preferably 0°C to 40°C.

Reaction time is 0.5 hours to 24 hours, preferably 0.5 hours to 12 hours.

### (Synthetic method 2)

Compound represented by formula (I) whose R³ is -Y-Z, and -Y- is -NR⁷-Y²-(wherein a bond from Y¹ connects to Z, -Y²- is a spacer of any combination selected from the group consisting of a bond, -O-, -S-, -NR⁷-, -C (=O)-, -SO-, -SO₂-, substituted or unsubstituted alkylene, substituted or unsubstituted alkenylene, substituted or unsubstituted alkynylene, substituted or unsubstituted aromatic carbocyclediyl, substituted or unsubstituted non-aromatic carbocyclediyl, substituted or unsubstituted aromatic heterocyclediyl, and substituted or unsubstituted non-aromatic heterocyclediyl.) can be synthesized as following: 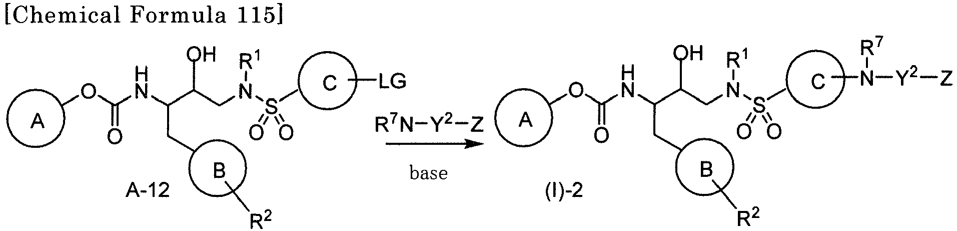 wherein each symbol is defined as the same above.

### Step 7

Reaction solvent includes N,N-dimethylformamide, N,N-dimethylacetamide, dimethyl sulfoxide, a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of nitrile (example: acetonitrile and the like), a kind of alcohol (example: methanol, ethanol, t-buthanol and the like), water and the like. These can be used solely or by mixture.

Reaction solvent is preferably N,N-dimethylformamide, N,N-dimethylacetamide, dimethyl sulfoxide, a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of alcohol (example: methanol, ethanol, t-buthanol and the like), water and the like. These can be used solely or by mixture.

Base includes, for example, metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, organic amine (example: triethylamine, diisopropylethylamine, DBU, 2,6-lutidine and the like), pyridine and the like.

Base is preferably metal carbonate (example: sodium carbonate, calcium carbonate, cesium carbonate and the like), sodium hydrogen carbonate, organic amine (example: triethylamine, diisopropylethylamine, DBU, 2,6-lutidine and the like), pyridine and the like.

Reaction temperature is 0°C to 150°C, preferably room temperature to 150°C.

Reaction time is 0.5 hours to 24 hours, preferably 0.5 hours to 12 hours.

### (Synthetic method 3)

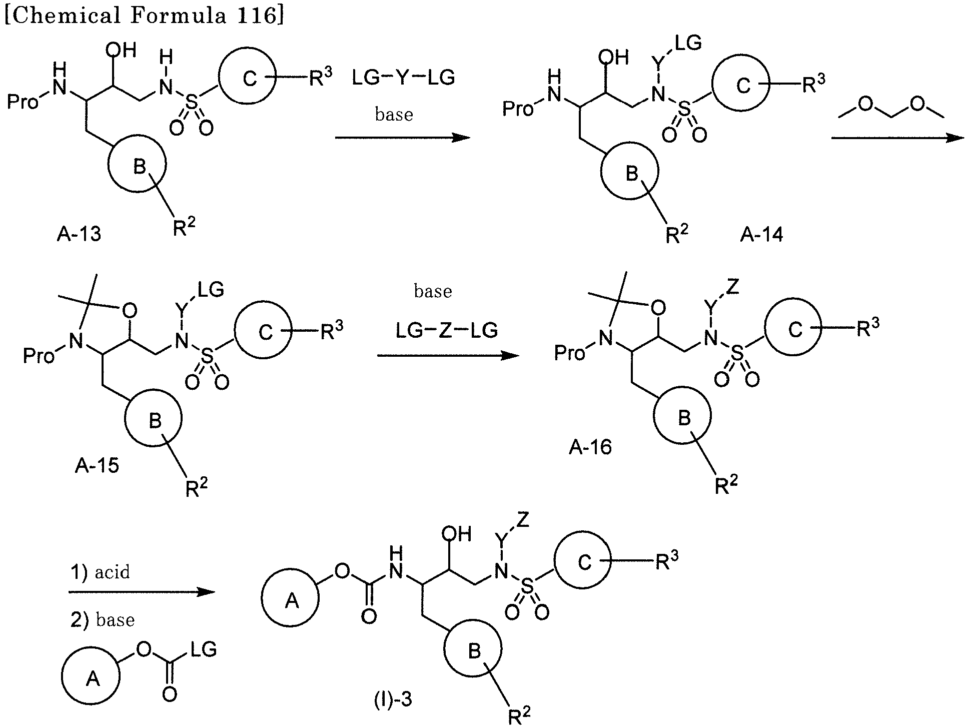

wherein each symbol is defined as the same above,

Known Compounds or Compound synthesized from known Compound can be used as Compound represented by formula (A-13).

### Step 8

Compound represented by formula (A-14) can be synthesized from Compound represented by formula (A-13).

Reaction solvent includes N,N-dimethylformamide, dimethyl sulfoxide, a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of ester (example: methyl acetate, ethyl acetate and the like), a kind of ketone (example: acetone, methylethyl ketone and the like), a kind of nitrile (example: acetonitrile and the like), a kind of alcohol (example: methanol, ethanol, t-buthanol and the like), water and the like. These can be used solely or by mixture.

Reaction solvent is preferably N,N-dimethylformamide, a kind of ether (example: tetrahydrofuran, diethyl ether, dioxane, 1,2-dimethoxyethane and the like), a kind of nitrile (example: acetonitrile and the like), a kind of alcohol (example: methanol, ethanol, t-buthanol and the like) and the like. These can be used solely or by mixture.

Base includes, for example, metal hydride (example: sodium hydride and the like), metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, metal amide, organic amine (example: triethylamine, diisopropylethylamine, DBU, 2,6-lutidine and the like), pyridine and the like.

Base is preferably, for example, metal carbonate (example: sodium carbonate, calcium carbonate, cesium carbonate and the like), sodium hydrogen carbonate, organic amine (example: triethylamine, diisopropylethylamine, DBU, 2,6-lutidine and the like), pyridine and the like.

Reaction temperature is 0°C to reflux temperature of solvent, preferably room temperature to reflux temperature of solvent.

Reaction time is 0.5 to 24 hours, preferably 0.5 hours to 12 hours.

### Step 9

Compound represented by formula (A-15) can be synthesized from Compound represented by formula (A-14).

Step 9 is same as above Step 1.

### Step 10

Compound represented by formula (A-16) can be synthesized from Compound represented by formula (A-15).

Reaction solvent includes N,N-dimethylformamide, dimethyl sulfoxide, a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like), a kind of nitrile (example: acetonitrile and the like), a kind of alcohol (example: methanol, ethanol, t-buthanol and the like), water and the like. These can be used solely or by mixture.

Reaction solvent is preferably a kind of aromatic hydrocarbon (example: toluene, benzene, xylene and the like), a kind of ether (example: tetrahydrofuran, diethyl ether, dioxane, 1,2-dimethoxyethane and the like), a kind of nitrile (example: acetonitrile and the like), a kind of alcohol (example: methanol, ethanol, t-buthanol and the like), water and the like. These can be used solely or by mixture.

Reaction solvent is preferably a kind of aromatic hydrocarbon (example: toluene, benzene, xylene and the like), a kind of halogenated hydrocarbon (example: dichloromethane, chloroform, 1,2-dichloroethane and the like), a kind of ether (example: tetrahydrofuran, diethyl ether, dioxane, 1,2-dimethoxyethane and the like) and the like. These can be used solely or by mixture.

Base includes, for example, metal hydride (example: sodium hydride and the like), metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate, organic amine (example: triethylamine, diisopropylethylamine, DBU, 2,6-lutidine and the like), pyridine and the like.

Base is preferably, for example, metal hydroxide (example: sodium hydroxide, potassium hydroxide, lithium hydroxide, barium hydroxide and the like), metal carbonate (example: sodium carbonate, calcium carbonate, cesium carbonate and the like), metal alkoxide (example: sodium methoxide, sodium ethoxide, potassium t-butoxide and the like), sodium hydrogen carbonate and the like.

Reaction temperature is 0°C to reflux temperature of solvent, preferably 40°C to reflux temperature of solvent.

Reaction time is 0.5 hours to 12 hours, preferably 2 hours to 12 hours.

### Step 11

Compound represented by formula (I)-3 can be synthesized from Compound represented by formula (A-16).

Step 9 is same as above Step 5.

Since Compound of the present invention has protease inhibitory action, the Compound is useful as a therapeutic or preventive agent for virus infection disease (for example: AIDS).

The Compound of the present invention has not only protease inhibitory action but also is useful as a medicine and has any or all of the following excellent characteristics:
a) The compound is a weak inhibitor of CYP enzymes (for example: CYP1A2, CYP2C9, CYP2C19, CYP2D6, CYP3A4 and the like).
b) The compound demonstrates good pharmacokinetics, such as a high bioavailability, moderate clearance and the like.
c) The compound has a high metabolic stability, and half-life is long.
d) The compound has no irreversible inhibitory action against CYP enzymes (e.g., CYP3A4) when the concentration is within the range described in the present description as the measurement conditions.
e) The compound has no mutagenicity.
f) The compound is associated with a low cardiovascular risk.

Especially, above effects b) and/or c) can be achieved by introducing a side chain represented by -Y-Z having acid group into known HIV protease inhibitor with increasing protein binding, preferably albumin binding, and without decreasing anti-HIV action remarkably.

Thereby long acting of drug efficacy has increased as injection medicine.

A pharmaceutical composition of the present invention can be administered orally or parenterally. Methods for parenteral administration include dermal, subcutaneous, intravenous, intraarterial, intramuscular, intraperitoneal, transmucosal, inhalation, transnasal, ophthalmic, inner ear or vaginal administration and the like.

In case of oral administration, any forms, which are usually used, such as oral solid formulations (e.g., tablets, powders, granules, capsules, pills, films or the like), oral liquid formulations (e.g., suspension, emulsion, elixir, syrup, lemonade, spirit, aromatic water, extract, decoction, tincture or the like) and the like may prepared according to the usual method and administered. The tablets can be sugar-coated tablets, film-coated tablets, enteric-coating tablets, sustained-release tablets, troche tablets, sublingual tablets, buccal tablets, chewable tablets or orally disintegrated tablets. Powders and granules can be dry syrups. Capsules can be soft capsules, micro capsules or sustained-release capsules.

In case of parenteral administration, any forms, which are usually used, such as injections, drips, external preparations (e.g., ophthalmic drops, nasal drops, ear drops, aerosols, inhalations, lotion, infusion, liniment, mouthwash, enema, ointment, plaster, jelly, cream, patch, cataplasm, external powder, suppository or the like) and the like can be preferably administered. Injections can be emulsions whose type is O/W, W/O, O/W/O, W/O/W or the like.

The pharmaceutical composition may be manufactured by mixing an effective amount of the compound of the present invention with various pharmaceutical additives suitable for the formulation, such as excipients, binders, moistening agents, disintegrants, lubricants, diluents and the like. Furthermore, the pharmaceutical composition can be for pediatric patients, geriatric patients, serious cases or operations by appropriately changing the effective amount of the compound of the present invention, formulation and/or various pharmaceutical additives. The pediatric pharmaceutical compositions are preferably administered to patients under 12 or 15 years old. In addition, the pediatric pharmaceutical compositions can be administered to patients who are under 27 days old after the birth, 28 days to 23 months old after the birth, 2 to 11 years old, 12 to 16 years old, or 18 years old. The geriatric pharmaceutical compositions are preferably administered to patients who are 65 years old or over.

Although the dosage of a pharmaceutical composition of the present invention should be determined in consideration of the patient's age and body weight, the type and degree of diseases, the administration route and the like, a usual oral dosage is 0.05 to 100 and preferably 0.1 to 10 mg/kg/day. For parenteral administration, although the dosage highly varies with administration routes, a usual dosage is 0.005 to 10 and preferably 0.01 to 1 mg/kg/day. The dosage may be administered in one to several divisions per day.

The compound of the present invention can be used in combination of reverse transcriptase inhibitor, protease inhibitor, integrase inhibitor, CCR5 inhibitor or the like (hereinafter referred to as a co-administered drug) to increase the activity of the compound, reduce the dose of the compound, or the like. In this case, the timing of administration for a compound of the present invention and the co-administered drug is not limited. They can be administered to the subjects to be treated, at a time or at different times. Furthermore, a compound of the present invention and the co-administered drug can be administered as two formulations independently comprising each active ingredient or a single formulation comprising the both active ingredients.

The dose for co-administered drugs may be appropriately selected in reference to the clinical dose. The compounding ratio of the compounds of the present invention and co-administered drugs may be appropriately selected depending on the subject to be treated, administration route, disease to be treated, symptoms, combination of the drugs and the like. For administration in humans, for example, 1 part by weight of the compounds of the present invention may be used in combination with 0.01 to 100 parts by weight of co-administered drugs.

### EXAMPLES

The present invention will be described in more detail with reference to, but not limited to, the following Examples, Reference Examples and Test Examples.

In this description, meaning of each abbreviation is as follows:
Ac: acetyl
AIBN: azobisisobutyronitrile
BINAP: 2,2'-Bis(diphenylphosphino)-1,1'-binaphthyl
BOP: (Benzotriazol-1-yloxy)-tris(dimetylamino)phosphonium hexafluorophosphate
CDI: carbonyldiimidazole
DAST: N,N-diethylamino sulfurtrifluoride
DIBAL-H: diisobutylaluminium hydride
DIPEA: N,N-diisopropylethylamine
DMA: N,N-dimethylacetoamide
DMEAD: di-2-methoxyethyl azodicarboxylate
DMF: N,N-dimethylformamide
DMSO: dimethyl sulfoxide
HATU: O-(7-Azabenzotriazol-1-yl)-1,1,3,3-tetramethyluronium Hexafluorophosphate
HOBt: 1-hydroxybenzotriazole
HPLC: high performance liquid chromatography
MsCl: Methanesulfonyl Chloride
NaBH₄: sodium borohydride
NaN₃: sodium azide
NBS: N-Bromosuccinimide
NCS: N-Chlorosuccinimide
NIS: N-Iodosuccinimide
Pd/C: palladium on carbon
PdCl₂ (dppf): [1,1'-Bis(diphenylphosphino)ferrocene]palladium(II) Dichloride
PdCl₂ (dppf)CH₂ Cl₂: [1,1'-Bis(diphenylphosphino)ferrocene]palladium(II) Dichloride Dichloromethane Adduct
PdCl₂ (PPh₃)₂: Bis(triphenylphosphine)palladium(II) dichloride
Pd(OH)₂: Palladium(II) hydroxide
Pd(OAc)₂: : Palladium(II) Acetate
RT: retention time
TBAF: Tetrabutylammonium Fluoride
TBDPSCl: tert-Butyldiphenylchlorosilane
TFA: trifluoroacetic acid
THF: tetrahydrofuran
WSCD: Water Soluble Carbodiimide

NMR analysis of each example was performed by 300 MHz using DMSO-d₆ or CDCl₃.

LC/MS and HPLC are measured under the conditions as below:
(1) Condition A
   Column: ACQUITY UPLC(R) BEH C18 (1.7µm i.d. 2.1x50mm) (Waters) Flow rate: 0.8mL/min
   UV detection wavelength: 254nm
   Mobile phases: [A] is 0.1% formic acid solution, and [B] is 0.1% formic acid in acetonitrile solvent.
   Gradient: linear gradient of 5% to 100% solvent [B] for 3.5 minutes was performed, and 100 % solvent [B] was maintained for 0.5 minute.
(2) Condition B
   Column: Shim-pack XR-ODS (2.2µm, i.d. 50x3.0mm) (Shimadzu)
   Flow rate: 1.6mL/min
   UV detection wavelength: 254nm
   Mobile phases: [A] is 0.1% formic acid solution, and [B] is 0.1% formic acid in acetonitrile solvent.
   Gradient: linear gradient of 5% to 100% solvent [B] for 3 minutes was performed, and 100 % solvent [B] was maintained for 0.5 minute.
(3) Condition C
   Column: Gemini-NX (5µm, i.d. 4.6x50mm) (Phenomenex)
   Flow rate: 3.0mL/min
   UV detection wavelength: 254nm
   Mobile phases: [A] is 0.1% formic acid solution, and [B] is 0.1% formic acid in acetonitrile solvent.
   Gradient: linear gradient of 5% to 100% solvent [B] for 3.5 minutes was performed, and 100 % solvent [B] was maintained for 0.5 minute.
(4) Condition D
   Column: ACQUITY UPLC(R) BEH C18 (1.7µm i.d. 2.1x50mm) (Waters)
   Flow rate: 0.55mL/min
   UV detection wavelength: 254nm
   Mobile phases: [A] is 0.1% formic acid solution, and [B] is 0.1% formic acid in acetonitrile solvent.
   Gradient: linear gradient of 5% to 100% solvent [B] for 3 minutes was performed, and 100 % solvent [B] was maintained for 0.5 minute.
(5) Condition E
   Column: ACQUITY UPLC(R) BEH C18 (1.7µm i.d. 2.1x50mm) (Waters)
   Flow rate: 0.8mL/min
   UV detection wavelength: 254 nm
   Mobile phases: [A] is 10mM ammonium carbonate solution, and [B] is acetonitrile. Gradient: linear gradient of 5% to 100% solvent [B] for 3.5 minutes was performed, and 100 % solvent [B] was maintained for 0.5 minute.

### Reference example 1

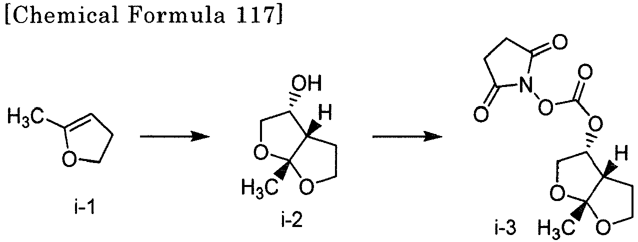

### Step 1-2

After Compound i-2 was synthesized from Compound i-1 by the method written in Organic Process Research & Development 2007, 11, 972-980, Compound i-3 was synthesized by the method written in J. Org. Chem. 2004, 69, 7822-7829.
Compound i-2;
¹H-NMR (CDCl₃)δ: 1.53 (s, 3H), 1.82 (s, br), 1.90-1.98 (1, m), 2.27-2.33 (m, 1H), 2.55 (m, 1H), 3.69 (m, 1H), 3.91-4.04 (m, 2H), 4.12 (m, 2H), 4.46 (m, 1H).
Compound i-3;
¹H-NMR (CDCl₃)δ: 1.52 (s, 3H), 2.03-2.14 (m, 2H), 2.79-2.84 (m, 1H), 2.86 (s, 4H), 3.95-4.03 (m, 3H), 4.13-4.17 (m, 1H), 5.25-5.30 (m, 1H).

### Reference example 2

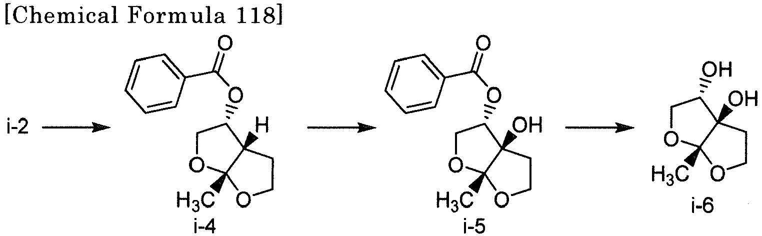

### Step 1

After triethylamine (7.87mL, 56.8mmol) and benzoyl chloride (4.40mL, 37.9mmol) were added into dichloromethane (30mL) solution of Compound i-2 (2.73g, 18.9mmol) under nitrogen atmosphere, the mixture was stirred overnight at room temperature. After saturated sodium bicarbonate aqueous solution was added into reaction mixture, the mixture was extracted with ethyl acetate. After organic layer was washed with water and brine, the mixture was dried with magnesium sulfate anhydrous, and solvent was removed in vacuo. The obtained residue was purified by silica gel column chromatography (hexane-ethyl acetate) to give Compound i-4 (3.7g, 70%).
¹H-NMR (CDCl₃)δ: 1.56 (3H, s), 1.93-2.18 (2H, m), 2.89 (1H, t, J = 8.6 Hz), 3.95-4.08 (3H, m), 4.23 (1H, t, J = 8.6 Hz), 5.35-5.66 (0H, m), 5.51 (1H, q, J = 7.4 Hz), 7.47 (2H, t, J = 7.4 Hz), 7.60 (1H, t, J = 7.4 Hz), 8.04 (2H, d, J = 7.4 Hz).

### Step 2

### [Seeds culture]

The aqueous solution (including 11.2g/L M9 Minimal salts 5x, 10g/L Casamino acids, 4g/L D-glucose, 0.02g/L Thymine, 0.02g/L CaCl₂·2H₂O, 0.5g/L MgSO₄·7H₂O and 0.1g/L Carbenicillin) was prepared. After sterilization by filtration (bore size: 0.2µm), sterilized solution (0.1g/ml FeSO₄·7H₂O) 280µl/L was added to be as seed cultural medium. The expressed vector (pET-CYP107Dhomolog -camA-camB) inserted CYP107D homolog gene (sequence number: 1) was transformed into E. coli BL21 (DE3) deletion of tolC (WO1200/8105513). The transformed E. coli was cultured at 25°C for 24 hours in the reciprocal shaker at 300rpm to be seeds.

### [Main culture]

The overnight auto induction system (SolutionI 20ml/L, SolutionII 50ml/L, SolutionIII 1ml/L; Merck) was added into the aqueous solution (including 11.2g/L M9 Minimal salts 5x, 10g/L Casamino acids, 10ml/L Glycerol, 0.02g/L Thymine, 0.08g/L 5-aminolevulinic acid and 0.1g/L Carbenicillin). After sterilization by filtration (bore size: 0.2µm), sterilized solution (0.1g/ml FeSO₄·7H₂O) 280µl/L was added to be as main cultural medium. After each 2.5mL of the seeds was inoculated to each the main cultural medium 250ml in four 2L wide-mouth Erlenmeyer flask, the transformed E. coli was cultured at 25°C for 24 hours in the rotary shaker at 200rpm.

### [Bacterial cellular reaction]

Sterilized 3.1ml of 80% glycerol and 250µL of DMSO solution of 20mg/ml Compound i-4 as substrate was added into 250ml each of four cultural solutions. The reaction is carried out at 30°C for 24 hours in the rotary shaker at 200rpm. Through the reaction, sampling was carried out. After same volume of ethanol was added into picked up cultured solution, the solution was centrifuged and supernatant was provided to reverse-phase UPLC. The conversion rate was calculated. Compound i-5 was produced 50.3% at 24 hours.

### [Extract and Purification]

1L of cultural solution was separated to supernatant and bacterial body by centrifugation. After 1L of ethyl acetate was added to supernatant, organic layer and water layer was separated. After 500ml ethanol was added to precipitate and the mixture was suspended, the suspension was centrifuged. After the solvent of the supernatant was removed, ethyl acetate was added and the organic layer and water layer was separated. After the both ethyl acetate layer was combined, the solution was evaporated to 1ml DMSO solution. Fractionation was carried out with acetonitrile (including 0.1% formic acid) under the condition of gradient of 20%-55% by Symmetry prep column (C18, 7µm, 19x150mm: Waters). The fraction including product was collected and lyophilized to give Compound i-5 (87.6mg) (yield 43.8%, purity 99.7%).
¹H-NMR (CDCl₃) δ: 1.26 (1H, t, J = 7.5 Hz), 1.51 (3H, s), 1.91-1.98 (1H, m), 2.36-2.42 (1H, m), 3.95-4.04 (4H, m), 4.28 (1H, t, J = 7.5 Hz), 5.25 (1H, t, J = 8.3 Hz), 7.49 (2H, t, J = 7.5 Hz), 7.64 (1H, t, J = 7.5 Hz), 8.06 (2H, d, J = 7.5 Hz).

### Step 3

After 2 mol/L sodium hydroxide aqueous solution (3.54mL, 7.07mmol) was added into methanol (20mL) solution of Compound i-5 (623mg, 2.35mmol) under ice-cold, the mixture was stirred at room temperature for 2 hours. After 2 mol/L hydrochloric acid was added into the reaction mixture, the solvent was removed in vacuo. The obtained residue was purified by silica gel column chromatography (chloroform-methanol) to give Compound i-6 (377mg, 99%).
¹H-NMR (MeOD) δ: 1.54 (3H, s), 2.03-2.15 (1H, m), 2.83-2.88 (1H, m), 3.69-3.77 (1H, m), 4.02-4.21 (3H, m), 4.38-4.48 (1H, m).

### Reference example 3

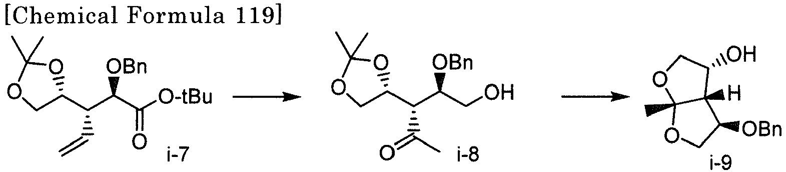

### Step 1

Lithium aluminium hydride (980mg, 25.8mmol) was added into THF (80ml) at 0°C under nitrogen atmosphere. After THF (40ml) solution of Compound i-7 (4.68g, 12.91mmol) was added by dropwise into the reaction mixture, the mixture was stirred at room temperature for 3 hours. The reaction mixture was diluted with THF under ice-cold. After sodium sulfate decahydrate was added, the mixture was stirred at room temperature for 5 hours. After the precipitate was filtrated, the solvent was removed in vacuo to give residue. After DMF (10ml), water (1.5ml), palladium (II) chloride (74mg, 0.417mmol) and copper chloride (I) (620mg, 6.26mmol) were added, the mixture was stirred under oxygen atmosphere at room temperature for an hour. After the obtained above residue was added, the mixture was further stirred at room temperature for 16 hours. After 2 mol/L hydrochloric acid was added, the mixture was extracted with ethyl acetate. After the organic layer was washed with water and brine, the organic layer was dried with magnesium sulfate anhydrous. The solvent was removed in vacuo. The obtained residue was purified by silica gel column chromatography (hexane-ethyl acetate) to give Compound i-8 (890mg, 66%). LC/MS (ESI): 326 (M+1)

### Step 2

After 4-methylbenzenesulfonic acid (2.79mg, 0.016mmol) and methanol (0.2ml) were added into dichloromethane (1mL) solution of Compound i-8 (50mg, 0.162mmol), the mixture was stirred at room temperature for 4 hours. After the reaction mixture was removed in vacuo, the obtained residue was purified by silica gel column chromatography (hexane-ethyl acetate) to give Compound i-9 (33mg, 81%).
LC/MS (ESI): 251 (M+1)

### Reference example 4

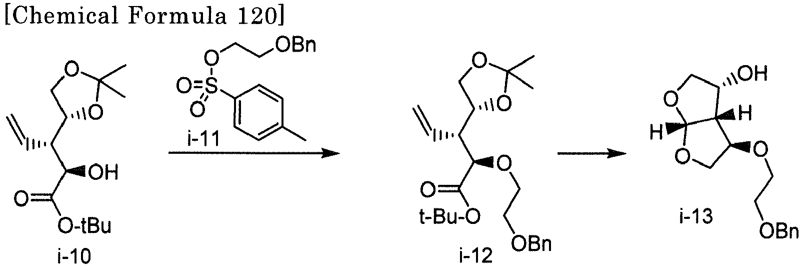

### Step 1

After Compound i-11 (2.87g, 9.36mmol), tetrabutylammonium iodide (230mg, 0.624mmol) and hexamethylphosphoric triamide (2.24g, 12.48mmol) were added into THF (17mL) solution of Compound i-10 (1.70g, 6.24mmol), the mixture was stirred at 0°C. After sodium hydride (299mg, 7.49mmol) was added into the reaction mixture, the mixture was stirred overnight at room temperature. After saturated ammonium chloride aqueous solution was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound i-12 (1.40g, 55%).
LC/MS (ESI): 429 (M+23)

### Step 2

After THF (1ml) solution of lithium aluminium hydride (24.3mg, 0.640mmol) was added by dropwise into THF solution of Compound i-12 (130mg, 0.320mmol) at 0°C under nitrogen atmosphere, the mixture was stirred at room temperature2 hours. , After the reaction mixture was diluted with THF under ice-cold, sodium sulfate decahydrate was added, and the mixture was stirred at room temperature for 5 hours. After the precipitate was filtrated, the solvent was removed to give residue. After dichloromethane (10ml) and methanol (2.5ml) were added into the obtained residue, the mixture was stirred at -78°C for 30 minutes under ozone atmosphere. After the reaction mixture was exchanged with nitrogen, dimethyl sulfide (98mg, 1.58mmol) was added, and the mixture was stirred at room temperature for 1.5 hours. After the solvent mixture was removed in vacuo, dichloromethane (10mL), methanol (2.5ml) and 4-methylbenzenesulfonic acid (5.99mg, 0.032mmol) were added, and the mixture was stirred overnight at room temperature. After the solvent mixture was removed in vacuo, the obtained residue was purified by silica gel column chromatography (hexane-ethyl acetate) to give Compound i-13 (44mg, 60%).
LC/MS (ESI): 303 (M+23)

### Reference example 5

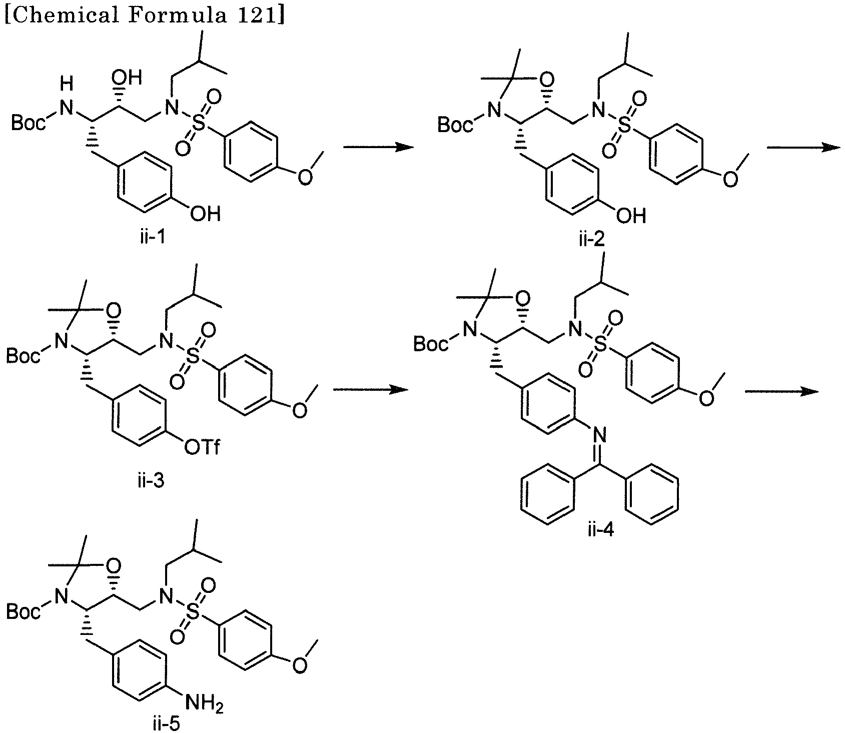

### Step 1

After 2,2-dimethoxypropane (2.49g, 23.9mmol) and tosilate hydrate (0.09g, 0.47mmol) were added into dichloromethane (25mL) solution of Compound ii-1 (2.5g, 4.78mmol), the mixture was refluxed for 5 hours. After water was added into the reaction mixture, the mixture was extracted with chloroform. After the organic layer was washed with water and saturated sodium bicarbonate aqueous solution, the mixture was dried with magnesium sulfate anhydrous. The solvent was removed in vacuo. The obtained residue was purified by silica gel column chromatography (hexane-ethyl acetate) to give Compound ii-2 (2.2g, 81%).
LC/MS (ESI): 563 (M+1)

### Step 2

After pyridine (170mg, 2.147mmol) and trifluoromethanesulfonic acid anhydrous (454mg, 1.610mmol) were added into dichloromethane (3mL) solution of Compound ii-2 (604mg, 1.073mmol) under ice-cold, the mixture was stirred at 0°C for 1.5 hours. After water was added into the reaction mixture, the mixture was extracted with ethyl acetate. After the organic layer was washed with 2 mol/L hydrochloric acid aqueous solution, water and brine, the mixture was dried with magnesium sulfate anhydrous. The solvent was removed in vacuo to give Compound ii-3 (727mg, 98%) as crude.
LC/MS (ESI): 695 (M+1)

### Step 3

After benzophenone imine (40.4mg, 0.223mmol), Pd (OAc)₂ (3.4mg, 0.015mmol), BINAP (18.5mg, 0.03mmol) and cesium carbonate (97mg, 0.297mmol) were added into THF (2mL) solution of Compound ii-3 (103.3mg, 0.149mmol) under nitrogen atmospher, the mixture was refluxed for 24 hours. After cooling the reaction mixture, the obtained solids by adding ethyl acetate were filtrated. The filtrate was removed in vacuo. The obtained residue was used to next reaction without purification.

### Step 4

After ammonium formate (94mg, 1.49mmol) and 10%Pd/C (50mg) were added into methanol (4mL) solution of Compound ii-4 (108mg, 0.149mmol), the mixture was refluxed for 5 hours. After cooling the reaction mixture, the obtained solids by adding dichloromethane were filtrated. The filtrated was removed in vacuo. The obtained residue was purified by silica gel column chromatography (hexane-ethyl acetate) to give Compound ii-5 (39.7mg, 48%).
LC/MS (ESI): 562 (M+1)

### Reference example 6

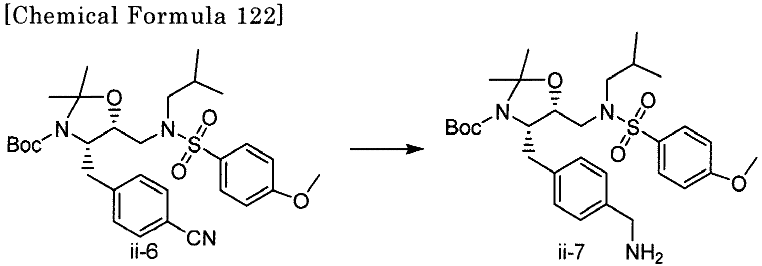

After DIBAL-H (1 mol/L hexane solution, 16.2mL, 16.2mmol) was added into THF (30mL) solution of Compound ii-6 (928mg, 1.62mmol) by dropwise at -20°C under nitrogen atmosphere, the mixture was stirred for 4.5 hours. Water and saturated potassium sodium tartrate aqueous solution were added to the reaction mixture. After the mixture was stirred at room temperature for 2 hours, the mixture was extracted with ethyl acetate. After the organic layer was washed with water and brine, the mixture was dried with sodium sulfate. The solvent was removed in vacuo. The obtained residue was purified by silica gel column chromatography (hexane-ethyl acetate to chloroform-methanol) to give Compound ii-7 (180mg, 19%). LC/MS (ESI): 576 (M+1)

### Reference example 7

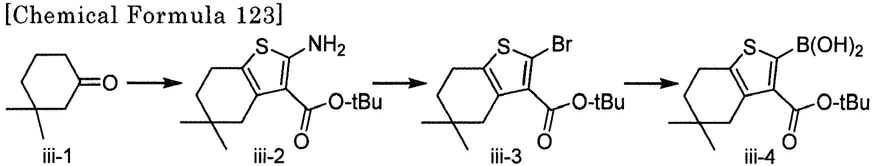

### Step 1-2

After Compound iii-2 was synthesized from Compound iii-1 by the method written in Bioorganic & Medicinal Chemistry Letters, 2010, 20 (19), 5753-5756, Compound iii-3 was synthesized by the method written in Tetrahedron Letters, 2012, 53 (52), 7135-7139.
Compound iii-2; LC/MS (ESI): 282 (M+1)
Compound iii-3; LC/MS (ESI): 345 (M+1)

### Step 3

Hexane solution of butyllithium (2.7 mol/L,11.9mL,32.02mmol) was added into THF (70mL) solution of Compound iii-3 (8.5g, 24.6mmol) by dropwise for 20 minutes at -70°C under nitrogen atmosphere. After triisopropyl borate (17.1mL,73.8mmol) was added into the reaction mixture, the mixture was stirred at room temperature for 2.5 hours. 2 mol/L hydrochloric acid was added into the reaction mixture. After the mixture was stirred for an hour at room temperature, the reaction mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound iii-4 (6.13g, 80%).
LC/MS (ESI): 311 (M+1)

### Reference example 8

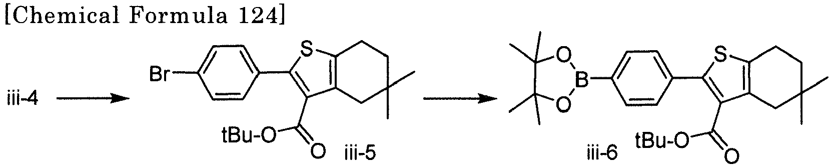

### Step 1

After 1-bromo-4-iodobenzene (293mg, 1.035mmol), PdCl₂ (dppf) (25.2mg, 0.034mmol) and 2 mol/L sodium carbonate aqueous solution (1.035mL, 2.07mmol) were added into THF (4mL) solution of Compound iii-4 (214.0mg, 0.690mmol) under nitrogen atmosphere, the mixture was reacted at 130°C for 30 minutes by microwave device. After water was added into the reaction mixture, the mixture was extracted with ethyl acetate and chloroform. After the organic layer was washed with brine, the solution was dried with magnesium sulfate anhydrous, and the solvent was removed in vacuo. The obtained residue was purified by silica gel column chromatography (hexane-ethyl acetate) to give Compound iii-5 (182.9mg, 62%). LC/MS (ESI): 421 (M+1)

### Step 2

Bis (pinacolato)diboron (331mg, 1.302mmol), PdCl₂ (dppf) CH₂Cl₂ (35.4mg, 0.043mmol) and potassium acetate (128mg, 1.302mmol) were added into DMF (2mL) solution of Compound iii-5 (182.9mg, 0.434mmol), the mixture was refluxed at 110°C for 4 hours. After water was added into the reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (hexane-ethyl acetate) to give Compound iii-6 (156.2mg, 76%).
LC/MS (ESI): 469 (M+1)

### Reference example 9

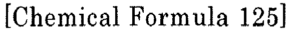

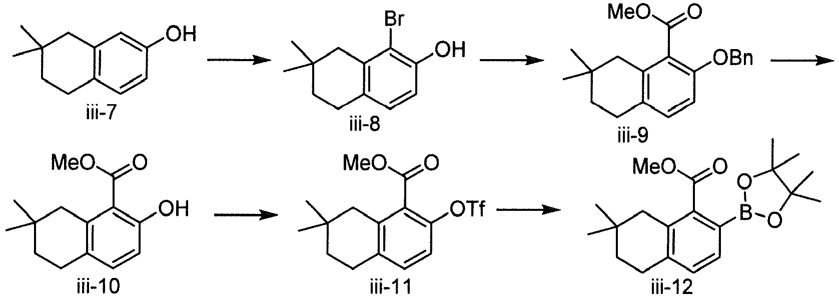

### Step 1

After NBS (5.58g, 31.3mmol) was added into dichloromethane (50mL) solution of Compound iii-8 (5.26g, 29.8mmol) under cooling at 0°C, the mixture was stirred at room temperature for 20 minutes. After water was added into the reaction mixture, the reaction mixture was extracted with chloroform. After the organic layer was washed with water, the mixture was dried with sodium sulfate, and the solvent was removed in vacuo. The obtained residue was purified by silica gel column chromatography (hexane-ethyl acetate) to give an isomeric mixture of Compound iii-8 (6.26g).
LC/MS (ESI): 255 (M+1)

### Step 2

After potassium carbonate (6.17g, 44.7mmol) and benzyl bromide (5.31mL, 44.7mmol) were added into DMF (57mL) solution of Compound iii-8 (5.7g, 22.34mmol), the mixture was stirred at room temperature for 1.5 hours. 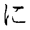 2 mol/L hydrochloric acid was added into the reaction mixture, the mixture was extracted with ethyl acetate. After organic layer was washed with water, the mixture was dried with sodium sulfate, and solvent was removed in vacuo. The obtained residue was purified by silica gel column chromatography (hexane-ethyl acetate) to give an isomeric mixture of Compound (7.88g).

Hexane solution (13.03mL, 33.9mmol) of 2.6 mol/L n-butyllithium was added into THF (78mL) solution of the obtained Compound under cooling at -78°C, and the mixture was stirred for 10 minutes. After methyl chloroformate (3.49mL, 45.2mmol) was added into the reaction mixture, the mixture was stirred at -78°C for 1.5 hours. After 2 mol/L hydrochloric acid was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give an isomeric mixture of Compound iii-9 (4.4g).
LC/MS (ESI): 325 (M+1)

### Step 3

10%Pd/C (440mg) was added into methanol (44mL) solution of Compound iii-9 (4.4g, 13.56mmol) under hydrogen atmosphere for 2 hours. After the reaction mixture was filtrated by Celite^{®}, the solvent was removed in vacuo. The obtained residue was purified by silica gel column chromatography (hexane-ethyl acetate) to give Compound iii-10 (1.8g, 57%).
LC/MS (ESI): 235 (M+1)

### Step 4

After pyridine (0.259mL, 3.2mmol), trifluoromethanesulfonic acidanhydrous (0.43mL, 2.56mmol) was added into dichloromethane (5mL) solution of Compound iii-10 (500mg, 2.134mmol) in an ice-bath under nitrogen atmosphere, the mixture was stirred at 0°C for an hour. After 2 mol/L hydrochloric acid was added into the reaction mixture, the mixture was extracted with chloroform. After the organic layer was washed with water, the mixture was dried with sodium the mixture was dried with sulfate, and the solvent was removed in vacuo. The obtained residue was purified by silica gel column chromatography (hexane-ethyl acetate) to give Compound iii-11 (800mg, 100%).
¹H-NMR (CDCl₃)δ: 0.98 (s, 6H), 1.57 (t, J=6.7, 2H), 2.54 (s, 2H), 2.83 (t, J=6.7,2H), 3.95 (s, 3H), 7.07 (d, J=8.8, 1H), 7.23 (d, J=8.8, 1H)

### Step 5

potassium acetate (241mg, 2.457mmol), 4,4,4',4',5,5,5',5'-octamethyl-2,2'-bi-1,3,2-dioxaborolane (312mg, 1.228mmol) and PdCl₂ (dppf) (59.9mg, 0.082mmol) were added into dioxane (3mL) solution of Compound iii-11 (300mg, 0.819mmol) under nitrogen atmosphere, and the mixture was stirred under heat reflux for 3 hours. After saturated ammonium chloride aqueous solution was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give Compound iii-12 (170mg, 60%). LC/MS (ESI): 367 (M+1)

### Reference example 10

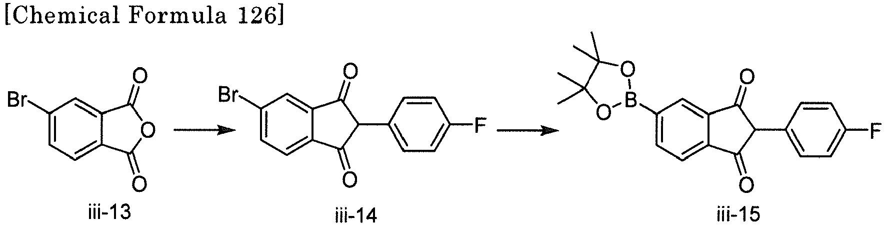

### Step 1

After 2-(4-fluorophenyl)acetic acid (3.39g, 22.03mmol) was added into acetic anhydride (15.61mL, 165mmol) solution of Compound iii-13 (5g, 22.03mmol), the mixture was stirred at 100°C for 30 minutes. After triethylamine (9.16mL, 66.1mmol) was added into the mixture, the mixture was stirred at 100°C for 30 minutes. After 2 mol/L hydrochloric acid was added into the reaction mixture, the mixture was extracted with ethyl acetate. After the organic layer was washed with water, the mixture was dried with sodium sulfate, and solvent was removed in vacuo. The obtained residue was purified by silica gel column chromatography (hexane-ethyl acetate) to give Compound iii-14 (3.5g, 50%).
LC/MS (ESI): 319 (M+1)

### Step 2

After potassium acetate (461mg, 4.7mmol), 4,4,4',4',5,5,5',5'-octamethyl-2,2'-bi-1,3,2-dioxaborolane (597mg, 2.35mmol), PdCl₂ (dppf) (115mg, 0.157mmol) were added into dioxane (5mL) solution of Compound iii-14 (500mg, 1.567mmol), the mixture was stirred under heat reflux for 1.5 hours. After 2 mol/L hydrochloric acid was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give Compound iii-15 (330mg, 58%). LC/MS (ESI): 364 (M-1)

### Reference example 11

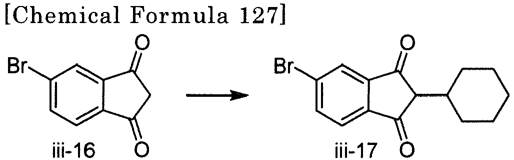

After cyclohexanone (0.145mL, 1.40mmol), L-proline (30.7mg, 0.267mmol) and 2,6-dimethyl-1,4-dihydropyridine-3,5-diethyl dicarboxylate (338mg, 1.33mmol) were added into DMSO (6mL) solution of Compound iii-16 (300mg, 1.33mmol) under nitrogen atmosphere, the mixture was stirred at room temperature for 24 hours. After 0.1 mol/L hydrochloric acid was added into the reaction mixture, the mixture was extracted with ethyl acetate. After the organic layer was washed with 0.1 mol/L hydrochloric acid, water and brine, the mixture was dried with sodium sulfate, and the solvent was removed in vacuo. The obtained residue was purified by silica gel column chromatography (hexane-ethyl acetate) to give Compound iii-17 (294mg, 72%). LC/MS (ESI): 305 (M-1)

### Reference example 12

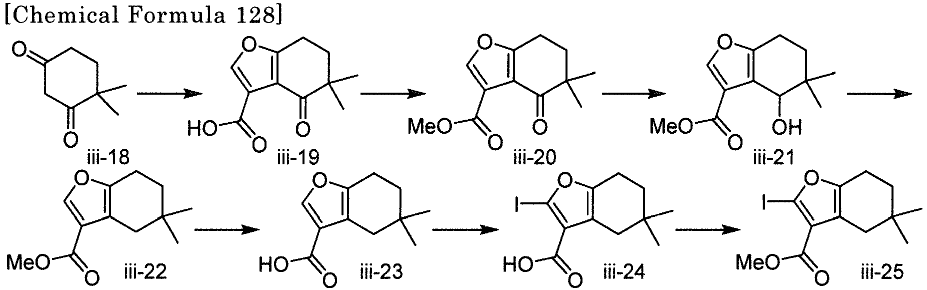

### Step 1

Compound iii-18 (6.8g, 48.5mmol) and 3-bromo-2-oxopropanoic acid (9.72g, 58.2mmol) were added into water (20mL) solution of sodium hydroxide (5.82g, 146mmol) in an ice-bath under nitrogen atmosphere, and stirred at 80°C for 4.5 hours. Concentrated hydrochloric acid (16.17mL, 194mmol) was added, and the mixture was stirred at 70°C for 1.5 hours. After water added into the reaction mixture, the mixture was extracted with chloroform. After the organic layer was washed with water, the mixture was dried with sodium sulfate, and the solvent was removed in vacuo. The obtained residue was purified by silica gel column chromatography (hexane-ethyl acetate) to give an isomeric mixture of Compound iii-19 (4.9g).
LC/MS (ESI): 209 (M+1)

### Step 2

After potassium carbonate (7.57g, 54.8mmol) and iodomethane (3.42mL, 54.8mmol) were added into DMF (17mL) solution of Compound iii-19 (5.7g, 27.4mmol), the mixture was stirred at room temperature for 40 minutes. After water was added into the reaction mixture, and the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give an isomeric mixture of Compound iii-20 (2.97g).
LC/MS (ESI): 223 (M+1)

### Step 3

NaBH₄ (494mg, 13.05mmol) was added into methanol (30mL) solution of Compound iii-20 (2.9g, 13.05mmol), and the mixture was stirred at room temperature for 20 minutes. After 2 mol/L hydrochloric acid was added into the reaction mixture, the mixture was extracted with chloroform. After the organic layer was washed with water, the mixture was dried with sodium sulfate, and solvent was removed in vacuo. The obtained residue was purified by silica gel column chromatography (hexane-ethyl acetate) to give Compound iii-21 (790mg, 3 Step, 6%).
LC/MS (ESI): 207 (M-17)

### Step 4

TFA (0.748mL, 9.71mmol) was added into dichloromethane (8mL) solution of Compound iii-21 (726mg, 3.24mmol) and triethylsilane (5.17mL, 32.4mmol) under ice-cold under nitrogen atmosphere, and the mixture was stirred at 0°C for 30 minutes. Further the mixture was stirred at room temperature for 5 hours. After saturated sodium hydrogen carbonate aqueous solution was added into the reaction mixture under ice-cold, the mixture was extracted with chloroform. After the organic layer was washed with water and brine, the mixture was dried with sodium sulfate, solvent was removed in vacuo. The obtained residue was purified by silica gel column chromatography (hexane-ethyl acetate) to give Compound iii-22 (528mg, 78%). ¹H-NMR (CDCl₃)δ: 1.0 (s, 6H), 1.56-1.59 (m, 2H), 2.44 (s, 2H), 2.57 (t, J=6.3, 2H), 3.80 (s, 3H), 7.85 (s, 1H)

### Step 5

2 mol/L sodium hydroxide aqueous solution (3.80mL, 7.61mmol) was added into methanol (12mL)-THF (2mL) solution of Compound iii-22 (528mg, 2.54mmol), and the mixture was stirred at 45°C for 8 hours. After 0.1 mol/L hydrochloric acid was added into the reaction mixture, the mixture was extracted with chloroform. After the organic layer the mixture was dried with sodium sulfate, the solvent was removed in vacuo to give Compound iii-23 (470mg, 95%).
LC/MS (ESI): 195 (M+H)

### Step 6

Hexane solution (4.51mL, 11.73mmol) of 2.6 mol/L n-butyllithium was added into THF (20mL) solution of Compound iii-23 (447mg, 2.30mmol) by dropwise under cooling at -78°C, and the mixture was stirred at 0°C for 1.5 hours. Iodine (1.81g, 7.13mmol) was added, and the mixture was stirred at 0°C for 15 minutes. After 0.1 mol/L hydrochloric acid was added into the reaction mixture, the mixture was extracted with chloroform. After the organic layer was dried with sodium sulfate, the solvent was removed in vacuo. The obtained residue was purified by silica gel column chromatography (hexane-ethyl acetate) to give Compound iii-24 (420mg, 57%). LC/MS (ESI): 321 (M+H)

### Step 7

After potassium carbonate (352mg, 2.55mmol) and iodomethane (0.239mL, 3.82mmol) were added into DMF (6mL) solution of Compound iii-24 (447mg, 2.30mmol) under nitrogen atmosphere at 0°C, the mixture was stirred at room temperature for 30 minutes. After 0.1 mol/L hydrochloric acid was added into the reaction mixture, the mixture was extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound iii-25 (417mg, 95%). LC/MS (ESI): 335 (M+H)

### Reference example 13

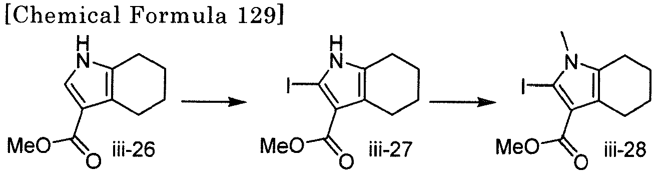

### Step 1

NIS (377mg, 1.67mmol) was added into DMF (2mL) solution of Compound iii-26 (200mg, 1.11mmol) under cooling in an ice-bath, and the mixture was stirred at 0°C for 20 minutes. After saturated sodium bicarbonate aqueous solution was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give Compound iii-27 (300mg, 88%). LC/MS (ESI): 306 (M+1)

### Step 2

Potassium carbonate (136mg, 0.983mmol) and iodomethane (0.061mL, 0.983mmol) were added into DMF (1mL) solution of Compound iii-27 (100mg, 0.328mmol), and the mixture was stirred at room temperature for 40 minutes. After saturated ammonium chloride aqueous solution was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give Compound iii-28 (27mg, 26%).
¹H-NMR (CDCl₃)δ: 1.66-1.83 (m, 4H), 2.55 (t, J=6.0Hz, 2H), 2.72 (t, J=6.0Hz 2H), 3.51 (s,3H), 3.81 (s, 3H)

### Reference example 14

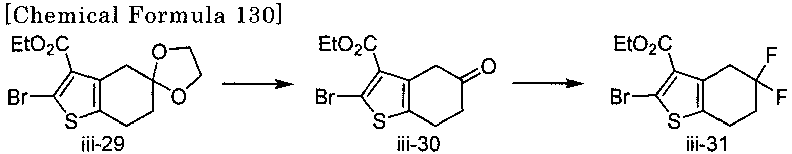

### Step 1

TFA (1mL) was added into dichloromethane (5mL) solution of Compound iii-29 (700mg, 2.02mmol), and the mixture was reacted at room temperature for 18 hours. After the solvent was removed in vacuo, the obtained residue was purified by silica gel column chromatography (hexane-ethyl acetate) to give Compound iii-30 (500mg, 82%).
LC/MS (ESI): 303 (M+1)

### Step 2

DAST (0.33mL, 2.47mmol) was added into dichloromethane (5mL) solution of Compound iii-30 (250mg, 0.83mmol) at -78°C. After the mixture was stirred reacted at -78°C for an hour, the mixture was reacted at room temperature for an hour. After saturated ammonium chloride aqueous solution was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound iii-31 (61mg, 23%).
LC/MS (ESI): 325 (M+1)

### Reference example 15

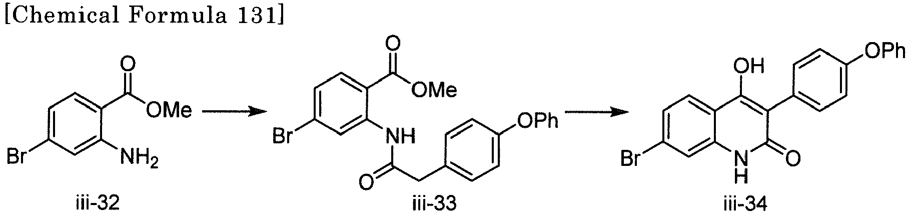

### Step 1

Triethylamine (6.03mL, 43.5mmol) and HATU (9.92g, 26.1mmol) were added into Compound iii-32 (4.96g, 21.7mmol), and the mixture was stirred at room temperature for 3 hours. After water was added into the reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (hexane-ethyl acetate) to give Compound iii-33 (5.76g, 60%). LC/MS (ESI): 440 (M+1)

### Step 2

1 mol/L sodium hexamethyldisilazane THF solution (41.9mL, 41.9mmol) was added into THF (115mL) solution of Compound iii-33 (5.76g, 13.1mmol), and the mixture was stirred at room temperature for 30 minutes. After methanol (30mL) was added into the reaction mixture, the solvent was removed in vacuo. After 1 mol/L hydrochloric acid (45mL) was added into the obtained residue, the mixture made to be acid. After the obtained white solids were filtrated, the solids were washed with diethyl ether to give Compound iii-34 (4.53g, 85%).
LC/MS (ESI): 408 (M+1)

### Reference example 16

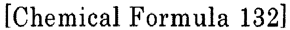

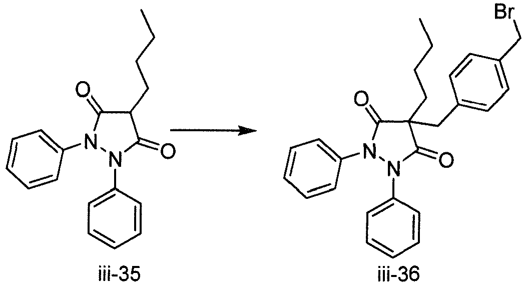

1,4-bis(bromomethyl)benzene (2.57g, 9.73mmol) and DIPEA (0.850mL, 4.86mmol) were added into DMA (15mL) solution of Compound iii-35 (1.00g, 3.24mmol), and the mixture was stirred and heated at 140°C for 30 minutes by microwave device. After water added into the reaction mixture, the mixture was extracted with ethyl acetate. After the organic layer was washed with brine, and the mixture was dried with magnesium sulfate anhydrous, the solvent was removed in vacuo. The obtained residue was purified by silica gel column chromatography (hexane-chloroform) to give Compound iii-36 (1.12g, 70%).
LC/MS (ESI): 491 (M+1)

### Reference example 17

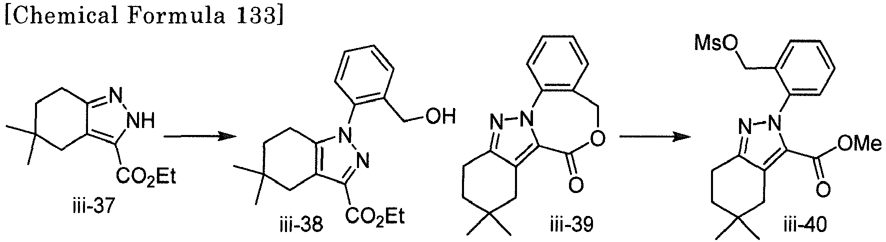

### Step 1

Compound iii-37 (240mg, 1.08mmol), 2-iodobenzylalchol (303mg, 1.30mmol), copper (I) iodide (41.1mg, 0.216mmol), N,N-dimethylglycinehydrochloric acid salt (60.3mg, 0.432mmol) and potassium carbonate (448mg, 3.24mmol) were dissolved into DMSO (4mL) under nitrogen atmosphere, and the mixture was stirred at 150°C for 45 minutes under microwave irradiation. After 0.1 mol/L hydrochloric acid was added into the reaction mixture, the mixture was extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound iii-38 (202mg, purity 70%) and Compound iii-39 (80mg, 26%).
Compound iii-38; LC/MS (ESI): 329 (M+1)
Compound iii-39; LC/MS (ESI): 283 (M+1)

### Step 2

Concentrated sulfuric acid (0.153mL, 2.87mmol) was added into methanol (10mL) solution of Compound iii-39 (270mg, 0.956mmol) under nitrogen atmosphere, and the mixture was stirred under heat reflux for 27 hours. After saturated sodium hydrogen carbonate was added into the reaction mixture, the mixture was extracted with ethyl acetate. After the organic layer was washed with water, the mixture was dried with sodium sulfate, and the solvent was removed in vacuo. After the obtained residue was dissolved into dichloromethane (4mL) and added into MsCl (0.112mL, 1.43mmol), triethylamine (0.212mL, 1.53mmol) was added, and the mixture was stirred at room temperature for 30 minutes. After 0.1 mol/L hydrochloric acid was added into the reaction mixture, the mixture was extracted with ethyl acetate. After the organic layer was washed with saturated sodium hydrogen carbonate aqueous solution and brine, the mixture was dried with sodium sulfate, and the solvent was removed in vacuo. The obtained residue was purified by silica gel column chromatography (hexane-ethyl acetate) to give Compound iii-40 (165mg, 44%). LC/MS (ESI): 393 (M+1)

### Reference example 18

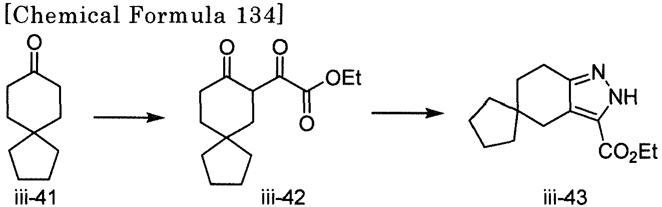

### Step 1

Ethanol solution (11mL) of Compound iii-41 (1.15g, 7.55mmol) was added into ethanol (44mL) solution of 20% sodium ethoxide (ethanol solution, 9.52mL, 22.7mmol) by dropwise at 0°C under nitrogen atmosphere. After diethyl oxalate (1.24mL,9.06mmol) was added, the mixture was stirred at room temperature for 6 hours. After the mixture was condensed under depressing, the mixture was poring to 0.1 mol/L hydrochloric acid/ice and extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound iii-42 (727mg, 38%).
¹H-NMR (CDCl₃)δ: 15.17 (s, 1H), 4.34 (q, J = 7.2 Hz, 2H), 2.49 (t, J = 6.8 Hz, 2H), 2.32 (s, 2H), 1.67-1.62 (m, 6H), 1.50-1.36 (m, 7H).

### Step 2

After hydrazine monohydrate (0.154mL, 3.16mmol) was added into ethanol (15mL) solution of Compound iii-42 (725mg, 2.87mmol) by dropwise at 0°C under nitrogen atmosphere, the mixture was stirred for an hour. After the reaction mixture was condensed under depressing, water was added, and the mixture was extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound iii-43 (685mg, 96%).
LC/MS (ESI): 249 (M+1)

### Reference example 19

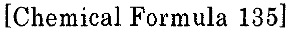

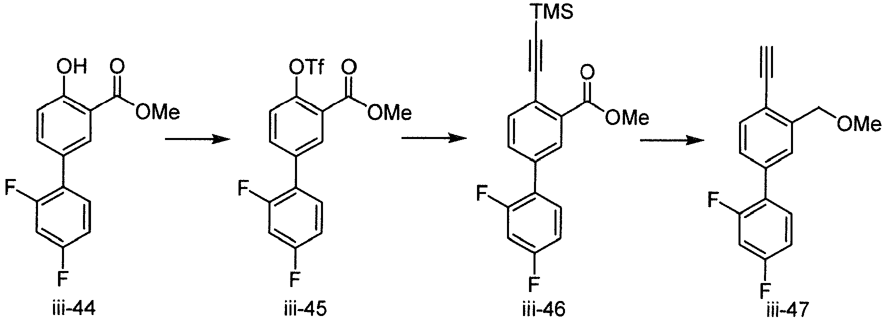

### Step 1

Pyridine (0.23mL, 2.84mmol) and trifluoromethanesulfonic acidanhydrous (0.38mL, 2.27mmol) were added into dichloromethane (30mL) solution of Compound iii-44 (500mg, 1.89mmol) under cooling in an ice-bath under nitrogen atmosphere, and the miture was stirred at room temperature for an hour. After 2 mol/L hydrochloric acid was added into the reaction mixture, the mixture was extracted with chloroform. After the organic layer was washed with water, the mixture was dried with sodium sulfate, and the solvent was removed in vacuo. The obtained residue was purified by silica gel column chromatography (hexane-ethyl acetate) to give Compound iii-45 (725mg, 97%).
¹H-NMR (CDCl₃)δ: 3.98 (s, 3H), 6.93-7.03 (m, 2H), 7.37-7.46 (m,2H), 7.75 (dt, J=8.7Hz, 2.0Hz, 1H), 8.19-8.20 (m, 1H)

### Step 2

Triethylamine (0.315mL, 2.27mmol), copper (I) iodide (14.4mg, 0.076mmol) and trimethylsilylacetylene (0.323mL, 2.27mmol) were added into THF (3mL) solution of Compound iii-45 (300mg, 0.757mmol) under nitrogen atmosphere, and the mixture was stirred at 60°C for 3.5 hours. After saturated ammonium chloride aqueous solution was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give Compound iii-46 as crude.

### Step 3

Potassium carbonate (740mg, 5.36mmol) was added into methanol (3mL) solution of crude Compound iii-46, and the mixture was stirred at room temperature for 30 minutes. After saturated ammonium chloride aqueous solution was added into the reaction mixture, the mixture was extracted with ethyl acetate. After the organic layer was washed with water, the mixture was dried with sodium sulfate, and solvent was removed in vacuo. The obtained residue was purified by silica gel column chromatography (hexane-ethyl acetate) to give Compound iii-47 (230mg, 2Step, 79%).
¹H-NMR (CDCl₃)δ: 3.45 (s, 1H), 3.95 (s, 3H), 6.91-7.01 (m, 2H), 7.40-7.46 (m,1H), 7.61-7.7 (m, 2H), 8.07-8.10 (m, 1H)

### Reference example 20

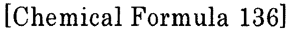

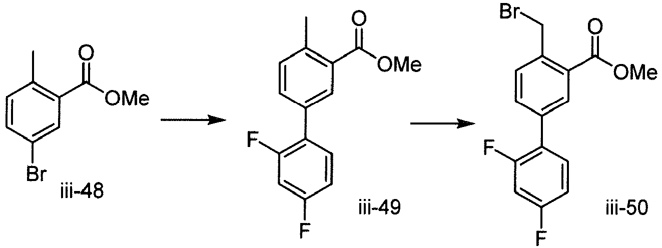

### Step 1

2 mol/L potassium carbonate aqueous solution (22.7mL, 45.4mmol), 2,4-difluorobenzeneboronic acid (7.17g, 45.4mmol) and Pd (PPh₃)₄ were added into DMF (50mL) solution of Compound iii-48 (5.2g, 22.7mmol) under nitrogen atmosphere, and the mixture was stirred at 100°C for 2 hours. After 2 mol/L hydrochloric acid was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give Compound iii-49 (5.6g, 94%).
LC/MS (ESI): 263 (M+1)

### Step 2

NBS (0.71g, 4.0mmol) was added into carbon tetrachloride (10mL solution of Compound iii-49 (1g, 3.81mmol) under nitrogen atmosphere, and the mixture was stirred under heat reflux for 2 hours. After water was added into the reaction mixture, the mixture was extracted with chloroform. After the organic layer was washed with water, the mixture was dried with sodium sulfate, and solvent was removed in vacuo. The obtained residue was purified by silica gel column chromatography (hexane-ethyl acetate) to give Compound iii-50 (1.03g, 79%). ¹H-NMR (CDCl₃)δ: 3.97 (s, 3H), 5.00 (s, 2H), 6.91-7.01 (m, 2H), 7.40-7.66 (m, 3H), 8.11 (s, 1H)

### Reference example 21

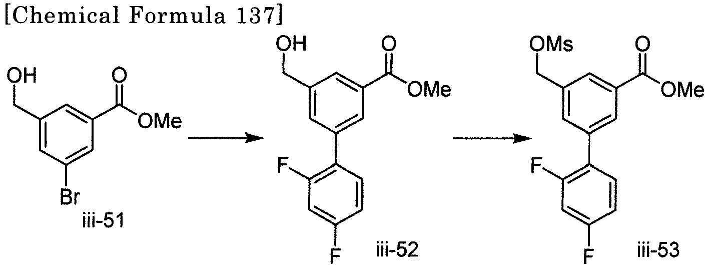

### Step 1

Compound iii-51 (300mg, 1.22mmol), (2,4-difluorophenyl)boronic acid (290mg, 1.84mmol), PdCl₂ (dppf) (90mg, 0.12mmol) and potassium phosphate (780mg, 3.67mmol) were dissolved into toluene (12mL) under nitrogen atmosphere under microwave irradiation, and the mixture was stirred at 140°C for 30 minutes. After the reaction mixture was filtrated by Celite^{®}, the solvent was removed in vacuo. The obtained residue was purified by silica gel column chromatography (hexane-ethyl acetate) to give Compound iii-52 (304mg, 89%).
¹H-NMR (CDCl₃) δ: 8.09-8.05 (m, 2H), 7.72 (d, J = 1.5 Hz, 1H), 7.46-7.40 (m, 1H), 7.00-6.91 (m, 2H), 4.82 (d, J = 5.8 Hz, 2H), 3.94 (s, 3H), 1.83 (t, J = 5.9 Hz, 1H).

### Step 2

MsCl (0.102mL, 1.31mmol) was added into triethylamine (0.226mL, 1.63mmol)-dichloromethane (6mL) solution of Compound iii-52 (303mg, 1.09mmol) under ice-cold, and the mixture was stirred for 3 hours under nitrogen atmosphere. After 0.1 mol/L hydrochloric acid was added into the reaction mixture, the mixture was extracted with ethyl acetate. After the organic layer was washed with saturated sodium hydrogen carbonate aqueous solution and brine, the mixture was dried with sodium sulfate, and the solvent was removed in vacuo. The obtained residue was purified by silica gel column chromatography (hexane-ethyl acetate) to give Compound iii-53 (350mg, 90%).
LC/MS (ESI): 379 (M+23)

### Reference example 22

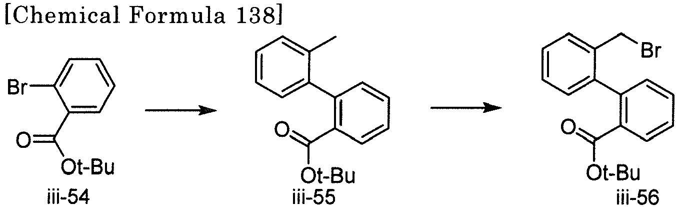

### Step 1

Compound iii-54 (800mg, 3.11mmol), PdCl₂ (dppf)CH₂Cl₂ (254mg, 0.331mmol) and 2 mol/L sodium carbonate aqueous solution (6.22mL, 12.5mmol) were added into THF (16mL) solution of o-tolylboronic acid (465mg, 3.42mmol), and the mixture was stirred for 30 minutes with heating to 130°C by microwave device. After brine was added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate) to give Compound iii-55 (674mg, 81%). Measurement condition A, HPLC: RT=2.92min

### Step 2

NBS (415mg, 2.53mmol) and AIBN (4.0mg, 0.025mmol) were added into carbon tetrachloride (6.6mL) solution of Compound iii-55 (465mg, 3.42mmol), and the mixture was stirred for an hour under heat reflux. After saturated sodium hydrogen carbonate aqueous solution was added into the reaction mixture, the mixture was extracted with chloroform. After the organic layer was dried with anhydrous sodium sulfate, the solvent was removed in vacuo. The obtained residue was purified by silica gel column chromatography (hexane-ethyl acetate) to give Compound iii-56 (822mg, 96%).
Measurement condition A, HPLC: RT=2.91min

### Reference example 23

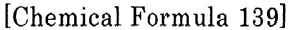

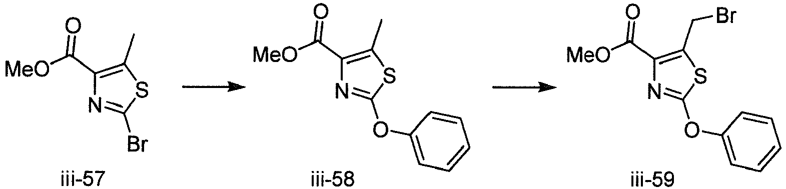

### Step 1

Phenol (518mg, 5.51mmol) and cesium carbonate (2.76g, 8.47mmol) were added into DMF (10.0mL) solution of Compound iii-57 (1.00g, 4.24mmol), and the mixture was stirred at 45°C for an hour. After the water was added into the reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (chloroform) to give Compound iii-58 (775mg, 73%).
LC/MS (ESI): 250 (M+1)

### Step 2

NBS (589mg, 3.31mmol) and benzoyl peroxide (15.3mg, 0.062mmol) were added into carbon tetrachloride (8.85mL) solution of Compound iii-58 (785mg, 3.15mmol), and the mixture was stirred for 30 minutes with heating to 100°C by microwave device. After saturated sodium hydrogen carbonate aqueous solution and water were added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate) to give Compound iii-59 (930mg, 90%). LC/MS (ESI): 328 (M+1)

### Reference example 24

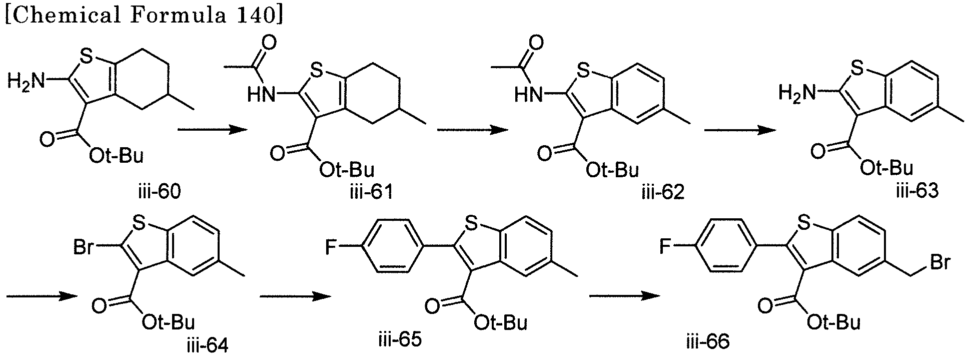

### Step 1

Triethylamine (4.67mL, 33.7mmol) and 4-dimethylaminopyridine (137mg, 1.12mmol) were added into THF (90mL) solution of Compound iii-60 (6.00g, 22.4mmol), and acetyl chloride (1.92mL, 26.9mmol) was added under ice-cold. The mixture was stirred at room temperature for 1.5 hours. After water was added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate) to give Compound iii-61 (3.2g, 46%).
LC/MS (ESI): 311 (M+1)

### Step 2

2,3-dichloro-5,6-dicyano-p-benzoquinone (5.64g, 24.8mmol) was added into 1,4-dioxane (64mL) solution of Compound iii-61 (3.2g, 10.3mmol), and the mixture was stirred at 70°C for 2 hours. After the reaction mixture was cooled to room temperature, insoluble matter was filtrated. After filtrated solvent was removed in vacuo, saturated sodium bicarbonate aqueous solution was added into the obtained residue and stirred. 2 mol/L hydrochloric acid was added, and the mixture made to be acid. The solution was extracted with chloroform. After the organic layer was dried with magnesium sulfate anhydrous, the solvent was removed in vacuo. After ethyl acetate and hexane were added into the obtained residue, the obtained solids were filtrated to give Compound iii-62 (2.48g, 79%).
LC/MS (ESI): 306 (M+1)

### Step 3

Pyrrolidine (6.72mL, 81.0mmol) was added into toluene (124mL) solution of Compound iii-62 (2.48g, 8.12mmol), and the mixture was stirred at 100°C for 20 hours. After the solvent was removed in vacuo, the obtained residue was purified by silica gel column chromatography (hexane-chloroform) to give Compound iii-63 (1.09g, 51%).
LC/MS (ESI): 264 (M+1)

### Step 4-6

Compound iii-66 was obtained by the same manner of synthesis above Reference example.
LC/MS (ESI): 421 (M+1)

### Reference example 25

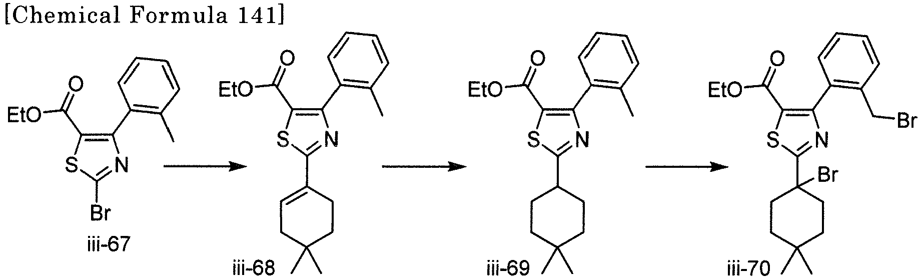

### Step 1

2-(4,4-dimethylcyclohex-1-ene-1-yl)-4, 4, 5, 5-tetramethyl-1, 3, 2-dioxaborolane (1738mg, 7.36mmol), PdCl₂ (dppf)CH₂Cl₂ (400mg, 0.490mmol) and 2 mol/L sodium carbonate aqueous solution (9.80mL, 19.6mmol) were added into THF (16mL) solution of Compound iii-67 (800mg, 2.45mmol). The mixture was stirred for an hour under heating to 120 to 130°C by microwave device. After water was added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate) to give Compound iii-68 (872mg, 100%).
LC/MS (ESI): 356 (M+1)

### Step 2

10%Pd/C (including 50% water) (31.7mg) was added into ethyl acetate (21mL) solution of Compound iii-68 (1.06g, 2.98mmol). The mixture was stirred at room temperature for 5 hours under hydrogen one atmosphere. After the reaction mixture was filtrated by Celite^{®}, the filtrated solvent was removed in vacuo. The obtained residue was purified by silica gel column chromatography (hexane-ethyl acetate) to give Compound iii-69 (623mg, 58%).
LC/MS (ESI): 358 (M+1)

### Step 3

NBS (296mg, 1.66mmol) and benzoyl peroxide (4.02mg, 0.017mmol) were added into carbon tetrachloride (30mL) solution of Compound iii-69 (594mg, 1.66mmol), and the mixture was stirred for 4 hours with heat reflux. After saturated sodium hydrogen carbonate aqueous solution and water were added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate).

Further NBS (130mg, 0.729mmol) and benzoyl peroxide (1.76mg, 0.007mmol) were added into carbon tetrachloride (16mL) solution of the obtained Compound (318mg, 0.729mmol), and the mixture was stirred for 5 hours with heat reflux. After saturated sodium hydrogen carbonate aqueous solution and water were added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate) to give Compound iii-70 (221mg, 2 Step, 49%).
LC/MS (ESI): 514 (M+1)

### Reference example 26

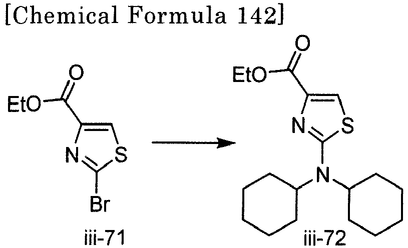

1,4-dioxane (20mL), Compound iii-71 (1.00g, 4.24mmol), cesium carbonate (8.28g, 25.4mmol), 2-dicyclohexylphosphino-2',6'-diisopropoxybiphenyl (592mg, 1.27mmol) and Pd (OAc)₂ (190mg, 0.848mmol) were added into dicyclohexylamine (1844mg, 10.16mmol), and the mixture was stirred for an hour with heating to 120 to 130°C by microwave device. After water was added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate) to give Compound iii-72 (290mg, 20%).
LC/MS (ESI): 337 (M+1)

### Reference example 27

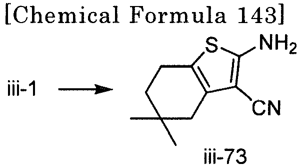

Sulfur (2.03g, 63.4mmol) was added into ethanol (40mL) solution of Compound iii-1 (8.0g, 63.4mmol), malononitrile (6.28g, 95mmol) and diethylamine (3.31mL, 31.7mmol). After the mixture was stirred overnight at room temperature, the mixture was stirred at 50°C for 5 hours. After water was added into the reaction mixture, the mixture was extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound iii-73 (4.55g, 35%). LC/MS (ESI): 207 (M+1)

### Reference example 28

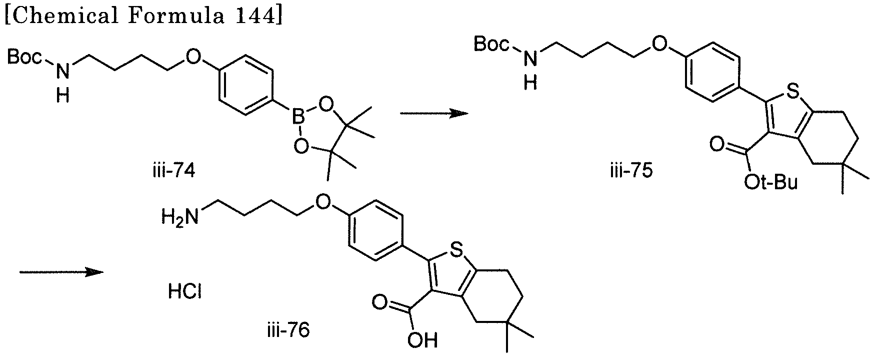

### Step 1

1,4-dioxane (15mL), Compound iii-3 (773mg, 2.24mmol), tetrakistriphenylphosphine palladium complex (1.29g, 1.12mmol) and potassium phosphate (1.43g, 6.72mmol) were added into Compound iii-74 (876mg, 2.24mmol). The mixture was stirred for an hour with heating to 125°C by using microwave device. After brine was added into the reaction mixture, the mixture was extracted with chloroform. After the organic layer was dried with magnesium sulfate anhydrous, the solvent was removed in vacuo. The obtained residue was purified by silica gel column chromatography (hexane-ethyl acetate) to give Compound iii-75 (448mg, 38%).
Measurement condition A, HPLC: RT=3.45min

### Step 2

4 mol/L hydrochloric acid dioxane solution (4.6mL, 18.4mmol) was added into Compound iii-75 (488mg, 0.921mmol), and the mixture was stirred at room temperature for 18 hours. The solvent was removed in vacuo to give Compound iii-76 (376mg, 99.5%).
Measurement conditionA, HPLC: RT=1.84min

### Reference example 29

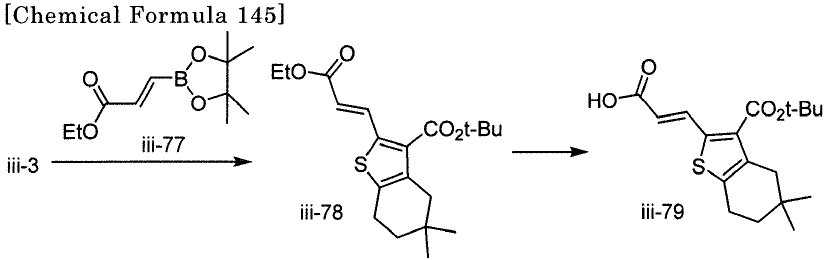

### Step 1

Compound iii-77 (1.31g, 5.79mmol), PdCl₂ (dppf)CH₂Cl₂ (424mg, 0.58mmol) and 2 mol/L sodium carbonate aqueous solution (11.6mL, 23.2mmol) were added into THF (15mL) solution of Compound iii-3 (2.00g, 5.79mmol), and the mixture was stirred for 5 hours under heat reflux under nitrogen current. After water was added into the reaction mixture, the mixture was extracted with ethyl acetate. After the organic layer was washed with water, saturated sodium bicarbonate aqueous solution, the mixture was dried with magnesium sulfate anhydrous, and the solvent was removed in vacuo. The obtained residue was purified by silica gel column chromatography (hexane-ethyl acetate) to give Compound iii-78 (1.82g, 86%). LC/MS (ESI): 382 (M+18)

### Step 2

2 mol/L sodium hydroxide aqueous solution (5mL, 9.99mmol) was added into methanol (10mL) solution of Compound iii-78 (1.82g, 4.99mmol), and the mixture was stirred at room temperature for 3 hours under hydrogen flow. After 2 mol/L hydrochloric acid aqueous solution was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound iii-79 (1.53g, 91%). LC/MS (ESI): 354 (M+18)

### Reference example 30

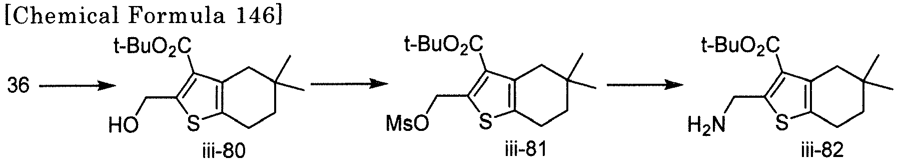

### Step 1

NaBH₄ (51mg, 1.36mmol) was added into methanol (10mL solution of Compound 36 (400mg, 1.36mmol), and the mixture was stirred at 0°C for 10 minutes further at room temperature for an hour. After saturated ammonium chloride aqueous was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound iii-80 (375mg, 93%).
LC/MS (ESI): 297 (M+1)

### Step 2

Triethylamine (0.33mL, 2.36mmol) and MsCl (203mg, 1.77mmol) were added into dichloromethane (10mL solution of Compound iii-80 (350mg, 1.18mmol), and the mixture was stirred at 0°C for an hour. After saturated ammonium chloride aqueous solution was added into the reaction mixture, the mixture was extracted with ethyl acetate. After the organic layer was washed with water and brine, the mixture was dried with magnesium sulfate anhydrous, and the solvent was removed in vacuo to give Compound iii-81.
LC/MS (ESI): 375 (M+1)

### Step 3

Compound iii-81 was dissolved into DMF (10mL), and NaN₃ (230mg, 3.54mmol) was added. The mixture was stirred at room temperature for 5 hours. After water was added into the reaction mixture, the mixture was extracted with ethyl acetate. After the organic layer was washed with water and brine, the mixture was dried with magnesium sulfate anhydrous, and solvent was removed in vacuo.

The obtained residue was dissolved into ethyl acetate (10mL), and Pd (OH)₂ (38mg, 10%wt) was added. The mixture was stirred at room temperature for 3 hours under hydrogen flow. After the reaction mixture was filtrated, the solvent was removed in vacuo. The obtained residue was purified by silica gel column chromatography (hexane-ethyl acetate) to give Compound iii-82 (317mg, 91%). LC/MS (ESI): 296 (M+1)

### Reference example 31

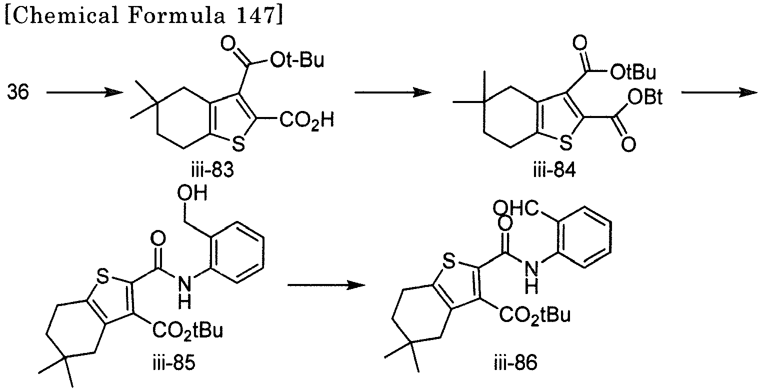

### Step 1

2-methyl-2-butene (15mL) and sodium chlorite (4.61g, 50.9mmol)-sodium dihydrogen phosphate (4.69g, 39.1mmol) aqueous solution (20mL) were added into 1,4-dioxane (75mL) suspension of Compound 36 (5.00g, 17.0mmol), and the mixture was stirred at room temperature for 18 hours. After water was added into the reaction mixture, the mixture was extracted with ethyl acetate. After the organic layer was washed with brine and dried with anhydrous sodium sulfate, the solvent was removed in vacuo. The obtained residue was purified by silica gel column chromatography (chloroform-ethyl acetate) to give Compound iii-83 (4.37g, 83%). ¹H-NMR (CDCl₃) δ: 1.00 (s, 6H), 1.59 (t, J = 6.4 Hz, 2H), 1.64 (s, 9H), 2.55 (s, 2H), 2.81 (t, J = 6.4 Hz, 2H), 14.28 (s, 1H).

### Step 2

HOBt (479mg, 3.54mmol) and WSCDhydrochloric acid salt (741mg, 3.87mmol) were added into dichloromethane (10mL suspension of Compound iii-83 (1.00g, 3.22mmol) under ice-cold, and the mixture was stirred at room temperature for an hour. After the reaction mixture was diluted with ethyl acetate, 1 mol/L sodium hydroxide aqueous solution was added, the mixture was extracted with ethyl acetate. After the organic layer was washed with water and dried with magnesium sulfate anhydrous, the solvent was removed in vacuo. The obtained residue was purified by silica gel column chromatography (hexane-ethyl acetate) to give Compound iii-84 (864mg, 63%).
LC/MS (ESI): 428 (M+1)

### Step 3

(2-aminophenyl)methanol (79.8mg, 0.648mmol) was added into DMA (2mL) solution of Compound iii-84 (222mg, 0.518mmol), and the mixture was stirred at 80°C for 5 hours. After water was added into the reaction mixture, the mixture was extracted with ethyl acetate. After the organic layer was washed with water and brine, and dried with anhydrous sodium sulfate, the solvent was removed in vacuo. The obtained residue was purified by silica gel column chromatography (hexane-ethyl acetate) to give Compound iii-85 (215mg, 69%).
LC/MS (ESI): 416 (M+1)

### Step 4

1,1,1-triacetoxy-1,1-dihydro-1,2-benzoiodoquisole-3-(1H)-one (159mg, 0.375mmol) was added into dichloromethane (10mL suspension of Compound iii-85 (120mg, 17.0mml), and the mixture was stirred for 2 hours under ice-cold. After 5% thiosodium sulfate aqueous solution was added into the reaction mixture, the mixture was extracted with ethyl acetate. After the organic layer was washed with brine, and dried with anhydrous sodium sulfate, the solvent was removed in vacuo. The obtained residue was purified by silica gel column chromatography (hexane-ethyl acetate) to give Compound iii-86 (106mg, 89%).
LC/MS (ESI): 414 (M+1)

### Reference example 32

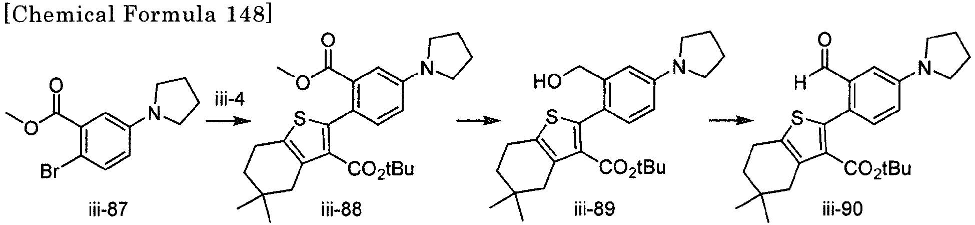

### Step 1

Compound iii-88 was obtained by same manner of synthesis of Compound iii-78.
¹H-NMR (CDCl₃) δ: 1.02 (s, 6H), 1.22 (s, 9H), 1.60 (t, J = 6.3 Hz, 2H), 2.00-2.07 (m, 4H), 2.63 (s, 2H), 2.74 (t, J = 6.3 Hz, 2H), 3.30-3.37 (m, 4H), 3.66 (s, 3H), 6.64 (d, J = 8.3 Hz, 1H), 7.11 (s, 1H), 7.16 (d, J = 8.3 Hz, 1H).

### Step 2

1.02m mol/L DIBAL-Hhexane solution (2.64mL, 2.70mmol) was added into THF (10mL) solution of Compound iii-88 (422mg, 0.099mmol) under ice-cold, and the mixture was stirred at 0°C for 1.5 hours. After L-(+)-potassium sodium tartrate aqueous solution was added into the reaction mixture, the mixture was stirred at room temperature for 2 hours. the mixture was extracted with ethyl acetate. After the organic layer was washed with brine and dried with anhydrous sodium sulfate, the solvent was removed in vacuo. The obtained residue was purified by silica gel column chromatography (hexane-ethyl acetate) to give Compound iii-89 (303mg, 76%). ¹H-NMR (CDCl₃) δ: 1.03 (s, 6H), 1.30 (s, 9H), 1.60 (t, J = 5.8 Hz, 2H), 1.97-2.05 (m, 4H), 2.57 (s, 2H), 2.73 (t, J = 5.8 Hz, 2H), 3.29-3.36 (m, 4H), 4.48 (s, 2H), 6.47 (dd, J = 8.3, 1.5 Hz, 1H), 6.69 (d, J = 1.5 Hz, 1H), 7.06 (d, J = 8.3 Hz, 1H).

### Step 3

1,1,1-triacetoxy-1,1-dihydro-1,2-benzoiodoquisole-3-(1H)-one (187mg, 0.442mmol) was added into dichloromethane (1mL) solution of Compound iii-89 (150mg, 0.340mml) under ice-cold, and the mixture was stirred at 0°C for an hour. After saturated sodium hydrogen carbonate aqueous solution was added into the reaction mixture, the mixture was extracted with chloroform. After the organic layer was washed with brine, and dried with anhydrous sodium sulfate, the solvent was removed in vacuo. The obtained residue was purified by silica gel column chromatography (hexane-ethyl acetate) to give Compound iii-90 (85.4mg, 57%). ¹ H-NMR (CDCl₃) δ: 1.04 (s, 6H), 1.22 (s, 9H), 1.62 (t, J = 6.4 Hz, 2H), 2.00-2.07 (m, 4H), 2.63 (s, 2H), 2.76 (t, J = 6.4 Hz, 2H), 3.32-3.39 (m, 4H), 6.75 (dd, J = 8.3, 2.8 Hz, 1H), 7.10 (d, J = 2.8 Hz, 1H), 7.22 (d, J = 8.3 Hz, 1H), 9.93 (s, 1H).

### Reference example 33

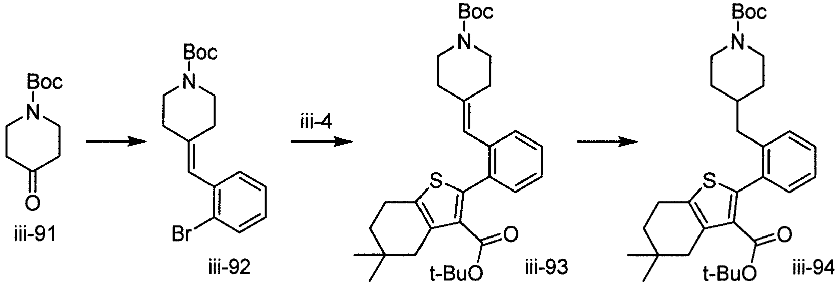

### Step 1

60% sodium hydride (77.0mg, 1.93mmol) was added into THF (5mL) suspension of (2-bromobenzyl)triphenylphosphonium bromide (990mg, 1.93mmol), and the mixture was stirred at room temperature for 2 hours. After Compound iii-91 (350mg, 1.76mmol) was added into the reaction mixture, the mixture was stirred for 17 hours under heating reflux. After saturated ammonium chloride aqueous solution was added into the reaction mixture, the mixture was extracted with ethyl acetate. After the organic layer was washed with water and brine, and dried with magnesium sulfate anhydrous, the solvent was removed in vacuo. The obtained residue was purified by silica gel column chromatography (hexane-ethyl acetate) to give Compound iii-92 (337mg, 55%).
¹H-NMR (CDCl₃) δ: 1.47 (s, 9H), 2.28 (dd, J = 5.5, 5.5 Hz, 2H), 2.37 (dd, J = 5.5, 5.5 Hz, 2H), 3.40 (dd, J = 5.5, 5.5 Hz, 2H), 3.53 (dd, J = 5.5, 5.5 Hz, 2H), 6.31 (s, 1H), 7.10 (dd, J = 7.9, 7.0 Hz, 1H), 7.17 (d, J = 7.4 Hz, 1H), 7.23-7.28 (m, 1H), 7.57 (d, J = 7.9 Hz, 1H).

### Step 2

Compound iii-93 was obtained by the same manner of synthesis of Compound iii-78.
¹ H-NMR (CDCl₃) δ: 1.02 (s, 6H), 1.19 (s, 9H), 1.45 (s, 9H), 1.60 (t, J = 6.4 Hz, 2H), 2.14-2.28 (m, 4H), 2.61 (s, 2H), 2.74 (t, J = 6.4 Hz, 2H), 3.18-3.27 (m, 2H), 3.32-3.42 (m, 2H), 6.18 (s, 1H), 7.16-7.31 (m, 4H).

### Step 3

10% Palladium on carbon (40.0mg, 0.018mmol) was added into methanol (1mL)-THF (1mL) solution of Compound iii-93 (135mg, 0.251mmol), and the mixture was stirred at room temperature for 2.5 hours under hydrogen atmosphere. After insoluble matter was removed by filtration, the solvent was removed in vacuo to give Compound iii-94 (143mg).
LC/MS (ESI): 540 (M+1)

### Example 1

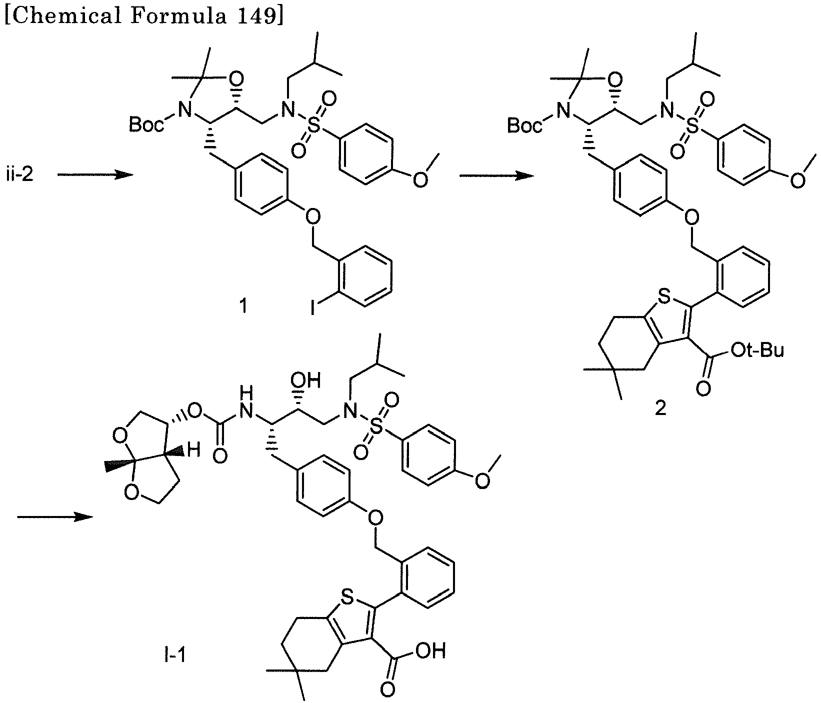

### Step 1

2-Iodobenzylbromide (208mg, 0.70mmol) and cesium carbonate (228mg, 0.70mmol) were added into acetonitrile (5mL) solution of Compound ii-2 (263mg, 0.46mmol), and the mixture was stirred for 2 hours under reflux. After cooling, ethyl acetate was added into the reaction mixture, the obtained solids were removed by filtration, and the filtrate was removed in vacuo. The obtained residue was purified by silica gel column chromatography (hexane-ethyl acetate) to give Compound 1 (348mg, 95%).
LC/MS (ESI): 779 (M+1)

### Step 2

Compound iii-4 (51mg, 0.16mmol), PdCl₂ (dppf)CH₂Cl₂ (13mg, 0.017mmol) and 2 mol/L sodium carbonate aqueous solution (0.33mL, 0.67mmol) were added into THF (4mL) solution of Compound 1 (130mg, 0.16mmol) under nitrogen atmosphere, and the mixture was reacted at 130°C for 30 minutes by using microwave device. After water was added into reaction mixture, the mixture was extracted with ethyl acetate. After the organic layer was washed with water and saturated sodium bicarbonate aqueous solution, the mixture was dried with magnesium sulfate anhydrous, and the solvent was removed in vacuo. The obtained residue was purified by silica gel column chromatography (hexane-ethyl acetate) to give Compound 2 (117mg, 76%). LC/MS (ESI): 934 (M+18)

### Step 3

TFA (0.5mL) was added into dichloromethane (2mL) solution of Compound 2 (117mg, 0.13mmol), and the mixture was reacted at room temperature for 4 hours. After the solvent of the reaction mixture was removed, dichloromethane (2mL), triethylamine (0.177mL, 1.27mmol) and Compound i-3 (43mg, 0.15mmol) were added into the obtained residue. After the mixture was stirred overnight at room temperature, the solvent of the reaction mixture was removed in vacuo. The obtained residue was purified by silica gel column chromatography (hexane-ethyl acetate) to give Compound I-1 (93mg, 81%).
Measurement condition: A, LC/MS (ESI): 908 (M+18)

### Example 2

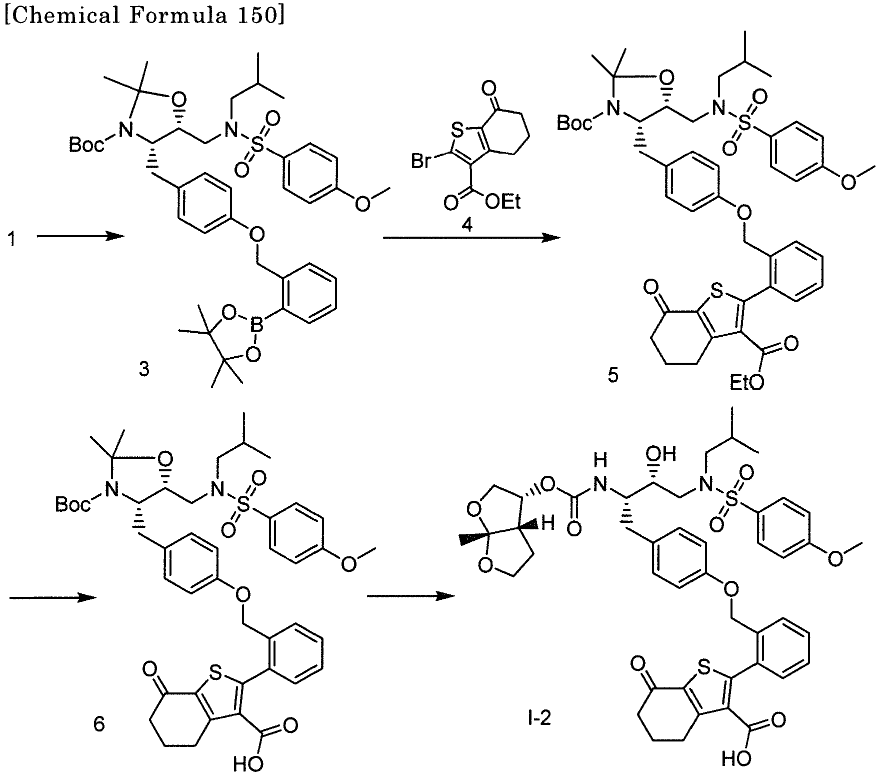

### Step 1

Potassium acetate (454mg, 4.62mmol), bis (pinacolato)diboron (978mg, 3.85mmol) and PdCl₂ (dppf)CH₂Cl₂ (113mg, 0.154mmol) were added into DMF (12mL) solution of Compound 1 (1.2g, 1.54mmol) under nitrogen atmosphere, and the mixture was stirred with heating at 80°C for 3 hours. After cooling the reaction mixture, the mixture was diluted with ethyl acetate and water, after that, the mixture was filtrated by Celite^{®}. After filtrate was extracted with ethyl acetate and the organic layer was washed with water and brine, the mixture was dried with magnesium sulfate anhydrous, and the solvent was removed in vacuo. The obtained residue was purified by silica gel column chromatography (hexane-ethyl acetate) to give Compound 3 (1.18g, 98%).
LC/MS (ESI): 779 (M+1)

### Step 2

Compound 4 (39.4mg, 0.130mmol), PdCl₂ (dppf)CH₂Cl₂ (9.65mg, 0.012mmol) and 2 mol/L sodium carbonate aqueous solution (0.236mL, 0.473mmol) were added into THF (1mL) solution of Compound 3 (92mg, 0.118mmol), and the mixture was reacted at 130°C for 30 minutes by using a microwave device. After water was added into the reaction mixture, the mixture was extracted with ethyl acetate. After the organic layer was washed with water and brine, the mixture was dried with magnesium sulfate anhydrous, and solvent was removed in vacuo. The obtained residue was purified by silica gel column chromatography (hexane-ethyl acetate) to give Compound 5 (77mg, 75%).
LC/MS (ESI): 875 (M+1)

### Step 3

Compound 6 was obtained by the same synthesis method of Compound iii-23. LC/MS (ESI): 847 (M+1)

### Step 4

Compound I-2 was obtained by the same synthesis method of Compound I-1. Measurement condition: A, LC/MS (ESI): 877 (M+1)

### Example 3

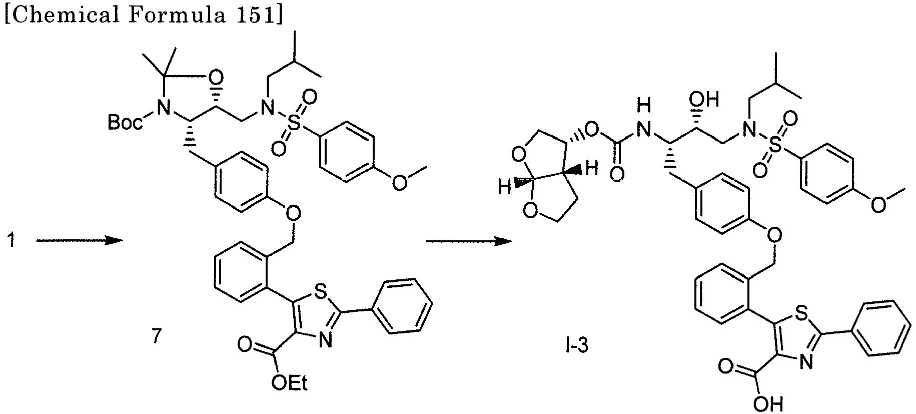

### Step 1

DMF (5.0mL), Compound 1 (250mg, 0.321mmol), cesium carbonate (314mg, 0.963mmol), 2-dicyclohexylphosphino-2',6'-diisopropoxybiphenyl (30.0mg, 0.064mmol) and Pd (OAc)₂ (7.21mg, 0.032mmol) were added into ethyl 2-phenylthiazole-4-carboxylate (150mg, 0.642mmol), and the mixture was stirred at 140°C for 30 minutes by using a microwave device. After water was added into the reaction mixture, the mixture was extracted with ethyl acetate. After the organic layer was dried with magnesium sulfate anhydrous, the solvent was removed in vacuo. The obtained residue was purified by silica gel column chromatography (chloroform) to give Compound 7 (132mg, 47%).
LC/MS (ESI): 885 (M+1)

### Step 2

Compound I-3 was obtained by the same synthesis method of Compound 1-2. Measurement condition: A, LC/MS (ESI): 872 (M+1)

### Example 4

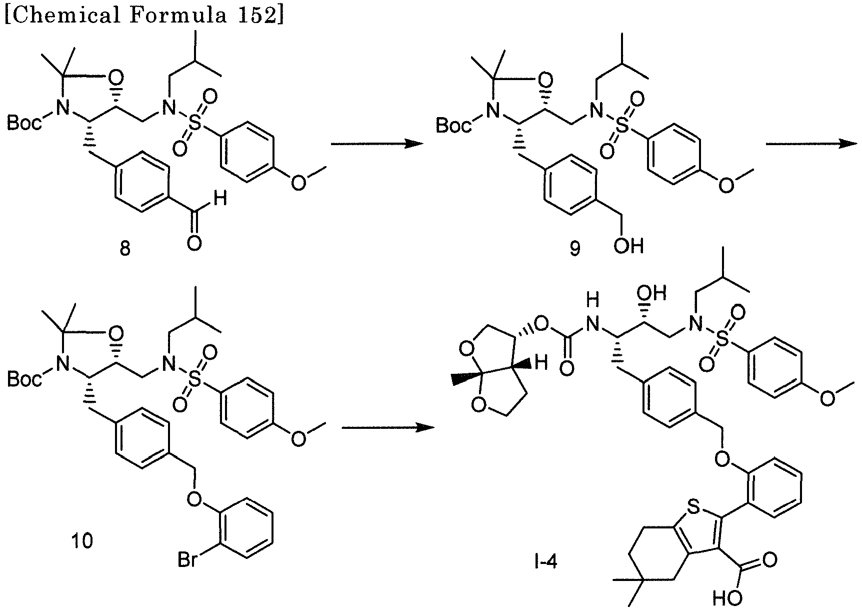

### Step 1

NaBH₄ (6.58mg, 0.174mmol) was added into THF (0.5ml)-methanol (1ml) solution of Compound 8 (100mg, 0.174mmol) at 0°C, the mixture was stirred at 0°C for an hour. After water was added into the reaction mixture, the mixture was extracted with ethyl acetate. After the organic layer was washed with water, the mixture was dried with magnesium sulfate anhydrous, and solvent was removed in vacuo to give Compound 9 as crude.
LC/MS (ESI): 577 (M+1)

### Step 2

Triethylamine (25.8mg, 0.255mmol) was added into dichloromethane (1ml) solution of Compound 9 (105mg, 0.182mmol), and the mixture was stirred at 0°C. After MsCl (25.0mg, 0.218mmol) was added into the reaction mixture, the mixture was stirred at 0°C for 1.5 hours. After water was added into the reaction mixture, the mixture was extracted with ethyl acetate. After the organic layer was washed with water, the mixture was dried with magnesium sulfate anhydrous, and the solvent was removed in vacuo.

2-Bromophenol (14mg, 0.081mmol) and cesium carbonate (31.6mg, 0.097mmol) were added into DMF (1ml) solution of the obtained crude (53mg, 0.081mmol), and the mixture was stirred at 50°C for 2 hour. After water was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 10 (37mg, 63%).
LC/MS (ESI): 731 (M+1)

### Step 3

Compound I-4 was obtained by the same synthesis method of Compound I-1. Measurement condition: A, LC/MS (ESI): 891 (M+1)

### Example 5

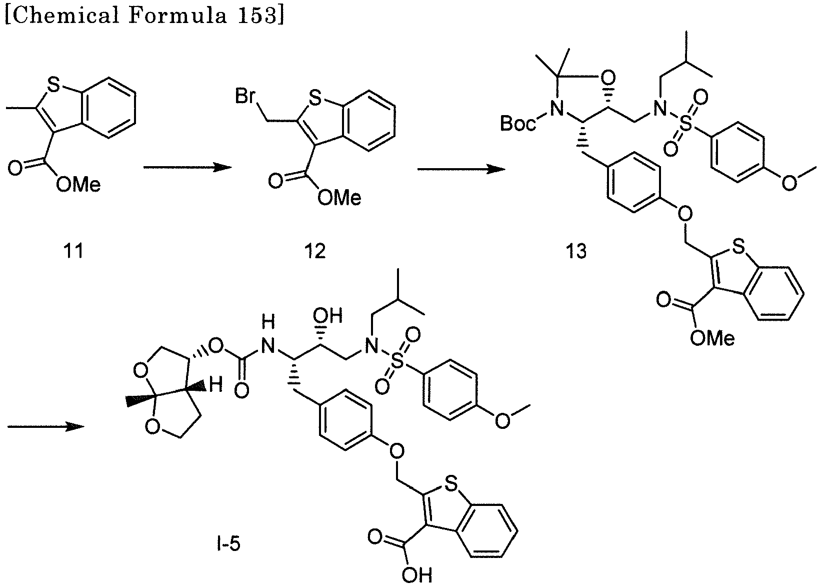

### Step 1

Carbon tetrachloride (1mL), NBS (44mg, 0.247mmol) and AIBN (3.98mg, 0.024mmol) were added into Compound 11 (50mg, 0.242mmol), and the mixture was stirred at 70°C for 4 hours. After the reaction mixture was filtrated, the solvent was removed in vacuo to give Compound 12 as crude.
LC/MS (ESI): 285 (M+18)

### Step 2

Compound 13 was obtained by the same synthesis method of Compound 1. LC/MS (ESI): 767 (M+1)

### Step 3

Compound I-5 was obtained by the same synthesis method of Compound I-2. Measurement condition: A, LC/MS (ESI): 783 (M+1)

### Example 6

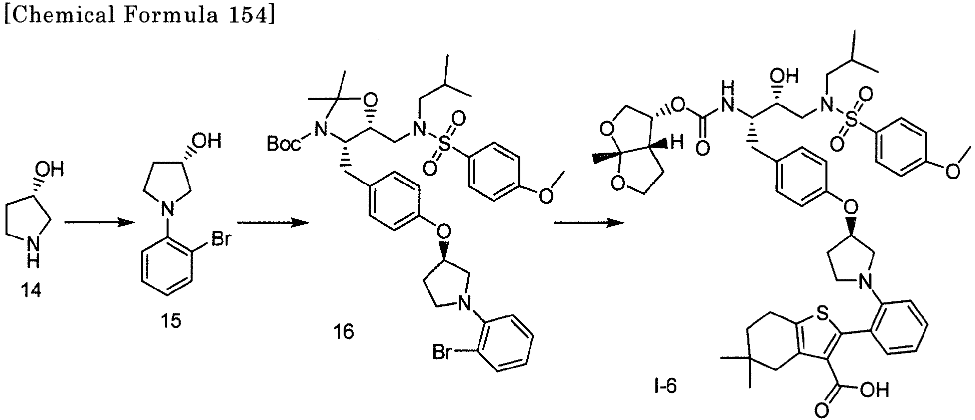

### Step 1

1-Bromo-2-iodobenzene (1786mg, 6.31mmol), sodium hydroxide (459mg, 11.5mmol) and copper (I) iodide (32.8mg, 0.172mmol) were added into isopropanol (6mL) solution of Compound 14 (500mg, 5.74mmol), and the mixture was for 12 hours at 80°C. After water was added into the reaction mixture, the mixture was extracted with chloroform. After the organic layer was washed with water and saturated sodium bicarbonate aqueous solution and water, the mixture was dried with anhydrous sodium sulfate, and the solvent was removed in vacuo. The obtained residue was purified by silica gel column chromatography (hexane-ethyl acetate) to give Compound 15 (390mg, 28%).
LC/MS (ESI): 242 (M+1)

### Step 2

Diisopropyl azodicarboxylate (0.078mL, 0.40mmol) was added into THF (2mL) solution of Compound ii-2 (150mg, 0.267mmol), Compound 15 (77mg, 0.32mmol) and triphenylphosphine (105mg, 0.40mmol) under cooling at 0°C, and the mixture was incubated for 12 hours at room temperature. After water was added into the reaction mixture, the mixture was extracted with ethyl acetate. After the organic layer was washed with water, saturated sodium bicarbonate aqueous solution and water, the mixture was dried with anhydrous sodium sulfate, and the solvent was removed in vacuo. The obtained residue was purified by silica gel column chromatography (hexane-ethyl acetate) to give Compound 16 (133mg, 64%).
LC/MS (ESI): 786 (M+1)

### Step 3

Compound I-6 was obtained by the same synthesis method of Compound I-1. Measurement condition: A, LC/MS (ESI): 946 (M+1)

### Example 7

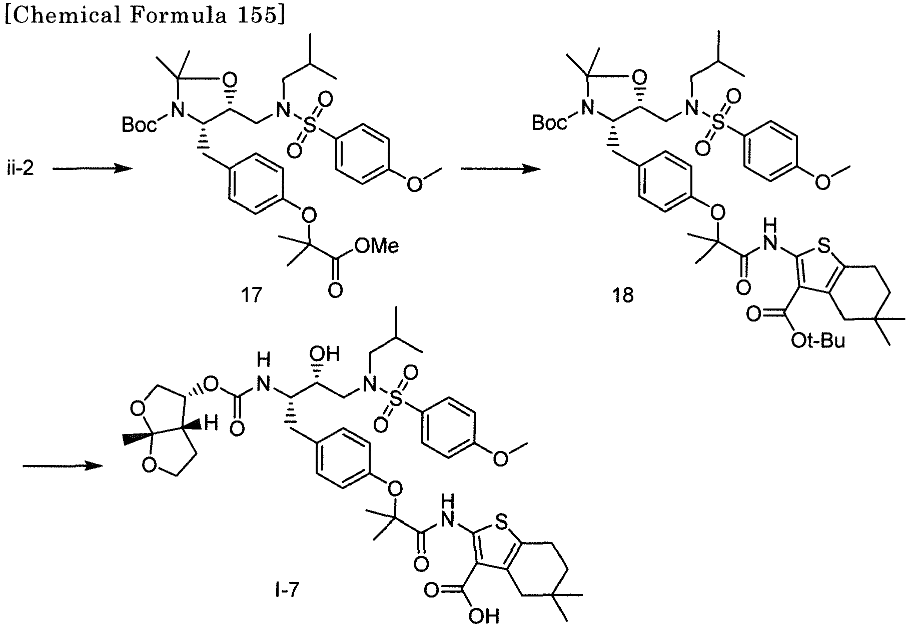

### Step 1

Methyl 2-bromo-2-methylpropanoate (257mg, 1.422mmol) and cesium carbonate (347mg, 1.067mmol) were added into DMF (4mL) solution of Compound ii-2 (400mg, 0.711mmol), and the mixture was stirred overnight at room temperature. After the reaction mixture was extracted with ethyl acetate, and the organic layer was washed with water and brine, the mixture was dried with magnesium sulfate anhydrous, and the solvent was removed in vacuo. The obtained residue was purified by silica gel column chromatography (hexane-ethyl acetate) to give Compound 17 (446mg, 95%).
LC/MS (ESI): 685 (M+23)

### Step 2

2 mol/L sodium hydroxide aqueous solution (1.68ml, 3.36mmol) was added into THF (4.5mL)-methanol (2.5ml) solution of Compound 17 (445mg, 0.671mmol), and the mixture was stirred at 60°C for 2.5 hours. After 10% citric acid aqueous solution was added into the reaction mixture, the mixture was extracted with ethyl acetate. After the organic layer was washed with water and brine, the mixture was dried with magnesium sulfate anhydrous, and the solvent was removed in vacuo.

DMF (2ml), Compound iii-2 (47.4mg, 0.168mmol), DIPEA (26.8mg, 0.207mmol), HATU (73.8mg, 0.194mmol) and dimethylaminopyridine equivalent of catalyst were added into the obtained residue (84mg, 0.129mmol), and the mixture was stirred overnight at 90°C. After the reaction mixture was extracted with ethyl acetate, and the organic layer was washed with water and brine, the mixture was dried with magnesium sulfate anhydrous, and the solvent was removed in vacuo. The obtained residue was purified by silica gel column chromatography (chloroform-methanol) to give Compound 18 (27mg, 23%).
LC/MS (ESI): 912 (M+1)

### Step 3

Compound I-7 was obtained by the same synthesis method of Compound I-1.
Measurement condition: A, LC/MS (ESI): 903 (M+18)

### Example 8

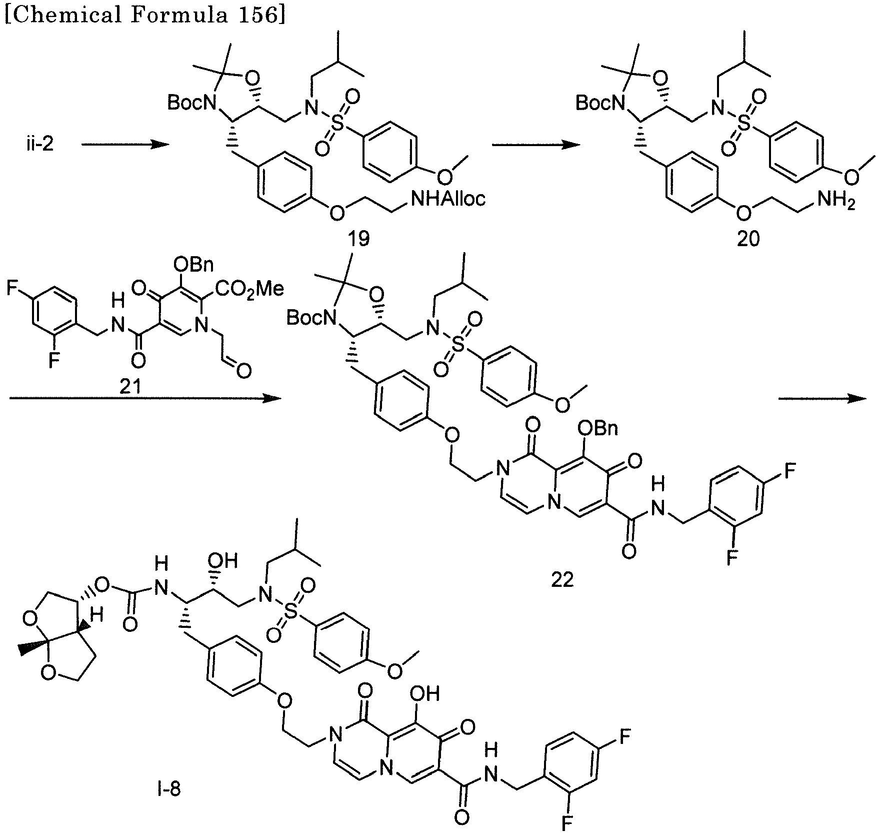

### Step 1

Compound 19 was obtained by the same synthesis method of Compound 1. LC/MS (ESI): 590 (M+1)

### Step 2

Morpholine (143mg, 1.64mmol) and Pd (Ph₃P)₄ (95mg, 0.08mmol) were added into THF (5mL) solution of Compound 19 (564mg, 0.82mmol), and the mixture was stirred at room temperature for 2 hours. After water was added into reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 20 (440mg, 89%).
LC/MS (ESI): 606 (M+1)

### Step 3

Compound 20 (78mg, 0.17mmol) and acetic acid (0.01mL, 0.17mmol) were added into toluene (10mL solution of Compound 21 (100mg, 0.17mmol), and the mixture was refluxed for 6 hours. After water was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 226 (110mg, 63%). LC/MS (ESI): 1026 (M+1)

### Step 4

Compound I-8 was obtained by the same synthesis method of Compound I-1. Measurement condition: A, LC/MS (ESI): 966 (M+1)

### Example 9

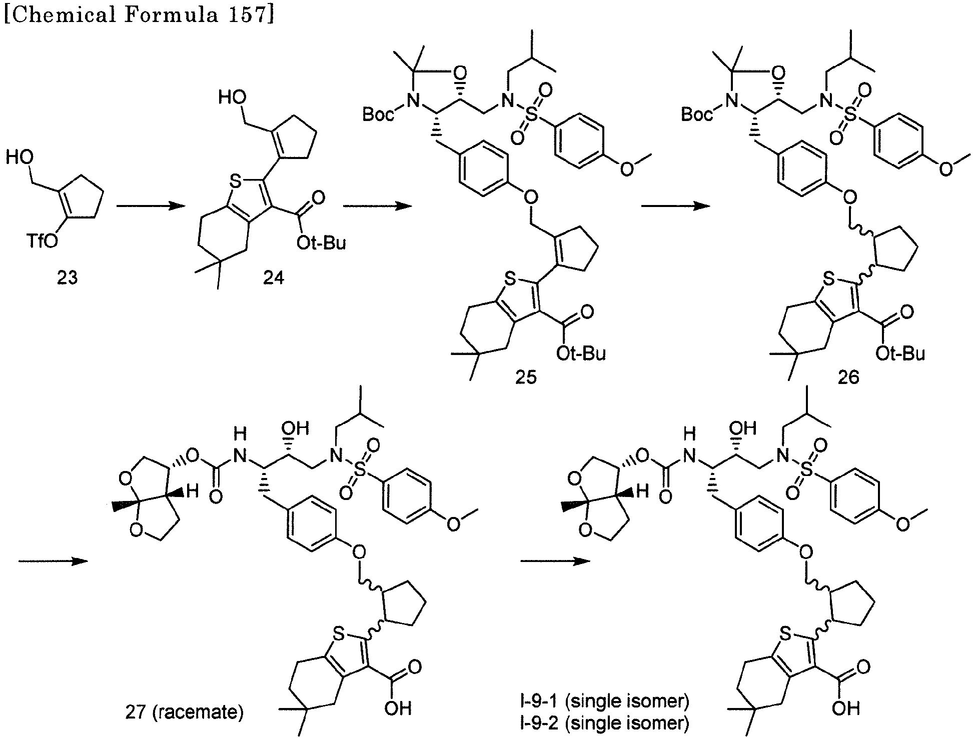

### Step 1

Water (0.4mL), Compound iii-4 (290mg, 0.934mmol), sodium carbonate (198mg, 1.868mmol) and Pd (PPh₃)₄ (108mg, 0.093mmol) were added into dioxane (4mL) solution of Compound 23 (230mg, 0.934mmol) under nitrogen atmosphere, and the mixture was stirred at 90°C for 1.5 hours. After the reaction mixture was extracted with ethyl acetate, the organic layer was washed with water and brine. After the mixture was dried with magnesium sulfate anhydrous, the solvent was removed in vacuo. The obtained residue was purified by silica gel column chromatography (hexane-ethyl acetate) to give Compound 24 (151mg, 45%).
LC/MS (ESI): 385 (M+23)

### Step 2

Triethylamine (31.4mg, 0.310mmol), trimethylaminehydrochloric acid salt (1.98mg, 0.021mmol) and p-toluenesulphonylchrolide (47.3mg, 0.248mmol) were added into dichloromethane (1.5mL) solution of Compound 24 (75mg, 0.207mmol), and the mixture was stirred at 0°C for an hour. After the reaction mixture was extracted with ethyl acetate, the organic layer was washed with water and brine. After the mixture was dried with magnesium sulfate anhydrous, the solvent was removed in vacuo. After DMF (1mL), Compound ii-2 (116mg, 0.207mmol) and cesium carbonate (88mg, 0.269mmol) were added into the obtained residue, the mixture was stirred at 50°C for 1.5 hours. After the reaction mixture was extracted with ethyl acetate, the organic layer was washed with water and brine. After the mixture was dried with magnesium sulfate anhydrous, the solvent was removed in vacuo. The obtained residue was purified by silica gel column chromatography (hexane-ethyl acetate) to give Compound 25 (91mg, 49%).
LC/MS (ESI): 929 (M+23)

### Step 3

Palladium hydroxide (30mg) was added into methanol (0.8mL)-THF (0.8ml) solution of Compound 25 (89mg, 0.098mmol), and the mixture was stirred at room temperature for an hour under hydrogen atmosphere. After the reaction mixture was filtrated by Celite^{®}, the solvent was removed in vacuo. The obtained residue was purified by silica gel column chromatography (hexane-ethyl acetate) to give Compound 26 (72mg, 81%).
LC/MS (ESI): 931 (M+23)

### Step 4

Compound 27 was obtained by the same synthesis method of Compound I-1.
LC/MS (ESI): 900 (M+18)

### Step 5 Optical resolution by SFC

### (Analysis)

### <Analysis SFC (JASCO Corporation)>

Column: CHIRALPAK IF/SFC (5µm, i.d.250x4.6mm) (DAICEL)
Flow speed: 2.0 mL/ minutes
UV detective wave length: 220nm
Analytical condition: solution sending for 15 minutes with MeOH/CO₂ =40/60.
Elution time: front peak is 9.1 minutes (Compound I-9-1), rear peak is 11.3 minutes

### (Compound I-9-2)

### (Fractionation)

### <SFC30 system (Waters)>

Column: CHIRALPAK IF/SFC (5µm, i.d.250x20mm) (DAICEL)
Flow speed: 30 mL/ minutes
UV detective wave length: 241nm
Analytical condition: solution sending for 15 minutes with MeOH/CO₂ =55/45.
Elution time: front peak is 8.2 minutes (Compound I-9-1), rear peak is 10.0 minutes

### (Compound I-9-2)

### Example 10

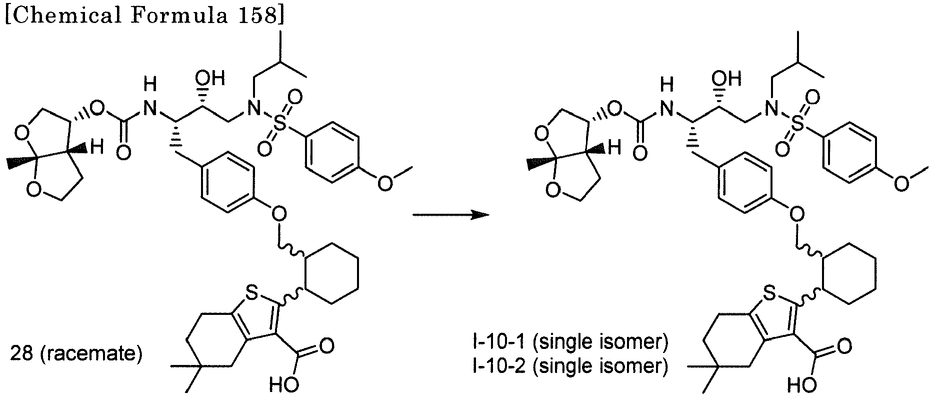

Compound 28; LC/MS (ESI): 915 (M+18)
Optical resolution by SFC
(analysis)

### <Analysis SFC (JASCO Corporation)>

Column: CHIRALPAK IC-3/SFC (3µm, i.d.250x4.6mm) (DAICEL)
Flow speed: 2.0 mL/ minutes
UV detective wave length: 250nm
Analytical condition: solution sendinf for 15 minutes with MeOH/CO₂ =30/70.
Elution time: front peak is 12.2 minutes (Compound I-10-1), rear peak is 13.1 minutes (Compound I-10-2)

### (Fractionation)

### <Semi-fractionation SFC (JASCO Corporation)>

Column: CHIRALPAK IC/SFC (5µm, i.d.250x20mm) (DAICEL) with tandem of the two column
Flow speed: 40 mL/ minutes
UV detective wave length: 250nm
Analytical condition: solution sending for 21 minutes with MeOH/CO₂ =30/70. Elution time: front peak is 16.7 minutes (Compound I-10-1), rear peak is 18.1 minutes (Compound I-10-2)

### Example 11

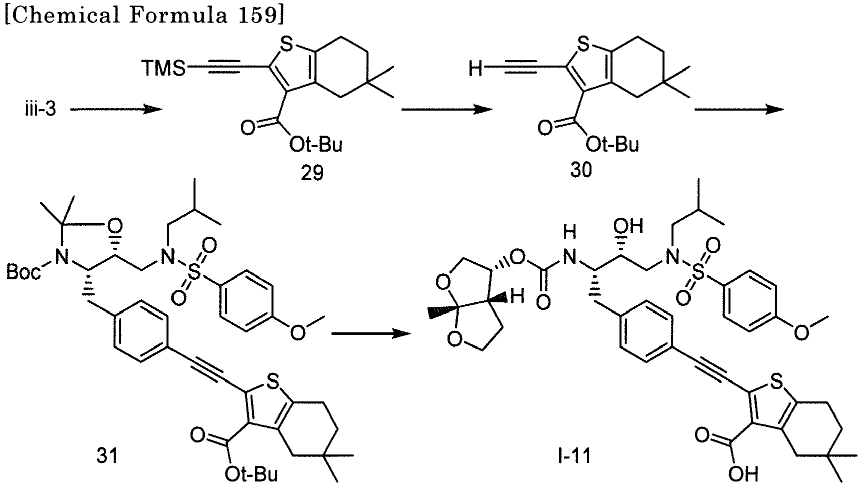

### Step 1

Triethylamine (91mg, 0.904mmol), PdCl₂ (PPh₃)₂ (10.6mg, 0.015mmol), copper (I) iodide (5.74mg, 0.030mmol) and ethynyltrimethylsilane (89mg, 0.904mmol) were added into DMF (1mL) solution of Compound iii-3 (104mg, 0.301mmol) under nitrogen atmosphere, and the mixture was stirred under heating at 100°C for an hour. After water was added into the reaction mixture, the mixture was extracted with ethyl acetate. After the organic layer was washed with water, the mixture was dried with magnesium sulfate anhydrous, and the solvent was removed in vacuo. The obtained residue was purified by silica gel column chromatography (hexane-ethyl acetate) to give Compound 29 (108mg, 99%).
LC/MS (ESI): 363 (M+1)

### Step 2

Potassium carbonate (120mg, 0.869mmol) was added into methanol (3mL) solution of Compound 29 (300mg, 0.827mmol), and the mixture was stirred at 0°C for 30 minutes. After water was added into the reaction mixture, the mixture was extracted with ethyl acetate. After the organic layer was washed with water, the mixture was dried with magnesium sulfate anhydrous, and the solvent was removed in vacuo, Compound 30 as crude.
LC/MS (ESI): 291 (M+1)

### Step 3

Compound 31 was obtained by the same synthesis method of Compound 29. LC/MS (ESI): 835 (M+1)

### Step 4

Compound I-11 was obtained by the same synthesis method of Compound I-1. Measurement condition: A, LC/MS (ESI): 809 (M+1)

### Example 12

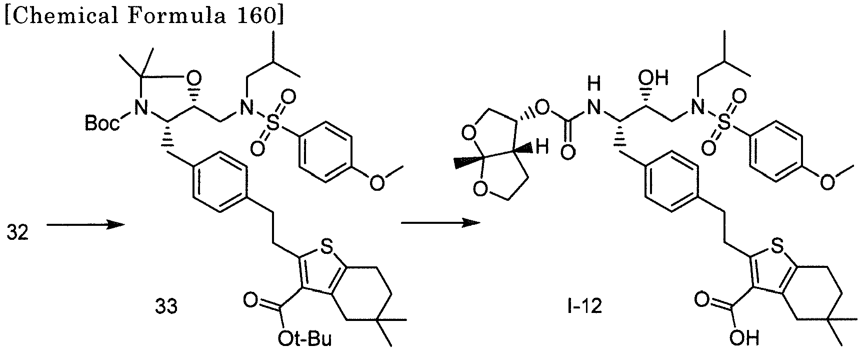

### Step 1

Palladium hydroxide (30mg) was added into methanol (1mL)-THF (0.2ml) solution of Compound 32 (87mg, 0.104mmol), and the mixture was stirred at room temperature under hydrogen atmosphere for 1.5 hours. After the reaction mixture was filtrated by Celite^{®}, the solvent was removed in vacuo to give Compound 33 as crude.
LC/MS (ESI): 856 (M+18)

### Step 2

Compound I-12 was obtained by the same synthesis method of Compound I-1. Measurement condition: A, LC/MS (ESI): 813 (M+1)

### Example 13

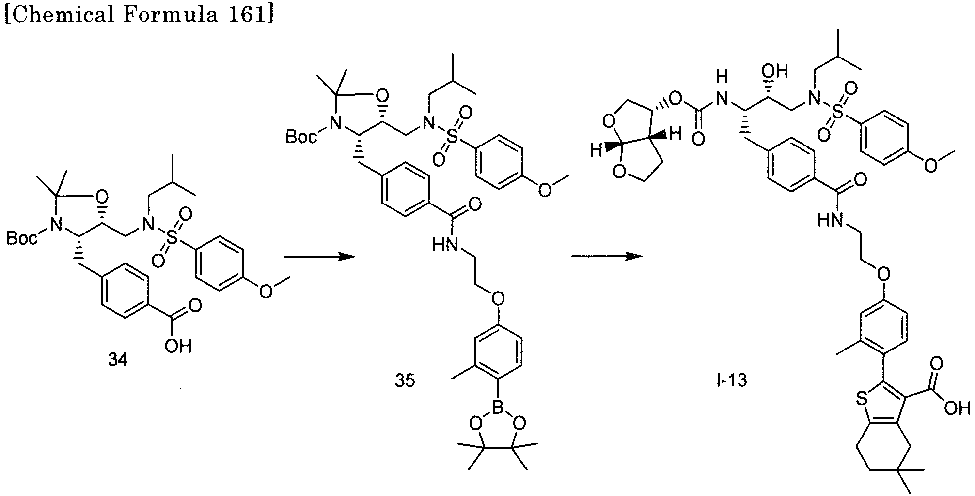

### Step 1

2-(3-Methyl-4-(4, 4, 5, 5-tetramethyl-1, 3, 2-dioxaborolan-2-yl)phenoxy)ethan-1-amine hydrochloride (97mg, 0.308mmol), DMF (1ml), triethylamine (78mg, 0.770mmol), WSCD HC1 (38.4mg, 0.200mmol) and HOBt (29.1mg, 0.216mmol) were added into Compound 34 (91mg, 0.154mmol), and the mixture was stirred at 70°C for 5 hours. After water was added into the reaction mixture, the mixture was extracted with ethyl acetate. After the organic layer was washed with water, the mixture was dried with magnesium sulfate anhydrous, and the solvent was removed in vacuo. The obtained residue was purified by silica gel column chromatography (chloroform-methanol) to give Compound 35 (51mg, 39%).
LC/MS (ESI): 850 (M+1)

### Step 2

Compound I-13 was obtained by the same synthesis method of Compound 5 and Compound I-1.
Measurement condition: A, LC/MS (ESI): 948 (M+1)

### Example 14

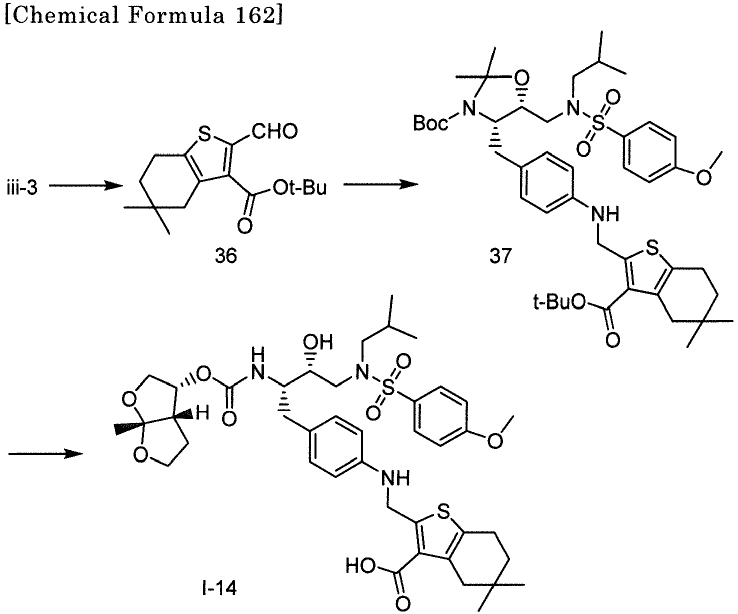

### Step 1

Hexane solution (1.5M, 0.7mL, 1.13mmol) of n-butyllithium was added by dropwise to THF (6mL) solution of Compound iii-3 (293mg, 0.85mmol) for 10 minutes under cooling at -70°C under nitrogen atmosphere, and the mixture was stirred at - 70°C for an hour. DMF (0.13mL, 1.70mmol) was added, and the mixture was stirred for an hour. After saturated ammonium chloride was added into reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 36 (250mg, 100%). LC/MS (ESI): 295 (M+1)

### Step 2

Acetic acid (0.1mL), Compound 36 (25.0mg, 0.085mmol) and picolineborane (11.3mg, 0.106mmol) were added into methanol (2mL) solution of Compound ii-5 (39.7mg, 0.071mmol), and the mixture was stirred at room temperature for an hour. After saturated sodium bicarbonate aqueous solution was added into the reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (hexane-ethyl acetate) to give Compound 37 (59.4mg, 100%). LC/MS (ESI): 840 (M+1)

### Step 3

Compound I-14 was obtained by the same synthesis method of Compound I-1. Measurement condition: A, LC/MS (ESI): 814 (M+1)

### Example 15

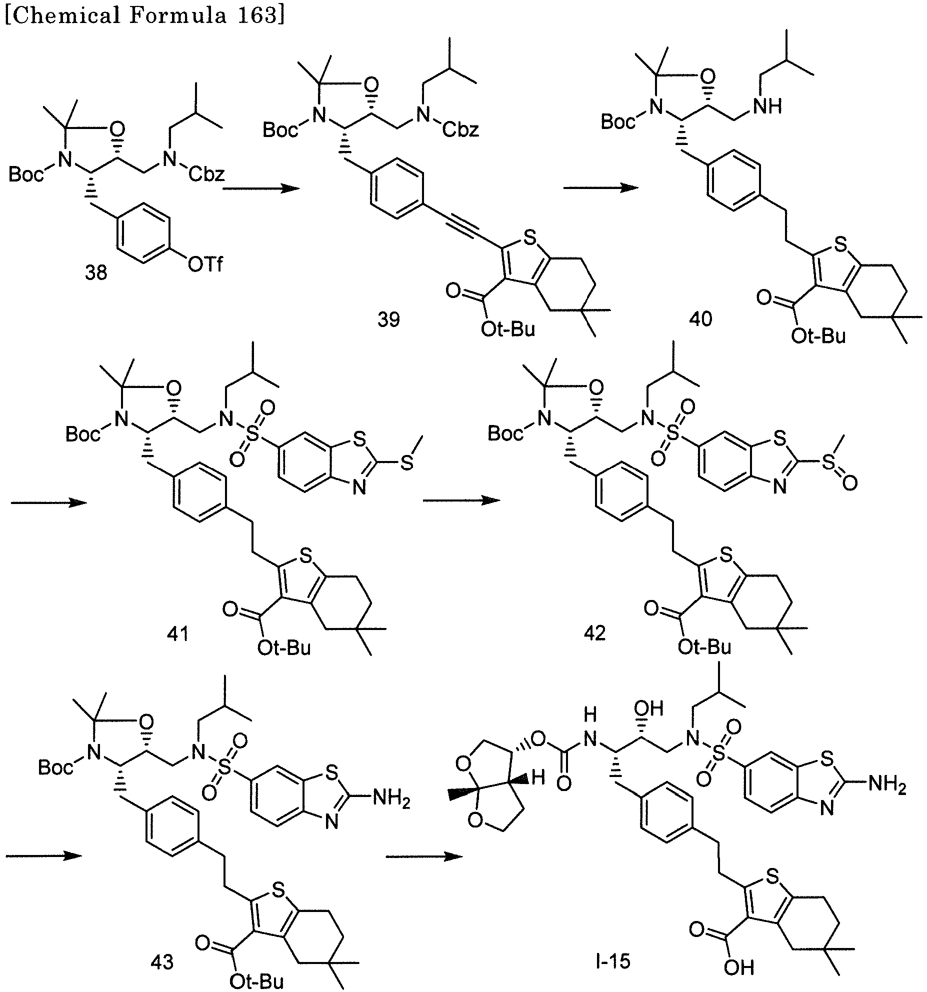

### Step 1 and 2

Compound 39 and 40 was obtained by the same synthesis method of Compound 29 and 33.
Compound 39; LC/MS (ESI): 816 (M+18)
Compound 40; LC/MS (ESI): 669 (M+1)

### Step 3

Triethylamine (0.03mL,0.24mmol) and 2- (methylthio)benzo[d]thiazole-6-sulfonyl chloride (56.0mg, 0.20mmol) were added into dichloromethane (1mL) solution of Compound 40 (115mg, 0.17mmol), and the mixture was stirred at room temperature for 4 hours. The reaction mixture was purified by silica gel column chromatography (hexane-ethyl acetate) to give Compound 41 (106mg, 67%).
LC/MS (ESI): 912 (M+1)

### Step 4

After m-chloroperoxybenzoic acid (45mg, 0.169mmol) was added into dichloromethane (2mL) solution of Compound 41 (103mg, 0.113mmol) under ice-cold, the mixture was stirred at room temperature for an hour. After saturated thiosodium sulfate aqueous solution was added into the reaction mixture, the mixture was extracted with chloroform. After the organic layer was washed with saturated sodium bicarbonate aqueous solution and brine, the mixture was dried with magnesium sulfate anhydrous, and the solvent was removed in vacuo to give Compound 42 as crude.

### Step 5

2 mol/L Ammonia ethanol solution (0.496ml, 0.991mmol) was added into dioxane (2mL) solution of Compound 42 (92mg, 0.099mmol), and the mixture was stirred at 80°C under heating for 19 hours. After the reaction mixture was removed in vacuo, the obtained residue was purified by silica gel column chromatography (hexane-ethyl acetate) to give Compound 43 (76mg, 87%).
LC/MS (ESI): 881 (M+1)

### Step 6

Compound 1-15 was obtained by the same synthesis method of Compound 1-1. Measurement condition: A, LC/MS (ESI): 855 (M+1)

### Example 16

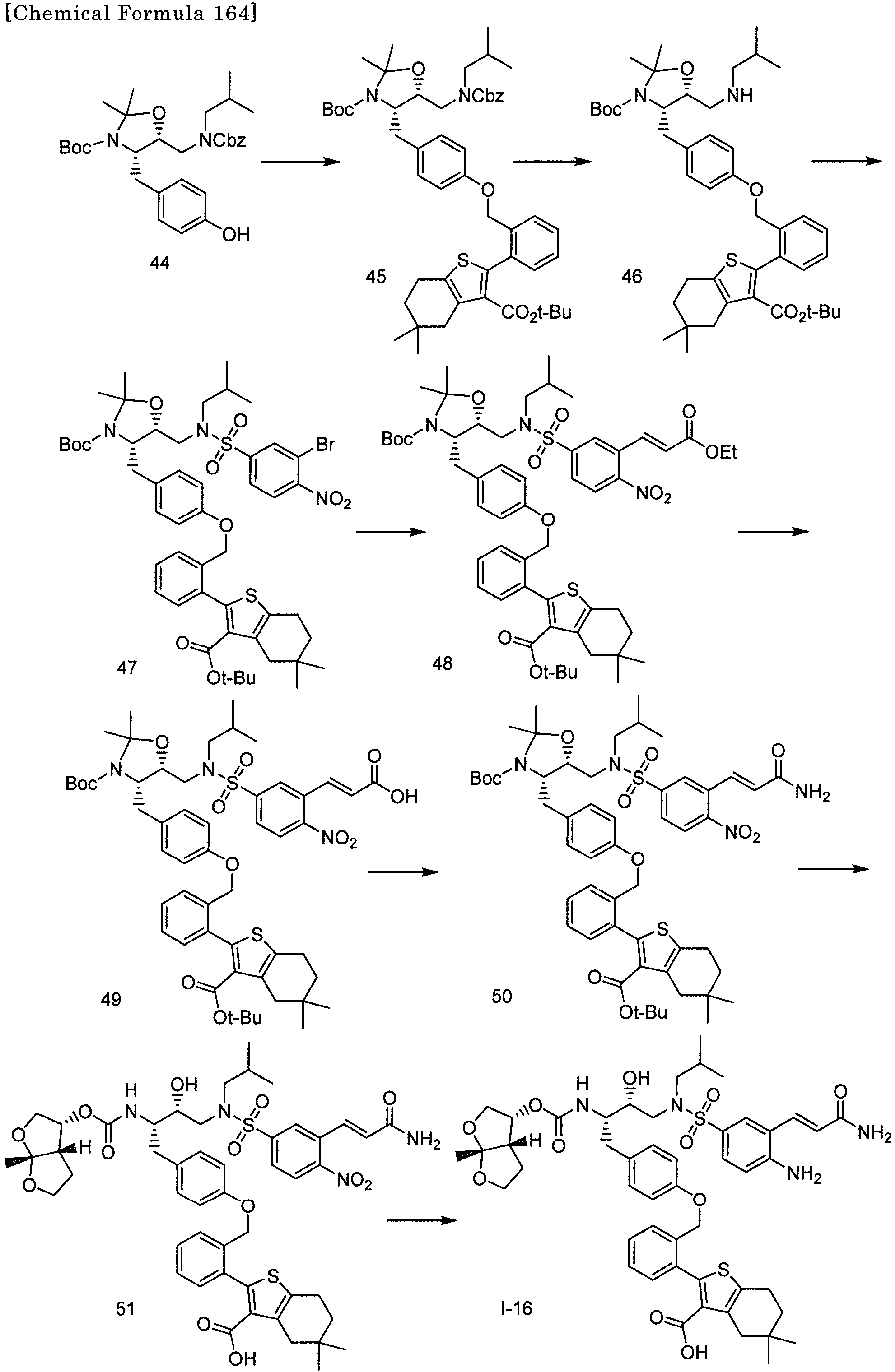

### Step 1 and 2

Compound 45 and 46 was obtained by the same synthesis method of Compound 2 and 28.
Compound 56; LC/MS (ESI): 747 (M+1)

### Step 3

Triethylamine (0.074mL, 0.535mmol) and 3-bromo-4-nitrobenzene-l-sulfonyl chloride (121mg, 0.402mmol) were added into dichloromethane (3.0mL) solution of Compound 46 (200mg, 0.268mmol), and the mixture was stirred at room temperature for an hour. After water was added into the reaction mixture, the mixture was extracted with chloroform. After the mixture was dried with magnesium sulfate anhydrous, the solvent was removed in vacuo. The obtained residue was purified by silica gel column chromatography (chloroform-methanol) to give Compound 47 (271mg, 100%).

### Step 4

Ethyl (E)-3-(4,4,5,5-tetramethyl-1,3,2-dioxaborolane-2-yl)acrylate (125mg, 0.554mmol), PdCl₂ (dppf) CH₂Cl₂ (22.6mg, 0.028mmol) and 2 mol/L sodium carbonate aqueous solution (0.554mL, 1.11mmol) were added into THF (5.6mL) solution of Compound 47 (271mg, 0.268mmol), and the mixture was stirred at 130°C by using a microwave device for 30 minutes. After brine was added into the reaction mixture, the mixture was extracted with chloroform. After the organic layer was dried with magnesium sulfate anhydrous, the solvent was removed in vacuo. The obtained residue was purified by silica gel column chromatography (chloroform-methanol) to give Compound 48 (244mg, 88%).

### Step 5

2 mol/L Sodium hydroxide aqueous solution (1.07mL, 2.14mmol) was added into methanol (4.4mL)-water (2.2mL)-THF (4.4mL) solution of Compound 48 (220mg, 0.214mmol), and the mixture was stirred at room temperature for 3 hours. Saturated ammonium chloride aqueous solution and saturated sodium chloride aqueous solution were added into the reaction mixture, and the mixture was extracted with chloroform. After the organic layer was dried with magnesium sulfate anhydrous, the solvent was removed in vacuo to give Compound 49 (213mg, 99.5%).

### Step 6

Triethylamine (0.118mL, 0.850mmol), ammonium chloride (34.1mg, 0.638mmol) and HATU (97mg, 0.26mmol) were added into DMF (2.1mL) solution of Compound 49 (213mg, 0.213mmol), and the mixture was stirred at room temperature for 16 hours. After water was added into the reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (chloroform-methanol) to give Compound 50 (173mg, 81%).
LC/MS (ESI): 1002 (M+1)

### Step 7

Compound 51 was obtained by the same synthesis method of Compound I-1. LC/MS (ESI): 975 (M+1)

### Step 8

Ammonium chloride (52.1mg, 0.974mmol) and iron powder (27.2mg, 0.487mmol) were added into ethanol (0.95mL)-water (0.32mL) solution of Compound 51 (95mg, 0.097mmol), and the mixture was stirred at 80°C for 2 hours. After water was added into the reaction mixture, the mixture was extracted with chloroform. After the organic layer was dried with anhydrous sodium sulfate, the solvent was removed in vacuo. The obtained residue was purified by silica gel column chromatography (chloroform-methanol) to give Compound 1-16 (32mg, 35%). Measurement condition: A, LC/MS (ESI): 946 (M+1)

### Example 17

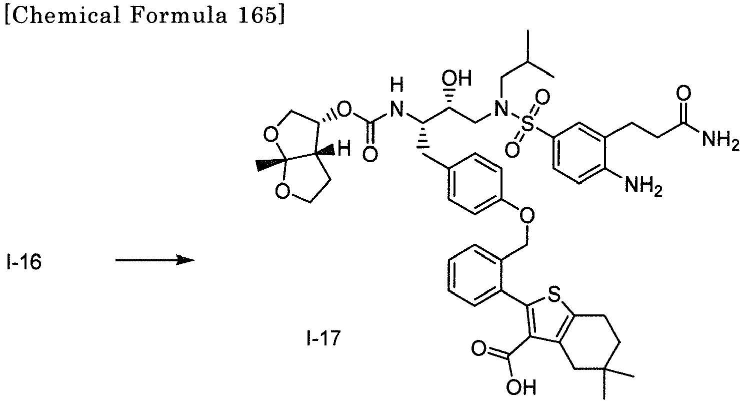

10%Pd/C (including 50% water) (3.38mg) was added into THF (3.0mL) solution of Compound 1-16 (30mg, 0.032mmol), and the mixture was stirred under hydrogen atmosphere at room temperature for 1.5 hours. After the reaction mixture was filtrated by Celite^{®}, the solvent was removed in vacuo. The obtained residue was purified by silica gel column chromatography (chloroform-methanol) to give Compound 1-17 (19.8mg, 66%).
Measurement condition: A, LC/MS (ESI): 948 (M+1)

### Example 18

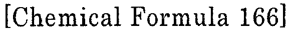

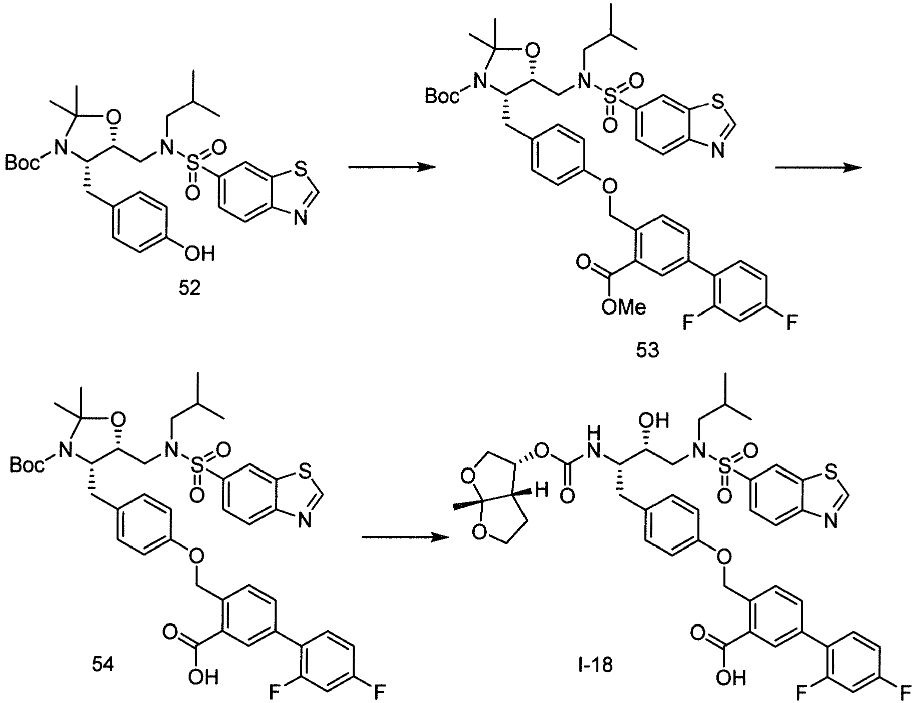

### Step 1

Cesium carbonate (110mg, 0.34mmol) and Compound iii-50 (116mg, 0.34mmol) were added into DMF (1mL) solution of Compound 52 (100mg, 0.17mmol), and the mixture was stirred at room temperature for 5 hours. After saturated ammonium chloride aqueous solution was added into the reaction mixture, the mixture was extracted with ethyl acetate. After the organic layer was washed with water, the mixture was dried with sodium sulfate, and the solvent was removed in vacuo. The obtained residue was purified by silica gel column chromatography (hexane-ethyl acetate) to give Compound 53 (134mg, 93%).
LC/MS (ESI): 872 (M+23)

### Step 2-3

Compound I-18 was obtained by the same synthesis method of Compound I-2. Measurement condition: B, LC/MS (ESI): 864 (M-1)

### Example 19

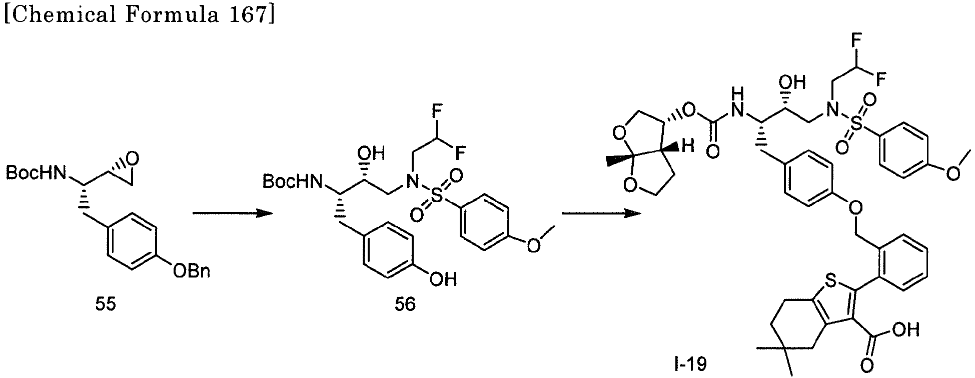

### Step 1 and 2

After Compound 56 was synthesized from Compound 55 by the method written in WO2005/064008, Compound I-19 was obtained by the same synthesis method of Compound I-1.
Measurement condition: A, LC/MS (ESI): 897 (M+1)

### Example 20

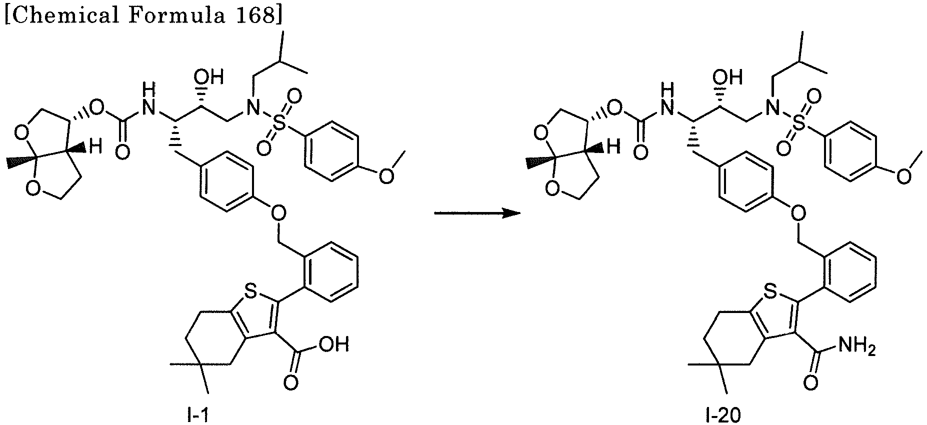

Triethylamine (0.17mL, 1.21mmol), ammonium chloride (4.38mg, 0.082mmol) and HATU (18.7mg, 0.049mmol) were added into DMF (0.5mL) solution of Compound I-1 (36.5mg, 0.041mmol), and the mixture was stirred at room temperature for an hour. After water was added into the reaction mixture, the mixture was extracted with ethyl acetate. After the organic layer was washed with brine, the mixture was dried with anhydrous sodium sulfate, and the solvent was removed in vacuo. The obtained residue was purified by silica gel column chromatography (chloroform-methanol) to give Compound 1-20 (28mg, 77%).
Measurement condition: A, LC/MS (ESI): 890 (M+1)

### Example 21

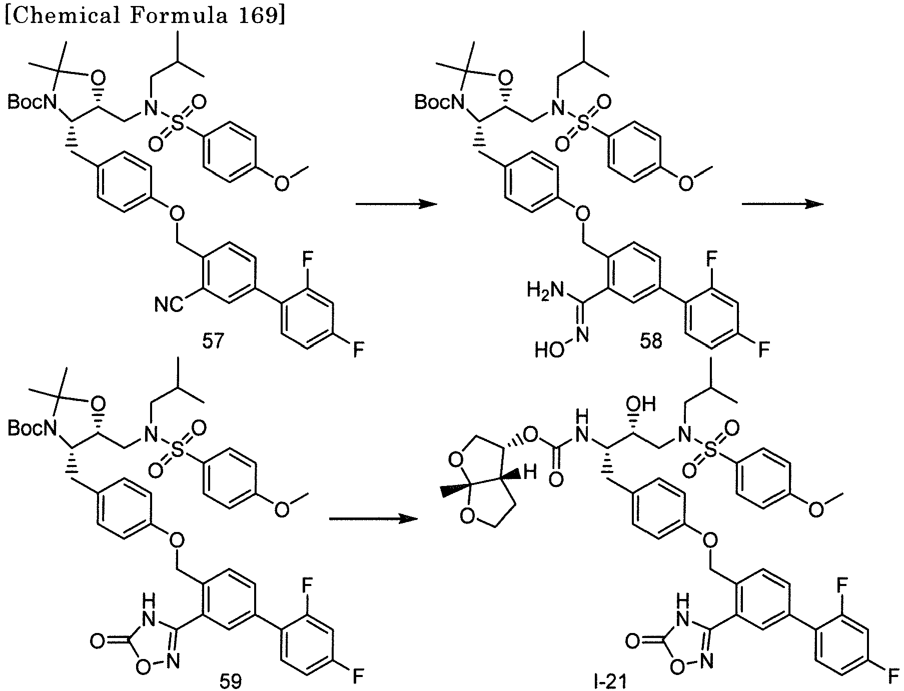

### Step 1

50% Hydroxylamine aqueous solution (125mg, 1.90mmol) was added into ethanol (3mL) solution of Compound 57 (150mg, 0.190mmol), and the mixture was stirres at room temperature for 38 hours. After the reaction mixture was condensed, water was added, and the mixture was extracted with ethyl acetate. After the organic layer was washed with water and brine, the mixture was dried with sodium sulfate, and the solvent was removed in vacuo to give Compound 58 as crude. LC/MS (ESI): 823 (M+1)

### Step 2

CDI (44mg, 0.272mmol) was added into THF (6mL) solution of Compound 58 (149mg, 0.181mmol) under nitrogen atmosphere. After the mixutre was stirred at room temperature for 30 minutes, the mixture was stirred at 85°C for 8 hours. After citric acid aqueous solution and water were added into the reaction mixture, the mixture was extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound 59 (94mg, 61%).
LC/MS (ESI): 847 (M-1)

### Step 3

Compound 1-21 was obtained by the same synthesis method of Compound I-1. Measurement condition: B, LC/MS (ESI): 877 (M-1)

### Example 22

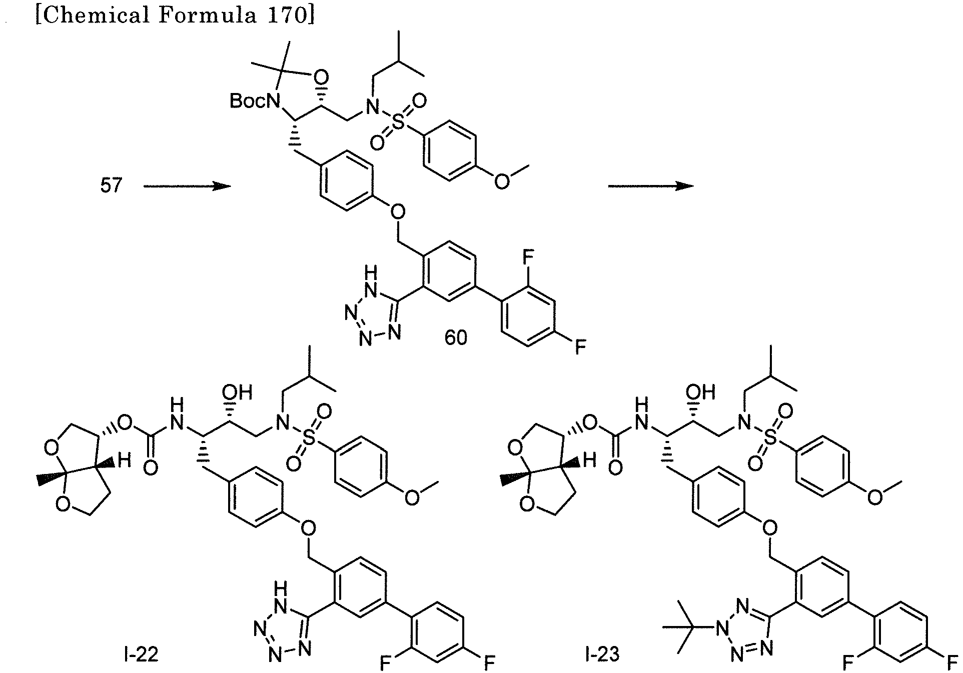

### Step 1

NaN₃ (54.3mg, 0.836mmol) and ammonium chloride (44.7mg, 0.836mmol) were added into DMA (2.4mL) solution of Compound 57 (66mg, 0.084mmol) under nitrogen atmosphere, and the mixture was stirred at 140°C for 8 hours. After water was added into the reaction mixture, the mixture made to be acid with 10% citric acid aqueous solution, and the mixture was extracted with ethyl acetate. After the organic layer was washed with water and brine, the mixture was dried with sodium sulfate, and the solvent was removed in vacuo. The obtained residue was purified by silica gel column chromatography (chloroform-methanol) to give Compound 60 (46mg, 66%). LC/MS (ESI): 831 (M-1)

### Step 2

TFA (0.5mL) was added into dichloromethane (2.5mL) solution of Compound 60 (57mg, 0.068mmol) and anisole (0.149mL, 1.37mmol), and the mixture was stirred at room temperature for 2 hours. After the solvent was removed in vacuo, dichloromethane (2.5mL) and triethylamine (0.142mL, 1.03mmol) were added into the obtained residue. Compound i-3 (20mg, 0.068mmol) was added, and the mixture was stirred at room temperature for 3 hours. After sodium hydrogen carbonate aqueous solution was added into the reaction mixture, the mixture is made to be acid with 10% citric acid aqueous solution, and the mixture was extracted with ethyl acetate. After the organic layer was washed with water and brine, the mixture was dried with sodium sulfate, and the solvent was removed in vacuo. The obtained residue was reverse phase HPLC to give Compound I-22 (23mg, 39%) and Compound I-23 (7mg, 11%).
Compound I-22; Measurement condition: B, LC/MS (ESI): 861 (M-1)
Compound I-23; Measurement condition: B, LC/MS (ESI): 941 (M+23)

### Example 23

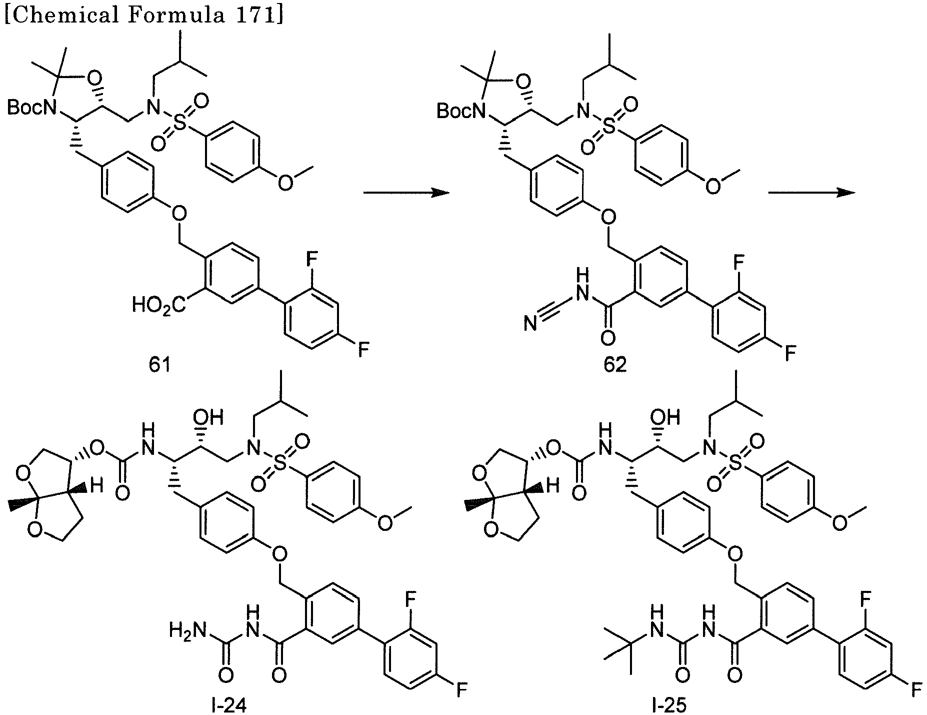

### Step 1

DIPEA (0.130mL, 0.742mmol) was added into DMF (2mL) solution of Compound 61 (100mg, 0.124mmol), cyanamide (20.8mg, 0.494mmol) and BOP (65.6mg, 0.148mmol) under nitrogen atmosphere, and the mixture was stirred overnight at room temperature. After water was added into the reaction mixture, the mixture was made to be acid with 10% citric acid aqueous solution. The mixture was extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound 62 (96mg, 93%).
LC/MS (ESI): 831 (M-1)

### Step 2

Compound I-24 and Compound I-25 was obtained by the same synthesis method of Compound I-22 and Compound I-23.
Compound I-24; Measurement condition: B, LC/MS (ESI): 879 (M-1)
Compound I-25; Measurement condition: B, LC/MS (ESI): 935 (M-1)

### Example 24

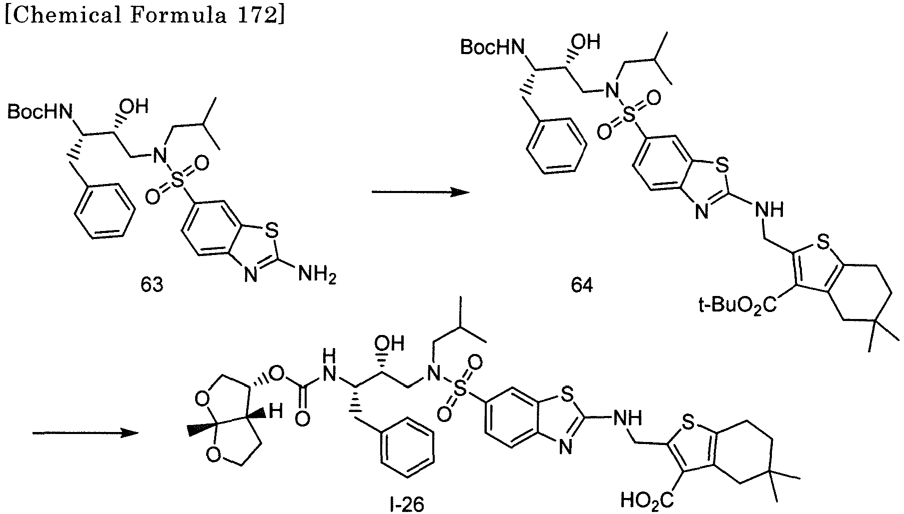

### Step 1

p-Toluenesulfonic acid monohydrate (2mg, 10.5µmol) was added into toluene (6mL) solution of Compound 63 (50mg, 0.091mmol) and Compound 36 (29.5mg, 0.100mmol) under nitrogen atmosphere, and the mixture was stirred under heat reflux for 7 hours. After the solvent of the reaction mixture was removed in vacuo, THF (1mL) was added into the obtained residue. NaBH₄ (8.62mg, 0.228mmol) and methanol (0.5mL) were added into at 0°C, and the mixture was stirred for 30 minutes. After saturated sodium hydrogen carbonate aqueous solution was added into the reaction mixture, the mixture was stirred at room temperature for 30 minutes, and the mixture was extracted with ethyl acetate. After the organic layer was washed with water and brine, the mixture was dried with sodium sulfate, and the solvent was removed in vacuo. The obtained residue was purified by silica gel column chromatography (hexane-ethyl acetate) to give Compound 64 (60mg, 80%). LC/MS (ESI): 825 (M-1)

### Step 2

Compound I-26 was obtained by the same synthesis method of Compound I-1. Measurement condition: B, LC/MS (ESI): 841 (M+1)

### Example 25

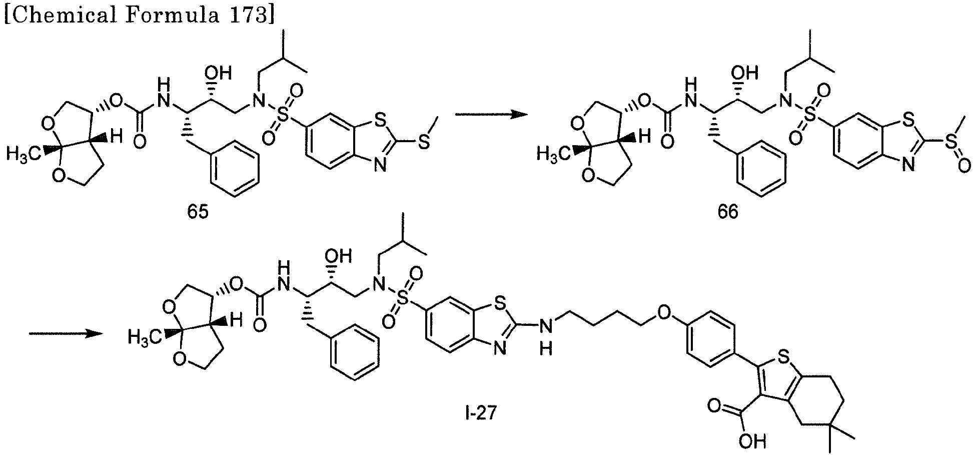

### Step 1

m-Chloroperoxybenzoic acid (39.8mg, 0.231mmol) was added into dichloromethane (2mL) solution of Compound 65 (100mg, 0.154mmol) under ice-cold, and the mixture was stirred at 0°C for an hour. After saturated thiosodium sulfate aqueous solution was added into the reaction mixture, the mixture was extracted with dichloromethane. After the organic layer was washed with brine, the mixture was dried with anhydrous sodium sulfate, and the solvent was removed in vacuo, Compound 66 (100mg, 98%).
LC/MS (ESI): 666 (M+1)

### Step 2

Triethylamine (0.214mL, 1.54mmol) and Compound iii-76 (190mg, 0.231mmol) were added into DMA (2mL) solution of Compound 66 (100mg, 0.150mmol), and the mixture was stirred at 95°C by using a microwave device for 20 minutes. After water and 2 mol/L hydrochloric acid were added into the reaction mixture, the mixture was extracted with ethyl acetate. After the organic layer was washed with brine, the mixture was dried with anhydrous sodium sulfate, and the solvent was removed in vacuo. The obtained residue was HPLC (acetonitrile-water containing 0.1% formic acid) to give Compound 1-27 (44mg, 30%).
Measurement condition: A, LC/MS (ESI): 976 (M+1)

### Example 26

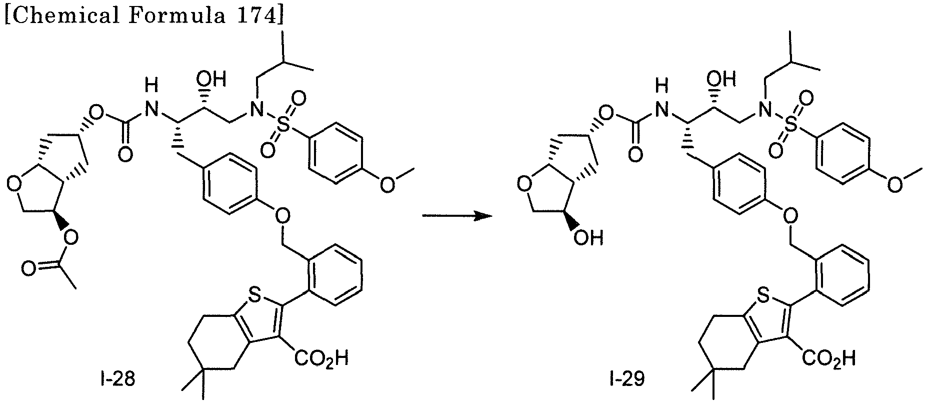

Potassium carbonate (6mg, 0.04mmol) was added into methanol (5mL) solution of the synthesized Compound I-28 (38mg, 0.04mmol), and the mixture was reacted at room temperature for an hour. Saturated ammonium chloride aqueous solution was added into the reaction mixture. The mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound I-29 (35mg, 97%).
Compound I-28; Measurement condition: A, LC/MS (ESI): 933 (M+1)
Compound I-29; Measurement condition: A, LC/MS (ESI): 891 (M+1)

### Example 27

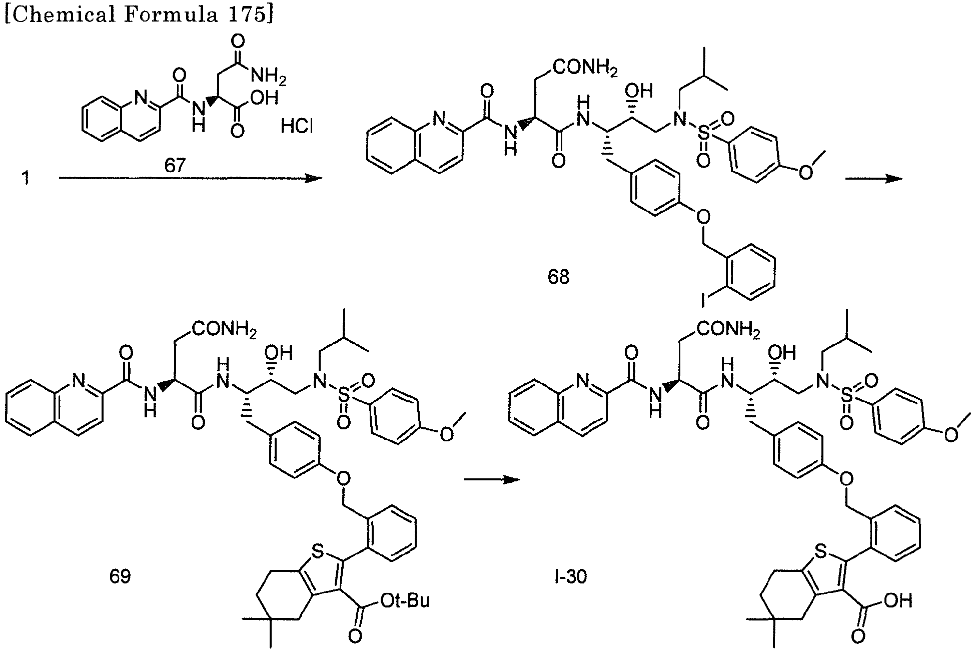

### Step 1

TFA (0.5mL) was added into dichloromethane (1mL) solution of Compound 1 (82mg, 0.10mmol) under nitrogen atmosphere, and the mixture was stirred at room temperature for an hour. After the solvent was removed, the obtained residue was dissolved into DMA (1mL). Compound 67 (51mg, 0.16mmol), DIPEA (0.18mL, 0.18mmol) and HATU (60mg, 0.16mmol) were added into, and the mixture was stirred at room temperature for 3 days. After water was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 68 (40mg, 41%). LC/MS (ESI): 908 (M+1)

### Step 2

Compound 69 was obtained by the same synthesis method of Compound 2. LC/MS (ESI): 1046 (M+1)

### Step 3

TFA (0.5mL) was added into dichloromethane (1mL) solution of Compound 69 (24mg, 0.02mmol) under nitrogen atmosphere, and the mixture was stirred at room temperature for an hour. The solvent was removed. After the obtained residue was adjusted to pH about 5 with saturated sodium bicarbonate aqueous solution, the mixture was extracted with chloroform. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 1-30 (22mg, 96%). Measurement condition: A, LC/MS (ESI): 990 (M+1)

### Example 28

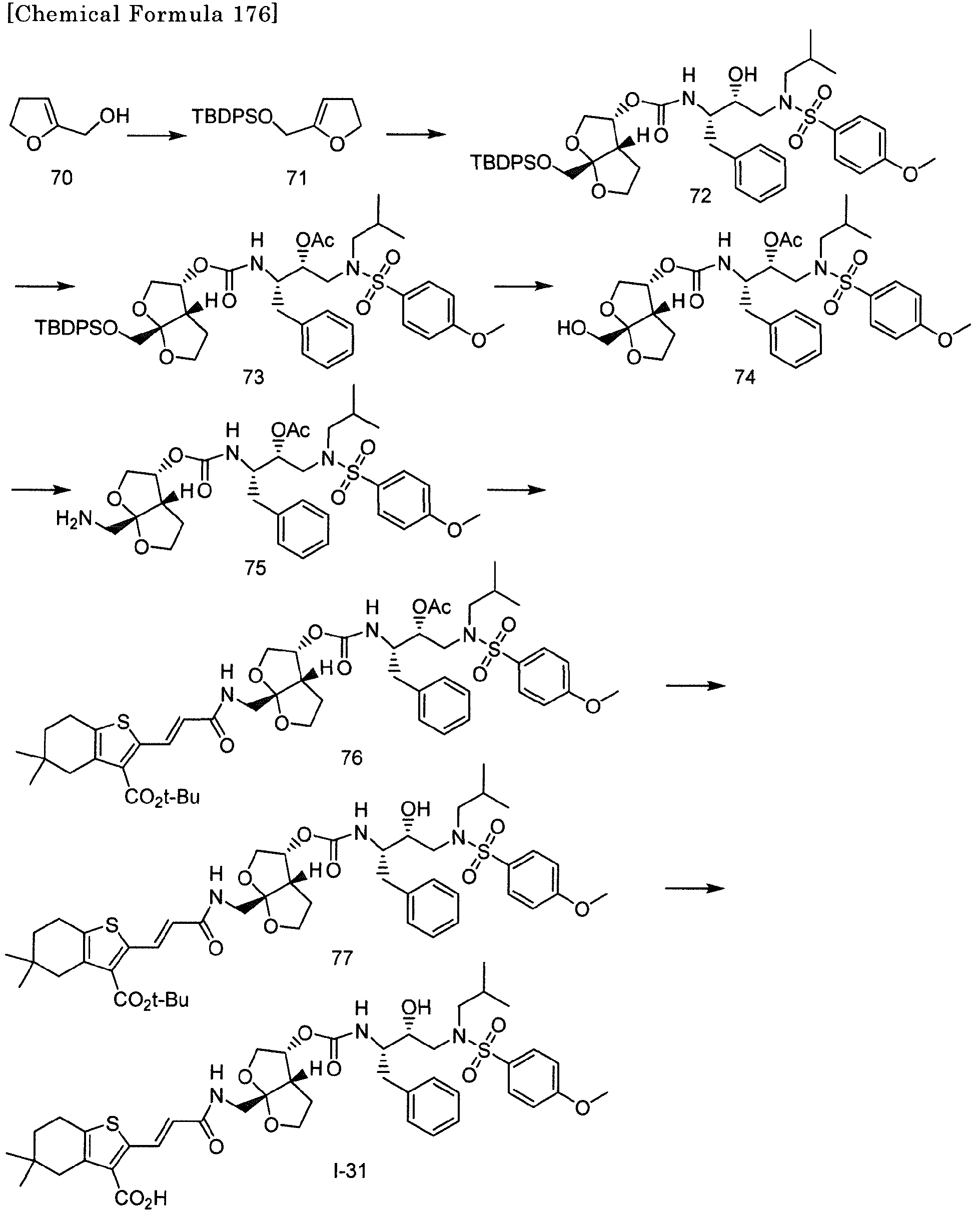

### Step 1

Imidazole (7.75g, 114mmol) and 1 mol/L TBDPSCl-CH₂Cl₂ solution (114mL, 114mmol) were added into THF (300mL) solution of Compound 70 (11.5g, 114mmol) under nitrogen flow, and the mixture was stirred at room temperature for 18 hours. After water was added into the reaction mixture, the mixture was extracted with ethyl acetate. After the organic layer was washed with water and brine, the mixture was dried with magnesium sulfate anhydrous, and the solvent was removed in vacuo, Compound 71 (15.9g, 16%).
¹H-NMR (CDCl₃)δ: 1.07 (s, 9H), 2.61 (t, 2H, J=2.1 Hz), 4.18 (s, 2H), 4.33 (t, 2H, J=2.1 Hz), 4.89 (s, 1H), 7.05-7.90 (m, 10H).

### Step 2

Compound 72 was obtained by the same synthesis method of Compound (I-1). LC/MS (ESI): 831 (M+1)

### Step 3

Pyridine (0.29mL, 3.61mmol) and acetic anhydride (0.34mL, 3.61mmol) were added into dichloromethane (30mL) solution of Compound 72 (3.0g, 3.61mmol), and the mixture was stirred at room temperature for 24 hours. After water was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 73 (2.99g, 95%).
LC/MS (ESI): 873 (M+1)

### Step 4

Acetic acid (0.15mL, 3.42mmol) and 1 mol/L TBAF-THF solution (17.1mL, 17.1mmol) were added into THF (30mL) solution of Compound 73 (2.99g, 3.42mmol), and the mixture was stirred at room temperature for 24 hours. After water was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 74 (1.30g, 60%).
LC/MS (ESI): 635 (M+1)

### Step 5

Pyridine (0.20mL, 2.46mmol) and anhydrous trifluoroemethanesulfonic acid (0.69g, 2.46mmol) were added into dichloromethane (20mL) solution of Compound 74 (1.30g, 2.05mmol), and the mixture was stirred at 0°C for 2 hours. After 2 mol/L hydrochloric acid aqueous solution was added into the reaction mixture, the mixture was extracted with ethyl acetate. After the organic layer was washed with water and brine, the mixture was dried with magnesium sulfate anhydrous, and the solvent was removed in vacuo.

The obtained residue was dissolved into DMF (10mL), and NaN₃ (0.40g, 6.14mmol) was added. The mixture was stirred at room temperature for 5 hours. Water was added into the reaction mixture, and the mixture was extracted with ethyl acetate. After the organic layer was washed with water and brine, the mixture was dried with magnesium sulfate anhydrous, and the solvent was removed in vacuo.

The obtained residue was dissolved into ethyl acetate (10mL), and Pd (OH)₂ (135mg, 10%wt) was added. The mixture was stirred under hydrogen flow at room temperature for 3 hours. The reaction mixture was filtrated, and filtrate was removed in vacuo. The obtained residue was purified by silica gel column chromatography (hexane-ethyl acetate) to give Compound 75 (1.10g, 3 Step 85%). LC/MS (ESI): 634 (M+1)

### Step 6

Triethylamine (15.9mg, 0.16mmol), Compound iii-79 (50mg, 0.08mmol), WSCD· HCl (18.2mg, 0.10mmol) and HOBt (12.8mg, 0.10mmol) were added into dichloromethane (5mL) solution of Compound 75 (27mg, 0.08mmol), and the mixture was stirred at room temperature for 3 hours. After water was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 76 (42mg, 59%).
LC/MS (ESI): 953 (M+1)

### Step 7

Potassium carbonate (11.1mg, 0.08mmol) was added into methanol (5mL) solution of Compound 76 (42mg, 0.04mmol), and the mixture was stirred at room temperature for 3 hours. After saturated ammonium chloride aqueous solution was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 77 (35mg, 96%).
LC/MS (ESI): 911 (M+1)

### Step 8

TFA (0.2mL) was added into dichloromethane (2mL) solution of Compound 77 (35mg, 0.04mmol), and the mixture was reacted at room temperature for 24 hours. The solvent was removed in vacuo. The obtained residue was purified by silica gel column chromatography (hexane-ethyl acetate) to give Compound 1-31 (32mg, 94%). Measurement condition: A, LC/MS (ESI): 855 (M+1)

### Example 29

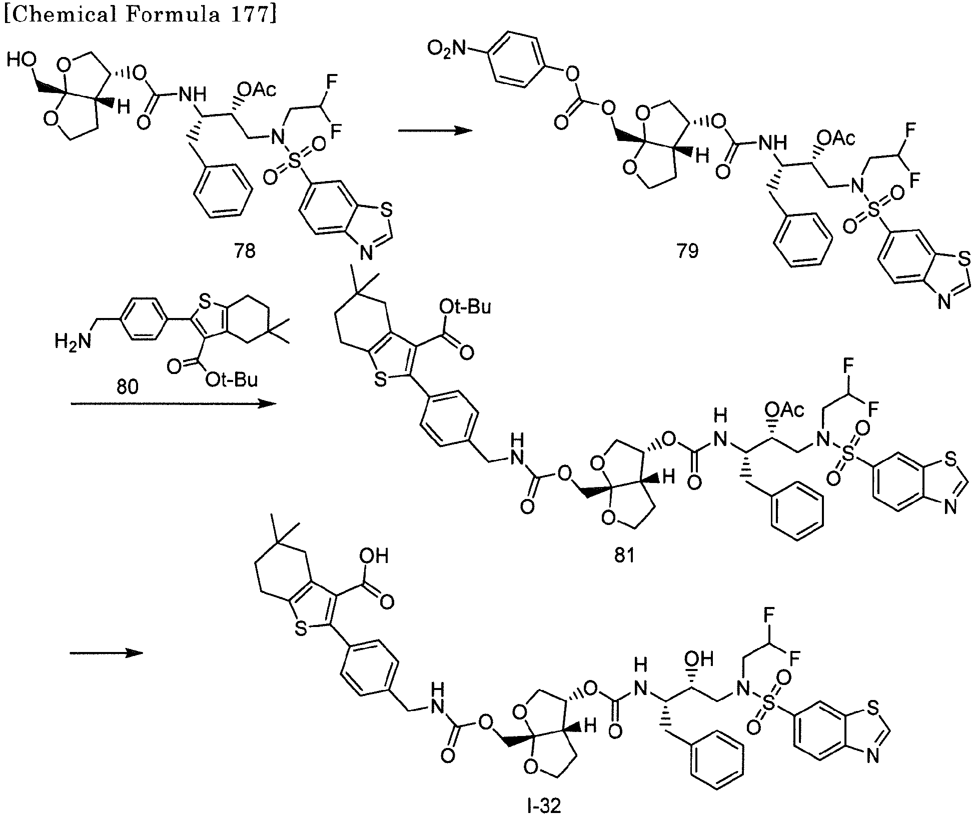

### Step 1

Dichloromethane (1mL), pyridine (11.81mg, 0.149mmol) and 4-nitrophenyl carbonochloridate (30mg, 0.149mmol) were added into Compound 78 (50mg, 0.075mmol) synthesized by the same method of Compound 74, and the mixture was stirred at 0°C for 3 hours. The solvent of reaction mixture was removed in vacuo. The obtained residue was purified by silica gel column chromatography (chloroform-methanol) to give Compound 79 (50mg, 80%).
LC/MS (ESI): 835 (M+1)

### Step 2

DIPEA (10.44mg, 0.081mmol) and THF (1ml) solution of Compound 80 (49.4mg, 0.059mmol) were added into acetonitrile (1mL) solution of Compound 79 (20mg, 0.054mmol), and the mixture was stirred at room temperature for 8 hours. After water was added into the reaction mixture, the mixture was extracted with ethyl acetate. After the organic layer was washed with water, the mixture was dried with magnesium sulfate anhydrous, and the solvent was removed in vacuo. The obtained residue was purified by silica gel column chromatography (chloroform-methanol) to give Compound 81 (33mg, 57%).
LC/MS (ESI): 1067 (M+1)

### Step 3

Compound 1-32 was obtained by the same synthesis method of Compound 1-31. Measurement condition: A, LC/MS (ESI): 969 (M+1)

### Example 30

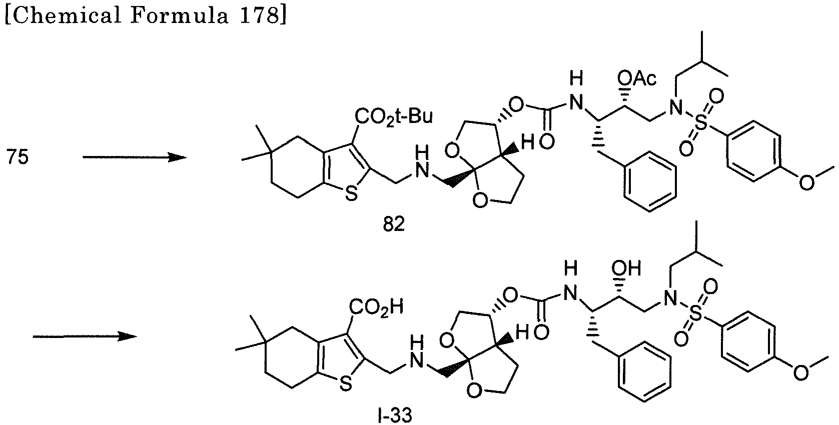

### Step 1

DIPEA (103mg, 0.80mmol) and Compound iii-81 (67mg, 0.18mmol) were added into DMA (5mL) solution of Compound 75 (100mg, 0.16mmol), and the mixture was stirred at 80°C for 18 hours. After saturated ammonium chloride aqueous solution was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 82 (95mg, 65%).
LC/MS (ESI): 912 (M+1)

### Step 2

Compound 1-33 was obtained by the same synthesis method of Compound 1-31. Measurement condition: A, LC/MS (ESI): 815 (M+1)

### Example 31

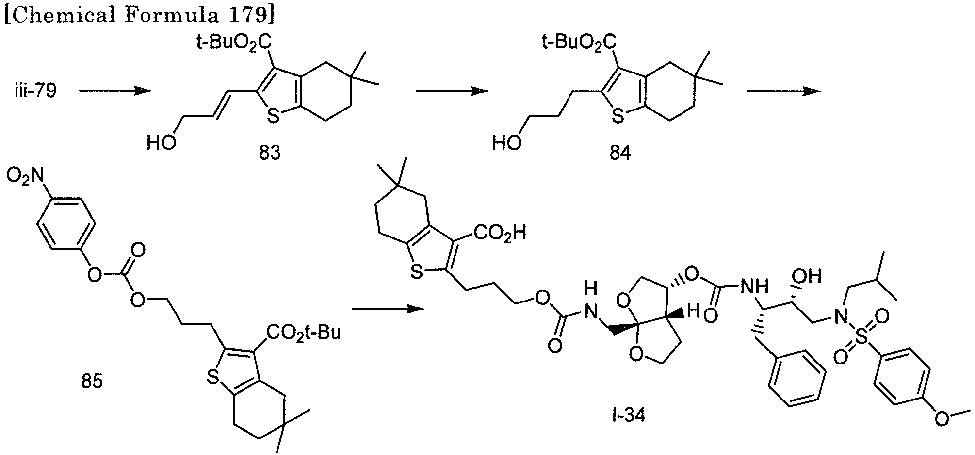

### Step 1

Triethylamine (0.32mL, 2.27mmol) and isobuthylchlorocarbonate (248mg, 1.82mmol) were added into THF (15mL) solution of Compound iii-79 (510mg, 1.52mmol), and the mixture was stirred at 0°C for 5 hours. After methanol (5mL) was added into reaction mixture, NaBH₄ (115mg, 3.03mmol) was added. The mixture was stirred at 0°C for 10 minutes after that at room temperature for 5 hours. After saturated ammonium chloride aqueous solution was added into the reaction mixture, the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 83 (280mg, 57%).
LC/MS (ESI): 323 (M+1)

### Step 2

Pd (OH)₂ (87mg, 0.12mmol) was added into ethyl acetate (5mL) solution of Compound 83 (400mg, 1.24mmol), and the mixture was stirred under hydrogen flow at room temperature for 2 hours. After the reaction mixture was filtrated, the solvent was removed in vacuo. The obtained residue was purified by silica gel column chromatography (hexane-ethyl acetate) to give Compound 84 (403mg, 100%). LC/MS (ESI): 325 (M+1)

### Step 3

Compound 85 was obtained by the same synthesis method of Compound 79. LC/MS (ESI): 490 (M+1)

### Step 4

Compound 1-34 was obtained by the same synthesis method of Compound 1-32. Measurement condition: A, LC/MS (ESI): 886 (M+1)

### Example 32

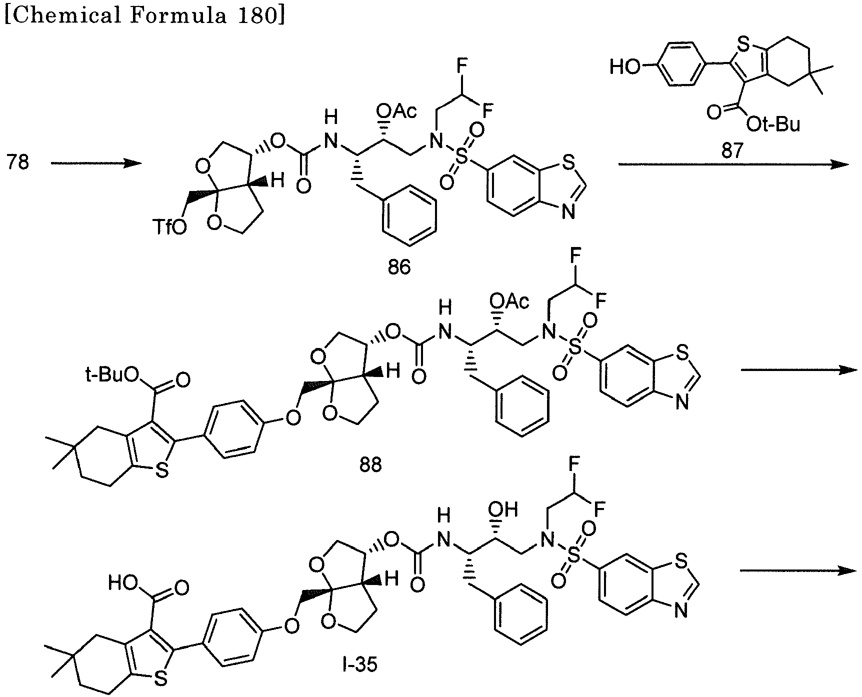

### Step 1

Pyridine (0.058mL, 0.716mmol) and anhydrous trifluoromethanesulfonic acid (0.048mL, 0.286mmol) were added into dichloromethane (1.6mL) solution of Compound 78 (80mg, 0.12mmol) under ice-cold, and the mixture was stirred at 0°C for 3 hours. The solvent was removed in vacuo. The obtained residue was purified by silica gel column chromatography (chloroform-methanol) to give Compound 86 (29mg, 30%).
LC/MS (ESI): 802 (M+1)

### Step 2

Sodium hydride (5.78mg, 0.144mmol) was added into DMF (0.87mL) solution of Compound 87 (15.6mg, 0.043mmol), and the mixture was stirred at room temperature for 1.5 hours. Compound 86 (29mg, 0.036mmol) was added into the reaction mixture, and the mixture was stirred at room temperature for 14 hours. After saturated ammonium chloride aqueous solution was added into the reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (hexane-ethyl acetate) to give Compound 88 (15.8mg, 43%). LC/MS (ESI): 1010 (M+1)

### Step 3

Compound 1-35 was obtained by the same synthesis method of Compound 1-31. Measurement condition: A, LC/MS (ESI): 912 (M+1)

### Example 33

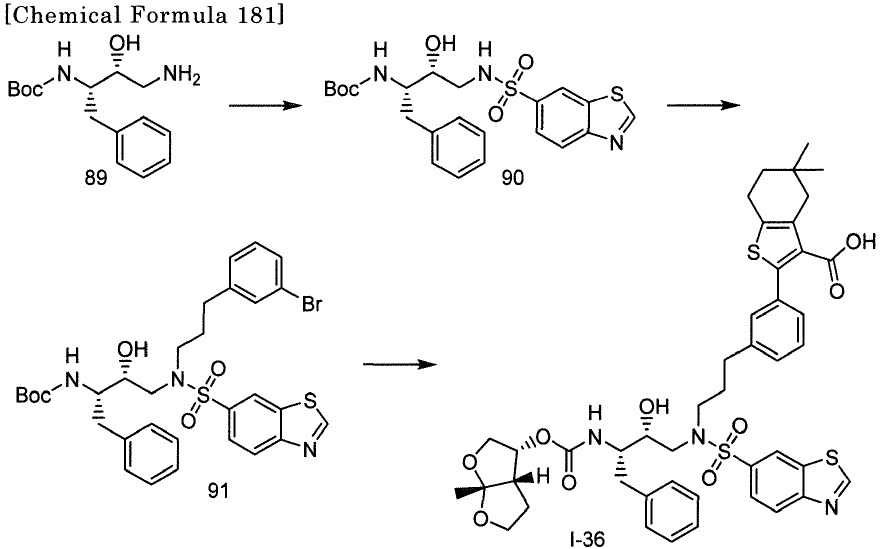

### Step 1

Pyridine (734mg, 9.27mmol) and benzo[d]-thiazole-6-sulfonyl chloride (2.17g, 9.27mmol) were added into dichloromethane (40mL) solution of Compound 89 (2.0g, 7.13mmol), and the mixture was stirred at room temperature for 18 hours. After the reaction mixture was condensed, the obtained residue was purified by silica gel column chromatography (chloroform-methanol) to give Compound 90 (931mg, 27%). LC/MS (ESI): 478 (M+1)

### Step 2

1-bromo-3- (3-bromopropyl)benzene (69.8mg, 0.251mmol) and potassium carbonate (43.4mg, 0.314mmol) were added into acetonitrile (1mL) solution of Compound 90 (100mg, 0.209mmol), and the mixture was stirred for 3 hours under heat reflux. After the reaction mixture was extracted with ethyl acetate, the solvent was removed in vacuo. The obtained residue was purified by silica gel column chromatography (hexane-ethyl acetate) to give Compound 91 (106mg, 75%).
LC/MS (ESI): 674 (M+1)

### Step 3

Compound I-36 was obtained by the same synthesis method of Compound I-1. Measurement condition: A, LC/MS (ESI): 874 (M+1)

### Example 34

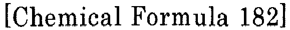

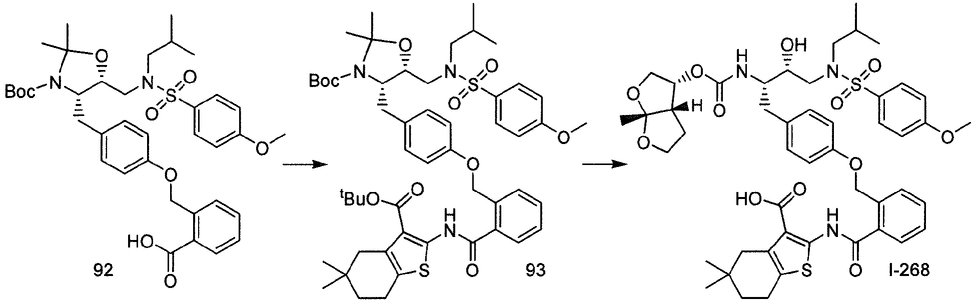

### Step 1

1-Chloro-N,N,2-trimethyl-1-propenylamine (0.046mL, 0.344mmol) was added into THF (1.6mL) solution of Compound 92 (80.0mg, 0.115mmol) under ice-cold, and the mixture was stirred under ice-cold for an hour. Compound iii-2 (97.0mg, 0.344mmol) and pyridine (0.056mL, 0.689mmol) were added into the reaction mixture, and the mixture was stirred at room temperature for 4 hours. After saturated ammonium chloride aqueous solution was added into reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (hexane-ethyl acetate) to give Compound 93 (75.0mg, 68%).
LC/MS (ESI): 961 (M+1)

### Step 2

Compound I-268 was obtained by the same synthesis method of Compound I-1. Measurement condition: A, LC/MS (ESI): 934 (M+1)

### Example 35

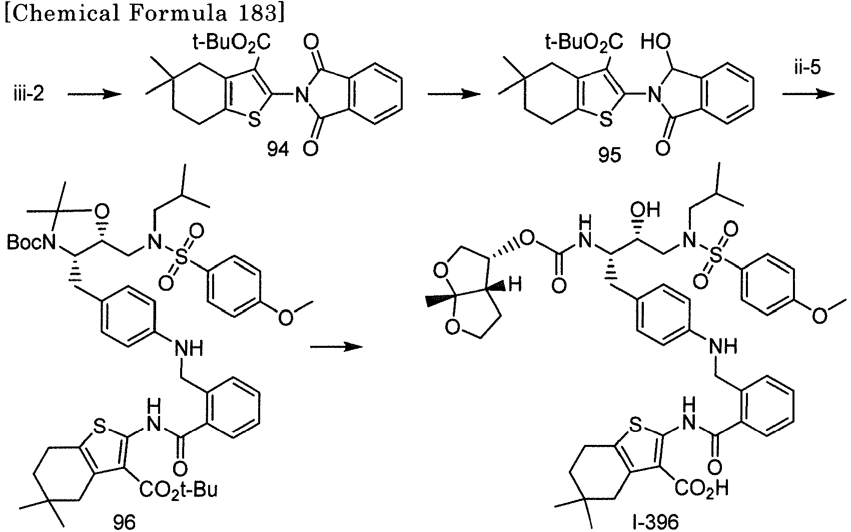

### Step 1

Suspension of Compound iii-2 (10.0g, 35.5mmol) and dichloromethane solution (100mL of phthalic anhydride (5.26g, 35.5mmol) was stirred at room temperature for 2 hours. N,N,N',N'-tetramethyl-O-(benzotriazole-1-yl)uronium Hexafluorophosphate (16.2g, 42.6mmol) and triethylamine (7.39mL, 53.3mmol) were added into the reaction mixture, and the mixture was stirred at room temperature for 16 hours. After 1 mol/L hydrochloric acid aqueous solution was added into the reaction mixture, the mixture was extracted with dichloromethane. Insoluble matter was filtrated and removed. After the organic layer was washed with water, the solvent was removed in vacuo. The obtained solids were washed with isopropanol to give Compound 94 (13.2g, 91%). ¹ H-NMR (CDCl₃) δ: 1.03 (s, 6H), 1.25 (s, 9H), 1.61 (t, J = 6.3 Hz, 2H), 2.65 (s, 2H), 2.77 (t, J = 6.3 Hz, 2H), 7.77-7.83 (m, 2H), 7.94-7.99 (m, 2H).

### Step 2

Sodium borohydride (1.82g, 48.2mmol) was added into methanol (66mL)-THF (132mL) solution of Compound 94 (13.2g, 32.1mmol) at -40°C, and the mixture was stirred with heating to -25°C for 80 minutes. After 1 mol/L hydrochloric acid ethyl acetate solution was added by dropwise into the reaction mixture, the mixture was extracted with ethyl acetate. After the organic layer was washed with water, the mixture was dried with magnesium sulfate anhydrous, and the solvent was removed in vacuo. The obtained residue was washed with diisopropyl ether to give Compound 95 (13.5g).
¹ H-NMR (CDCl₃) δ: 1.01 (s, 3H), 1.04 (s, 3H), 1.37 (s, 9H), 1.60 (t, J = 5.8 Hz, 2H), 2.51 (d, J = 16.9 Hz, 1H), 2.62 (d, J = 16.9 Hz, 1H), 2.74 (t, J = 5.8 Hz, 2H), 4.10 (d, J = 10.4 Hz, 1H), 6.06 (d, J = 10.4 Hz, 1H), 7.56 (ddd, J = 7.3, 7.3, 1.8 Hz, 1H), 7.63-7.70 (m, 2H), 7.88 (d, J = 7.3 Hz, 1H).

### Step 3

Methanol (45mL)-dichloromethane (15mL)-acetic acid (3mL) solution of Compound ii-5 (3.00g, 5.34mmol) and Compound 95 (2.21g, 5.34mmol) was stirred under nitrogen atmosphere at room temperature for 5 minutes. 2-Picolineborane (857mg, 8.01mmol) was added into the reaction mixture, and the mixture was stirred at room temperature for 18 hours. The reaction mixture was diluted with dichloromethane, and the mixture was washed sodium hydrogen carbonate aqueous solution and water. After the mixture was dried with magnesium sulfate anhydrous, the solvent was removed in vacuo. The obtained residue was purified by silica gel column chromatography (hexane-ethyl acetate) to give Compound 96 (3.62g, 71%). LC/MS (ESI): 959 (M+1)

### Step 4

Compound I-396 was obtained by the same synthesis method of Compound I-1. Measurement condition: A, LC/MS (ESI): 934 (M+1)

### Example 36

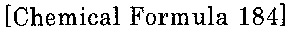

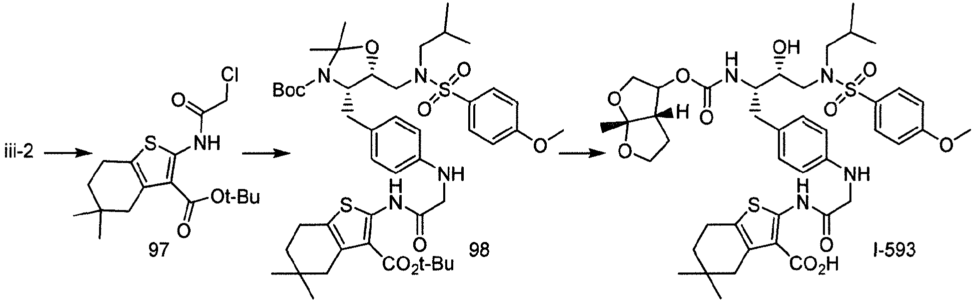

### Step 1

Chloroacetyl chloride (0.040mL, 0.497mmol) was added into chloroform (1.4mL) solution of Compound iii-2 (70.0mg, 0.249mmol), and the mixture was stirred under heat reflux for 2 hours. The reaction mixture was condensed in vacuo to give Compound 97 (29.0mg, 33%).
¹H-NMR (CDCl₃) δ: 0.98 (s, 6H), 1.56 (t, J = 6.4 Hz, 2H), 1.60 (s, 9H), 2.53 (s, 2H), 2.67 (t, J = 6.4 Hz, 2H), 4.23 (s, 2H), 12.13 (s, 1H).

### Step 2

DMA (0.58mL) solution of Compound ii-5 (45.5mg, 0.081mmol) and Compound 97 (29.0mg, 0.081mmol) was stirred at 120°C for 2.5 hours. After water was added into the reaction mixture, the mixture was extracted with ethyl acetate. After the organic layer was washed with water and brine, the mixture was dried with anhydrous sodium sulfate, and the solvent was removed in vacuo. The obtained residue was purified by silica gel column chromatography (hexane-ethyl acetate) to give Compound 98 (38.0mg, 53%).
LC/MS (ESI): 883 (M+1)

### Step 3

Compound I-593 was obtained by the same synthesis method of Compound 1-1. Measurement condition: A, LC/MS (ESI): 857 (M+1)

### Example 37

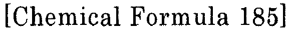

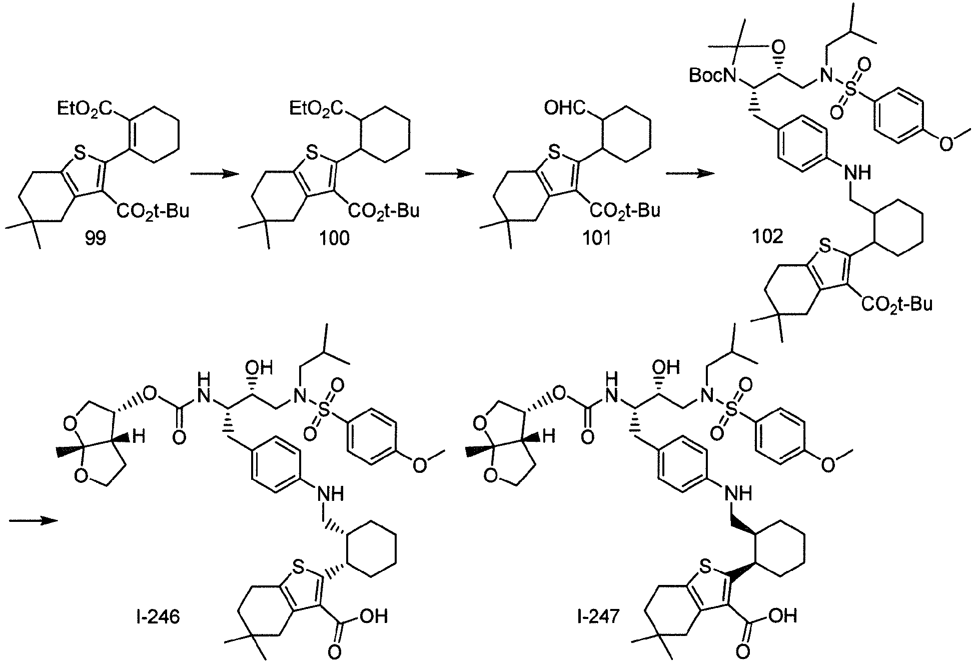

### Step 1

Acetic acid (2.25mL) and 10%palladium hydroxide (400mg, 0.285mmol) were added into methanol (4.5mL)-THF (4.5mL) solution of Compound 99 (900mg, 2.15mmol), and the mixture was stirred under hydrogen atmosphere (5 atm) at 60°C for 6 hours. After the insoluble matter was filtrated and removed, the solvent was removed in vacuo. The obtained residue was purified by silica gel column chromatography (hexane-ethyl acetate) to give Compound 100 (408mg, 45%). ¹H-NMR (CDCl₃) δ: 0.96 (s, 3H), 0.97 (s, 3H), 1.07 (t, J = 7.1 Hz, 3H), 1.23-1.54 (m, 4H), 1.58 (s, 9H), 1.68-1.81 (m, 3H), 1.84-1.89 (m, 1H), 1.98-2.04 (m, 1H), 2.21 (ddd, J = 24.0, 11.8, 3.4 Hz, 1H), 2.51 (s, 2H), 2.66 (t, J = 6.5 Hz, 2H), 3.08 (ddd, J = 4.2, 4.2, 4.2 Hz, 1H), 3.87 (ddd, J = 11.5, 4.2, 4.2 Hz, 1H), 3.96 (q, J = 7.1 Hz, 2H).

### Step 2

Compound 101 was obtained by the same synthesis method of Compound iii-90. ¹H-NMR (CDCl₃) δ: 0.98 (s, 6H), 1.39-1.65 (m, 15H), 1.76-1.89 (m, 2H), 1.94-2.01 (m, 1H), 2.15-2.22 (m, 1H), 2.53 (s, 2H), 2.68 (t, J = 6.3 Hz, 2H), 2.95-3.01 (m, 1H), 3.99 (ddd, J = 11.8, 4.1, 4.1 Hz, 1H), 9.75 (s, 1H).

### Step 3

Compound 102 was obtained by the same synthesis method of Compound 37. LC/MS (ESI): 922 (M+1)

### Step 4

Compound I-246 and Compound I-247 was obtained by the same synthesis method of Compound I-9-1 and Compound I-9-2.
Compound I-24; Measurement condition: A, LC/MS (ESI): 896 (M+1)
Compound I-24; Measurement condition: A, LC/MS (ESI): 896 (M+1)

### Example 38

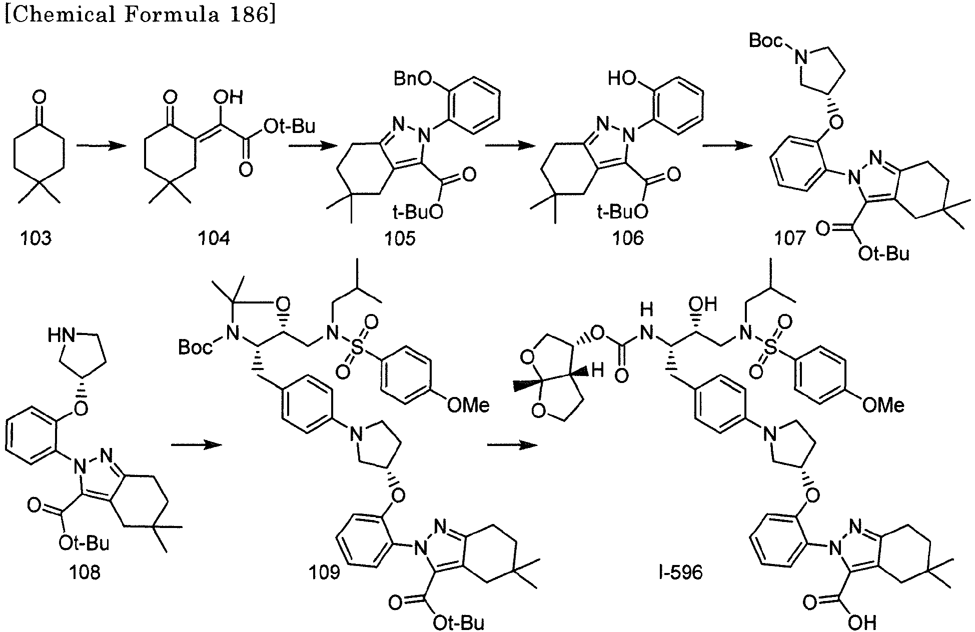

### Step 1

THF solution (41.4mL, 41.4mmol) of 1 mol/L lithium bis(trimethylsilyl)amide was added into THF (50mL) solution of Compound 103 (5.23g, 41.4mmol) under nitrogen atmosphere at -78°C, and the mixture was stirred at -78°C for 30 minutes. THF (20mL) solution of di-t-butyl oxalate (8.38g, 41.4mmol) was added into the reaction mixture, and the mixture was stirred at room temperature with heating for 2.5 hours. The reaction mixture was poured to mixture of ethyl acetate and saturated ammonium chloride aqueous solution, and the mixture was extracted with ethyl acetate. After the organic layer was washed with brine, the mixture was dried with magnesium sulfate anhydrous, and the solvent was removed in vacuo. The obtained residue was purified by silica gel column chromatography (hexane-ethyl acetate) to give Compound 104 (7.45g, 71%).
¹H-NMR (CDCl₃) δ: 0.98 (s, 6H), 1.51 (t, J = 6.8 Hz, 2H), 1.57 (s, 9H), 2.18 (s, 2H), 2.44 (t, J = 6.8 Hz, 2H), 14.97 (s, 1H).

### Step 2

Ethanol solution (30mL) of [2-(benzyloxy)phenyl]hydrazine (3.51g, 16.4mmol) was added by dropwise into ethanol (70.2mL) solution of Compound 104 (4.17g, 16.4mmol) under ice-cold. After the mixture was stirred at 0°C for 1.5 hours, the mixture was stirred at 80°C for 3 hours. The reaction mixture was condensed in vacuo. The obtained residue was purified by silica gel column chromatography (hexane-ethyl acetate) to give Compound 105 (3.6g, 51%).
¹H-NMR (CDCl₃) δ: 1.06 (s, 6H), 1.25 (s, 9H), 1.64 (t, J = 6.6 Hz, 2H), 2.61 (s, 2H), 2.76 (t, J = 6.6 Hz, 2H), 4.99 (s, 2H), 6.99 (dd, J = 7.6, 1.2 Hz, 1H), 7.06 (ddd, J = 7.6, 7.6, 1.2 Hz, 1H), 7.17-7.21 (m, 2H), 7.21-7.31 (m, 3H), 7.33 (ddd, J = 7.6, 7.6, 1.6 Hz, 1H), 7.44 (dd, J = 7.6, 1.6 Hz, 1H).

### Step 3

10% Palladium hydroxide (350mg, 0.250mmol) was added into ethanol (144mL) solution of Compound 105 (3.60g, 8.32mmol), and the mixture was stirred under hydrogen atmosphere at room temperature for 2.1 hours. After the insoluble matter was filtrated and removed, the reaction mixture was condensed in vacuo. After water was added into the obtained residue, the mixture was extracted with dichloromethane. After the organic layer was dried with magnesium sulfate anhydrous, the solvent was removed in vacuo, Compound 106 (2.89g).
¹H-NMR (CDCl₃) δ: 1.05 (s, 6H), 1.41 (s, 9H), 1.64 (t, J = 6.6 Hz, 2H), 2.57 (s, 2H), 2.74 (t, J = 6.6 Hz, 2H), 6.91 (ddd, J = 8.2, 8.2, 1.4 Hz, 1H), 7.08 (dd, J = 8.2, 1.4 Hz, 1H), 7.12 (dd, J = 8.2, 1.4 Hz, 1H), 7.23 (ddd, J = 8.2, 8.2, 1.4 Hz, 1H), 7.93 (s, 1H).

### Step 4

Compound 107 was obtained by the same synthesis method of Compound 16. LC/MS (ESI): 512 (M+1)

### Step 5

Compound 107 (120mg, 0.235mmol) was dissolved with acetonitrile (1.2mL), and p-toluenesulfonic acid hydrate (223mg, 1.17mmol) was added. The mixture was stirred at room temperature for 2.5 hours. After saturated sodium hydrogen carbonate aqueous solution was added into the reaction mixture, the mixture was extracted with chloroform. After the organic layer was washed with water and brine, the mixture was dried with magnesium sulfate anhydrous, and the solvent was removed in vacuo. The obtained residue was purified by silica gel column chromatography (chloroform-methanol) to give Compound 108 (97.0mg, 74%). LC/MS (ESI): 412 (M+1)

### Step 6

Compound 108 (71.0mg, 0.173mmol), cesium carbonate (112mg, 0.345mmol), 2-dicyclohexylphosphino-2',6'-diisopropoxybiphenyl (8.05mg, 0.017mmol) and 2nd generation RuPhos pre-catalyst (13.4mg, 0.017mmol) were added into toluene (1.5mL) solution of Compound ii-3 (120mg, 0.173mmol), and the mixture was stirred at 110°C for 17 hours. After the insoluble matter was filtrated and removed, water was added into the reaction mixture, and the mixture was extracted with ethyl acetate. After the organic layer was washed with water and brine, the mixture was dried with magnesium sulfate anhydrous, and the solvent was removed in vacuo. The obtained residue was purified by silica gel column chromatography (hexane-ethyl acetate) to give Compound 109 (150mg, 91%).
LC/MS (ESI): 956 (M+1)

### Step 7

Compound I-596 was obtained by the same synthesis method of Compound 1-1. Measurement condition: A, LC/MS (ESI): 931 (M+1)

### Example 39

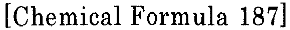

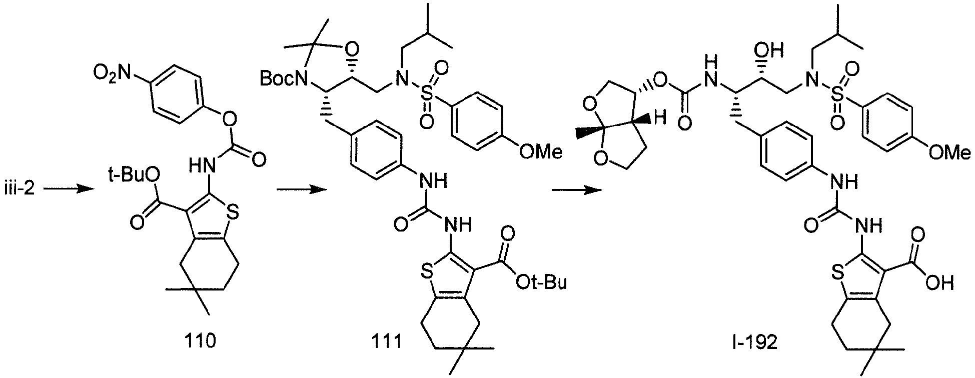

### Step 1

Compound 110 was obtained by the same synthesis method of Compound 79. LC/MS (ESI): 447 (M+1)

### Step 2

Triethylamine (0.050mL, 0.364mmol) was added into THF (1.5mL) solution of Compound ii-5 (68.2mg, 0.121mmol) and Compound 110 (88.4mg, 81.0mg, 0.182mmol), and the mixture was stirred at 50°C for 4 hours. After water was added into the reaction mixture, the mixture was extracted with ethyl acetate. After the organic layer was washed with brine, the mixture was dried with magnesium sulfate anhydrous, and the solvent was removed in vacuo. The obtained residue was purified by silica gel column chromatography (hexane-ethyl acetate) to give Compound 111 (76.9mg, 73%).
LC/MS (ESI): 869 (M+1)

### Step 3

Compound I-192 was obtained by the same synthesis method of Compound I-1. Measurement condition: A, LC/MS (ESI): 843 (M+1)

### Example 40

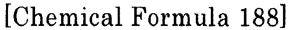

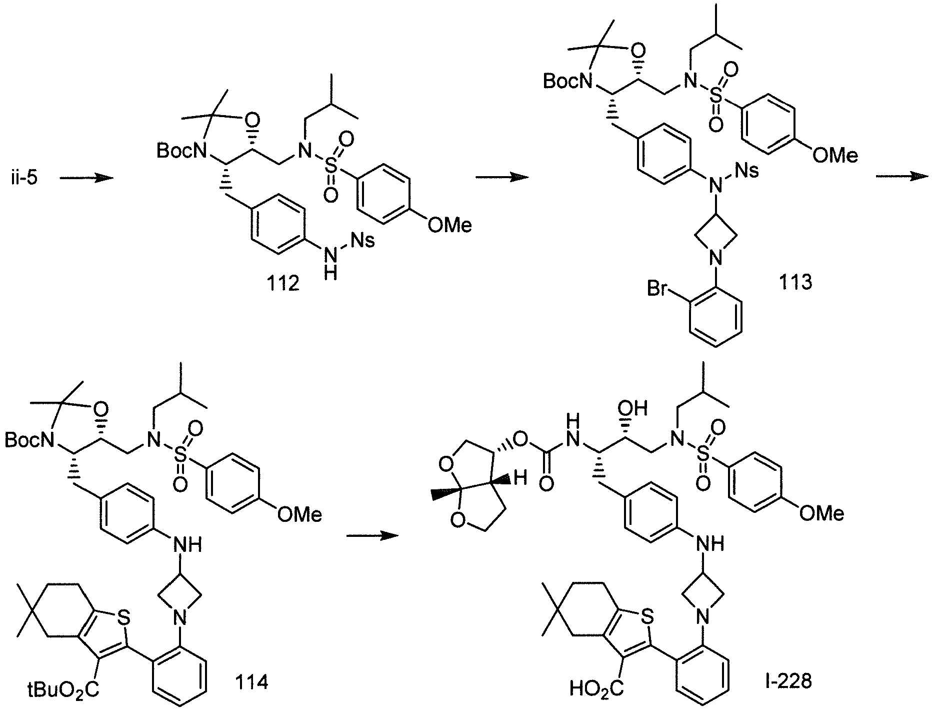

### Step 1

Pyridine (0.034mL, 0.427mmol) and 2-nitrobenzenesulfonyl chloride (87.0mg, 0.392mmol) were added into dichloromethane (2mL) solution of Compound ii-5 (200mg, 0.356mmol) under ice-cold, and the mixture was stirred at room temperature for 2 hours. After saturated ammonium chloride aqueous solution was added into the reaction mixture, the mixture was extracted with chloroform. After the organic layer was washed with brine, the mixture was dried with anhydrous sodium sulfate, and the solvent was removed in vacuo. The obtained residue was purified by silica gel column chromatography (hexane-ethyl acetate) to give Compound 112 (216mg, 81%).
LC/MS (ESI): 747 (M+1)

### Step 2

Compound 113 was obtained by the same synthesis method of Compound 16. LC/MS (ESI): 956,958 (M+1)

### Step 3

Acetonitrile (1mL) suspension of Compound 113 (148mg, 0.130mmol), dodecane-1-thiol (0.154mL, 0.648mmol) and cesium carbonate (254mg, 0.778mmol) was stirred at 50°C for 20 hours. After water was added into the reaction mixture, the mixture was extracted with ethyl acetate. After the organic layer was washed with brine, the mixture was dried with anhydrous sodium sulfate, and the solvent was removed in vacuo. The obtained residue was purified by silica gel column chromatography (hexane-ethyl acetate) to give Compound 114 (108mg, 2 Step, 70%). LC/MS (ESI): 958 (M+1)

### Step 4

Compound I-228 was obtained by the same synthesis method of Compound I-1.

### Measurement condition: A, LC/MS (ESI): 931 (M+1)

The following compounds were synthesized by the same synthesis method of above Reference example or Example by using compounds of Reference example or known compounds. The LC/MS measurement results of each compounds were represented as following.

In the tables, "No." is compound number, "Structure" is chemical structure formula, "method" is above LC/MS (liquid chromatography-mass spectrometry) measurement condition, and "RT" is retention time (minutes).

**[Table 1]**

| | | | | | | |
|---|---|---|---|---|---|---|
| 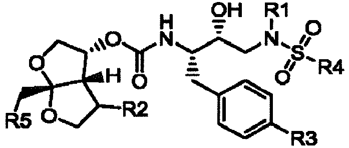 | | | | | | |

| No. | R1 | R2 | R3 | R4 | R5 | salt |
|---|---|---|---|---|---|---|
| I-37 | 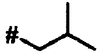 | H | 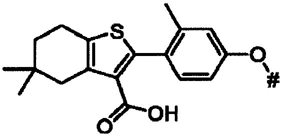 | 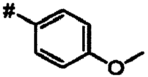 | H | |
| I-38 | 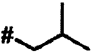 | H | 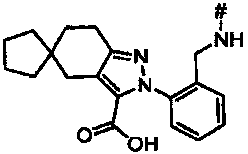 | 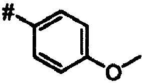 | H | |
| I-39 | 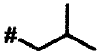 | H | 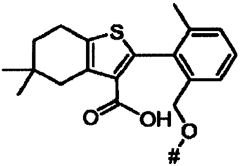 | 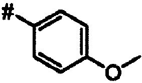 | H | |
| I-40 | 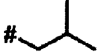 | H | 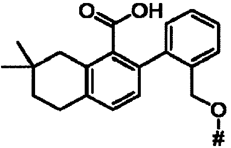 | 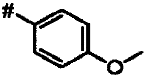 | H | |
| I-41 | 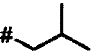 | H | 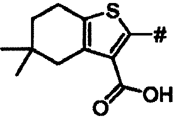 | 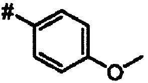 | H | |
| I-42 | 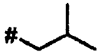 | H | 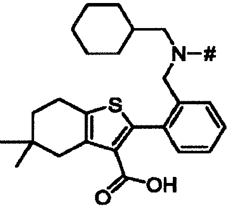 | 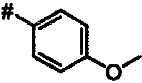 | H | |
| I-43 | 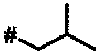 | H | 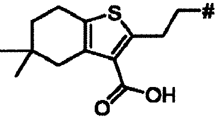 | 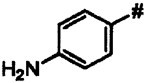 | H | |
| I-44 | 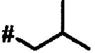 | H | 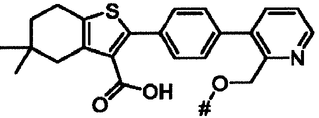 | 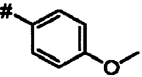 | H | |

**[Table 2]**

| No. | R1 | R2 | R3 | R4 | R5 | salt |
|---|---|---|---|---|---|---|
| I-45 | 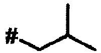 | H | 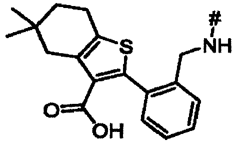 | 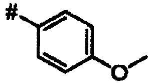 | H | |
| I-46 | 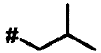 | H | 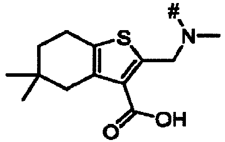 | 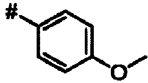 | H | |
| I-47 | 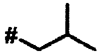 | H | 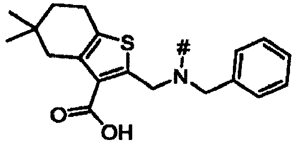 | 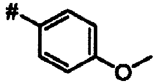 | H | |
| I-48 | 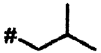 | H | 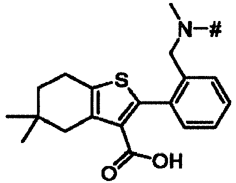 | 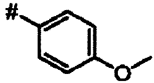 | H | |
| I-49 | 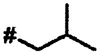 | H | 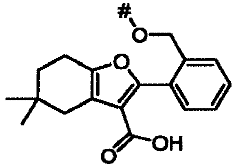 | 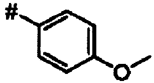 | H | |
| I-50 | 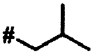 | H | 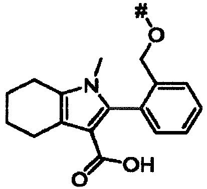 | 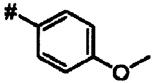 | H | |
| I-51 | 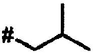 | H | 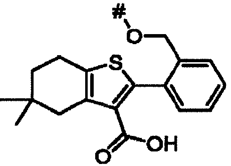 | 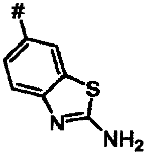 | H | |
| I-52 | 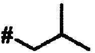 | H | 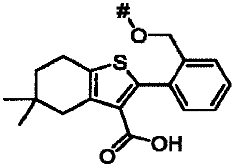 | 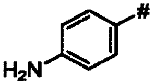 | H | |

**[Table 3]**

| No. | R1 | R2 | R3 | R4 | R5 | salt |
|---|---|---|---|---|---|---|
| I-53 | 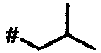 | H | 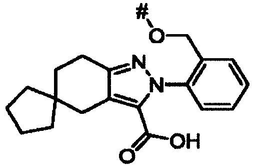 | 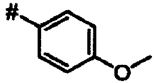 | H | |
| I-54 | 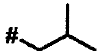 | H | 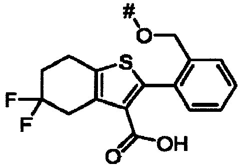 | 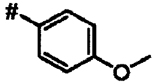 | H | |
| I-55 | 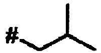 | 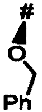 | 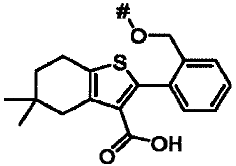 | 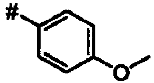 | H | |
| I-56 | 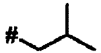 | 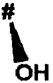 | 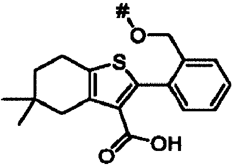 | 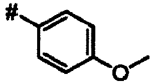 | H | |
| I-57 | 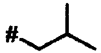 | H | 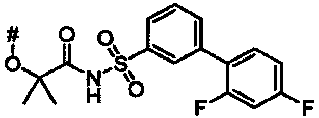 | 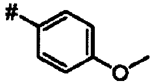 | H | |
| I-58 | 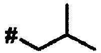 | H | 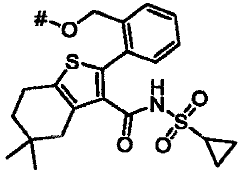 | 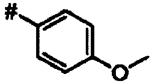 | H | |
| I-59 | 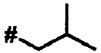 | H | 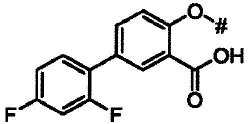 | 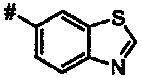 | H | |
| I-60 | 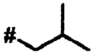 | H | 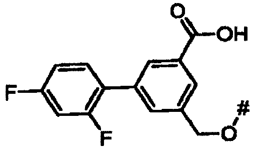 | 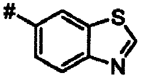 | H | |
| I-61 | 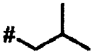 | H | 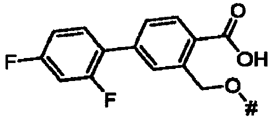 | 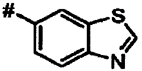 | H | |

**[Table 4]**

| No. | R1 | R2 | R3 | R4 | R5 | salt |
|---|---|---|---|---|---|---|
| I-62 | 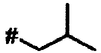 | H | 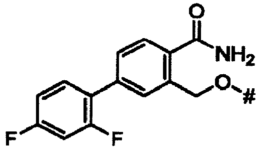 | 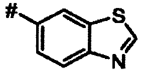 | H | |
| I-63 | 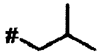 | H | 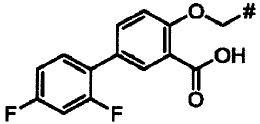 | 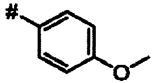 | H | |
| I-64 | 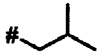 | H | 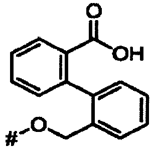 | 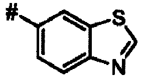 | H | |
| I-65 | 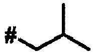 | H | 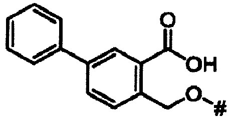 | 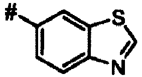 | H | |
| I-66 | 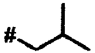 | H | 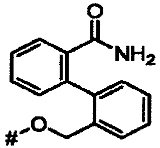 | 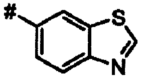 | H | |
| I-67 | 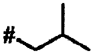 | H | 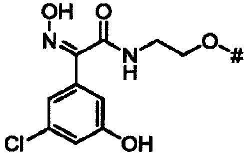 | 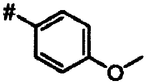 | H | |
| I-68 | 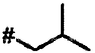 | H | 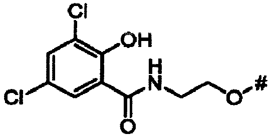 | 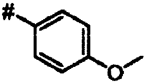 | H | |
| I-69 | 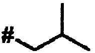 | H | 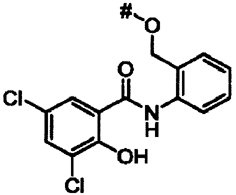 | 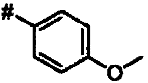 | H | |
| I-70 | 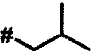 | H | 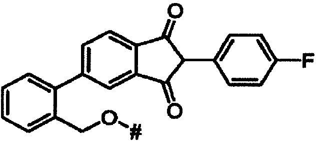 | 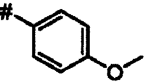 | H | |

**[Table 5]**

| No. | R1 | R2 | R3 | R4 | R5 | salt |
|---|---|---|---|---|---|---|
| I-71 | 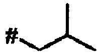 | H | 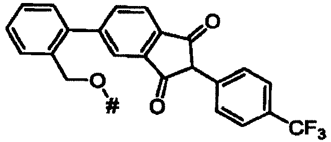 | 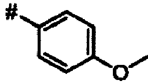 | H | |
| I-72 | 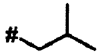 | H | 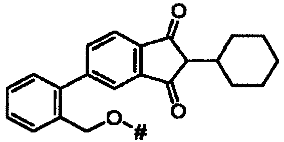 | 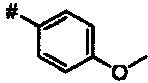 | H | |
| I-73 | 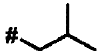 | H | 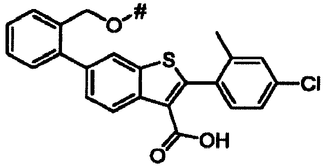 | 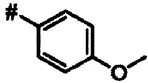 | H | |
| I-74 | 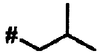 | H | 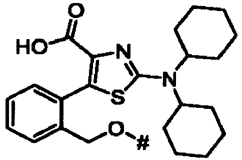 | 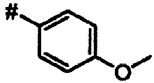 | H | |
| I-75 | 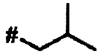 | H | 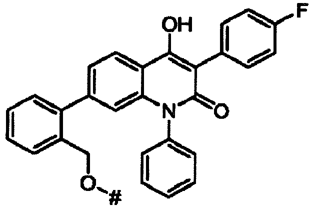 | 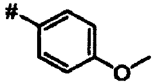 | H | |
| I-76 | 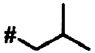 | H | 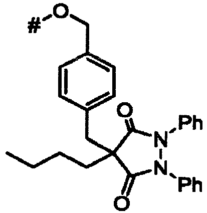 | 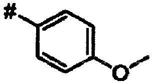 | H | |
| I-77 | 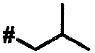 | H | 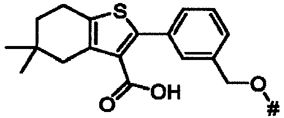 | 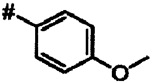 | H | |
| I-78 | 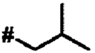 | H | 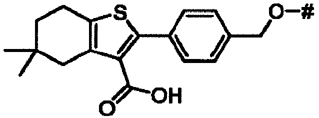 | 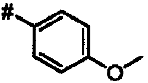 | H | |

**[Table 6]**

| No. | R1 | R2 | R3 | R4 | R5 | salt |
|---|---|---|---|---|---|---|
| I-79 | 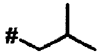 | H | 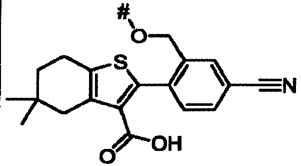 | 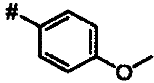 | H | |
| I-80 | 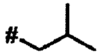 | H | 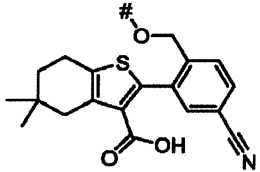 | 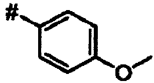 | H | |
| I-81 | 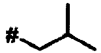 | H | 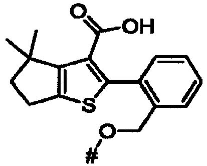 | 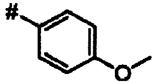 | H | |
| I-82 | 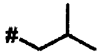 | H | 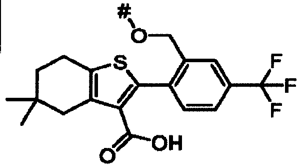 | 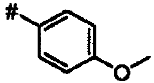 | H | |
| I-83 | 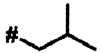 | H | 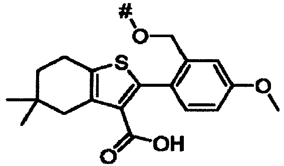 | 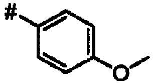 | H | |
| I-84 | 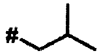 | H | 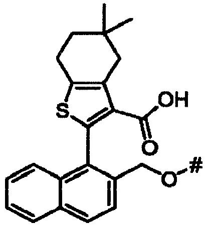 | 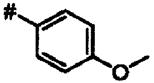 | H | |
| I-85 | 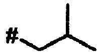 | H | 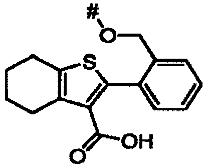 | 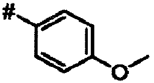 | H | |
| I-86 | 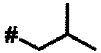 | H | 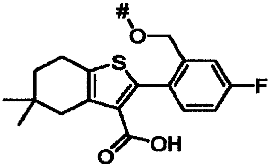 | 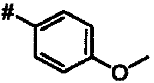 | H | |

**[Table 7]**

| No. | R1 | R2 | R3 | R4 | R5 | salt |
|---|---|---|---|---|---|---|
| I-87 | 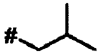 | H | 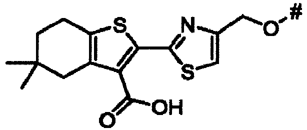 | 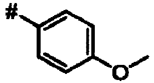 | H | |
| I-88 | 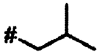 | H | 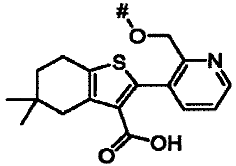 | 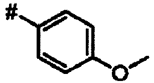 | H | |
| I-89 | 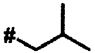 | H | 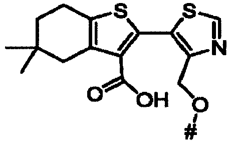 | 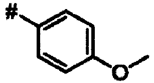 | H | |
| I-90 | 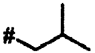 | H | 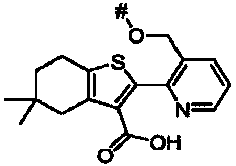 | 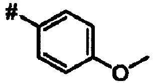 | H | |
| I-91 | 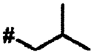 | H | 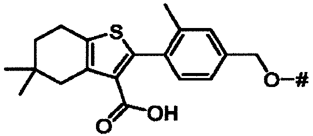 | 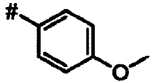 | H | |
| I-92 | 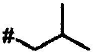 | H | 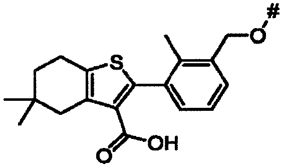 | 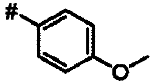 | H | |
| I-93 | 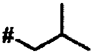 | H | 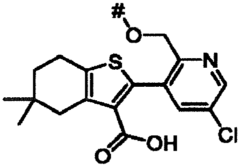 | 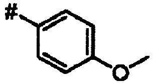 | H | |
| I-94 | 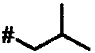 | H | 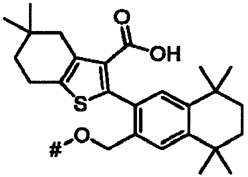 | 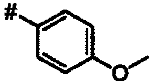 | H | |

**[Table 8]**

| No. | R1 | R2 | R3 | R4 | R5 | salt |
|---|---|---|---|---|---|---|
| I-95 | 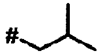 | H | 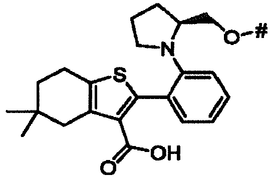 | 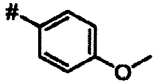 | H | |
| I-96 | 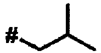 | H | 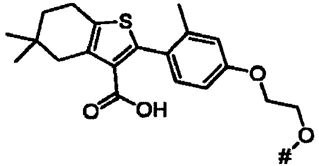 | 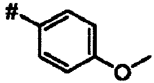 | H | |
| I-97 | 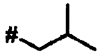 | H | 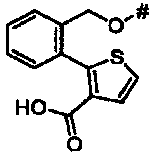 | 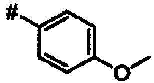 | H | |
| I-98 | 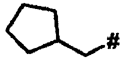 | H | 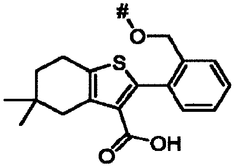 | 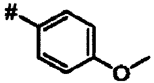 | H | |
| I-99 | 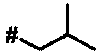 | H | 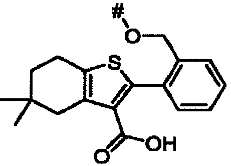 | 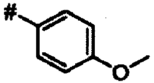 | OH | |
| 1-100 | 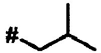 | H | 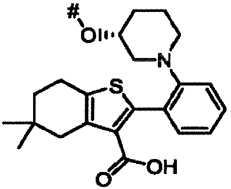 | 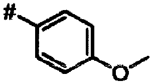 | H | |
| I-101 | 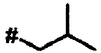 | H | 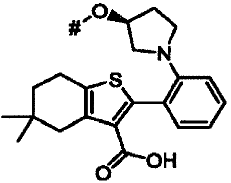 | 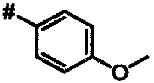 | H | |
| I-102 | 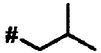 | H | 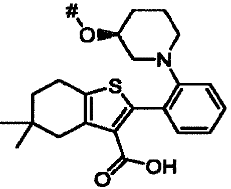 | 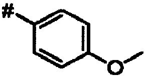 | H | |

**[Table 9]**

| No. | R1 | R2 | R3 | R4 | R5 | salt |
|---|---|---|---|---|---|---|
| I-103 | 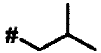 | H | 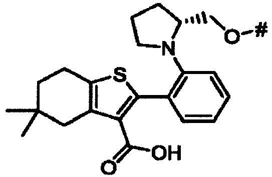 | 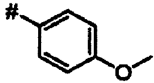 | H | |
| I-104 | 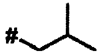 | H | 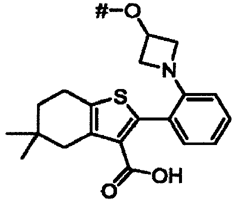 | 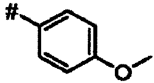 | H | |
| I-105 | 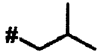 | H | 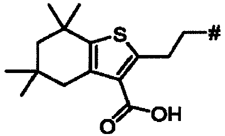 | 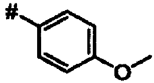 | H | |
| I-106 | 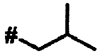 | H | 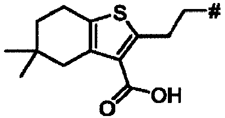 | 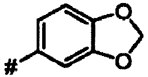 | H | |
| I-107 | 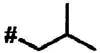 | H | 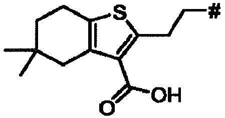 | 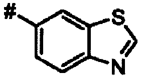 | H | |
| I-108 | 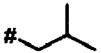 | H | 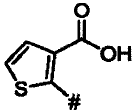 | 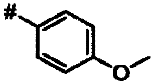 | H | |
| I-109 | 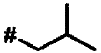 | H | 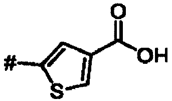 | 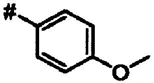 | H | |
| I-110 | 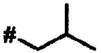 | H | 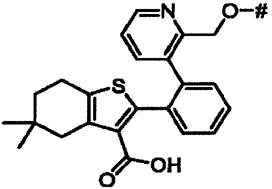 | 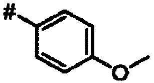 | H | |
| I-111 | 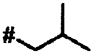 | H | 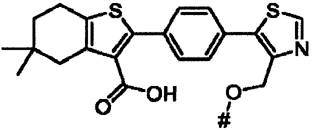 | 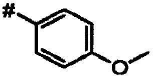 | H | |

**[Table 10]**

| No. | R1 | R2 | R3 | R4 | R5 | salt |
|---|---|---|---|---|---|---|
| I-112 | 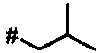 | H | 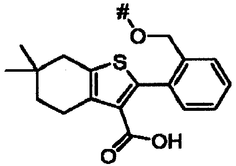 | 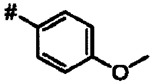 | H | |
| I-113 | 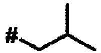 | H | 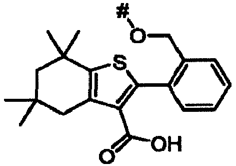 | 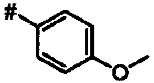 | H | |
| I-114 | 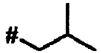 | H | 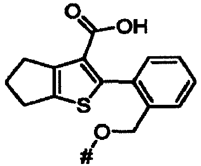 | 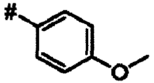 | H | |
| I-115 | 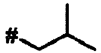 | H | 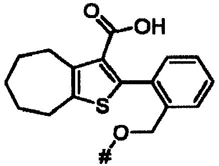 | 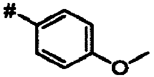 | H | |
| I-116 | 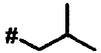 | H | 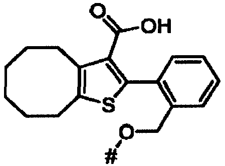 | 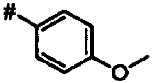 | H | |
| I-117 | 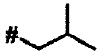 | H | 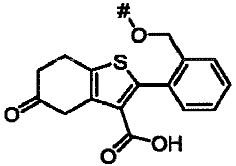 | 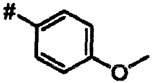 | H | |
| I-118 | 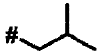 | H | 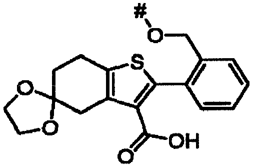 | 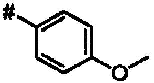 | H | |
| I-119 | 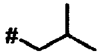 | H | 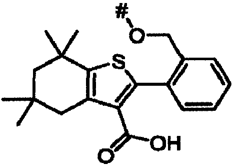 | 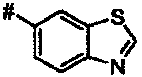 | H | |

**[Table 11]**

| No. | R1 | R2 | R3 | R4 | R5 | salt |
|---|---|---|---|---|---|---|
| I-120 | 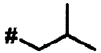 | H | 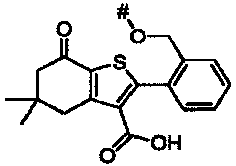 | 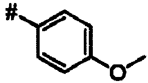 | H | |
| I-121 | 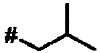 | H | 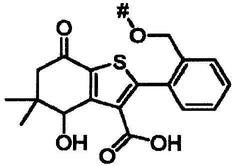 | 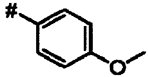 | H | |
| I-122 | 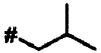 | H | 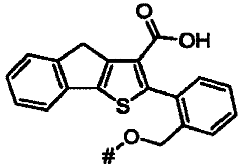 | 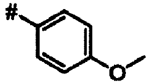 | H | |
| I-123 | 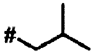 | H | 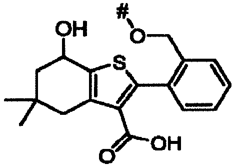 | 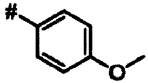 | H | |
| I-124 | 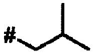 | H | 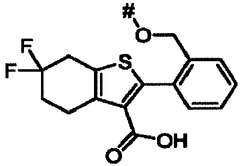 | 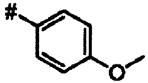 | H | |
| I-125 | 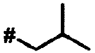 | H | 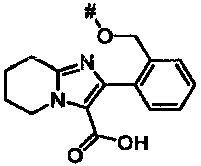 | 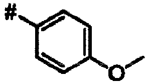 | H | |
| I-126 | 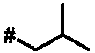 | H | 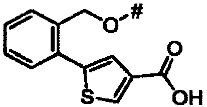 | 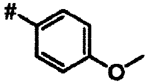 | H | |
| I-127 | 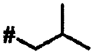 | H | 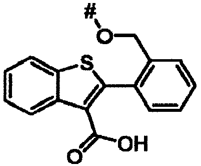 | 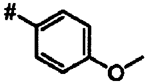 | H | |

**[Table 12]**

| No. | R1 | R2 | R3 | R4 | R5 | salt |
|---|---|---|---|---|---|---|
| I-128 | 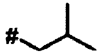 | H | 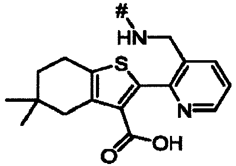 | 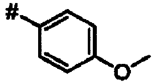 | H | |
| I-129 | 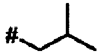 | H | 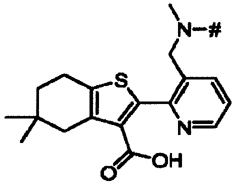 | 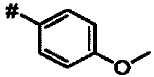 | H | |
| I-130 | 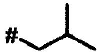 | H | 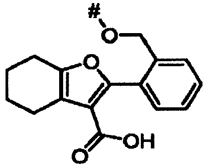 | 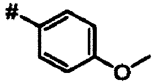 | H | |
| I-131 | 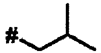 | H | 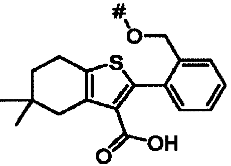 | 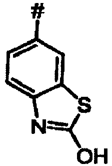 | H | |
| I-132 | 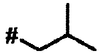 | H | 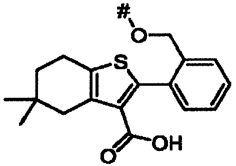 | 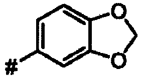 | H | |
| I-133 | 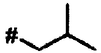 | H | 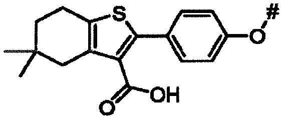 | 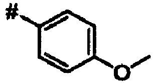 | H | |
| I-134 | 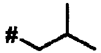 | H | 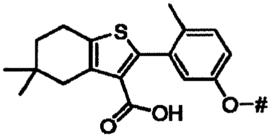 | 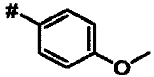 | H | |
| I-135 | 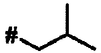 | H | 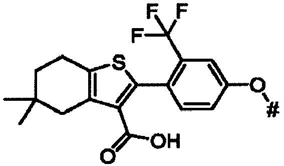 | 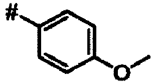 | H | |

**[Table 13]**

| No. | R1 | R2 | R3 | R4 | R5 | salt |
|---|---|---|---|---|---|---|
| I-136 | 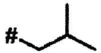 | H | 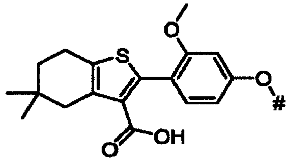 | 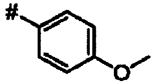 | H | |
| I-137 | 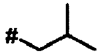 | H | 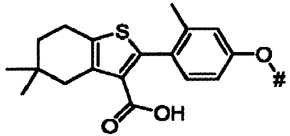 | 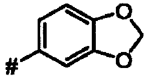 | H | |
| I-138 | 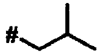 | H | 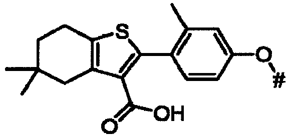 | 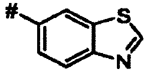 | H | |
| I-139 | 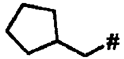 | H | 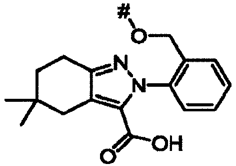 | 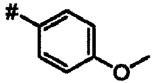 | H | |
| I-140 | 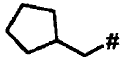 | H | 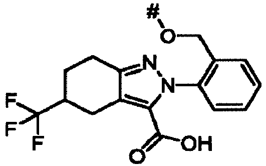 | 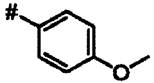 | H | |
| I-141 | 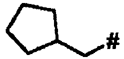 | H | 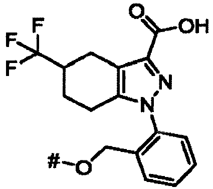 | 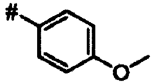 | H | |
| I-142 | 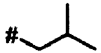 | H | 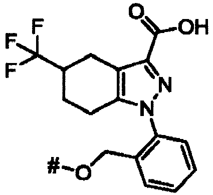 | 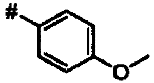 | H | |
| I-143 | 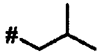 | H | 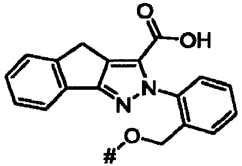 | 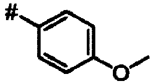 | H | |

**[Table 14]**

| No. | R1 | R2 | R3 | R4 | R5 | salt |
|---|---|---|---|---|---|---|
| I-144 | 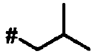 | H | 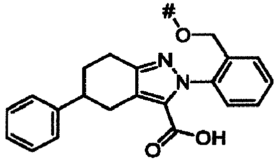 | 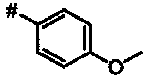 | H | |
| I-145 | 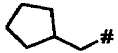 | H | 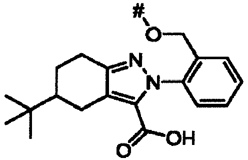 | 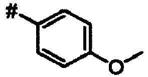 | H | |
| I-146 | 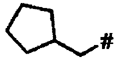 | H | 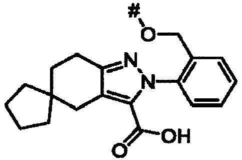 | 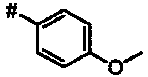 | H | |
| I-147 | 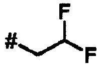 | H | 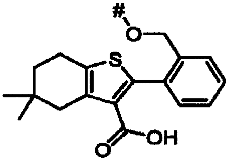 | 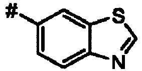 | H | |
| I-148 | 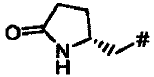 | H | 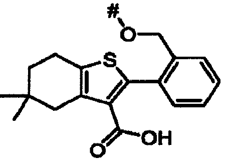 | 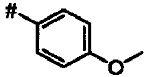 | H | |
| I-149 | 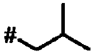 | H | 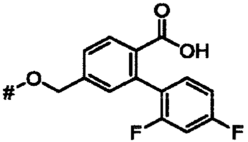 | 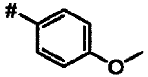 | H | |
| I-150 | 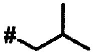 | H | 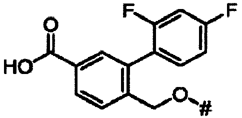 | 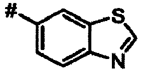 | H | |
| I-151 | 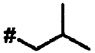 | H | 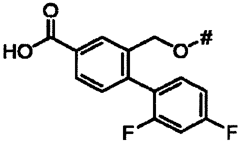 | 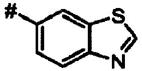 | H | |
| I-152 | 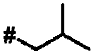 | H | 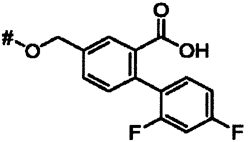 | 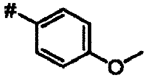 | H | |

**[Table 15]**

| No. | R1 | R2 | R3 | R4 | R5 | salt |
|---|---|---|---|---|---|---|
| I-153 | 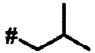 | H | 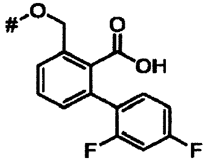 | 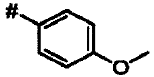 | H | |
| I-154 | 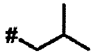 | H | 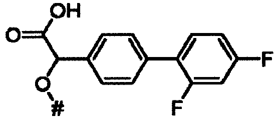 | 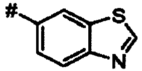 | H | |
| I-155 | 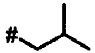 | H | 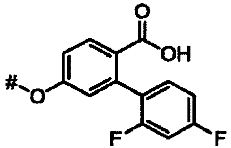 | 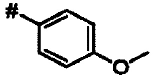 | H | |
| I-156 | 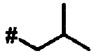 | H | 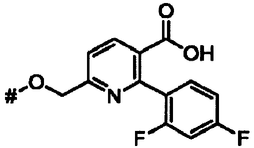 | 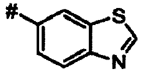 | H | |
| I-157 | 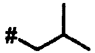 | H | 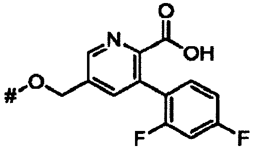 | 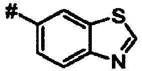 | H | |
| I-158 | 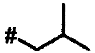 | H | 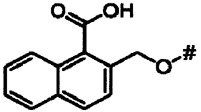 | 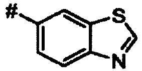 | H | |
| I-159 | 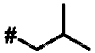 | H | 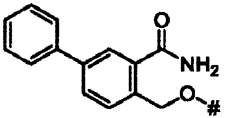 | 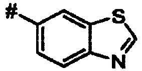 | H | |
| I-160 | 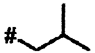 | H | 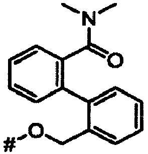 | 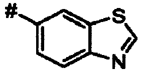 | H | |
| I-161 | 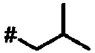 | H | 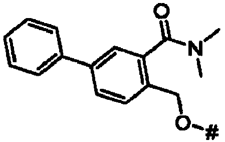 | 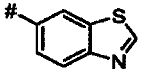 | H | |
| I-162 | 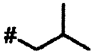 | H | 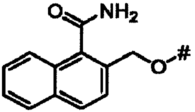 | 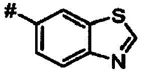 | H | |

**[Table 16]**

| No. | R1 | R2 | R3 | R4 | R5 | salt |
|---|---|---|---|---|---|---|
| I-163 | 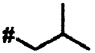 | H | 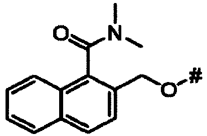 | 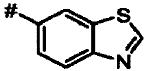 | H | |
| I-164 | 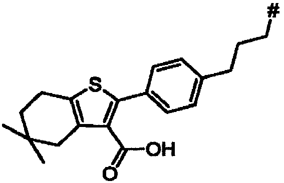 | H | H | 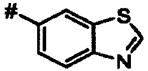 | H | |
| I-165 | 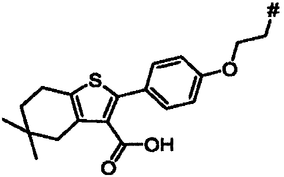 | H | H | 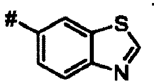 | H | |
| I-166 | 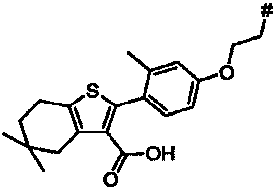 | H | H | 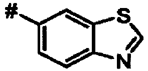 | H | |
| I-167 | 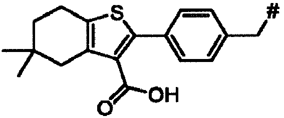 | H | H | 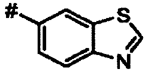 | H | |
| I-168 | 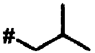 | H | 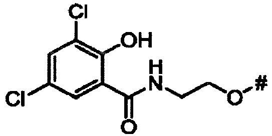 | 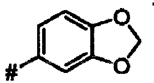 | H | |
| I-169 | 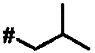 | H | 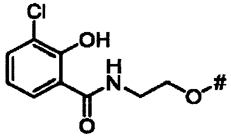 | 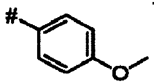 | H | |
| I-170 | 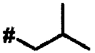 | H | 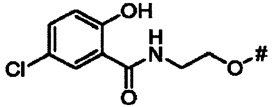 | 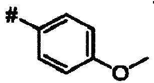 | H | |
| I-171 | 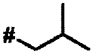 | H | 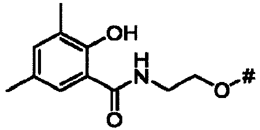 | 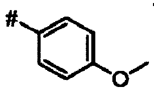 | H | |

**[Table 17]**

| No. | R1 | R2 | R3 | R4 | R5 | salt |
|---|---|---|---|---|---|---|
| I-172 | 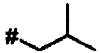 | H | 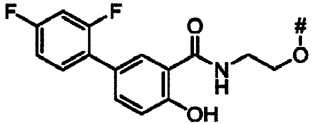 | 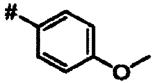 | H | |
| I-173 | 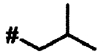 | H | 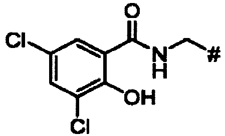 | 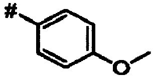 | H | |
| I-174 | 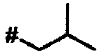 | H | 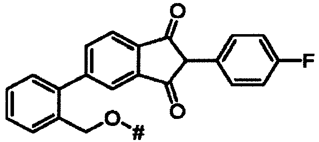 | 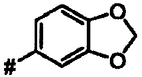 | H | |
| I-175 | 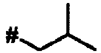 | H | 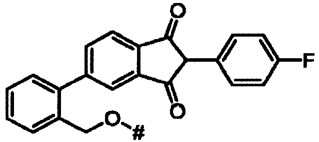 | 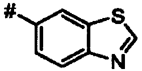 | H | |
| I-176 | 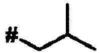 | H | 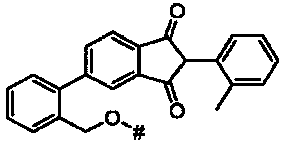 | 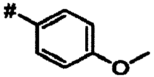 | H | |
| I-177 | 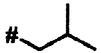 | H | 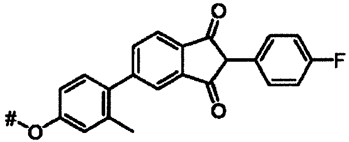 | 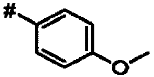 | H | |
| I-178 | 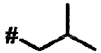 | H | 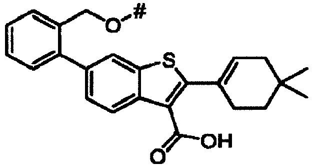 | 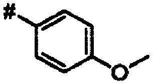 | H | |
| I-179 | 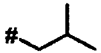 | H | 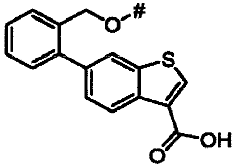 | 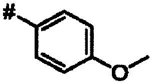 | H | |
| I-180 | 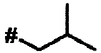 | H | 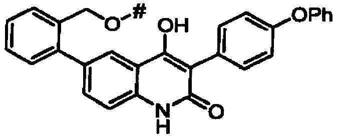 | 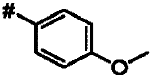 | H | |

**[Table 18]**

| No. | R1 | R2 | R3 | R4 | R5 | salt |
|---|---|---|---|---|---|---|
| I-181 | 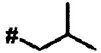 | H | 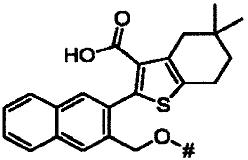 | 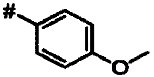 | H | |
| I-182 | 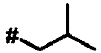 | H | 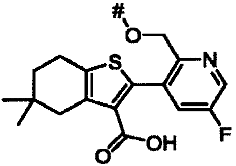 | 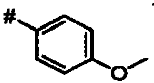 | H | |
| I-183 | 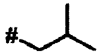 | H | 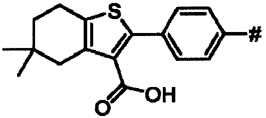 | 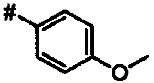 | H | |
| I-184 | 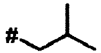 | H | 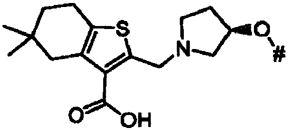 | 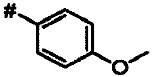 | H | |
| I-185 | 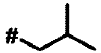 | H | 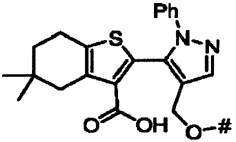 | 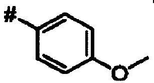 | H | |
| I-186 | 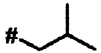 | H | 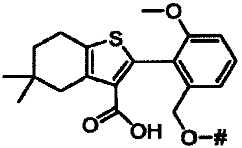 | 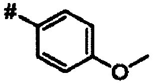 | H | |
| I-187 | 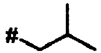 | H | 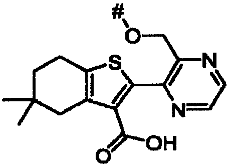 | 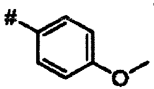 | H | |
| I-188 | 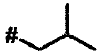 | H | 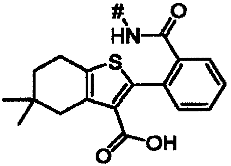 | 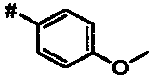 | H | |
| I-189 | 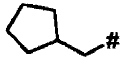 | H | 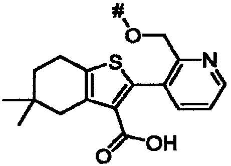 | 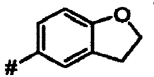 | H | |

**[Table 19]**

| No. | R1 | R2 | R3 | R4 | R5 | salt |
|---|---|---|---|---|---|---|
| I-190 | 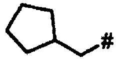 | H | 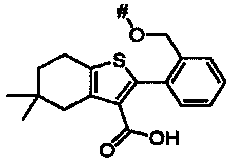 | 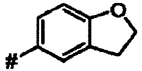 | H | |
| I-191 | 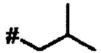 | H | 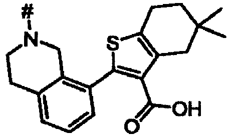 | 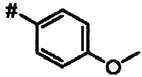 | H | |
| I-193 | 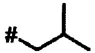 | H | 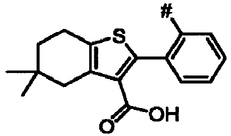 | 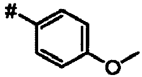 | H | |
| I-194 | 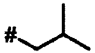 | H | 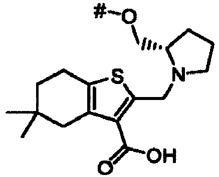 | 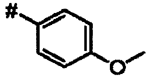 | H | |
| I-195 | 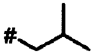 | H | 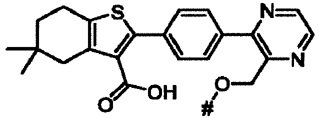 | 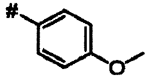 | H | |
| I-196 | 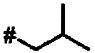 | H | 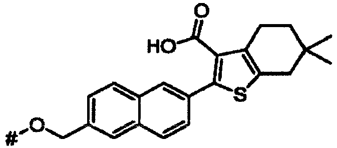 | 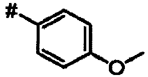 | H | |
| I-197 | 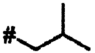 | H | 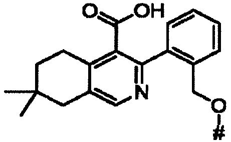 | 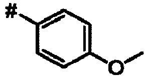 | H | |
| I-198 | 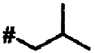 | H | 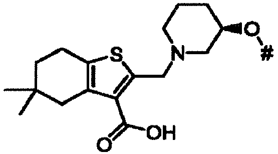 | 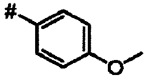 | H | |
| I-199 | 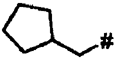 | H | 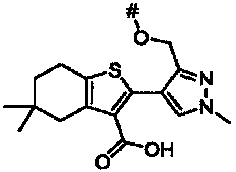 | 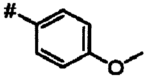 | H | |

**[Table 20]**

| No. | R1 | R2 | R3 | R4 | R5 | salt |
|---|---|---|---|---|---|---|
| I-200 | 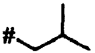 | H | 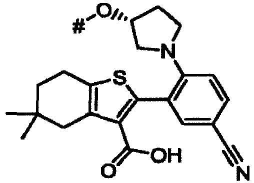 | 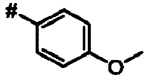 | H | |
| I-201 | 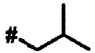 | H | 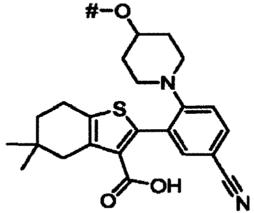 | 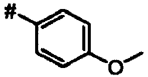 | H | |
| I-202 | 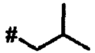 | H | 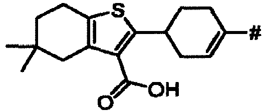 | 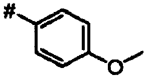 | H | |
| I-203 | 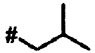 | H | 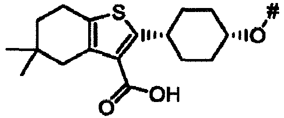 | 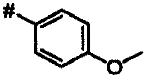 | H | |
| I-204 | 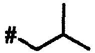 | H | 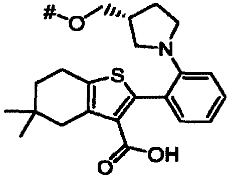 | 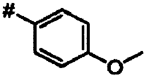 | H | |
| I-205 | 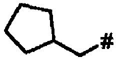 | H | 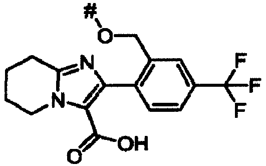 | 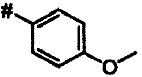 | H | |
| I-206 | 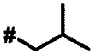 | H | 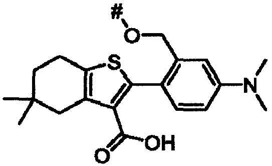 | 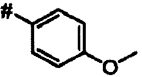 | H | |
| I-207 | 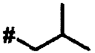 | H | 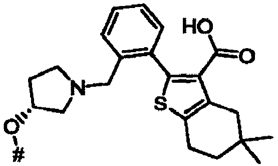 | 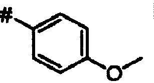 | H | |
| I-208 | 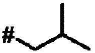 | H | 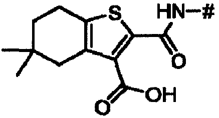 | 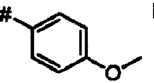 | H | |

**[Table 21]**

| No. | R1 | R2 | R3 | R4 | R5 | salt |
|---|---|---|---|---|---|---|
| I-209 | 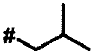 | H | 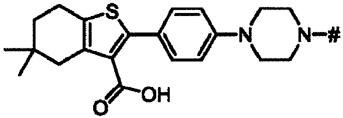 | 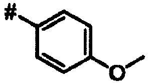 | H | |
| I-210 | 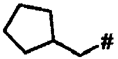 | H | 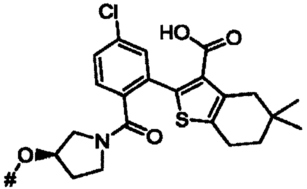 | 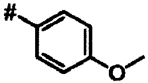 | H | |
| I-211 | 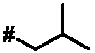 | H | 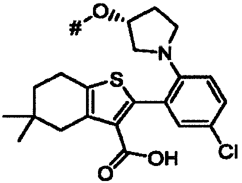 | 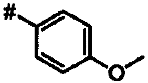 | H | |
| I-212 | 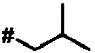 | H | 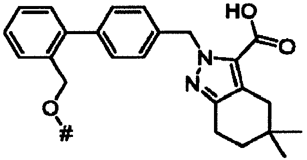 | 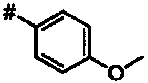 | H | |
| I-213 | 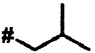 | H | 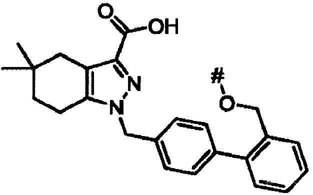 | 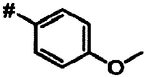 | H | |
| I-214 | 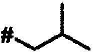 | H | 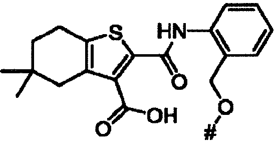 | 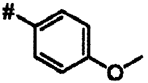 | H | |
| I-215 | 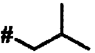 | H | 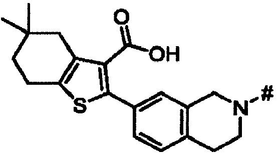 | 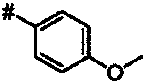 | H | |
| I-216 | 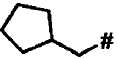 | H | 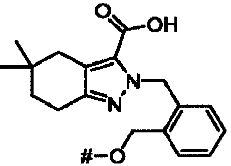 | 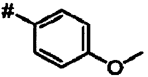 | H | |

**[Table 22]**

| No. | R1 | | R2 | R3 | R4 | R5 | salt |
|---|---|---|---|---|---|---|---|
| I-217 | 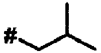 | | H | 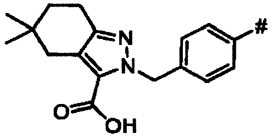 | 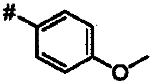 | H | |
| I-218 | 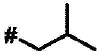 | | H | 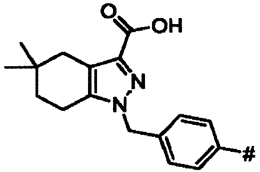 | 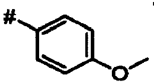 | H | |
| I-219 | 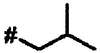 | | H | 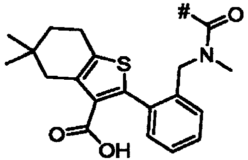 | 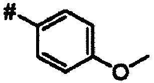 | H | |
| I-220 | 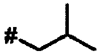 | | H | 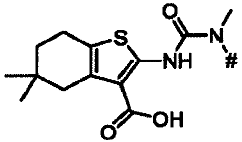 | 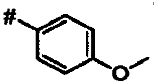 | H | |
| I-221 | 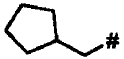 | | H | 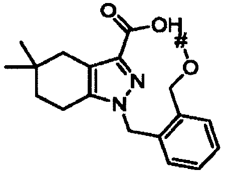 | 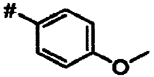 | H | |
| I-222 | 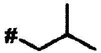 | | H | 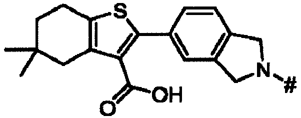 | 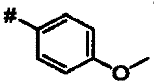 | H | |
| I-223 | 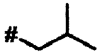 | | H | 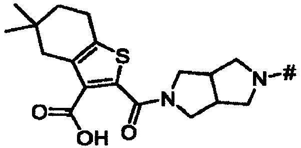 | 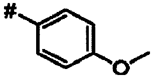 | H | |
| I-224 | 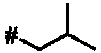 | | H | 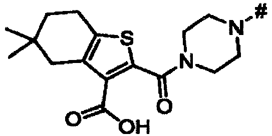 | 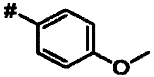 | H | |
| I-225 | 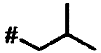 | | H | 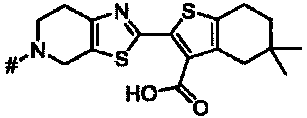 | 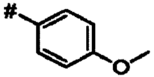 | H | |

**[Table 23]**

| No. | R1 | R2 | R3 | R4 | R5 | salt |
|---|---|---|---|---|---|---|
| I-226 | 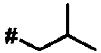 | H | 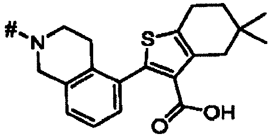 | 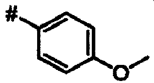 | H | |
| I-227 | 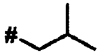 | H | 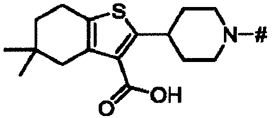 | 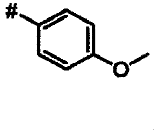 | H | |
| I-229 | 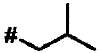 | H | 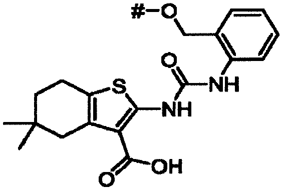 | 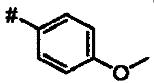 | H | |
| I-230 | 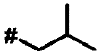 | H | 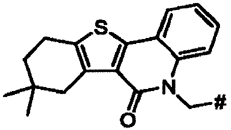 | 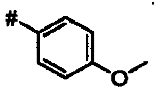 | H | |
| I-231 | 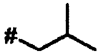 | H | 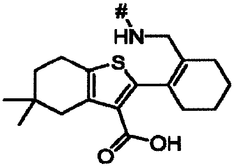 | 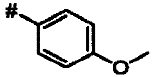 | H | |
| I-232 | 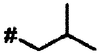 | H | 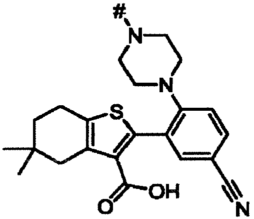 | 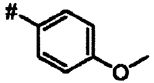 | H | |
| I-233 | 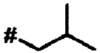 | H | 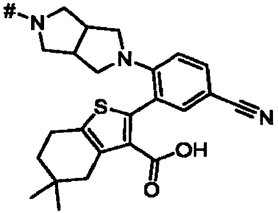 | 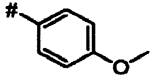 | H | |
| I-234 | 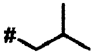 | H | 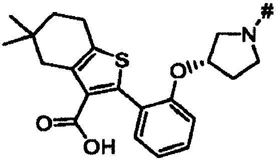 | 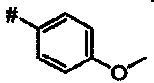 | H | |

**[Table 24]**

| No. | R1 | R2 | R3 | R4 | R5 | salt |
|---|---|---|---|---|---|---|
| I-235 | 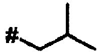 | H | 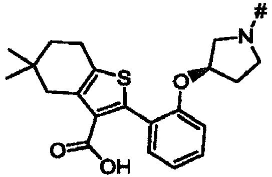 | 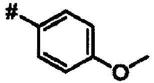 | H | |
| I-236 | 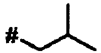 | H | 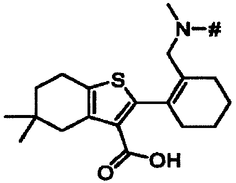 | 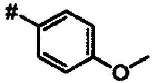 | H | |
| I-237 | 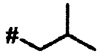 | H | 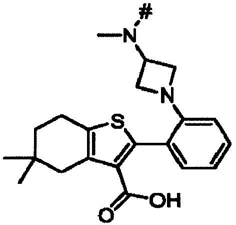 | 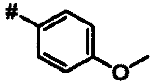 | H | |
| I-238 | 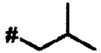 | H | 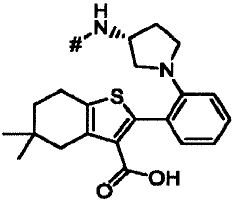 | 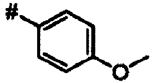 | H | |
| I-239 | 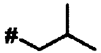 | H | 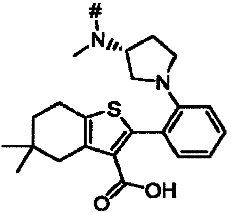 | 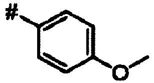 | H | |
| I-240 | 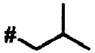 | H | 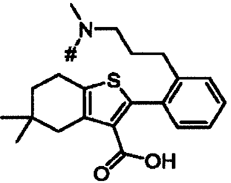 | 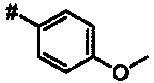 | H | |
| I-241 | 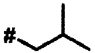 | H | 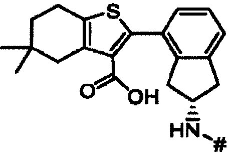 | 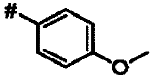 | H | |

**[Table 25]**

| No. | R1 | R2 | R3 | R4 | R5 | salt |
|---|---|---|---|---|---|---|
| I-242 | 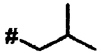 | H | 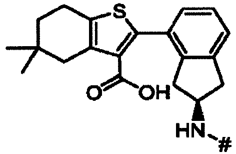 | 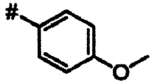 | H | |
| I-243 | 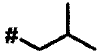 | H | 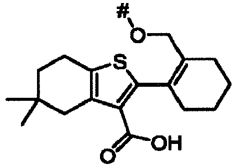 | 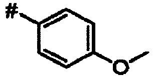 | H | |
| I-244 | 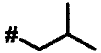 | H | 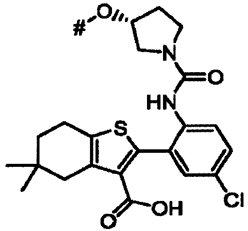 | 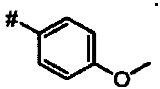 | H | |
| I-245 | 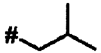 | H | 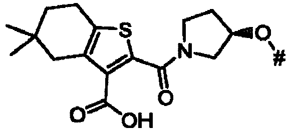 | 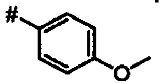 | H | |
| I-248 | 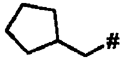 | H | 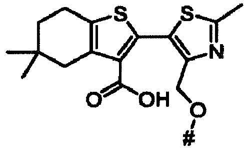 | 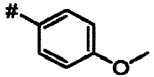 | H | |
| I-249 | 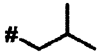 | H | 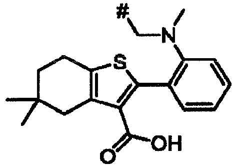 | 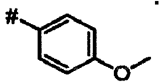 | H | |
| I-250 | 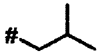 | H | 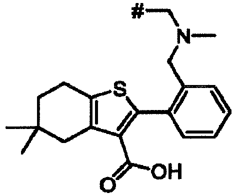 | 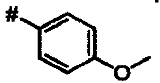 | H | |
| I-251 | 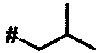 | H | 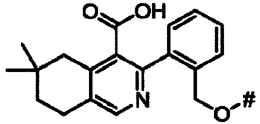 | 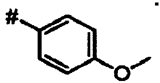 | H | |

**[Table 26]**

| No. | R1 | R2 | R3 | R4 | R5 | salt |
|---|---|---|---|---|---|---|
| I-252 | 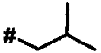 | H | 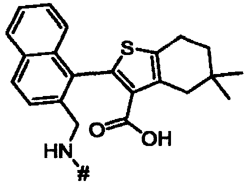 | 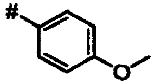 | H | |
| I-253 | 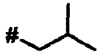 | H | 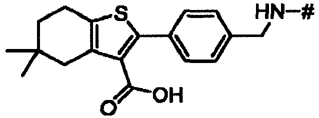 | 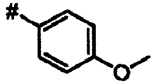 | H | |
| I-254 | 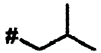 | H | 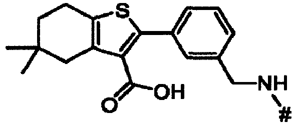 | 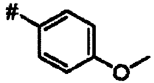 | H | |
| I-255 | 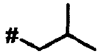 | H | 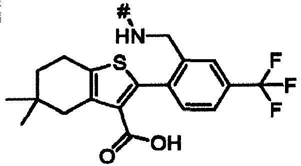 | 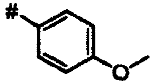 | H | |
| I-256 | 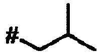 | H | 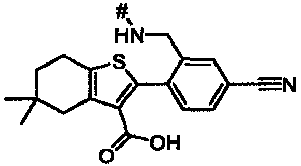 | 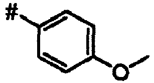 | H | |
| I-257 | 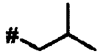 | H | 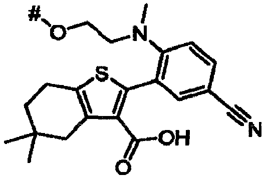 | 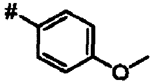 | H | |
| I-258 | 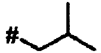 | H | 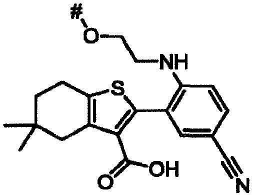 | 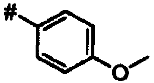 | H | |
| I-259 | 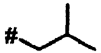 | H | 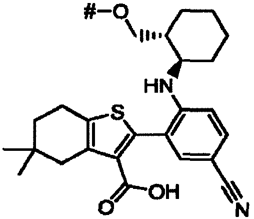 | 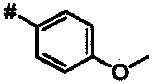 | H | |

**[Table 27]**

| No. | R1 | R2 | R3 | R4 | R5 | salt |
|---|---|---|---|---|---|---|
| I-260 | 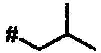 | H | 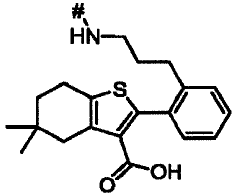 | 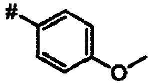 | H | |
| I-261 | 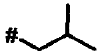 | H | 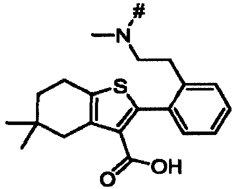 | 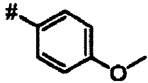 | H | |
| I-262 | 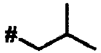 | H | 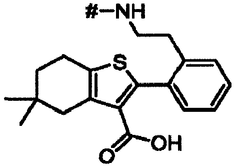 | 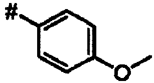 | H | |
| I-263 | 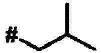 | H | 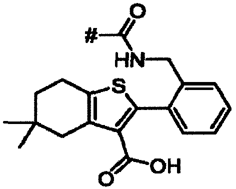 | 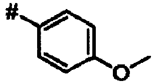 | H | |
| I-264 | 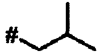 | H | 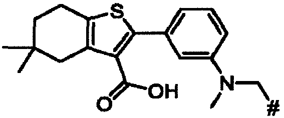 | 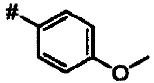 | H | |
| I-265 | 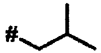 | H | 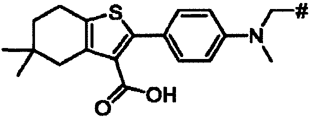 | 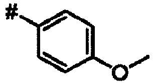 | H | |
| I-266 | 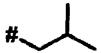 | H | 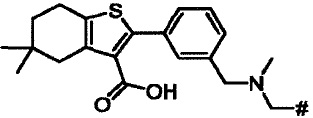 | 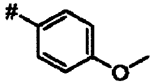 | H | |
| I-267 | 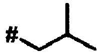 | H | 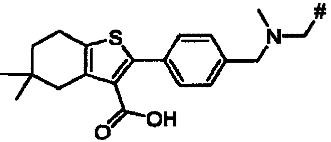 | 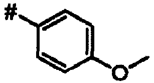 | H | |

**[Table 28]**

| No. | R1 | R2 | R3 | R4 | R5 | salt |
|---|---|---|---|---|---|---|
| I-269 | 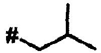 | H | 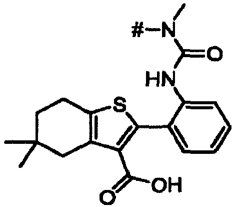 | 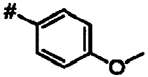 | H | |
| I-270 | 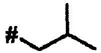 | H | 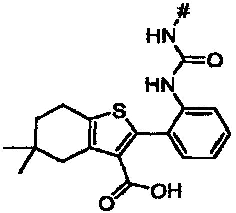 | 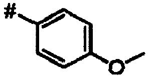 | H | |
| I-271 | 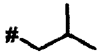 | H | 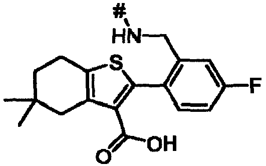 | 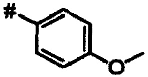 | H | |
| I-272 | 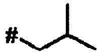 | H | 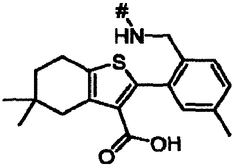 | 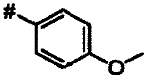 | H | |
| I-273 | 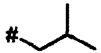 | H | 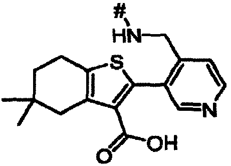 | 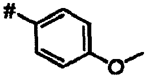 | H | |
| I-274 | 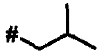 | H | 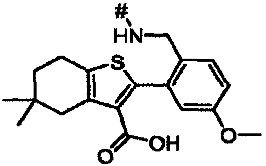 | 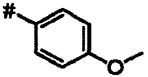 | H | |
| I-275 | 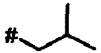 | H | 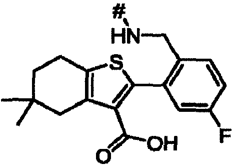 | 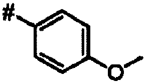 | H | |
| I-276 | 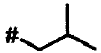 | H | 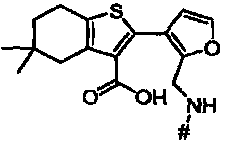 | 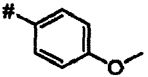 | H | |

**[Table 29]**

| No. | R1 | R2 | R3 | R4 | R5 | salt |
|---|---|---|---|---|---|---|
| I-277 | 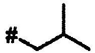 | H | 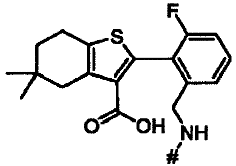 | 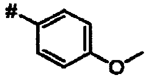 | H | |
| I-278 | 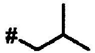 | H | 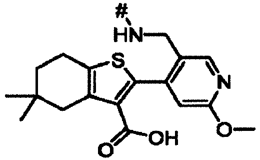 | 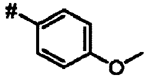 | H | |
| I-279 | 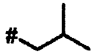 | H | 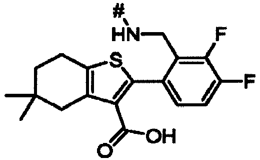 | 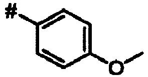 | H | |
| I-280 | 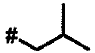 | H | 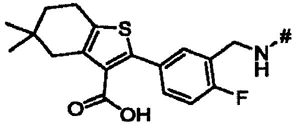 | 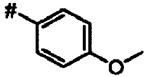 | H | |
| I-281 | 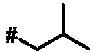 | H | 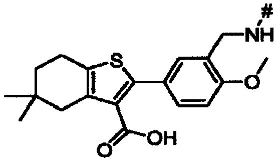 | 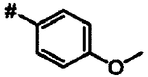 | H | |
| I-282 | 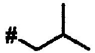 | H | 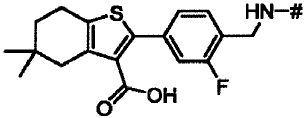 | 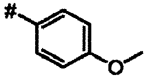 | H | |
| I-283 | 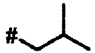 | H | 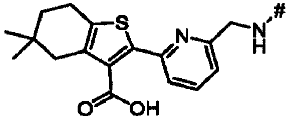 | 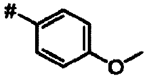 | H | |
| I-284 | 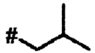 | H | 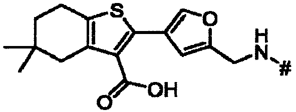 | 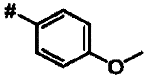 | H | |
| I-285 | 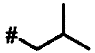 | H | 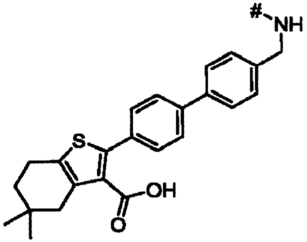 | 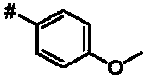 | H | |

**[Table 30]**

| No. | R1 | R2 | R3 | R4 | R5 | salt |
|---|---|---|---|---|---|---|
| I-286 | 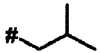 | H | 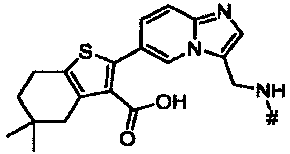 | 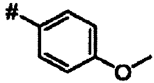 | H | |
| I-287 | 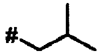 | H | 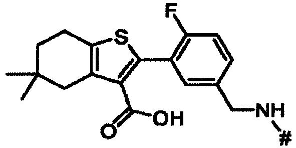 | 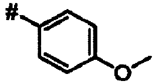 | H | |
| I-288 | 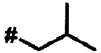 | H | 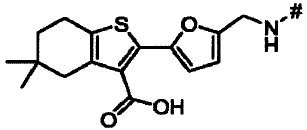 | 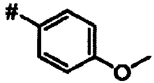 | H | |
| I-289 | 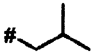 | H | 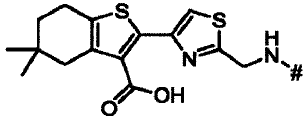 | 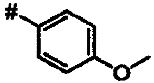 | H | |
| I-290 | 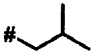 | H | 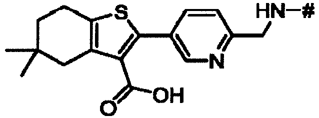 | 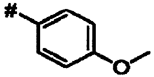 | H | |
| I-291 | 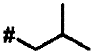 | H | 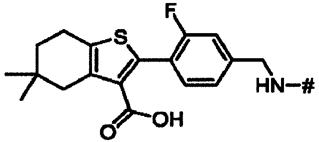 | 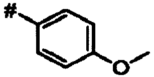 | H | |
| I-292 | 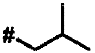 | H | 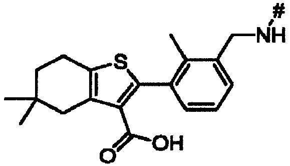 | 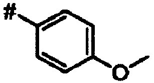 | H | |
| I-293 | 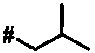 | H | 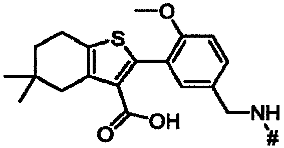 | 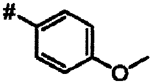 | H | |
| I-294 | 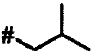 | H | 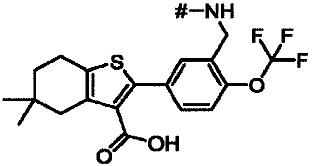 | 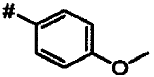 | H | |

**[Table 31]**

| No. | R1 | R2 | R3 | R4 | R5 | salt |
|---|---|---|---|---|---|---|
| I-295 | 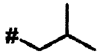 | H | 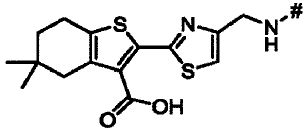 | 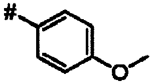 | H | |
| I-296 | 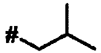 | H | 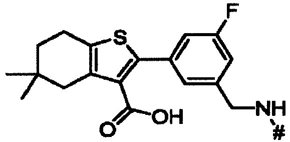 | 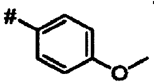 | H | |
| I-297 | 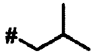 | H | 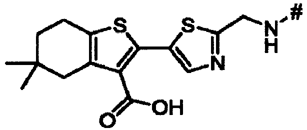 | 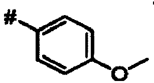 | H | |
| I-298 | 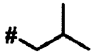 | H | 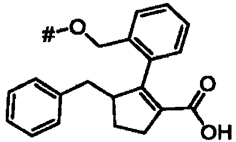 | 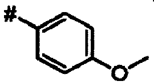 | H | |
| I-299 | 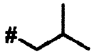 | H | 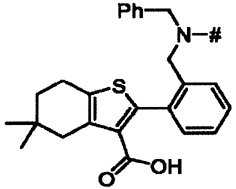 | 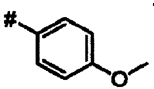 | H | |
| I-300 | 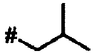 | H | 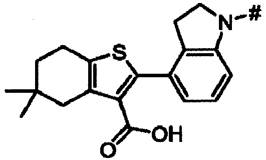 | 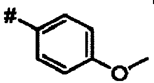 | H | |
| I-301 | 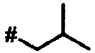 | H | 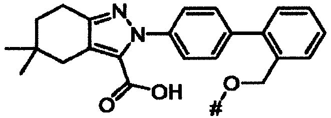 | 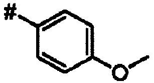 | H | |
| I-302 | 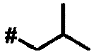 | H | 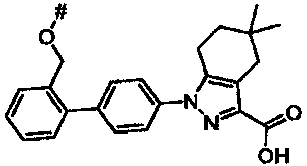 | 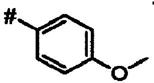 | H | |
| I-303 | 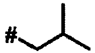 | H | 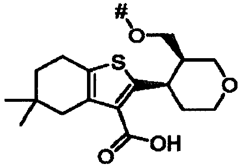 | 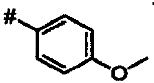 | H | |

**[Table 32]**

| No. | R1 | R2 | R3 | R4 | R5 | salt |
|---|---|---|---|---|---|---|
| I-304 | 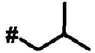 | H | 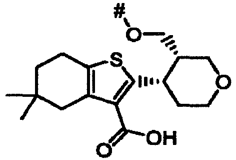 | 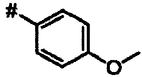 | H | |
| I-305 | 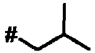 | H | 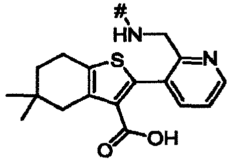 | 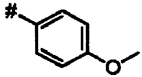 | H | |
| I-306 | 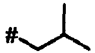 | H | 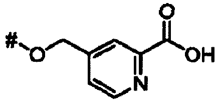 | 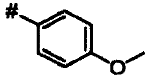 | H | |
| I-307 | 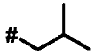 | H | 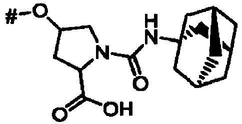 | 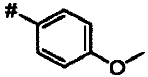 | H | |
| I-308 | 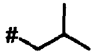 | H | 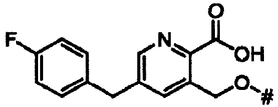 | 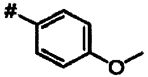 | H | |
| I-308 | 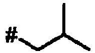 | H | 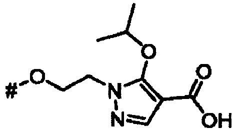 | 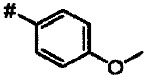 | H | |
| I-310 | 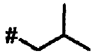 | H | 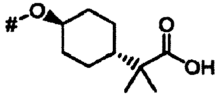 | 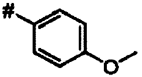 | H | |
| I-311 | 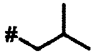 | H | 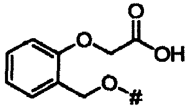 | 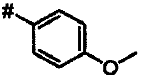 | H | |
| I-312 | 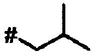 | H | 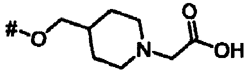 | 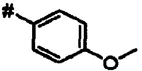 | H | |
| I-313 | 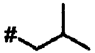 | H | 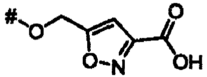 | 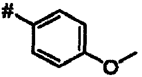 | H | |
| I-314 | 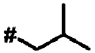 | H | 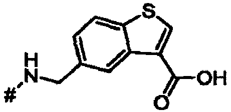 | 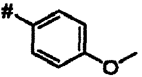 | H | |
| I-315 | 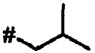 | H | 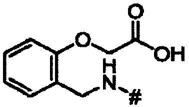 | 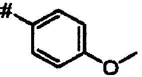 | H | |

**[Table 33]**

| No. | R1 | R2 | R3 | R4 | R5 | salt |
|---|---|---|---|---|---|---|
| I-316 | 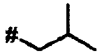 | H | 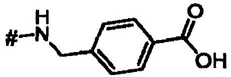 | 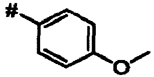 | H | |
| I-317 | 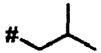 | H | 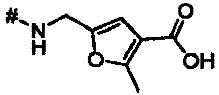 | 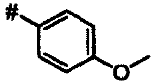 | H | |
| I-318 | 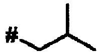 | H | 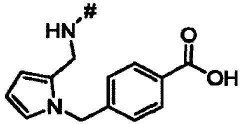 | 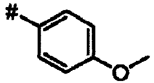 | H | |
| I-319 | 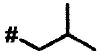 | H | 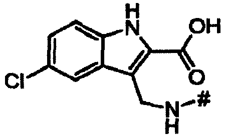 | 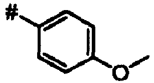 | H | |
| I-320 | 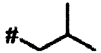 | H | 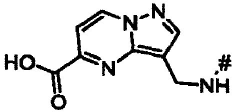 | 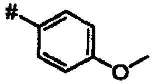 | H | |
| I-321 | 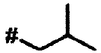 | H | 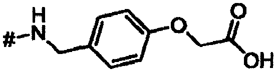 | 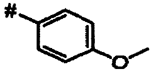 | H | |
| I-322 | 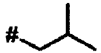 | H | 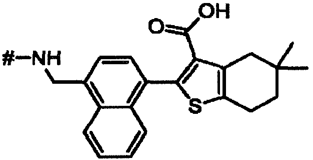 | 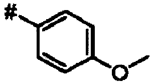 | H | |
| I-323 | 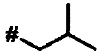 | H | 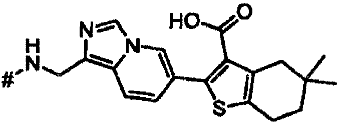 | 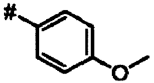 | H | |
| I-324 | 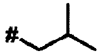 | H | 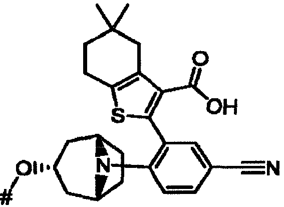 | 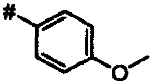 | H | |
| I-325 | 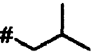 | H | 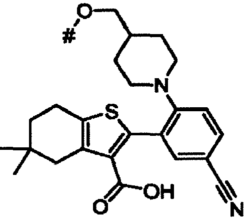 | 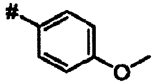 | H | |

**[Table 34]**

| No. | R1 | R2 | R3 | R4 | R5 | salt |
|---|---|---|---|---|---|---|
| I-326 | 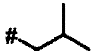 | H | 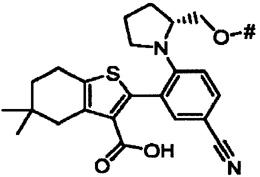 | 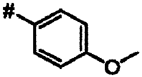 | H | |
| I-327 | 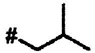 | H | 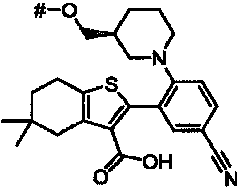 | 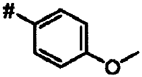 | H | |
| I-328 | 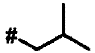 | H | 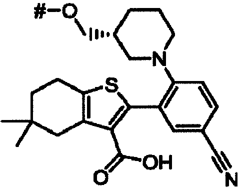 | 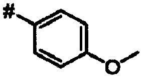 | H | |
| I-329 | 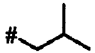 | H | 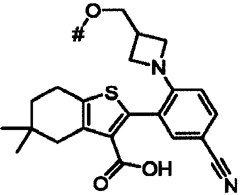 | 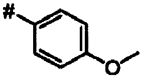 | H | |
| I-330 | 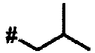 | H | 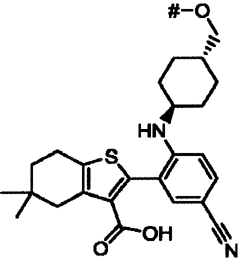 | 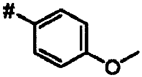 | H | |
| I-331 | 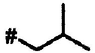 | H | 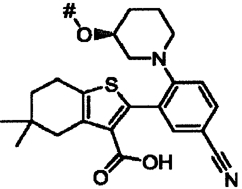 | 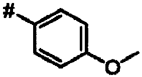 | H | |
| I-332 | 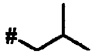 | H | 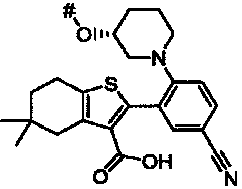 | 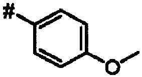 | H | |

**[Table 35]**

| No. | R1 | R2 | R3 | R4 | R5 | salt |
|---|---|---|---|---|---|---|
| I-333 | 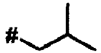 | H | 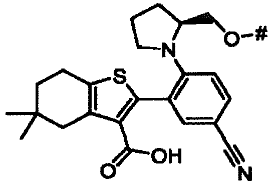 | 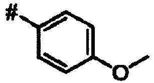 | H | |
| I-334 | 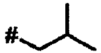 | H | 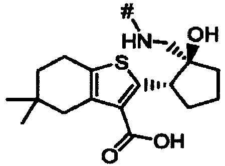 | 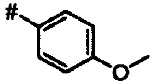 | H | |
| I-335 | 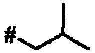 | H | 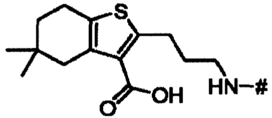 | 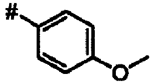 | H | |
| I-336 | 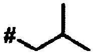 | H | 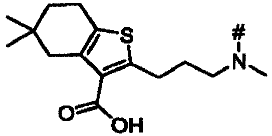 | 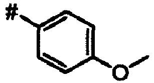 | H | |
| I-337 | 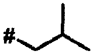 | H | 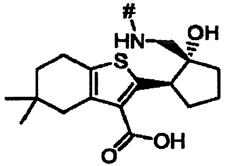 | 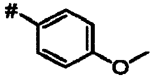 | H | |
| I-338 | 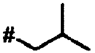 | H | 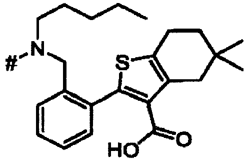 | 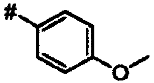 | H | |
| I-339 | 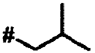 | H | 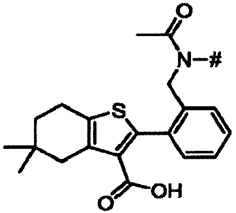 | 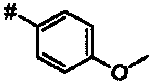 | H | |
| I-340 | 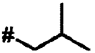 | H | 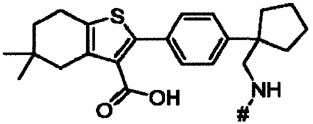 | 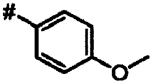 | H | |

**[Table 36]**

| No. | R1 | R2 | R3 | R4 | R5 | salt |
|---|---|---|---|---|---|---|
| I-341 | 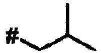 | H | 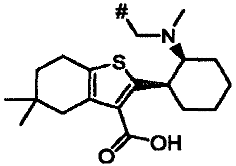 | 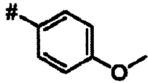 | H | |
| I-342 | 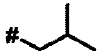 | H | 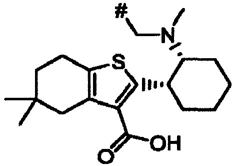 | 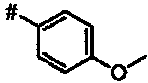 | H | |
| I-343 | 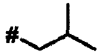 | H | 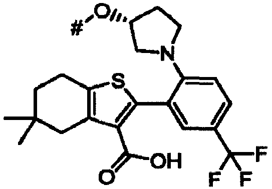 | 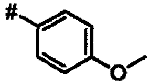 | H | |
| I-344 | 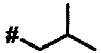 | H | 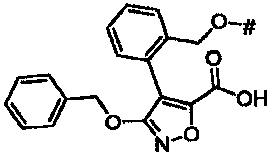 | 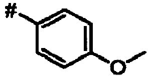 | H | |
| I-345 | 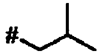 | H | 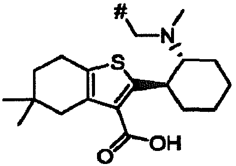 | 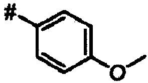 | H | |
| I-346 | 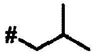 | H | 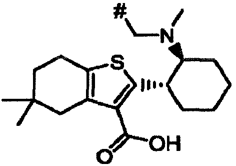 | 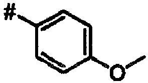 | H | |
| I-347 | 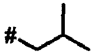 | H | 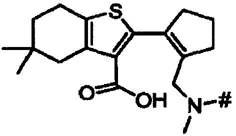 | 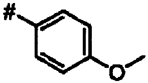 | H | |
| I-348 | 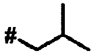 | H | 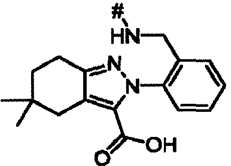 | 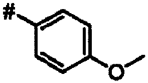 | H | |

**[Table 37]**

| No. | R1 | R2 | R3 | R4 | R5 | salt |
|---|---|---|---|---|---|---|
| I-349 | 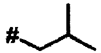 | H | 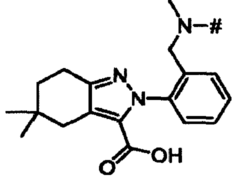 | 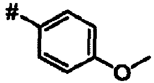 | H | |
| I-350 | 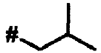 | H | 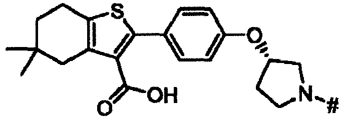 | 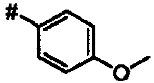 | H | |
| I-351 | 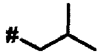 | H | 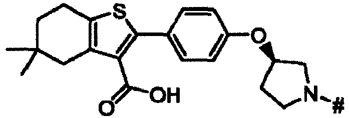 | 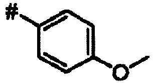 | H | |
| I-352 | 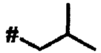 | H | 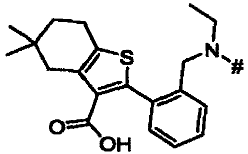 | 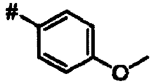 | H | |
| I-353 | 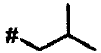 | H | 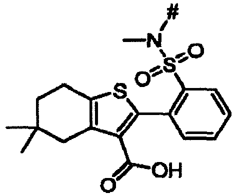 | 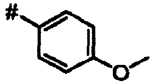 | H | |
| I-354 | 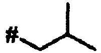 | H | 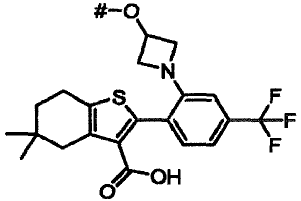 | 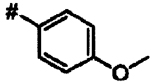 | H | |
| I-355 | 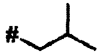 | H | 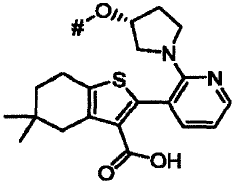 | 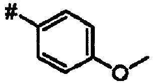 | H | |
| I-356 | 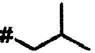 | H | 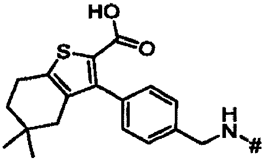 | 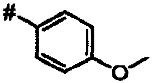 | H | |

**[Table 38]**

| No. | R1 | R2 | R3 | R4 | R5 | salt |
|---|---|---|---|---|---|---|
| I-357 | 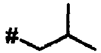 | H | 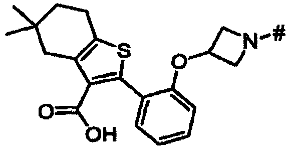 | 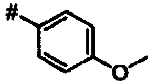 | H | |
| I-358 | 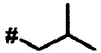 | H | 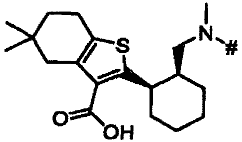 | 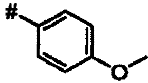 | H | |
| I-359 | 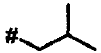 | H | 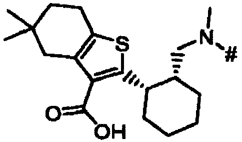 | 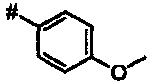 | H | |
| I-360 | 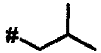 | H | 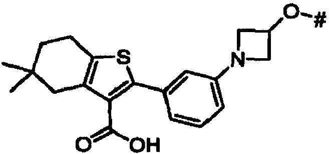 | 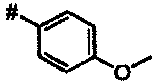 | H | |
| I-361 | 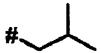 | H | 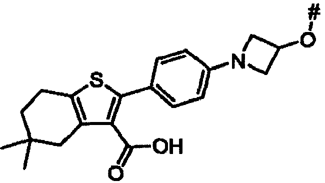 | 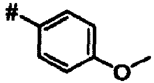 | H | |
| I-362 | 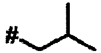 | H | 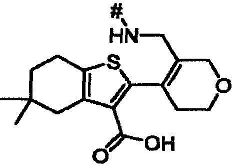 | 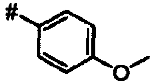 | H | |
| I-363 | 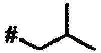 | H | 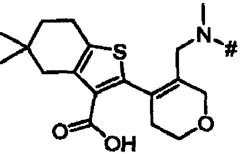 | 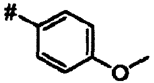 | H | |
| I-364 | 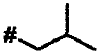 | H | 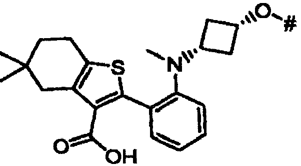 | 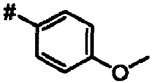 | H | |
| I-365 | 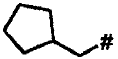 | H | 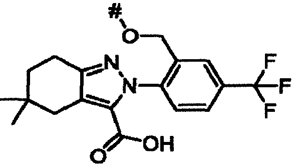 | 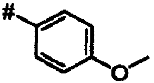 | H | |

**[Table 39]**

| No. | R1 | R2 | R3 | R4 | R5 | salt |
|---|---|---|---|---|---|---|
| I-366 | 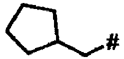 | H | 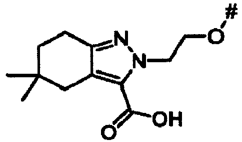 | 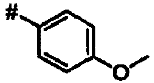 | H | |
| I-367 | 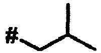 | H | 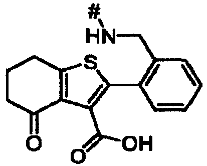 | 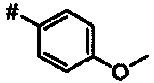 | H | |
| I-368 | 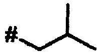 | H | 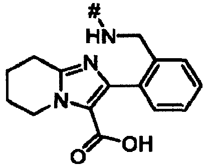 | 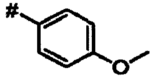 | H | |
| I-369 | 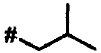 | H | 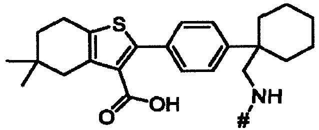 | 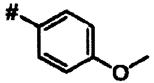 | H | |
| I-370 | 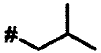 | H | 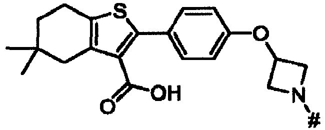 | 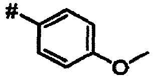 | H | |
| I-371 | 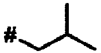 | H | 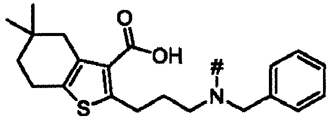 | 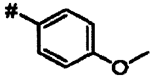 | H | |
| I-372 | 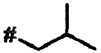 | H | 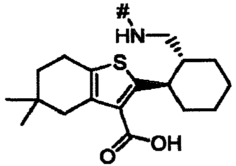 | 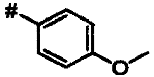 | H | |
| I-373 | 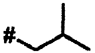 | H | 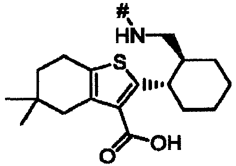 | 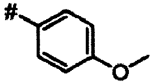 | H | |
| I-374 | 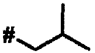 | H | 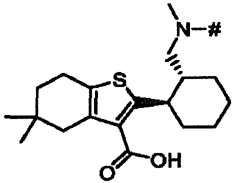 | 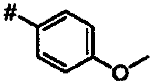 | H | |

**[Table 40]**

| No. | R1 | R2 | R3 | R4 | R5 | salt |
|---|---|---|---|---|---|---|
| I-375 | 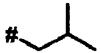 | H | 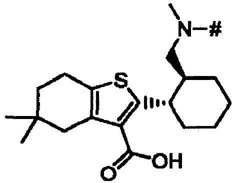 | 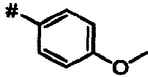 | H | |
| I-376 | 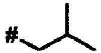 | H | 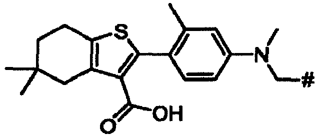 | 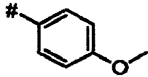 | H | |
| I-377 | 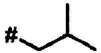 | H | 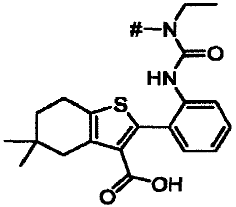 | 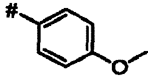 | H | |
| I-378 | 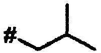 | H | 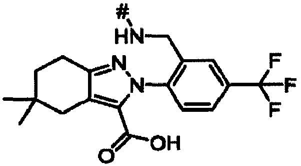 | 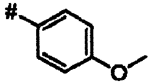 | H | |
| I-379 | 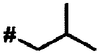 | H | 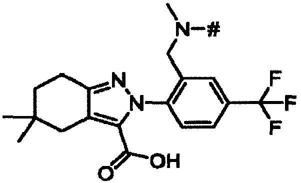 | 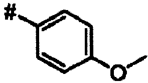 | H | |
| I-380 | 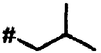 | H | 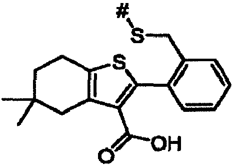 | 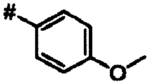 | H | |
| I-381 | 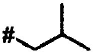 | H | 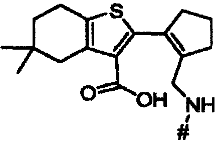 | 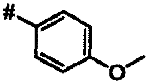 | H | |
| I-382 | 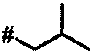 | H | 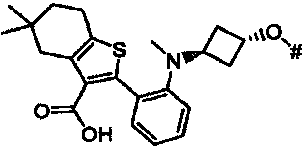 | 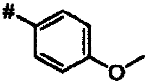 | H | |

**[Table 41]**

| No. | R1 | R2 | R3 | R4 | R5 | salt |
|---|---|---|---|---|---|---|
| I-383 | 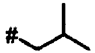 | H | 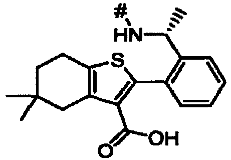 | 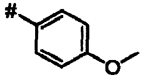 | H | |
| I-384 | 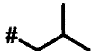 | H | 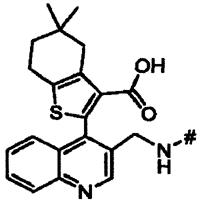 | 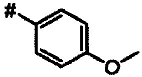 | H | |
| I-385 | 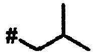 | H | 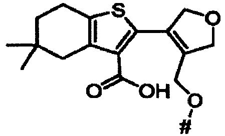 | 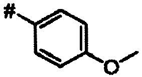 | H | |
| I-386 | 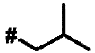 | H | 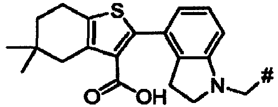 | 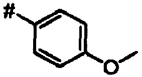 | H | |
| I-387 | 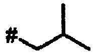 | H | 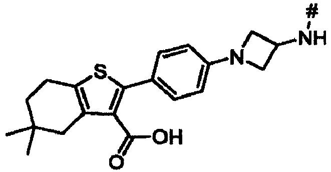 | 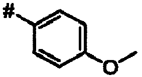 | H | |
| I-388 | 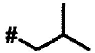 | H | 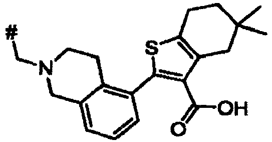 | 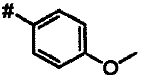 | H | |
| I-389 | 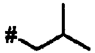 | H | 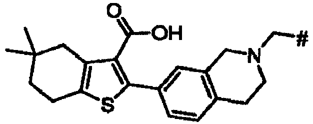 | 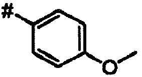 | H | |
| I-390 | 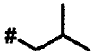 | H | 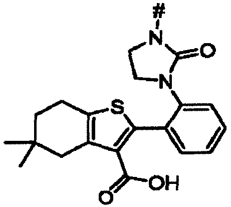 | 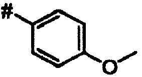 | H | |
| I-391 | 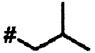 | H | 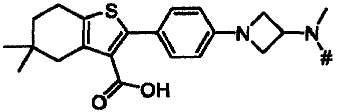 | 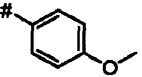 | H | |

**[Table 42]**

| No. | R1 | R2 | R3 | R4 | R5 | salt |
|---|---|---|---|---|---|---|
| I-392 | 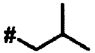 | H | 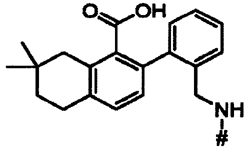 | 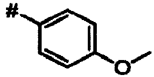 | H | |
| I-393 | 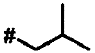 | H | 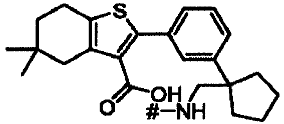 | 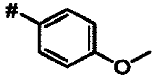 | H | |
| I-394 | 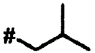 | H | 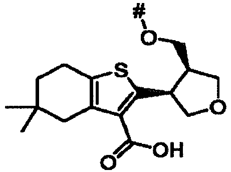 | 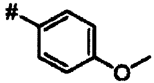 | H | |
| I-395 | 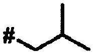 | H | 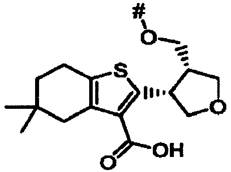 | 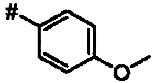 | H | |
| I-397 | 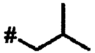 | | 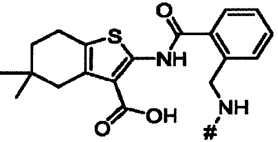 | 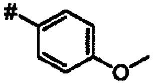 | | Na |
| I-398 | 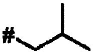 | H | 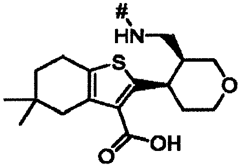 | 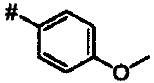 | H | |
| I-399 | 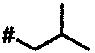 | H | 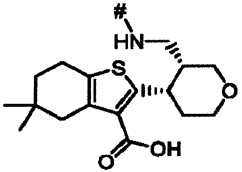 | 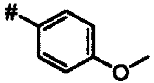 | H | |
| I-400 | 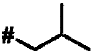 | H | 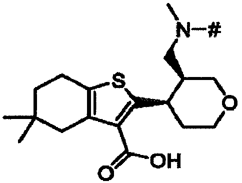 | 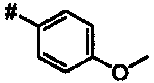 | H | |

**[Table 43]**

| No. | R1 | R2 | R3 | R4 | R5 | salt |
|---|---|---|---|---|---|---|
| I-401 | 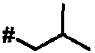 | H | 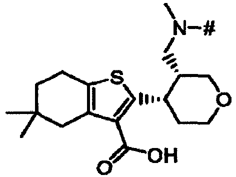 | 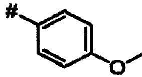 | H | |
| I-402 | 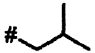 | H | 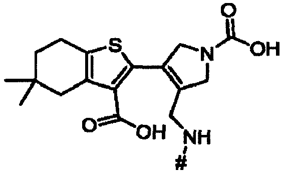 | 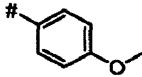 | H | |
| I-403 | 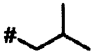 | H | 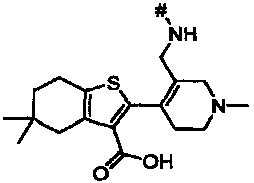 | 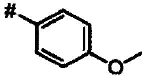 | H | |
| I-404 | 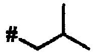 | H | 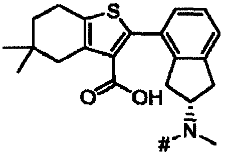 | 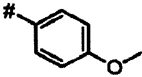 | H | |
| I-405 | 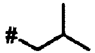 | H | 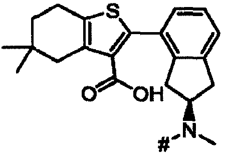 | 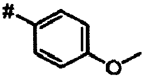 | H | |
| I-406 | 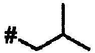 | H | 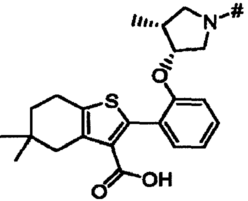 | 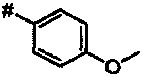 | H | |
| I-407 | 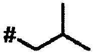 | H | 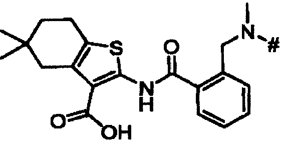 | 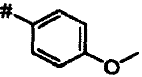 | H | |
| I-408 | 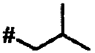 | H | 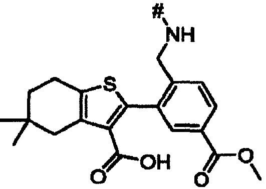 | 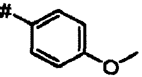 | H | |

**[Table 44]**

| No. | R1 | R2 | R3 | R4 | R5 | salt |
|---|---|---|---|---|---|---|
| I-409 | 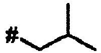 | H | 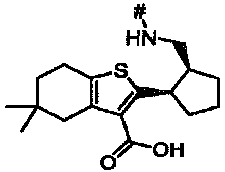 | 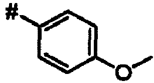 | H | |
| I-410 | 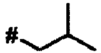 | H | 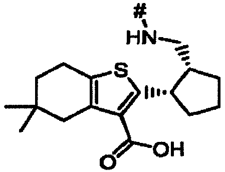 | 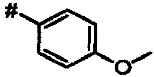 | H | |
| I-411 | 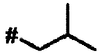 | H | 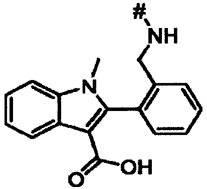 | 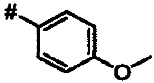 | H | |
| I-412 | 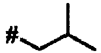 | H | 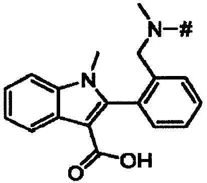 | 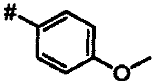 | H | |
| I-413 | 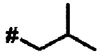 | H | 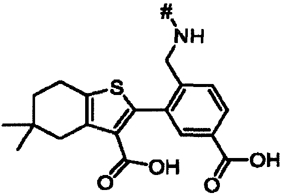 | 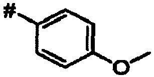 | H | |
| I-414 | 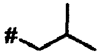 | H | 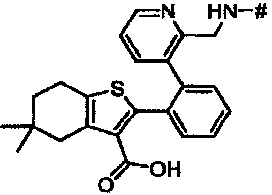 | 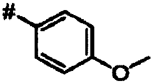 | H | |
| I-415 | 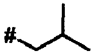 | H | 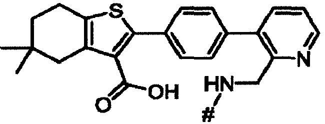 | 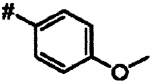 | H | |

**[Table 45]**

| No. | R1 | R2 | R3 | R4 | R5 | salt |
|---|---|---|---|---|---|---|
| I-416 | 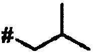 | H | 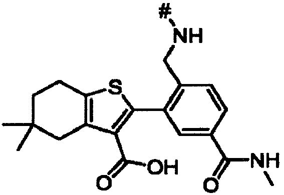 | 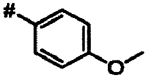 | H | |
| I-417 | 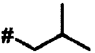 | H | 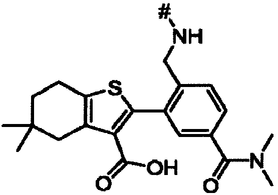 | 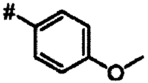 | H | |
| I-418 | 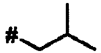 | H | 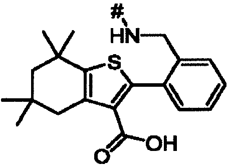 | 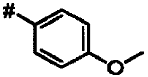 | H | |
| I-419 | 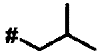 | H | 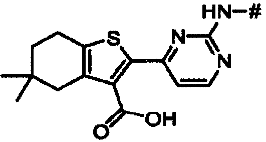 | 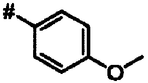 | H | |
| I-420 | 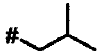 | H | 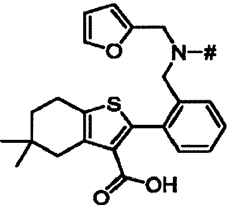 | 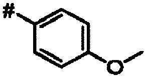 | H | |
| I-421 | 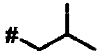 | H | 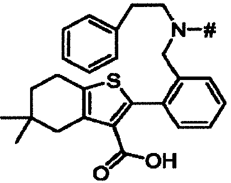 | 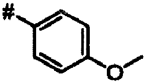 | H | |
| I-422 | 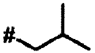 | H | 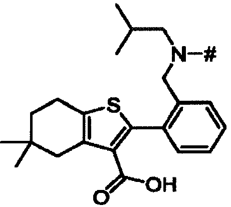 | 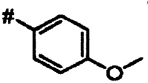 | H | |

**[Table 46]**

| No. | R1 | R2 | R3 | R4 | R5 | salt |
|---|---|---|---|---|---|---|
| I-423 | 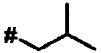 | H | 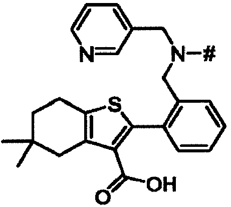 | 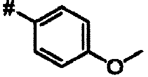 | H | |
| I-424 | 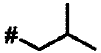 | H | 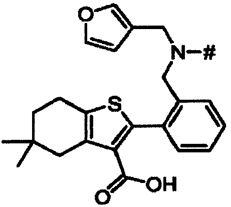 | 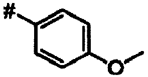 | H | |
| I-425 | 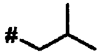 | H | 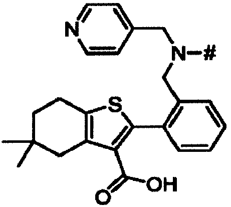 | 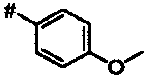 | H | |
| I-426 | 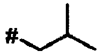 | H | 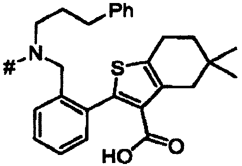 | 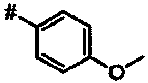 | H | |
| I-427 | 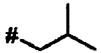 | H | 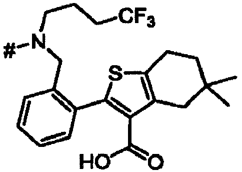 | 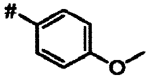 | H | |
| I-428 | 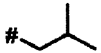 | H | 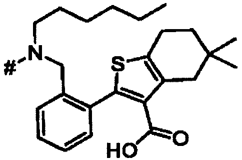 | 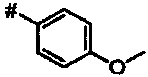 | H | |
| I-429 | 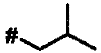 | H | 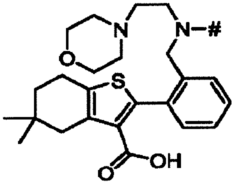 | 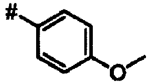 | H | |

**[Table 47]**

| No. | R1 | R2 | R3 | R4 | R5 | salt |
|---|---|---|---|---|---|---|
| I-430 | 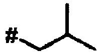 | H | 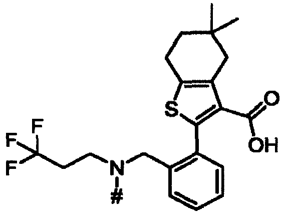 | 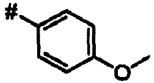 | H | |
| I-431 | 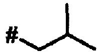 | H | 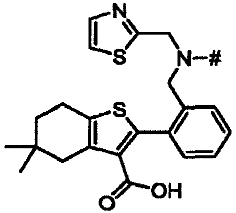 | 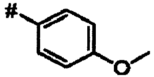 | H | |
| I-432 | 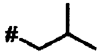 | H | 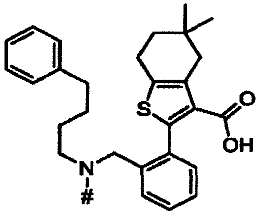 | 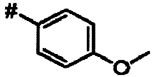 | H | |
| I-433 | 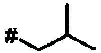 | H | 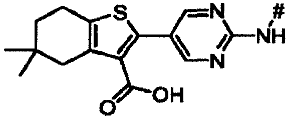 | 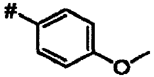 | H | |
| I-434 | 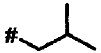 | H | 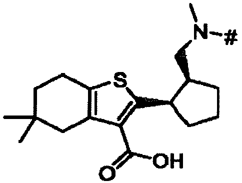 | 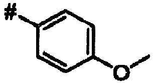 | H | |
| I-435 | 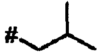 | H | 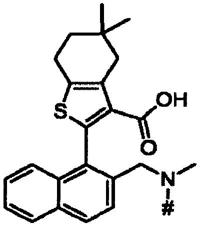 | 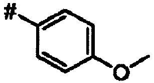 | H | |
| I-436 | 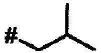 | H | 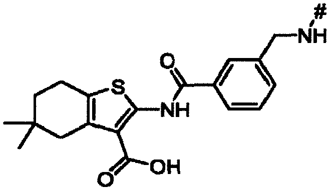 | 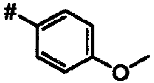 | H | |

**[Table 48]**

| No. | R1 | R2 | R3 | R4 | R5 | salt |
|---|---|---|---|---|---|---|
| I-437 | 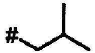 | H | 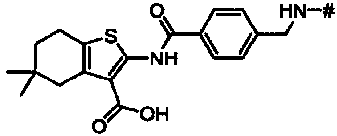 | 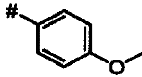 | H | |
| I-438 | 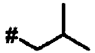 | H | 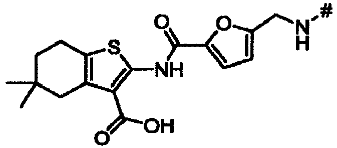 | 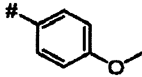 | H | |
| I-439 | 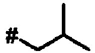 | H | 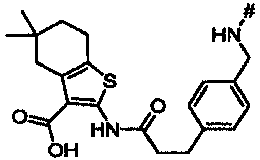 | 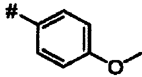 | H | |
| I-440 | 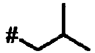 | H | 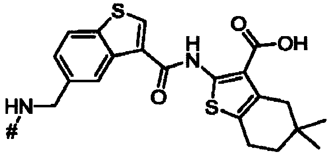 | 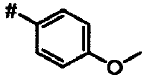 | H | |
| I-441 | 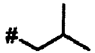 | H | 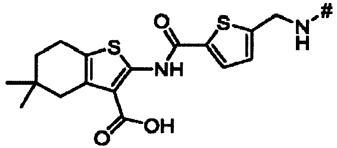 | 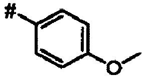 | H | |
| I-442 | 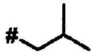 | H | 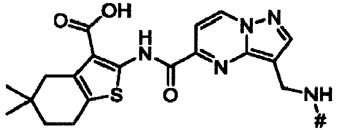 | 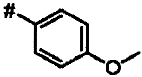 | H | |
| I-443 | 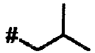 | H | 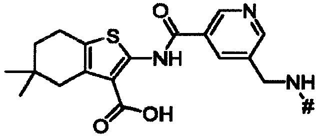 | 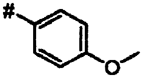 | H | |
| I-444 | 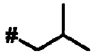 | H | 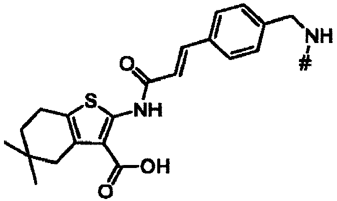 | 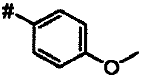 | H | |
| I-445 | 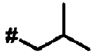 | H | 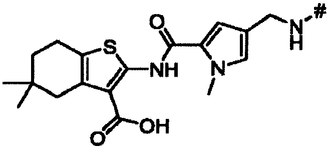 | 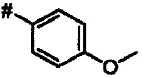 | H | |

**[Table 49]**

| No. | R1 | R2 | R3 | R4 | R5 | salt |
|---|---|---|---|---|---|---|
| I-446 | 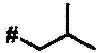 | H | 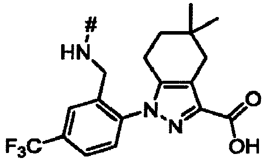 | 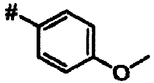 | H | |
| I-447 | 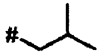 | H | 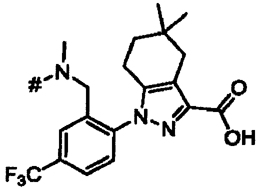 | 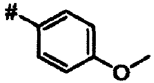 | H | |
| I-448 | 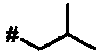 | H | 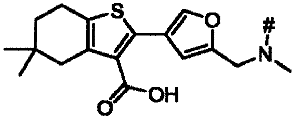 | 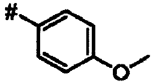 | H | |
| I-449 | 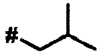 | H | 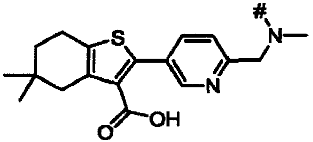 | 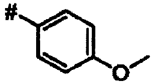 | H | |
| I-450 | 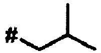 | H | 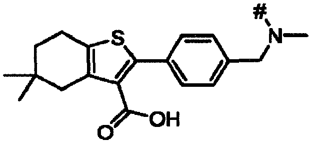 | 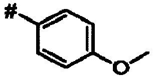 | H | |
| I-451 | 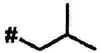 | H | 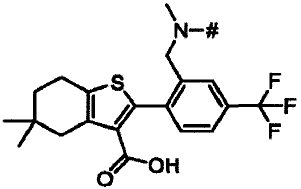 | 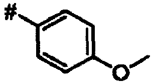 | H | |
| I-452 | 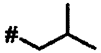 | H | 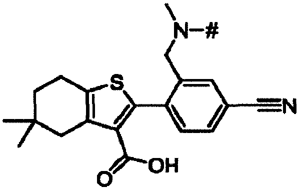 | 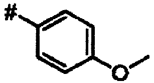 | H | |
| I-453 | 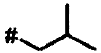 | H | 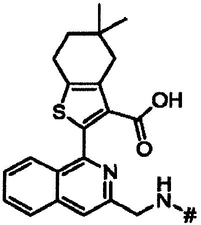 | 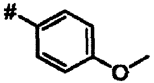 | H | |

**[Table 50]**

| No. | R1 | R2 | R3 | R4 | R5 | salt |
|---|---|---|---|---|---|---|
| I-454 | 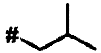 | H | 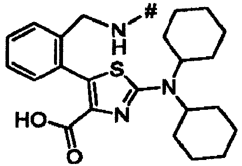 | 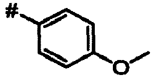 | H | |
| I-455 | 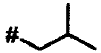 | H | 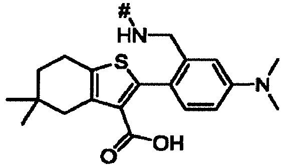 | 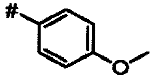 | H | |
| I-456 | 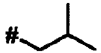 | H | 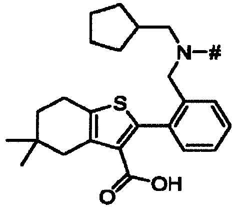 | 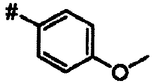 | H | |
| I-457 | 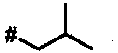 | H | 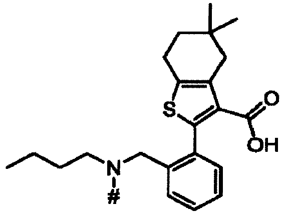 | 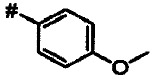 | H | |
| I-458 | 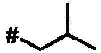 | H | 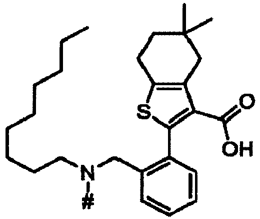 | 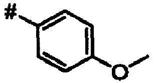 | H | |
| I-459 | 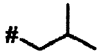 | H | 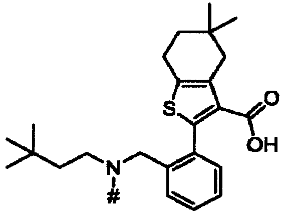 | 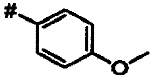 | H | |
| I-460 | 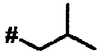 | H | 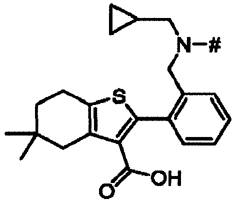 | 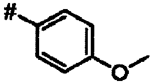 | H | |

**[Table 51]**

| No. | R1 | R2 | R3 | R4 | R5 | salt |
|---|---|---|---|---|---|---|
| I-461 | 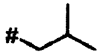 | H | 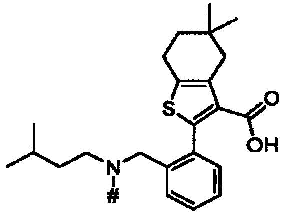 | 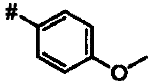 | H | |
| I-462 | 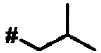 | H | 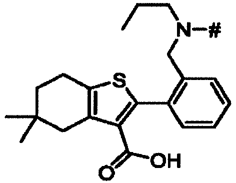 | 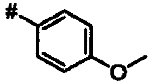 | H | |
| I-463 | 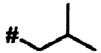 | H | 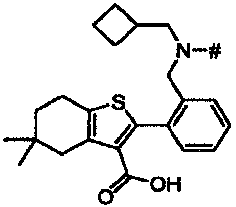 | 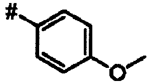 | H | |
| I-464 | 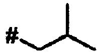 | H | 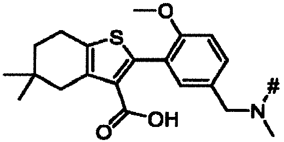 | 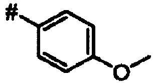 | H | |
| 1-465 | 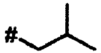 | H | 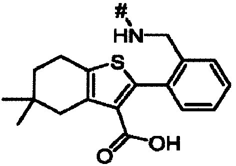 | 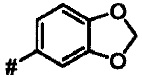 | H | |
| I-466 | 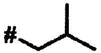 | H | 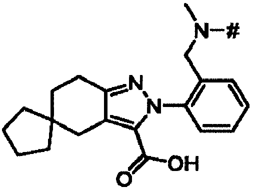 | 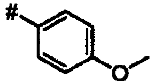 | H | |
| I-467 | 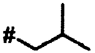 | H | 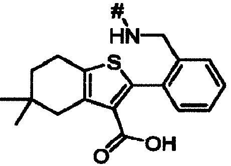 | 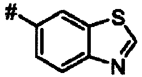 | H | |
| I-468 | 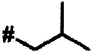 | H | 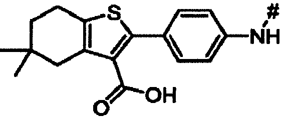 | 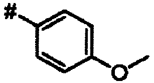 | H | |

**[Table 52]**

| No. | R1 | R2 | R3 | R4 | R5 | salt |
|---|---|---|---|---|---|---|
| I-469 | 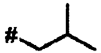 | H | 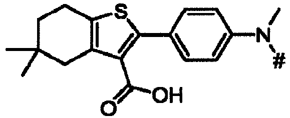 | 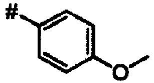 | H | |
| I-470 | 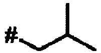 | H | 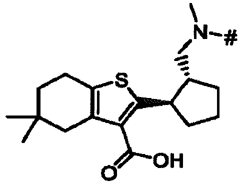 | 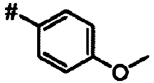 | H | |
| I-471 | 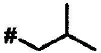 | H | 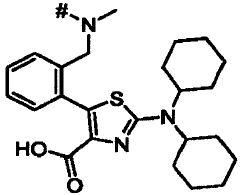 | 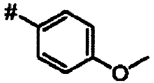 | H | |
| I-472 | 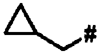 | H | 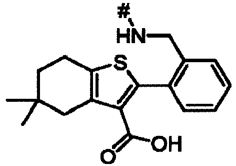 | 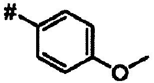 | H | |
| I-473 | 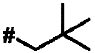 | H | 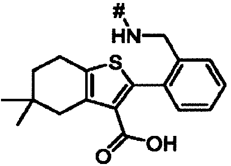 | 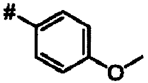 | H | |
| I-474 | 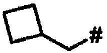 | H | 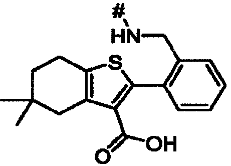 | 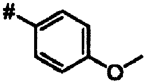 | H | |
| I-475 | 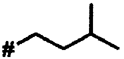 | H | 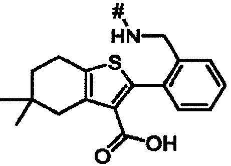 | 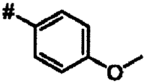 | H | |
| I-476 | 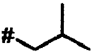 | H | 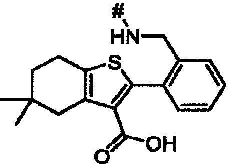 | 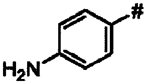 | H | |

**[Table 53]**

| No. | R1 | R2 | R3 | R4 | R5 | salt |
|---|---|---|---|---|---|---|
| I-477 | 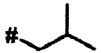 | H | 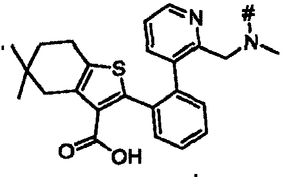 | 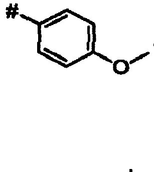 | H | |
| I-478 | 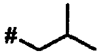 | H | 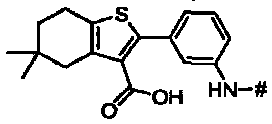 | 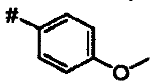 | H | |
| I-479 | 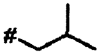 | H | 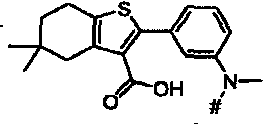 | 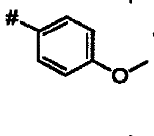 | H | |
| I-480 | 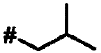 | H | 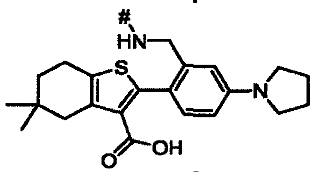 | 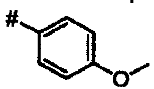 | H | |
| I-481 | 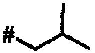 | H | 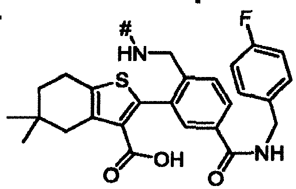 | 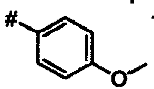 | H | |
| I-482 | 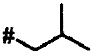 | H | 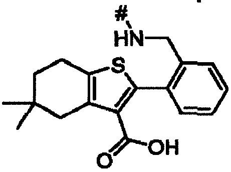 | 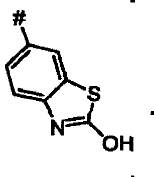 | H | |
| I-483 | 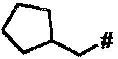 | H | 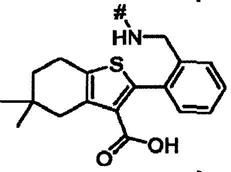 | 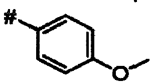 | H | |
| I-484 | 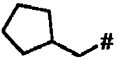 | H | 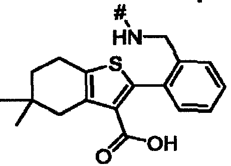 | 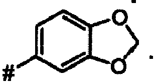 | H | |

**[Table 54]**

| No. | R1 | R2 | R3 | R4 | R5 | salt |
|---|---|---|---|---|---|---|
| I-485 | 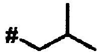 | H | 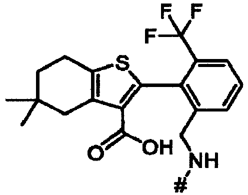 | 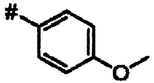 | H | |
| I-486 | 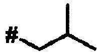 | H | 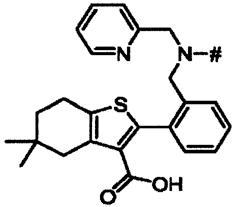 | 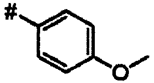 | H | |
| I-487 | 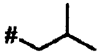 | H | 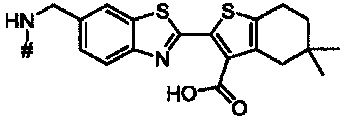 | 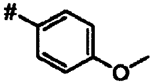 | H | |
| I-488 | 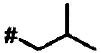 | H | 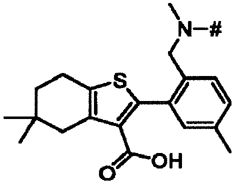 | 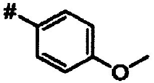 | H | |
| I-489 | 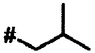 | H | 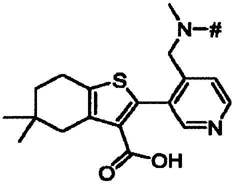 | 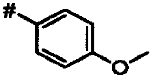 | H | |
| I-490 | 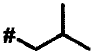 | H | 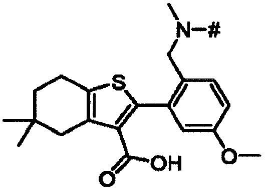 | 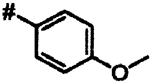 | H | |
| I-491 | 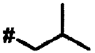 | H | 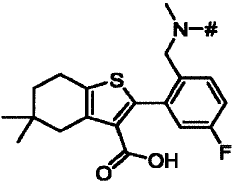 | 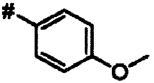 | H | |
| I-492 | 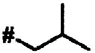 | H | 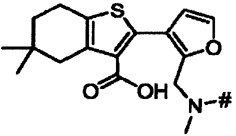 | 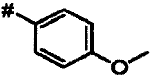 | H | |

**[Table 55]**

| No. | R1 | R2 | R3 | R4 | R5 | salt |
|---|---|---|---|---|---|---|
| I-493 | 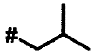 | H | 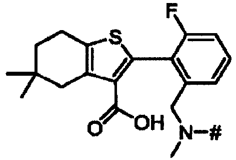 | 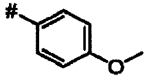 | H | |
| I-494 | 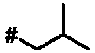 | H | 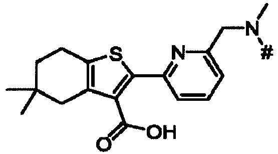 | 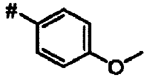 | H | |
| I-495 | 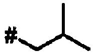 | H | 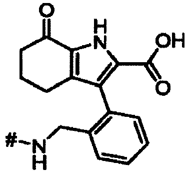 | 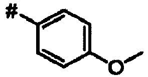 | H | |
| I-496 | 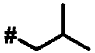 | H | 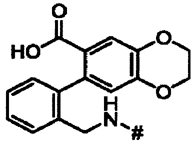 | 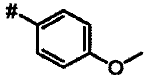 | H | |
| I-497 | 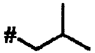 | H | 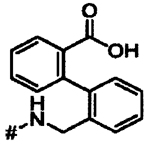 | 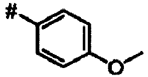 | H | |
| I-498 | 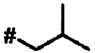 | H | 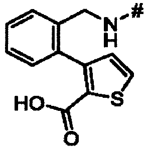 | 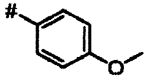 | H | |
| I-499 | 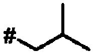 | H | 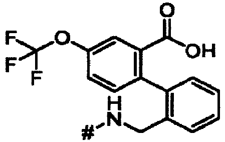 | 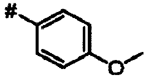 | H | |
| I-500 | 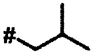 | H | 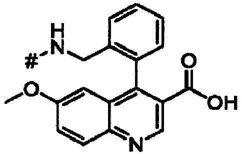 | 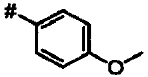 | H | |
| I-501 | 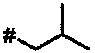 | H | 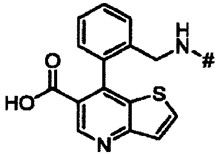 | 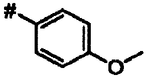 | H | |

**[Table 56]**

| No. | R1 | R2 | R3 | R4 | R5 | salt |
|---|---|---|---|---|---|---|
| I-502 | 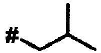 | H | 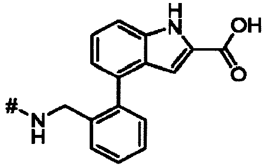 | 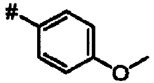 | H | |
| I-503 | 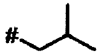 | H | 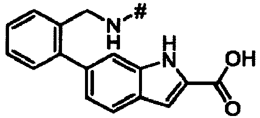 | 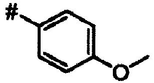 | H | |
| I-504 | 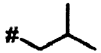 | H | 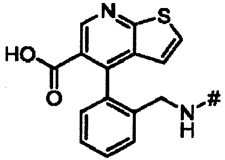 | 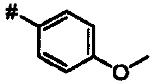 | H | |
| I-505 | 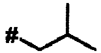 | H | 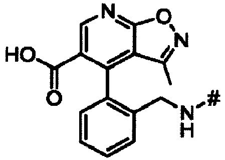 | 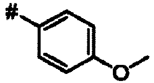 | H | |
| I-506 | 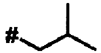 | H | 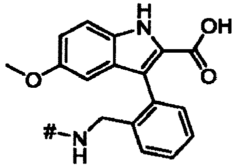 | 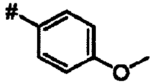 | H | |
| I-507 | 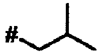 | H | 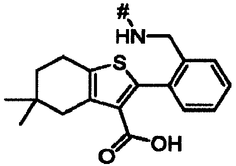 | 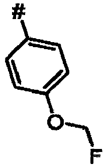 | H | |
| I-508 | 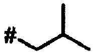 | H | 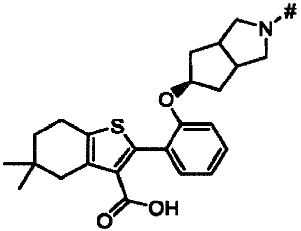 | 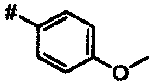 | H | |
| I-509 | 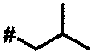 | H | 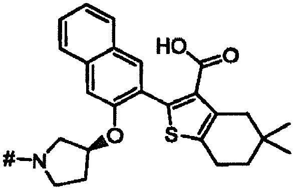 | 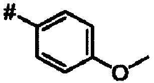 | H | |

**[Table 57]**

| No. | R1 | R2 | R3 | R4 | R5 | salt |
|---|---|---|---|---|---|---|
| I-510 | 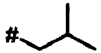 | H | 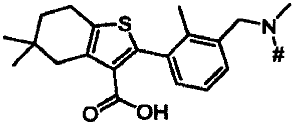 | 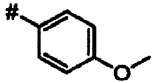 | H | |
| I-511 | 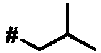 | H | 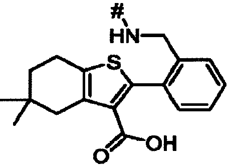 | 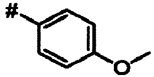 | OH | |
| I-512 | 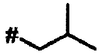 | H | 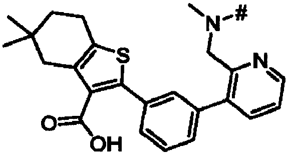 | 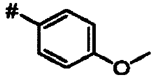 | H | |
| I-513 | 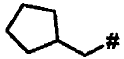 | H | 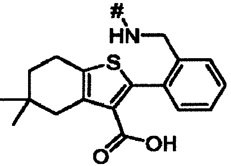 | 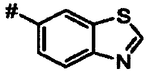 | H | |
| I-514 | 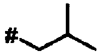 | H | 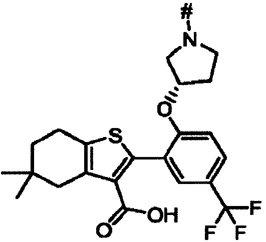 | 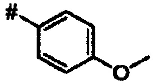 | H | |
| I-515 | 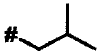 | H | 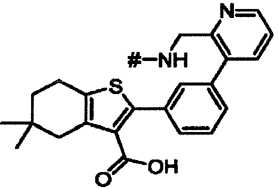 | 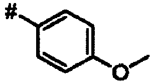 | H | |
| I-516 | 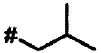 | H | 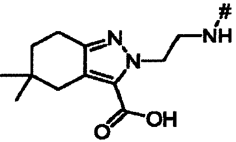 | 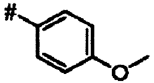 | H | |
| I-517 | 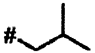 | H | 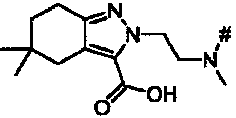 | 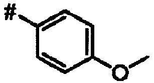 | H | |

**[Table 58]**

| No. | R1 | R2 | R3 | R4 | R5 | salt |
|---|---|---|---|---|---|---|
| I-518 | 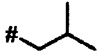 | H | 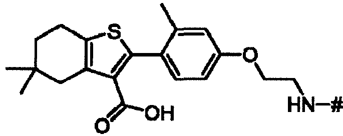 | 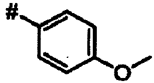 | H | |
| I-519 | 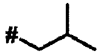 | H | 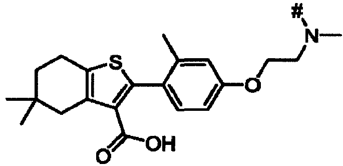 | 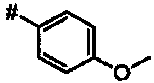 | H | |
| I-520 | 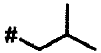 | H | 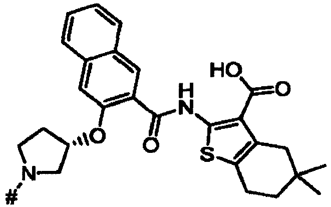 | 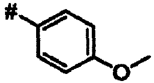 | H | |
| I-521 | 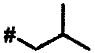 | H | 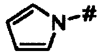 | 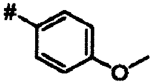 | H | |
| I-522 | 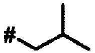 | H | 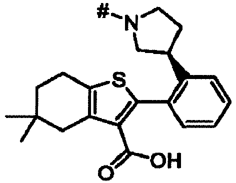 | 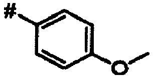 | H | |
| I-523 | 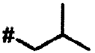 | H | 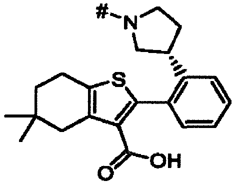 | 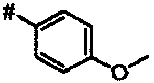 | H | |
| I-524 | 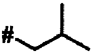 | H | 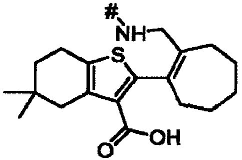 | 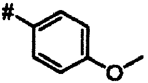 | H | |
| I-525 | 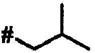 | H | 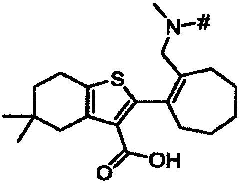 | 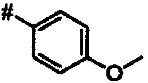 | H | |

**[Table 59]**

| No. | R1 | R2 | R3 | R4 | R5 | salt |
|---|---|---|---|---|---|---|
| I-526 | 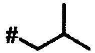 | H | 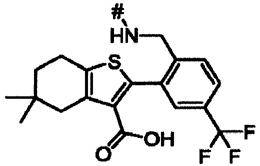 | 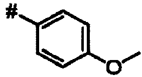 | H | |
| I-527 | 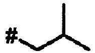 | H | 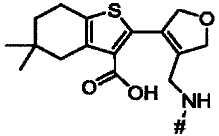 | 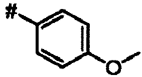 | H | |
| I-528 | 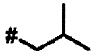 | H | 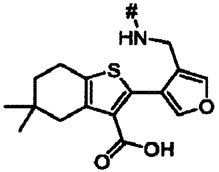 | 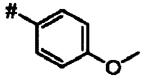 | H | |
| I-529 | 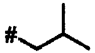 | H | 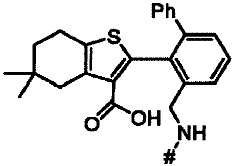 | 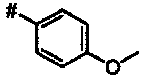 | H | |
| I-530 | 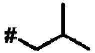 | H | 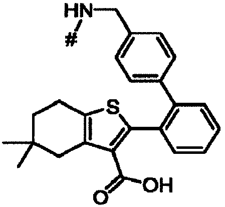 | 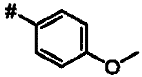 | H | |
| I-531 | 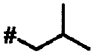 | H | 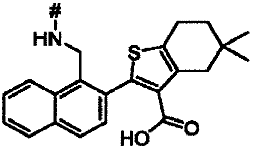 | 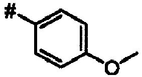 | H | |
| I-532 | 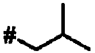 | H | 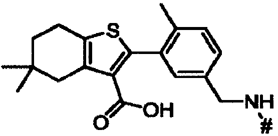 | 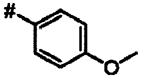 | H | |
| I-533 | 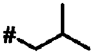 | H | 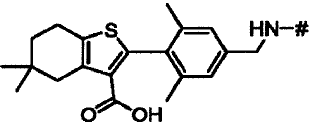 | 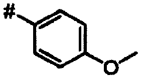 | H | |
| I-534 | 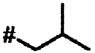 | H | 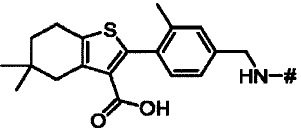 | 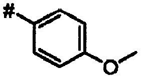 | H | |

**[Table 60]**

| No. | R1 | R2 | R3 | R4 | R5 | salt |
|---|---|---|---|---|---|---|
| I-535 | 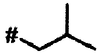 | H | 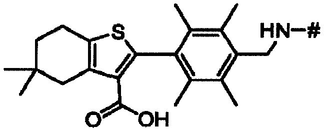 | 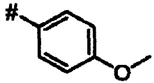 | H | |
| I-536 | 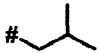 | H | 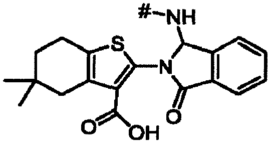 | 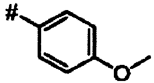 | H | |
| I-537 | 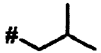 | H | 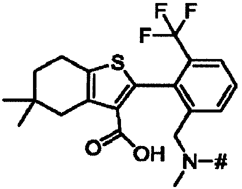 | 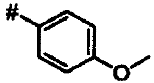 | H | |
| I-538 | 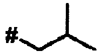 | H | 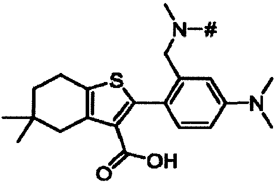 | 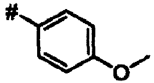 | H | |
| I-539 | 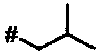 | H | 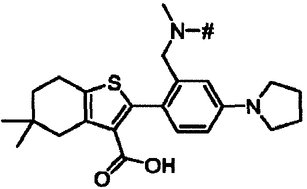 | 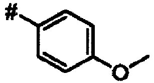 | H | |
| I-540 | 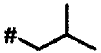 | H | 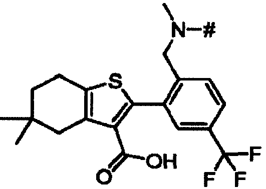 | 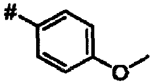 | H | |
| I-541 | 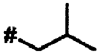 | H | 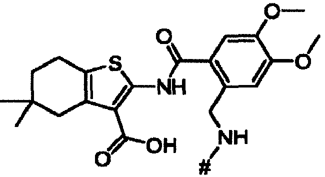 | 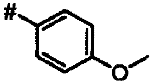 | H | |
| I-542 | 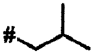 | H | 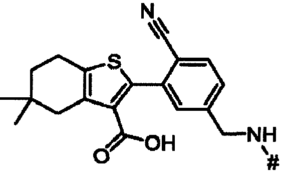 | 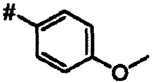 | H | |

**[Table 61]**

| No. | R1 | R2 | R3 | R4 | R5 | salt |
|---|---|---|---|---|---|---|
| I-543 | 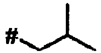 | H | 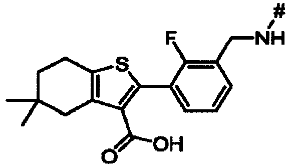 | 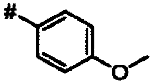 | H | |
| I-544 | 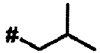 | H | 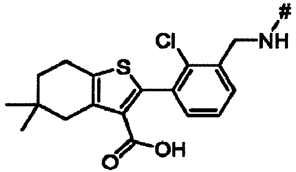 | 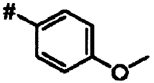 | H | |
| I-545 | 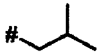 | H | 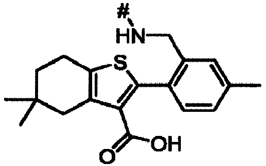 | 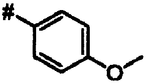 | H | |
| I-546 | 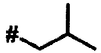 | H | 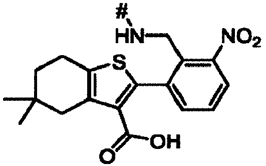 | 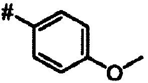 | H | |
| I-547 | 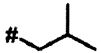 | H | 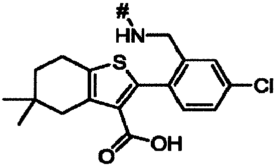 | 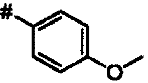 | H | |
| I-548 | 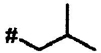 | H | 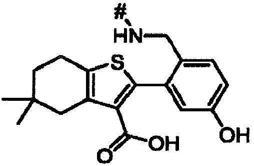 | 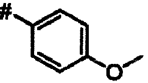 | H | |
| I-549 | 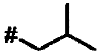 | H | 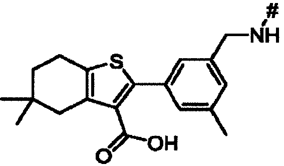 | 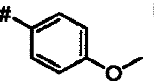 | H | |
| I-550 | 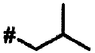 | H | 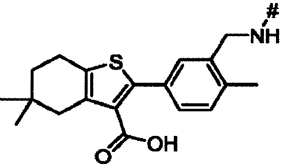 | 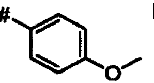 | H | |

**[Table 62]**

| No. | R1 | R2 | R3 | R4 | R5 | salt |
|---|---|---|---|---|---|---|
| I-551 | 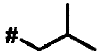 | H | 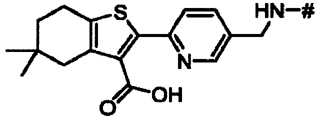 | 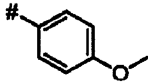 | H | |
| I-552 | 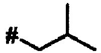 | H | 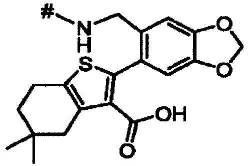 | 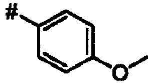 | H | |
| I-553 | 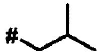 | H | 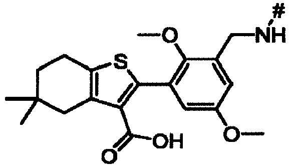 | 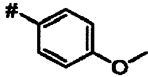 | H | |
| I-554 | 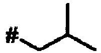 | H | 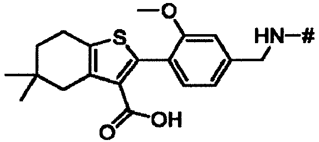 | 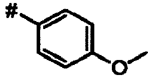 | H | |
| I-555 | 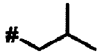 | H | 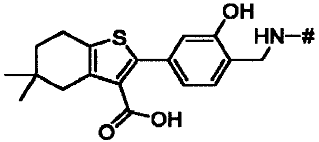 | 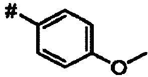 | H | |
| I-556 | 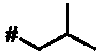 | H | 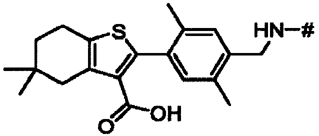 | 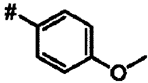 | H | |
| I-557 | 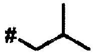 | H | 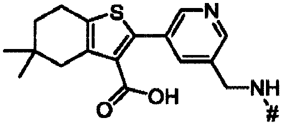 | 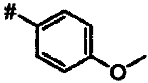 | H | |
| I-558 | 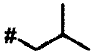 | H | 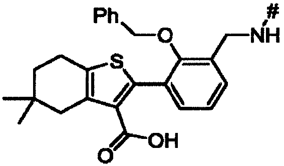 | 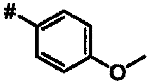 | H | |
| I-559 | 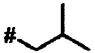 | H | 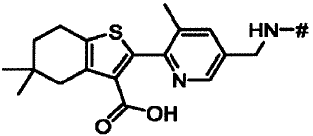 | 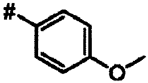 | H | |

**[Table 63]**

| No. | R1 | R2 | R3 | R4 | R5 | salt |
|---|---|---|---|---|---|---|
| I-560 | 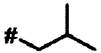 | H | 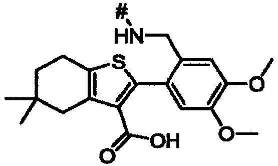 | 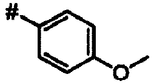 | H | |
| I-561 | 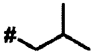 | H | 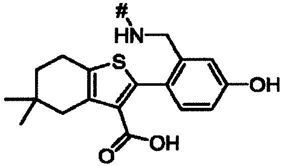 | 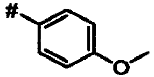 | H | |
| I-562 | 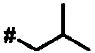 | H | 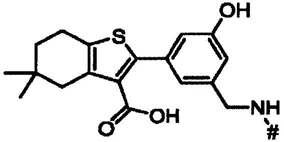 | 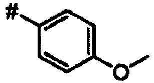 | H | |
| I-563 | 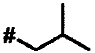 | H | 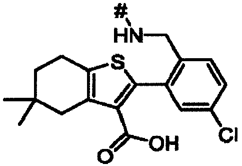 | 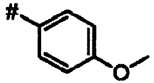 | H | |
| I-564 | 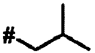 | H | 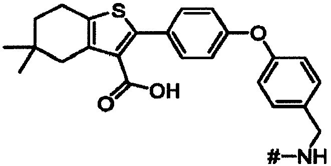 | 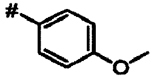 | H | |
| I-565 | 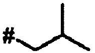 | H | 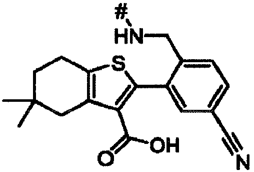 | 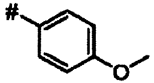 | H | |
| I-566 | 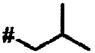 | H | 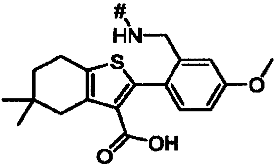 | 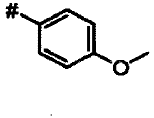 | H | |
| I-567 | 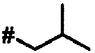 | H | 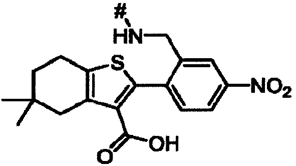 | 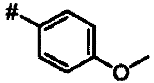 | H | |

**[Table 64]**

| No. | R1 | R2 | R3 | R4 | R5 | salt |
|---|---|---|---|---|---|---|
| I-568 | 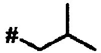 | H | 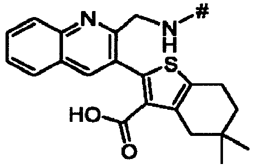 | 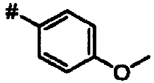 | H | |
| I-569 | 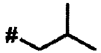 | H | 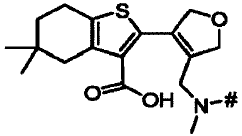 | 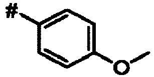 | H | |
| I-570 | 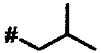 | H | 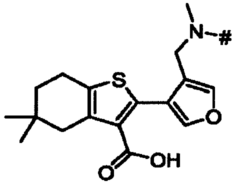 | 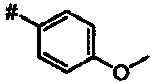 | H | |
| I-571 | 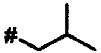 | H | 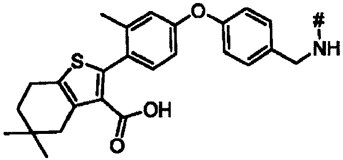 | 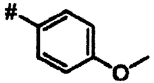 | H | |
| I-572 | 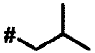 | H | 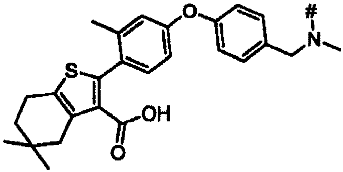 | 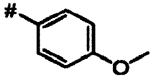 | H | |
| I-573 | 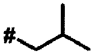 | H | 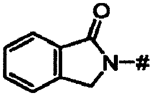 | 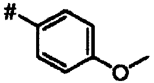 | H | |
| I-574 | 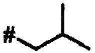 | H | 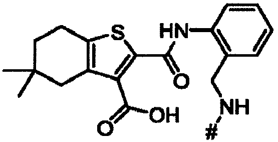 | 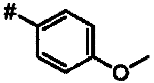 | H | |
| I-575 | 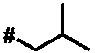 | H | 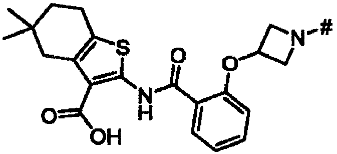 | 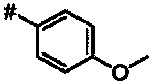 | H | |
| I-576 | 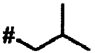 | H | 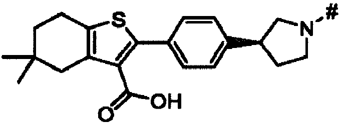 | 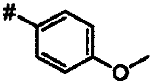 | H | |

**[Table 65]**

| No. | R1 | R2 | R3 | R4 | R5 | salt |
|---|---|---|---|---|---|---|
| I-577 | 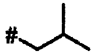 | H | 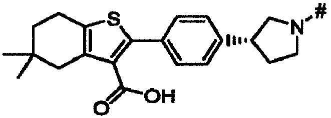 | 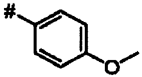 | H | |
| I-578 | 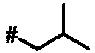 | H | 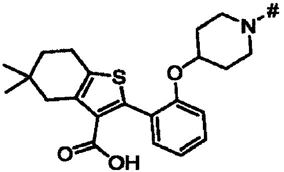 | 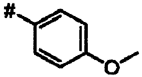 | H | |
| I-579 | 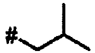 | H | 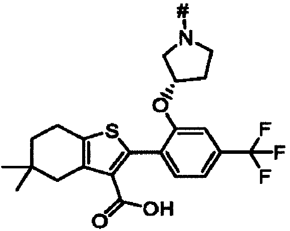 | 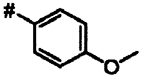 | H | |
| I-580 | 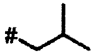 | H | 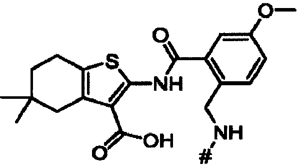 | 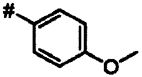 | H | |
| I-581 | 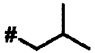 | H | 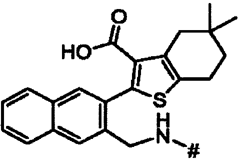 | 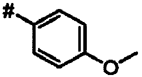 | H | |
| I-582 | 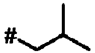 | H | 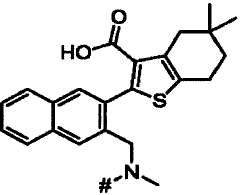 | 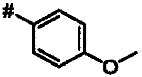 | H | |
| I-583 | 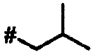 | H | 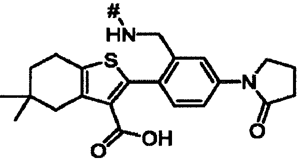 | 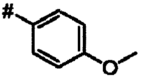 | H | |
| I-584 | 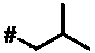 | H | 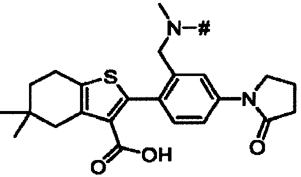 | 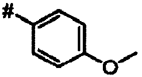 | H | |

**[Table 66]**

| No. | R1 | R2 | R3 | R4 | R5 | salt |
|---|---|---|---|---|---|---|
| I-585 | 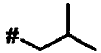 | H | 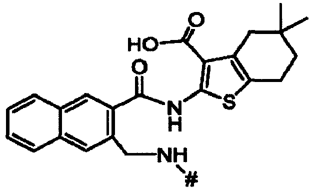 | 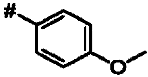 | H | |
| I-586 | 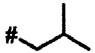 | H | 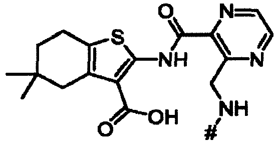 | 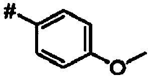 | H | |
| I-587 | 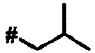 | H | 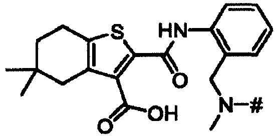 | 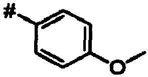 | H | |
| I-588 | 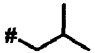 | H | 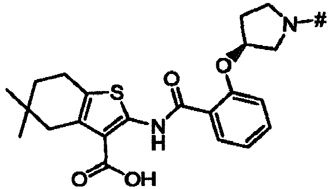 | 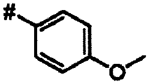 | H | |
| I-589 | 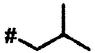 | H | 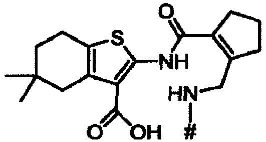 | 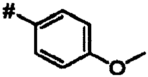 | H | |
| I-590 | 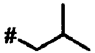 | H | 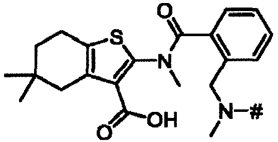 | 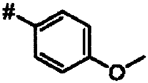 | H | |
| I-591 | 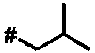 | H | 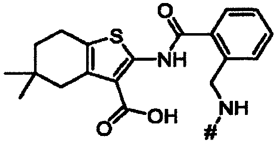 | 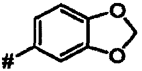 | H | |
| I-592 | 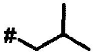 | H | 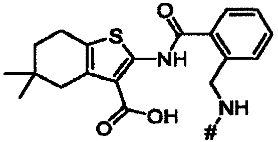 | 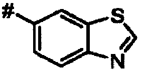 | H | |
| I-594 | 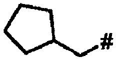 | H | 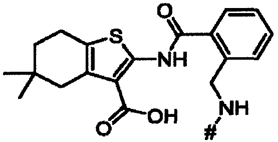 | 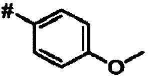 | H | |

**[Table 67]**

| No. | R1 | R2 | R3 | R4 | R5 | salt |
|---|---|---|---|---|---|---|
| I-595 | 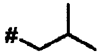 | H | 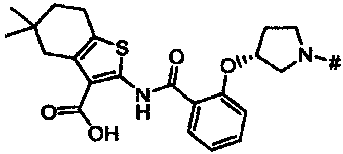 | 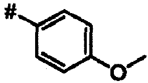 | H | |
| I-597 | 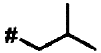 | H | 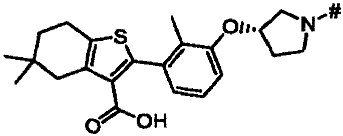 | 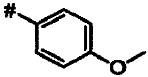 | H | |
| I-598 | 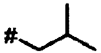 | H | 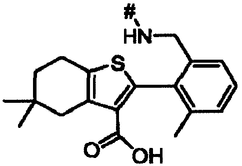 | 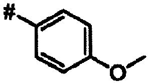 | H | |
| I-599 | 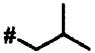 | H | 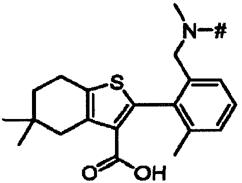 | 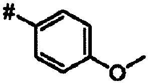 | H | |
| I-600 | 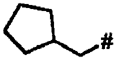 | H | 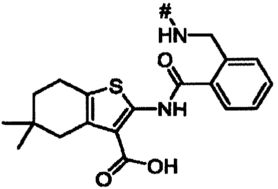 | 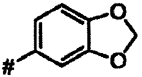 | H | |
| I-601 | 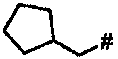 | H | 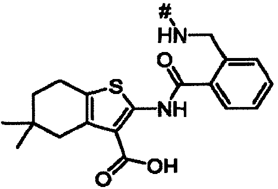 | 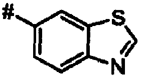 | H | |
| I-602 | 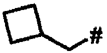 | H | 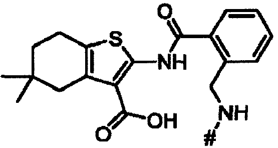 | 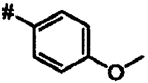 | H | |
| I-603 | 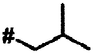 | H | 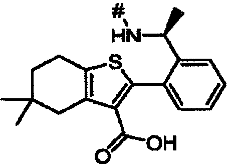 | 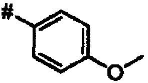 | H | |

**[Table 68]**

| No. | R1 | R2 | R3 | R4 | R5 | salt |
|---|---|---|---|---|---|---|
| I-604 | 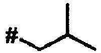 | H | 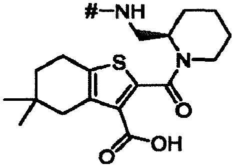 | 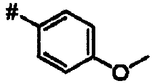 | H | |
| I-605 | 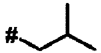 | H | 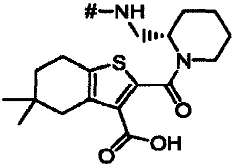 | 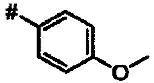 | H | |
| I-606 | 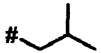 | H | 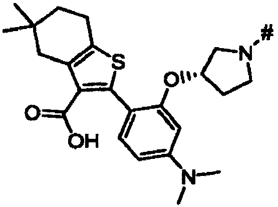 | 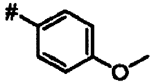 | H | |
| I-607 | 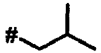 | H | 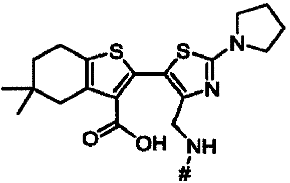 | 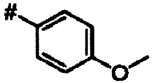 | H | |
| I-608 | 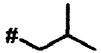 | H | 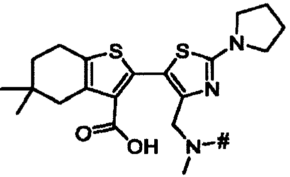 | 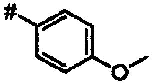 | H | |
| I-609 | 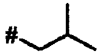 | H | 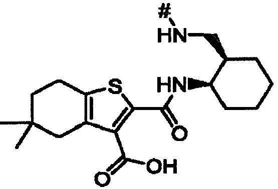 | 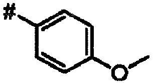 | H | |
| I-610 | 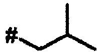 | H | 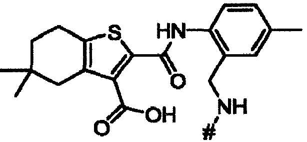 | 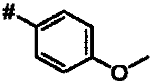 | H | |
| I-611 | 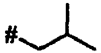 | H | 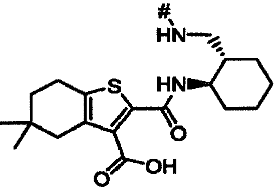 | 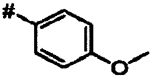 | H | |

**[Table 69]**

| No. | R1 | R2 | R3 | R4 | R5 | salt |
|---|---|---|---|---|---|---|
| I-612 | 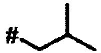 | H | 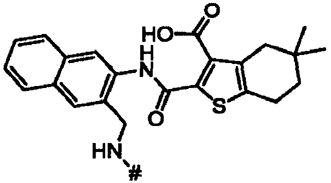 | 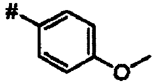 | H | |
| I-613 | 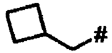 | H | 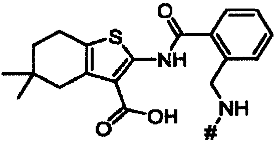 | 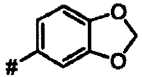 | H | |
| I-614 | 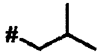 | H | 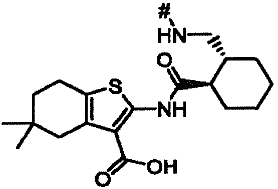 | 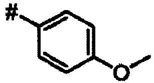 | H | |
| I-615 | 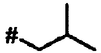 | H | 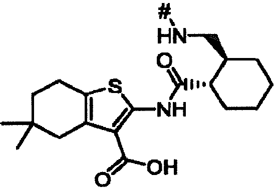 | 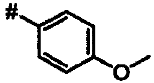 | H | |
| I-616 | 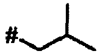 | H | 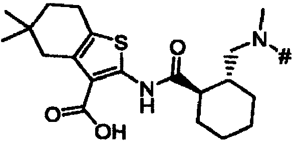 | 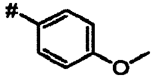 | H | |
| I-617 | 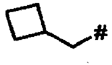 | H | 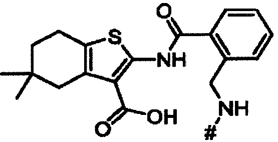 | 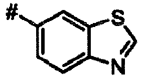 | H | |
| I-618 | 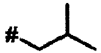 | H | 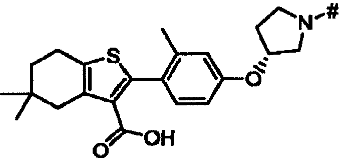 | 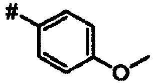 | H | |
| I-619 | 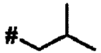 | H | 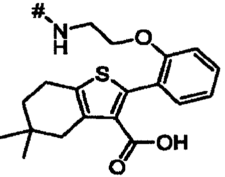 | 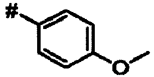 | H | |

**[Table 70]**

| No. | R1 | R2 | R3 | R4 | R5 | salt |
|---|---|---|---|---|---|---|
| I-620 | 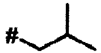 | H | 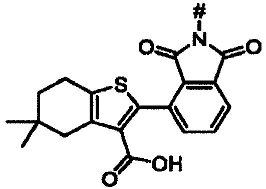 | 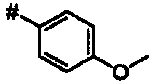 | H | |
| I-621 | 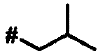 | H | 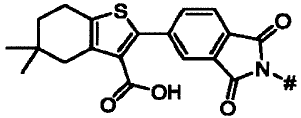 | 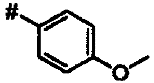 | H | |
| I-622 | 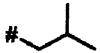 | H | 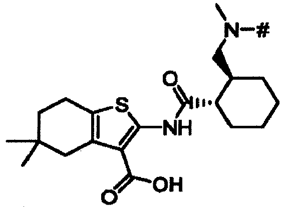 | 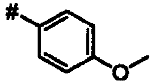 | H | |
| I-623 | 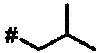 | H | 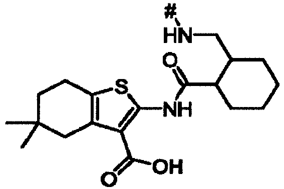 | 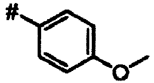 | H | |
| I-624 | 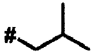 | H | 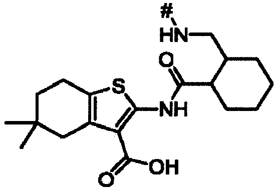 | 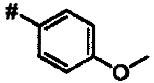 | H | |
| I-625 | 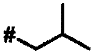 | H | 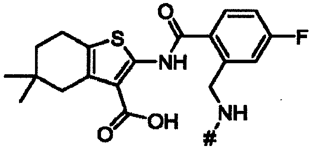 | 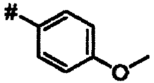 | H | |
| I-626 | 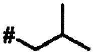 | H | 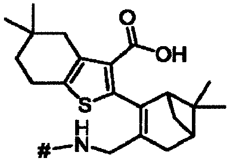 | 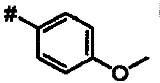 | H | |
| I-627 | 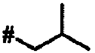 | H | 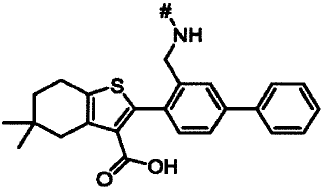 | 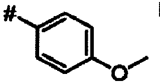 | H | |

**[Table 71]**

| No. | R1 | R2 | R3 | R4 | R5 | salt |
|---|---|---|---|---|---|---|
| I-628 | 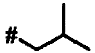 | H | 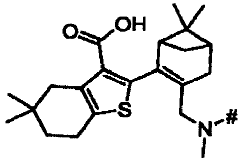 | 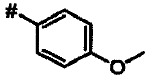 | H | |
| I-629 | 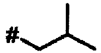 | H | 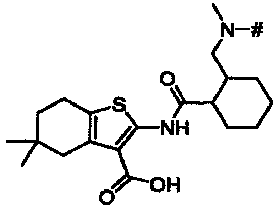 | 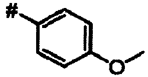 | H | |
| I-630 | 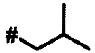 | H | 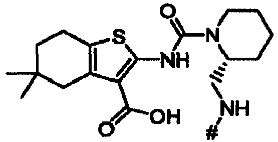 | 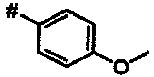 | H | |
| I-631 | 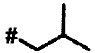 | H | 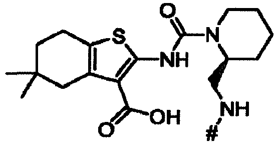 | 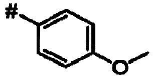 | H | |
| I-632 | 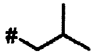 | H | 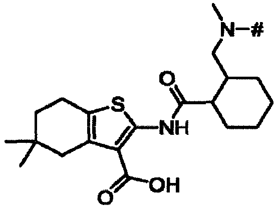 | 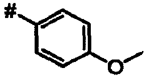 | H | |
| I-633 | 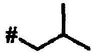 | H | 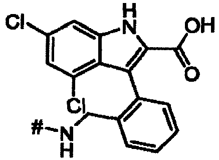 | 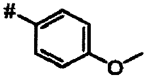 | H | |
| I-634 | 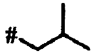 | H | 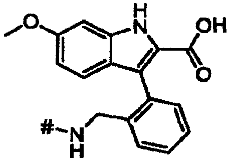 | 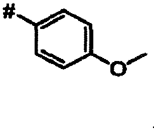 | H | |
| I-635 | 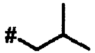 | H | 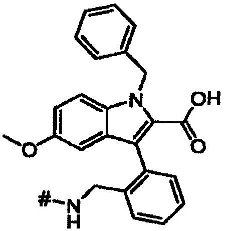 | 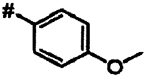 | H | |

**[Table 72]**

| No. | R1 | R2 | R3 | R4 | R5 | salt |
|---|---|---|---|---|---|---|
| I-636 | 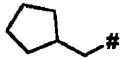 | H | 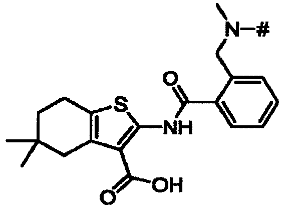 | 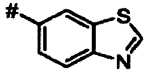 | H | |
| I-637 | 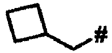 | H | 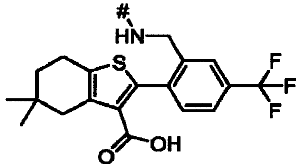 | 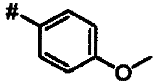 | H | |
| I-638 | 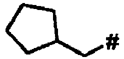 | H | 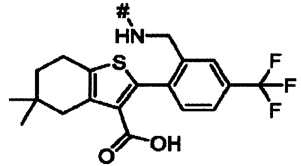 | 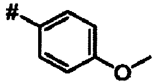 | H | |
| I-639 | 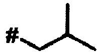 | H | 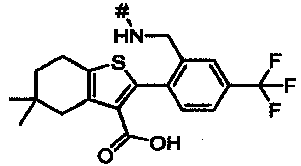 | 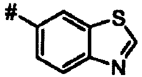 | H | |
| I-640 | 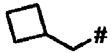 | H | 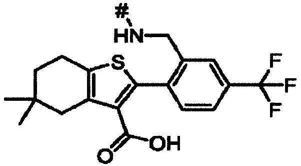 | 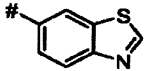 | H | |
| I-641 | 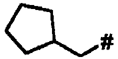 | H | 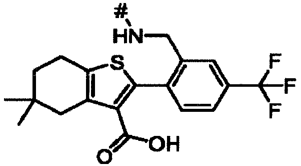 | 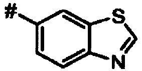 | H | |
| I-642 | 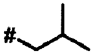 | H | 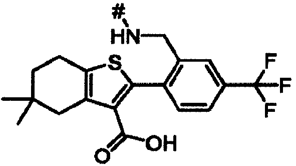 | 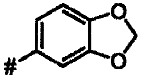 | H | |
| I-643 | 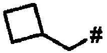 | H | 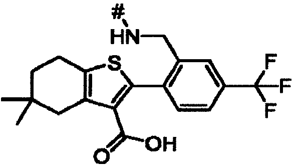 | 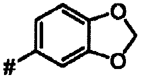 | H | |

**[Table 73]**

| No. | R1 | R2 | R3 | R4 | R5 | salt |
|---|---|---|---|---|---|---|
| I-644 | 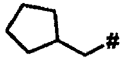 | H | 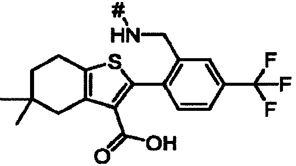 | 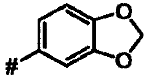 | H | |
| I-645 | 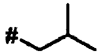 | H | 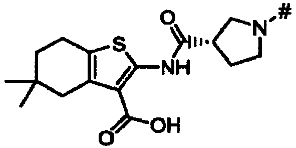 | 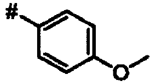 | H | |
| I-646 | 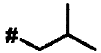 | H | 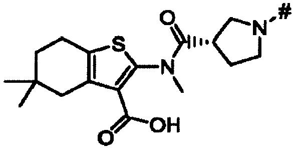 | 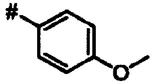 | H | |
| I-647 | 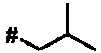 | H | 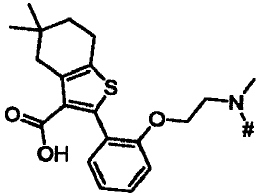 | 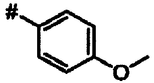 | H | |
| I-648 | 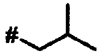 | H | 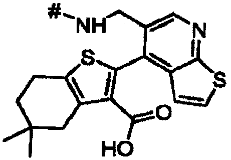 | 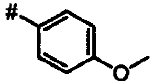 | H | |
| I-649 | 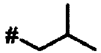 | H | 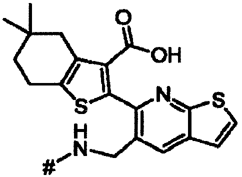 | 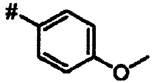 | H | |
| I-650 | 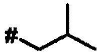 | H | 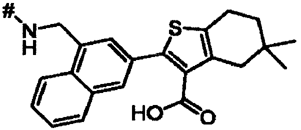 | 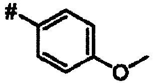 | H | |
| I-651 | 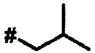 | H | 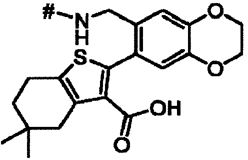 | 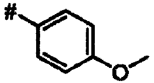 | H | |

**[Table 74]**

| No. | R1 | R2 | R3 | R4 | R5 | salt |
|---|---|---|---|---|---|---|
| I-652 | 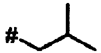 | H | 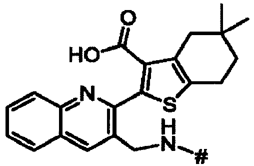 | 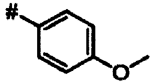 | H | |
| I-653 | 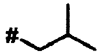 | H | 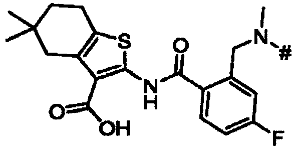 | 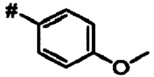 | H | |
| I-654 | 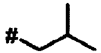 | H | 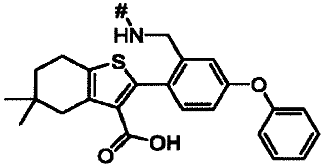 | 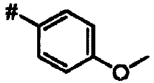 | H | |
| I-655 | 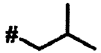 | H | 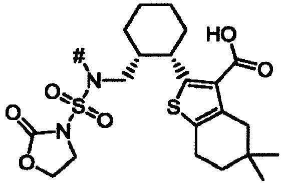 | 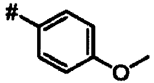 | H | |
| I-656 | 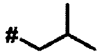 | H | 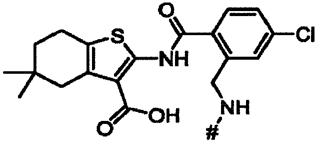 | 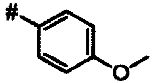 | H | |
| I-657 | 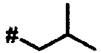 | H | 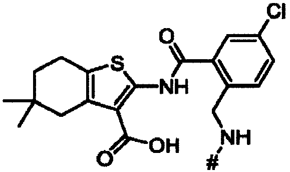 | 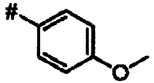 | H | |
| I-658 | 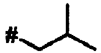 | H | 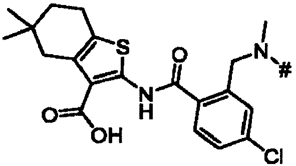 | 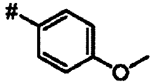 | H | |
| I-659 | 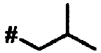 | H | 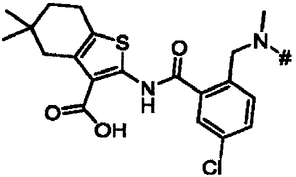 | 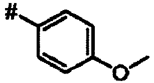 | H | |

**[Table 75]**

| No. | R1 | R2 | R3 | R4 | R5 | salt |
|---|---|---|---|---|---|---|
| I-660 | 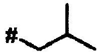 | H | 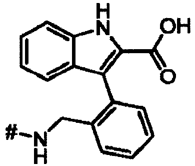 | 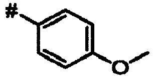 | H | |
| I-661 | 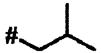 | H | 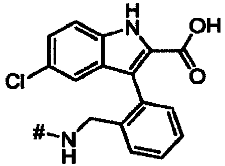 | 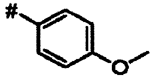 | H | |
| I-662 | 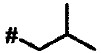 | H | 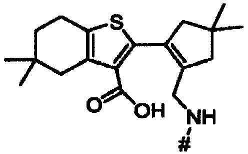 | 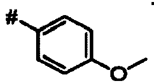 | H | |
| I-663 | 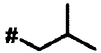 | H | 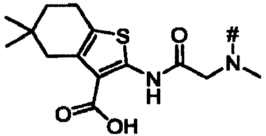 | 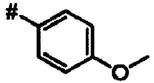 | H | |
| I-664 | 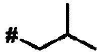 | H | 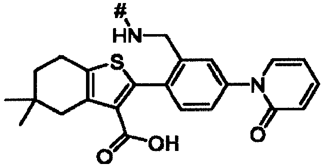 | 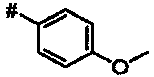 | H | |
| I-665 | 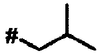 | H | 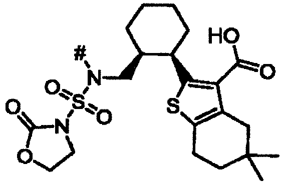 | 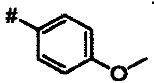 | H | |
| I-666 | 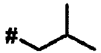 | H | 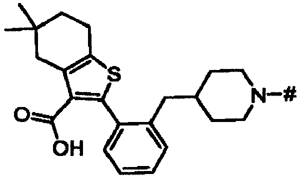 | 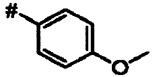 | H | |
| I-667 | 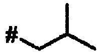 | H | 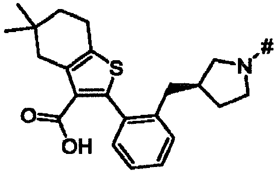 | 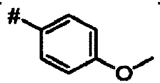 | H | |

**[Table 76]**

| No. | R1 | R2 | R3 | R4 | R5 | salt |
|---|---|---|---|---|---|---|
| I-668 | 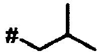 | H | 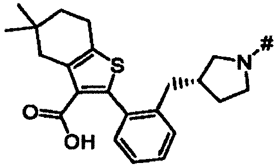 | 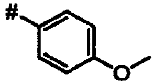 | H | |
| I-669 | 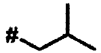 | H | 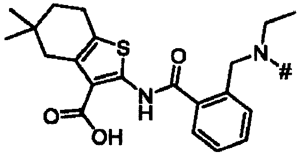 | 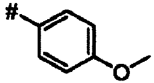 | H | |
| I-670 | 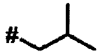 | H | 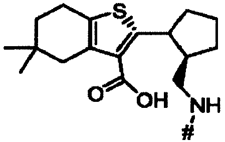 | 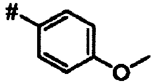 | H | |
| I-671 | 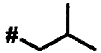 | H | 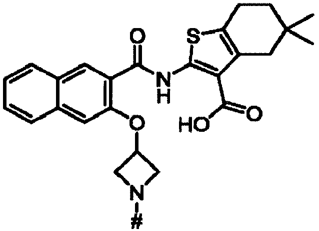 | 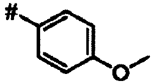 | H | |
| I-672 | 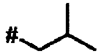 | H | 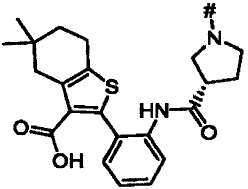 | 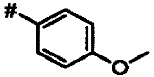 | H | |
| I-673 | 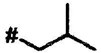 | H | 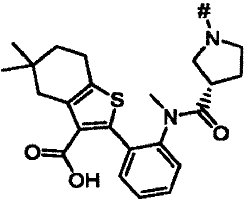 | 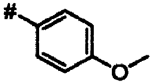 | H | |
| I-674 | 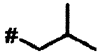 | H | 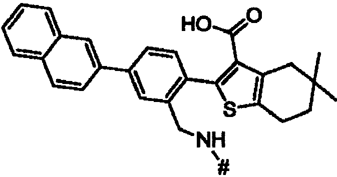 | 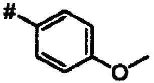 | H | |
| I-675 | 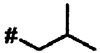 | H | 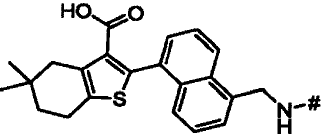 | 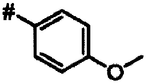 | H | |

**[Table 77]**

| No. | R1 | R2 | R3 | R4 | R5 | salt |
|---|---|---|---|---|---|---|
| I-676 | 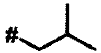 | H | 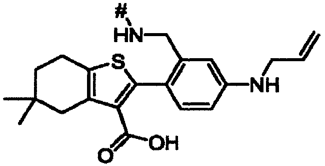 | 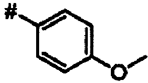 | H | |
| I-677 | 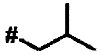 | H | 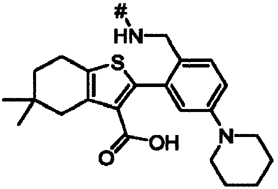 | 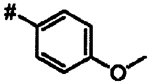 | H | |
| I-678 | 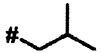 | H | 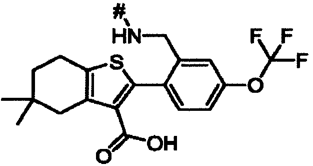 | 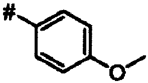 | H | |
| I-679 | 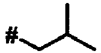 | H | 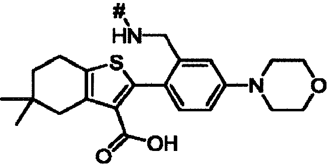 | 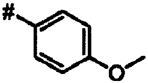 | H | |
| I-680 | 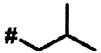 | H | 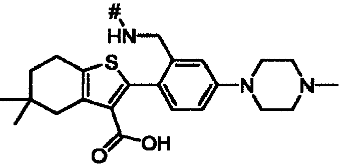 | 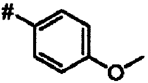 | H | |
| I-681 | 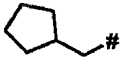 | H | 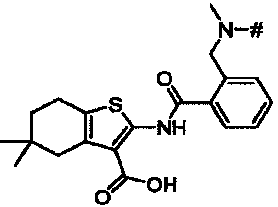 | 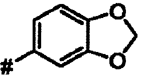 | H | |
| I-682 | 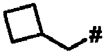 | H | 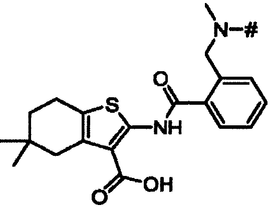 | 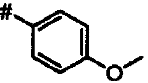 | H | |

**[Table 78]**

| No. | R1 | R2 | R3 | R4 | R5 | salt |
|---|---|---|---|---|---|---|
| I-683 | 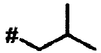 | H | 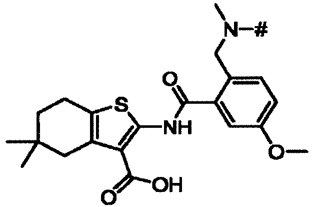 | 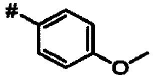 | H | |
| I-684 | 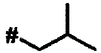 | H | 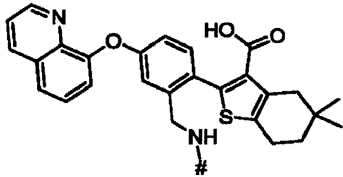 | 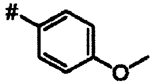 | H | |
| I-685 | 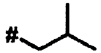 | H | 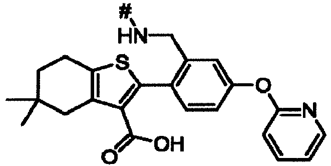 | 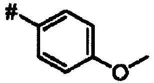 | H | |
| I-686 | 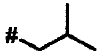 | H | 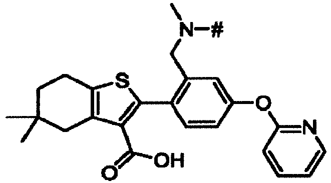 | 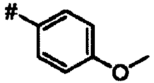 | H | |
| I-687 | 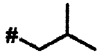 | H | 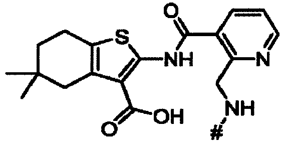 | 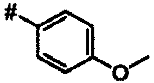 | H | |
| I-688 | 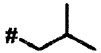 | H | 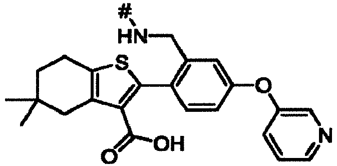 | 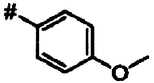 | H | |
| I-689 | 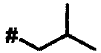 | H | 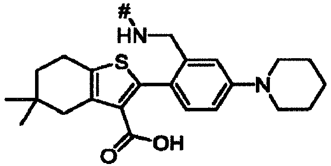 | 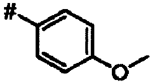 | H | |
| I-690 | 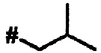 | H | 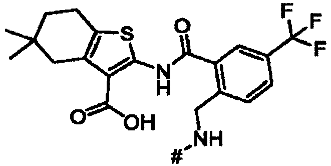 | 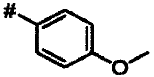 | H | |

**[Table 79]**

| No. | R1 | R2 | R3 | R4 | R5 | salt |
|---|---|---|---|---|---|---|
| I-691 | 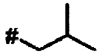 | H | 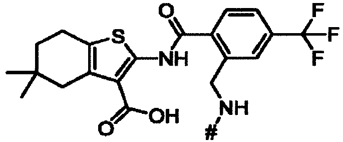 | 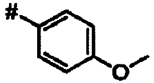 | H | |
| I-692 | 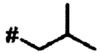 | H | 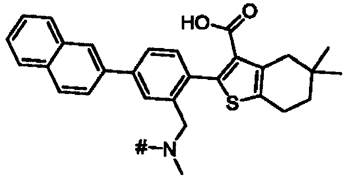 | 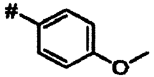 | H | |
| I-693 | 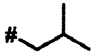 | H | 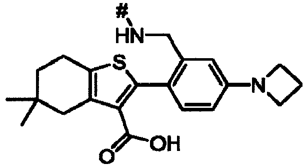 | 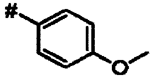 | H | |
| I-694 | 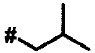 | H | 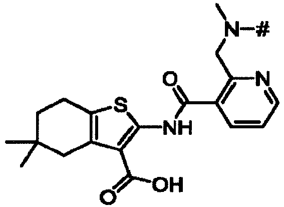 | 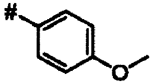 | H | |
| I-695 | 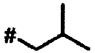 | H | 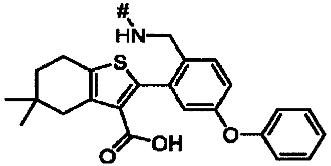 | 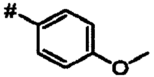 | H | |
| I-696 | 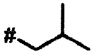 | H | 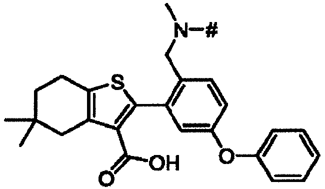 | 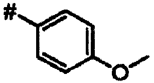 | H | |
| I-697 | 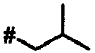 | H | 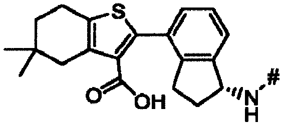 | 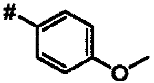 | H | |
| I-698 | 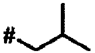 | H | 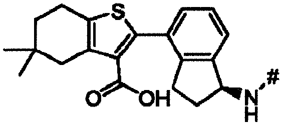 | 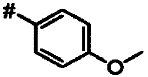 | H | |

**[Table 80]**

| No. | R1 | R2 | R3 | R4 | R5 | salt |
|---|---|---|---|---|---|---|
| I-699 | 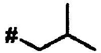 | H | 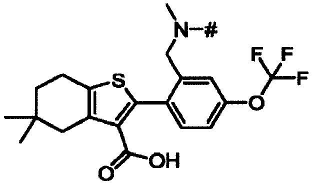 | 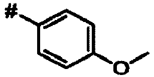 | H | |
| I-700 | 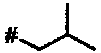 | H | 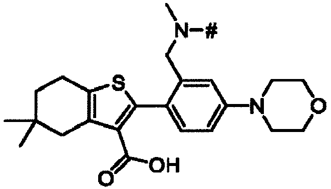 | 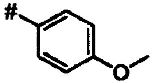 | H | |
| I-701 | 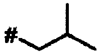 | H | 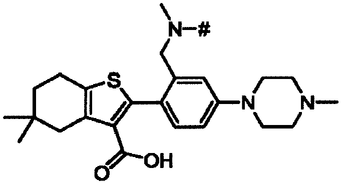 | 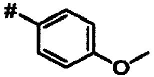 | H | |
| I-702 | 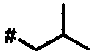 | H | 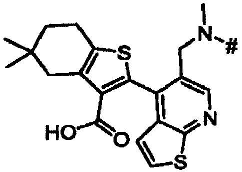 | 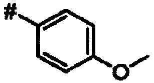 | H | |
| I-703 | 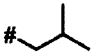 | H | 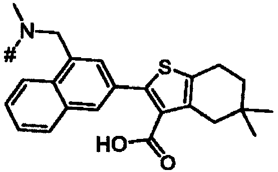 | 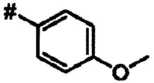 | H | |
| I-704 | 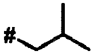 | H | 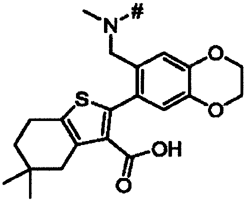 | 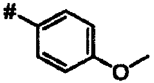 | H | |
| I-705 | 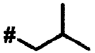 | H | 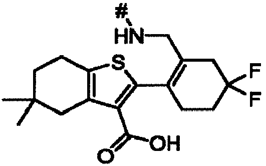 | 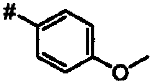 | H | |

**[Table 81]**

| No. | R1 | R2 | R3 | R4 | R5 | salt |
|---|---|---|---|---|---|---|
| I-706 | 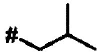 | H | 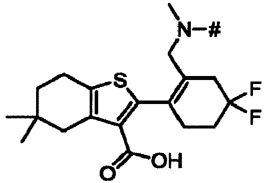 | 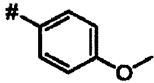 | H | |
| I-707 | 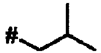 | H | 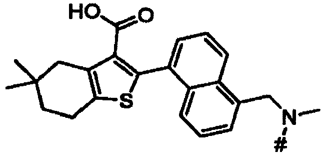 | 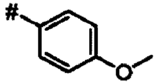 | H | |
| I-708 | 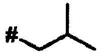 | H | 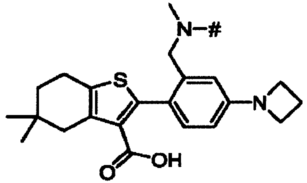 | 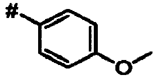 | H | |
| I-709 | 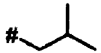 | H | 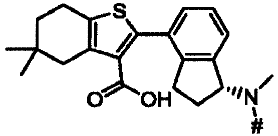 | 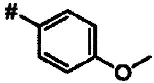 | H | |
| I-710 | 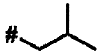 | H | 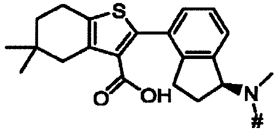 | 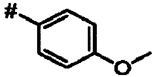 | H | |
| I-711 | 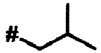 | H | 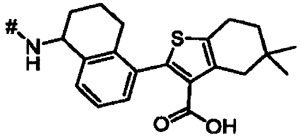 | 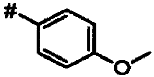 | H | |
| I-712 | 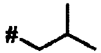 | H | 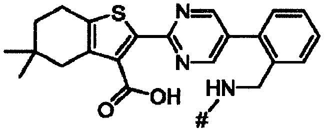 | 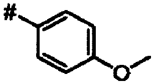 | H | |
| I-713 | 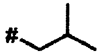 | H | 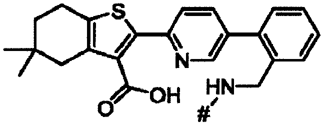 | 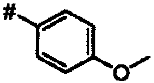 | H | |
| I-714 | 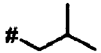 | H | 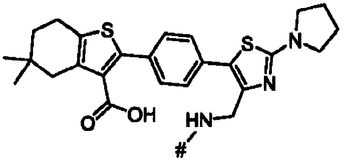 | 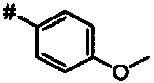 | H | |

**[Table 82]**

| No. | R1 | R2 | R3 | R4 | R5 | salt |
|---|---|---|---|---|---|---|
| I-715 | 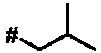 | H | 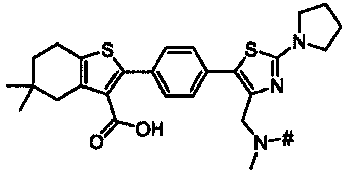 | 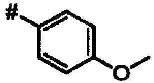 | H | |
| I-716 | 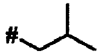 | H | 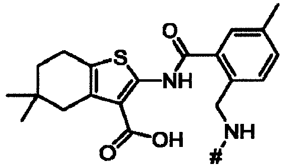 | 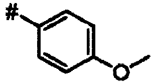 | H | |
| I-717 | 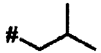 | H | 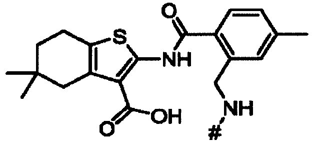 | 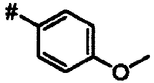 | H | |
| I-718 | 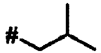 | H | 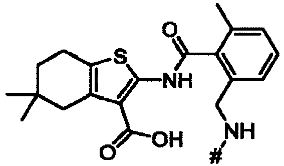 | 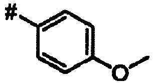 | H | |
| I-719 | 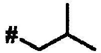 | H | 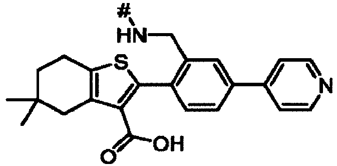 | 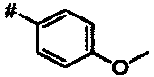 | H | |
| I-720 | 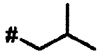 | H | 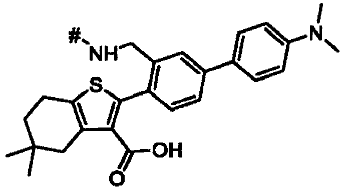 | 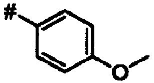 | H | |
| I-721 | 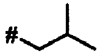 | H | 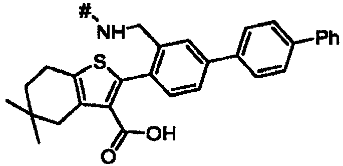 | 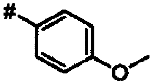 | H | |
| I-722 | 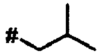 | H | 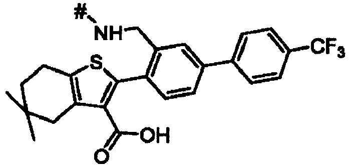 | 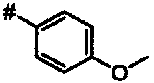 | H | |

**[Table 83]**

| No. | R1 | R2 | R3 | R4 | R5 | salt |
|---|---|---|---|---|---|---|
| I-723 | 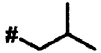 | H | 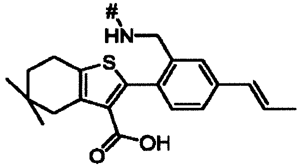 | 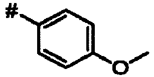 | H | |
| I-724 | 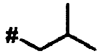 | H | 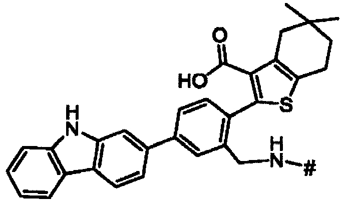 | 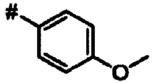 | H | |
| I-725 | 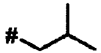 | H | 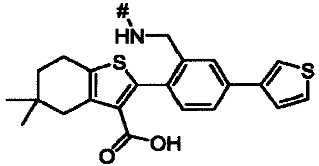 | 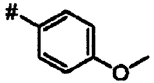 | H | |
| I-726 | 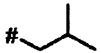 | H | 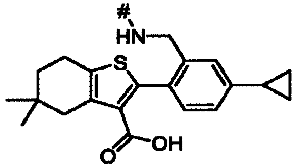 | 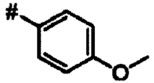 | H | |
| I-727 | 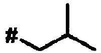 | H | 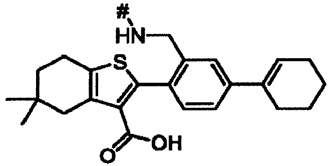 | 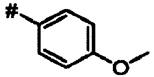 | H | |
| I-728 | 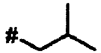 | H | 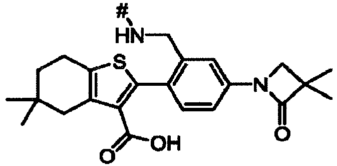 | 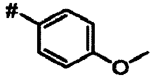 | H | |
| I-729 | 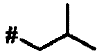 | H | 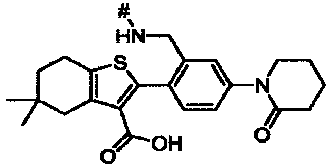 | 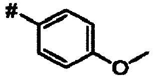 | H | |
| I-730 | 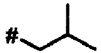 | H | 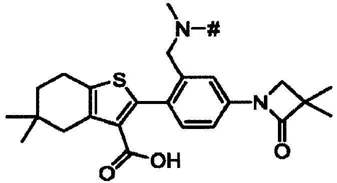 | 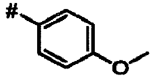 | H | |

**[Table 84]**

| No. | R1 | R2 | R3 | R4 | R5 | salt |
|---|---|---|---|---|---|---|
| I-731 | 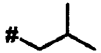 | H | 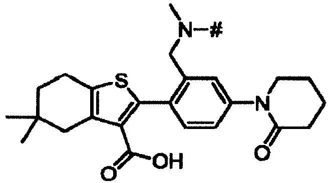 | 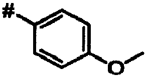 | H | |
| I-732 | 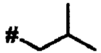 | H | 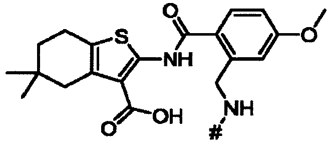 | 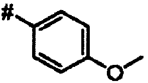 | H | |
| I-733 | 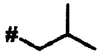 | H | 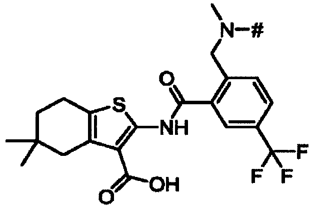 | 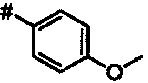 | H | |
| I-734 | 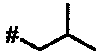 | H | 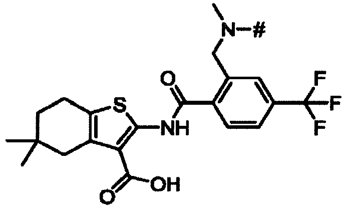 | 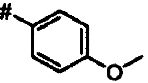 | H | |
| I-735 | 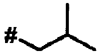 | H | 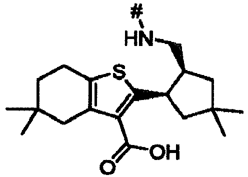 | 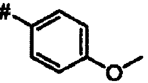 | H | |
| I-736 | 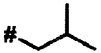 | H | 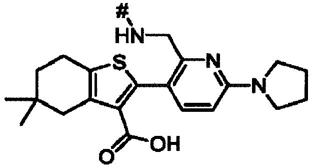 | 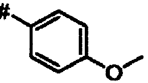 | H | |
| I-737 | 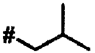 | H | 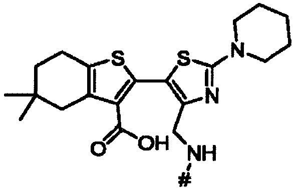 | 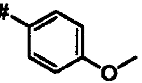 | H | |

**[Table 85]**

| No. | R1 | R2 | R3 | R4 | R5 | salt |
|---|---|---|---|---|---|---|
| I-738 | 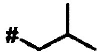 | H | 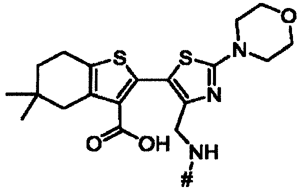 | 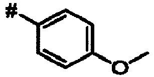 | H | |
| I-739 | 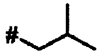 | H | 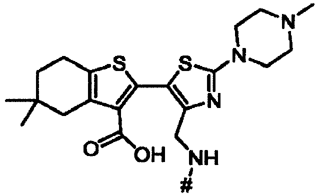 | 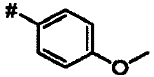 | H | |
| I-740 | 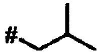 | H | 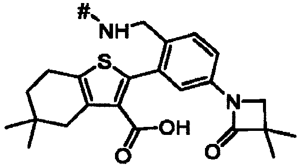 | 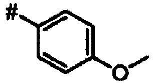 | H | |
| I-741 | 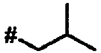 | H | 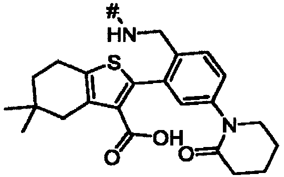 | 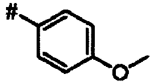 | H | |
| I-742 | 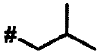 | H | 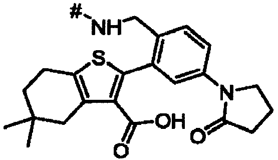 | 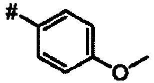 | H | |
| I-743 | 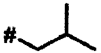 | H | 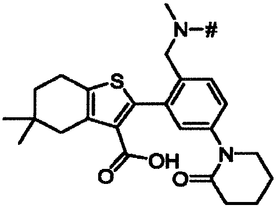 | 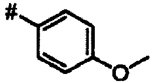 | H | |
| I-744 | 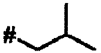 | H | 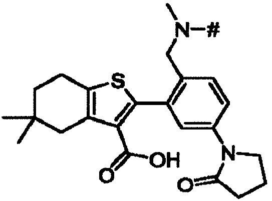 | 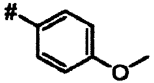 | H | |

**[Table 86]**

| No. | R1 | R2 | R3 | R4 | R5 | salt |
|---|---|---|---|---|---|---|
| I-745 | 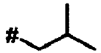 | H | 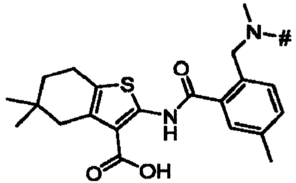 | 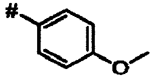 | H | |
| I-746 | 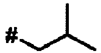 | H | 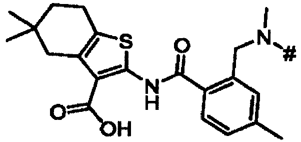 | 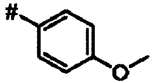 | H | |
| I-747 | 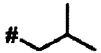 | H | 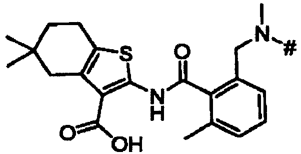 | 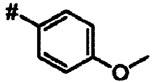 | H | |
| I-748 | 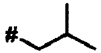 | H | 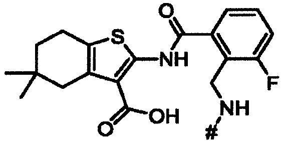 | 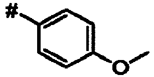 | H | |
| I-749 | 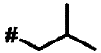 | H | 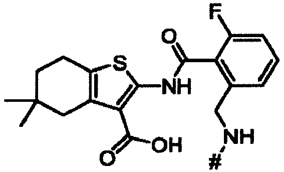 | 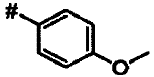 | H | |
| I-750 | 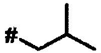 | H | 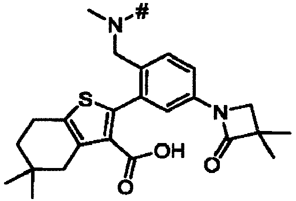 | 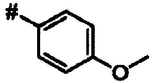 | H | |
| I-751 | 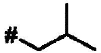 | H | 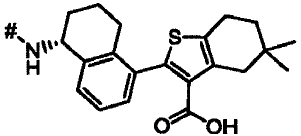 | 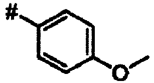 | H | |
| I-752 | 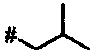 | H | 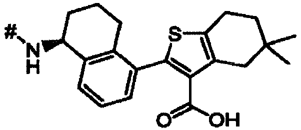 | 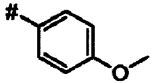 | H | |

**[Table 87]**

| No. | R1 | R2 | R3 | R4 | R5 | salt |
|---|---|---|---|---|---|---|
| I-753 | 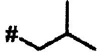 | H | 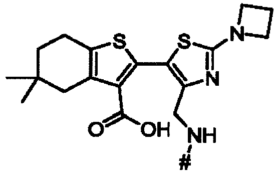 | 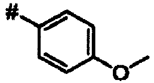 | H | |
| I-754 | 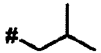 | H | 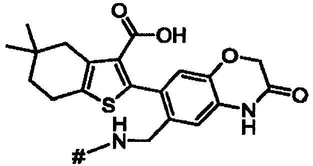 | 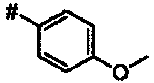 | H | |
| I-755 | 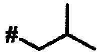 | H | 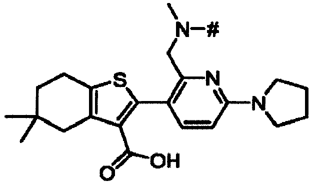 | 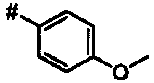 | H | |
| I-756 | 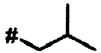 | H | 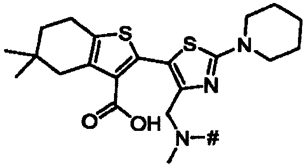 | 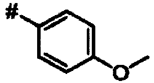 | H | |
| I-757 | 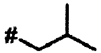 | H | 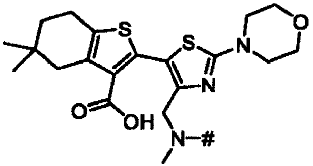 | 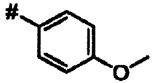 | H | |
| I-758 | 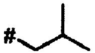 | H | 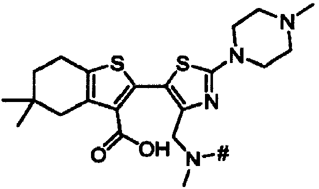 | 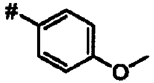 | H | |
| I-759 | 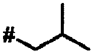 | H | 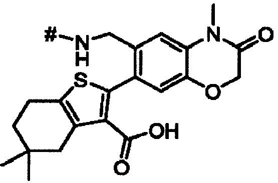 | 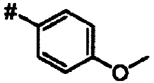 | H | |
| I-760 | 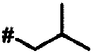 | H | 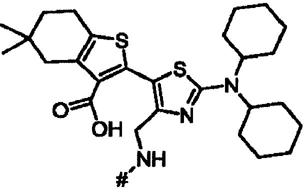 | 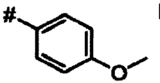 | H | |

**[Table 88]**

| No. | R1 | R2 | R3 | R4 | R5 | salt |
|---|---|---|---|---|---|---|
| I-761 | 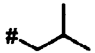 | H | 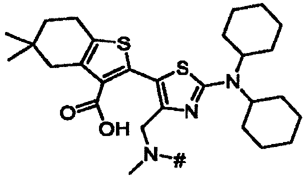 | 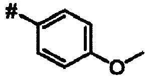 | H | |
| I-762 | 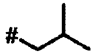 | H | 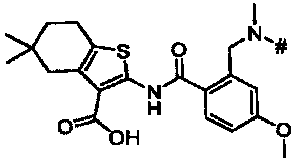 | 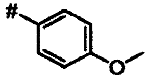 | H | |
| I-763 | 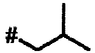 | H | 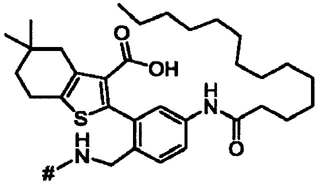 | 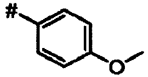 | H | |
| I-764 | 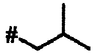 | H | 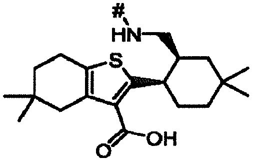 | 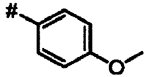 | H | |
| I-765 | 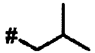 | H | 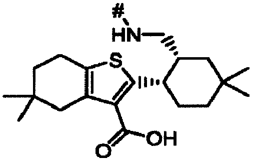 | 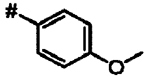 | H | |
| I-766 | 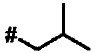 | H | 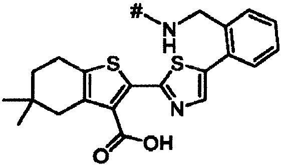 | 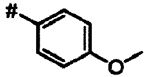 | H | |
| I-767 | 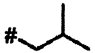 | H | 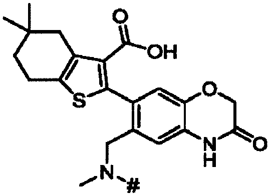 | 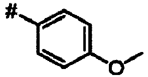 | H | |
| I-768 | 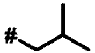 | H | 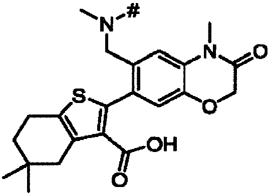 | 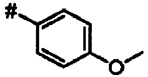 | H | |

**[Table 89]**

| No. | R1 | R2 | R3 | R4 | R5 | salt |
|---|---|---|---|---|---|---|
| I-769 | 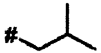 | H | 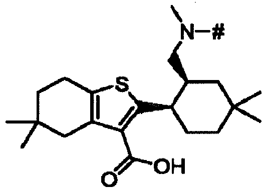 | 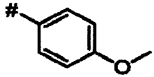 | H | |
| I-770 | 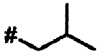 | H | 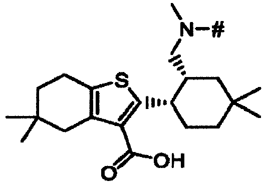 | 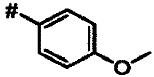 | H | |
| I-771 | 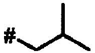 | H | 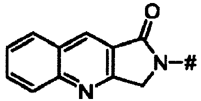 | 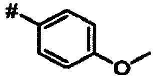 | H | |
| I-772 | 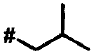 | H | 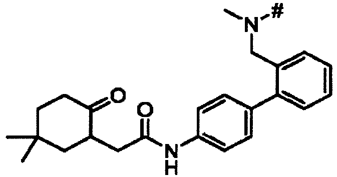 | 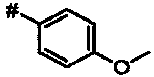 | H | |
| I-773 | 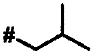 | H | 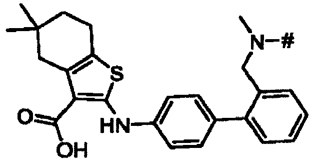 | 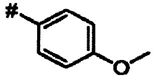 | H | |
| I-774 | 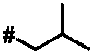 | H | 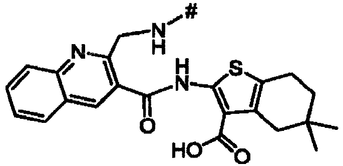 | 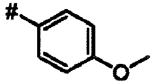 | H | |
| I-775 | 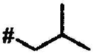 | H | 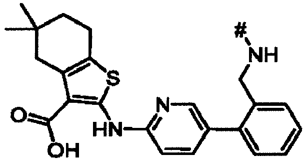 | 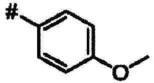 | H | |
| I-776 | 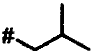 | H | 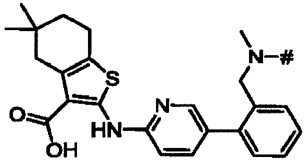 | 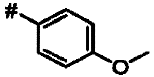 | H | |

**[Table 90]**

| No. | R1 | R2 | R3 | R4 | R5 | salt |
|---|---|---|---|---|---|---|
| I-777 | 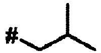 | H | 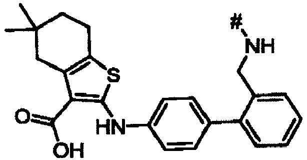 | 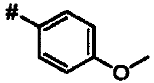 | H | |
| I-778 | 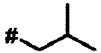 | H | 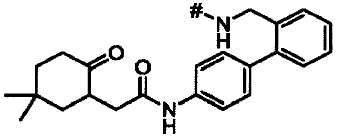 | 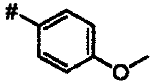 | H | |
| I-779 | 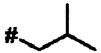 | H | 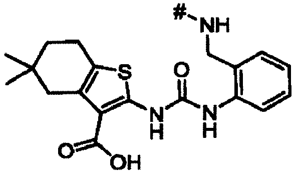 | 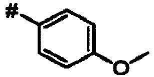 | H | |

**[Table 91]**

| No. | R1 | R2 | R3 | R4 | R5 |
|---|---|---|---|---|---|
| I-780 | 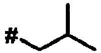 | H | H | 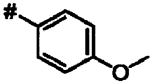 | 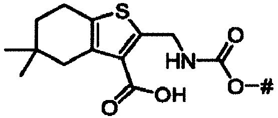 |
| I-781 | 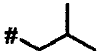 | H | H | 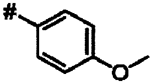 | 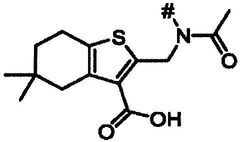 |
| I-782 | 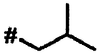 | H | H | 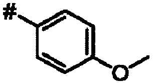 | 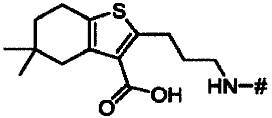 |
| I-783 | 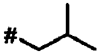 | H | H | 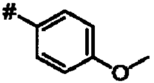 | 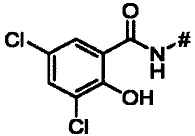 |

**[Table 92]**

| No. | R1 | R2 | R3 | R4 | R5 |
|---|---|---|---|---|---|
| I-784 | 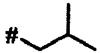 | H | 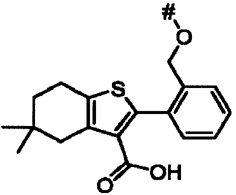 | 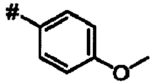 | 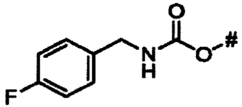 |
| I-785 | 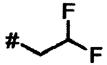 | H | H | 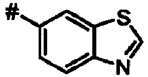 | 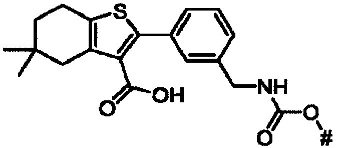 |
| I-786 | 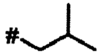 | H | H | 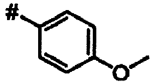 | 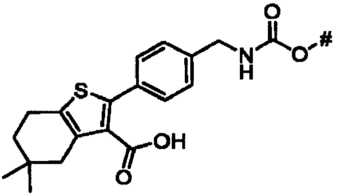 |
| I-787 | 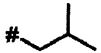 | H | H | 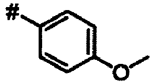 | 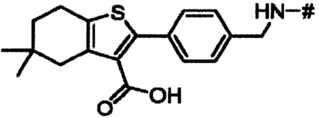 |
| I-788 | 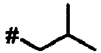 | H | H | 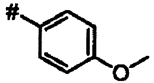 | 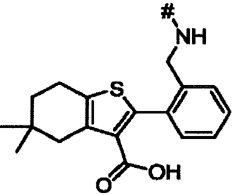 |
| I-789 | 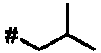 | H | 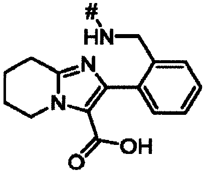 | 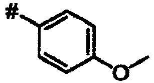 | 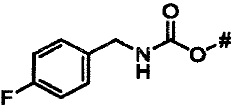 |
| I-790 | 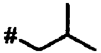 | H | 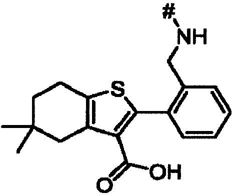 | 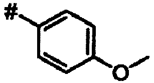 | 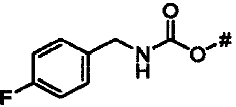 |

**[Table 93]**

| No. | R1 | R2 | R3 | R4 | R5 |
|---|---|---|---|---|---|
| I-791 | 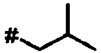 | H | 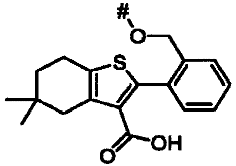 | 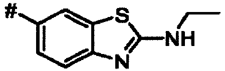 | H |
| I-792 | 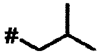 | H | 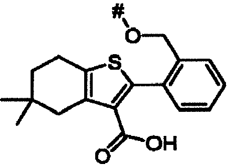 | 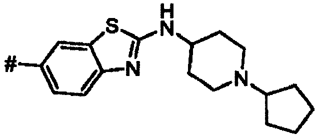 | H |
| I-793 | 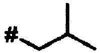 | H | 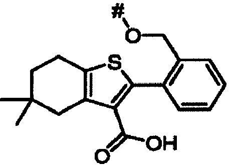 | 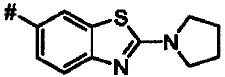 | H |
| I-794 | 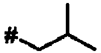 | H | 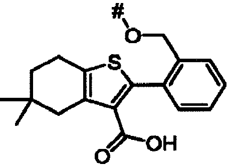 | 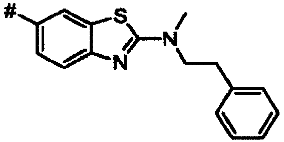 | H |
| I-795 | 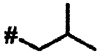 | H | 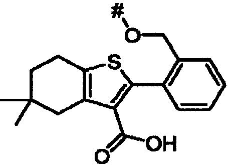 | 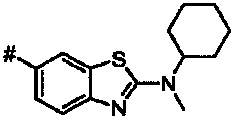 | H |
| I-796 | 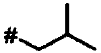 | H | H | 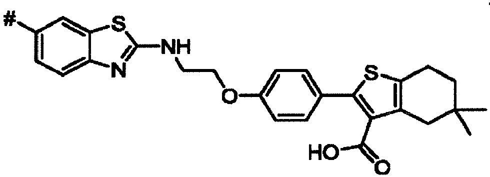 | H |
| I-797 | 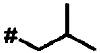 | H | H | 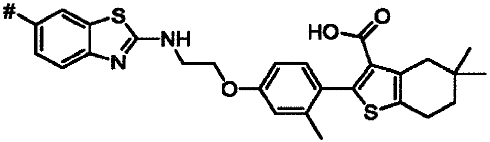 | H |
| I-798 | 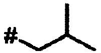 | H | H | 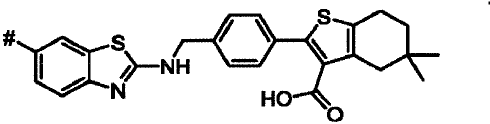 | H |

**[Table 94]**

| No. | R1 | R2 | R3 | R4 | R5 |
|---|---|---|---|---|---|
| I-799 | 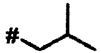 | H | H | 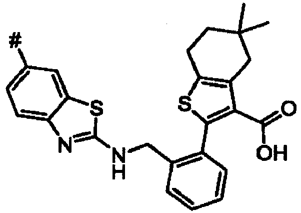 | H |
| I-800 | 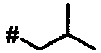 | H | H | 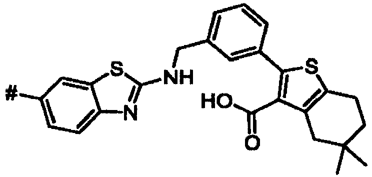 | H |
| I-801 | 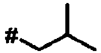 | H | H | 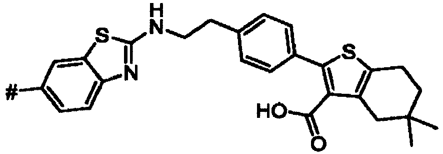 | H |
| I-802 | 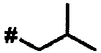 | H | H | 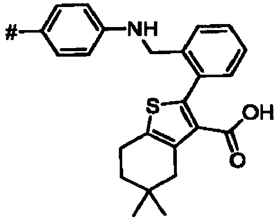 | H |
| I-803 | 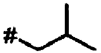 | H | H | 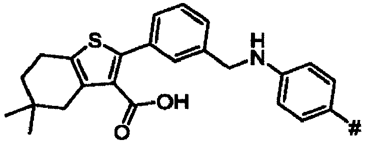 | H |
| I-804 | 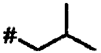 | H | H | 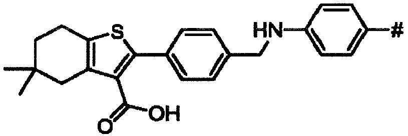 | H |
| I-805 | 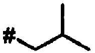 | H | H | 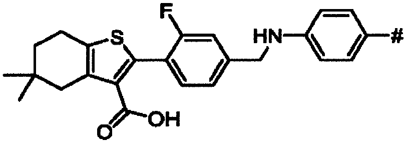 | H |

**[Table 95]**

| | | | | |
|---|---|---|---|---|
| 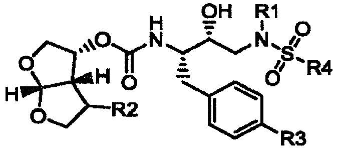 | | | | |

| No. | R1 | R2 | R3 | R4 |
|---|---|---|---|---|
| I-806 | 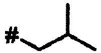 | H | 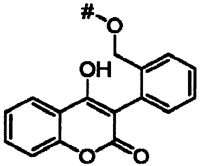 | 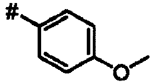 |
| I-807 | 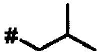 | H | 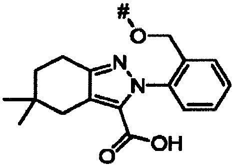 | 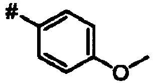 |
| I-808 | 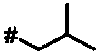 | 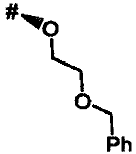 | 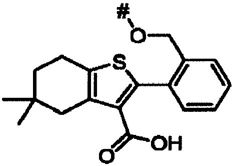 | 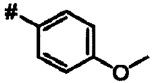 |
| I-809 | 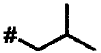 | 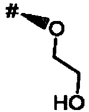 | 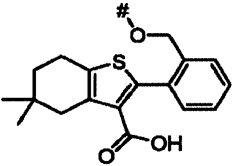 | 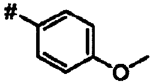 |
| I-810 | 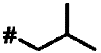 | H | 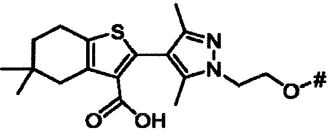 | 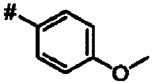 |
| I-811 | 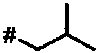 | H | 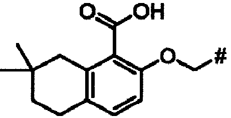 | 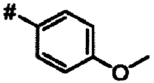 |
| I-812 | 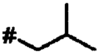 | H | 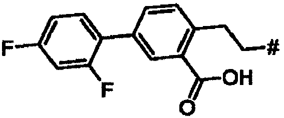 | 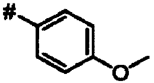 |
| I-813 | 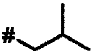 | H | 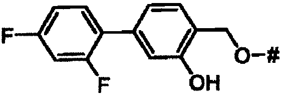 | 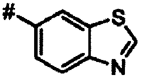 |
| I-814 | 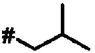 | H | 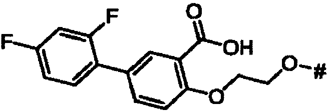 | 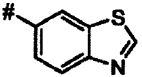 |

**[Table 96]**

| No. | R1 | R2 | R3 | R4 |
|---|---|---|---|---|
| I-815 | 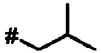 | H | 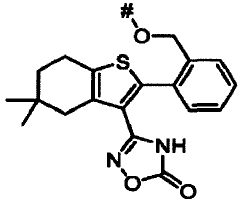 | 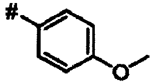 |
| I-816 | 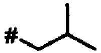 | H | H | 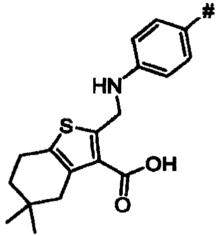 |
| I-817 | 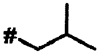 | H | 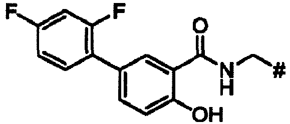 | 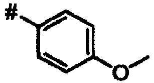 |
| I-818 | 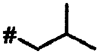 | H | 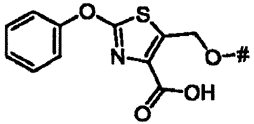 | 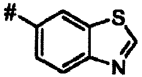 |
| I-819 | 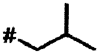 | H | 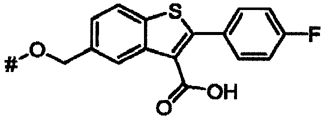 | 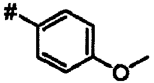 |
| I-820 | 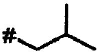 | H | 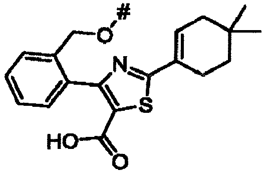 | 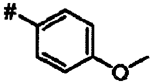 |
| I-821 | 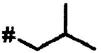 | H | 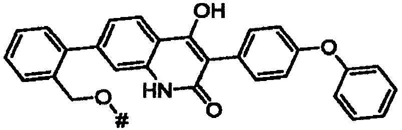 | 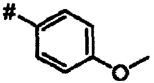 |
| I-822 | 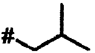 | H | 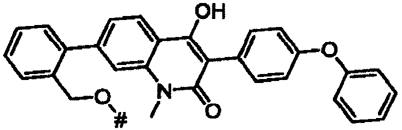 | 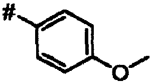 |
| I-823 | 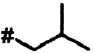 | H | 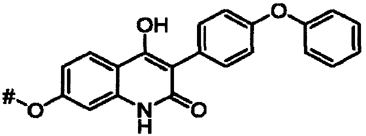 | 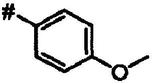 |

**[Table 97]**

| No. | R1 | R2 | R3 | R4 |
|---|---|---|---|---|
| I-824 | 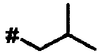 | H | 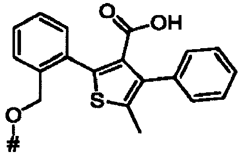 | 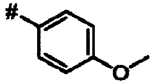 |
| I-825 | 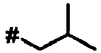 | H | 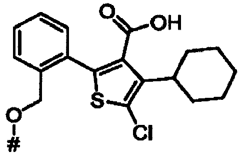 | 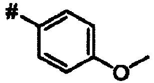 |
| I-826 | 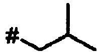 | H | 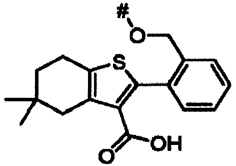 | 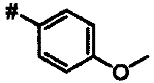 |
| I-827 | 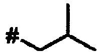 | H | 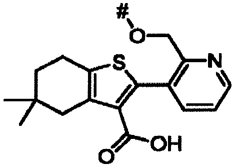 | 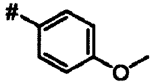 |
| I-828 | 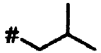 | H | 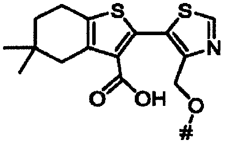 | 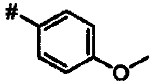 |
| I-829 | 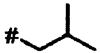 | H | 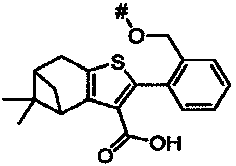 | 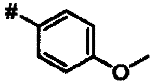 |
| I-830 | 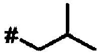 | H | 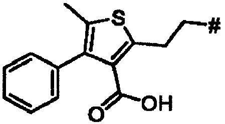 | 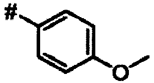 |
| I-831 | 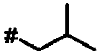 | H | 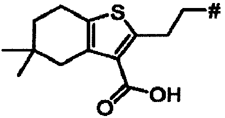 | 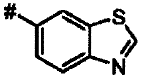 |
| I-832 | 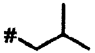 | H | 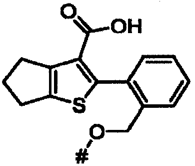 | 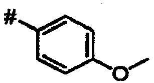 |

**[Table 98]**

| No. | R1 | R2 | R3 | R4 |
|---|---|---|---|---|
| I-833 | 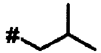 | H | 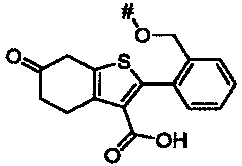 | 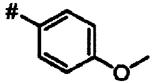 |
| I-834 | 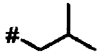 | H | 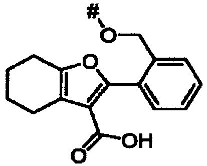 | 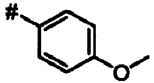 |
| I-835 | 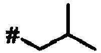 | H | 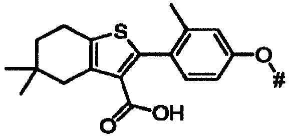 | 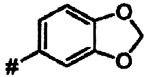 |
| I-836 | 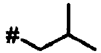 | H | 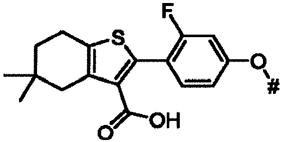 | 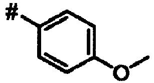 |
| I-837 | 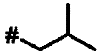 | H | 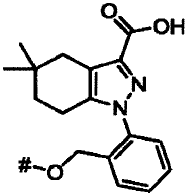 | 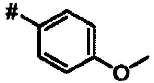 |
| I-838 | 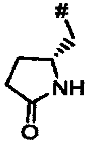 | H | 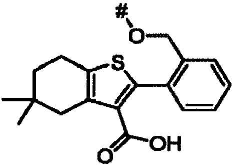 | 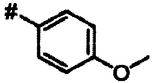 |
| I-839 | 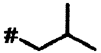 | H | 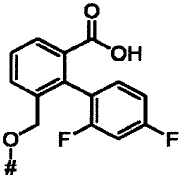 | 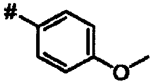 |
| I-840 | 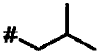 | H | 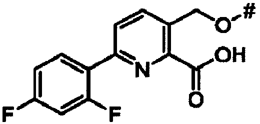 | 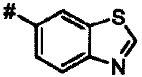 |

**[Table 99]**

| No. | R1 | R2 | R3 | R4 |
|---|---|---|---|---|
| I-841 | 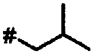 | H | 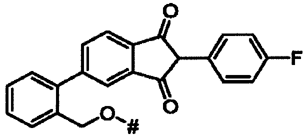 | 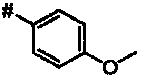 |
| I-842 | 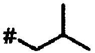 | H | 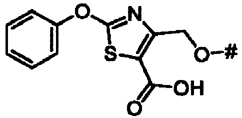 | 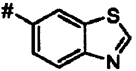 |
| I-843 | 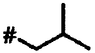 | H | 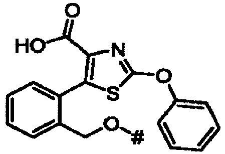 | 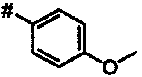 |
| I-844 | 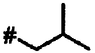 | H | 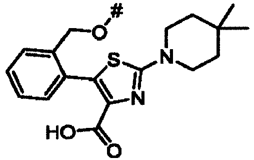 | 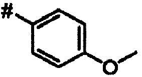 |
| I-845 | 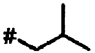 | H | 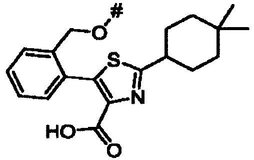 | 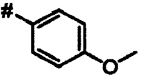 |
| I-846 | 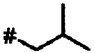 | H | 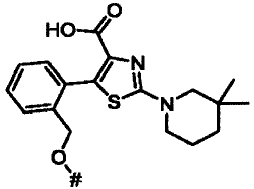 | 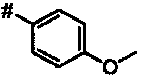 |
| I-847 | 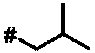 | H | 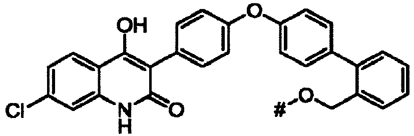 | 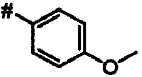 |
| I-848 | 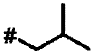 | H | 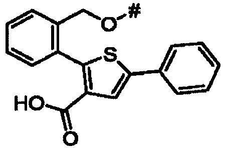 | 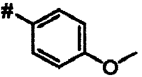 |
| I-849 | 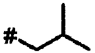 | H | 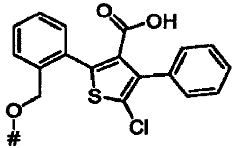 | 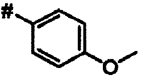 |

**[Table 100]**

| No. | R1 | R2 | R3 | R4 |
|---|---|---|---|---|
| I-850 | 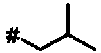 | H | 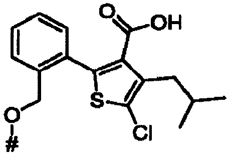 | 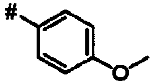 |
| I-851 | 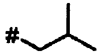 | H | 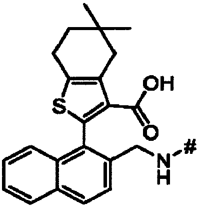 | 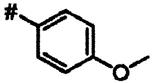 |
| I-852 | 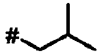 | H | 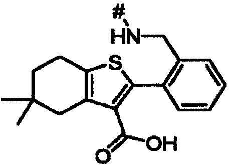 | 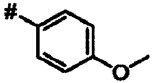 |
| I-853 | 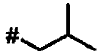 | 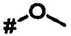 | 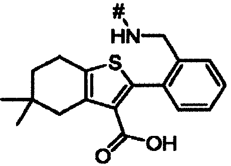 | 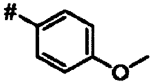 |
| I-854 | 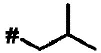 | H | 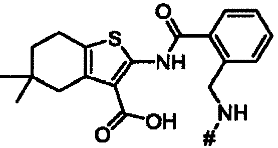 | 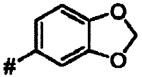 |
| I-855 | 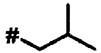 | H | 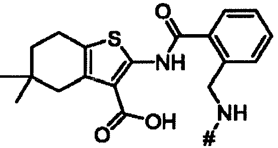 | 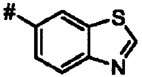 |
| I-856 | 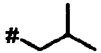 | H | 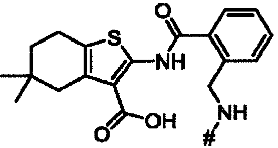 | 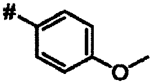 |
| I-857 | 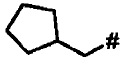 | H | 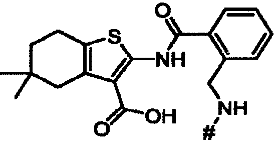 | 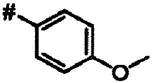 |

**[Table 101]**

| No. | R1 | R2 | R3 | R4 |
|---|---|---|---|---|
| I-858 | 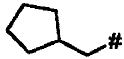 | H | 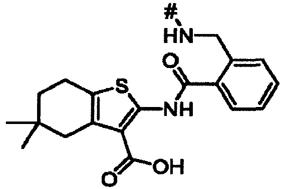 | 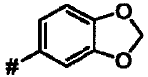 |
| I-859 | 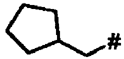 | H | 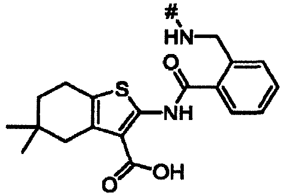 | 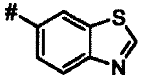 |
| I-860 | 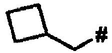 | H | 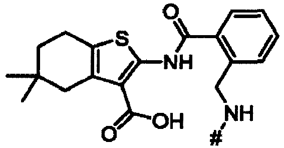 | 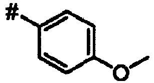 |
| I-861 | 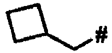 | H | 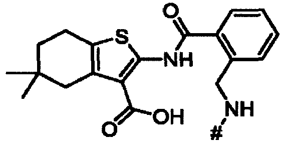 | 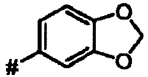 |
| I-862 | 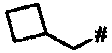 | H | 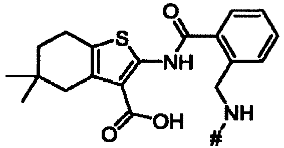 | 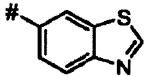 |
| I-863 | 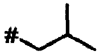 | H | 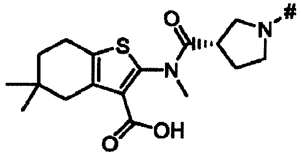 | 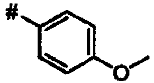 |
| I-864 | 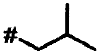 | H | 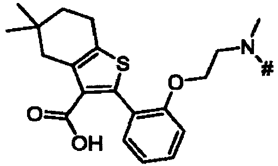 | 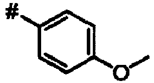 |
| I-865 | 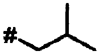 | H | 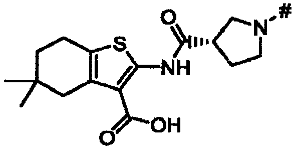 | 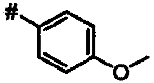 |

**[Table 102]**

| No. | R1 | R2 | R3 | R4 |
|---|---|---|---|---|
| I-866 | 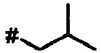 | H | 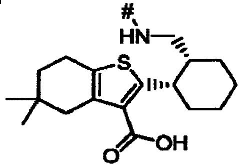 | 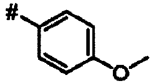 |
| I-867 | 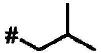 | H | 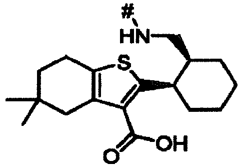 | 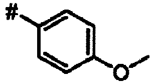 |
| I-868 | 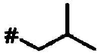 | H | 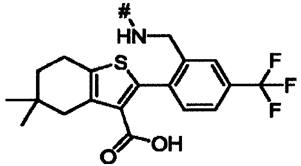 | 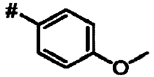 |
| I-869 | 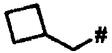 | H | 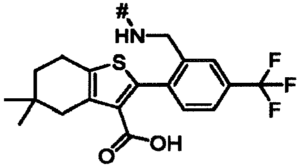 | 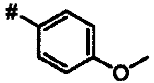 |
| I-870 | 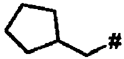 | H | 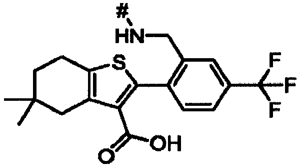 | 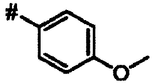 |
| I-871 | 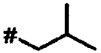 | H | 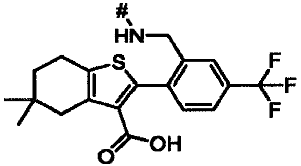 | 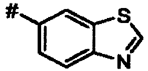 |
| I-872 | 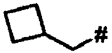 | H | 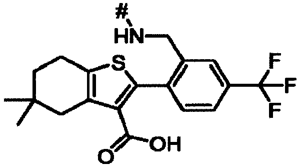 | 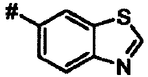 |
| I-873 | 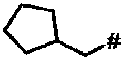 | H | 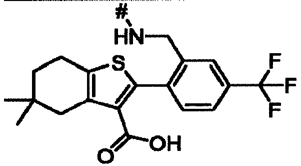 | 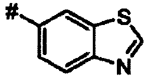 |

**[Table 103]**

| No. | R1 | R2 | R3 | R4 |
|---|---|---|---|---|
| I-874 | 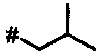 | H | 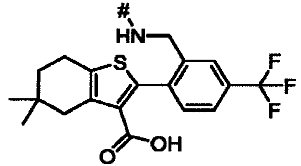 | 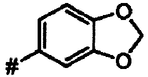 |
| I-875 | 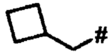 | H | 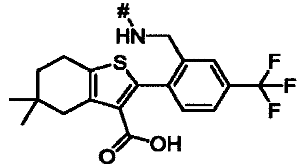 | 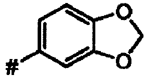 |
| I-876 | 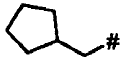 | H | 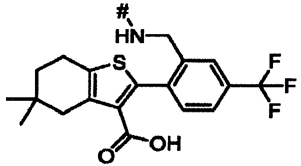 | 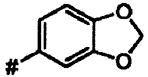 |
| I-877 | 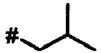 | H | 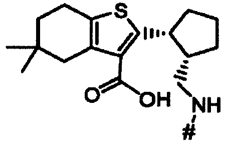 | 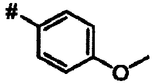 |
| I-878 | 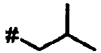 | H | 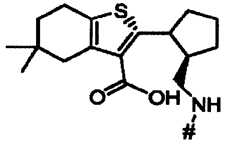 | 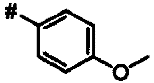 |
| I-879 | 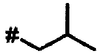 | H | 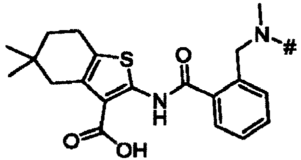 | 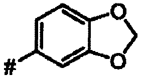 |
| I-880 | 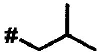 | H | 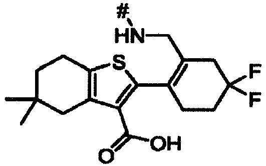 | 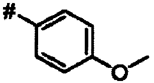 |
| I-881 | 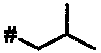 | H | 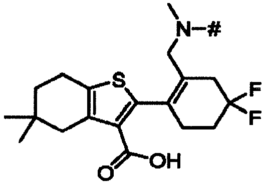 | 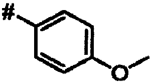 |

**[Table 104]**

| No. | R1 | R2 | R3 | R4 |
|---|---|---|---|---|
| I-882 | 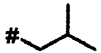 | H | 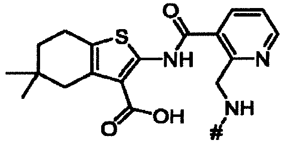 | 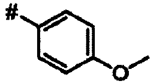 |
| I-883 | 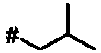 | H | 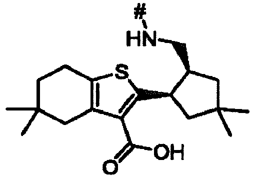 | 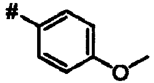 |
| I-884 | 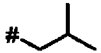 | H | 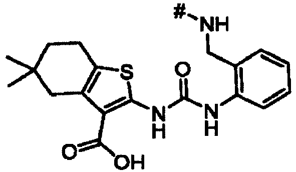 | 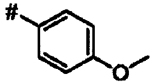 |
| I-885 | 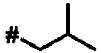 | H | 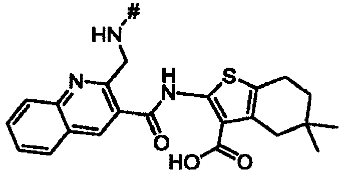 | 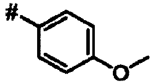 |
| I-886 | 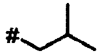 | H | 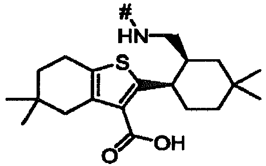 | 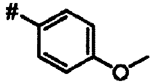 |
| I-887 | 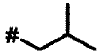 | H | 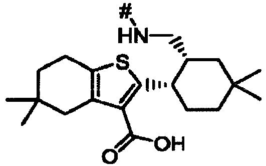 | 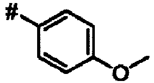 |
| I-888 | 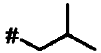 | H | 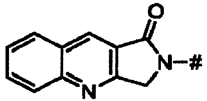 | 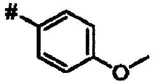 |

**[Table 105]**

| | | | |
|---|---|---|---|
| 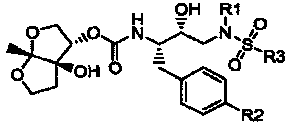 | | | |

| No. | R1 | R2 | R3 |
|---|---|---|---|
| I-889 | 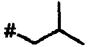 | 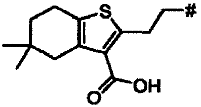 | 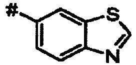 |
| I-890 | 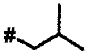 | 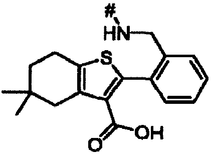 | 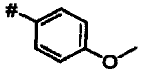 |
| I-891 | 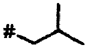 | 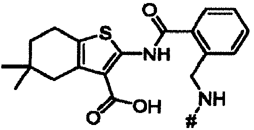 | 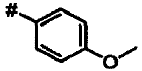 |
| I-892 | 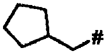 | 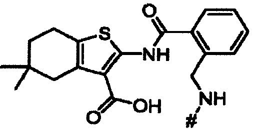 | 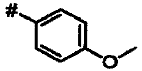 |
| I-893 | 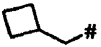 | 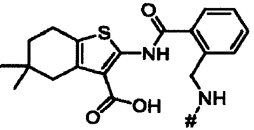 | 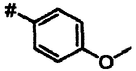 |
| I-894 | 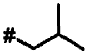 | 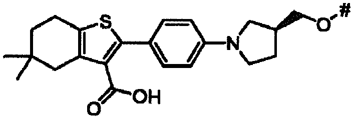 | 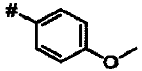 |
| I-895 | 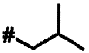 | 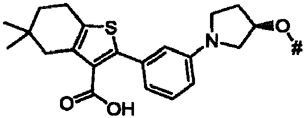 | 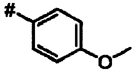 |
| I-896 | 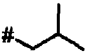 | 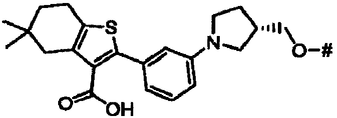 | 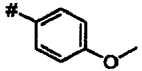 |

**[Table 106]**

| | | | |
|---|---|---|---|
| 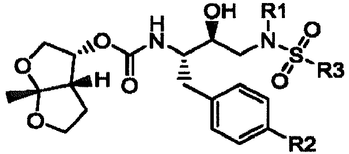 | | | |

| No. | R1 | R2 | R3 |
|---|---|---|---|
| I-897 | 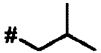 | 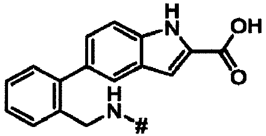 | 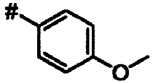 |
| I-898 | 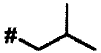 | 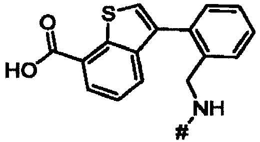 | 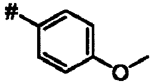 |
| I-899 | 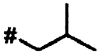 | 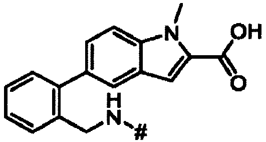 | 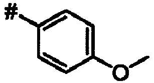 |

**[Table 107]**

| | | | |
|---|---|---|---|
| 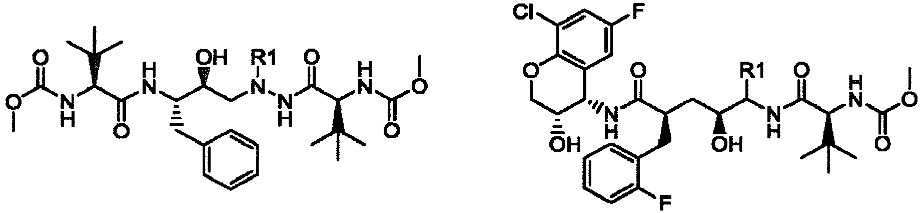 | | | |

| No. | R1 | No. | R1 |
|---|---|---|---|
| I-900 | 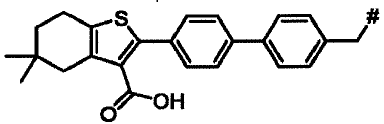 | I-902 | 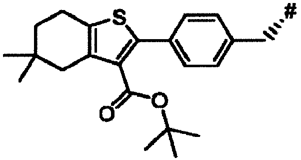 |
| I-901 | 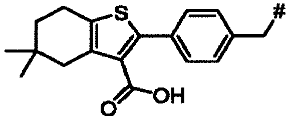 | I-903 | 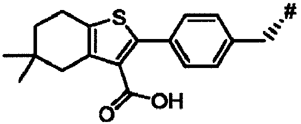 |
| | | I-904 | 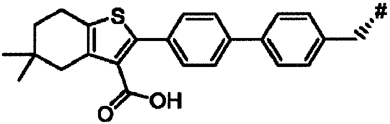 |

**[Table 108]**

| No. | Structure | No. | Structure |
|---|---|---|---|
| I-905 | 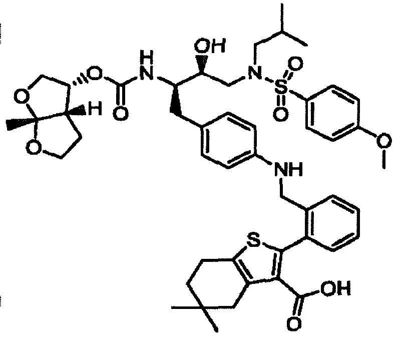 | I-908 | 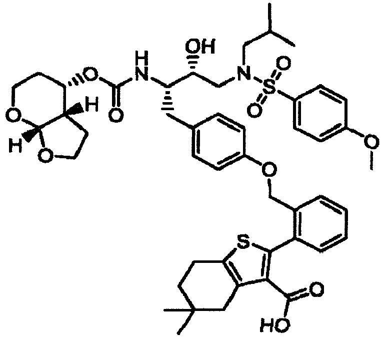 |
| I-906 | 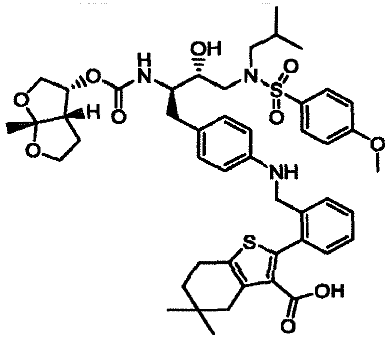 | I-909 | 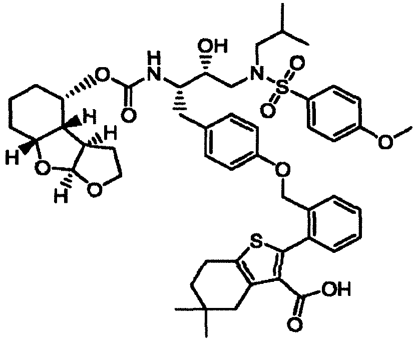 |
| I-907 | 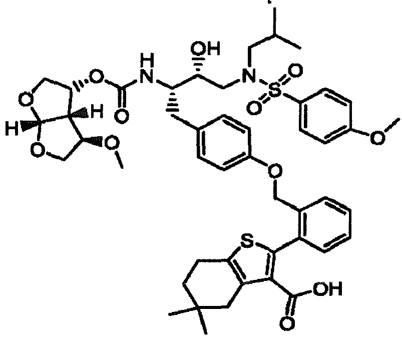 | I-910 | 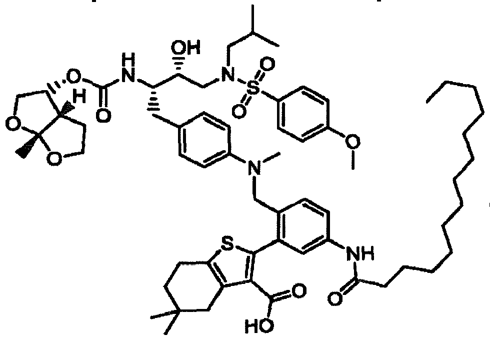 |

**[Table 109]**

| No. | method | RT | MS | | No. | method | RT | MS | | No. | method | RT | MS | |
|---|---|---|---|---|---|---|---|---|---|---|---|---|---|---|
| I-37 | A | 3.14 | 909 | M+18 | I-96 | A | 3.13 | 953 | M+18 | I-155 | B | 2.33 | 847 | M+23 |
| I-38 | A | 2.65 | 900 | M+1 | I-97 | A | 2.41 | 810 | M+1 | I-156 | A | 2.20 | 868 | M+1 |
| I-39 | A | 3.13 | 923 | M+18 | I-98 | A | 3.11 | 934 | M+18 | I-157 | A | 2.12 | 868 | M+1 |
| I-40 | B | 2.73 | 883 | M-1 | I-99 | A | 2.85 | 908 | M+1 | I-158 | A | 2.27 | 805 | M+1 |
| I-41 | A | 2.71 | 785 | M+1 | I-100 | A | 3.02 | 960 | M+1 | I-159 | A | 2.30 | 830 | M+1 |
| I-42 | A | 3.59 | 986 | M+1 | I-101 | A | 3.14 | 946 | M+1 | I-160 | A | 2.34 | 858 | M+1 |
| I-43 | A | 2.73 | 799 | M+1 | I-102 | A | 3.14 | 960 | M+1 | I-161 | A | 2.45 | 858 | M+1 |
| I-44 | A | 2.86 | 968 | M+1 | I-103 | A | 3.19 | 960 | M+1 | I-162 | A | 2.10 | 804 | M+1 |
| I-45 | A | 2.86 | 890 | M+1 | I-104 | A | 3.11 | 932 | M+1 | I-163 | A | 2.30 | 832 | M+1 |
| I-46 | A | 2.75 | 828 | M+1 | I-105 | A | 3.15 | 841 | M+1 | I-164 | A | 2.79 | 874 | M+1 |
| I-47 | A | 3.01 | 904 | M+1 | I-106 | A | 2.91 | 828 | M+1 | I-165 | A | 2.77 | 876 | M+1 |
| I-48 | A | 3.05 | 904 | M+1 | I-107 | A | 2.79 | 840 | M+1 | I-166 | A | 2.89 | 890 | M+1 |
| I-49 | B | 2.71 | 873 | M-1 | I-108 | A | 2.15 | 703 | M+1 | I-167 | B | 2.36 | 845 | M-1 |
| I-50 | B | 2.48 | 858 | M-1 | I-109 | A | 2.12 | 704 | M+1 | I-168 | A | 2.64 | 836 | M-1 |
| I-51 | B | 2.57 | 931 | M-1 | I-110 | A | 2.81 | 968 | M+1 | I-169 | A | 2.52 | 788 | M-1 |
| I-52 | B | 2.63 | 875 | M-1 | I-111 | A | 3.07 | 974 | M+1 | I-170 | A | 2.44 | 788 | M-1 |
| I-53 | B | 2.63 | 901 | M+1 | I-112 | A | 2.97 | 931 | M-1 | I-171 | A | 2.67 | 784 | M+1 |
| I-54 | A | 2.68 | 897 | M-1 | I-113 | A | 3.29 | 919 | M+1 | I-172 | A | 2.64 | 868 | M+1 |
| I-55 | A | 3.14 | 1014 | M+18 | I-114 | A | 2.68 | 866 | M+18 | I-173 | B | 2.57 | 793 | M-1 |
| I-56 | A | 2.76 | 929 | M+23 | I-115 | A | 2.96 | 894 | M+18 | I-174 | B | 2.67 | 933 | M-1 |
| I-57 | B | 2.55 | 928 | M-1 | I-116 | B | 2.93 | 908 | M+18 | I-175 | B | 2.62 | 947 | M-1 |
| I-58 | B | 2.77 | 994 | M+1 | I-117 | B | 2.37 | 894 | M+18 | I-176 | B | 2.72 | 915 | M-1 |
| I-59 | B | 2.34 | 850 | M-1 | I-118 | B | 2.51 | 938 | M+18 | I-177 | B | 2.77 | 918 | M+1 |
| I-60 | B | 2.32 | 865 | M-1 | I-119 | A | 3.08 | 945 | M-1 | I-178 | A | 3.28 | 968 | M+1 |
| I-61 | B | 2.41 | 865 | M-1 | I-120 | A | 2.72 | 922 | M+18 | I-179 | A | 2.67 | 859 | M+1 |
| I-62 | B | 2.28 | 865 | M+1 | I-121 | A | 2.51 | 938 | M+18 | I-180 | A | 2.74 | 1011 | M+1 |
| I-63 | B | 2.46 | 837 | M-1 | I-122 | A | 3.12 | 915 | M+18 | I-181 | A | 3.19 | 958 | M+18 |
| I-64 | A | 2.34 | 831 | M+1 | I-123 | A | 2.42 | 925 | M+18 | I-182 | A | 2.89 | 910 | M+1 |
| I-65 | A | 2.51 | 831 | M+1 | I-124 | A | 2.71 | 916 | M+18 | I-183 | A | 3.01 | 861 | M+1 |
| I-66 | A | 2.16 | 830 | M+1 | I-125 | A | 1.75 | 847 | M+1 | I-184 | A | 2.20 | 884 | M+1 |
| I-67 | A | 2.06 | 855 | M+23 | I-126 | A | 2.49 | 827 | M+18 | I-185 | A | 2.92 | 957 | M+1 |
| I-68 | A | 2.69 | 848 | M+23 | I-127 | A | 2.68 | 877 | M+18 | I-186 | A | 2.96 | 919 | M-1 |
| I-69 | A | 2.82 | 884 | M-1 | I-128 | A | 2.41 | 891 | M+1 | I-187 | A | 2.58 | 893 | M+1 |
| I-70 | B | 2.70 | 919 | M-1 | I-129 | A | 2.62 | 905 | M+1 | I-188 | A | 2.64 | 921 | M+18 |
| I-71 | B | 2.86 | 969 | M-1 | I-130 | B | 2.57 | 845 | M-1 | I-189 | A | 2.81 | 930 | M+1 |
| I-72 | B | 2.90 | 907 | M-1 | I-131 | A | 2.80 | 933 | M-1 | I-190 | A | 3.06 | 928 | M-1 |
| I-73 | A | 3.16 | 983 | M+1 | I-132 | A | 2.96 | 905 | M+1 | I-191 | A | 2.99 | 916 | M+1 |
| I-74 | A | 3.37 | 990 | M+1 | I-133 | A | 3.02 | 877 | M+1 | I-193 | A | 2.91 | 861 | M+1 |
| I-75 | A | 2.83 | 1013 | M+1 | I-134 | A | 3.17 | 889 | M-1 | I-194 | A | 2.22 | 898 | M+1 |
| I-76 | A | 3.00 | 1004 | M+1 | I-135 | A | 3.19 | 944 | M-1 | I-195 | A | 2.88 | 969 | M+1 |
| I-77 | A | 3.00 | 889 | M+18 | I-136 | A | 3.01 | 905 | M-1 | I-196 | A | 3.17 | 958 | M+18 |
| I-78 | A | 2.95 | 909 | M+18 | I-137 | A | 3.15 | 922 | M+18 | I-197 | A | 2.26 | 886 | M+1 |
| I-79 | A | 2.89 | 916 | M+1 | I-138 | A | 3.06 | 918 | M+1 | I-198 | A | 2.20 | 898 | M+1 |
| I-80 | A | 2.96 | 933 | M+18 | I-139 | B | 2.62 | 901 | M+1 | I-199 | A | 2.73 | 921 | M+1 |
| I-81 | A | 2.93 | 894 | M+18 | I-140 | A | 2.66 | 941 | M+1 | I-200 | A | 3.02 | 971 | M+1 |
| I-82 | A | 3.18 | 976 | M+18 | I-141 | A | 2.63 | 941 | M+1 | I-201 | A | 3.08 | 985 | M+1 |
| I-83 | A | 3.01 | 938 | M+18 | I-142 | A | 2.58 | 915 | M+1 | I-202 | A | 3.19 | 865 | M+1 |
| I-84 | A | 3.20 | 959 | M+18 | I-143 | A | 2.55 | 881 | M+1 | I-203 | A | 3.01 | 883 | M+1 |
| I-85 | A | 2.86 | 864 | M+1 | I-144 | A | 2.70 | 924 | M+1 | I-204 | A | 3.17 | 960 | M+1 |
| I-86 | A | 2.97 | 926 | M+18 | I-145 | A | 2.97 | 929 | M+1 | I-205 | A | 2.13 | 941 | M+1 |
| I-87 | A | 2.87 | 898 | M+1 | I-146 | B | 2.73 | 927 | M+1 | I-206 | A | 3.07 | 934 | M+1 |
| I-88 | A | 2.64 | 893 | M+1 | I-147 | A | 2.70 | 926 | M+1 | I-207 | A | 2.37 | 960 | M+1 |
| I-89 | A | 2.75 | 898 | M+1 | I-148 | A | 1.64 | 932 | M+1 | I-208 | A | 2.64 | 828 | M+1 |
| I-90 | A | 2.63 | 892 | M+1 | I-149 | B | 2.31 | 837 | M-1 | I-209 | A | 3.05 | 945 | M+1 |
| I-91 | A | 3.19 | 905 | M+1 | I-150 | B | 2.24 | 864 | M-1 | I-210 | A | 2.94 | 1034 | M+1 |
| I-92 | A | 3.17 | 927 | M+23 | I-151 | B | 2.33 | 866 | M+1 | I-211 | A | 3.32 | 981 | M+1 |
| I-93 | A | 3.00 | 926 | M+1 | I-152 | B | 2.37 | 837 | M-1 | I-212 | A | 2.95 | 966 | M+1 |
| I-94 | A | 3.60 | 1023 | M+23 | I-153 | B | 2.41 | 837 | M-1 | I-213 | A | 2.83 | 966 | M+1 |
| I-95 | A | 3.07 | 960 | M+1 | I-154 | B | 2.31 | 864 | M-1 | I-214 | A | 2.81 | 934 | M+1 |

**[Table 110]**

| No. | method | RT | MS | | No. | method | RT | MS | | No. | method | RT | MS | |
|---|---|---|---|---|---|---|---|---|---|---|---|---|---|---|
| I-215 | A | 2.99 | 916 | M+1 | I-278 | A | 2.84 | 921 | M+1 | I-337 | A | 2.69 | 899 | M+18 |
| I-216 | A | 2.86 | 915 | M+1 | I-279 | A | 2.99 | 926 | M+1 | I-338 | A | 3.42 | 961 | M+1 |
| I-217 | A | 2.66 | 860 | M+1 | I-280 | A | 2.95 | 908 | M+1 | I-339 | A | 2.69 | 933 | M+1 |
| I-218 | A | 2.54 | 860 | M+1 | I-281 | A | 2.86 | 920 | M+1 | I-340 | A | 3.21 | 958 | M+1 |
| I-219 | A | 1.99 | 932 | M+1 | I-282 | A | 2.99 | 908 | M+1 | I-341 | A | 2.48 | 911 | M+1 |
| I-220 | A | 2.75 | 857 | M+1 | I-283 | A | 2.79 | 891 | M+1 | I-342 | A | 2.50 | 911 | M+1 |
| I-221 | A | 2.76 | 915 | M+1 | I-284 | A | 2.84 | 880 | M+1 | I-343 | A | 3.29 | 1014 | M+1 |
| I-222 | A | 3.11 | 902 | M+1 | I-285 | A | 3.16 | 966 | M+1 | I-344 | A | 2.58 | 900 | M+1 |
| I-223 | A | 2.63 | 923 | M+1 | I-286 | A | 2.20 | 930 | M+1 | I-345 | A | 2.23 | 911 | M+1 |
| I-224 | A | 2.59 | 897 | M+1 | I-287 | A | 2.86 | 908 | M+1 | I-346 | A | 2.28 | 911 | M+1 |
| I-225 | A | 2.94 | 923 | M+1 | I-288 | A | 2.80 | 880 | M+1 | I-347 | A | 3.07 | 895 | M+1 |
| I-226 | A | 3.08 | 916 | M+1 | I-289 | A | 2.77 | 897 | M+1 | I-348 | A | 2.52 | 875 | M+1 |
| I-227 | A | 2.46 | 868 | M+1 | I-290 | A | 2.65 | 891 | M+1 | I-349 | A | 2.65 | 889 | M+1 |
| I-229 | A | 2.81 | 949 | M+1 | I-291 | A | 2.95 | 908 | M+1 | I-350 | A | 3.08 | 946 | M+1 |
| I-230 | A | 3.30 | 889 | M+18 | I-292 | A | 3.05 | 904 | M+1 | I-351 | A | 3.10 | 946 | M+1 |
| I-231 | A | 2.97 | 894 | M+1 | I-293 | A | 2.73 | 920 | M+1 | I-352 | A | 3.01 | 919 | M+1 |
| I-232 | A | 2.98 | 970 | M+1 | I-294 | A | 3.11 | 974 | M+1 | I-353 | A | 2.82 | 971 | M+18 |
| I-233 | A | 3.06 | 996 | M+1 | I-295 | A | 2.80 | 897 | M+1 | I-354 | A | 3.15 | 1000 | M+1 |
| I-234 | A | 3.08 | 946 | M+1 | I-296 | A | 2.97 | 908 | M+1 | I-355 | A | 2.42 | 948 | M+1 |
| I-235 | A | 3.06 | 946 | M+1 | I-297 | A | 2.74 | 897 | M+1 | I-356 | A | 2.81 | 891 | M+1 |
| I-236 | A | 3.27 | 908 | M+1 | I-298 | A | 2.85 | 900 | M+18 | I-357 | A | 3.01 | 932 | M+1 |
| I-237 | A | 3.02 | 945 | M+1 | I-299 | A | 3.26 | 980 | M+1 | I-358 | A | 3.09 | 911 | M+1 |
| I-238 | A | 2.98 | 945 | M+1 | I-300 | A | 3.11 | 902 | M+1 | I-359 | A | 3.04 | 911 | M+1 |
| I-239 | A | 2.77 | 959 | M+1 | I-301 | A | 2.89 | 952 | M+1 | I-360 | A | 3.11 | 932 | M+1 |
| I-240 | A | 2.86 | 932 | M+1 | I-302 | A | 2.91 | 952 | M+1 | I-361 | A | 3.12 | 932 | M+1 |
| I-241 | A | 2.90 | 916 | M+1 | I-303 | A | 2.87 | 899 | M+1 | I-362 | A | 2.66 | 897 | M+1 |
| I-242 | A | 2.90 | 916 | M+1 | I-304 | A | 2.90 | 899 | M+1 | I-363 | A | 2.85 | 911 | M+1 |
| I-243 | A | 3.15 | 895 | M+1 | I-305 | A | 2.45 | 891 | M+1 | I-364 | A | 3.17 | 960 | M+1 |
| I-244 | A | 2.94 | 1040 | M+18 | I-306 | A | 1.82 | 728 | M+1 | I-365 | A | 2.94 | 970 | M+1 |
| I-245 | A | 2.46 | 899 | M+1 | I-307 | A | 2.02 | 893 | M+1 | I-366 | A | 2.58 | 840 | M+1 |
| I-248 | A | 2.87 | 938 | M+1 | I-308 | A | 2.34 | 836 | M+1 | I-367 | A | 2.22 | 876 | M+1 |
| I-249 | A | 2.96 | 904 | M+1 | I-309 | A | 2.17 | 789 | M+1 | I-368 | A | 1.69 | 846 | M+1 |
| I-250 | A | 2.38 | 918 | M+1 | I-310 | A | 2.45 | 761 | M+1 | I-369 | A | 3.29 | 972 | M+1 |
| I-251 | A | 2.21 | 887 | M+1 | I-311 | A | 2.30 | 757 | M+1 | I-370 | A | 3.05 | 932 | M+1 |
| I-252 | A | 3.11 | 940 | M+1 | I-312 | A | 2.23 | 624 | M+1 | I-371 | A | 2.13 | 932 | M+1 |
| I-253 | A | 2.92 | 890 | M+1 | I-313 | A | 1.94 | 668 | M+1 | I-372 | A | 2.91 | 897 | M+1 |
| I-254 | A | 2.90 | 890 | M+1 | I-314 | A | 2.27 | 782 | M+1 | I-373 | A | 2.92 | 897 | M+1 |
| I-255 | A | 3.11 | 958 | M+1 | I-315 | A | 1.87 | 756 | M+1 | I-374 | A | 2.96 | 911 | M+1 |
| I-256 | A | 2.91 | 915 | M+1 | I-316 | A | 2.09 | 726 | M+1 | I-375 | A | 2.95 | 911 | M+1 |
| I-257 | A | 2.17 | 959 | M+1 | I-317 | A | 2.09 | 730 | M+1 | I-376 | A | 3.06 | 919 | M+1 |
| I-258 | A | 3.23 | 945 | M+1 | I-318 | A | 2.12 | 779 | M+1 | I-377 | A | 2.74 | 947 | M+1 |
| I-259 | A | 3.26 | 1013 | M+1 | I-319 | A | 2.12 | 799 | M+1 | I-378 | A | 2.73 | 943 | M+1 |
| I-260 | A | 2.81 | 918 | M+1 | I-320 | A | 1.69 | 767 | M+1 | I-379 | A | 2.87 | 957 | M+1 |
| I-261 | A | 2.98 | 918 | M+1 | I-321 | A | 1.93 | 756 | M+1 | I-380 | A | 3.03 | 907 | M+1 |
| I-262 | A | 2.87 | 904 | M+1 | I-322 | A | 3.16 | 940 | M+1 | I-381 | A | 2.89 | 880 | M+1 |
| I-263 | A | 2.77 | 918 | M+1 | I-323 | A | 2.41 | 930 | M+1 | I-382 | A | 3.10 | 961 | M+1 |
| I-264 | A | 3.03 | 904 | M+1 | I-324 | A | 3.15 | 1011 | M+1 | I-383 | A | 2.89 | 904 | M+1 |
| I-265 | A | 3.04 | 904 | M+1 | I-325 | A | 3.20 | 999 | M+1 | I-384 | A | 2.72 | 941 | M+1 |
| I-266 | A | 2.43 | 919 | M+1 | I-326 | A | 3.16 | 985 | M+1 | I-385 | A | 2.79 | 884 | M+1 |
| I-267 | A | 2.42 | 919 | M+1 | I-327 | A | 3.19 | 999 | M+1 | I-386 | A | 3.11 | 916 | M+1 |
| I-269 | A | 2.81 | 933 | M+1 | I-328 | A | 3.19 | 999 | M+1 | I-387 | A | 3.06 | 932 | M+1 |
| I-270 | A | 2.76 | 919 | M+1 | I-329 | A | 3.04 | 971 | M+1 | I-388 | A | 2.56 | 930 | M+1 |
| I-271 | A | 2.98 | 908 | M+1 | I-330 | A | 3.22 | 1013 | M+1 | I-389 | A | 2.49 | 930 | M+1 |
| I-272 | A | 3.00 | 904 | M+1 | I-331 | A | 3.09 | 985 | M+1 | I-390 | A | 2.71 | 946 | M+1 |
| I-273 | A | 2.38 | 891 | M+1 | I-332 | A | 3.08 | 985 | M+1 | I-391 | A | 3.12 | 945 | M+1 |
| I-274 | A | 2.93 | 920 | M+1 | I-333 | A | 3.16 | 985 | M+1 | I-392 | A | 2.57 | 884 | M+1 |
| I-275 | A | 2.98 | 908 | M+1 | I-334 | A | 2.69 | 899 | M+18 | I-393 | A | 3.28 | 958 | M+1 |
| I-276 | A | 2.82 | 880 | M+1 | I-335 | A | 2.55 | 842 | M+1 | I-394 | A | 2.72 | 885 | M+1 |
| I-277 | A | 2.91 | 908 | M+1 | I-336 | A | 2.54 | 856 | M+1 | I-395 | A | 2.72 | 885 | M+1 |

**[Table 111]**

| No. | method | RT | MS | | No. | method | RT | MS | | No. | method | RT | MS | |
|---|---|---|---|---|---|---|---|---|---|---|---|---|---|---|
| I-397 | A | 2.74 | 934 | M+1 | I-456 | A | 3.50 | 972 | M+1 | I-515 | A | 2.62 | 976 | M+1 |
| I-398 | A | 2.83 | 899 | M+1 | I-457 | A | 3.36 | 946 | M+1 | I-516 | A | 2.38 | 812 | M+1 |
| I-399 | A | 2.78 | 898 | M+1 | I-458 | A | 3.86 | 1017 | M+1 | I-517 | A | 2.49 | 826 | M+1 |
| I-400 | A | 2.87 | 913 | M+1 | I-459 | A | 3.48 | 974 | M+1 | I-518 | A | 3.01 | 934 | M+1 |
| I-401 | A | 2.93 | 913 | M+1 | I-460 | A | 3.20 | 944 | M+1 | I-519 | A | 3.10 | 948 | M+1 |
| I-402 | A | 2.82 | 926 | M+1 | I-461 | A | 3.43 | 960 | M+1 | I-520 | A | 3.25 | 1039 | M+1 |
| I-403 | A | 2.36 | 909 | M+1 | I-462 | A | 3.26 | 932 | M+1 | I-521 | A | 2.54 | 642 | M+1 |
| I-404 | A | 2.76 | 931 | M+1 | I-463 | A | 3.39 | 958 | M+1 | I-522 | A | 3.25 | 930 | M+1 |
| I-405 | A | 2.76 | 931 | M+1 | I-464 | A | 2.77 | 934 | M+1 | I-523 | A | 3.24 | 930 | M+1 |
| I-406 | A | 3.18 | 960 | M+1 | I-465 | A | 2.85 | 904 | M+1 | I-524 | A | 3.10 | 908 | M+1 |
| I-407 | A | 2.86 | 947 | M+1 | I-466 | A | 2.80 | 914 | M+1 | I-525 | A | 3.30 | 922 | M+1 |
| I-408 | A | 2.82 | 948 | M+1 | I-467 | A | 2.75 | 917 | M+1 | I-526 | A | 3.15 | 958 | M+1 |
| I-409 | A | 2.81 | 882 | M+1 | I-468 | A | 2.90 | 876 | M+1 | I-527 | A | 2.87 | 882 | M+1 |
| I-410 | A | 2.87 | 883 | M+1 | I-469 | A | 3.06 | 890 | M+1 | I-528 | A | 2.85 | 880 | M+1 |
| I-411 | A | 2.47 | 855 | M+1 | I-470 | A | 2.80 | 897 | M+1 | I-529 | A | 3.10 | 966 | M+1 |
| I-412 | A | 2.80 | 869 | M+1 | I-471 | A | 3.45 | 1002 | M+1 | I-530 | C | 3.06 | 966 | M+1 |
| I-413 | A | 2.48 | 935 | M+1 | I-472 | B | 2.63 | 888 | M+1 | I-531 | A | 3.15 | 940 | M+1 |
| I-414 | A | 2.44 | 968 | M+1 | I-473 | B | 2.78 | 904 | M+1 | I-532 | A | 2.98 | 904 | M+1 |
| I-415 | A | 2.66 | 968 | M+1 | I-474 | B | 2.72 | 902 | M+1 | I-533 | A | 3.13 | 918 | M+1 |
| I-416 | A | 2.40 | 947 | M+1 | I-475 | B | 2.76 | 904 | M+1 | I-534 | A | 3.03 | 904 | M+1 |
| I-417 | A | 2.50 | 961 | M+1 | I-476 | A | 2.63 | 875 | M+1 | I-535 | A | 3.35 | 946 | M+1 |
| I-418 | A | 2.96 | 918 | M+1 | I-477 | B | 2.47 | 982 | M+1 | I-536 | A | 2.46 | 931 | M+1 |
| I-419 | A | 2.56 | 879 | M+1 | I-478 | B | 2.91 | 876 | M+1 | I-537 | A | 3.15 | 972 | M+1 |
| I-420 | A | 3.17 | 970 | M+1 | I-479 | B | 3.06 | 890 | M+1 | I-538 | E | 2.31 | 947 | M+1 |
| I-421 | A | 3.37 | 994 | M+1 | I-480 | A | 3.12 | 959 | M+1 | I-539 | E | 2.53 | 973 | M+1 |
| I-422 | A | 3.37 | 946 | M+1 | I-481 | A | 2.74 | 1041 | M+1 | I-540 | A | 3.27 | 972 | M+1 |
| I-423 | A | 2.55 | 981 | M+1 | I-482 | A | 2.74 | 933 | M+1 | I-541 | A | 2.65 | 993 | M+1 |
| I-424 | A | 3.15 | 970 | M+1 | I-483 | A | 2.91 | 916 | M+1 | I-542 | A | 2.78 | 932 | M+18 |
| I-425 | A | 2.45 | 981 | M+1 | I-484 | A | 2.97 | 930 | M+1 | I-543 | A | 2.96 | 908 | M+1 |
| I-426 | A | 3.43 | 1008 | M+1 | I-485 | A | 3.14 | 958 | M+1 | I-544 | A | 3.04 | 924 | M+1 |
| I-427 | A | 3.24 | 1000 | M+1 | I-486 | A | 2.84 | 981 | M+1 | I-545 | A | 2.99 | 904 | M+1 |
| I-428 | A | 3.58 | 974 | M+1 | I-487 | A | 3.09 | 947 | M+1 | I-546 | A | 2.91 | 935 | M+1 |
| I-429 | A | 2.39 | 1003 | M+1 | I-488 | A | 3.22 | 918 | M+1 | I-547 | A | 3.07 | 924 | M+1 |
| I-430 | A | 3.22 | 986 | M+1 | I-489 | A | 2.63 | 905 | M+1 | I-548 | A | 2.51 | 906 | M+1 |
| I-431 | A | 3.00 | 987 | M+1 | I-490 | A | 3.06 | 934 | M+1 | I-549 | A | 2.99 | 904 | M+1 |
| I-432 | A | 3.50 | 1022 | M+1 | I-491 | A | 3.20 | 922 | M+1 | I-550 | A | 2.99 | 904 | M+1 |
| I-433 | A | 2.83 | 878 | M+1 | I-492 | A | 3.01 | 894 | M+1 | I-551 | A | 2.72 | 891 | M+1 |
| I-434 | A | 3.25 | 1039 | M+1 | I-493 | A | 3.15 | 922 | M+1 | I-552 | A | 2.87 | 934 | M+1 |
| I-435 | A | 3.15 | 958 | M+1 | I-494 | A | 3.00 | 905 | M+1 | I-553 | A | 2.95 | 950 | M+1 |
| I-436 | A | 2.92 | 933 | M+1 | I-495 | A | 2.29 | 859 | M+1 | I-554 | A | 2.86 | 920 | M+1 |
| I-437 | A | 2.92 | 933 | M+1 | I-496 | A | 2.39 | 860 | M+1 | I-555 | A | 2.63 | 906 | M+1 |
| I-438 | A | 2.83 | 923 | M+1 | I-497 | A | 2.43 | 802 | M+1 | I-556 | A | 3.14 | 918 | M+1 |
| I-439 | A | 2.84 | 961 | M+1 | I-498 | A | 2.41 | 808 | M+1 | I-557 | A | 2.51 | 891 | M+1 |
| I-440 | A | 2.98 | 989 | M+1 | I-499 | A | 2.67 | 886 | M+1 | I-558 | A | 3.20 | 996 | M+1 |
| I-441 | A | 2.89 | 939 | M+1 | I-500 | A | 2.28 | 883 | M+1 | I-559 | A | 2.55 | 905 | M+1 |
| I-442 | A | 2.80 | 974 | M+1 | I-501 | A | 2.30 | 859 | M+1 | I-560 | A | 2.77 | 950 | M+1 |
| I-443 | A | 2.72 | 934 | M+1 | I-502 | A | 2.46 | 841 | M+1 | I-561 | A | 2.68 | 906 | M+1 |
| I-444 | A | 2.94 | 959 | M+1 | I-503 | A | 2.51 | 841 | M+1 | I-562 | A | 2.65 | 906 | M+1 |
| I-445 | A | 2.69 | 936 | M+1 | I-504 | A | 2.42 | 859 | M+1 | I-563 | A | 3.11 | 924 | M+1 |
| I-446 | A | 2.74 | 942 | M+1 | I-505 | A | 2.36 | 858 | M+1 | I-564 | A | 3.15 | 982 | M+1 |
| I-447 | A | 2.81 | 956 | M+1 | I-506 | A | 2.43 | 871 | M+1 | I-565 | A | 2.88 | 915 | M+1 |
| I-448 | A | 2.96 | 894 | M+1 | I-507 | A | 2.81 | 908 | M+1 | I-566 | A | 2.92 | 920 | M+1 |
| I-449 | A | 2.78 | 905 | M+1 | I-508 | A | 3.18 | 986 | M+1 | I-567 | A | 2.96 | 935 | M+1 |
| I-450 | A | 3.02 | 904 | M+1 | I-509 | A | 3.23 | 996 | M+1 | I-568 | A | 2.83 | 941 | M+1 |
| I-451 | A | 3.27 | 972 | M+1 | I-510 | A | 3.12 | 918 | M+1 | I-569 | A | 2.91 | 896 | M+1 |
| I-452 | A | 3.07 | 929 | M+1 | I-511 | A | 2.72 | 906 | M+1 | I-570 | A | 2.78 | 894 | M+1 |
| I-453 | A | 2.79 | 941 | M+1 | I-512 | A | 2.60 | 981 | M+1 | I-571 | A | 3.20 | 996 | M+1 |
| I-454 | A | 3.21 | 988 | M+1 | I-513 | A | 2.81 | 943 | M+1 | I-572 | A | 3.31 | 1010 | M+1 |
| I-455 | E | 2.21 | 933 | M+1 | I-514 | A | 3.35 | 1014 | M+1 | I-573 | A | 2.15 | 726 | M+1 |

**[Table 112]**

| No. | method | RT | MS | | No. | method | RT | MS | | No. | method | RT | MS | |
|---|---|---|---|---|---|---|---|---|---|---|---|---|---|---|
| I-574 | A | 2.68 | 933 | M+1 | I-635 | A | 2.63 | 961 | M+1 | I-694 | A | 2.72 | 949 | M+1 |
| I-575 | A | 3.08 | 976 | M+1 | I-636 | A | 2.91 | 1000 | M+1 | I-695 | A | 3.10 | 983 | M+1 |
| I-576 | A | 3.23 | 930 | M+1 | I-637 | A | 3.05 | 958 | M+1 | I-696 | A | 3.28 | 997 | M+1 |
| I-577 | A | 3.23 | 930 | M+1 | I-638 | A | 3.08 | 970 | M+1 | I-697 | A | 3.00 | 917 | M+1 |
| I-578 | A | 2.55 | 960 | M+1 | I-639 | A | 3.15 | 984 | M+1 | I-698 | A | 3.00 | 917 | M+1 |
| I-579 | A | 3.24 | 1014 | M+1 | I-640 | A | 2.95 | 985 | M+1 | I-699 | A | 3.24 | 989 | M+1 |
| I-580 | B | 2.59 | 963 | M+1 | I-641 | A | 2.99 | 997 | M+1 | I-700 | A | 2.93 | 990 | M+1 |
| I-581 | A | 3.12 | 940 | M+1 | I-642 | A | 3.06 | 1011 | M+1 | I-701 | E | 2.35 | 1003 | M+1 |
| I-582 | A | 3.33 | 954 | M+1 | I-643 | A | 3.01 | 972 | M+1 | I-702 | A | 2.96 | 962 | M+1 |
| I-583 | A | 2.65 | 974 | M+1 | I-644 | A | 3.04 | 984 | M+1 | I-703 | A | 3.15 | 955 | M+1 |
| I-584 | A | 2.81 | 988 | M+1 | I-645 | A | 3.11 | 897 | M+1 | I-704 | A | 3.03 | 962 | M+1 |
| I-585 | B | 2.76 | 981 | M+1 | I-646 | A | 2.90 | 911 | M+1 | I-705 | A | 2.87 | 930 | M+1 |
| I-586 | B | 2.62 | 933 | M+1 | I-647 | A | 3.06 | 934 | M+1 | I-706 | A | 3.03 | 944 | M+1 |
| I-587 | A | 2.74 | 947 | M+1 | I-648 | A | 2.82 | 947 | M+1 | I-707 | A | 3.22 | 955 | M+1 |
| I-588 | A | 3.13 | 989 | M+1 | I-649 | A | 2.77 | 947 | M+1 | I-708 | A | 3.08 | 960 | M+1 |
| I-589 | A | 2.82 | 923 | M+1 | I-650 | A | 3.08 | 940 | M+1 | I-709 | A | 3.08 | 931 | M+1 |
| I-590 | A | 2.72 | 961 | M+1 | I-651 | A | 2.79 | 948 | M+1 | I-710 | A | 3.10 | 931 | M+1 |
| I-591 | A | 2.69 | 947 | M+1 | I-652 | A | 2.70 | 941 | M+1 | I-711 | A | 3.12 | 931 | M+1 |
| I-592 | A | 2.72 | 960 | M+1 | I-653 | A | 2.97 | 985 | M+1 | I-712 | A | 2.96 | 968 | M+1 |
| I-594 | A | 2.99 | 959 | M+1 | I-654 | A | 3.18 | 982 | M+1 | I-713 | A | 2.97 | 967 | M+1 |
| I-595 | A | 3.05 | 989 | M+1 | I-655 | A | 2.80 | 917 | M+1 | I-714 | A | 2.81 | 1042 | M+1 |
| I-597 | A | 3.28 | 960 | M+1 | I-656 | A | 2.99 | 967 | M+1 | I-715 | A | 2.98 | 1056 | M+1 |
| I-598 | A | 2.99 | 904 | M+1 | I-657 | A | 3.00 | 967 | M+1 | I-716 | A | 2.81 | 947 | M+1 |
| I-599 | A | 3.18 | 918 | M+1 | I-658 | A | 3.15 | 981 | M+1 | I-717 | A | 2.82 | 947 | M+1 |
| I-600 | A | 3.00 | 973 | M+1 | I-659 | A | 3.16 | 981 | M+1 | I-718 | A | 2.92 | 947 | M+1 |
| I-601 | A | 2.76 | 986 | M+1 | I-660 | A | 2.34 | 841 | M+1 | I-719 | A | 2.37 | 967 | M+1 |
| I-602 | A | 1.62 | 945 | M+1 | I-661 | A | 2.49 | 875 | M+1 | I-720 | A | 3.10 | 1009 | M+1 |
| I-603 | A | 2.93 | 904 | M+1 | I-662 | A | 3.11 | 908 | M+1 | I-721 | A | 3.34 | 1043 | M+1 |
| I-604 | A | 2.70 | 925 | M+1 | I-663 | A | 2.71 | 871 | M+1 | I-722 | A | 3.29 | 1034 | M+1 |
| I-605 | A | 2.69 | 925 | M+1 | I-664 | A | 2.65 | 983 | M+1 | I-723 | A | 3.10 | 930 | M+1 |
| I-606 | A | 3.14 | 989 | M+1 | I-665 | D | 2.88 | 875 | M+1 | I-724 | A | 3.24 | 1056 | M+1 |
| I-607 | A | 2.75 | 966 | M+1 | I-666 | A | 2.76 | 958 | M+1 | I-725 | A | 3.03 | 973 | M+1 |
| I-608 | A | 2.89 | 980 | M+1 | I-667 | A | 3.19 | 944 | M+1 | I-726 | A | 3.00 | 931 | M+1 |
| I-609 | B | 2.32 | 940 | M+1 | I-668 | A | 3.19 | 944 | M+1 | I-727 | A | 3.36 | 970 | M+1 |
| I-610 | B | 2.58 | 948 | M+1 | I-669 | A | 2.90 | 961 | M+1 | I-728 | A | 2.84 | 988 | M+1 |
| I-611 | B | 2.48 | 939 | M+1 | I-670 | A | 2.81 | 882 | M+1 | I-729 | A | 2.70 | 988 | M+1 |
| I-612 | B | 2.71 | 983 | M+1 | I-671 | A | 3.29 | 1025 | M+1 | I-730 | A | 3.10 | 1002 | M+1 |
| I-613 | A | 2.85 | 959 | M+1 | I-672 | A | 3.15 | 973 | M+1 | I-731 | A | 2.86 | 1001 | M+1 |
| I-614 | A | 2.94 | 939 | M+1 | I-673 | A | 3.30 | 1057 | M+1 | I-732 | A | 2.77 | 963 | M+1 |
| I-615 | A | 2.96 | 939 | M+1 | I-674 | A | 3.33 | 1016 | M+1 | I-733 | A | 2.40 | 1016 | M+1 |
| I-616 | A | 3.13 | 953 | M+1 | I-675 | A | 3.15 | 940 | M+1 | I-734 | A | 2.38 | 1016 | M+1 |
| I-617 | A | 2.81 | 972 | M+1 | I-676 | A | 2.89 | 945 | M+1 | I-735 | A | 3.08 | 910 | M+1 |
| I-618 | A | 3.30 | 960 | M+1 | I-677 | A | 2.54 | 973 | M+1 | I-736 | A | 2.65 | 961 | M+1 |
| I-619 | A | 2.95 | 920 | M+1 | I-678 | A | 3.10 | 974 | M+1 | I-737 | A | 3.03 | 981 | M+1 |
| I-620 | A | 2.80 | 929 | M-1 | I-679 | A | 2.82 | 975 | M+1 | I-738 | A | 2.73 | 983 | M+1 |
| I-621 | A | 2.81 | 929 | M-1 | I-680 | A | 2.14 | 988 | M+1 | I-739 | A | 2.49 | 499 | M/2+1 |
| I-622 | A | 2.92 | 954 | M+1 | I-681 | A | 2.99 | 987 | M+1 | I-740 | A | 2.86 | 988 | M+1 |
| I-623 | A | 2.83 | 940 | M+1 | I-682 | A | 2.95 | 959 | M+1 | I-741 | A | 2.65 | 988 | M+1 |
| I-624 | A | 2.88 | 940 | M+1 | I-683 | A | 2.79 | 977 | M+1 | I-742 | A | 2.65 | 973 | M+1 |
| I-625 | A | 2.81 | 951 | M+1 | I-684 | A | 2.85 | 1033 | M+1 | I-743 | A | 2.82 | 1001 | M+1 |
| I-626 | A | 3.17 | 935 | M+1 | I-685 | A | 2.85 | 984 | M+1 | I-744 | A | 2.82 | 987 | M+1 |
| I-627 | A | 3.06 | 967 | M+1 | I-686 | A | 3.02 | 998 | M+1 | I-745 | A | 2.96 | 961 | M+1 |
| I-628 | A | 3.38 | 949 | M+1 | I-687 | A | 2.62 | 935 | M+1 | I-746 | A | 2.95 | 961 | M+1 |
| I-629 | A | 2.95 | 954 | M+1 | I-688 | A | 2.79 | 983 | M+1 | I-747 | A | 3.08 | 961 | M+1 |
| I-630 | A | 2.89 | 940 | M+1 | I-689 | A | 2.70 | 973 | M+1 | I-748 | A | 2.85 | 951 | M+1 |
| I-631 | A | 2.86 | 940 | M+1 | I-690 | A | 2.96 | 1001 | M+1 | I-749 | A | 2.82 | 951 | M+1 |
| I-632 | A | 2.97 | 954 | M+1 | I-691 | A | 2.92 | 1001 | M+1 | I-750 | A | 3.05 | 1001 | M+1 |
| I-633 | A | 2.66 | 909 | M+1 | I-692 | A | 3.42 | 1031 | M+1 | I-751 | A | 3.19 | 931 | M+1 |
| I-634 | A | 2.32 | 871 | M+1 | I-693 | A | 2.94 | 946 | M+1 | I-752 | A | 3.19 | 931 | M+1 |

**[Table 113]**

| No. | method | RT | MS | | No. | method | RT | MS | | No. | method | RT | MS | |
|---|---|---|---|---|---|---|---|---|---|---|---|---|---|---|
| I-753 | A | 2.80 | 953 | M+1 | I-806 | A | 2.35 | 847 | M+18 | I-859 | A | 2.80 | 972 | M+1 |
| I-754 | A | 2.67 | 962 | M+1 | I-807 | B | 2.46 | 861 | M+1 | I-860 | A | 1.55 | 931 | M+1 |
| I-755 | A | 2.82 | 975 | M+1 | I-808 | A | 3.10 | 1049 | M+18 | I-861 | A | 2.80 | 945 | M+1 |
| I-756 | A | 3.24 | 995 | M+1 | I-809 | A | 2.81 | 954 | M+18 | I-862 | A | 2.73 | 958 | M+1 |
| I-757 | A | 2.89 | 997 | M+1 | I-810 | B | 2.59 | 907 | M-1 | I-863 | A | 2.87 | 897 | M+1 |
| I-758 | A | 2.75 | 1010 | M+2 | I-811 | B | 2.49 | 793 | M-1 | I-864 | A | 3.00 | 920 | M+1 |
| I-759 | A | 2.75 | 976 | M+1 | I-812 | B | 2.56 | 843 | M+23 | I-865 | A | 2.89 | 883 | M+1 |
| I-760 | A | 3.32 | 1077 | M+1 | I-813 | A | 2.51 | 824 | M+1 | I-866 | A | 2.80 | 883 | M+1 |
| I-761 | A | 3.49 | 1091 | M+1 | I-814 | A | 2.44 | 883 | M+1 | I-867 | A | 2.83 | 883 | M+1 |
| I-762 | A | 2.93 | 977 | M+1 | I-815 | B | 2.68 | 917 | M+1 | I-868 | A | 3.00 | 944 | M+1 |
| I-763 | A | 3.78 | 1116 | M+1 | I-816 | A | 2.60 | 770 | M+1 | I-869 | A | 3.03 | 956 | M+1 |
| I-764 | A | 3.22 | 924 | M+1 | I-817 | B | 2.52 | 847 | M+23 | I-870 | A | 3.10 | 970 | M+1 |
| I-765 | A | 3.22 | 924 | M+1 | I-818 | A | 2.24 | 839 | M+1 | I-871 | A | 2.90 | 971 | M+1 |
| I-766 | A | 2.97 | 974 | M+1 | I-819 | A | 2.70 | 863 | M+1 | I-872 | A | 2.94 | 983 | M+1 |
| I-767 | A | 2.79 | 976 | M+1 | I-820 | A | 2.92 | 905 | M+1 | I-873 | A | 3.01 | 997 | M+1 |
| I-768 | A | 2.84 | 990 | M+1 | I-821 | A | 2.74 | 996 | M+1 | I-874 | A | 2.96 | 958 | M+1 |
| I-769 | A | 3.18 | 938 | M+1 | I-822 | A | 2.93 | 1010 | M+1 | I-875 | A | 2.99 | 970 | M+1 |
| I-770 | A | 3.18 | 938 | M+1 | I-823 | A | 2.52 | 906 | M+1 | I-876 | A | 3.06 | 984 | M+1 |
| I-771 | A | 2.15 | 759 | M+1 | I-824 | B | 2.62 | 884 | M-1 | I-877 | A | 2.75 | 868 | M+1 |
| I-772 | A | 3.24 | 954 | M+1 | I-825 | B | 2.92 | 909 | M-1 | I-878 | A | 2.77 | 868 | M+1 |
| I-773 | A | 3.71 | 996 | M+1 | I-826 | B | 2.75 | 877 | M+1 | I-879 | A | 2.81 | 947 | M+1 |
| I-774 | A | 2.86 | 984 | M+1 | I-827 | A | 2.58 | 879 | M+1 | I-880 | A | 2.81 | 916 | M+1 |
| I-775 | A | 3.19 | 982 | M+1 | I-828 | A | 2.68 | 884 | M+1 | I-881 | A | 3.04 | 930 | M+1 |
| I-776 | A | 3.28 | 997 | M+1 | I-829 | B | 2.77 | 889 | M+1 | I-882 | A | 2.60 | 920 | M+1 |
| I-777 | A | 3.59 | 980 | M+1 | I-830 | B | 2.54 | 804 | M-1 | I-883 | A | 3.01 | 896 | M+1 |
| I-778 | A | 3.10 | 941 | M+1 | I-831 | A | 2.82 | 826 | M+1 | I-884 | A | 2.69 | 935 | M+1 |
| I-779 | A | 2.73 | 948 | M+1 | I-832 | B | 2.61 | 835 | M+1 | I-885 | A | 2.79 | 970 | M+1 |
| I-780 | A | 2.62 | 859 | M+1 | I-833 | A | 2.36 | 880 | M+18 | I-886 | A | 3.15 | 910 | M+1 |
| I-781 | A | 2.67 | 857 | M+1 | I-834 | B | 2.51 | 831 | M-1 | I-887 | A | 3.13 | 910 | M+1 |
| I-782 | A | 2.36 | 842 | M+1 | I-835 | A | 3.06 | 891 | M+1 | I-888 | A | 2.18 | 745 | M+1 |
| I-783 | A | 2.75 | 781 | M+1 | I-836 | A | 3.01 | 881 | M+1 | I-889 | A | 2.78 | 856 | M+1 |
| I-784 | A | 3.06 | 1058 | M+1 | I-837 | B | 2.44 | 859 | M-1 | I-890 | A | 2.87 | 906 | M+1 |
| I-785 | A | 2.65 | 970 | M+1 | I-838 | A | 2.56 | 918 | M+1 | I-891 | A | 2.76 | 949 | M+1 |
| I-786 | A | 2.88 | 935 | M+1 | I-839 | B | 2.23 | 823 | M+1 | I-892 | A | 2.86 | 975 | M+1 |
| I-787 | A | 2.50 | 891 | M+1 | I-840 | A | 2.44 | 853 | M+1 | I-893 | A | 2.79 | 961 | M+1 |
| I-788 | A | 2.61 | 890 | M+1 | I-841 | B | 2.67 | 906 | M-1 | I-894 | A | 3.28 | 960 | M+1 |
| I-789 | A | 1.88 | 1013 | M+1 | I-842 | A | 2.24 | 839 | M+1 | I-895 | A | 3.17 | 964 | M+1 |
| I-790 | A | 3.21 | 987 | M+1 | I-843 | A | 2.57 | 888 | M+1 | I-896 | A | 3.26 | 960 | M+1 |
| I-791 | A | 2.83 | 961 | M+1 | I-844 | A | 2.78 | 908 | M+1 | I-897 | A | 2.48 | 841 | M+1 |
| I-792 | A | 2.43 | 1084 | M+1 | I-845 | A | 2.93 | 907 | M+1 | I-898 | A | 2.63 | 858 | M+1 |
| I-793 | A | 2.98 | 987 | M+1 | I-846 | A | 2.76 | 908 | M+1 | I-899 | A | 2.62 | 855 | M+1 |
| I-794 | A | 3.22 | 1051 | M+1 | I-847 | A | 2.84 | 1030 | M+1 | I-900 | A | 2.99 | 912 | M+1 |
| I-795 | A | 3.35 | 1029 | M+1 | I-848 | B | 2.62 | 870 | M-1 | I-901 | A | 2.72 | 836 | M+1 |
| I-796 | A | 2.92 | 947 | M+1 | I-849 | B | 2.67 | 903 | M-1 | I-902 | A | 3.19 | 966 | M+1 |
| I-797 | A | 3.04 | 961 | M+1 | I-850 | B | 2.79 | 883 | M-1 | I-903 | A | 2.79 | 910 | M+1 |
| I-798 | A | 2.85 | 917 | M+1 | I-851 | A | 2.99 | 926 | M+1 | I-904 | A | 2.97 | 987 | M+1 |
| I-799 | B | 2.51 | 917 | M+1 | I-852 | A | 2.91 | 876 | M+1 | I-905 | B | 2.68 | 890 | M+1 |
| I-800 | A | 2.88 | 917 | M+1 | I-853 | A | 2.91 | 906 | M+1 | I-906 | B | 2.70 | 890 | M+1 |
| I-801 | B | 2.67 | 932 | M+1 | I-854 | A | 2.66 | 933 | M+1 | I-907 | A | 2.97 | 907 | M+1 |
| I-802 | A | 3.01 | 860 | M+1 | I-855 | A | 2.70 | 946 | M+1 | I-908 | A | 2.95 | 891 | M+1 |
| I-803 | A | 2.96 | 860 | M+1 | I-856 | A | 2.81 | 919 | M+1 | I-909 | A | 2.99 | 932 | M+1 |
| I-804 | A | 2.99 | 860 | M+1 | I-857 | A | 2.92 | 945 | M+1 | I-910 | A | 3.88 | 1130 | M+1 |
| I-805 | A | 2.97 | 878 | M+1 | I-858 | A | 3.05 | 959 | M+1 | | | | | |

### Test Example 1: HIV-protease inhibitory activity assay

The HIV-1 PR assay was performed by using a FRET (Fluorescence Resonance Energy Transfer) peptide substrate (AnaSpec, Inc., Fremont, CA). Initially in the intact FRET peptide, the fluorescence of HiLyte Fluor 488 is quenched by QXL 520. Upon substrate cleavage by PR, the fluorescence is recovered and can be monitored. Test compounds diluted in DMSO was plated to the wells of 384-well plate. Then, mixed with assay buffer (0.1mol/L NaAc containing 0.5mol/L NaCl, 1mmol/L EDTA, 1mmol/L DTT, and 0.05% Tween 20, pH 4.8) in the presence or absence of HIV-1 PR (0.83 ng), and let it stand for 5 minutes at room temperature. Background fluorescence intensity was measured and let it stand for another 15 minutes at room temperature. The enzyme reaction was started by adding substrate diluted in assay buffer at final concentration of 2µmol/L, and let it stand for 60 minutes at room temperature. After 60 minutes, the fluorescence intensity was measured. Fluorescence intensity was measured with excitation and emission wavelengths of 485 nm and 535 nm, respectively.

IC₅₀ values were determined using non-linear regression four-parameter logistic equation, $y = A + \left(\left(B-A\right)/\left(1+\left({\left(C/x\right)}^{∧}D\right)\right)\right)$ where A = minimum inhibition, B = maximum inhibition, C = log IC₅₀, D = slope factor, x = concentration of compound and y = % inhibition.

### Test Example 2: Protein binding test

Unbound ratios of the present invention compounds in the sera of some animal species, fu(%), were evaluated.

Assay condition: Evaluation method; Equilibrium dialysis method, Reaction time; 24hours, Reaction temperature; 37°C, Assay concentration; 2µg/mL

A solution of the compound was added to each serum and mixed to prepare the serum samples with above concentration. The serum sample was put into one side of an equilibrium dialysis apparatus with dialysis membrane and phosphate buffered saline (PBS) was also put to another side of the apparatus. The apparatus was incubated at 37°C for 24 hours. After incubation, the serum and PBS samples were collected and the compound concentrations in both samples were measured by LC/MS/MS. Fu was calculated by dividing the concentration in PBS by the concentration in the serum.

### Test Example 3: Evaluation of total body clearance (CLtot)

### (Study design and method)

Animal: Rat, Sprague-Dawley (SD)
Animal care condition: Rats were allowed free access to the sterilized tap water and the solid laboratory food.
Dose: Intravenous administration with designated doses was selected. (Doses were changed depending on compounds.) $0.5 ⋅ 10 mg / kg \left(n=2-3\right)$

Preparation of dosing formulation: The dosing solution was prepared by dissolving in an adequate solvent.

Administration method: The dosing solution was injected into the tail vein by a syringe with a needle

Blood sampling: The blood was collected at the scheduled time and obtained the plasma. Plasma concentrations of the compound were measured by LC/MS/MS. Statistical analysis: Area under the plasma concentration-time curve (AUC) of the compound was calculated by pharmacokinetic analysis software, WinNonlinTM and CLtot was calculated by dividing the dose by AUC.

The results of Test example 1, 2 and 3 were shown below.

**[Table 114]**

| No. | IC50 (nM) | No. | IC50 (nM) | No. | IC50 (nM) | No. | IC50 (nM) | No. | IC50 (nM) | No. | IC50 (nM) | No. | IC50 (nM) | No. | IC50 (nM) |
|---|---|---|---|---|---|---|---|---|---|---|---|---|---|---|---|
| I-1 | 1.29 | I-57 | 1.07 | I-115 | 1.16 | I-173 | 1.69 | I-231 | 3.91 | I-289 | 2.40 | I-347 | 4.40 | I-405 | 2.42 |
| I-2 | 1.05 | I-58 | 1.11 | I-116 | 0.87 | I-174 | 2.08 | I-232 | 4.10 | I-290 | 2.08 | I-348 | 1.96 | I-406 | 1.89 |
| I-3 | 1.02 | I-59 | 1.19 | I-117 | 0.75 | I-175 | 2.00 | I-233 | 6.22 | I-291 | 3.54 | I-349 | 2.33 | I-407 | 3.77 |
| I-4 | 1.69 | I-60 | 1.60 | I-118 | 0.87 | I-176 | 1.01 | I-234 | 3.50 | I-292 | 2.68 | I-350 | 5.32 | I-408 | 1.80 |
| I-5 | 1.15 | I-61 | 1.90 | I-119 | 1.23 | I-177 | 3.17 | I-235 | 3.56 | I-293 | 1.90 | I-351 | 5.09 | I-409 | 2.45 |
| I-6 | 1.36 | I-62 | 0.82 | I-120 | 0.94 | I-178 | 3.14 | I-236 | 2.90 | I-294 | 3.92 | I-352 | 4.22 | I-410 | 1.99 |
| I-7 | 2.31 | I-63 | 0.89 | I-121 | 0.97 | I-179 | 1.10 | I-237 | 3.56 | I-295 | 2.19 | I-353 | 3.09 | I-411 | 1.30 |
| I-8 | 11.6 | I-64 | 1.30 | I-122 | 1.02 | I-180 | 2.71 | I-238 | 2.52 | I-296 | 1.91 | I-354 | 44.2 | I-412 | 1.51 |
| I-9-1 | 1.53 | I-65 | 1.28 | I-123 | 1.00 | I-181 | 7.46 | I-239 | 2.74 | I-297 | 1.82 | I-355 | 2.35 | I-413 | 1.84 |
| I-9-2 | 1.56 | I-66 | 0.84 | I-124 | 0.89 | I-182 | 1.44 | I-240 | 2.02 | I-298 | 2.29 | I-356 | 3.30 | I-414 | 1.71 |
| I-10-1 | 5.17 | I-67 | 1.88 | I-125 | 1.58 | I-183 | 2.39 | I-241 | 2.95 | I-299 | 16.5 | I-357 | 4.41 | I-415 | 2.55 |
| I-10-2 | 4.47 | I-68 | 1.83 | I-126 | 0.76 | I-184 | 1.64 | I-242 | 3.11 | I-300 | 4.78 | I-358 | 3.89 | I-416 | 1.63 |
| I-11 | 1.40 | I-69 | 1.17 | I-127 | 1.14 | I-185 | 1.71 | I-243 | 4.24 | I-301 | 3.14 | I-359 | 3.77 | I-417 | 1.64 |
| I-12 | 1.82 | I-70 | 1.06 | I-128 | 2.19 | I-186 | 2.14 | I-244 | 2.01 | I-302 | 2.28 | I-360 | 4.54 | I-418 | 2.21 |
| I-13 | 0.74 | I-71 | 1.77 | I-129 | 3.09 | I-187 | 2.21 | I-245 | 2.01 | I-303 | 1.86 | I-361 | 6.22 | I-419 | 1.83 |
| I-14 | 1.70 | I-72 | 1.45 | I-130 | 1.12 | I-188 | 2.22 | I-246 | 2.07 | I-304 | 1.98 | I-362 | 3.07 | I-420 | 13.1 |
| I-15 | 2.27 | I-73 | 4.73 | I-131 | 2.12 | I-189 | 2.53 | I-247 | 2.25 | I-305 | 1.99 | I-363 | 3.36 | I-421 | 14.7 |
| I-16 | 2.22 | I-74 | 1.71 | I-132 | 2.66 | I-190 | 5.59 | I-248 | 2.26 | I-306 | 1.89 | I-364 | 5.39 | I-422 | 8.58 |
| I-17 | 1.24 | I-75 | 1.19 | I-133 | 1.53 | I-191 | 2.22 | I-249 | 2.26 | I-307 | 1.87 | I-365 | 3.20 | I-423 | 2.80 |
| I-18 | 1.63 | I-76 | 1.35 | I-134 | 2.04 | I-192 | 2.27 | I-250 | 2.20 | I-308 | 1.91 | I-366 | 2.72 | I-424 | 5.41 |
| I-19 | 1.18 | I-77 | 2.16 | I-135 | 6.80 | I-193 | 3.48 | I-251 | 2.30 | I-309 | 2.00 | I-367 | 2.61 | I-425 | 2.65 |
| I-20 | 1.77 | I-78 | 1.00 | I-136 | 1.40 | I-194 | 1.63 | I-252 | 4.22 | I-310 | 1.96 | I-368 | 2.11 | I-426 | 8.10 |
| I-21 | 1.39 | I-79 | 1.74 | I-137 | 3.99 | I-195 | 2.15 | I-253 | 2.59 | I-311 | 1.88 | I-369 | 15.8 | I-427 | 10.4 |
| I-22 | 0.99 | I-80 | 1.75 | I-138 | 2.66 | I-196 | 7.42 | I-254 | 2.64 | I-312 | 1.58 | I-370 | 5.02 | I-428 | 15.9 |
| I-23 | 3.38 | I-81 | 1.88 | I-139 | 0.78 | I-197 | 1.94 | I-255 | 2.39 | I-313 | 1.81 | I-371 | 10.5 | I-429 | 2.21 |
| I-24 | 1.39 | I-82 | 3.32 | I-140 | 1.42 | I-198 | 1.95 | I-256 | 2.82 | I-314 | 1.93 | I-372 | 3.26 | I-430 | 17.5 |
| I-25 | 2.07 | I-83 | 1.32 | I-141 | 1.74 | I-199 | 2.35 | I-257 | 4.98 | I-315 | 1.98 | I-373 | 2.92 | I-431 | 3.72 |
| I-26 | 2.01 | I-84 | 4.12 | I-142 | 1.72 | I-200 | 5.08 | I-258 | 5.23 | I-316 | 1.85 | I-374 | 4.35 | I-432 | 8.42 |
| I-27 | 3.70 | I-85 | 1.33 | I-143 | 0.99 | I-201 | 10.2 | I-259 | 10.9 | I-317 | 1.86 | I-375 | 3.87 | I-433 | 2.49 |
| I-28 | 1.63 | I-86 | 1.76 | I-144 | 0.80 | I-202 | 12.1 | I-260 | 2.96 | I-318 | 1.63 | I-376 | 9.86 | I-434 | 1.98 |
| I-29 | 1.41 | I-87 | 1.32 | I-145 | 0.81 | I-203 | 5.02 | I-261 | 3.55 | I-319 | 2.09 | I-377 | 5.40 | I-435 | 6.27 |
| I-30 | 3.67 | I-88 | 0.86 | I-146 | 0.92 | I-204 | 2.04 | I-262 | 3.95 | 1-320 | 1.95 | I-378 | 4.10 | I-436 | 3.17 |
| I-31 | 0.73 | I-89 | 1.11 | I-147 | 1.01 | I-205 | 2.03 | I-263 | 3.41 | I-321 | 1.90 | I-379 | 4.42 | I-437 | 4.42 I |
| I-32 | 1.37 | I-90 | 1.32 | I-148 | 1.16 | I-206 | 3.38 | I-264 | 3.53 | I-322 | 10.5 | I-380 | 5.51 | I-438 | 2.55 |
| I-33 | 0.72 | I-91 | 1.35 | I-149 | 1.27 | I-207 | 2.10 | I-265 | 5.07 | I-323 | 2.41 | I-381 | 4.69 | I-439 | 3.45 |
| I-34 | 1.28 | I-92 | 1.88 | I-150 | 0.93 | I-208 | 1.86 | I-266 | 2.61 | I-324 | 19.6 | I-382 | 7.26 | I-440 | 4.50 |
| I-35 | 2.85 | I-93 | 0.99 | I-151 | 1.88 | I-209 | 3.07 | I-267 | 2.40 | I-325 | 31.7 | I-383 | 6.47 | I-441 | 2.61 |
| I-36 | 1.88 | I-94 | 40.2 | I-152 | 1.15 | I-210 | 2.15 | I-268 | 4.46 | I-326 | 9.19 | I-384 | 6.62 | I-442 | 2.95 |
| I-37 | 2.22 | I-95 | 2.09 | I-153 | 1.08 | I-211 | 11.7 | I-269 | 3.41 | I-327 | 10.4 | I-385 | 1.25 | I-443 | 1.88 |
| I-38 | 3.85 | I-96 | 2.14 | I-154 | 1.87 | I-212 | 2.86 | I-270 | 3.38 | I-328 | 12.8 | I-386 | 5.89 | I-444 | 5.10 |
| I-39 | 1.79 | I-97 | 0.82 | I-155 | 1.37 | I-213 | 1.76 | I-271 | 2.23 | I-329 | 7.67 | I-387 | 5.49 | I-445 | 2.78 |
| I-40 | 1.90 | I-98 | 2.18 | I-156 | 1.30 | I-214 | 1.89 | I-272 | 2.11 | I-330 | 40.6 | I-388 | 1.53 | I-446 | 2.12 |
| I-41 | 1.11 | I-99 | 0.81 | I-157 | 1.22 | I-215 | 2.01 | I-273 | 2.25 | I-331 | 12.3 | I-389 | 1.79 | I-447 | 2.37 |
| I-42 | 23.4 | I-100 | 1.98 | I-158 | 0.88 | I-216 | 1.93 | I-274 | 2.11 | I-332 | 7.39 | I-390 | 1.84 | I-448 | 2.31 |
| I-43 | 2.00 | I-101 | 2.15 | I-159 | 1.07 | I-217 | 1.95 | I-275 | 2.11 | I-333 | 11.0 | I-391 | 3.94 | I-449 | 2.19 |
| I-44 | 1.09 | I-102 | 3.83 | I-160 | 0.96 | I-218 | 1.95 | I-276 | 2.16 | I-334 | 1.91 | I-392 | 2.40 | I-450 | 2.68 |
| I-45 | 2.57 | I-103 | 3.75 | I-161 | 0.94 | I-219 | 2.06 | I-277 | 1.94 | I-335 | 1.81 | I-393 | 14.5 | I-451 | 9.46 |
| I-46 | 1.86 | I-104 | 4.02 | I-162 | 0.96 | I-220 | 2.08 | I-278 | 1.83 | I-336 | 2.13 | I-394 | 1.64 | I-452 | 2.60 |
| I-47 | 5.41 | I-105 | 2.42 | I-163 | 1.10 | I-221 | 2.11 | I-279 | 1.60 | I-337 | 2.03 | I-395 | 1.86 | I-453 | 2.63 |
| I-48 | 2.01 | I-106 | 2.57 | I-164 | 2.07 | I-222 | 2.90 | I-280 | 3.24 | I-338 | 4.70 | I-396 | 2.76 | I-454 | 5.51 |
| I-49 | 2.50 | I-107 | 2.37 | I-165 | 3.21 | I-223 | 1.97 | I-281 | 2.95 | I-339 | 1.83 | I-397 | 2.64 | I-455 | 2.38 |
| I-50 | 1.13 | I-108 | 1.12 | I-166 | 8.83 | I-224 | 1.96 | I-282 | 4.04 | I-340 | 5.60 | I-398 | 1.67 | I-456 | 13.6 |
| I-51 | 2.65 | I-109 | 1.20 | I-167 | 7.10 | I-225 | 2.31 | I-283 | 2.28 | I-341 | 1.89 | I-399 | 1.94 | I-457 | 5.63 |
| I-52 | 2.34 | I-110 | 1.67 | I-168 | 1.83 | I-226 | 4.48 | I-284 | 2.98 | I-342 | 2.06 | I-400 | 1.69 | I-458 | 51.0 |
| I-53 | 0.83 | I-111 | 1.48 | I-169 | 0.88 | I-227 | 3.26 | I-285 | 7.76 | I-343 | 15.9 | I-401 | 1.77 | I-459 | 18.4 |
| I-54 | 1.24 | I-112 | 1.07 | I-170 | 0.91 | I-228 | 3.98 | I-286 | 2.06 | I-344 | 2.83 | I-402 | 1.89 | I-460 | 3.58 |
| I-55 | 7.34 | I-113 | 1.84 | I-171 | 0.94 | I-229 | 3.70 | I-287 | 2.02 | I-345 | 1.82 | I-403 | 2.30 | I-461 | 7.09 |
| I-56 | 1.21 | I-114 | 3.08 | I-172 | 1.02 | I-230 | 6.34 | I-288 | 2.19 | I-346 | 1.98 | I-404 | 1.88 | I-462 | 3.33 |

**[Table 115]**

| No. | IC50 (nM) | No. | IC50 (nM) | No. | IC50 (nM) | No. | IC50 (nM) | No. | IC50 (nM) | No. | IC50 (nM) | No. | IC50 (nM) | No. | IC50 (nM) |
|---|---|---|---|---|---|---|---|---|---|---|---|---|---|---|---|
| I-463 | 5.13 | I-521 | 2.84 | I-579 | 4.38 | I-637 | 4.45 | I-695 | 4.07 | I-753 | 2.39 | I-811 | 0.81 | I-870 | 9.33 |
| I-464 | 2.52 | I-522 | 13.6 | I-580 | 3.23 | I-638 | 6.95 | I-696 | 12.2 | I-754 | 2.49 | I-812 | 0.97 | I-871 | 4.93 |
| I-465 | 2.97 | I-523 | 9.44 | I-581 | 4.02 | I-639 | 3.93 | I-697 | 2.90 | I-755 | 2.69 | I-813 | 1.06 | I-872 | 4.99 |
| I-466 | 4.28 | I-524 | 5.03 | I-582 | 9.36 | I-640 | 5.07 | I-486 | 3.80 | I-756 | 3.46 | I-814 | 0.75 | I-873 | 8.43 |
| I-467 | 2.42 | I-525 | 5.75 | I-583 | 1.96 | I-641 | 5.26 | I-699 | 10.3 | I-757 | 3.02 | I-815 | 1.63 | I-874 | 5.12 |
| I-468 | 5.60 | I-526 | 6.47 | I-584 | 2.30 | I-642 | 4.65 | I-700 | 2.02 | I-758 | 1.62 | I-816 | 5.78 | I-875 | 6.78 |
| I-469 | 5.16 | I-527 | 1.99 | I-585 | 11.1 | I-486 | 3.57 | I-7011 | 1.49 | I-759 | 2.55 | I-817 | 1.56 | I-876 | 7.13 |
| I-470 | 2.67 | I-528 | 2.36 | I-586 | 2.72 | I-644 | 6.68 | I-702 | 2.15 | I-760 | 3.38 | I-818 | 0.64 | I-877 | 2.12 |
| I-471 | 4.69 | I-529 | 4.98 | I-587 | 1.71 | I-645 | 2.51 | I-703 | 4.60 | I-761 | 6.76 | I-819 | 0.72 | I-878 | 2.94 |
| I-472 | 2.27 | I-530 | 8.31 | I-588 | 3.29 | I-646 | 2.92 | I-7041 | 2.39 | I-762 | 6.23 | I-820 | 0.94 | I-879 | 3.78 |
| I-473 | 2.71 | I-531 | 6.98 | I-589 | 3.48 | I-647 | 2.59 | I-705 | 4.87 | I-763 | 3.07 | I-821 | 2.24 | I-880 | 4.70 |
| I-474 | 2.14 | I-532 | 4.14 | I-590 | 2.82 | I-648 | 2.31 | I-706 | 6.62 | I-764 | 2.81 | I-822 | 2.08 | I-881 | 5.89 |
| I-475 | 2.42 | I-533 | 10.6 | I-591 | 3.16 | I-649 | 2.25 | I-707 | 12.1 | I-765 | 2.96 | I-824 | 1.01 | I-882 | 2.28 |
| I-476 | 1.99 | I-534 | 4.11 | I-592 | 3.19 | I-650 | 4.31 | I-708 | 1.65 | I-766 | 3.28 | I-825 | 1.02 | I-883 | 2.15 |
| I-477 | 2.00 | I-535 | 43.1 | I-593 | 1.71 | I-651 | 2.00 | I-709 | 7.20 | I-767 | 3.29 | I-826 | 1.18 | I-884 | 2.03 |
| I-478 | 2.25 | I-536 | 2.10 | I-594 | 3.95 | I-652 | 2.46 | I-710 | 4.26 | I-768 | 2.95 | I-827 | 0.81 | I-885 | 3.48 |
| I-479 | 2.49 | I-537 | 5.50 | I-595 | 2.17 | I-653 | 3.69 | I-711 | 8.10 | I-769 | 5.98 | I-828 | 0.96 | I-886 | 3.68 |
| I-480 | 2.36 | I-538 | 2.86 | I-596 | 1.54 | I-654 | 8.75 | I-712 | 2.02 | I-770 | 5.56 | I-829 | 1.20 | I-887 | 2.85 |
| I-481 | 3.72 | I-539 | 2.91 | I-597 | 7.37 | I-655 | 51.0 | I-713 | 1.69 | I-771 | 2.00 | I-830 | 0.83 | I-888 | 2.10 |
| I-482 | 2.27 | I-540 | 27.8 | I-598 | 2.12 | I-656 | 6.57 | I-714 | 4.50 | I-772 | 21.0 | I-831 | 1.49 | I-889 | 1.49 |
| I-483 | 2.75 | I-541 | 2.34 | I-599 | 2.46 | I-657 | 8.14 | I-715 | 5.31 | I-773 | 51.0 | I-832 | 1.16 | I-890 | 1.74 |
| I-484 | 2.43 | I-542 | 1.34 | I-600 | 5.07 | I-658 | 13.7 | I-716 | 3.95 | I-774 | 6.26 | I-833 | 1.54 | I-891 | 2.88 |
| I-485 | 2.28 | I-543 | 2.14 | I-601 | 3.26 | I-659 | 9.51 | I-717 | 2.77 | I-775 | 3.64 | I-834 | 1.15 | I-892 | 4.06 |
| I-486 | 4.59 | I-544 | 3.65 | I-602 | 3.67 | I-660 | 2.12 | I-718 | 3.97 | I-776 | 6.40 | I-835 | 2.81 | I-893 | 5.83 |
| I-487 | 3.03 | I-545 | 1.85 | I-603 | 3.65 | I-661 | 2.35 | I-719 | 1.90 | I-777 | 51.0 | I-836 | 1.76 | I-894 | 20.4 |
| I-488 | 2.20 | I-546 | 1.86 | I-604 | 2.19 | I-662 | 2.51 | I-720 | 2.42 | I-778 | 13.8 | I-837 | 0.82 | I-895 | 6.59 |
| I-489 | 1.81 | I-547 | 2.28 | I-605 | 1.96 | I-663 | 2.80 | I-721 | 2.24 | I-779 | 2.74 | I-838 | 0.76 | I-896 | 16.7 |
| I-490 | 2.01 | I-548 | 1.49 | I-606 | 2.25 | I-664 | 1.86 | I-722 | 5.07 | I-780 | 1.64 | I-839 | 0.74 | I-897 | 2.46 |
| I-491 | 1.92 | I-549 | 1.55 | I-607 | 1.80 | I-665 | 51.0 | I-723 | 1.65 | I-781 | 1.02 | I-840 | 0.71 | I-898 | 1.78 |
| I-492 | 1.84 | I-550 | 1.75 | I-608 | 2.01 | I-666 | 4.70 | I-724 | 1.83 | I-782 | 1.02 | I-841 | 2.21 | I-899 | 1.46 |
| I-493 | 2.43 | 1-551 | 1.34 | I-609 | 1.90 | I-667 | 7.95 | I-725 | 2.17 | I-783 | 6.62 | I-842 | 0.69 | I-900 | 2.90 |
| I-494 | 1.46 | I-552 | 1.41 | I-610 | 2.16 | I-668 | 7.06 | I-726 | 2.82 | I-784 | 7.30 | I-843 | 1.33 | I-901 | 2.34 |
| I-495 | 1.48 | I-553 | 1.92 | I-611 | 1.95 | I-669 | 8.54 | I-727 | 3.38 | I-785 | 2.46 | I-844 | 1.01 | I-902 | 6.83 |
| I-496 | 1.81 | I-554 | 1.77 | I-612 | 2.11 | I-670 | 2.67 | I-728 | 2.96 | I-786 | 5.25 | I-845 | 0.85 | I-903 | 2.23 |
| I-497 | 1.96 | I-555 | 1.68 | I-613 | 2.55 | I-671 | 34.8 | I-729 | 1.64 | I-787 | 1.97 | I-846 | 1.20 | I-904 | 3.69 |
| I-498 | 1.83 | I-556 | 5.45 | I-614 | 2.91 | I-672 | 2.05 | I-730 | 2.84 | I-788 | 2.11 | I-847 | 3.38 | I-905 | 51.0 |
| I-499 | 1.73 | I-557 | 1.67 | I-615 | 3.37 | I-673 | 2.81 | I-731 | 1.96 | I-789 | 1.82 | I-848 | 0.89 | I-906 | 51.0 |
| I-500 | 1.78 | I-558 | 4.99 | I-616 | 4.20 | I-674 | 4.89 | I-732 | 3.26 | I-790 | 1.76 | I-849 | 0.78 | I-907 | 1.27 |
| I-501 | 1.82 | I-559 | 1.87 | I-617 | 3.13 | I-675 | 13.4 | I-733 | 6.24 | I-791 | 51.0 | I-850 | 1.03 | I-908 | 1.21 |
| I-502 | 1.87 | I-560 | 1.65 | I-618 | 6.83 | I-676 | 2.42 | I-734 | 15.4 | I-792 | 25.0 | 1-851 | 2.57 | I-909 | 3.25 |
| I-503 | 1.68 | I-561 | 1.35 | I-619 | 2.48 | I-677 | 2.32 | I-735 | 1.56 | I-793 | 17.7 | I-852 | 1.99 | I-910 | 7.84 |
| I-504 | 1.93 | I-562 | 1.98 | I-620 | 2.22 | I-486 | 5.48 | I-736 | 1.73 | I-794 | 38.4 | I-853 | 1.98 | | |
| I-505 | 1.55 | I-563 | 2.13 | I-621 | 1.84 | I-679 | 2.82 | I-737 | 1.99 | I-795 | 0.73 | I-854 | 3.16 | | |
| I-506 | 1.70 | I-564 | 14.2 | I-622 | 3.71 | I-680 | 2.10 | I-738 | 1.80 | I-796 | 1.02 | I-855 | 2.66 | | |
| I-507 | 1.85 | I-565 | 1.75 | I-623 | 4.24 | I-681 | 7.97 | I-739 | 1.93 | I-797 | 0.85 | I-856 | 2.64 | | |
| I-508 | 8.48 | I-566 | 1.61 | I-624 | 3.77 | I-682 | 4.37 | I-740 | 1.60 | I-798 | 0.99 | I-857 | 3.41 | | |
| I-509 | 1.96 | I-567 | 3.12 | I-625 | 5.07 | I-683 | 5.74 | I-741 | 2.07 | I-799 | 3.35 | I-558 | 4.23 | | |
| I-510 | 10.6 | I-568 | 1.54 | I-626 | 4.58 | I-684 | 2.83 | I-742 | 2.08 | I-800 | 2.22 | I-859 | 3.41 | | |
| I-511 | 2.14 | I-569 | 2.22 | I-627 | 4.06 | I-685 | 2.98 | I-743 | 2.32 | I-801 | 0.84 | I-860 | 3.56 | | |
| I-512 | 1.63 | I-570 | 1.87 | I-628 | 5.03 | I-686 | 3.60 | I-744 | 2.14 | I-802 | 1.22 | I-861 | 2.80 | | |
| I-513 | 1.76 | I-571 | 22.2 | I-629 | 2.35 | I-687 | 2.48 | I-745 | 3.39 | I-803 | 1.12 | I-862 | 2.61 | | |
| I-514 | 6.21 | I-572 | 30.0 | I-630 | 2.11 | I-688 | 2.59 | I-746 | 2.81 | I-804 | 1.69 | I-863 | 2.55 | | |
| I-515 | 2.79 | I-573 | 1.39 | I-631 | 1.93 | I-689 | 2.01 | I-747 | 4.35 | I-805 | 3.53 | I-864 | 2.25 | | |
| I-516 | 2.29 | I-574 | 2.93 | I-632 | 3.81 | I-690 | 7.70 | I-748 | 2.39 | I-806 | 1.06 | I-865 | 2.21 | | |
| I-517 | 2.56 | I-575 | 8.32 | I-633 | 2.48 | I-691 | 6.44 | I-749 | 2.33 | I-807 | 0.86 | I-866 | 2.57 | | |
| I-518 | 5.63 | I-576 | 4.95 | I-634 | 2.26 | I-692 | 26.1 | I-750 | 4.60 | I-808 | 2.67 | I-867 | 2.64 | | |
| I-519 | 8.93 | I-577 | 6.47 | I-635 | 2.10 | I-693 | 3.30 | I-751 | 15.7 | I-809 | 1.30 | I-868 | 4.30 | | |
| I-520 | 5.26 | I-578 | 2.39 | I-636 | 4.37 | I-694 | 2.27 | I-752 | 13.3 | I-810 | 1.03 | I-869 | 6.45 | | |

**[Table 116]**

| No. | rat_CLt (mL/min/kg) | rat_fu (%) | No. | rat_CLt (mL/min/kg) | rat_fu (%) | No. | rat_CLt (mL/min/kg) | rat_fu (%) | No. | rat_CLt (mL/min/kg) | rat_fu (%) |
|---|---|---|---|---|---|---|---|---|---|---|---|
| I-1 | 0.542 | <0.1 | I-181 | 0.117 | <0.1 | I-371 | 4.69 | | I-528 | 4.05 | |
| I-2 | 5.11 | <0.1 | I-182 | 3.84 | <0.1 | I-372 | 0.868 | <0.1 | I-530 | 1.57 | |
| I-6 | 1.22 | <0.1 | I-188 | 2.47 | <0.1 | I-374 | 3.94 | <0.1 | I-531 | 0.0543 | |
| I-7 | 0.238 | <0.1 | I-190 | 1.4 | <0.1 | I-376 | 0.485 | <0.1 | I-532 | 0.551 | |
| I-10-2 | 0.092 | <0.1 | I-195 | 6.74 | <0.1 | I-378 | 0.173 | <0.1 | I-537 | 0.328 | |
| I-12 | 2.9 | <0.1 | I-197 | 3.81 | <0.1 | I-379 | 0.173 | <0.1 | I-538 | 0.598 | |
| I-13 | 1.09 | <0.1 | I-205 | 1.68 | <0.1 | I-380 | 0.702 | <0.1 | I-539 | 0.156 | |
| I-15 | 4.86 | <0.1 | I-206 | 0.162 | <0.1 | I-384 | 0.253 | <0.1 | I-540 | 1.5 | |
| I-19 | 1.7 | <0.1 | I-211 | 3.07 | <0.1 | I-386 | 4.96 | <0.1 | I-541 | 0.195 | |
| I-29 | 1.54 | <0.1 | I-212 | 3.97 | <0.1 | I-389 | 14 | <0.1 | I-545 | 1.15 | |
| I-30 | 1.52 | <0.1 | I-214 | 0.297 | | I-391 | 10.3 | <0.1 | I-550 | 5.29 | |
| I-37 | 0.784 | <0.1 | I-228 | 0.144 | <0.1 | I-396 | 0.0572 | <0.1 | I-552 | 0.985 | |
| I-39 | 0.363 | <0.1 | I-231 | 1.02 | <0.1 | I-399 | 0.487 | | I-555 | 5.98 | |
| I-43 | 3.19 | <0.1 | I-234 | 1.49 | <0.1 | I-405 | 3.27 | <0.1 | I-560 | 4.5 | |
| I-44 | 0.535 | <0.1 | I-235 | 4.05 | <0.1 | I-406 | 4.61 | <0.1 | 1-561 | 4.48 | |
| I-45 | 1.71 | <0.1 | I-236 | 0.763 | <0.1 | I-407 | 0.31 | | I-563 | 0.883 | |
| I-47 | 3.51 | <0.1 | I-237 | 1.1 | <0.1 | I-409 | 0.873 | | I-566 | 0.727 | |
| I-48 | 2.28 | <0.1 | I-238 | 3.05 | <0.1 | I-410 | 1.07 | | I-567 | 2.81 | |
| I-49 | 2.23 | <0.1 | I-240 | 4.5 | <0.1 | I-415 | 0.66 | <0.1 | I-574 | 1.01 | |
| I-51 | 1.47 | <0.1 | I-241 | 0.404 | <0.1 | I-418 | 1.49 | <0.1 | I-575 | 0.267 | |
| I-55 | 0.752 | <0.1 | I-242 | 0.408 | <0.1 | I-434 | 1.73 | | I-578 | 3.16 | |
| I-74 | 3.88 | <0.1 | I-243 | 0.477 | <0.1 | I-436 | 1.72 | | I-579 | 1.66 | |
| I-82 | 3.15 | <0.1 | I-247 | 0.148 | <0.1 | I-440 | 0.871 | | I-580 | 0.0683 | |
| I-82 | 0.285 | <0.1 | I-248 | 2.47 | <0.1 | I-448 | 5.28 | | I-581 | 0.204 | |
| I-83 | 0.362 | <0.1 | I-252 | 0.113 | <0.1 | I-449 | 11.1 | | I-582 | 0.179 | |
| I-84 | 0.117 | <0.1 | I-253 | 4.65 | <0.1 | I-450 | 3.7 | | I-583 | 0.107 | |
| I-85 | 5.54 | <0.1 | I-255 | 0.455 | <0.1 | I-451 | 0.211 | | I-584 | 0.77 | |
| I-86 | 1.09 | <0.1 | I-256 | 3.83 | <0.1 | I-453 | 0.593 | | I-585 | 0.0436 | |
| I-88 | 2.5 | <0.1 | I-260 | 0.801 | <0.1 | I-454 | 4.02 | | I-586 | 2.51 | |
| I-93 | 2.22 | <0.1 | I-261 | 2.78 | <0.1 | I-455 | 0.236 | | I-588 | 0.291 | |
| I-94 | 2.02 | <0.1 | I-262 | 0.343 | <0.1 | I-465 | 0.675 | | I-589 | 0.311 | |
| I-98 | 0.933 | <0.1 | I-265 | 4.01 | <0.1 | I-466 | 1.75 | | I-594 | 0.106 | |
| I-99 | 0.576 | <0.1 | I-267 | 5.01 | <0.1 | I-467 | 2.36 | | I-596 | 0.213 | |
| I-101 | 0.985 | <0.1 | I-268 | 0.0553 | <0.1 | I-471 | 2.17 | | I-598 | 0.532 | |
| I-102 | 4.24 | <0.1 | I-271 | 2.42 | <0.1 | I-473 | 1.22 | | I-599 | 0.479 | |
| I-104 | 0.07 | <0.1 | I-272 | 0.715 | <0.1 | I-474 | 1.28 | | I-600 | 0.104 | |
| I-105 | 2.29 | <0.1 | I-274 | 0.659 | <0.1 | I-475 | 2.28 | | I-601 | 0.164 | |
| I-106 | 3.49 | <0.1 | I-275 | 2.12 | <0.1 | I-480 | 0.0997 | | I-602 | 0.0667 | |
| I-113 | 1.18 | <0.1 | I-277 | 0.512 | <0.1 | I-481 | 3.29 | | I-603 | 5.83 | |
| I-119 | 2.29 | <0.1 | I-287 | 2.73 | | I-483 | 2.41 | | I-606 | 2.2 | |
| I-120 | 1.48 | <0.1 | I-292 | 0.166 | <0.1 | I-484 | 1.58 | | I-607 | 0.797 | |
| I-125 | 3.89 | | I-297 | 3.54 | <0.1 | I-485 | 0.45 | | I-608 | 0.826 | |
| I-127 | 7.74 | <0.1 | I-304 | 0.41 | <0.1 | I-487 | 3.16 | | I-613 | 0.0573 | |
| I-131 | 2.27 | <0.1 | I-305 | 1.55 | | I-488 | 0.501 | | I-614 | 0.146 | |
| I-132 | 0.376 | <0.1 | I-334 | 3.87 | <0.1 | I-490 | 0.88 | | I-617 | 0.098 | |
| I-134 | 3.92 | <0.1 | I-338 | 5.7 | <0.1 | I-491 | 3.32 | | I-618 | 1.78 | |
| I-137 | 1.5 | <0.1 | I-343 | 3.31 | <0.1 | I-492 | 17.3 | | I-623 | 1.83 | |
| I-138 | 1.6 | <0.1 | I-347 | 5.18 | <0.1 | I-507 | 1.61 | | I-625 | 0.0688 | |
| I-139 | 0.286 | <0.1 | I-348 | 0.585 | <0.1 | I-509 | 0.377 | | I-627 | 0.0474 | |
| I-140 | 0.111 | <0.1 | I-349 | 0.71 | <0.1 | I-510 | 1.08 | | I-631 | 0.699 | |
| I-145 | 1.29 | <0.1 | I-351 | 3.08 | <0.1 | I-513 | 3.23 | | I-632 | 0.176 | |
| I-146 | 0.584 | <0.1 | I-352 | 3.26 | <0.1 | I-514 | 3.01 | | I-636 | 0.372 | |
| I-147 | 1.68 | <0.1 | I-353 | 4.97 | <0.1 | I-519 | 0.429 | | I-637 | 0.503 | |
| I-148 | 2.5 | <0.1 | I-354 | 0.0682 | <0.1 | I-520 | 0.0285 | | I-638 | 0.802 | |
| I-165 | 1.08 | <0.1 | I-357 | 1.55 | <0.1 | I-522 | 0.144 | | I-648 | 0.783 | |
| I-166 | 0.194 | <0.1 | I-362 | 4.62 | <0.1 | I-524 | 0.23 | | I-649 | 0.622 | |
| I-168 | 5.68 | <0.1 | I-363 | 3.39 | <0.1 | I-525 | 0.127 | | I-651 | 0.536 | |
| I-175 | 4.44 | <0.1 | I-365 | 0.119 | <0.1 | I-526 | 0.632 | | I-652 | 0.641 | |

**[Table 117]**

| No. | rat_CLt (mL/min/kg) | rat_fu (%) | No. | rat_CLt (mL/min/kg) | rat_fu (%) | No. | rat_CLt (mL/min/kg) | rat_fu (%) | No. | rat_CLt (mL/min/kg) | rat_fu (%) |
|---|---|---|---|---|---|---|---|---|---|---|---|
| I-662 | 1.11 | | I-722 | 0.0366 | | I-825 | 5.22 | <0.1 | I-860 | 0.0981 | |
| I-664 | 1.5 | | I-726 | 0.212 | | I-826 | 0.823 | <0.1 | I-861 | 0.0804 | |
| I-666 | 0.668 | | I-727 | 0.0528 | | I-827 | 4.21 | <0.1 | I-862 | 0.14 | |
| I-674 | 0.0392 | | I-736 | 0.0528 | | I-832 | 4.09 | <0.1 | I-867 | 0.372 | |
| I-676 | 0.145 | | I-742 | 5.03 | | I-835 | 1.63 | <0.1 | I-869 | 1.15 | |
| I-679 | 1.65 | | I-780 | 2.23 | <0.1 | I-836 | 4.31 | <0.1 | I-877 | 2.31 | |
| I-680 | 2.77 | | I-781 | 5.37 | <0.1 | I-841 | 2.38 | <0.1 | I-882 | 0.26 | |
| I-689 | 0.385 | | I-782 | 1.16 | <0.1 | I-847 | 6 | <0.1 | I-890 | 0.652 | |
| I-693 | 0.247 | | I-786 | 4.34 | <0.1 | I-851 | 0.298 | <0.1 | I-903 | 0.0436 | <0.1 |
| I-701 | 2.71 | | I-788 | 3.87 | <0.1 | I-852 | 2.25 | | I-907 | 0.73 | <0.1 |
| I-704 | 0.724 | | I-798 | 4.54 | <0.1 | I-856 | 0.0831 | | I-908 | 0.63 | <0.1 |
| I-714 | 0.0783 | | I-799 | 0.324 | <0.1 | I-857 | 0.132 | | | | |
| I-719 | 0.066 | | I-807 | 0.61 | <0.1 | I-858 | 0.142 | | | | |
| I-720 | 0.0677 | | I-808 | 1.86 | <0.1 | I-859 | 0.2 | | | | |

### (Reference example)

The results of measurements of HIV protease inhibitory activity, serum protein binding rate and total body clearance of Darunavir according to the description of Test Example 1, 2 and 3.
Enzyme inhibitory activity: 1.05nM
Serum protein binding rate: 9.71%
Total body clearance: 35.5mL/min/kg

The compounds of the present invention are having excellent long acting performance in blood without decreasing drug efficacy drastically in comparison with Darunavir from the above results.

Further useful for medicine can be examined by the following tests etc.

### Test Example 4: CYP inhibition test

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenedine hydroxylation (CYP3A4) as typical substrate metabolism reactions of human main five CYP enzyme forms (CYP1A2, 2C9, 2C19, 2D6, 3A4), an inhibitory degree of each metabolite production amount by a compound of the present invention was assessed.

The reaction conditions were as follows: substrate, 0.5 µmol/L ethoxyresorufin (CYP1A2), 100 µmol/L tolbutamide (CYP2C9), 50 µmol/L S-mephenytoinmephenitoin (CYP2C19), 5 µmol/L dextromethorphan (CYP2D6), 1 µmol/L terfenedine (CYP3A4); reaction time, 15 minutes; reaction temperature, 37°C; enzyme, pooled human hepatic microsome 0.2 mg protein/mL; concentration of a compound of the present invention, 1, 5, 10, 20 µmol/L (four points).

Each five kinds of substrates, human hepatic microsome, or a compound of the present invention in 50 mmol/L Hepes buffer as a reaction solution was added to a 96-well plate at the composition as described above, NADPH, as a cofactor was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent multilabel counter and toltributamide hydroxide (CYP2C9P metabolite), mephenytoin 4' hydroxide (CYP2C19 metabolite), dextromethorphan (CYP2D6 metabolite), and terfenadine alcohol (CYP3A4 metabolite) were quantified by LC/MS/MS.

Addition of only DMSO being a solvent dissolving a compound of the present invention to a reaction system was adopted as a control (100%), remaining activity (%) was calculated at each concentration of a compound of the present invention added as the solution and IC50 was calculated by reverse presumption by a logistic model using a concentration and an inhibition rate.

### Test Example 5: Metabolism Stability test

Using commercially available pooled human hepatic microsomes, a compound of the present invention was reacted for a constant time, and a remaining rate was calculated by comparing a reacted sample and an unreacted sample, thereby, a degree of metabolism in liver was assessed.

A reaction was performed at 37 °C for 60 minutes or 120 minutes in a CO2 incubator in 30 µL of William's Medium E containing 0.5 mg protein/mL of human liver microsomes (final concentration 1x10⁶ cells/mL). After the reaction, 30 µL of the reaction solution was added to 120 µL of a methanol/acetonitrile = 1/1 (v/v), mixed and centrifuged at 3000 rpm for 15 minutes. The compound of the present invention in the supernatant was quantified by LC/MS/MS, and a remaining amount of the compound of the present invention after the reaction was calculated, letting a compound amount at 0 minute reaction time to be 100%.

### Test Example 6: CYP3A4 fluorescent MBI test

The CYP3A4 fluorescent MBI test is a test of investigating enhancement of CYP3A4 inhibition of a compound of the present invention by a metabolism reaction, and the test was performed using, as CYP3A4 enzyme expressed in Escherichia coli and employing, as an index, a reaction in which 7-benzyloxytrifluoromethylcoumarin (7-BFC) is debenzylated by the CYP3A4 enzyme to produce a metabolite, 7-hydroxytrifluoromethylcoumarin (HFC) emitting fluorescent light.

The reaction conditions were as follows: substrate, 5.6 µmol/L 7-BFC; pre-reaction time, 0 or 30 minutes; reaction time, 15 minutes; reaction temperature, 25°C (room temperature); CYP3A4 content (expressed in Escherichia coli), at pre-reaction 62.5 pmol/mL, at reaction 6.25 pmol/mL (at 10-fold dilution); test drug concentration of a compound of the present invention, 0.625, 1.25, 2.5, 5, 10, 20 µmol/L (six points).

An enzyme in a K-Pi buffer (pH 7.4) and a solution of a compound of the present invention as a pre-reaction solution were added to a 96-well plate at the above composition of the pre-reaction, a part of it was transferred to another 96-well plate so that it was 1/10 diluted with a substrate and a K-Pi buffer, NADPH as a co-factor was added to initiate a reaction as an index (without preincubation) and, after a predetermined time of a reaction, acetonitrile/0.5 mol/L Tris (trishydroxyaminomethane) = 4/1 (V/V) was added to stop the reaction. In addition, NADPH was added to a remaining preincubation solution to initiate a preincubation (with preincubation) and, after a predetermined time of a preincubation, a part was transferred to another plate so that it was 1/10 diluted with a substrate and a K-Pi buffer to initiate a reaction as an index. After a predetermined time of a reaction, acetonitrile/0.5 mol/L Tris (trishydroxyaminomethane) = 4/1 (V/V) was added to stop the reaction. For the plate on which each index reaction had been performed, a fluorescent value of 7-HFC which is a metabolite was measured with a fluorescent plate reader. (Ex = 420 nm, Em = 535 nm).

Addition of only DMSO which is a solvent dissolving a compound of the present invention to a reaction system was adopted as a control (100 %), remaining activity (%) was calculated at each concentration of a compound of the present invention added as the solution, and IC₅₀ was calculated by reverse-presumption by a logistic model using a concentration and an inhibition rate. When a difference between IC₅₀ values is 5 µmol/L or more, this was defined as (+) and, when the difference is 3 µmol/L or less, this was defined as (-).

### Test Example 6-2: CYP3A4(MDZ) MBI test

CYP3A4(MDZ) MBI test is a test of investigating mechanism based inhibition (MBI) ability on CYP3A4 inhibition of a compound by enhancement of a metabolism reaction. CYP3A4 inhibition is evaluated using 1-hydroxylation reaction of midazolam (MDZ) by pooled human liver microsomes as an index.

The reaction conditions were as follows: substrate, 10 µmol/L MDZ; pre-reaction time, 0 or 30 minutes; substrate reaction time, 2 minutes; reaction temperature, 37 °C; protein content of pooled human liver microsomes, at pre-reaction time 0.5 mg/mL, at reaction time 0.05 pmg/mL (at 10-fold dilution); concentrations of the compound of the present invention, 1, 5, 10, 20 µmol/L (four points).

Pooled human liver microsomes in a K-Pi buffer (pH 7.4) and a compound of the present invention solution as a pre-reaction solution were added to a 96-well plate at the composition of the pre-reaction. A part of pre-reaction solution was transferred to another 96-well plate, and 1/10 diluted by a substrate in a K-Pi buffer. NADPH as a co-factor was added to initiate a reaction as an index (without preincubation). After a predetermined time of a reaction, methanol/acetonitrile=1/1 (v/v) solution was added to stop the reaction. On the other hand, NADPH was also added to a remaining pre-reaction solution in order to initiate a preincubation (with preincubation). After a predetermined time of a preincubation, a part was transferred to another 96-well plate, and 1/10 diluted by a substrate in a K-Pi buffer in order to initiate a reaction as an index. After a predetermined time of a reaction, methanol/acetonitrile=1/1 (v/v) solution was added to stop the reaction. After centrifuged at 3000rpm for 15 minutes, 1-hydroxymidazolam in the supernatant was quantified by LC/MS/MS.

The sample adding DMSO to a reaction system instead of compound of the present invention solution was adopted as a control (100 %) because DMSO was used as a solvent to dissolve a compound of the present invention. Remaining activity (%) was calculated at each concentration of the compound of the present invention added as the solution, and IC50 value was calculated by reverse-presumption by a logistic model using a concentration and an inhibition rate. Shifted IC value was calculated as "IC of preincubation at 0 min/ IC of preincubation at 30min". When a shifted IC was 1.5 or more, this was defined as (+). When a shifted IC was 1.0 or less, this was defined as (-).

### Test Example 7: Fluctuation Ames test

Mutagenicity of compounds of the present invention was evaluated. 20 µL of freezing-stored rat typhoid bacillus (Salmonella typhimurium TA98 strain, TA100 strain) was inoculated on 10 mL of a liquid nutrient medium (2.5% Oxoid nutrient broth No.2), and this was cultured before shaking at 37°C for 10 hours. 9 mL of a bacterial solution of the TA98 strain was centrifuged (2000 x g, 10 minutes) to remove a culturing solution. The bacteria was suspended in 9 mL of a Micro F buffer (K₂HPO₄: 3.5 g/L, KH₂PO₄: 1 g/L, (NH₄)₂SO₄: 1 g/L, trisodium citrate dehydrate: 0.25 g/L, MgSO₄ · 7H₂O: 0.1 g/L), the suspension was added to 110 mL of an Exposure medium (Micro F buffer containing Biotin: 8 µg/mL, histidine: 0.2 µg/mL, glucose: 8 mg/mL). The TA100 strain was added to 120 mL of the Exposure medium relative to 3.16 mL of the bacterial solution to prepare a test bacterial solution. Each 12 µL of DMSO solution of a compound of the present invention (several stage dilution from maximum dose 50 mg/mL at 2 to 3 fold ratio), DMSO as a negative control, and 50 µg/mL of 4-nitroquinoline-1-oxide DMSO solution for the TA98 strain, 0.25 µg/mL of 2-(2-furyl)-3-(5-nitro-2-furyl)acrylamide DMSO solution for the TA100 strain under the non-metabolism activating condition, 40 µg/mL of 2-aminoanthracene DMSO solution for the TA98 strain, 20 µg/mL of 2-aminoanthracene DMSO solution for the TA100 strain under the metabolism activating condition as a positive control, and 588 µL of the test bacterial solution (a mixed solution of 498 µl of the test bacterial solution and 90 µL of S9 mix under the metabolism activating condition) were mixed, and this was shaking-cultured at 37°C for 90 minutes. 460 µL of the bacterial solution exposed to a compound of the present invention was mixed with 2300 µL of an Indicator medium (Micro F buffer containing biotin: 8 µg/mL, histidine: 0.2 µg/mL, glucose: 8 mg/mL, Bromo Cresol Purple: 37.5 µg/mL), each 50 µL was dispensed into microplate 48 wells/dose, and this was subjected to stationary culturing at 37°C for 3 days. Since a well containing a bacterium which has obtained the proliferation ability by mutation of an amino acid (histidine) synthesizing enzyme gene turns from purple to yellow due to a pH change, the bacterium proliferation well which has turned to yellow in 48 wells per dose is counted, and was assessed by comparing with a negative control group. (-) means that mutagenicity is negative and (+) is positive.

### Test Example 8: hERG test

For the purpose of assessing risk of an electrocardiogram QT interval prolongation of the compound of the present invention, effects of the compound of the present invention on delayed rectifier K+ current (I_{Kr}), which plays an important role in the ventricular repolarization process, was studied using HEK293 cells expressing human ether-a-go-go related gene (hERG) channel.

After a cell was retained at a membrane potential of -80 mV by whole cell patch clamp method using an automated patch clamp system (PatchXpress 7000A, Axon Instruments Inc.), I_{Kr} induced by depolarization pulse stimulation at +40 mV for 2 seconds and, further, repolarization pulse stimulation at -50 mV for 2 seconds, was recorded. After the generated current was stabilized, extracellular solution (NaCl: 135 mmol/L, KCl: 5.4 mmol/L, NaH₂PO₄: 0.3 mmol/L, CaCl₂ · 2H₂O: 1.8 mmol/L, MgCl₂ · 6H₂O: 1 mmol/L, glucose: 10 mmol/L, HEPES (4-(2-hydroxyethyl)-1-piperazineethanesulfonic acid): 10 mmol/L, pH=7.4), in which the compound of the present invention had been dissolved at an objective concentration, was applied to the cell at room temperature for 10 minutes. From the recording I_{Kr}, an absolute value of the tail peak current was measured based on the current value at the resting membrane potential using analysis software (DataXpress ver.1, Molecular Devices Corporation). Further, the % inhibition relative to the tail peak current before application of the compound of the present invention was calculated, and compared with the vehicle-applied group (0.1% dimethyl sulfoxide solution) to assess influence of the compound of the present invention on I_{Kr}.

### Test Example 9: Solubility test

The solubility of the compound of the present invention was determined under 1% DMSO addition conditions. A 10 mmol/L solution of the compound was prepared with DMSO, and 2 µL of the solution of the compound of the present invention was added, respectively, to 198 µL of JP-1 solution (water were added to 2.0 g of sodium chloride and 7.0 mL of hydrochloric acid to reach 1000 mL) and JP-2 solution (1 volume of water were added to 1 volume of the solution which 3.40 g of potassium dihydrogen phosphate and 3.55 g of anhydrous disodium hydrogen phosphate to reach 1000 mL). The mixture was shaked for 1 hour at a room temperature, and the mixture was filtered. The filtrate was ten-fold diluted with methanol/water = 1/1(v/v), and the compound concentration in the filtrate was measured with LC/MS or SPE/MS by the absolute calibration method.

### Test Example 10: Powder solubility test

Appropriate quantity of the compound of the present invention was put in a suitable container and 200 µL of JP-1 solution (water was added to 2.0 g of sodium chloride and 7.0 mL of hydrochloric acid to reach 1000 mL), JP-2 solution (500 mL of water was added to 500 mL of phosphate buffer with a pH of 6.8) or 20 mmol/L sodium taurocholate (TCA)/JP-2 solution (JP-2 solution was added to 1.08 g of TCA to reach 100 mL) was independently added to each container. When total amount was dissolved after adding the test reagent, the compound of the present invention was added appropriately. After sealing and shaking at 37 °C for 1 hour, solution was filtrated and 100µL of methanol was added to 100 µL of each filtrate to dilute twofold. The dilution rate was changed as necessary. After checking that there is no bubble and deposit, the container was sealed and shaken. The compound of the present invention was measured using HPLC by absolute calibration curve method.

### Formulation Example

The following Formulation Examples are only exemplified and not intended to limit the scope of the invention.

### Formulation Example 1: Tablets

The compounds of the present invention, lactose and calcium stearate are mixed. The mixture is crushed, granulated and dried to give a suitable size of granules. Next, calcium stearate is added to the granules, and the mixture is compressed and molded to give tablets.

### Formulation Example 2: Capsules

The compounds of the present invention, lactose and calcium stearate are mixed uniformly to obtain powder medicines in the form of powders or fine granules. The powder medicines are filled into capsule containers to give capsules.

### Formulation Example 3: Granules

The compounds of the present invention, lactose and calcium stearate are mixed uniformly and the mixture is compressed and molded. Then, it is crushed, granulated and sieved to give suitable sizes of granules.

### Formulation Example 4: Orally disintegrated tablets

The compounds of the present invention and crystalline cellulose are mixed, granulated and tablets are made to give orally disintegrated tablets.

### Formulation Example 5: Dry syrups

The compounds of the present invention and lactose are mixed, crushed, granulated and sieved to give suitable sizes of dry syrups.

### Formulation Example 6: Injections

The compounds of the present invention and phosphate buffer are mixed to give injection.

### Formulation Example 7: Infusions

The compounds of the present invention and phosphate buffer are mixed to give injection.

### Formulation Example 8: Inhalations

The compound of the present invention and lactose are mixed and crushed finely to give inhalations.

### Formulation Example 9: Ointments

The compounds of the present invention and petrolatum are mixed to give ointments.

### Formulation Example 10: Patches

The compounds of the present invention and base such as adhesive plaster or the like are mixed to give patches.

### INDUSTRIAL APPLICABILITY

The compound of the present invention has protease inhibitory activity and/or cell growth inhibitory activity against virus especially HIV or resistant virus. Therefore, it is useful for treatment or prevention against a variety of disease relating to protease or virus infections (ex. AIDS). Especially, it is useful for long acting injection of pharmaceutical active ingredient.

## Claims

1. A compound represented by formula (I): 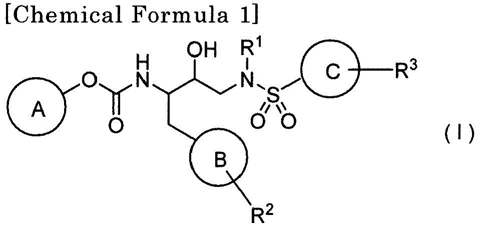
wherein Ring A is a group of formula: 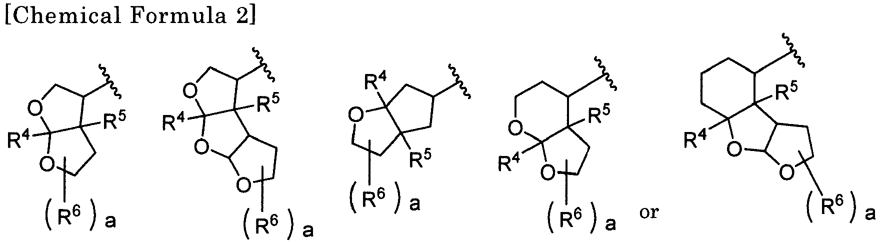
R⁴ is a group represented by the formula: -Y-Z, a hydrogen atom, halogen, hydroxy, carboxy, cyano, nitro, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted amino, substituted or unsubstituted carbamoyl, substituted or unsubstituted sulfamoyl, substituted or unsubstituted aminocarbonyloxyalkyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R⁵ is a hydrogen atom, halogen, hydroxy, carboxy, cyano, nitro, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted carbamoyl, or substituted or unsubstituted sulfamoyl,
R⁶ are each independently halogen, hydroxy, carboxy, formyl, formyloxy, sulfo, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
Said R⁶ may be connected to any connectable positions on ring,
a is an integer of 0 to 7,
Ring B is substituted or unsubstituted aromatic carbocyclyl, or substituted or unsubstituted aromatic heterocyclyl,
Ring C is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R¹ is a group represented by the formula: -Y-Z, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylalkyl, substituted or unsubstituted non-aromatic carbocyclylalkyl, substituted or unsubstituted aromatic heterocyclylalkyl, or substituted or unsubstituted non-aromatic heterocyclylalkyl,
R² and R³ are each independently a group represented by the formula: -Y-Z, or a hydrogen atom,
The group at least one of R¹, R², R³ and R⁴ is a group represented by the formula: -Y-Z,
Y is a bond, or a spacer of any combination selected from the group consisting of -O-, -S-, -NR⁷-, -C(=O)-, -SO-, -SO₂-, -NR⁷-C(=O)-, -C(=O)-NR⁷-, -NR⁷-C(=O)-NR⁷-, -O-C(=O)-NR⁷-, -NR⁷-C(=O)-O-, -SO₂-NR⁷-, -NR⁷-SO₂-, substituted or unsubstituted alkylene, substituted or unsubstituted alkenylene, substituted or unsubstituted alkynylene, substituted or unsubstituted aromatic carbocyclyldiyl, substituted or unsubstituted non-aromatic carbocyclyldiyl, substituted or unsubstituted aromatic heterocyclyldiyl, and substituted or unsubstituted non-aromatic heterocyclyldiyl, provided that Y is excluded in the case that the groups selected from the group consisting of -O-, -S- and -NR⁷- are connected adjacently, and
provided that Y is excluded in the case that the groups selected from the group consisting of -C(=O)-, -SO-, -SO₂-, -NR⁷ -C(=O)-, -C(=O)-NR⁷-, -NR⁷-C(=O)-NR⁷-, -O-C(=O)-NR⁷-, -NR⁷-C(=O)-O-, -SO₂-NR⁷- and -NR⁷-SO₂- are connected adjacently,
R⁷ are each independently a hydrogen atom, hydroxy, carboxy, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, substituted or unsubstituted non-aromatic heterocyclylsulfonyl,
Z is substituted aromatic carbocyclyl, substituted non-aromatic carbocyclyl, substituted aromatic heterocyclyl, or substituted non-aromatic heterocyclyl,
provided that when R⁴ is a hydrogen atom, at least one of substituent of Z is - COOH,
provided that the following compounds are excluded: 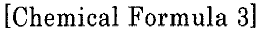 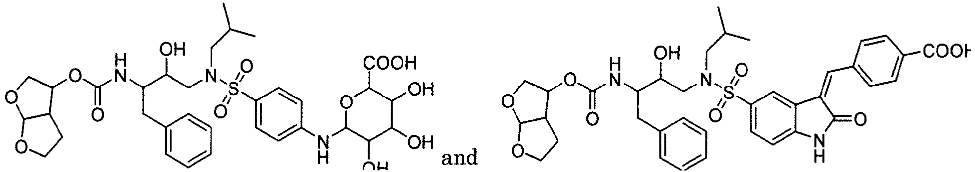
or a pharmaceutically acceptable salt thereof.

2. The compound or its pharmaceutically acceptable salt according to claim 1, wherein R² is a group represented by the formula: -Y-Z.

3. The compound or its pharmaceutically acceptable salt according to claim 1 or 2, wherein R⁴ is substituted or unsubstituted alkyl.

4. The compound or its pharmaceutically acceptable salt according to any one of claims 1 to 3, wherein Ring B is substituted or unsubstituted phenyl.

5. The compound or its pharmaceutically acceptable salt according to any one of claims 1 to 4, wherein Ring C is substituted or unsubstituted aromatic carbocyclylor substituted or unsubstituted bicyclic aromatic heterocyclyl.

6. The compound or its pharmaceutically acceptable salt according to claim 2, wherein Y is a bond, a group of formula: 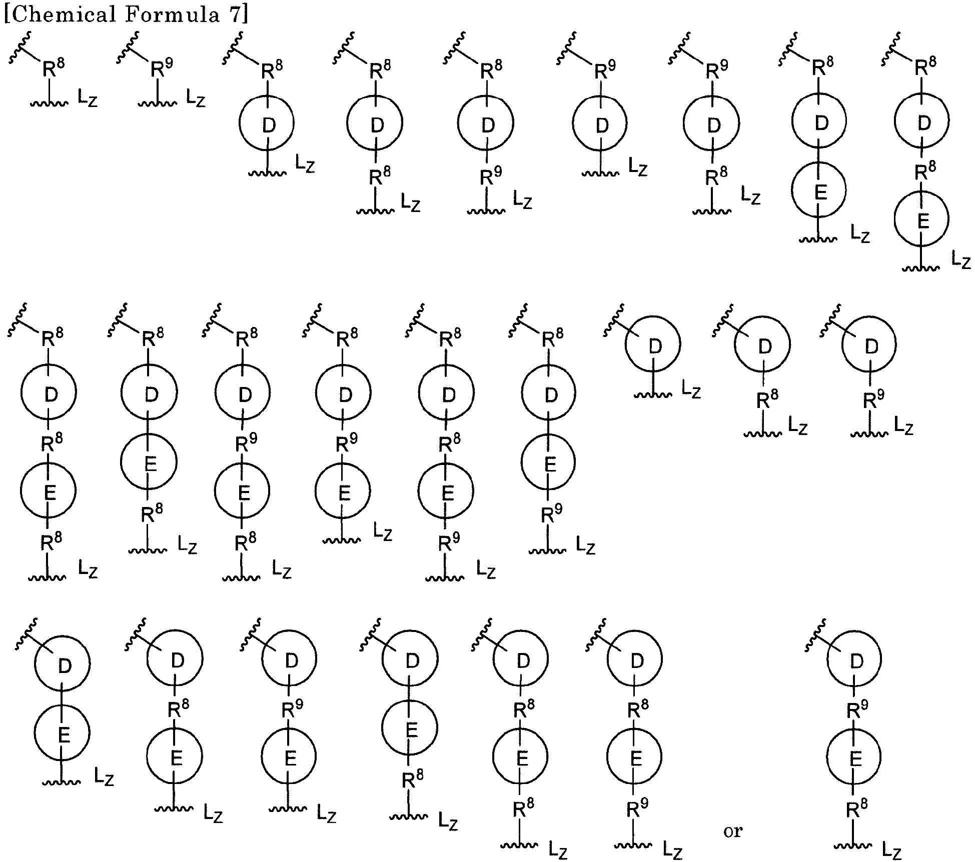
wherein a bond Lz is connecting to Z,
R⁸ are each independently -O-, -S-, -NR⁷-, substituted or unsubstituted alkylene which may be intervened with one or more groups selected from the group consisting of -O-, -NR⁷-, -C(=O)-NR⁷- and -NR⁷-C(=O)-, substituted or unsubstituted alkenylene which may be intervened with one or more groups selected from the group consisting of -O-, -NR⁷-, -C(=O)-NR⁷- and -NR⁷-C(=O)-, or substituted or unsubstituted alkynylene which may be intervened with one or more groups selected from the group consisting of -O-, -NR⁷-, -C(=O)-NR⁷- and -NR⁷-C(=O)-,
provided that R⁸ is excluded in the case that the groups selected from the group consisting of -O-, -NR⁷-, -C(=O)-NR⁷ and -NR⁷-C(=O)- are connected adjacently,
Ring D and Ring E are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R⁹ is -C(=O)-, -C(=O)-NR⁷-, -NR⁷-C(=O)-, -NR⁷-C(=O)-NR⁷-, -NR⁷SO₂-,-SO₂NR⁷-,
R⁷ is defined as the same in claim 1.

7. The compound or its pharmaceutically acceptable salt according to claim 6, wherein Y is a group of formula: 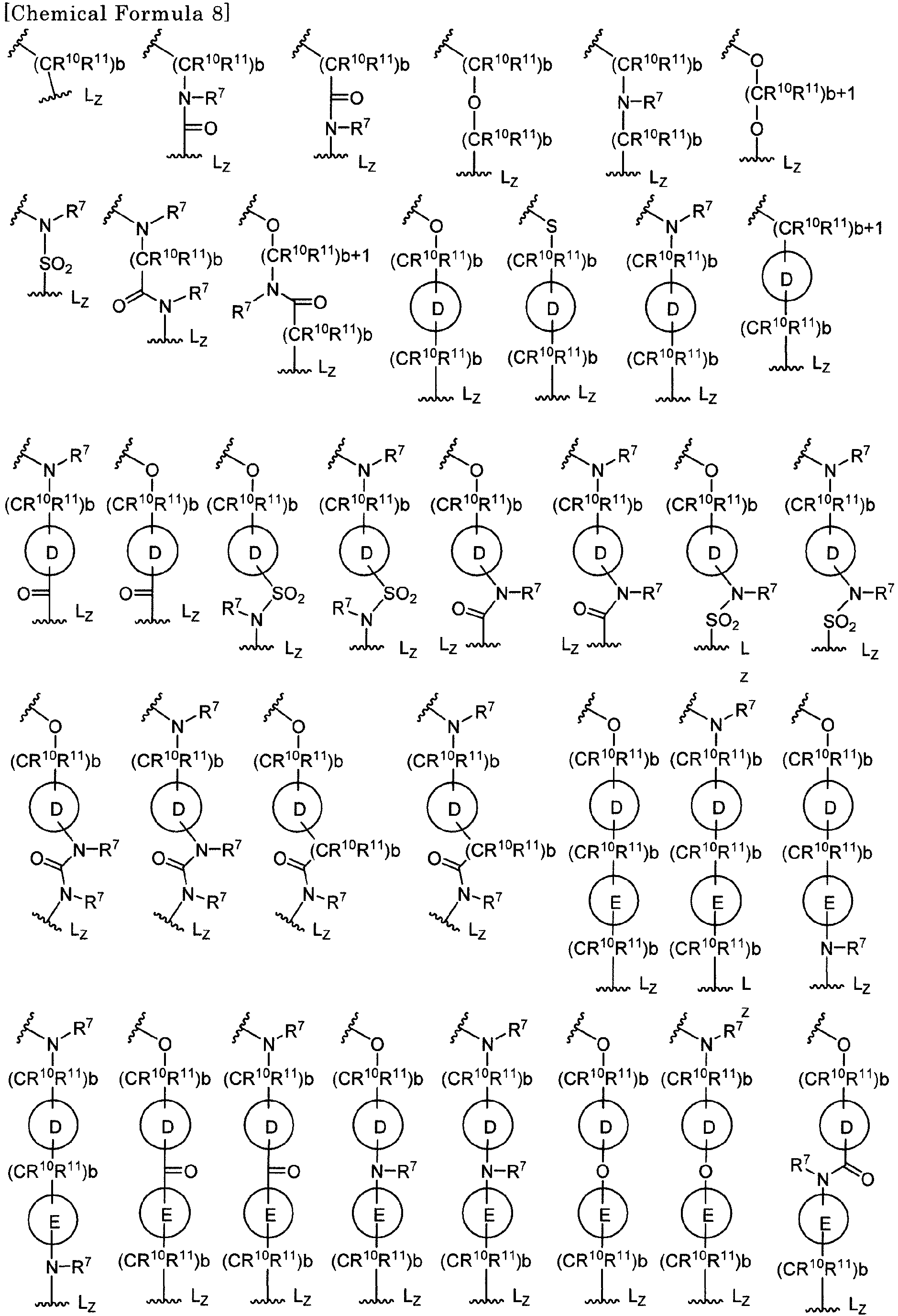 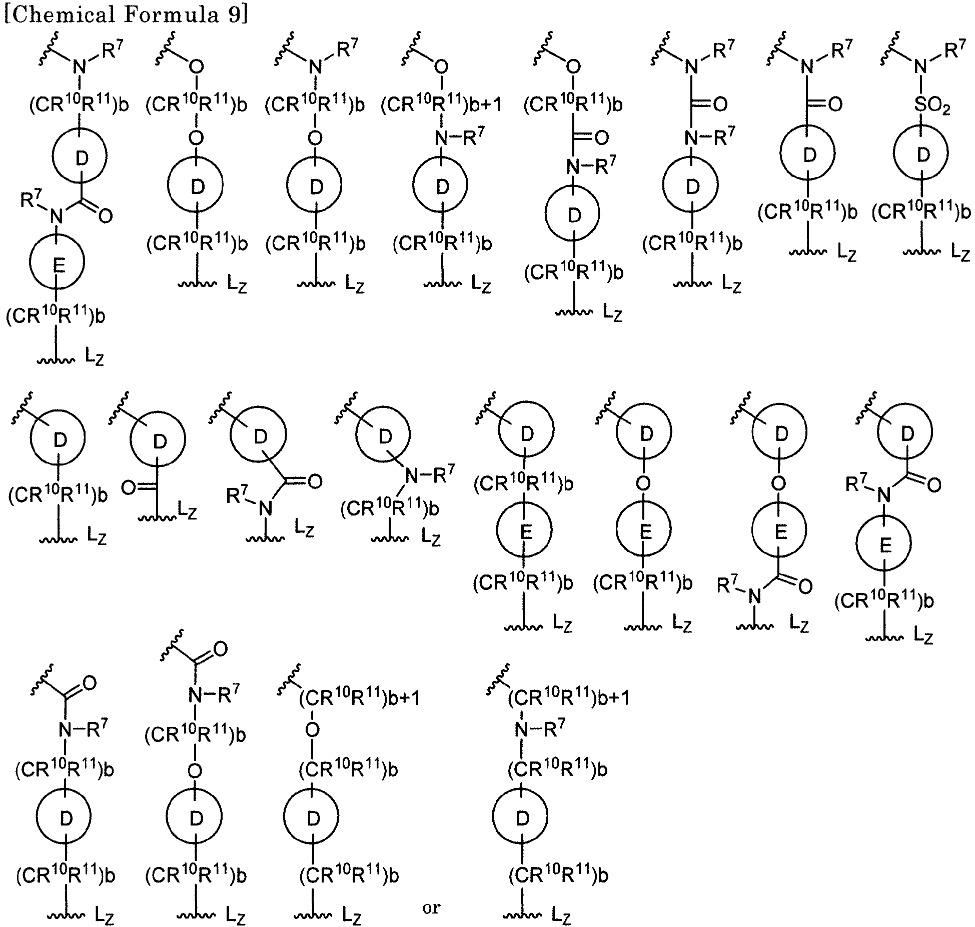 wherein a bond Lz is connecting to Z,
Ring D and Ring E are each independently substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R¹⁰ and R¹¹ are each independently a hydrogen atom, halogen, hydroxy, carboxy, sulfanyl, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
R¹⁰ and R¹¹ which are connected to a same carbon atom may be taken together to form substituted or unsubstituted imino, substituted or unsubstituted non-aromatic carbocyclyl, or non-aromatic heterocyclyl,
The two R¹⁰ and/or R¹¹ connected to the adjacent carbon atom may be taken together to form a bond,
R⁷ is defined as the same in claim 1,
b are each independently an integer of 0 to 4.

8. The compound or its pharmaceutically acceptable salt according to claim 6, wherein Y is a group of formula: 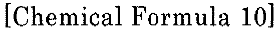 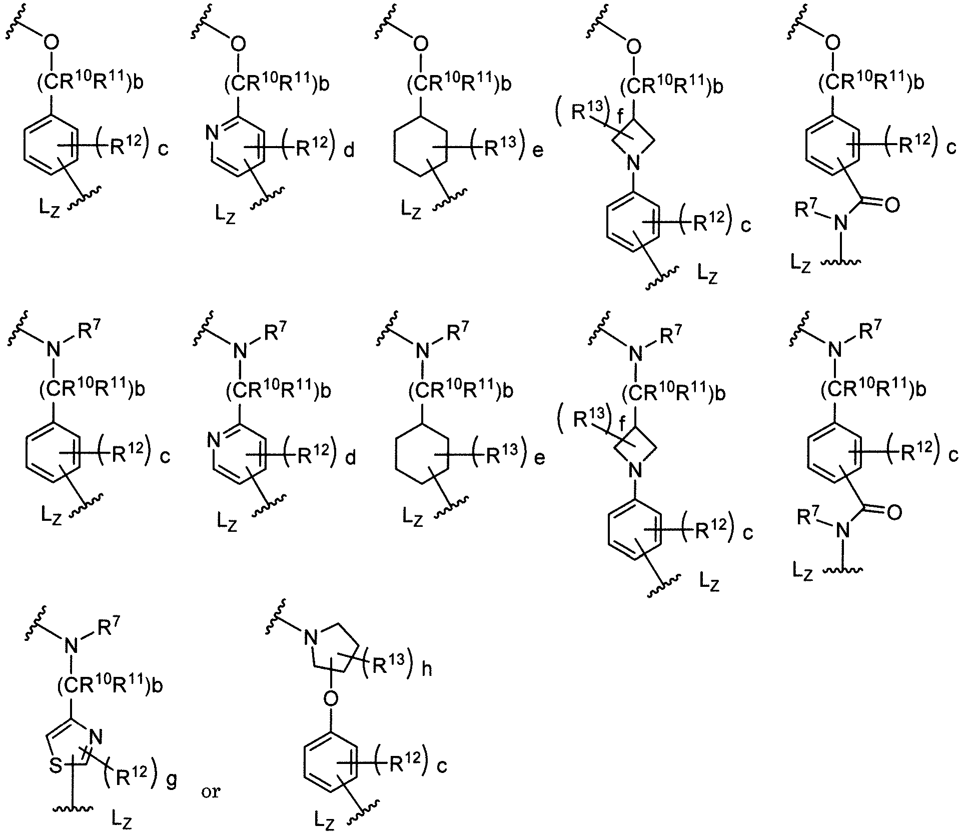
wherein a bond Lz is connecting to Z,
R¹² are each independently halogen, hydroxy, carboxy, sulfo, cyano, nitro, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
The two R¹² connected to the adjacent carbon atom composing ring may be taken together to form substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R¹³ are each independently halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
R¹³ connecting to the separated carbon atoms may be taken together to form alkylene,
R⁷ is defined as the same in claim 1,
R¹⁰, R¹¹ and b are defined as the same in claim 7,
c is an integer of 0 to 4,
d is an integer of 0 to 3,
e is an integer of 0 to 10,
f is an integer of 0 to 5,
g is 0 or 1,
h is an integer of 0 to 7.

9. The compound or its pharmaceutically acceptable salt according to any one of claims 1 to 8, wherein Z is bicyclic or tricyclic substituted non-aromatic carbocyclyl or bicyclic or tricyclic substituted non-aromatic heterocyclyl.

10. The compound or its pharmaceutically acceptable salt according to claim 9, wherein one of the substituents of bicyclic or tricyclic substituted non-aromatic carbocyclyl or bicyclic or tricyclic substituted non-aromatic heterocyclyl is -COOH or a its biologically equivalent group.

11. The compound or its pharmaceutically acceptable salt according to claim 10, wherein Z is a group of formula: 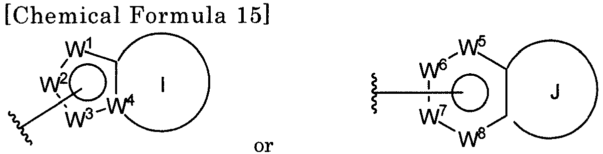
wherein W¹, W², W³, W⁵, W⁶, W⁷ and W⁸ are each independently C, CR²⁶, O, S, N or NR²⁷,
W⁴ is C, or N,
R²⁶ are each independently -COOH or a its biologically equivalent group, a hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
provided that at least one of W¹, W² and W³ is CR²⁶, and at least one of said R²⁶ is -COOH or a its biologically equivalent group,
provided that at least one of W⁵, W⁶, W⁷ and W⁸ is CR²⁶, and at least one of said R²⁶ is -COOH or a its biologically equivalent group,
R²⁷ are each independently a hydrogen atom, carboxy, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
Ring I and Ring J are each independently substituted or unsubstituted non-aromatic carbocyclyl, or substituted or unsubstituted non-aromatic heterocyclyl.

12. The compound or its pharmaceutically acceptable salt according to claim 11, wherein Z is a group of formula: 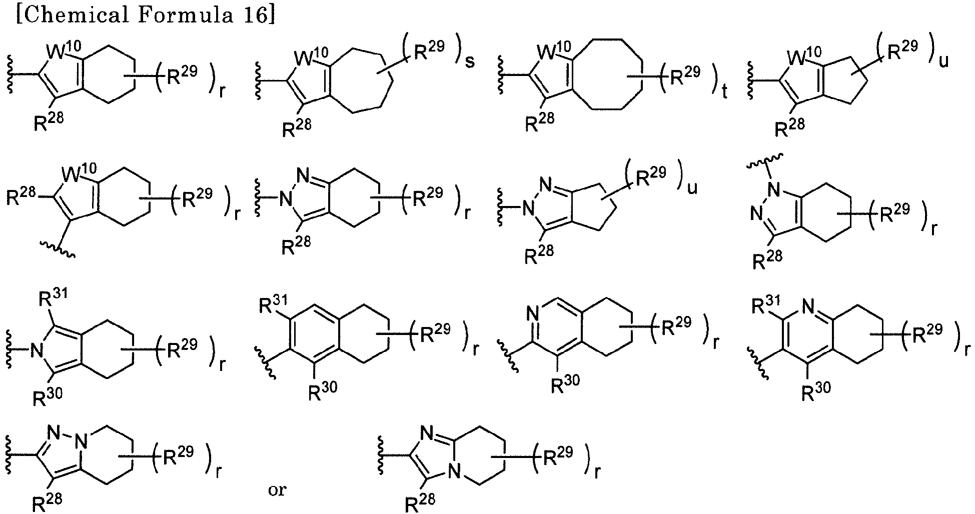
wherein W¹⁰ is -S-, -O- or -NR²⁷-,
R²⁷ is defined as the same in claim 11,
R²⁸ is -COOH or a its biologically equivalent group,
R³⁰ and R³¹ are each independently -COOH or a its biologically equivalent group, a hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
provided that at least one of R³⁰ and R³¹ is -COOH or a its biologically equivalent group,
R²⁹ are each independently halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
two R²⁹ connecting to adjacent carbon atoms may be taken together to form substituted or unsubstituted aromatic carbocyclyl, substituted or unsubstituted non-aromatic carbocyclyl, or substituted or unsubstituted non-aromatic heterocyclyl,
two R²⁹ connecting to the separated carbon atoms may be taken together to form substituted or unsubstituted alkylene,
two R²⁹ connecting to the same carbon atom may be taken together to form substituted or unsubstituted non-aromatic carbocyclylor substituted or unsubstituted non-aromatic heterocyclyl,
two R²⁹ connecting to the same carbon atom may be taken together to form oxo,
r is an integer of 0 to 8,
s is an integer of 0 to 10,
t is an integer of 0 to 12,
u is an integer of 0 to 6.

13. A compound represented by formula (IV):
X-Y-Z
wherein X is a compound residue of active ingredient,
Y is a bond, or a spacer of any combination selected from the group consisting of -O-, -S-, -NR⁷-, -C(=O)-, -SO-, -SO₂-, -NR⁷-C(=O)-, -C(=O)-NR⁷-, -NR⁷-C(=O)-NR⁷-, -NR⁷-C(=O)-O-, -SO₂-NR⁷-, -NR⁷-SO₂-, substituted or unsubstituted alkylene, substituted or unsubstituted alkenylene, substituted or unsubstituted alkynylene, substituted or unsubstituted aromatic carbocyclyldiyl, substituted or unsubstituted non-aromatic carbocyclyldiyl, substituted or unsubstituted aromatic heterocyclyldiyl, and substituted or unsubstituted non-aromatic heterocyclyldiyl,
provided that Y is excluded in the case connecting adjacently the group selected from the group consisting of -O-, -S- and -NR⁷ -; or -C(=O)-, -SO-, -SO₂ -,-NR⁷-C(=O)-, -C(=O)-NR⁷-, -NR⁷-C(=O)-NR⁷-, -O-C(=O)-NR⁷-, -NR⁷-C(=O)-O-, -SO₂-NR⁷- and -NR⁷-SO₂-,
R⁷ are each independently a hydrogen atom, hydroxy, carboxy, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkylcarbonyloxy, substituted or unsubstituted alkenylcarbonyloxy, substituted or unsubstituted alkynylcarbonyloxy, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, substituted or unsubstituted non-aromatic heterocyclylsulfonyl,
Z is a group of formula: 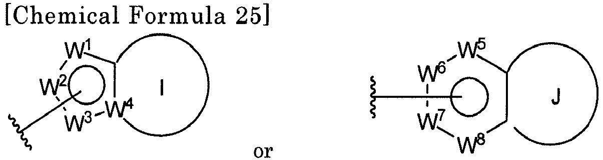
wherein W¹, W², W³, W⁵, W⁶, W⁷ and W⁸ are each independently C, CR²⁶, O, S, N or NR²⁷,
W⁴ is C or N,
R²⁶ are each independently -COOH or a its biologically equivalent group, a hydrogen atom, halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted alkylsulfonyl, substituted or unsubstituted alkenylsulfonyl, substituted or unsubstituted alkynylsulfonyl, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
provided that at least one of W¹, W² and W³ is CR²⁶, and at least one of said R²⁶ is -COOH or a its biologically equivalent group,
provided that at least one of W⁵, W⁶, W⁷ and W⁸ is CR²⁶, and at least one of said R²⁶ is -COOH or a its biologically equivalent group,
R²⁷ are each independently a hydrogen atom, carboxy, substituted or unsubstituted alkyl, substituted or unsubstituted alkenyl, substituted or unsubstituted alkynyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted carbamoyl, substituted or unsubstituted sulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, substituted or unsubstituted aromatic carbocyclylalkyl, substituted or unsubstituted non-aromatic carbocyclylalkyl, substituted or unsubstituted aromatic heterocyclylalkyl, substituted or unsubstituted non-aromatic heterocyclylalkyl, substituted or unsubstituted aromatic carbocyclylsulfonyl, substituted or unsubstituted non-aromatic carbocyclylsulfonyl, substituted or unsubstituted aromatic heterocyclylsulfonyl, or substituted or unsubstituted non-aromatic heterocyclylsulfonyl,
Ring I and Ring J are each independently substituted or unsubstituted non-aromatic carbocyclyl, or substituted or unsubstituted non-aromatic heterocyclyl,
A ring containing W¹ , W², W³ and W⁴ as atoms constituting a ring is an aromatic ring,
A ring containing W⁵, W⁶, W⁷ and W⁸ as atoms constituting a ring is an aromatic ring,
or a pharmaceutically acceptable salt thereof.

14. The compound or its pharmaceutically acceptable salt according to claim 13, wherein X is a compound residue having HIV protease inhibitor activity.

15. The compound or its pharmaceutically acceptable salt according to claim 14, wherein X is a residue of Amprenavir, Atazanavir, Darunavir, Fosamprenavir, Indinavir, Lopinavir, Ritonavir, Nelfinavir, Saquinavir, Tipranavir or its derivative.

16. The compound or its pharmaceutically acceptable salt according to claim 15, wherein X is a residue of Darunavir derivative or Atazanavir derivative.

17. The compound or its pharmaceutically acceptable salt according to any one of claims 13 to 16, wherein Z is a group of formula: 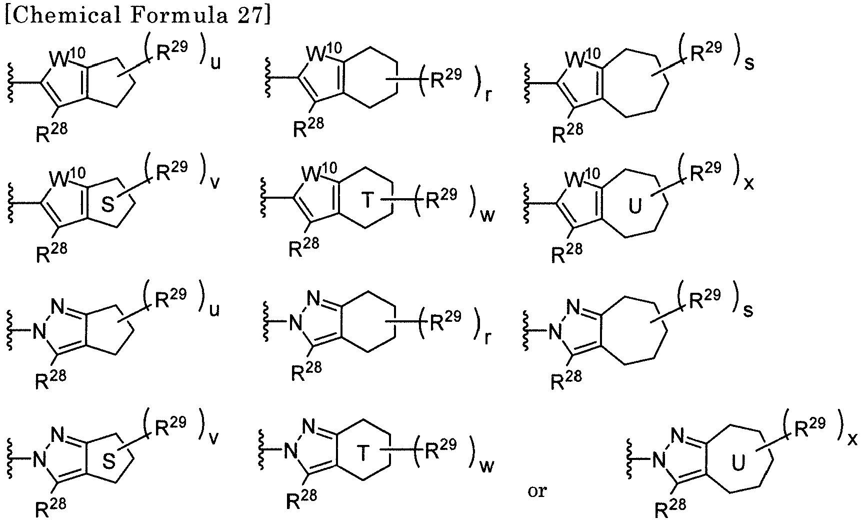
wherein W¹⁰ is -S-, -O- or -NR²⁷-,
Ring S is 5-membered non-aromatic heterocyclyl having a hetero atom selected from O, S or Nor²⁷,
provided that one of atoms on 5-membered ring other than condensed positional atoms is O, S or NR²⁷,
Ring T is 6-membered non-aromatic heterocyclyl having a hetero atom selected from O, S or NR²⁷,
provided that one of atoms on 6-membered ring other than condensed positional atoms is O, S or NR²⁷,
Ring U is 7-membered non-aromatic heterocyclyl having a hetero atom selected from O, Sor NR²⁷,
provided that one of atoms on 7-membered ring other than condensed positional atoms is O, S or NR²⁷,
R²⁸ is -COOH,
R²⁹ is each independently halogen, hydroxy, carboxy, cyano, ureido, amidino, guanidino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted amino, substituted or unsubstituted imino, substituted or unsubstituted alkylcarbonyloxy, substituted or unsubstituted alkenylcarbonyloxy, substituted or unsubstituted alkynylcarbonyloxy, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, or substituted or unsubstituted non-aromatic heterocyclyloxycarbonyl,
two R²⁹ connecting to adjacent carbon atoms may be taken together to form substituted or unsubstituted aromatic carbocyclyl, substituted or unsubstituted non-aromatic carbocyclyl, or substituted or unsubstituted non-aromatic heterocyclyl,
two R²⁹ connecting to the separated carbon atoms may be taken together to form substituted or unsubstituted alkylene,
two R²⁹ connecting to the same carbon atom may be taken together to form substituted or unsubstituted non-aromatic carbocyclylor substituted or unsubstituted non-aromatic heterocyclyl,
two R²⁹ connecting to the same carbon atom may be taken together to form oxo,
v are each independently an integer of 0 to 4,
w are each independently an integer of 0 to 6,
x are each independently an integer of 0 to 8.

18. The compound or its pharmaceutically acceptable salt according to any one of claims 13 to 16, wherein Z is a group of formula:

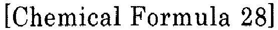

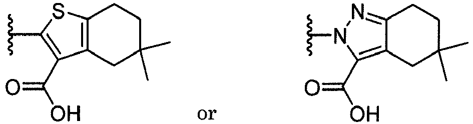

19. A compound represented by any one of the following formulae or its pharmaceutically acceptable salt.

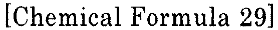

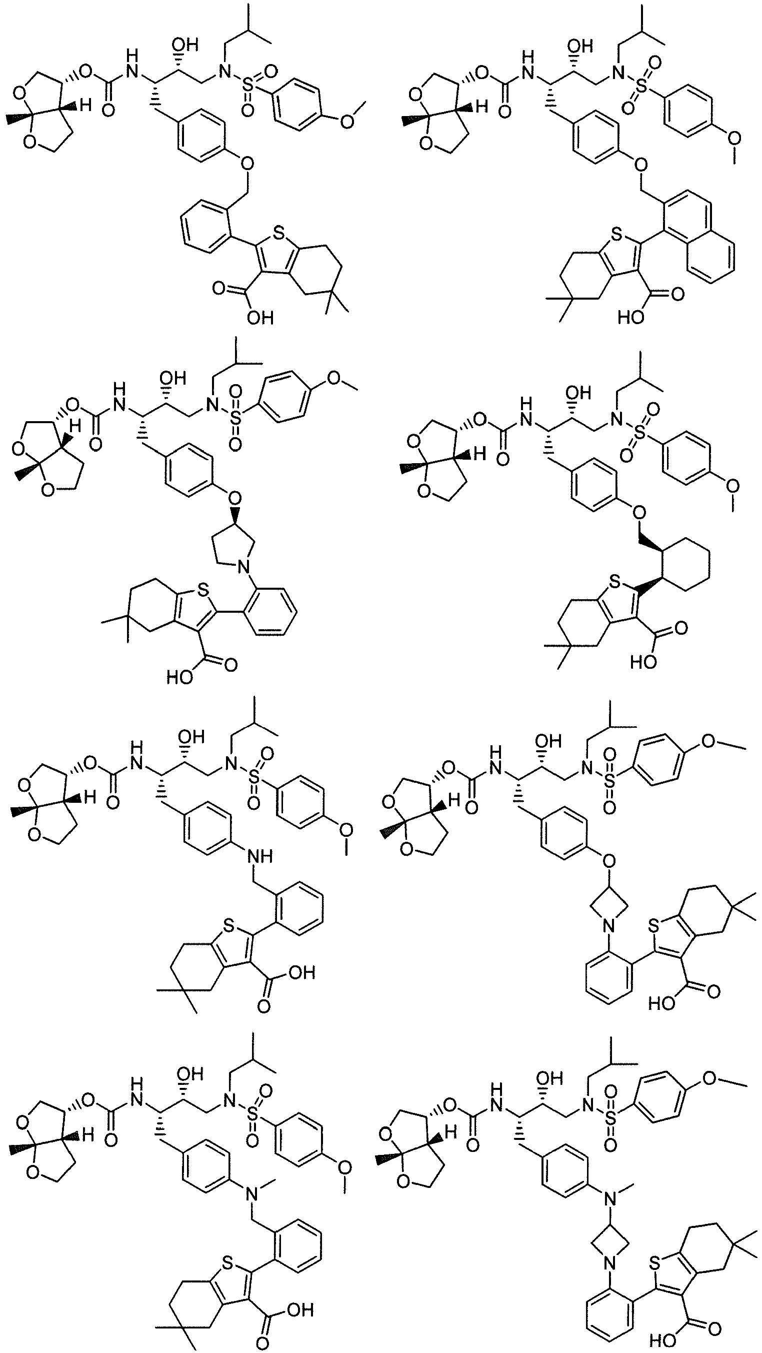

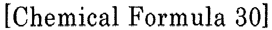

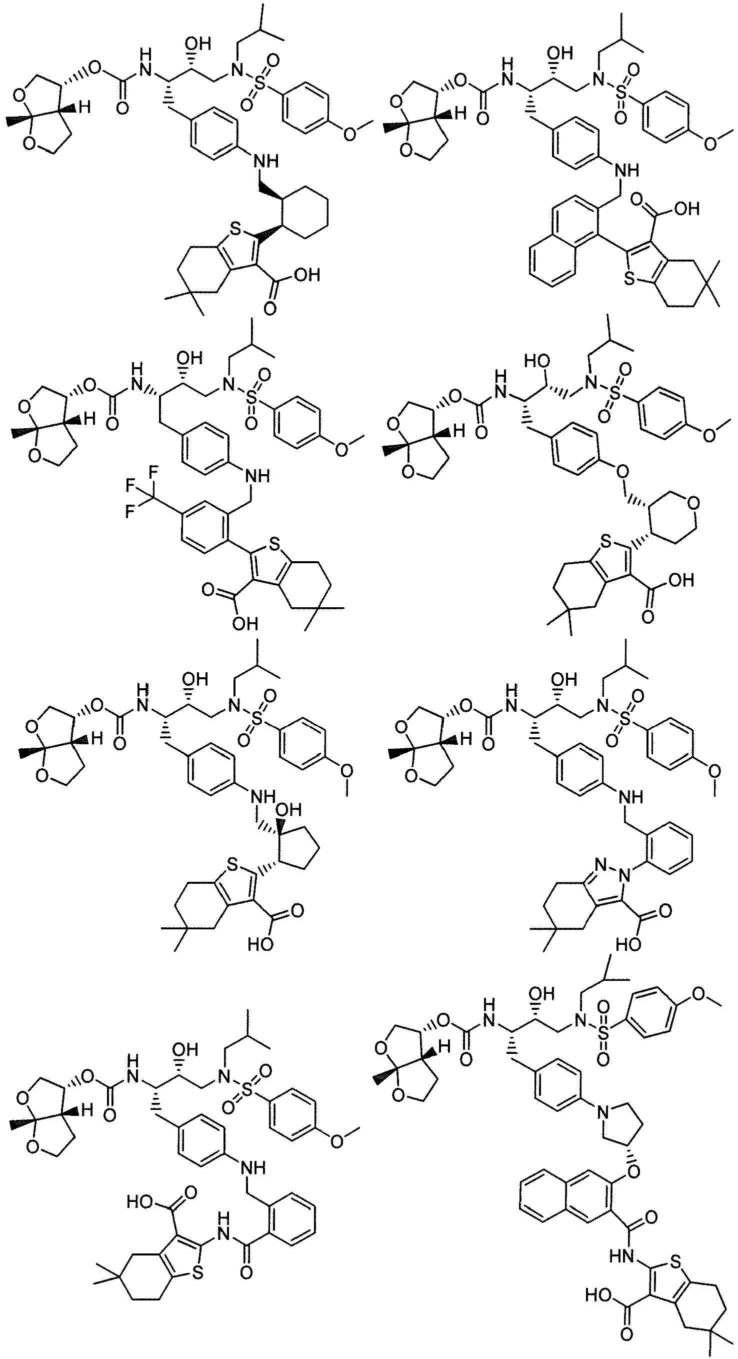

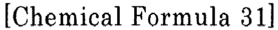

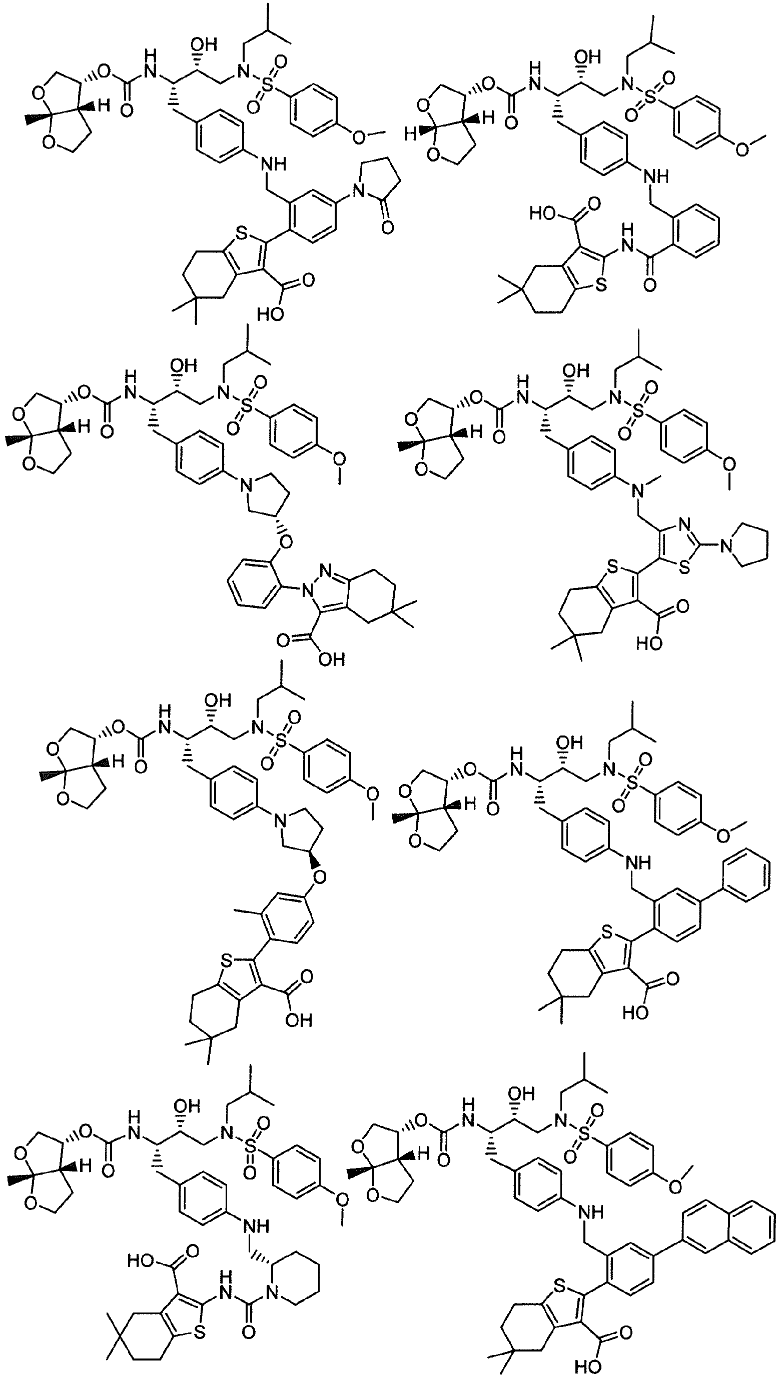

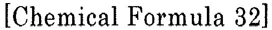

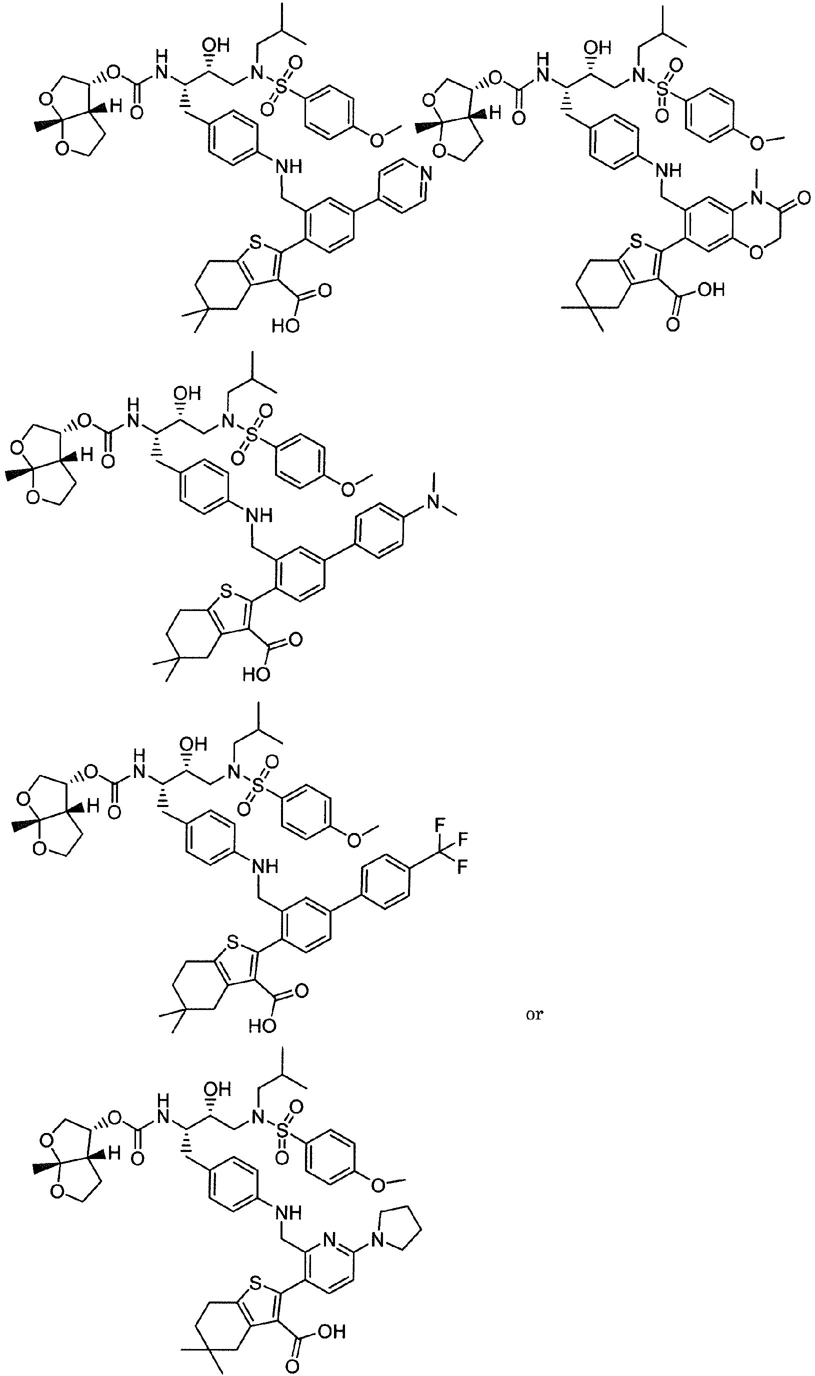

20. A method of lengthening half-life of active ingredient in pharmacokinetics and/or decreasing clearance by introducing a group represented by the following formula into active ingredient, 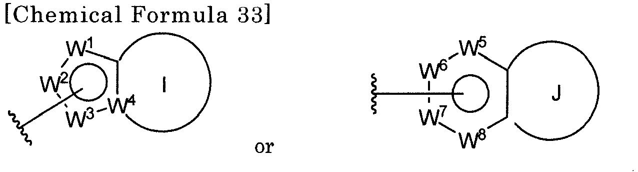 wherein each symbols are defined as the same in claim 13.

21. The method according to claim 20, wherein the group represented by the following formula: 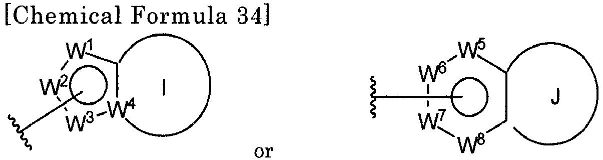 wherein each symbols are defined as the same in claim 13, is any one of the group represented by the following formulae: 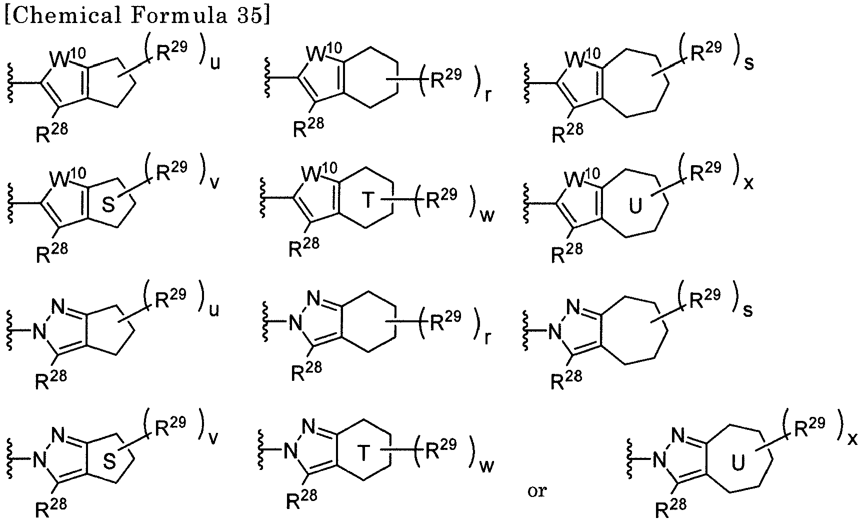 wherein each symbols are defined as the same in claim17.

22. The method according to claim 20, wherein the group represented by the following formula: 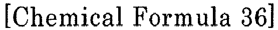 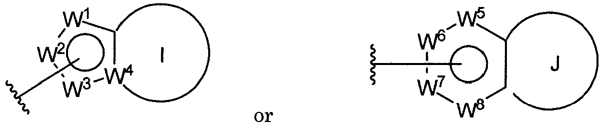 wherein each symbols are defined as the same in claim 13, is any one of the group represented by the following formula:

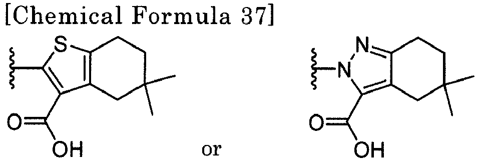

23. A pharmaceutical composition comprising the compound according to any of claims 1 to 19, or a pharmaceutically acceptable salt thereof.

24. The pharmaceutical composition according to claim 23, which has an HIV protease inhibitory activity.

25. The pharmaceutical composition according to claim 23 or 24, for medical treating or preventing HIV infection disease.

26. The pharmaceutical composition according to any one of claims 23 to 25, which is Long Acting injection.

27. The pharmaceutical composition according to any one of claims 23 to 26, wherein dosage interval is once in a month or more.

28. A method for treating or preventing HIV infection disease by administering the compound of any one of claims 1 to 19, or a pharmaceutically acceptable salt thereof.

29. The compound of any one of claims 1 to 19, or a pharmaceutically acceptable salt thereof for treating or preventing HIV infection disease.

30. A compound represented by the following formula: 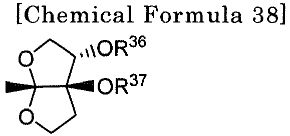
wherein R³⁶ is a hydrogen atom, a protecting group of hydroxy group or a group represented by the following formula: -C(=O)-R³⁸
wherein R³⁸ is leaving group,
R³⁷ is a protecting group of hydrogen atom or hydroxy group,
or a pharmaceutically acceptable salt thereof.

31. A compound represented by the following formula: 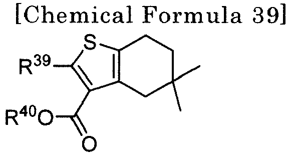
wherein R³⁹ is a hydrogen atom, halogen, boronic acid, boronate ester, or a group represented by formula: -OR⁴¹ or -NH(R⁴²),
R⁴¹ is methanesulfonyl group, trifluoromethylsulfonyl group, p-toluenesulfonyl group or nonafluorobutanesulfonyl group,
R⁴² is a protecting group of hydrogen atomor amino group,
R⁴⁰ is a protecting group of hydrogen atom or carboxy group,
provided that the following compounds are excluded: 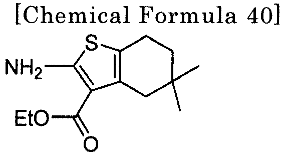 or a pharmaceutically acceptable salt thereof.

32. A compound represented by the following formula: 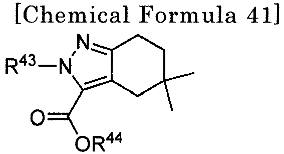
wherein R⁴³ is hydrogen, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, or a group represented by formula:-C(=O)-R⁴⁵ or -SO₂-R⁴⁶,
R⁴⁵ is substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted amino, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R⁴⁶ is substituted or unsubstituted alkyl, substituted or unsubstituted alkenyl, substituted or unsubstituted alkynyl, substituted or unsubstituted amino, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl,
R⁴⁴ is a protecting group of hydrogen atom or carboxy group,
provided that the following compounds are excluded: 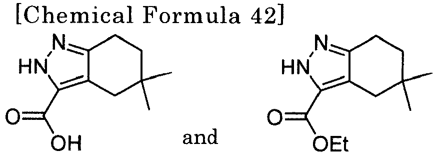 or a pharmaceutically acceptable salt thereof.

33. A compound represented by the following formula: 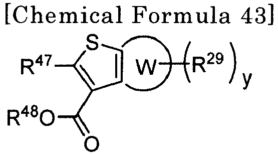
wherein Ring W is 5- to 8-membered non-aromatic carbocyclyl,
R²⁹ is defined as the same in claim 17,
when Ring W is 5-membered ring, y is an integer of 0 to 6,
when Ring W is 6-membered ring, y is an integer of 0 to 8,
when Ring W is 7-membered ring, y is an integer of 0 to 10,
when Ring W is 8-membered ring, y is an integer of 0 to 12,
R⁴⁷ is halogen, boronic acid, boronate ester, or a group represented by formula: -OR⁴⁹,
R⁴⁹ is methanesulfonyl group, trifluoromethylsulfonyl group, p-toluenesulfonyl group or nonafluorobutanesulfonyl group,
R⁴⁸ is a protecting group of hydrogen atom or carboxy group,
provided that the following compounds are excluded: 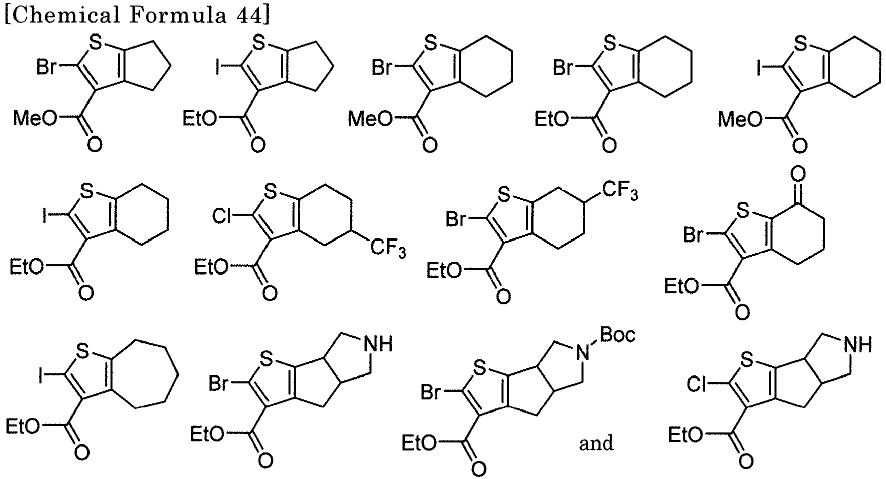
or a pharmaceutically acceptable salt thereof.

34. A compound represented by the following formula: 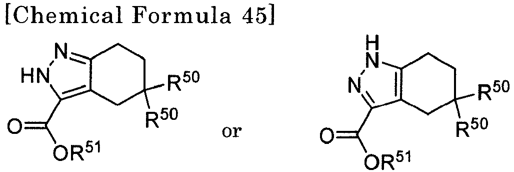
wherein R⁵⁰ are each independently a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or
substituted or unsubstituted non-aromatic heterocyclyl,
R⁵⁰ may be taken together to form substituted or unsubstituted non-aromatic carbocyclyl,
provided that R⁵⁰ is not a hydrogen atom at the same time,
R⁵¹ is a protecting group of hydrogen atom or carboxy group,
provided that the following compounds are excluded: 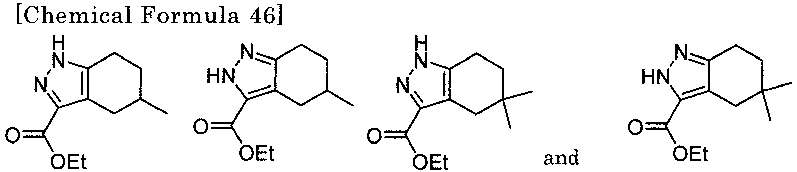
or a pharmaceutically acceptable salt thereof.
